# EUROPÄISCHE PATENTSCHRIFT

(11) **EP 2 215 092 B1**
(45) Veröffentlichungstag und Bekanntmachung des Hinweises auf die Patenterteilung: **25.01.2012**
(21) Anmeldenummer: 08839793.0
(22) Anmeldetag: 16.10.2008
(51) Int. Cl.: C07D 495/04, C07D 519/00, A61K 31/519, A61P 11/00

(54) **SUBSTITUIERTE PIPERIDINO-DIHYDROTHIENOPYRIMIDINE**
SUBSTITUTED PIPERIDINO-DIHYDROTHIENOPYRIMIDINES
PIPÉRIDINO-DIHYDROTHIÉNOPYRIMIDINES SUBSTITUÉES

(30) Priorität: 19.10.2007 EP 07118901
(43) Veröffentlichungstag der Anmeldung: 11.08.2010
(60) Teilanmeldung: 11174754.9
(73) Patentinhaber: Boehringer Ingelheim International GmbH, 55216 Ingelheim am Rhein (DE)
(72) Erfinder: POUZET, Pascale, 55216 Ingelheim am Rhein (DE); ANDERSKEWITZ, Ralf, 55216 Ingelheim am Rhein (DE); DOLLINGER, Horst, 55216 Ingelheim am Rhein (DE); FIEGEN, Dennis, 55216 Ingelheim am Rhein (DE); FOX, Thomas, 55216 Ingelheim am Rhein (DE); GOEGGEL, Rolf, 55216 Ingelheim am Rhein (DE); HOENKE, Christoph, 55216 Ingelheim am Rhein (DE); MARTYRES, Domnic, 55216 Ingelheim am Rhein (DE); NICKOLAUS, Peter, 55216 Ingelheim am Rhein (DE); KLINDER, Klaus, 55216 Ingelheim am Rhein (DE)
(74) Vertreter: Hammann, Heinz
(86) Internationale Anmeldenummer: PCT/EP2008/063999
(87) Internationale Veröffentlichungsnummer: WO 2009/050248

(56) Entgegenhaltungen:
- EP-A- 1 847 543
- WO-A-2006/111549
- CHAKRABORTI ET AL.: "3D-QSAR Studies on thieno[3,2-d]pyrimidines as Phosphodiesterase IV Inhibitors" BIOORGANIC & MEDICINAL CHEMISTRY LETTERS, OXFORD, GB, Bd. 13, Nr. 8, 2003, Seiten 1403-1408, XP002392463 ISSN: 0960-894X

## Beschreibung

Die Erfindung betrifft neue Piperidino-Dihydrothienopyrimidinsulfoxide der Formel 1, sowie pharmakologisch verträgliche Salze, Diastereomere, Enantiomere, Racemate, Hydrate oder Solvate davon, 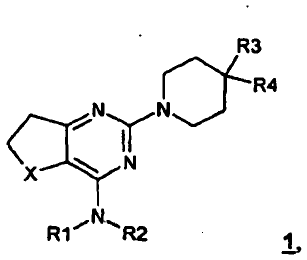
worin X SO oder SO₂, vorzugsweise jedoch SO, ist und worin
R¹ R², R³ und R⁴ die in Anspruch 1 genannten Bedeutungen haben kann, sowie pharmazeutische Zusammensetzungen, die diese Verbindungen beinhalten.

Diese neuen Piperidino-Dihydrothienopyrimidinsulfoxide sind geeignet zur Behandlung von Atemwegs- oder gastrointestinalen Beschwerden oder Erkrankungen, entzündlichen Erkrankungen der Gelenke, der Haut oder der Augen, Erkrankungen des periphären oder zentralen Nervensystems oder Krebserkrankungen.

### STAND DER TECHNIK

US 3,318,881 und BE 663693 offenbaren die Herstellung von Piperazino-Dihydrothieno-[3,2-d]pyrimidinen, die kardiovaskulären und sedative Eigenschaften besitzen. WO 2006/111549 und EP06112779.1 (EP1847543) offenbaren jeweils Dihydrothienopyrimidinsulfoxide, die mit Piperazin anstelle von Piperidin substituiert sind.

### BESCHREIBUNG DER ERFINDUNG

Überraschenderweise konnte nun gefunden werden, dass neben Piperazino-Dihydrothienopyrimidinsulfoxiden auch Piperidino-Dihydrothienopyrimidinsulfoxide der Formel **1**, in denen R³ und R⁴ die in Anspruch 1 definierten. Bedeutungen haben, insbesondere solche, in denen X SO bedeutet, besonders geeignet sind zur Behandlung entzündlicher Erkrankungen und gegenüber den entsprechenden Piperazino-Dihydrothienopyrimidinsulfoxiden aus dem Stand der Technik überlegen sind.

Gegenstand der vorliegenden Erfindung sind deshalb Verbindungen der Formel 1 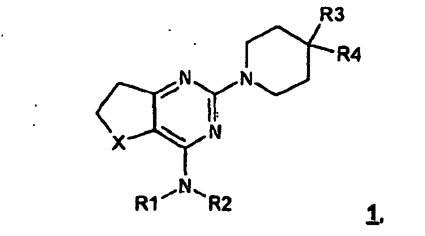 worin
- **X**: SO oder SO₂,
- **R¹**: H, C₁₋₆-Alkyl,
- **R²**: H ist oder ein Rest ausgewählt aus der Gruppe bestehend aus C₁₋₁₀-Alkyl und C₂₋₆-Alkenyl ist, der gegebenenfalls durch einen oder mehrere Reste ausgewählt aus Halogen und C₁₋₃-Fluoroalkyl substituiert sein kann oder der gegebenenfalls durch einen oder mehrere Reste ausgewählt aus der Gruppe bestehend aus OR^{2.1}, COOR^{2.1},CONR^{2.2}R^{2.3}, SR^{2.1},SO-R^{2.1}, SO₂-R^{2.1}, C₆₋₁₀-Aryl, -Het, Hetaryl, einem mono- oder bicyclischem -C₃₋₁₀-Cycloalkyl, CH₂-NR^{2.2}R^{2.3} und NR^{2.2}R^{2.3} substituiert sein kann, welcher wiederum gegebenenfalls durch einen oder mehrere Reste ausgewählt aus der Gruppe bestehend aus OH, Halogen, OR^{2.1} Oxo, CF₃, CHF₂, CH₂F, C₁₋₆-Alkyl, C₁₋₆-Alkanol, C₆₋₁₀-Aryl, COOR^{2.1}, CH₂-NR^{2.2}R^{2.3} und NR^{2.2}R^{2.3} substituiert sein kann,
worin
- **Het**: ein drei- bis elfgliedriger, mono- oder bicyclischer, gesättigter oder teilweise gesättigter, gegebenenfalls annellierter oder gegebenenfalls überbrückter Heterocyclus ist, der 1, 2, 3 oder 4 Heteroatome unabhängig voneinander ausgewählt aus der Gruppe bestehend aus N, S oder O enthält, und wobei
- **Hetaryl**: ein fünf- bis zehngliedriger, mono- oder bicyclisches, gegebenenfalls annelliertes Heteroaryl ist, das 1, 2, 3 oder 4 Heteroatome unabhängig voneinander ausgewählt aus der Gruppe bestehend aus N, S oder O enthält,
und wobei
- **Cycloalkyl**: gesättigt oder teilweise gesättigt sein kann,
- wobei R^{2.1}: H ist oder ein Rest ausgewählt ist aus der Gruppe bestehend aus C₁₋₆-Alkyl, C₁₋₆-Alkanol, C₁₋₃-Haloalkyl, mono- oder bicyclisches, -C₃₋₁₀-Cycloalkyl, C₆₋₁₀-Aryl-C₁₋₆-alkylen, Hetaryl-C₁₋₆-alkylen-, Het-C₁₋₆-alkylen-, C₃₋₁₀-Cycloalkyl-C₁₋₆-alkylen-, ein mono- oder bicyclisches C₆₋₁₀-Aryl, Heteroaryl und ein -Het, der gegebenenfalls durch einen oder mehrere Reste ausgewählt aus der Gruppe bestehend aus OH, O-(C₁₋₃-Alkyl), Halogen, C₁₋₄-Alkyl und C₆₋₁₀-Aryl substituiert sein kann,
- wobei R^{2.2} und R^{2.3}: unabhängig voneinander H sind oder ein Rest ausgewählt sind aus der Gruppe bestehend aus C₁₋₆-Alkyl, mono- oder bicyclisches C₃₋₁₀ Cycloalkyl, C₆₋₁₀₋Aryl-C₁₋₆-alkylen, Hetaryl-C₁₋₆-alkylen, mono- oder bicyclisches C₆₋₁₀-Aryl, Het, Hetaryl, CO-NH₂, CO-NHCH₃,-CO-N(CH₃)₂, SO₂-(C₁-C₂-Alkyl), CO-R^{2.1} und COOR^{2.1}, der gegebenenfalls durch einen oder mehrere Reste ausgewählt aus der Gruppe bestehend aus OH, Halogen, C₁₋₆-Alkyl, C₆₋₁₀-Aryl und COOR^{2.1} substituiert sein kann,
oder
- R²: ein mono- oder polycyclisches C₃₋₁₀ Cycloalkyl, das gegebenenfalls einfach oder mehrfach über C₁₋₃-Alkylgruppen verbrückt sein kann und das gegebenenfalls mit einem Rest ausgewählt aus der Gruppe bestehend aus verzweigtes oder unverzweigtes C₁₋₆-Alkanol, C₁₋₃-Fluoroalkyl, C₁₋₃-alkylen-OR^{2.1}, OR^{2.1}, COOR^{2.1}, -SO₂-NR^{2.2}R^{2.3}, Het, -NH-CO-O-(C₁₋₆-Alkyl), -NH-CO-(C₁₋₆-Alkyl), -NH-CO-O-(C₆₋₁₀-Aryl), -NH-CO-(C₆₋₁₀-Aryl), -NH-CO-O-Hetaryl, -MH-CO-Hetaryl, -NH-CO-O-(C₁₋₃-Alkylen)-(C₆₋₁₀-Aryl), -NH-CO-(C₁₋₃-Alkylen)-(C₆₋₁₀-Aryl), -N(C₁₋₃-Alkyl)-CO-(C₁₋₆-Alkyl), -N(C₁₋₃-Alkyl)-CO-O-(C₆₋₁₀-Aryl), -N(C₁₋₃-Alkyl)-CO-(C₆₋₁₀-Aryl), -N(C₁₋₃-Alkyl)-CO-O-Hetaryl, -N(C₁₋₃-Alkyl)-CO-Hetaryl, -N(C₁₋₃-Alkyl)-CO-O-(C₁₋₃-Alkylen)-(C₆₋₁₀₋Aryl),-N(C₁₋₃-Alkyl)-CO-(C₁₋₃-Alkylen)-(C₆₋₁₀₋Aryl), C₆₋₁₀₋Aryl, C₁₋₆-Alkyl, C₆₋₁₀-Aryl-C₁₋₆-alkylen, Hetaryl-C₁₋₆-alkylen, mono- oder bicyclisches C₃₋₁₀ Cycloalkyl und NR^{2.2}R^{2.3} substituiert sein kann, der gegebenenfalls durch einen oder mehrere Reste ausgewählt aus der Gruppe bestehend aus OH, OR^{2.1}, Oxo, Halogen, CF₃, CHF₂, CH₂F, C₁₋₆-Alkyl, C₆₋₁₀-Aryl und NR^{2.2}R^{2.3} substituiert sein kann,
oder
- **R²**: ein mono- oder polycyclisches C₆₋₁₀-Aryl, das gegebenenfalls durch OH, SH oder Halogen oder durch einen oder mehrere Reste ausgewählt aus der Gruppe bestehend aus OR^{2.1}, COOR^{2.1}, NR^{2.2}R^{2.3}, CH₂-NR^{2.2}R^{2.3},C₃₋₁₀-Cycloalkyl, Het, C₁₋₆-Alkyl, C₁₋₃-Fluoroalkyl, CF₃, CHF₂, CH₂F, C₆₋₁₀-Aryl-C₁₋₆-alkylen, Het-C₁₋₆-alkylen, Hetaryl-C₁₋₆-alkylen, C₆₋₁₀-Aryl, SO₂-CH₃, SO₂-CH₂CH₃ und SO₂-NR^{2.2}R^{2.3} substituiert sein kann, der wiederum gegebenenfalls durch einen oder mehrere oder mehrere Reste ausgewählt aus der Gruppe bestehend aus OH, OR^{2.1}, CF₃, CHF₂, CH₂F, Oxo, Halogen, CF₃, CHF₂, CH₂F, C₁₋₆-Alkyl, C₆₋₁₀-Aryl und NR^{2.2}R^{2.3} substituiert sein kann,

oder
- **R²**: ein Rest ausgewählt aus einer Gruppe bestehend aus Het und Hetaryl, welcher gegebenenfalls durch einen oder mehrere Reste ausgewählt aus der Gruppe Halogen, OH, Oxo, CF₃, CHF₂ und CH₂F oder durch einen oder mehrere Reste ausgewählt aus der Gruppe OR^{2.1}, C₁₋₃-alkylen-OR^{2.1}, SR^{2.1},SO-R^{2.1}, SO₂-R^{2.1}, COOR^{2.1}, COR^{2.1}, C₁₋₆-Alkanol, mono- oder bicyclisches C₃₋₁₀-Cycloalkyl, C₆₋₁₀-Aryl, C₁₋₆-Alkyl, C₆₋₁₀-Aryl-C₁₋₆-alkylen, Hetaryl-C₁₋₈-alkylen, Het, Hetaryl, C₁₋₃-alkylen-OR^{2.1} und NR^{2.2}R^{2.3} substituiert sein kann, der gegebenenfalls wiederum durch einen oder mehrere Reste ausgewählt aus der Gruppe bestehend aus OH, OR^{2.1}, Oxo, Halogen, CF₃, CHF₂, CH₂F, C₁₋₆-Alkyl, C₆₋₁₀-Aryl und NR^{2.2}R^{2.3} substituiert sein kann,
oder worin
- **NR¹R²**: gemeinsam einen heterocyclischen C₄₋₇-Ring bedeutet, der gegebenenfalls überbrückt sein kann, der 1, 2 oder 3 Heteroatome ausgewählt aus der Gruppe bestehend aus N, O und S enthält und der gegebenenfalls durch einen oder mehrere Reste ausgewählt aus der Gruppe bestehend aus OH, OR^{2.1}, C₁₋₃-alkylen-O^{R.1}, Oxo, Halogen, C₁₋₆-Alkyl, C₆₋₁₀-Aryl, COOR^{2.1}, CH₂-NR^{2.2}-COO-R^{2.1}, CH₂-NR^{2.2}-CO-R^{2.1}, CH₂-NR^{2.2}-CO-CH₂-NR^{2.2}R^{2.3}, CH₂-NR^{2.2}SO₂-C₁₋₃-Alkyl, CH₂-NR^{2.2}-SO₂-NR^{2.3}R^{2.3}, CH₂-NR^{2.2}-CO-NR^{2.2}R^{2.3}, CO-NR^{2.2}R^{2.3}, CH₂-NR^{2.2}R^{2.3} und NR^{2.2}R^{2.3} substituiert sein kann,
und worin
- **R³**: ein C₆₋₁₀-Aryl ist, welches gegebenenfalls in ortho, para oder meta-Stellung mit einem, zwei oder drei Resten unabhängig voneinander ausgewählt aus der Gruppe bestehend aus Fluor, Chlor, Brom, Hydroxy, CN, C₁₋₆-Alkyl, C₁₋₃-Fluoroalkyl, -C₁₋₃-alkylen-OR^{2.1}, -C₁₋₃-alkylen-NR^{2.2}R^{2.3}, -NR^{2.2}R^{2.3}, O-R^{2.1}, SO-R^{2.1}, SO₂R^{2.1}, COOR^{2.1}, -CO-NH-(C₁₋₆-Alkylen)-Hetaryl, -CO-NH-Hetaryl, -CO-N(CH₃)-Het -CO-N(CH₃)-(C₁₋₃-Alkylen)-Het,-CO-N(CH₃)-(C₁₋₃-Alkylen)-Hetaryl, -CO-N(C₃₋₇-Cycloalkyl)-Het, -CO-NR^{2.2}R^{2.3}, -CO-NH-(C₁₋₆-Alkylen)-Het, NR^{2.2}-CO-R^{2.1}, C₆₋₁₀-Aryl, C₆₋₁₀-Aryl-C₁₋₂-alkylen, Het-C₁₋₂- alkylen, -Het, -CO-Het, , CO-N(CH₃)-C₃₋₇-Cycloalkyl, C₃₋₇-Cycloalkyl, C₃₋₇-Cycloalkyl-C₁₋₂-alkylen, Hetaryl-C₁₋₂-alkylen und Hetaryl substituiert ist, wobei dieser Rest gegebenenfalls durch einen oder mehrere Reste ausgewählt aus der Gruppe bestehend aus OH, Halogen, -C₁₋₃-Fluoroalkyl, Oxo, Methyl und Phenyl substituiert sein kann,
oder worin
- R³: ein Rest ausgewählt aus der Gruppe bestehend aus Het und Hetaryl ist, der gegebenenfalls durch einen oder mehrere Reste ausgewählt aus der Gruppe bestehend aus Halogen, C₁₋₃-Fluoroalkyl, CN, OH, Oxo, -C₁₋₆-Alkyl, -C₁₋₃-alkylen-NR^{2.2}R^{2.3}, -NR^{2.2}R^{2.3}, SO-R^{2.1}, SO₂-R^{2.1}, -O-R^{2.1}, -COOR^{2.1}, SO₂-(CH₃), SO₂-(CH₂-CH₃), C₆₋₁₀-Aryl, Het, C₃₋₇-Cycloalkyl und Hetaryl substituiert sein kann, der wiederum gegebenenfalls mit einem oder mehreren Resten ausgewählt aus der Gruppe OH, Halogen, -C₁₋₃-Fluoroalkyl, C₁₋₄-Alkyl, C₆₋₁₀-Aryl, -COO(C₁₋₃-Alkyl) und O-(C₁₋₃-Alkyl) substituiert sein kann,
oder worin
- **R³**: -O-R^{3.1},
- wobei R^{3.1}: ein Rest ausgewählt aus der Gruppe bestehend aus -C₁₋₆-Alkyl, -C₆₋₁₀-Aryl,-C₁₋₃-Alkylen-C₆₋₁₀-Aryl, Hetaryl und Het ist, welcher gegebenenfalls in ortho, para oder meta-Stellung mit einem, zwei oder drei Resten unabhängig voneinander ausgewählt aus der Gruppe bestehend aus Fluor, Chlor, Brom, Hydroxy, CN, C₁₋₆-Alkyl, C₁₋₃-Fluoroalkyl, CO-(C₁₋₅-Alkyl), -CO-(C₁₋₃-Fluoroalkyl), -CO-NH-(C₁₋₆-Alkylen)-Hetaryl, -CO-N(C₁₋₃-Alkyl)-(C₁-₆-Alkylen)-Hetaryl, -CO-N(C₁₋₃-Alkyl)-Het, -CO-N(C₃₋₇-Cycloalkyl)-Het, -C₁₋₃-alkylen-OR^{2.1}, -C₁₋₃-alkylen-NR^{2.2}R^{2.3}, -NR^{2.2}R^{2.3}, O-R^{2.1}; SO-R^{2.1}, SO₂-R^{2.1}, COOH, COO-(C₁₋₄-Alkyl), -O-C₁₋₃-Alkylen-N(C₁₋₃-Alkyl)₂, CO-NR^{2.2}R^{2.3}, NR^{2.2}-CO-R^{2.1}, C₆₋₁₀-Aryl, C₆₋₁₀-Aryl-C₁₋₂-alkylen, Het-C₁₋₂-alkylen, -CO-Het, Het, -CO-C₃₋₇-Cycloalkyl, -CO-N(C₁₋₃-Alkyl)-C₃₋₇Cycloalkyl C₃₋₇-Cycloalkyl, C₃₋₇-Cycloalkyl-C₁₋₂-alkylen, Hetaryl-C₁₋₂-alkylen und Hetaryl substituiert ist, welcher wiederum gegebenenfalls mit 1, 2, 3 oder 4 Resten unabhängig voneinander ausgewählt aus der Gruppe bestehend aus F, Cl, Br, Methyl, O-Methyl, Ethyl, O-Ethyl, OH, Oxo und CH₂, substituiert sein kann.
und worin
- **R⁴**: H, CN, OH, CF₃, CHF₂, CH₂F, F, Methyl, Ethyl, -O-(C₁₋₃-Alkyl), -C₁₋₃-Alkylen-OH, -COO(C₁₋₃-Alkyl), -CO-Het, -(C₁₋₂-Alkylen)-NH-SO₂-(C₁₋₂-Alkyl), -(C₁₋₂-Alkylen)-N(C₁₋₃-Alkyl)-SO₂-(C₁₋₂-Alkyl), -(C₁₋₂-Alkylen)-O-(C₁₋₂-Alkylen)-C₆₋₁₀-Aryl, -C₁₋₃-Alkylen-O-C₁₋₃-Alkyl, -(C₁₋₂-Alkylen)-N(C₁₋₃-Alkyl)-CO-(C₁₋₂-Alkyl), -NH-CO-(C₁₋₃-Alkylen)-O-(C₁₋₃-Alkyl), -C₁₋₃-Alkylen-NH-CO-(C₁₋₃-Alkyl), -C₁₋₃-Alkylen-NH-CO-(C₁₋₃-Alkylen)-N(C₁₋₃-Alkyl)₂, -O-(C₁₋₂-Alkylen)-(C₆₋₁₀-Aryl), -C₁₋₃-Alkylen-NH-CO-(C₁₋₃-Alkylen)-O-(C₁₋₃-Alkyl), -CO-(C₆₋₁₀-Aryl), -(C₁₋₂-Alkylen)-N(C₁₋₃-Alkyl)-CO-(C₁₋₂-Alkylen)-O-(C₁₋₃-Alkyl), wobei das Aryl in den obigen Resten gegebenenfalls wiederum mit einem oder mehreren weiteren Resten ausgewählt aus der Gruppe F, Cl, Br, Methyl, Ethyl, Propyl, Isopropyl, Cyclopropyl, -O-Methyl, -O-Ethyl, -O-Propyl, -O-Isopropyl, -O-Cyclopropyl, -OH und CF₃ substituiert sein kann
oder worin
- **R³** und **R⁴**: gemeinsam einen mono- oder bicyclischen, ungesättigten, gesättigten oder teilweise gesättigten Heterocyclus bilden, der 1, 2 oder 3 Heteroatome ausgewählt aus der Gruppe bestehend aus N, O und S enthält und der gegebenenfalls mit einem oder mehreren Resten ausgewählt aus der Gruppe bestehend aus Halogen, OH, Oxo, C₁₋₃-Fluoroalkyl, CN, C₁₋₆-Alkyl, -O-R^{2.1},-COOR^{2.1}, SO-R^{2.1}, SO₂-R^{2.1}, -C₁₋₃-alkylen-NR^{2.2}R^{2.3}, -NR^{2.2}R^{2.3}, C₆₋₁₀-Aryl, C₃₋₇-Cycloalkyl, Het und Hetaryl substituiert sein kann,
sowie pharmakologisch verträgliche Salze, Diastereomere, Enantiomere, Racemate, Hydrate oder Solvate davon.

Ein Gegenstand der vorliegenden Erfindung sind ausdrücklich sowohl die R-Enantiomere der Formel A als auch die S-Enantiomere der Formel A' bezüglich des Stereozentrums am Sulfoxid-Schwefel-Atom der Verbindungen der Formel **1**,

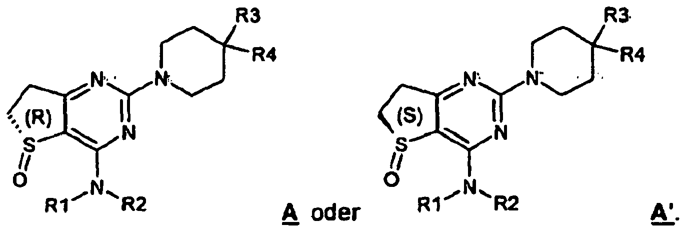

Bevorzugt sind weiterhin die oben genannten Verbindungen der Formel **1**, worin
- **X**: SO oder SO₂,
- **R¹**: H
- **R²**: H ist oder C₁₋₁₀-Alkyl ist, das gegebenenfalls durch einen oder mehrere Reste ausgewählt aus Halogen und C₁₋₃-Fluoroalkyl substituiert sein kann oder das gegebenenfalls durch einen oder mehrere Reste ausgewählt aus der Gruppe bestehend aus OR^{2.1}, COOR^{2.1},CONR^{2.2}R^{2.3}, SR^{2.1},SO-R^{2.1}, SO₂-R^{2.1}, Phenyl, Het, Hetaryl, einem monocyclischem C₃₋₇-Cycloalkyl, CH₂-NR^{2.2}R^{2.3} und NR^{2.2}R^{2.3} substituiert sein kann, welcher wiederum gegebenenfalls durch einen oder mehrere Reste ausgewählt aus der Gruppe bestehend aus OH, F, Cl, Br, OR^{2.1}, Oxo, CF₃, CHF₂, CH₂F, C₁₋₆-Alkyl, C₁₋₆-Alkanol, Phenyl, COOR^{2.1}, CH₂-NR^{2.2}R^{2.3} und NR^{2.2}R^{2.3} substituiert sein kann,
wobei
- **Het**: ein drei- bis siebengliedriger, monocyclischer, gesättigter oder teilweise gesättigter Heterocyclus oder ein sieben- bis elfgliedriger, bicyclischer, gesättigter oder teilweise gesättigter Heterocyclus ist, der 1, 2, 3 oder 4 Heteroatome unabhängig voneinander ausgewählt aus der Gruppe bestehend aus N, S oder O enthält, und wobei
- **Hetaryl**: ein fünf- bis sechsgliedriges, monocyclisches, aromatisches Heteroaryl oder ein sieben- bis elfgliedriges, bicyclisches, aromatisches Heteroaryl ist, das jeweils 1, 2, 3 oder 4 Heteroatome unabhängig voneinander ausgewählt aus der Gruppe bestehend aus N, S oder O enthält,
und wobei
- **Cycloalkyl**: gesättigt oder teilweise gesättigt sein kann,
- wobei R^{2.1}: H ist oder ein Rest ausgewählt ist aus der Gruppe bestehend aus C₁₋₆-Alkyl, C₁₋₆-Alkanol, C₁₋₃-Haloalkyl, monocyclisches C₃₋₇, Cycloalkyl, Phenyl-C₁₋₆-alkylen, Hetaryl-C₁₋₆-alkylen, Het-C₁₋₆-alkylen, -C₃₋₇-Cycloalkyl-C₁₋₆-alkylen, Phenyl, Hetaryl und ein Het, der gegebenenfalls durch einen oder mehrere Reste ausgewählt aus der Gruppe bestehend aus OH, F, Cl, C₁₋₆-Alkyl, -O-(C₁₋₃-Alkyl) und Phenyl substituiert sein kann,
- wobei R^{2.2} und: R^{2.3} unabhängig voneinander H sind oder ein Rest ausgewählt sind aus der Gruppe bestehend aus C₁₋₆-Alkyl, monocyclisches C₃₋₇ Cycloalkyl, Phenyl-C₁₋₃-alkylen, Hetaryl-C₁₋₃-.alkylen, Phenyl, Het, Hetaryl, CO-NH₂, -CO-NHCH₃, -CON(CH₃)₂, SO₂-(C₁₋₂-Alkyl), CO-R^{2.1} und COOR^{2.1}, der gegebenenfalls durch einen oder mehrere Reste ausgewählt aus der Gruppe bestehend aus OH, F, Cl, C₁₋₆-Alkyl, Phenyl und COOR^{2.1} substituiert sein kann,
oder
- **R²**: ein monocyclisches C₃₋₇ Cycloalkyl, das gegebenenfalls mit einem Rest ausgewählt aus der Gruppe bestehend aus verzweigtes oder unverzweigtes C₁₋₆-Alkanol, C₁₋₃-Fluoroalkyl, OR^{2.1}, C₁₋₃-alkylen-OR^{2.1},OR^{2.1}, COOR^{2.1}, SO₂-NR^{2.2}R^{2.3}, -Het, -NH-CO-O-(Phenyl), Phenyl, C₁₋₆-Alkyl, Phenyl-C₁₋₆-alkylen, -Hetaryl-C₁₋₆-alkylen, monocyclisches C₃₋₇ Cycloalkyl und NR^{2.2}R^{2.3} substituiert sein kann, der gegebenenfalls durch einen oder mehrere Reste ausgewählt aus der Gruppe bestehend aus OH, OR^{2.1}, Oxo, F, Cl, CF₃, CHF₂. CH₂F, C₁₋₆-Alkyl, Phenyl und -NR^{2.2}R^{2.3} substituiert sein kann,
oder
- **R²**: ein Phenyl, das gegebenenfalls durch OH, SH oder Halogen oder durch einen oder mehrere Reste ausgewählt aus der Gruppe bestehend aus OR^{2.1}, COOR^{2.1}, NR^{2.2}R^{2,3}, CH₂-NR^{2.2}R^{2.3},C₃₋₇-Cycloalkyl, C₃₋₇-Heterocyclus. C₁₋₆-Alkyl, C₁₋₃-Fluoroalkyl, Phenyl-C₁₋₆-alkylen, -Het-C₁₋₆-alkylen, -Hetaryl-C₁₋₆-alkylen, Phenyl, SO₂-CH₃, SO₂-CH₂CH₃ und SO₂-NR^{2.2}R^{2.3} substituiert sein kann, der wiederum gegebenenfalls durch einen oder mehrere oder mehrere Reste ausgewählt aus der Gruppe bestehend aus OH, OR^{2.1}, Oxo, F, Cl, CF₃, CHF₂, CH₂F, C₁₋₆-Alkyl, Phenyl und NR^{2.2}R^{2.3} substituiert sein kann,
oder
- **R²**: ein Rest ausgewählt aus einer Gruppe bestehend aus Het und Hetaryl, welcher gegebenenfalls durch einen oder mehrere Reste ausgewählt aus der Gruppe F, Cl. OH, Oxo, CF₃, CHF₂ und CH₂F oder durch einen oder mehrere Reste ausgewählt aus der Gruppe OR^{2.1}, -C₁₋₃-alkylen-OR^{2.1}, SR^{2.1},SO-R^{2.1}, SO₂-R^{2.1}, COOR^{2.1}, COR^{2.1}, C₁₋₆ Alkanol, monocyclisches C₃₋₇-Cycloalkyl, Phenyl, C₁₋₆-Alkyl, Phenyl-C₁₋₆-alkylen, -Hetaryl-C₁₋₆-alkylen, -Het, -Hetaryl, und NR^{2.2}R^{2.3} substituiert sein kann, der gegebenenfalls wiederum durch einen oder mehrere Reste ausgewählt aus der Gruppe bestehend aus OH, OR^{2.1}, Oxo, F, Cl, CF₃, CHF₂, CH₂F, C₁₋₆-Alkyl, Phenyl und NR^{2.2}R^{2.3} substituiert sein kann,
oder worin
- **NR¹R²**: gemeinsam einen heterocyclischen C₄₋₇-Ring bedeutet, der gegebenenfalls überbrückt sein kann, der 1, 2 oder 3 Heteroatome ausgewählt aus der Gruppe bestehend aus N, O und S enthält und der gegebenenfalls durch einen oder mehrere Reste ausgewählt aus der Gruppe bestehend aus OH, OR^{2.1}, C₁₋₃-alkylen-O^{R.1}, Oxo, F, Cl, C₁₋₆-Alkyl Phenyl, COOR^{2.1}, CH₂-NR^{2.2}-COO-R^{2.1}, CH₂-NR^{2.2}-CO-R^{2.1}, CH₂- NR^{2.2}-CO-CH₂-NR^{2.2}R^{2.3}, CH₂-NR^{2.2}-SO₂-C₁₋₃-Alkyl, CH₂-NR^{2.2}-SO₂-NR^{2.2}-R^{2.3}, CH₂-NR^{2.2}-CO-NR^{2.2}R^{2.3}, CO-NR^{2.2}R^{2.3}, CH₂-NR^{2.2}R^{2.3} und NR^{2.2}R^{2.3} substituiert sein kann,
und worin
- **R³**: ein Naphthalin oder Phenyl ist, welches gegebenenfalls in ortho, para oder meta-Stellung mit einem oder zwei Resten unabhängig voneinander ausgewählt aus der Gruppe bestehend aus Fluor, Chlor, Brom, Hydroxy, CN, C₁₋₆-Alkyl, C₁₋₃-Fluoroalkyl, -C₁₋₃-alkylen-OR^{2.1}, -C₁₋₃-alkylen-NR^{2.2}R^{2.3}, -NR^{2.2}R^{2.3}, O-R^{2.1}; SO-R^{2.1}, SO₂-R^{2.1}, COOR^{2.1}, -CO-NH-(C₁₋₆-Alkylen)-Hetaryl, -CO-NH-Hetaryl, -CO-N(CH₃)-Het, -CO-N(CH₃)-(C₁₋₃-Alkylen)-Het,-CO-N(CH₃)-(C₁₋₃-Alkylen)-Hetaryl, -CO-N(C₃₋₇-Cycloalkyl)-Het, CO-NR^{2.2}R^{2.3}, -CO-NH-(C₁₋₅-Alkylen)-Het, -NR^{2.2}-CO-R^{2.1}, Phenyl, Phenyl-C₁₋₂-alkylen, -Het-C₁₋₂-alkylen, -Het, -CO-Het, -CO-N(CH₃)-Het, CO-N(CH₃)-C₃₋₇-Cycloalkyl, C₃₋₇-Cycloalkyl, C₃₋₇-Cycloalkyl-C₁₋₂-alkylen, -Hetaryl-C₁₋₂-alkylen und -Hetaryl substituiert ist, wobei dieser Rest gegebenenfalls durch einen oder mehrere Reste ausgewählt aus der Gruppe bestehend aus OH, F, Cl, -C₁₋₃-Fluoroalkyl, Oxo, Methyl und Phenyl substituiert sein kann,
oder worin
- **R³**: ein Rest ausgewählt aus der Gruppe bestehend aus Het und Hetaryl, der gegebenenfalls durch einen oder mehrere Reste ausgewählt aus der Gruppe bestehend aus F, Cl, Br, C₁₋₃-Fluoroalkyl, CN, OH, Oxo, -C₁₋₆-Alkyl, -C₁₋₃-alkylen-NR^{2.2}R^{2.3}, -NR^{2.2}R^{2.3}, SO-R^{2.1}, SO₂-R^{2.1}, -O-R^{2.1}, -COOR^{2.1}, SO₂-(CH₃), SO₂-(CH₂-CH₃), Phenyl, Het, C₃₋₇-Cycloalkyl und Hetaryl substituiert sein kann, der wiederum gegebenenfalls mit einem oder mehreren Resten ausgewählt aus der Gruppe OH, F, Cl, Br, -C₁₋₃-Fluoroalkyl, C₁₋₆-Alkyl, Phenyl, -COO(C₁₋₃-Alkyl) und O-(C₁₋₃-Alkyl) substituiert sein kann,
oder worin
- **R³**: -O-R^{3.1},
- wobei **R^{3.1}**: ein Rest ausgewählt aus der Gruppe bestehend aus -C₁₋₆-Alkyl, -Phenyl,-C₁₋₃-Alkylen-Phenyl, Hetaryl und Het ist, welcher gegebenenfalls in ortho, para oder meta-Stellung mit einem, zwei oder drei Resten unabhängig voneinander ausgewählt aus der Gruppe bestehend aus Fluor, Chlor, Brom, Hydroxy, CN, C₁₋₆-Alkyl, C₁₋₃-fluoroalkyl, CO-(C₁₋₅-Alkyl), -CO-(C₁₋₃-Fluoroalkyl) -CO-NH-(C₁₋₆-Alkylen)-Hetaryl, -CO-N(CH₃)-(C₁₋₆-Alkylen)-Hetaryl, -CO-N(CH₃)-Het, -CO-N(C₃₋₇-Cycloalkyl)-Het, -C₁₋₃-alkylen-OR^{2.1}, -C₁₋₃-alkylen-NR^{2.2}R^{2.3}, -NR^{2.2}R^{2.3}, O-R^{2.1}; SO-R^{2.1}, SO₂-R^{2.1}, COOH, COO-(C₁₋₄-Alkyl), -O-C₁₋₃-Alkylen-N(C₁₋₃-Alkyl)₂, CO-NR^{2.2}R^{2.3}, NR^{2.2}-CO-R^{2.1}, Phenyl, Phenyl-C₁₋₂-alkylen, Het-C₁₋₂-alkylen, -CO-Het, Het, -CO-C₃₋₇-Cycloalkyl, -CO-N(CH₃)-C₃₋₇-Cycloalkyl C₃₋₇-Cycloalkyl, C₃₋₇-Cycloalkyl-C₁₋₂-alkylen, Hetaryl-C₁₋₂-alkylen und Hetaryl substituiert ist, welcher wiederum gegebenenfalls mit 1, 2, 3 oder 4 Resten unabhängig voneinander ausgewählt aus der Gruppe bestehend aus F, Cl, Br, Methyl, O-Methyl, Ethyl, O-Ethyl, OH, Oxo und CF₃ substituiert sein kann,
und worin
- **R⁴**: H, CN, OH, CF₃, CHF₂, CH₂F, F, Methyl, Ethyl, O-Methyl, O-Ethyl, O-Propyl, O-Isopropyl, -C₁₋₃-Alkylen-OH, -COO(C₁₋₃-Alkyl), -CO-Het, -(C₁₋₂-Alkylen)-NH-SO₂-(C₁₋₂-Alkyl), -(C₁₋₂-Alkylen)-N(CH₃)-SO₂-(C₁₋₂-Alkyl), -(C₁₋₂-Alkylen)-O-(C₁₋₂-Alkylen)-Phenyl, -C₁₋₃-Alkylen-O-C₁₋₃-Alkyl, -(C₁₋₂-Alkylen)-N(CH₃)-CO-(C₁₋₂-Alkyl), -NH-CO-(C₁₋₃-Alkylen)-O-(C₁₋₃-Alkyl), -C₁₋₃-Alkylen-NH-CO-(C₁₋₃-Alkyl), -C₁₋₃-Alkylen-NH-CO-(C₁₋₃-Alkylen)-N(C₁₋₃-Alkyl)₂, -O-(C₁₋₂-Alkylen)-Phenyl -C₁₋₃-Alkylen-NH-CO-(C₁₋₃-Alkylen)-O-(C₁₋₃-Alkyl), -CO-Phenyl, -(C₁₋₂-Alkylen)-N(CH₃)-CO-(C₁₋₂-Alkylen)-O-(C₁₋₃-Alkyl), wobei das Phenyl in den obigen Resten gegebenenfalls mit einem oder mehreren weiteren Resten ausgewählt aus der Gruppe F, Cl, Br, Methyl, Ethyl, Propyl, -O-Methyl, -O-Ethyl, -O-Propyl, -OH und CF₃ substituiert sein kann
oder worin
- **R³** und **R⁴**: gemeinsam einen mono- oder bicyclischen, ungesättigten, gesättigten oder teilweise gesättigten Heterocyclus bilden, der 1, 2 oder 3 Heteroatome ausgewählt aus der Gruppe bestehend aus N, O und S enthält und der gegebenenfalls mit einem oder mehreren Resten ausgewählt aus der Gruppe bestehend aus F, Cl. Br, OH, Oxo, C₁₋₃-Fluoroalkyl, CN, C₁₋₆-Alkyl, -O-R^{2.1},-COOR^{2.1}, SO-R^{2.1}, SO₂-R^{2.1}, -C₁₋₃-alkylen-NR^{2.2}R^{2.3}, -NR^{2.2}R^{2.3}, Phenyl, C₃₋₇-Cycloalkyl, Het und Hetaryl substituiert sein kann,
sowie pharmakologisch verträgliche Salze, Diastereomere, Enantiomere, Racemate, Hydrate oder Solvate davon.

Stärker bevorzugt sind außerdem die obigen Verbindungen der Formel 1, worin
- **X**: SO,
- **R¹**: H
- **R²**: H ist oder C₁₋₆-Alkyl ist, das gegebenenfalls durch einen oder mehrere Reste ausgewählt aus F, Cl, CF₃, CHF₂ oder CH₂F substituiert sein kann oder das gegebenenfalls durch einen oder mehrere Reste ausgewählt aus der Gruppe bestehend aus OR^{2.1}, COOR^{2.1},CONR^{2.2}R^{2.3}, SR^{2.1},SO-R^{2.1}, SO₂-R^{2.1}, Phenyl, Het, Hetaryl, einem monocyclischem C₃₋₇-Cycloalkyl, CH₂-NR^{2.2}R^{2.3} und NR^{2.2}R^{2.3} substituiert sein kann, welcher wiederum gegebenenfalls durch einen oder mehrere Reste ausgewählt aus der Gruppe bestehend aus OH, F, Cl, Br, CF₃, CHF₂, CH₂F, OR^{2.1}, Oxo, Methyl, Ethyl, Propyl, Isopropyl, Methanol, Ethanol, Phenyl, COOR^{2.1}, CH₂-NR^{2.2}R^{2.3} und NR^{2.2}R^{2.3} substituiert sein kann,
wobei
- **Het**: ein drei- bis siebengliedriger, monocyclischer, gesättigter oder teilweise gesättigter Heterocyclus ist, der 1, 2 oder 3 Heteroatome unabhängig voneinander ausgewählt aus der Gruppe bestehend aus N, S oder O enthält,
und wobei
- **Hetaryl**: ein fünf- bis sechsgliedriger, monocyclisches, aromatisches Heteroaryl ist, das 1, 2 oder 3 Heteroatome unabhängig voneinander ausgewählt aus der Gruppe bestehend aus N, S oder O enthält,
und wobei
- Cycloalkyl: gesättigt oder teilweise gesättigt sein kann, wobei R^{2.1} H ist oder ein Rest ausgewählt ist aus der Gruppe bestehend aus Methyl, Ethyl, Propyl, Isopropyl, Methanol, Ethanol, monocyclisches C₃₋₇ Cycloalkyl, Phenyl-C₁₋₂-alkylen, -Hetaryl-C₁₋₂-alkylen, -Het-C₁₋₂-alkylen, C₃₋₇-Cycloalkyl-C₁₋₂-alkylen, Phenyl, Hetaryl und ein Het, der gegebenenfalls durch einen oder mehrere Reste ausgewählt aus der Gruppe bestehend aus OH, F, Cl, Methyl, Ethyl, Propyl, Isopropyl, O-Methyl, O-Ethyl, O-Propyl. O-Isopropyl und Phenyl substituiert sein kann, wobei R^{2.2} und R^{2.3} unabhängig voneinander H oder ein Rest ausgewählt sind aus der Gruppe bestehend aus Methyl, Ethyl. Propyl, Isopropyl, monocyclisches C_{3-7,} Cycloalkyl, Phenyl-C₁₋₃-alkylen, Hetaryl-C₁₋₃-alkylen, Phenyl, -Het, -Hetaryl, CO-NH₂, CO-NHCH₃, CON(CH₃)₂, SO₂-(C₁₋₂-Alkyl), CO-R^{2.1} und COOR^{2.1},
der gegebenenfalls durch einen oder mehrere Reste ausgewählt aus der Gruppe bestehend aus OH, F, Cl, Methyl, Ethyl, Propyl, Isopropyl, Phenyl und COOR^{2.1} substituiert sein kann,
oder
- **R²**: ein monocyclisches C₃₋₇ Cycloalkyl, das gegebenenfalls mit einem Rest ausgewählt aus der Gruppe bestehend aus C₁₋₂-Alkanol, C₁₋₃-Fluoroalkyl, C₁₋₃-alkylen-OR^{2.1}, OR^{2.1}, COOR^{2.1}, SO₂-NR^{2.2}R^{2.3}, -Het, -NH-CO-O-(Phenyl), Methyl, Ethyl, Propyl, Isopropyl, Phenyl, Phenyl-C₁₋₂-alkylen, -Hetaryl-C₁₋₂-alkylen, monocyclisches C₃₋₇ Cycloalkyl und NR^{2.2}R^{2.3} substituiert sein kann, der gegebenenfalls durch einen oder mehrere Reste ausgewählt aus der Gruppe bestehend aus OH, OR^{2.1}, Oxo, F, Cl, CF₃, CHF₂, CH₂F, Methyl, Ethyl, Propyl, Isopropyl, Phenyl und NR^{2.2}R^{2.3} substituiert sein kann,
oder
- **R²**: ein Phenyl, das gegebenenfalls durch OH, SH, F, Cl oder Br oder durch einen oder mehrere Reste ausgewählt aus der Gruppe bestehend aus OR^{2.1}, COOR^{2.1}, NR^{2.2}R^{2.3}, CH₂-NR^{2.2}R^{2.3}, monocyclisches C₃₋₇-Cycloalkyl, -Het, Methyl, Ethyl, Propyl, Isopropyl, CF₃, CHF₂, CH₂F, Phenyl-C₁₋₂-alkylen, Het-C₁₋₂-alkylen, Hetaryl-C₁₋₂alkylen, Phenyl, SO₂-CH₃, SO₂-CH₂CH₃ und SO₂-NR^{2.2}R^{2.3} substituiert sein kann, der wiederum gegebenenfalls durch einen oder mehrere oder mehrere Reste ausgewählt aus der Gruppe bestehend aus OH, OR^{2.1}, Oxo, F, Cl, CF₃, CHF₂. CH₂F. Methyl, Ethyl, Propyl, Isopropyl, Phenyl und NR^{2.2}R^{2.3} substituiert sein kann,
oder
- **R²**: ein Rest ausgewählt aus einer Gruppe bestehend aus Het und Hetaryl, der gegebenenfalls durch einen oder mehrere Reste ausgewählt aus der Gruppe F, Cl, OH, Oxo, CF₃, CHF₂ und CH₂F oder durch einen oder mehrere Reste ausgewählt aus der Gruppe OR^{2.1}, C₁₋₃alkylen-OR^{2.1}, SR^{2.1}, SO-R^{2.1}, SO₂-R^{2.1}, COOR^{2.1}, COR^{2.1}, Methanol, Ethanol, monocyclisches C₃₋₇-Cycloalkyl, Phenyl, Methyl, Ethyl, Propyl, Isopropyl, Phenyl-C₁₋₂-alkylen, Hetaryl-C₁₋₂-alkylen, -Het, -Hetaryl und NR^{2.2}R^{2.3} substituiert sein kann, der gegebenenfalls wiederum durch einen oder mehrere Reste ausgewählt aus der Gruppe bestehend aus OH, OR^{2.1},Oxo, F, Cl, CF₃, CHF₂, CH₂F Methyl, Ethyl, Propyl, Isopropyl, Butyl, Isobutyl, Phenyl und NR^{2.2}R^{2.3} substituiert sein kann,
und worin
- **R³**: ein Naphthalin oder Phenyl ist, welches gegebenenfalls in ortho, para oder meta-Stellung mit einem oder zwei Resten unabhängig voneinander ausgewählt aus der Gruppe bestehend aus Fluor, Chlor, Brom, Hydroxy, CN, Methyl, Ethyl, Propyl, Isopropyl, Cyclopropyl, CF₃, CHF₂, CH₂F, -OCH₃, OCH₂CH₃, SO₂-CH₃, SO-CH₃, COOCH₃, COOCH₂CH₃, -CO-NH-(Methylen)-Hetaryl, -CO-NH-(Ethylen)-Hetaryl, -CO-NH-Hetaryl, -CO-N(CH₃)-Het,-CO-N(CH₃)-(Methylen)-Het, -CO-N(CH₃)-(Ethylen)-Het, -CO-N(CH₃)-(Methylen)-Hetaryl, -CO-N(CH₃)-(Ethylen)-Hetaryl, -CO-N(Cyclopropyl)-Het, CO-NH₂, CONH(CH₃), CON(CH₃)₂, -CO-NH-(Methylen)-Het, -CO-NH-(Ethylen)-Het, -NH-CO-Methyl, NCH₃-CO-Methyl,-NH-CO-Ethyl, NCH₃-CO-Ethyl, -NH-CO-Propyl, NCH₃-CO-Propyl, -NH-CO-Isopropyl, NCH₃-CO-Isopropyl, Phenyl, Phenyl-Methylen, Phenyl-Ethylen, Het-Methylen, Het-Ethylen, -Het, -CO-Het, -CO-N(CH₃)-Het, CO-N(CH₃)-Cyclopropyl, C₃₋₇-Cycloalkyl, C₃₋₇-Cycloalkyl-Methylen, C₃₋₇-Cycloalkyl-Ethylen, Hetaryl-methylen, Hetaryl-Ethylen, -Hetaryl, CH₂-NH₂, CH₂-NH(CH₃), CH₂-N(CH₃)₂, -NH₂, -NH(CH₉) und -N(CH₃)₂ substituiert sein kann, wobei dieser Rest gegebenenfalls durch einen oder mehrere Reste ausgewählt aus der Gruppe bestehend aus OH, F, Cl, -CF₂, CHF₂, CH₂F, Oxo, Methyl und Phenyl substituiert sein kann
oder worin
- **R³**: ein Rest ausgewählt aus der Gruppe bestehend aus einem Het und Hetaryl, der gegebenenfalls durch einen oder mehrere Reste ausgewählt aus der Gruppe bestehend aus F, Cl, Br, CF₃, CHF₂, CH₂F, CN, OH, Oxo, Methyl, Ethyl, Propyl, Isopropyl, Butyl, Isobutyl, Cyclopropyl, -O-Methyl, -O-Ethyl, -O-Propyl, -O-Isopropyl,-COO-Methyl, -COO-Ethyl, -COO-Propyl, -COO-Isopropyl, SO-(CH₃), SO-(CH₂-CH₃), SO₂-(CH₃), SO₂-(CH₂-CH₃), Phenyl, CH₂-NH₂, CH₂-NH(CH₃), CH₂-N(CH₂)₂, -NH₂,-NH(CH₃), -N(CH₃)₂. Het und Hetaryl substituiert sein kann, der wiederum gegebenenfalls mit einem oder mehreren Resten ausgewählt aus der Gruppe OH, F, Cl, CF₃, CHF₂, CH₂F, Methyl, Ethyl, Propyl, Isopropyl, Phenyl, -COO-Methyl, -COO-Ethyl und O-Methyl, O-Ethyl substituiert sein kann,
oder worin
- **R³**: -O-R^{3.1},
- wobei **R^{3.1}**: ein Rest ausgewählt aus der Gruppe bestehend aus -C₁₋₃-Alkyl, -Phenyl,-C₁₋₃-Alkylen-Phenyl, Hetaryl und Het ist, welcher gegebenenfalls in ortho, para oder meta-Stellung mit einem, zwei oder drei Resten unabhängig voneinander ausgewählt aus der Gruppe bestehend aus Fluor, Chlor, Brom, Hydroxy, CN, Methyl, Ethyl, Propyl, Isopropyl, Butyl, Isobutyl, CF₃, CHF₂, CH₂F, CO-(Methyl), CO-(Ethyl), CO-(Propyl), CO-(Isopropyl), -CO-(CF₃), -CO-NH-(Methylen)-Hetaryl, -CO-NH-(Ethylen)-Hetaryl, -CO-N(CH₃)-(Methylen)-Hetaryl, -CO-N(CH₃)-(Ethylen)-Hetaryl, -CO-N(CH₃)-(Propylen)-Hetaryl, -CO-N(CH₃)-(Isopropylen)-Hetaryl -CO-N(CH₃)-Het, -CO-N(Cyclopropyl)-Het, -CO-N(C₅₋₇Cycloalkyl)-Het, -Methylen-O-Methyl, -Ethylen-O-Methyl, -Propylen-O-Methyl, -Methylen-O-Ethyl, -Ethylen-O-Ethyl, -Propylen-O-Ethyl, -Methylen-NH₂, -Methylen-NHCH₃, -Methylen-N(CH₃)₂, -Ethylen-NH₂, -Ethylen-NHCH₃, -Ethylen-N(CH₃)₂, NH₂, N(CH₃)₂, NHCH₃, -O-Methyl, O-Ethyl, O-Propyl, O-Isopropyl, O-Butyl, O-Isobutyl, -SO-CH₃, SO-Ethyl, -SO-Propyl, -SO-Isopropyl, , SO₂-Methyl, -SO₂-Ethyl-, SO₂-Propyl, SO₂-Isopropyl, COOH, COO-(Methyl), COO-(Ethyl), COO-(Propyl), COO-(Isopropyl), -O-Methylen-N(Methyl)₂, -O-Ethylen-N(Methyl)₂, -O-Methylen-N(Ethyl)₂, -O-Ethylen-N(Ethyl)₂, CO-NH₂, CO-NH(CH₃), CO-N(CH₃)₂, -NH-CO-Methyl, -NCH₃-CO-Methyl, -NH-CO-Ethyl, NCH₃-CO-Ethyl, Phenyl, Phenyl-Methylen-, Phenyl-Ethylen-, Het-Methylen-, Het-Ethylen-, -CO-Het, Het, -CO-C₅₋₇-Cycloalkyl, -CO-Cyclopropyl, -CO-N(CH₃)-C₅₋₇Cycloalkyl, -CO-N(CH₃)-Cyclopropyl, C₅₋₇-Cycloalkyl, Cyclopropyl, C₅₋₇-Cycloalkyl-Methylen, C₅₋₇-Cycloalkyl-Ethylen, Cyclopropyl-Methylen, Cyclopropyl-Ethylen, Hetaryl-Methylen, Hetaryl-Ethylen und Hetaryl substituiert ist, welcher wiederum gegebenenfalls mit 1, 2, 3 oder 4 Resten unabhängig voneinander ausgewählt aus der Gruppe bestehend aus F, Cl, Br, Methyl, O-Methyl, Ethyl, O-Ethyl, OH, Oxo und CF₃ substituiert sein kann,
und worin
- **R⁴**: H, CN, OH, CF₃, CHF₂. CH₂F, F, Methyl, Ethyl, O-Methyl oder O-Ethyl, -Methylen-OH, -Ethylen-OH, -Propylen-OH, Isopropylen-OH, -COO(Methyl), -COO(Ethyl), -COO(Propyl). -COO(isopropyl), -CO-Het, -(Methylen)-NH-SO₂-(Methyl), -(Methylen)-NH-SO₂-(Ethyl), -(Ethylen)-NH-SO₂-(Methyl), -(Ethylen)-NH-SO₂-(Ethyl), -(Methylen)-N(CH₃)-SO₂-(Methyl), -(Methylen)-N(CH₃)-SO₂-(Ethyl), -(Ethylen)-N(CH₃)-SO₂-(Methyl), -(Ethylen)-N(CH₃)-SO₂-(Ethyl), -(Methylen)-O-(Methylen)-Phenyl, -(Methylen)-O-(Ethylen)-Phenyl, -(Ethylen)-O-(Methylen)-Phenyl, -(Ethylen)-O-(Ethylen)-Phenyl, -Methylen-O-Methyl, -Methylen-O-Ethyl, -Ethylen-O-Methyl -Ethylen-O-Ethyl, -(Methylen)-N(CH₃)-Co-(Methyl), -(Methylen)-N(CH₃)-CO-(Ethyl) -(Ethylen)-N(CH₃)-CO-(Methyl), -(Ethylen)-N(CH₃)-CO-(Ethyl), -NH-CO-(Methylen)-O-(Methyl), -NH-CO-(Methylen)-O-(Ethyl), -NH-CO-(Ethyten)-O-(Methyl), -NH-CO-(Ethylen)-O-(Ethyl), -Methylen-NH-CO-(Methyl), -Methylen-NH-CO-(Ethyl), -Ethylen-NH-CO-(Methyl), -Ethylen-NH-CO-(Ethyl),-Methylen-NH-CO-(Methylen)-N(Methyl)₂, -Methylen-NH-CO-(Ethylen)-N(Methyl)₂, -Ethylen-NH-CO-(Methylen)-N(Methyl)₂, -Ethylen-NH-CO-(Ethylen)-N(Methyl)₂, -Methylen-NH-CO-(Methylen)-O-(Methyl),-Methylen-NH-CO-(Ethylen)-O-(Methyl), -Ethylen-NH-CO-(Methylen)-O-(Methyl), -Methylen-NH-CO-(Methylen)-O-(Ethyl), -Methylen-NH-CO-(Ethylen)-O-(Ethyl), -Ethylen-NH-CO-(Methylen)-O-(Ethyl) -(Methylen)-N(CH₃)-CO-(Methylen)-O-(Methyl), -(Methylen)-N(CH₃)-CO-(Ethylen)-O-(Methyl) -(Ethylen)-N(CH₃)-CO-(Methylen)-O-(Methyl), -(Methylen)-N(CH₃)-CO-(Methylen)-O-(Ethyl), -(Methylen)-N(CH₃)-CO-(Ethylen)-O-(Ethyl), -(Ethylen)-N(CH3)-CO-(Methylen)-O-(Ethyl), -O-(Methylen)-Phenyl, -O-(Ethylen)-Phenyl, -CO-Phenyl,
wobei das Phenyl in den obigen Resten gegebenenfalls mit einem oder mehreren weiteren Resten ausgewählt aus der Gruppe F, Cl, Br, Methyl, Ethyl, Propyl, -O-Methyl, -O-Ethyl, -O-Propyl, -OH und CF₃ substituiert sein kann
oder worin
- **R³** und **R⁴**: gemeinsam einen mono- oder bicyclischen, ungesättigten, gesättigten oder teilweise gesättigten Heterocyclus bilden, der 1, 2 oder 3 Heteroatome ausgewählt aus der Gruppe bestehend aus N, O und S enthält und der gegebenenfalls mit einem oder mehreren Resten ausgewählt aus der Gruppe bestehend aus F, Cl, Br, OH, Oxo, CF₃, CHF₂, CH₂F, CN, Methyl, Ethyl, Propyl, Isopropyl, Cyclopropyl, COO-Methyl, -COO-Ethyl, O-Methyl, O-Ethyl, SO₂-(CH₃), SO₂-(CH₂CH₃), SO-(CH₃), SO-(CH₂CH₃), CH₂-NH₂, CH₂-NH(CH₃), CH₂-N(CH₃)₂, -NH₂, -NH(CH₃), -N(CH₃)₂, Phenyl, C₅₋₇cycloalkyl, Het und Hetaryl substituiert sein kann,
sowie pharmakologisch verträgliche Salze, Diastereomere, Enantiomere, Racemate, Hydrate oder Solvate davon.

Ein weiterer bevorzugter Gegenstand der vorliegenden Erfindung sind weiterhin die obigen Verbindungen der Formel **1,** worin
- **R²**: ein Rest nach Formel **2** 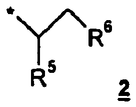
ist, und
- worin **R⁶**: OH oder NH₂ ist und
- worin **R⁵**: ein Rest ausgewählt aus der Gruppe bestehend aus C₁₋₄-Alkyl, einem fünf- bis sechsgliedrigen Heteroaryl mit 1, 2 oder 3 Heteroatomen aus der Gruppe S, O und N und Phenyl, welcher gegebenenfalls mit einem oder mehreren Resten ausgewählt aus der Gruppe bestehend aus OH, F, Br, OR^{2.1}, Oxo, Methyl, Ethyl, Methanol, Ethanol, Phenyl, COOR^{2.1}, CH₂-NR^{2.2}R^{2.3} und NR^{2.2}R^{2.3} substituiert sein kann,
bedeutet,
sowie pharmakotogisch verträgliche Salze, Diastereomere, Enantiomere, Racemate, Hydrate oder Solvate davon.

Ein weiterer bevorzugter Gegenstand der vorliegenden Erfindung sind weiterhin die obigen Verbindungen der Formel **1**, worin
- R²: ein Rest nach Formel **2** 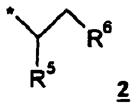
ist,
- worin **R⁶**: OH oder NH₂ ist und
- worin **R⁵**: Methyl, Ethyl, Propyl, Isopropyl ist
sowie pharmakologisch verträgliche Salze, Diastereomere, Enantiomere, Racemate, Hydrate oder Solvate davon.

Ein weiterer bevorzugter Gegenstand der vorliegenden Erfindung sind weiterhin die obigen Verbindungen der Formel **1**, worin
- **R²**: ein monocyclischer drei-, vier-, fünf-, sechs oder siebengliedriger Cycloalkyl-Ring ist, der gegebenenfalls in spiro-Stellung mit einem Rest ausgewählt aus der Gruppe bestehend aus -CH₂-OR^{2.1}, verzweigtes oder unverzweigtes C₂₋₆-Alkylen-OR^{2.1}, Methyl, Ethyl, Propyl, Isopropyl, Butyl, Isobutyl, Cyclopropyl, -CF₃, CHF₂. CH₂F und C₂₋₄-Fluoroalkyl substituiert sein kann, wobei
- R^{2.1}: ausgewählt ist aus der Gruppe bestehend aus Methyl, Ethyl, Propyl, Isopropyl, Butyl, Isobutyl, bedeutet,
sowie pharmakologisch verträgliche Salze, Diastereomere, Enantiomere, Racemate, Hydrate oder Solvate davon.

Ebenfalls bevorzugt sind die oben genannten Verbindungen der Formel **1**, worin
worin
- **R²**: ein Cyclopropyl ist, das gegebenenfalls mit einem weiteren Rest ausgewählt aus der Gruppe bestehend aus -NH₂, CH2- NH₂, -NH(CH₃), -N(CH₃)₂, Methyl, Ethyl, Propyl, Isopropyl, -NH-CO-(tert-Butyl), -NH-CO-O-(tert-Butyl), -N(CH₃)-CO-(tert-Butyl), -N(CH₃)-CO-O-(tert-Butyl), -CF₃, -CHF₂, CH₂F, F, Cl und Br substituiert sein kann,
sowie pharmakologisch verträgliche Salze, Diastereomere, Enantiomere, Racemate, Hydrate oder Solvate davon.

Weiterhin bevorzugt sind die oben genannten Verbindungen der Formel **1**, worin
- **R²**: ein Phenyl bedeutet, das gegebenenfalls in einer oder in beiden meta-Stellungen durch einen oder mehrere Reste ausgewählt aus der Gruppe bestehend aus Methyl, Ethyl, Propyl, Isopropyl, Cyclopropyl, F, Cl, Br, OH, OR^{2.1}, COOR^{2.1}, CF₃, CHF₂, CH₂F, NH₂, NH(CH₃) und N(CH₃)₂ substituiert sein kann, wobei R^{2.1} H, Methyl oder Ethyl, sein kann,
sowie pharmakologisch verträgliche Salze, Diastereomere, Enantiomere, Racemate, Hydrate oder Solvate davon.

Weiterhin bevorzugt sind die oben genannten Verbindungen der Formel **1**, worin
- **R²**: ein Rest ist ausgewählt aus einer Gruppe bestehend aus monocyclischen, gesättigten drei-, vier-, fünf-, sechs- oder siebengliedrigem Heterocyclus mit 1, 2 oder 3 Heteroatomen jeweils ausgewählt aus der Gruppe bestehend aus N, O und S, der gegebenenfalls durch einen oder mehrere Reste ausgewählt aus der Gruppe Fluor, Chlor, Brom, CF₃, CHF₂, CH₂F, OH und Oxo oder durch einen oder mehrere Reste ausgewählt aus der Gruppe OR^{2.1}, C₁₋₃-alkylen-OR^{2.1}, SR^{2.1}, SO-R^{2.1}, SO₂-R^{2.1}, COOR^{2.1}, COR^{2.1}, C₁₋₆Alkanol, C₃₋₁₀-Cycloalkyl, Phenyl, C₁₋₆Alkyl, Phenyl-C₁₋₆-alkylen, C₆₋₁₀-Heteroaryl-C₁₋₆-alkylen, C₅₋₁₀-Heterocyclus, C₅₋₁₀-Heteroaryl und NR^{2.2}R^{2.3} substituiert sein kann, der gegebenenfalls wiederum durch einen oder mehrere Reste ausgewählt aus der Gruppe bestehend aus OH, OR^{2.1}, Oxo, F, Cl, CF₃, CHF₂, CH₂F, C₁₋₆-Alkyl, Phenyl und NR^{2.2}R^{2.3} substituiert sein kann,
und worin **R^{2.1}**, **R^{2.2}** und **R^{2.3}** wie vorstehend definiert sind,
sowie pharmakologisch verträgliche Salze, Diastereomere, Enantiomere, Racemate, Hydrate oder Solvate davon.

Weitere bevorzugte Gegenstände der vorliegenden Erfindung sind die obigen Verbindungen der Formel **1**, worin
- **R²**: ein Rest ausgewählt aus einer Gruppe bestehend aus einem monocyclischen, gesättigten sechsgliedrigem Heterocyclus mit einem Heteroatom ausgewählt aus der Gruppe bestehend aus N, O und S ist, der gegebenenfalls durch einen oder mehrere Reste ausgewählt aus der Gruppe F, Cl, Br, CF₃, CHF₂, CH₂F, OH, Oxo, NH₂, NHCH₃ und N(CH₃)₂, Methyl, Ethyl, Propyl, Isopropyl, Cyclopropyl, Methoxy und Ethoxy substituiert sein kann,
bedeutet,
sowie pharmakologisch verträgliche Salze, Diastereomere, Enantiomere, Racemate, Hydrate oder Solvate davon.

Weitere bevorzugte Gegenstände der vorliegenden Erfindung sind die obigen Verbindungen der Formel **1**, worin
- **R²**: ein Rest ausgewählt aus einer Gruppe bestehend aus Piperidin oder Tetrahydropyran, der gegebenenfalls durch einen oder mehrere Reste ausgewählt aus der Gruppe F, Cl, Br, OH, CF₃, CHF₂, CH₂F, NH₂, NHCH₃, N(CH₃)₂, Oxo, Methyl und Methoxy substituiert sein kann,
bedeutet,
sowie pharmakologisch verträgliche Salze, Diastereomere, Enantiomere, Racemate, Hydrate oder Solvate davon.

Weitere bevorzugte Gegenstände der vorliegenden Erfindung sind die obigen Verbindungen der Formel **1**, worin
- **R³**: ein Naphthalin oder Phenyl ist, welches gegebenenfalls in beliebiger Stellung mit einem, zwei oder drei Resten unabhängig voneinander ausgewählt aus der Gruppe bestehend aus Fluor, Chlor, Brom, Hydroxy, CN, Methyl, Ethyl, Propyl, Isopropyl, Cyclopropyl, CF₃, CHF₂, CH₂F,-OCH₃, OCH₂CH₃; SO₂-CH₃, SO₂-CH₂CH₃, COOCH₃ und CO-O-CH₂CH₃, substituiert sein kann,
sowie pharmakologisch verträgliche Salze, Diastereomere, Enantiomere, Racemate, Hydrate oder Solvate davon.

Weitere bevorzugte Gegenstände der vorliegenden Erfindung sind die obigen Verbindungen der Formel **1**, worin
- **R³**: ein Rest ausgewählt aus der Gruppe bestehend aus Het und Hetaryl, der gegebenenfalls durch einen oder mehrere Reste ausgewählt aus der Gruppe bestehend aus F, Cl, Br, CF₃, CHF₂, CH₂F, CN, OH, Oxo, Methyl, Ethyl, Propyl, Isopropyl, Butyl, Isobutyl, Cyclopropyl, C₅₋₇-Cycloalkyl, -O-Methyl, -O-Ethyl, -O-Propyl, -O-Isopropyl, -COO-Methyl, -COO-Ethyl, -COO-Propyl, -COO-Isopropyl, SO₂-(CH₃), SO₂-(CH₂-CH₃), SO-(CH₃), SO-(CH₂-CH₃), Phenyl, -CH₂-NH₂, -CH₂-NHCH₃, -CH₂-N(CH₃)₂, NH₂, NHCH₃, N(CH₃)₂, Het und Hetaryl substituiert sein kann, der wiederum mit einem oder mehreren Resten ausgewählt aus der Gruppe OH, F, Cl, Br, CF₃, CHF₂, CH₂F, Methyl, Ethyl, Propyl, Isopropyl, Phenyl, -COO-Methyl, -COO-Ethyl, -COO-Propyl, -COO-Isopropyl und O-Methyl, O-Ethyl, O-Propyl und O-Isopropyl substituiert sein kann,
und worin
- **R⁴**: H, CN, OH, CF₃, CHF₂, CH₂F, F, Methyl, Ethyl, O-Methyl oder O-Ethyl,
wobei
- **Het**: ein drei- bis siebengliedriger, monocyclischer, gesättigter oder teilweise gesättigter Heterocyclus oder ein sieben- bis elfgliedriger, bicyclischer, annellierter, gesättigter oder teilweise gesättigter Heterocyclus ist, der 1, 2 oder 3 Heteroatome unabhängig voneinander ausgewählt aus der Gruppe bestehend aus N, S oder O enthält,
und wobei
- **Hetaryl**: ein fünf- bis sechsgliedriges, monocyclisches, aromatisches Heteroaryl oder ein sieben- bis elfgliedriges, bicyclisches, annelliertes, aromatisches Heteroaryl ist, das jeweils 1, 2 oder 3 Heteroatome unabhängig voneinander ausgewählt aus der Gruppe bestehend aus N, S oder O enthält,
und wobei
- **Cycloalkyl**: gesättigt oder teilweise gesättigt sein kann,
sowie pharmakologisch verträgliche Salze, Diastereomere, Enantiomere, Racemate, Hydrate oder Solvate davon.

Weitere besonders bevorzugte Gegenstände der vorliegenden Erfindung sind die obigen Verbindungen der Formel **1**, worin
- **R³**: ein Rest ausgewählt aus einem bicyclischen, sieben- bis elfgliedrigem, gesättigten oder teilweise gesättigten Heterocyclus oder einem bicyclischen, sieben- bis elfgliedrigem Heteroaryl ist, der ausgewählt ist aus der Gruppe bestehend aus Indol, Dihydroindol, Chinazolin, Dihydrochinazolin, Tetrahydrochinazolin, Benzoisoxazol, Dihydrobenzoisoxazol, Benzooxazin, Dihydrobenzooxazin, Benzothiazol, Dihydrobenzothiazol, Triazolopyridin, Dihydrotriazolopyridin, Benzofuran, Dihydrobenzofuran, Isobenzofuran und Dihydroisobenzofuran, welcher gegebenenfalls mit einem oder mehreren Resten ausgewählt aus der Gruppe bestehend aus aus F, Cl, Br, CF₃, CHF₂, CH₂F, CN, OH, Oxo, Methyl, Ethyl, Propyl, Isopropyl, Butyl, Isobutyl, Cyclopropyl, -O-Methyl, -O-Ethyl, -O-Propyl, -O-Isopropyl,-COO-Methyl, -COO-Ethyl, -COO-Propyl, -COO-Isopropyl, SO₂-(CH₃), SO₂-(CH₂-CH₃), SO-(CH₃), SO-(CH₂-CH₃), Phenyl, -CH₂-NH₂, -CH₂-NHCH₃, -CH₂-N(CH₃)₂, NH₂, NHCH₃, N(CH₃)₂, Furanyl und Pyridinyl substituiert sein kann, der wiederum mit einem oder mehreren Resten ausgewählt aus der Gruppe OH, F, Cl, Br, CF₃, CHF₂, CH₂F, Methyl, Ethyl, Propyl, Isopropyl, Phenyl, -COO-Methyl,-COO-Ethyl und O-Methyl, O-Ethyl substituiert sein kann,
bedeutet,
sowie pharmakologisch verträgliche Salze, Diastereomere, Enantiomere, Racemate, Hydrate oder Solvate davon.

Weitere besonders bevorzugte Gegenstände der vorliegenden Erfindung sind die obigen Verbindungen der Formel **1**, worin
- **R³**: ein Rest ausgewählt aus einem monocyclischen, gesättigten oder teilweise gesättigten, drei- bis siebengliedrigem Heterocyclus oder einem monocyclischen fünf-bis sechsgliedrigem Heteroaryl ist, der ausgewählt ist aus der Gruppe bestehend aus Imidazol, Dihydroimidazol, Oxadiazol, Oxadiazolidin, Pyrazol, Pyridin und Dihydropyrazol, welcher gegebenenfalls mit einem oder mehreren Resten ausgewählt aus der Gruppe bestehend aus aus F, Cl, Br, CF₃, CHF₂, CH₂F, CN, OH, Oxo, Methyl, Ethyl, Propyl, Isopropyl, Butyl, Isobutyl, Cyclopropyl, -O-Methyl, -O-Ethyl, -O-Propyl, -O-Isopropyl,-COO-Methyl, -COO-Ethyl, -COO-Propyl, -COO-Isopropyl, SO₂-(CH₃), SO₂-(CH₂-CH₃), SO-(CH₃), SO-(CH₂-CH₃), Phenyl, -CH₂-NH₂, -CH₂-NHCH₃, -CH₂-N(CH₃)₂, NH₂, NHCH₃, N(CH₃)₂, Furanyl und Pyridinyl substituiert sein kann, der wiederum mit einem oder mehreren Resten ausgewählt aus der Gruppe OH, F, Cl, Br, CF₃, CHF₂, CH₂F, Methyl, Ethyl, Propyl, Isopropyl, Phenyl, -COO-Methyl, -COO-Ethyl und O-Methyl, O-Ethyl substituiert sein kann,
bedeutet.
sowie pharmakologisch verträgliche Salze, Diastereomere, Enantiomere, Racemate, Hydrate oder Solvate davon.

Weitere besonders bevorzugte Gegenstände der vorliegenden Erfindung sind die obigen Verbindungen der Formel **1,** worin
- **R³** und **R⁴**: gemeinsam einen mono- oder bicyclischen, ungesättigten oder teilweise gesättigten, drei- bis elfgliedrigen Heterocyclus bilden, der 1, 2 oder 3 Heteroatome ausgewählt aus der Gruppe bestehend aus N, O und S enthält und der gegebenenfalls mit einem oder mehreren Resten ausgewählt aus der Gruppe bestehend aus F, Cl, Br, OH, Oxo, CF₃, CHF₂, CH₂F, CN, Methyl, Ethyl, Propyl, Isopropyl, Cyclopropyl, COO-Methyl, -COO-Ethyl, O-Methyl, O-Ethyl, SO₂-(CH₃), SO₂-(CH₂-CH₃), SO-(CH₃), SO-(CH₂-CH₃), Phenyl, -CH₂-NH₂, -CH₂NHCH₃, -CH₂-N(CH₃)₂, NH₂, NHCH₃, N(CH₃)₂, einem gesättigtem oder teilweise gesättigtem, fünf- bis sechsgliedrigem Heterocyclus und einem fünf- bis sechsgliedrigem Heteroaryl substituiert sein kann,
bedeuten,
sowie pharmakologisch verträgliche Salze, Diastereomere, Enantiomere, Racemate, Hydrate oder Solvate davon.

Weitere besonders bevorzugte Gegenstände der vorliegenden Erfindung sind die obigen Verbindungen der Formel 1, worin
- **R³** und **R⁴**: gemeinsam einen bicyclischen Heterocyclus ausgewählt aus der Gruppe bestehend aus Tetrahydrochinazolin, Tetrahydrobenzoxazin und Dihydroindol, Dihydroisobenzofuran bilden, der gegebenenfalls mit einem oder mehreren Resten ausgewählt aus der Gruppe bestehend aus F, Cl, Br, OH, Oxo, CF₃, CHF₂, CH₂F, CN, Methyl, Ethyl, Propyl, Isopropyl, Cyclopropyl, COO-Methyl, -COO-Ethyl, O-Methyl, O-Ethyl, SO₂-(CH₃), SO₂-(CH₂-CH₃), Phenyl, -CH₂-NH₂, -CH₂NHCH₃, -CH₂-N(CH₃)₂, NH₂, NHCH₃, N(CH₃)₂, einem gesättigtem oder teilweise gesättigten, fünf- oder sechsgliedrigem Heterocyclus und einem fünf- oder sechsgliedrigem Heteroaryl substituiert sein kann,
bedeuten,
sowie pharmakologisch verträgliche Salze, Diastereomere, Enantiomere, Racemate, Hydrate oder Solvate davon.

Die Erfindung betrifft weiterhin vorzugsweise solche Verbindungen nach Formel **1**, worin
- **R³**: -O-R^{3.1} ist,
- wobei R^{3.1}: ein Rest ausgewählt aus der Gruppe bestehend aus Methyl, Ethyl, Propyl, Isopropyl, Butyl, Isobutyl, Pentyl, Isopentyl, -Phenyl, -Methylen-Phenyl, -Ethylen-Phenyl, -Propylen-Phenyl, -Isopropylen-Phenyl, Hetaryl und Het ist, welcher gegebenenfalls in ortho, para oder meta-Stellung mit einem, zwei oder drei Resten unabhängig voneinander ausgewählt aus der Gruppe bestehend aus Fluor, Chlor, Brom, Hydroxy, CN, Methyl, Ethyl, Propyl, Isopropyl, Butyl, Isobutyl, -CF₃, CHF₂, CH₂F, CO-(Methyl), CO-(Ethyl), CO-(Propyl), CO-(Isopropyl), CO-(Butyl), CO-(isobutyl), -CO-(CF₃), -CO-(CH₂F), -CO-(CHF₂), -CO-NH-(Methylen)-Hetaryl, -CO-NH-(Ethylen)-Hetaryl, -CO-NH-(Propylen)-Hetaryl, -CO-NH-(Isopropylen)-Hetaryl, -CO-N(CH₃)-(Methylen)-Hetaryl, -CO-N(CH₃)-(Ethylen)-Hetaryl, -CO-N(CH₃)-(Propylen)-Hetaryl, -CO-N(CH₃)-(Isopropyren)-Hetaryl, -CO-N(CH₃)-Het, -CO-N(C₃₋₇-Cycloalkyl)-Het, -Methylen-O-Methyl, -Ethylen-O-Methyl, -Methylen-O-Ethyl, -Ethylen-O-Ethyl, -Methylen-NH₂, -Ethylen-NH₂, -Methylen-NHCH₃, -Ethylen-NHCH₃, -Methylen-N(CH₃)₂, -Ethylen-N(CH₃)₂, -NH₂, -NHCH₃, -N(CH₃)₂, -O-Methyl, -O-Ethyl, -O-Propyl, -O-Isopropyl, -SO-CH₃, -SO-(CH₂CH₃), -SO₂-CH₃, -SO₂-(CH₂CH₃), COOH, COO-(Methyl), COO-(Ethyl), COO-(Propyl), COO-(Isopropyl), -O-Methylen-N(Methyl)₂, -O-Ethylen-N(Methyl)₂,-O-Methylen-N(Ethyl)₂, -O-Ethylen-N(Ethyl)₂, CO-NH₂, CO-NHCH₃, CO-N(CH₃)₂, NH-CO-Methyl, NCH₃-CO-Methyl, NH-CO-Ethyl, N(CH₃)-CO-Ethyl, Phenyl, Phenyl-Methylen-, Phenyl-Ethylen-, Het-Methylen-, Het-Ethylen-, -CO-Het, Het, -CO-C₄₋₇-Cycloalkyl, -CO-Cyclopropyl, -CO-N(CH₃)-Cyclopropyl, -CO-N(CH₃)-C₄₋₇-Cycloalkyl, C₄₋₇-Cycloalkyl, Cyclopropyl, C₄₋₇-Cycloalkyl-Methylen-, Cyclopropyl-Methylen-, C₄₋₇-Cycloalkyl-Ethylen-, Cyclopropyl-Ethylen-, Hetaryl-Methylen-, Hetaryl-Ethylen- und Hetaryl substituiert ist, welcher wiederum gegebenenfalls mit 1, 2, 3 oder 4 Resten unabhängig voneinander ausgewählt aus der Gruppe bestehend aus F, Cl, Br, Methyl, O-Methyl, Ethyl, O-Ethyl, OH, Oxo und CF₃ substituiert sein kann,
und worin die übrigen Variablem wie vorstehend definiert sind,
sowie pharmakologisch verträgliche Salze, Diastereomere, Enantiomere, Racemate, Hydrate oder Solvate davon.

Weiterhin bevorzugt im Rahmen der Erfindung sind die Verbindungen der Formel 1, worin
- R⁴: H, CN, OH, CF₃, CHF₂, CH₂F, F, Methyl, Ethyl, O-Methyl oder O-Ethyl, -Methylen-OH, -Ethylen-OH, -Propylen-OH, Isopropylen-OH, -COO(Methyl), -COO(Ethyl), -COO(Propyl), -COO(Isopropyl), -CO-Het, -(Methylen)-NH-SO₂-(Methyl), -(Methylen)-NH-SO₂-(Ethyl), -(Ethylen)-NH-SO₂-(Methyl), -(Ethylen)-NH-SO₂-(Ethyl), -(Methylen)-N(CH₃)-SO₂-(Methyl), -(Methylen)-N(CH₉)-SO₂-(Ethyl), -(Ethylen)-N(CH₃)-SO₂-(Methyl), -(Ethylen)-N(CH₃)-SO₂-(Ethyl), -(Methylen)-O-(Methylen)-Phenyl, -(Methylen)-O-(Ethylen)-Phenyl, -(Ethylen)-O-(Methylen)-Phenyl, -(Ethylen)-O-(Ethylen)-Phenyl, -Methylen-O-Methyl, -Methylen-O-Ethyl, -Ethylen-O-Methyl -Ethylen-O-Ethyl, -(Methylen)-N(CH₃)-CO-(Methyl), -(Methylen)-N(CH₃)-CO-(Ethyl) -(Ethylen)-N(CH₃)-CO-(Methyl), -(Ethylen)-N(CH₃)-CO-(Ethyl), -NH-CO-(Methylen)-O-(Methyl), -NH-CO-(Methylen)-O-(Ethyl), -NH-CO-(Ethylen)-O-(Methyl), -NH-CO-(Ethylen)-O-(Ethyl), -Methylen-NH-CO-(Methyl), -Methylen-NH-CO-(Ethyl), -Ethylen-NH-CO-(Methyl), -Ethylen-NH-CO-(Ethyl), -Methylen-NH-CO-(Methylen)-N(Methyl)₂, -Methylen-NH-CO-(Ethylen)-N(Methyl)₂, -Ethylen-NH-CO-(Methylen)-N(Methyl)₂. -Ethylen-NH-CO-(Ethylen)-N(Methyl)₂, -Methylen-NH-CO-(Methylen)-O-(Methyl), -Methylen-NH-CO-(Ethyten)-O-(Methyl), -Ethylen-NH-CO-(Methylen)-O-(Methyl), -Methylen-NH-CO-(Methylen)-O-(Ethyl). -Methylen-NH-CO-(Ethylen)-O-(Ethyl), -Ethylen-NH-CO-(Methylen)-O-(Ethyl), -(Methylen)-N(CH₃)-CO(Methylen)-O-(Methyl), -(Methylen)-N(CH₃)-CO-(Ethylen)-O-(Methyl), -(Ethylen)-N(CH₃)-CO-(Methylen)-O-(Methyl), -(Methylen)-N(CH₃)-CO-(Methylen)-O-(Ethyl), -(Methylen)-N(CH₃)-CO-(Ethylen)-O-(Ethyl), -(Ethylen)-N(CH₃)-CO-(Methylen)-O-(Ethyl), -O-(Methylen)-Phenyl, -O-(Ethylen)-Phenyl, -CO-Phenyl,
wobei das Phenyl in den obigen Resten gegebenenfalls mit einem oder mehreren weiteren Resten ausgewählt aus der Gruppe F, Cl, Br, Methyl, Ethyl, Propyl, -0-Methyl, -O-Ethyl, -O-Propyl, -OH und CF₃ substituiert sein kann,
und worin die übrigen Variablem wie vorstehend definiert sind,
sowie pharmakologisch verträgliche Salze, Diastereomere, Enantiomere, Racemate, Hydrate oder Solvate davon.

Ferner sind solche Verbindungen der Formel **1** im Rahmen der Erfindung bevorzugt, worin
- **R³**: ein Rest ausgewählt ist aus der Gruppe bestehend aus Oxazol, Imidazol und Thiazol, wobei dieser Rest gegebenenfalls durch einen, zwei oder drei weitere Reste unabhängig voneinander ausgewählt aus der Gruppe bestehend aus Methyl, Ethyl, Propyl, Isopropyl. O-Methyl, O-Ethyl, O-Propyl, O-Isopropyl, OH, F, Cl, Br, CF₃, Phenyl, Hetaryl und C₃₋₆-Cycloalkyl substituiert sein kann,
und worin die übrigen Variablem wie vorstehend definiert sind,
sowie pharmakologisch verträgliche Salze, Diastereomere, Enantiomere, Racemate, Hydrate oder Solvate davon.

Ein weiterer bevorzugter Gegenstand der vorliegenden Erfindung sind die obigen Verbindungen der Formel **1**, worin
- worin **X**: SO₂ ist,
und worin die übrigen Variablem wie vorstehend definiert sind,
sowie pharmakologisch verträgliche Salze, Diastereomere, Enantiomere, Racemate, Hydrate oder Solvate davon.

Die Erfindung betrifft insbesondere Verbindungen nach Formel **1**, die ausgewählt sind aus der Gruppe bestehend aus 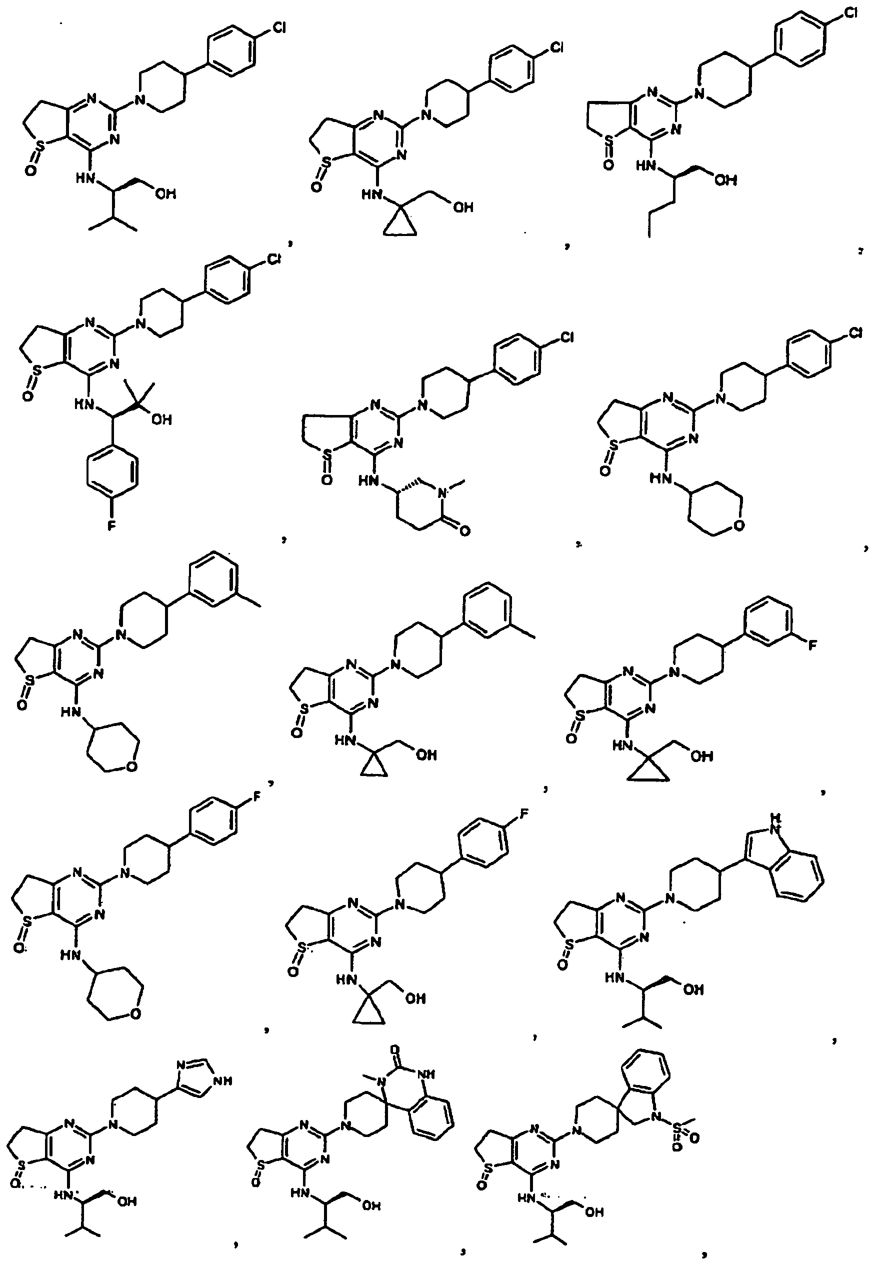 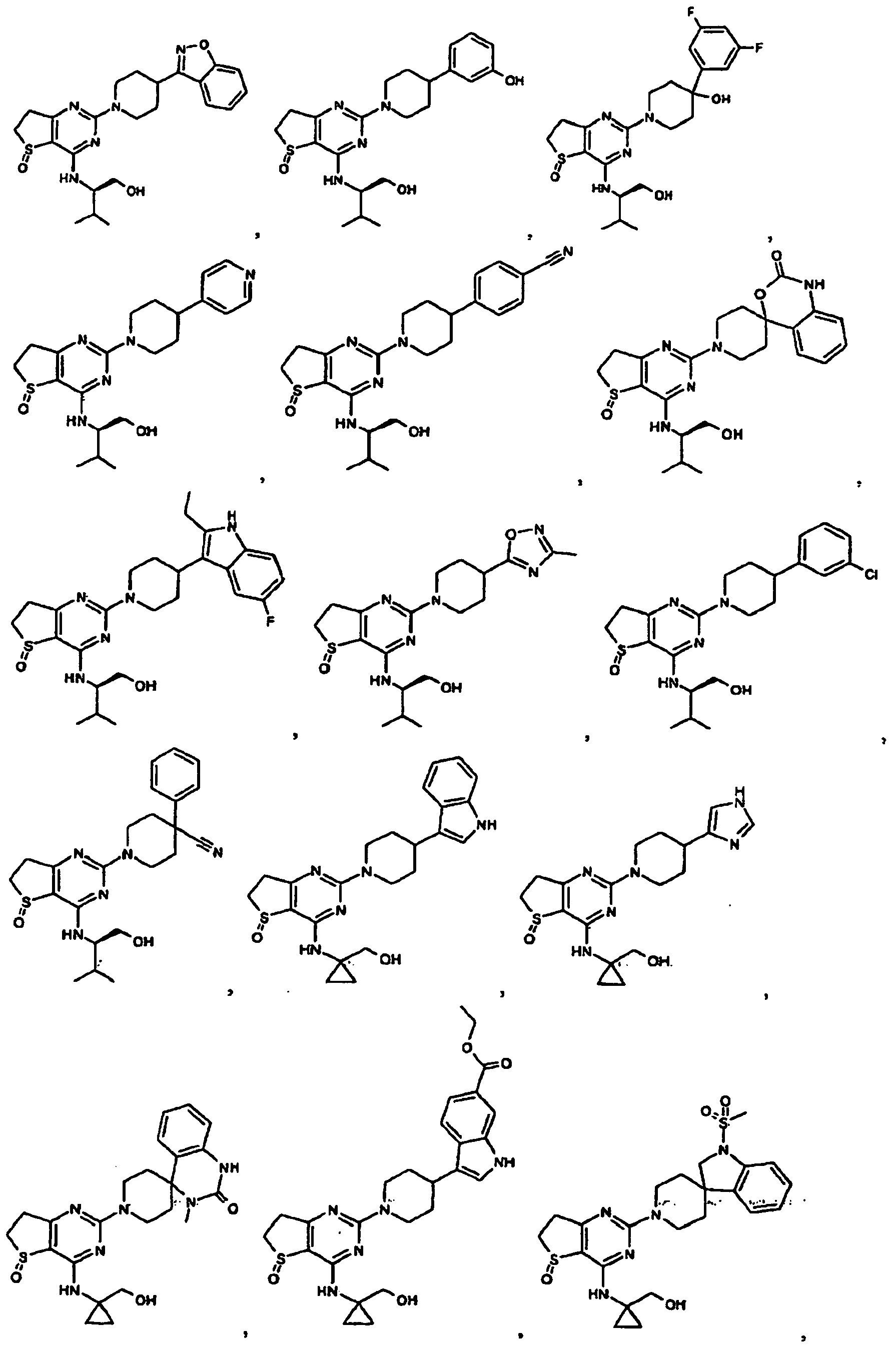 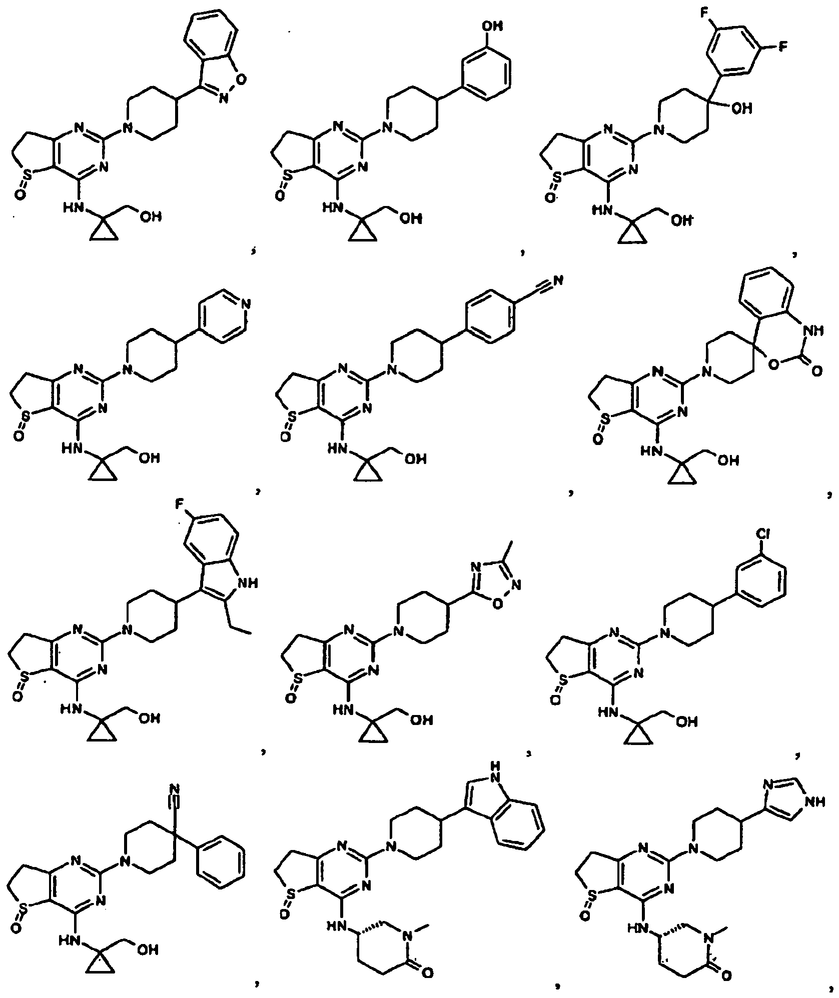 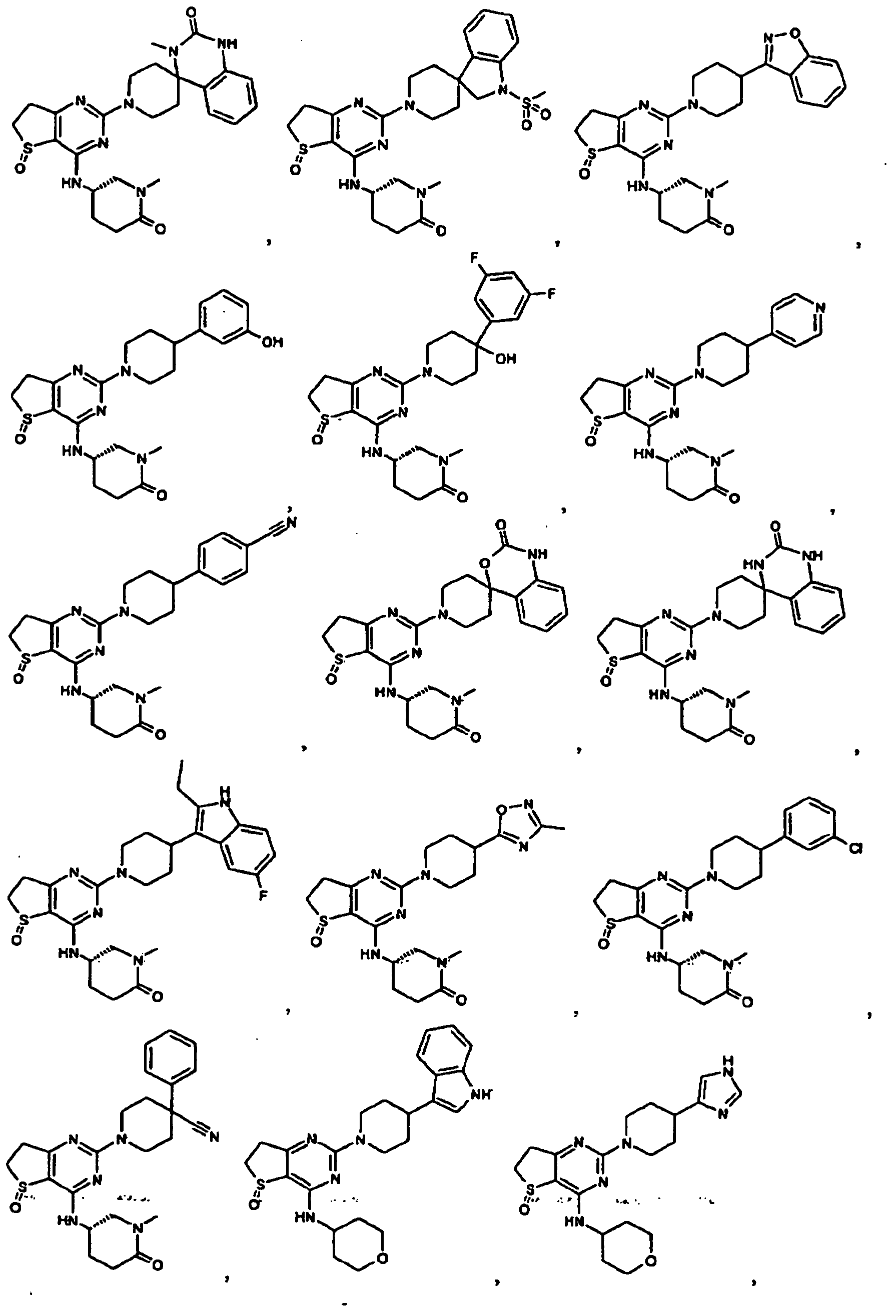 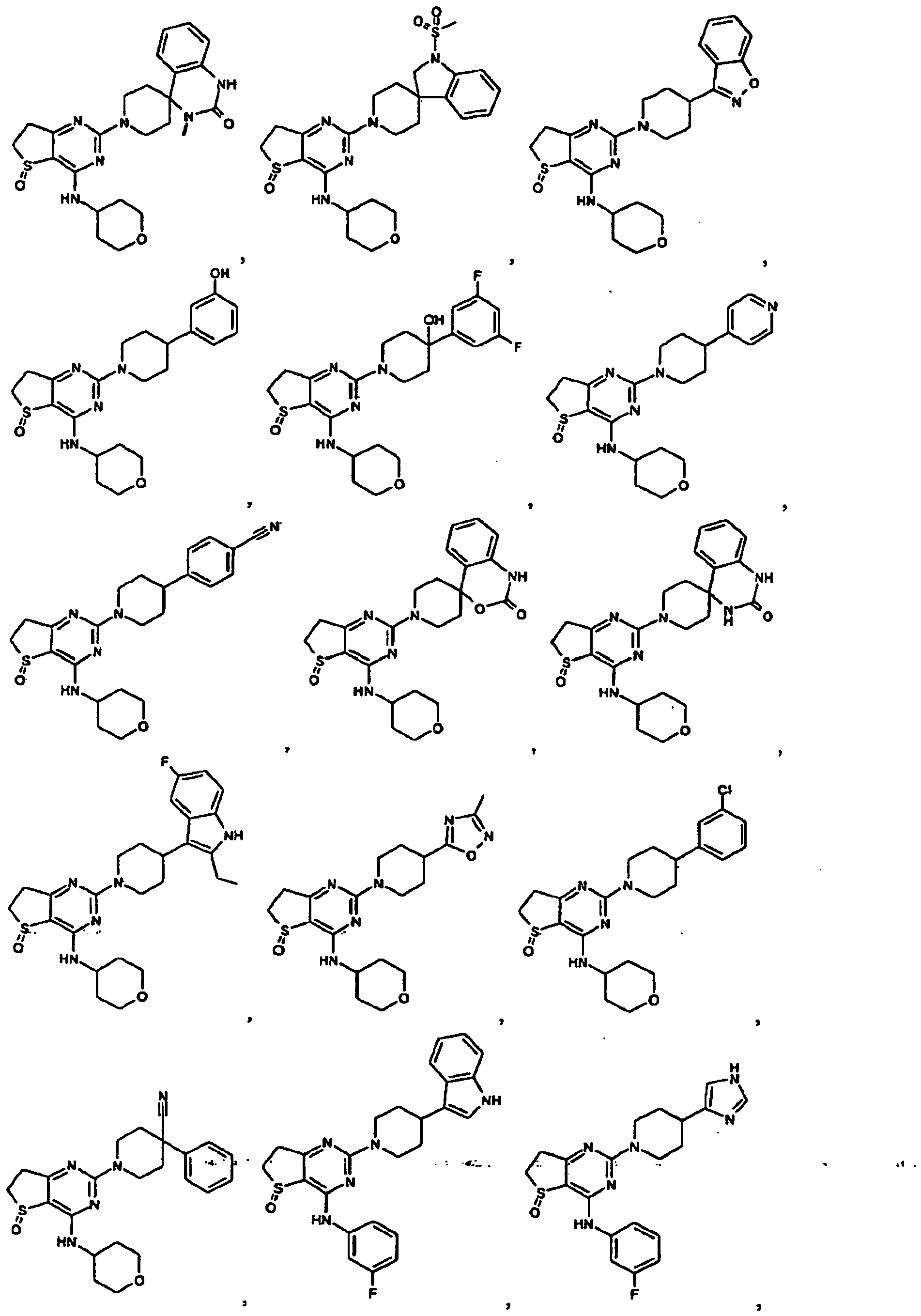 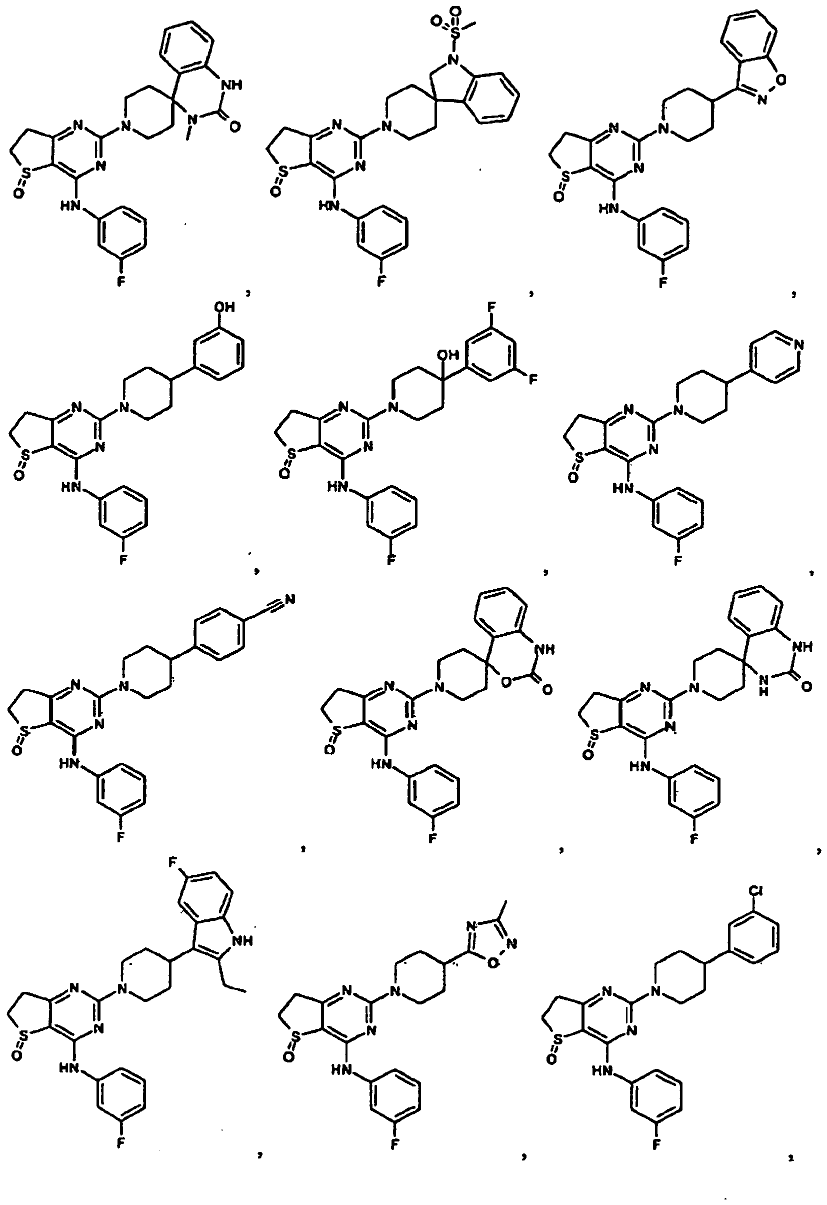 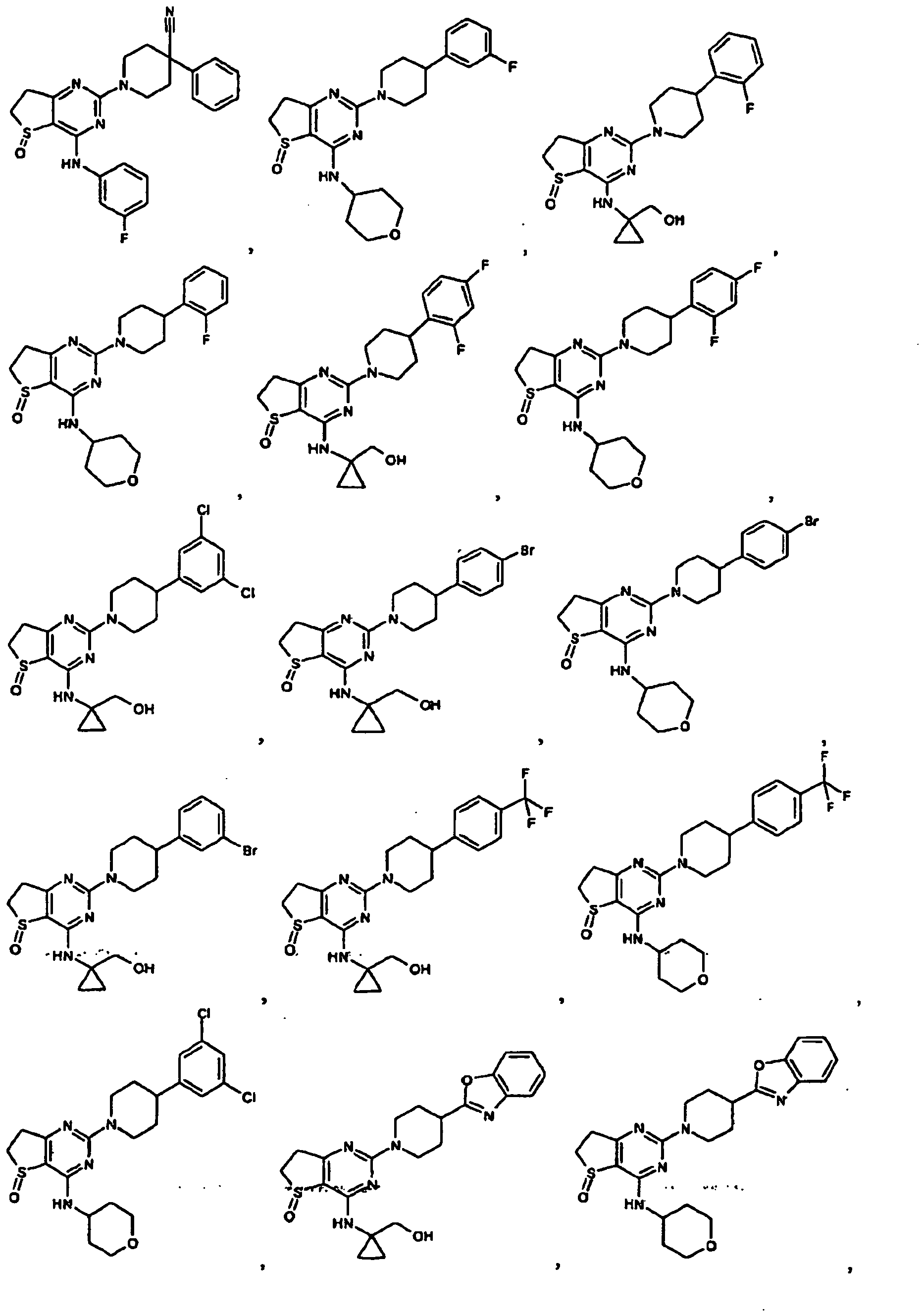 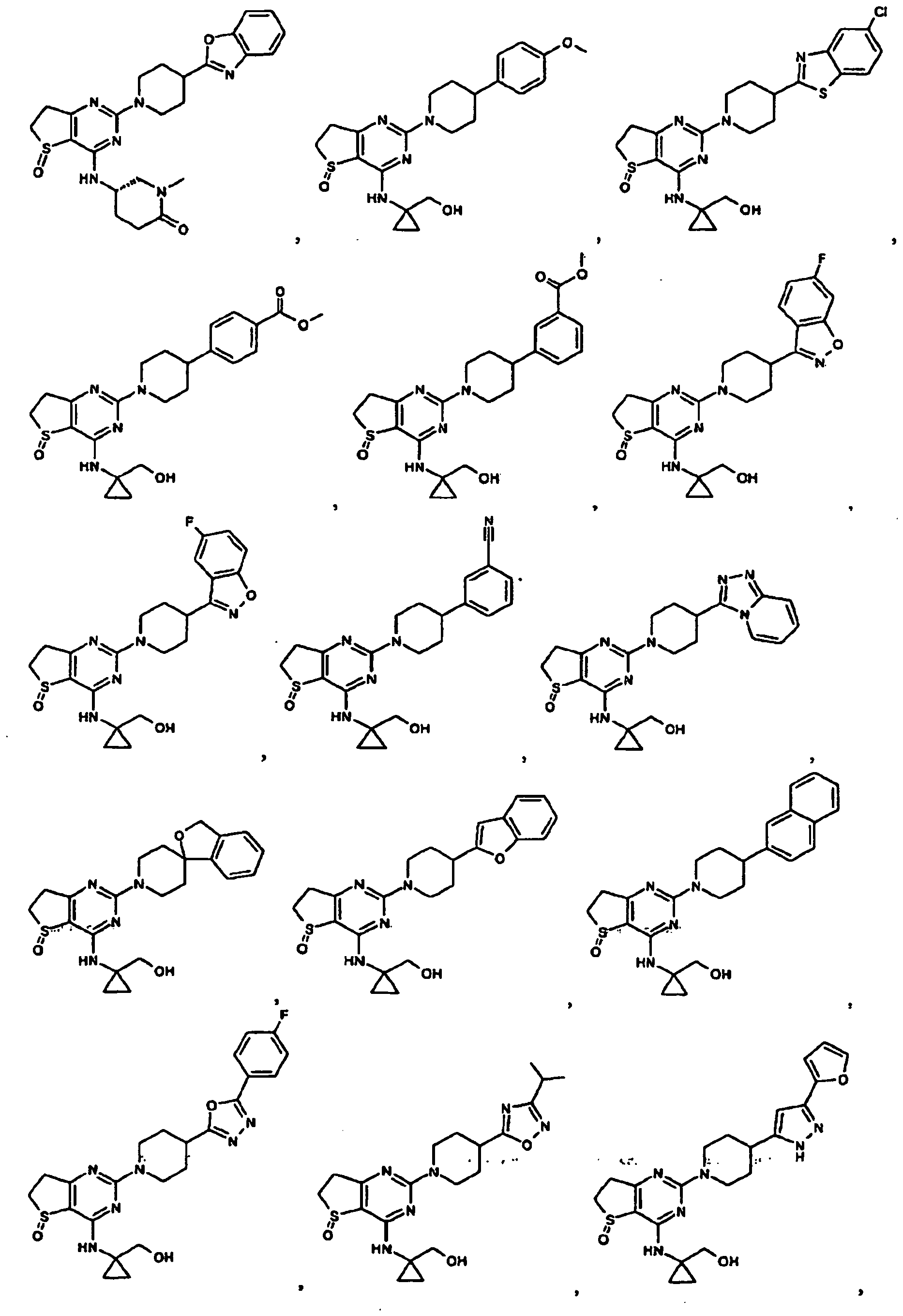 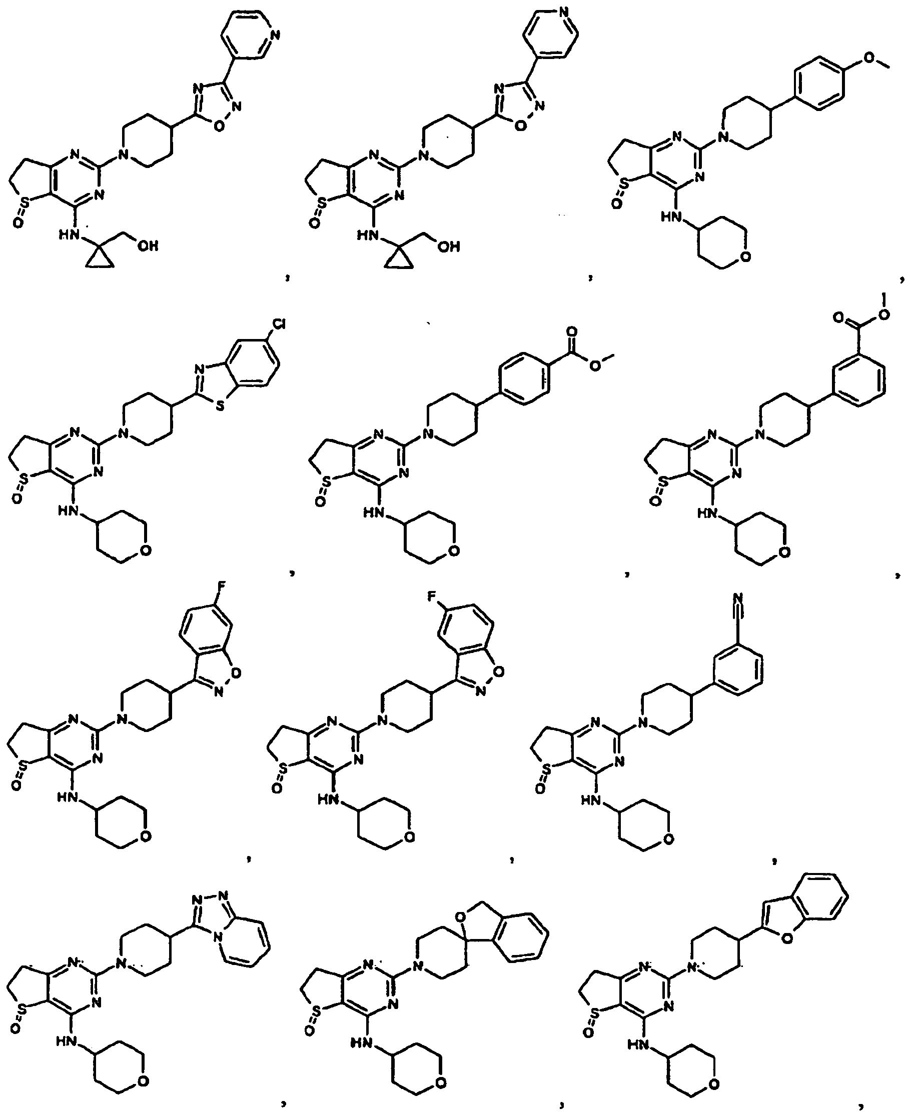 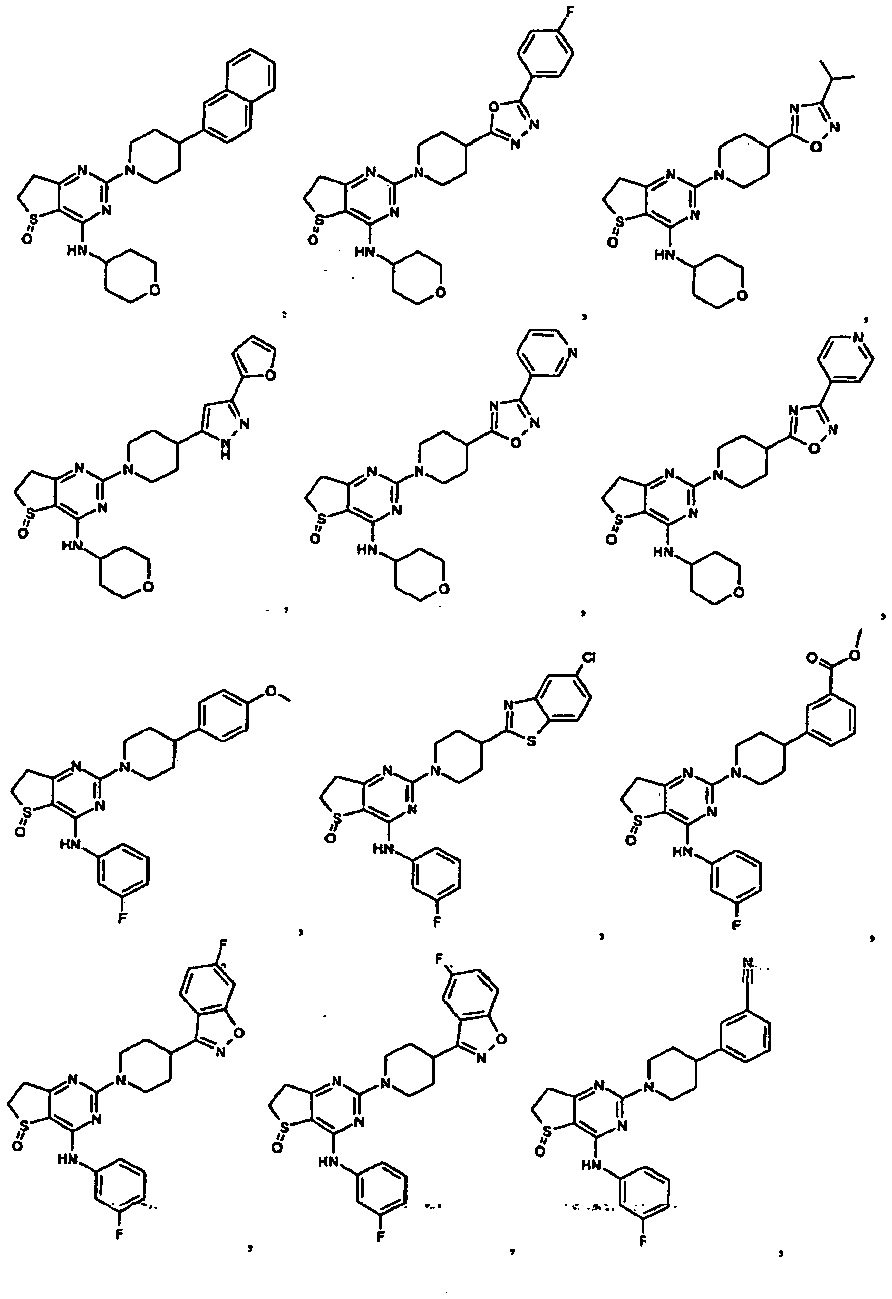 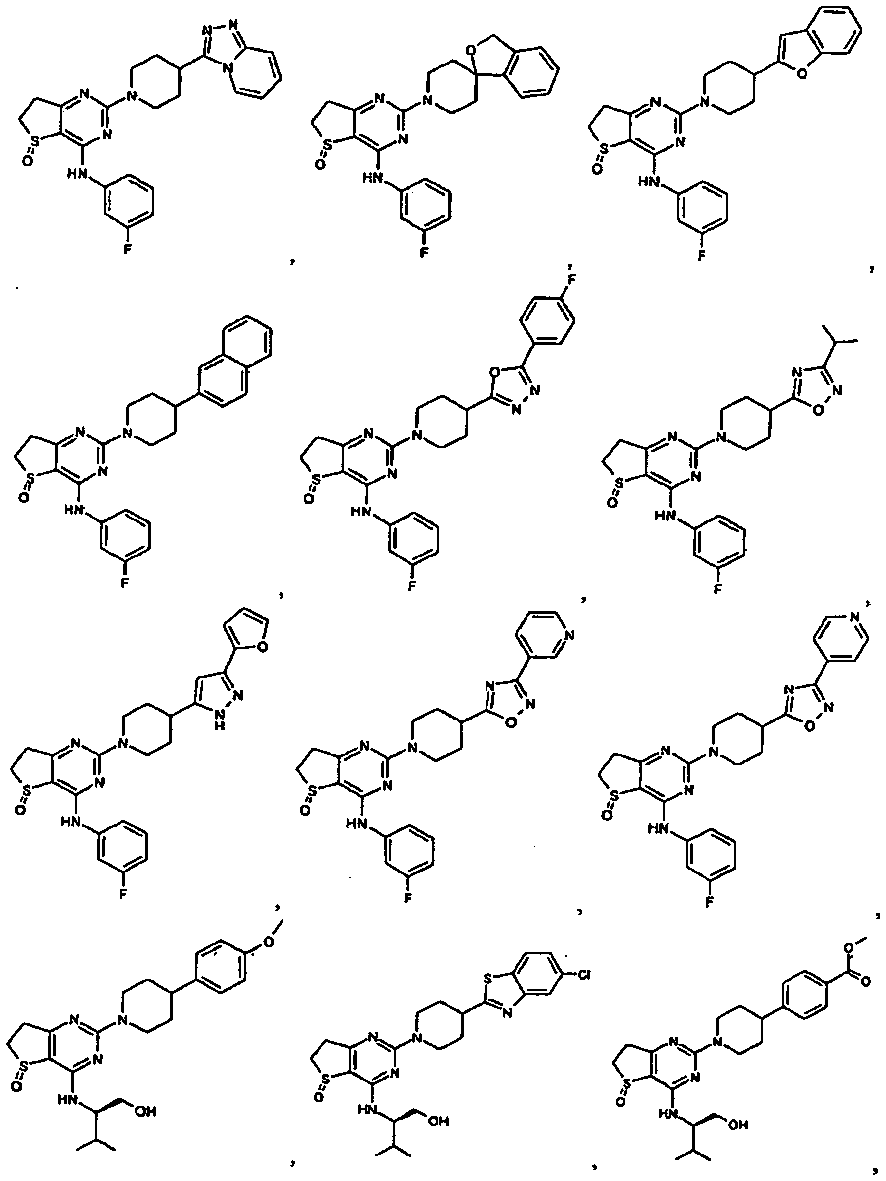 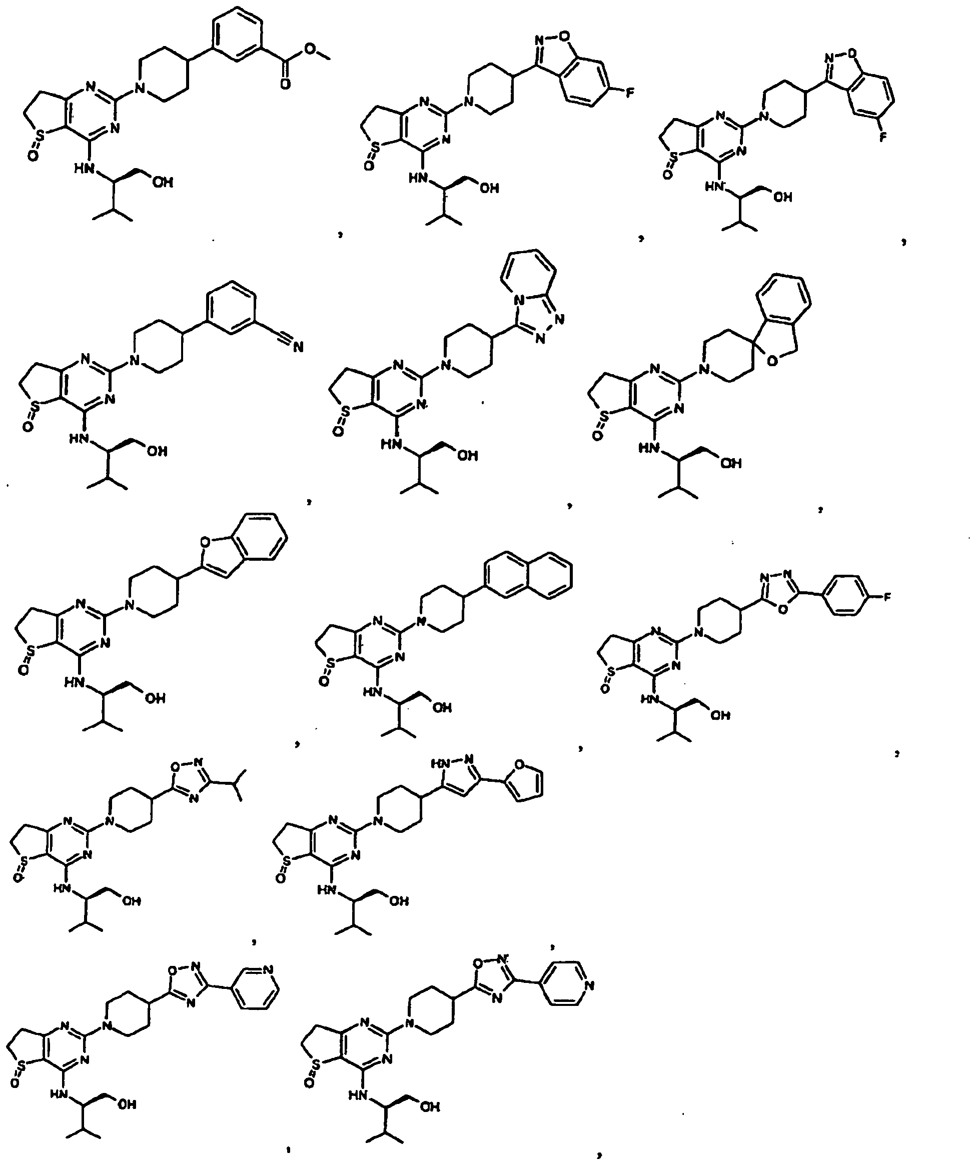 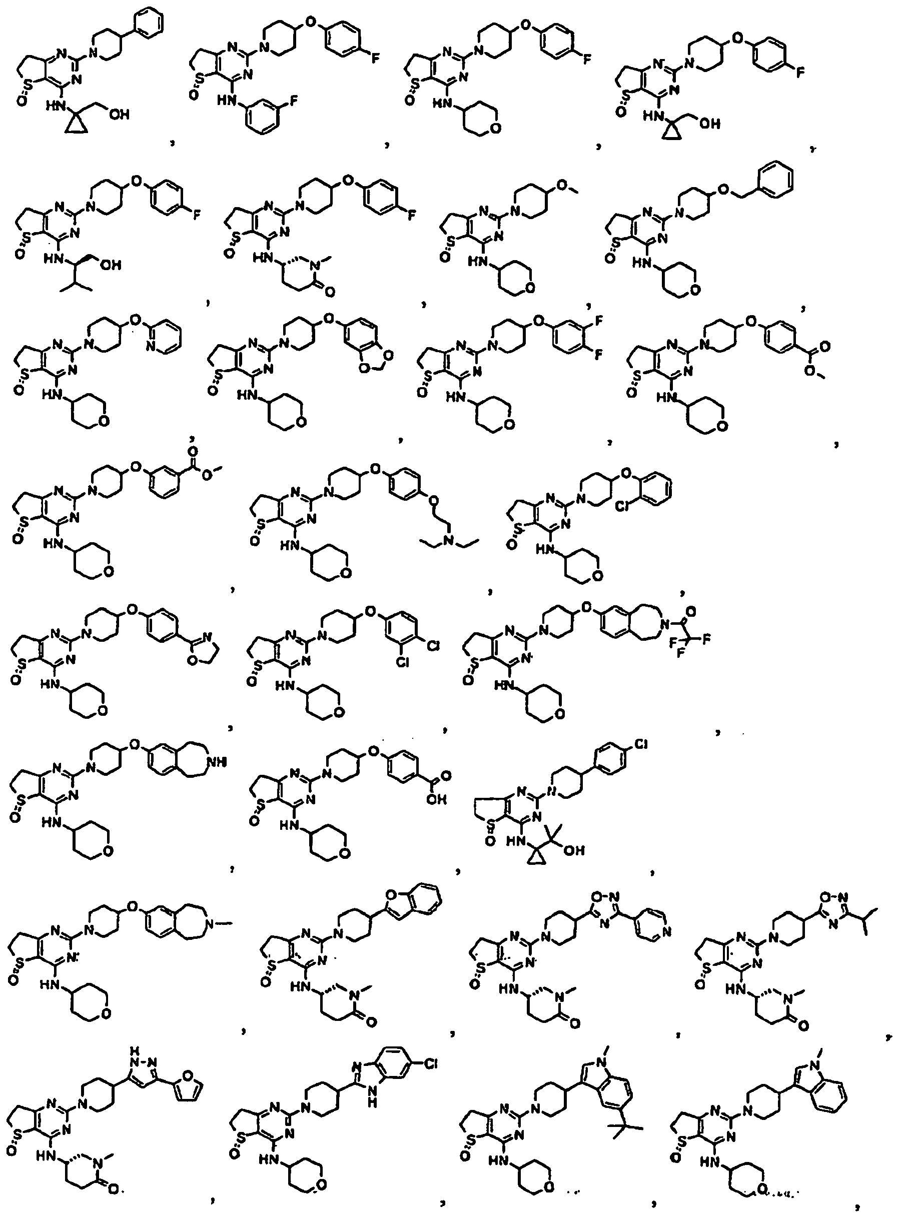 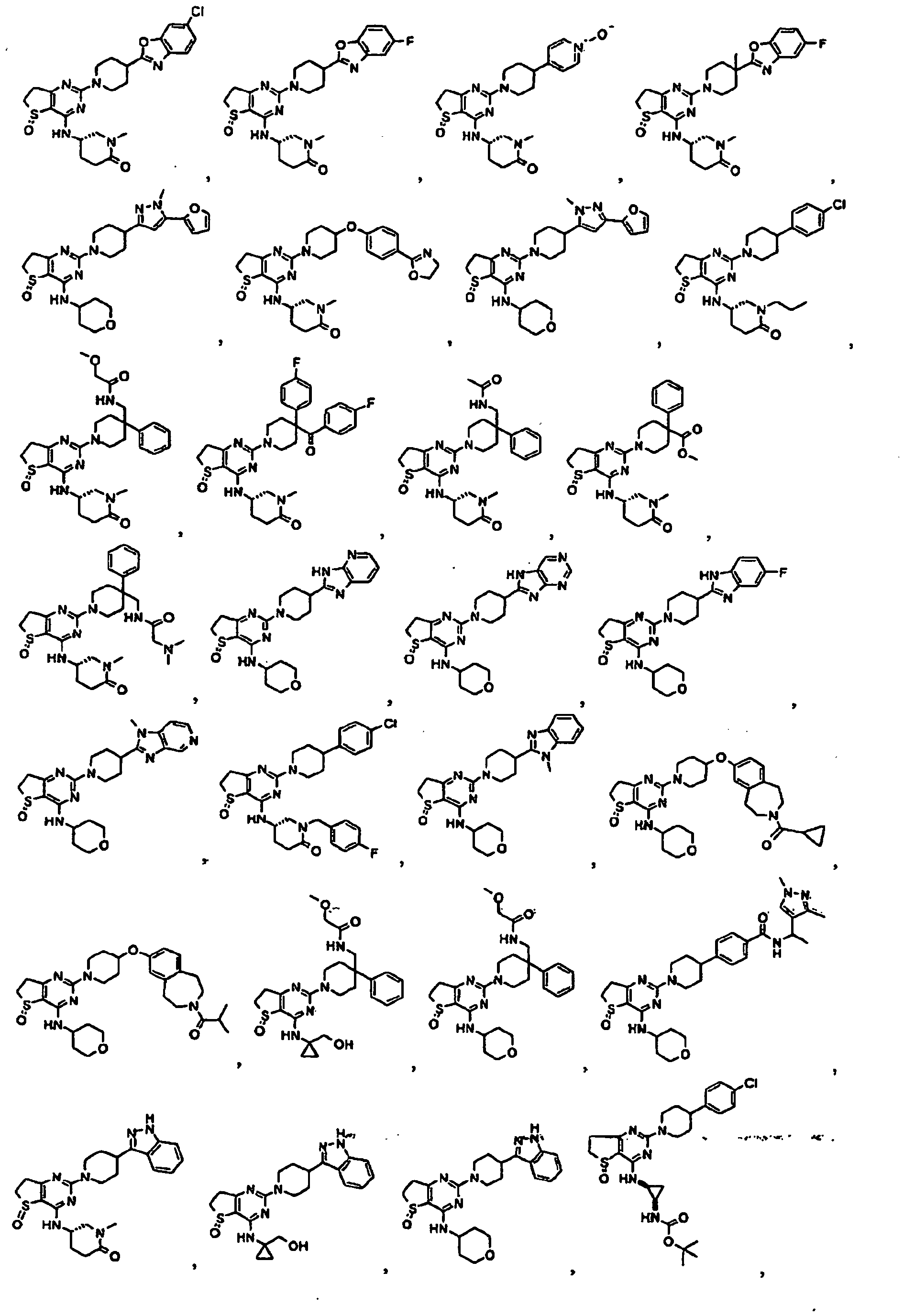 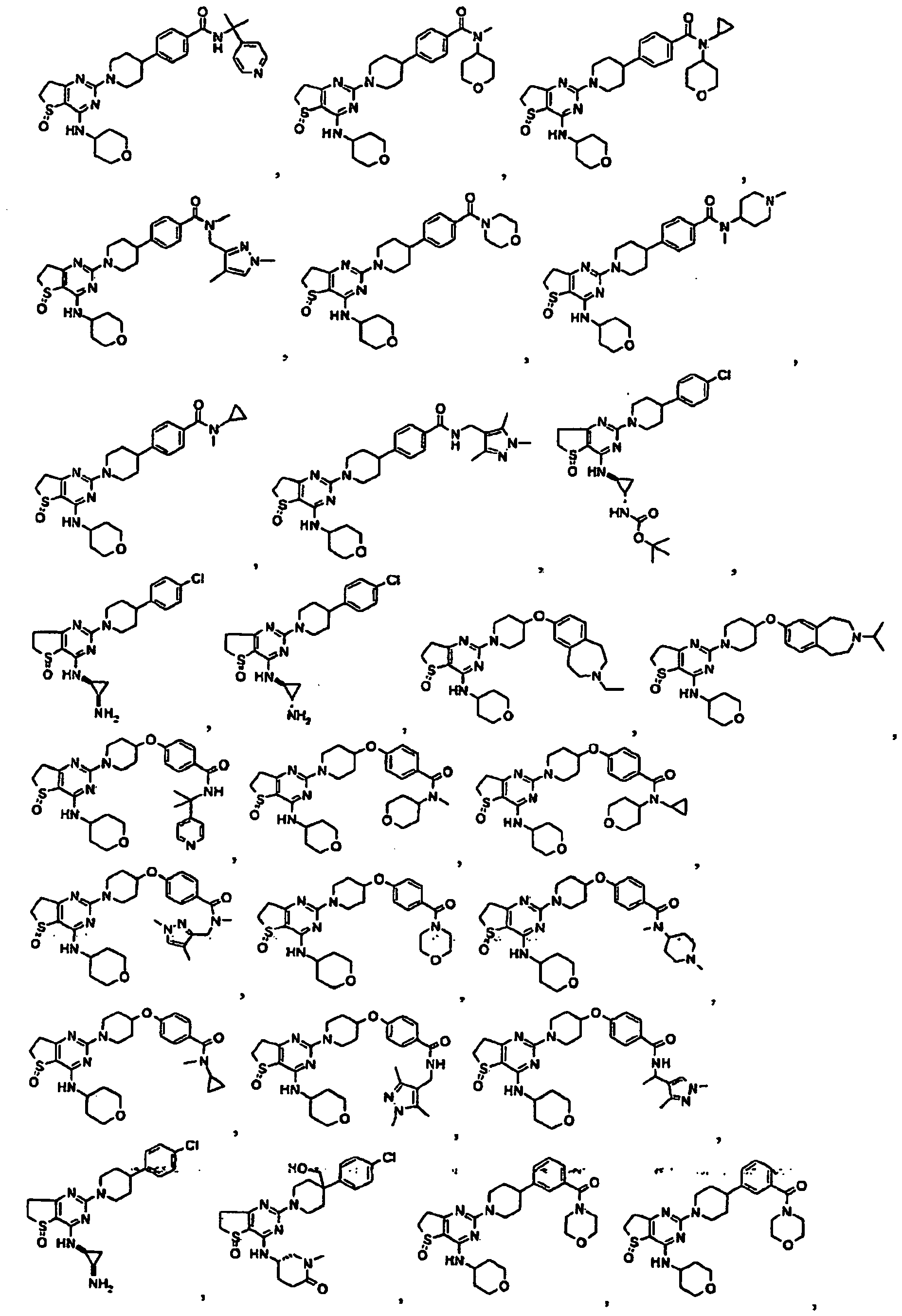 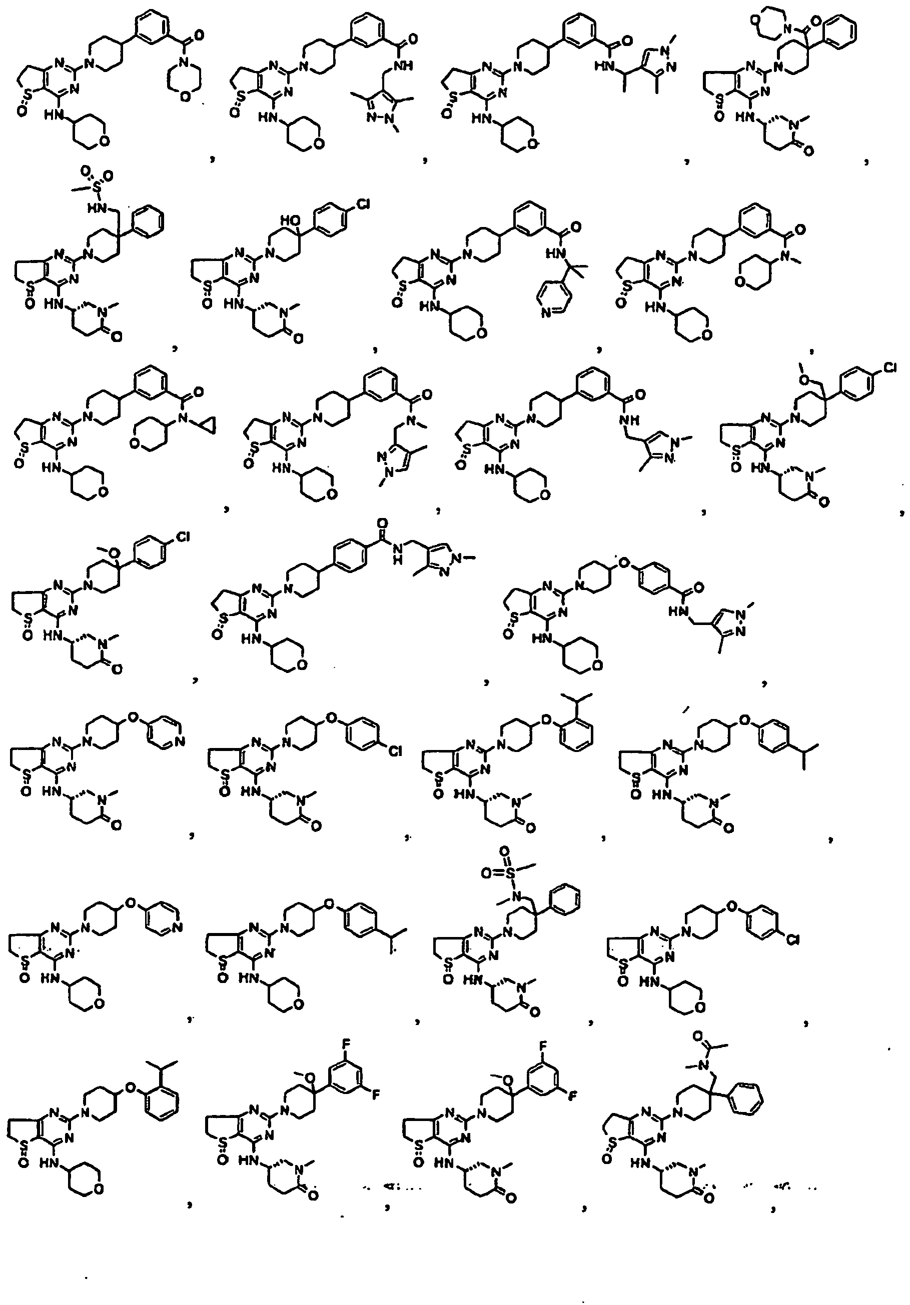 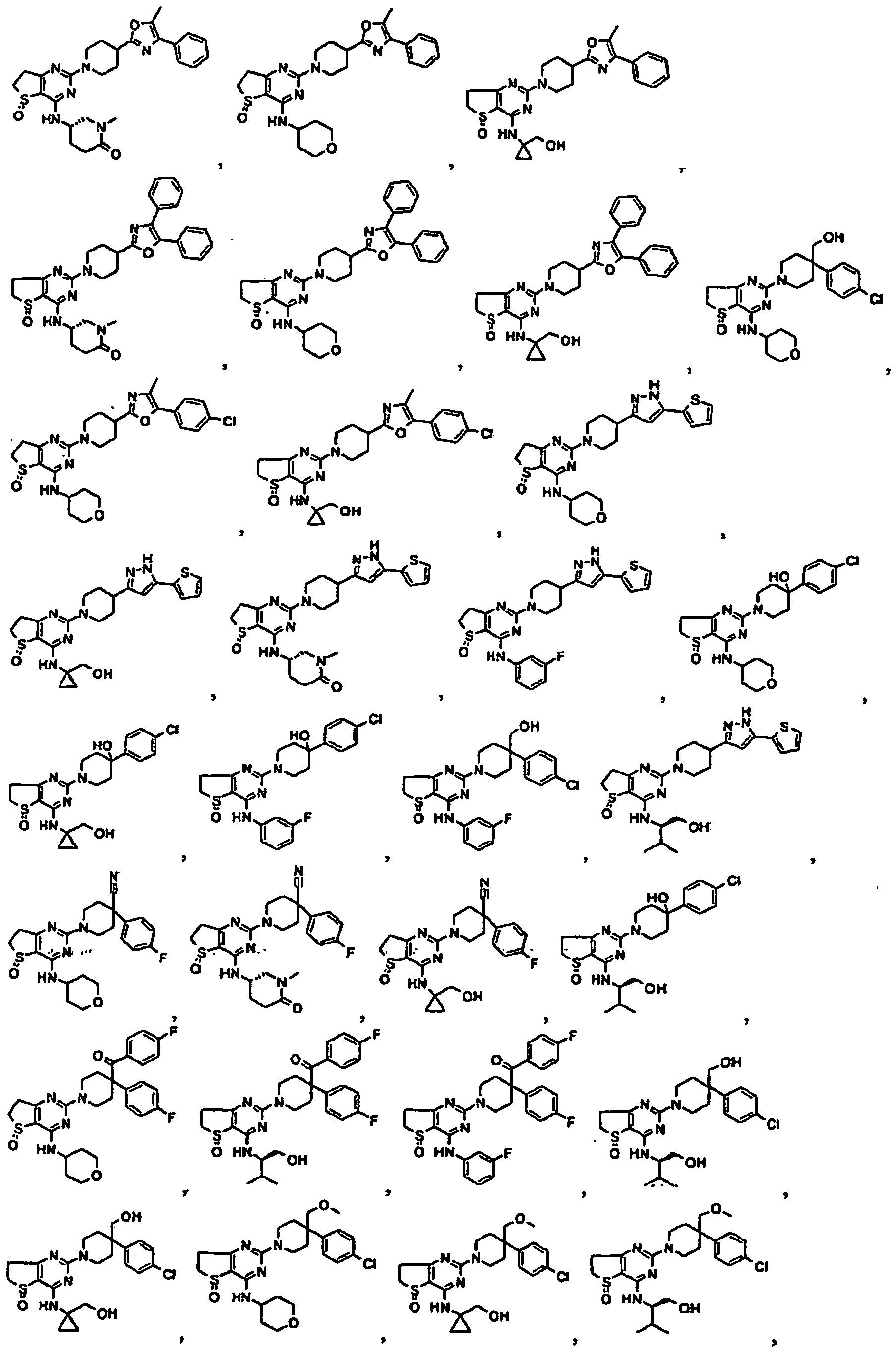 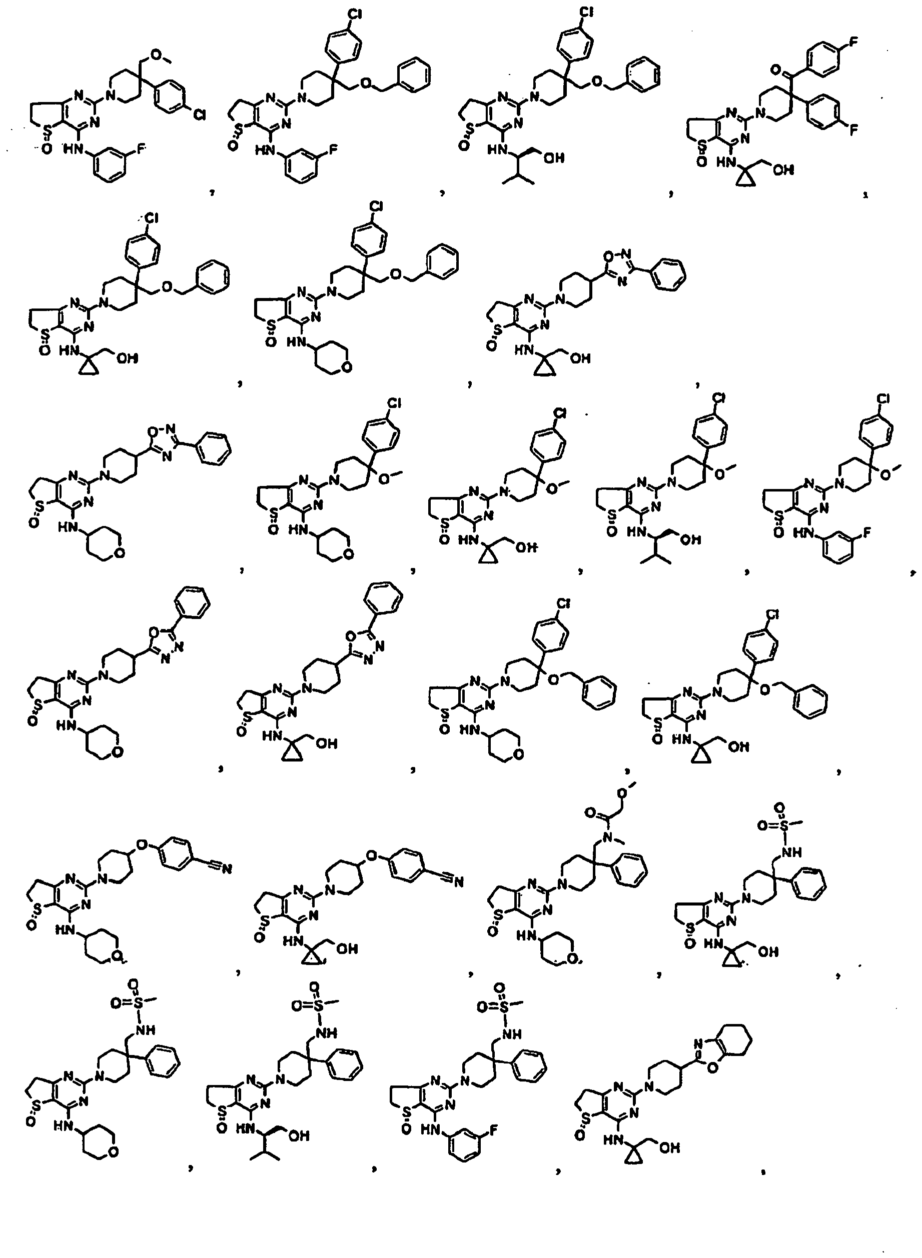 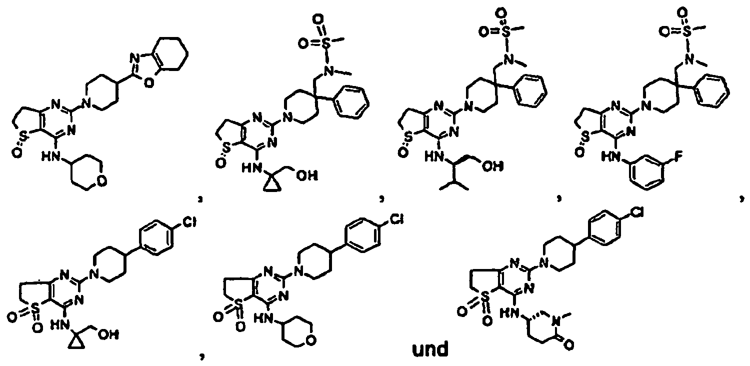 sowie pharmakologisch verträgliche Salze, Diastereomere, Enantiomere, Racemate, Hydrate oder Solvate davon, insbesondere die R-Enantiomere als auch die S-Enantiomere bezüglich des Stereozentrums am Sulfoxid-Schwefel-Atom der obigen Verbindungen.

Ein weiterer Gegenstand der Erfindung sind die obigen Verbindungen der Formel 1 als Arzneimittel.

Ein weiterer Gegenstand der Erfindung ist die Verwendung der obigen Verbindungen nach Formel 1 zur Herstellung eines Medikaments zur Behandlung von Krankheiten, die sich durch Inhibition des PDE4-Enzyms behandeln lassen.

Ein weiterer Gegenstand der Erfindung ist die Verwendung der obigen Verbindungen nach Formel 1 zur Herstellung eines Medikaments zur Behandlung von Atemwegs- oder gastrointestinalen Beschwerden oder Erkrankungen, wie auch entzündliche Erkrankungen der Gelenke, der Haut oder der Augen, Krebserkrankungen, sowie von Erkrankungen des periphären oder zentralen Nervensystems.

Ein weiterer Gegenstand der Erfindung ist die Verwendung der obigen Verbindungen nach Formel **1** zur Herstellung eines Medikaments zur Vorbeugung und Behandlung von Atemwegs- oder Lungenerkrankungen, die mit einer erhöhten Schleimproduktion, Entzündungen und / oder obstruktiven Erkrankungen der Atemwege einhergehen.

Ein weiterer Gegenstand der Erfindung ist die Verwendung der obigen Verbindungen nach Formel **1** zur Herstellung eines Medikament zur Behandlung von entzündlichen und obstruktiven Erkrankungen wie COPD, chronischer Sinusitis, Asthma, Morbus Crohn, Colitis ulcerosa.

Ein weiterer Gegenstand der Erfindung ist die Verwendung der obigen Verbindungen der Formel **1** zur Herstellung eines Medikaments zur Behandlung von entzündlichen Erkrankungen des Gastrointestinaltraktes.

Ein weiterer Gegenstand der Erfindung ist die Verwendung der obigen Verbindungen der Formel **1** zur Herstellung eines Medikaments zur Vorbeugung und Behandlung von Erkrankungen des periphären oder zentralen Nervensystems wie Depression, bipolare oder manische Depression, akute und chronische Angstzustände, Schizophrenie, Alzheimer'sche Erkrankung, Parkinson'sche Erkrankung, akute und chronische Multiple Sklerose oder akute und chronische Schmerzzustände sowie Verletzungen des Gehirns hervorgerufen durch Schlaganfall, Hypoxie oder Schädel-Hirn-Trauma.

Ein weiterer Gegenstand der Erfindung sind pharmazeutische Formulierungen, die eine oder mehrere der obigen Verbindungen nach Formel **1** enthalten.

Ein weiterer Gegenstand der Erfindung sind pharmazeutische Formulierungen enthaltend eine oder mehrere Verbindungen der Formel **1** in Kombination mit einem oder mehreren Wirkstoffen ausgewählt aus der Gruppe bestehend aus Betamimetika, Corticosteroiden, weiteren POE4-Inhibitoren, EGFR-Hemmem und LTD4-Antagonisten, CCR3-Inhibitoren, iNOS-Inhibitoren und SYK-Inhibitoren.

### VERWENDETE BEGRIFFE UND DEFINITIONEN

Soweit nicht anders angegeben, sind alle Substituenten voneinander unabhängig. Sollten an einer Gruppe beispielsweise mehrere C₁₋₈-Alkylgruppen als Substituenten möglich sein, so könnte, beilspielsweise im Fall von drei Substituenten C₁₋₆-Alkyl unabhängig voneinander beispielsweise einmal Methyl, einmal *n*-Propyl und einmal *tert*-Butyl bedeuten.

Im Rahmen dieser Anmeldung können bei der Definition von möglichen Substituenten, diese auch in Form einer Strukturformel dargestellt werden. Dabei wird ein Stern (*) in der Strukturformel des Substituenten als der Verknüpfungspunkt zum Rest des Moleküls verstanden. Weiterhin wird das auf den Verknüpfungspunkt folgende Atom des Substituenten als das Atom mit der Positionsnummer 1 verstanden. So werden zum Beispiel die Reste N-Piperidinyl (**I**), 4-Piperidinyl (**II**), 2-Tolyl (**III**), 3-Tolyl (**IV**) und 4-Tolyl (**V**) wie folgt dargestellt:

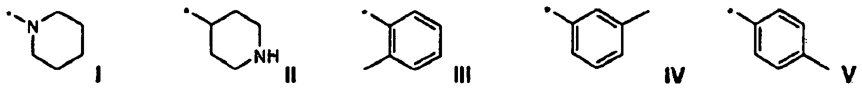

Befindet sich in der Strukturformel des Substituenten kein Stern (*), so kann an dem Substituenten jedes Wasserstoffatom entfernt werden und die dadurch frei werdende Valenz als Bindungsstelle zum Rest eines Molekül dienen, sofern der Anknüpfungspunkt zum Restmolekül nicht anderweitig bezeichnet oder definiert ist. So kann zum Beispiel **VI** die Bedeutung von 2-Tolyl, 3-Tolyl, 4-Tolyl und Benzyl haben.

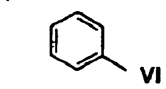

Unter dem Begriff "C₁₋₁₀-Alkyl" (auch soweit sie Bestandteil anderer Reste sind) werden verzweigte und unverzweigte Alkylgruppen mit 1 bis 10 Kohlenstoffatornen, unter dem Begriff "C₁₋₆-Alkyl" dementsprechend verzweigte und unverzweigte Alkylgruppen mit 1 bis 6 Kohlenstoffatomen verstanden. "C₁₋₄-Alkyl" steht entsprechend für verzweigte und unverzweigte Alkylgruppen mit 1 bis 4 Kohlenstoffatomen. Bevorzugt sind Alkylgruppen mit 1 bis 4 Kohlenstoffatomen. Beispielsweise werden hierfür genannt: Methyl, Ethyl, *n*-Propyl, *iso-*Propyl, *n*-Butyl, *iso*-Butyl, *sec*-Butyl, *tert*-Butyl, *n*-Pentyl, *iso*-Pentyl, *neo*-Pentyl oder Hexyl. Gegebenenfalls werden für vorstehend genannten Gruppen auch die Abkürzungen Me, Et, *n*-Pr, *i*-Pr, *n*-Bu, *i*-Bu, *t*-Bu, etc. verwendet. Sofern nicht anders beschrieben, umfassen die Definitionen Propyl, Butyl, Pentyl und Hexyl alle denkbaren isomeren Formen der jeweiligen Reste. So umfasst beispielsweise Propyl *n*-Propyl und *iso*-Propyl, Butyl umfasst *iso*-Butyl, *sec*-Butyl und *tert*-Butyl etc.

Unter dem Begriff "C₁₋₆-Alkylen" (auch soweit sie Bestandteil anderer Reste sind) werden verzweigte und unverzweigte Alkylengruppen mit 1 bis 6 Kohlenstoffatomen verstanden und unter dem Begriff "C₁₋₄-Alkylen" verzweigte und unverzweigte Alkylengruppen mit 1 bis 4 Kohlenstoffatomen verstanden. Bevorzugt sind Alkylengruppen mit 1 bis 4 Kohlenstoffatomen. Beispielsweise werden hierfür genannt: Methylen, Ethylen, Propylen, 1-Methylethylen, Butylen, 1-Methylpropylen, 1,1-Dimethylethylen, 1, 2-Dimethylethylen, Pentylen, 1, 1-Dimethylpropylen, 2, 2, -Dimethylpropylen, 1, 2-Dimethylpropylen, 1, 3-Dimethylpropylen oder Hexylen. Sofern nicht anders beschrieben, umfassen die Definitionen Propylen, Butylen, Pentylen und Hexylen alle denkbaren isomeren Formen der jeweiligen Reste gleicher Kohlenstoffanzahl. So umfasst beispielsweise Propyl auch 1-Methylethylen und Butylen umfasst 1-Methylpropylen, 1,1-Dimethylethylen, 1, 2-Dimethylethylen.

Sollte die Kohlenstoffkette mit einem Rest substituiert sein, der gemeinsam mit einem oder zwei Kohlenstoffatomen der Alkylenkette einen carbocyclischen Ring mit 3, 5 oder 6 Kohlenstoffatomen bildet, so sind damit unter anderem folgende Beispiele der Ringe umfasst:

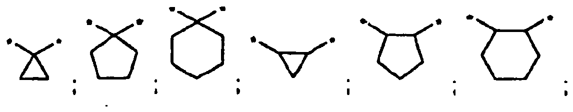

Unter dem Begriff "C₂₋₆-Alkenyl" (auch soweit sie Bestandteil anderer Reste sind) werden verzweigte und unverzweigte Alkenylgruppen mit 2 bis 6 Kohlenstoffatomen und unter dem Begriff "C₂₋₄-Alkenyl" verzweigte und unverzweigte Alkenylgruppen mit 2 bis 4 Kohlenstoffatomen verstanden, soweit sie mindestens eine Doppelbindung aufweisen. Bevorzugt sind Alkenylgruppen mit 2 bis 4 Kohlenstoffatome. Beispielsweise werden hierfür genannt: Ethenyl oder Vinyl, Propenyl, Butenyl, Pentenyl, oder Hexenyl. Sofern nicht anders beschrieben, umfassen die Definitionen Propenyl, Butenyl, Pentenyl und Hexenyl alle denkbaren isomeren Formen der jeweiligen Reste. So umfasst beispielsweise Propenyl 1-Propenyl und 2-Propenyl, Butenyl umfasst 1-, 2- und 3-Butenyl, 1-Methyl-1-propenyl, 1-Methyl-2-propenyl etc.

Unter dem Begriff "C₂₋₆-Alkenylen" (auch soweit sie Bestandteil anderer Reste sind) werden verzweigte und unverzweigte Alkenylengruppen mit 2 bis 6 Kohlenstoffatomen verstanden und unter dem Begriff "C₂₋₄-Alkenylen" verzweigte und unverzweigte Alkylengruppen mit 2 bis 4 Kohlenstoffatomen verstanden. Bevorzugt sind Alkenylengruppen mit 2 bis 4 Kohlenstoffatome. Beispielsweise werden hierfür genannt: Ethenylen, Propenylen, 1-Methylethenylen, Butenylen, 1-Methylpropenylen. 1, 1-Dimethylethenylen, 1, 2-Dimethylethenylen, Pentenylen, 1, 1-Dimethylpropenylen, 2, 2, -Dimethylpropenylen, 1, 2-Dimethylpropenylen, 1, 3-Dimethylpropenylen oder Hexenylen. Sofern nicht anders beschrieben, umfassen die Definitionen Propenylen, Butenylen, Pentenylen und Hexenylen alle denkbaren isomeren Formen der jeweiligen Reste gleicher Kohlenstoffanzahl. So umfasst beispielsweise Propenyl auch 1-Methylethenylen und Butenylen umfasst 1-Methylpropenylen, 1, 1-Dimethylethenylen, 1, 2-Dimethylethenylen.

Unter dem Begriff "C₂₋₈-Alkinyl" (auch soweit sie Bestandteil anderer Reste sind) werden verzweigte und unverzweigte Alkinylgruppen mit 2 bis 6 Kohlenstoffatomen und unter dem Begriff "C₂₋₄-Alkinyl" verzweigte und unverzweigte Alkinylgruppen mit 2 bis 4 Kohlenstoffatomen verstanden, soweit sie mindestens eine Dreifachbindung aufweisen.

Bevorzugt sind Alkinylgruppen mit 2 bis 4 Kohlenstoffatome. Beispielsweise werden hierfür genannt: Ethinyl, Propinyl, Butinyl, Pentinyl, oder Hexinyl. Sofern nicht anders beschrieben, umfassen die Definitionen Propinyl, Butinyl, Pentinyl und Hexinyl alle denkbaren isomeren Formen der jeweiligen Reste. So umfasst beispielsweise Propinyl 1-Propinyl und 2-Propinyl, Butinyl umfasst 1-, 2- und 3-Butinyl, 1-Methyl-1-propinyl, 1 -Methyl-2-propinyl etc.

Unter dem Begriff "C₂₋₆-Alkinylen" (auch soweit sie Bestandteil anderer Reste sind) werden verzweigte und unverzweigte Alkinylengruppen mit 2 bis 6 Kohlenstoffatomen verstanden und unter dem Begriff "C₂₋₄-Alkinylen" verzweigte und unverzweigte Alkylengruppen mit 2 bis 4 Kohlenstoffatomen verstanden. Bevorzugt sind Alkinylengruppen mit 2 bis 4 Kohlenstoffatome. Beispielsweise werden hierfür genannt: Ethinylen, Propinylen, 1-Methylethinylen, Butinylen, 1-Methylpropinylen, 1, 1-Dimethylethinylen, 1, 2-Dimethylethinylen, Pentinylen, 1, 1-Dimethylpropinylen, 2, 2, -Dimethylpropinylen, 1, 2-Dimethylpropinylen, 1, 3-Dimethylpropinylen oder Hexinylen. Sofern nicht anders beschrieben, umfassen die Definitionen Propinylen, Butinylen, Pentinylen und Hexinylen alle denkbaren isomeren Formen der jeweiligen Reste gleicher Kohlenstoffanzahl. So umfasst beispielsweise Propinyl auch 1-Methylethinylen und Butinylen umfasst 1-Methylpropinylen, 1, 1-Dimethylethinylen, 1, 2-Dimethylethinylen.

Unter dem Begriff "Aryl" (auch soweit sie Bestandteil anderer Reste sind) werden aromatische Ringsysteme mit 6 bis 10 Kohlenstoffatomen verstanden. Beispielsweise werden hierfür genannt: Phenyl oder Naphthyl, bevorzugter Arylrest ist Phenyl. Soweit nicht anders beschrieben, können die Aromaten substituiert sein mit einem oder mehreren Resten ausgewählt aus der Gruppe bestehend aus Methyl, Ethyl, *iso*-Propyl, *tert*-Butyl, Hydroxy, Fluor, Chlor, Brom und Jod.

Unter dem Begriff "Aryl-C₁₋₆-alkylen" (auch soweit sie Bestandteil anderer Reste sind) werden verzweigte und unverzweigte Akylengruppen mit 1 bis 6 Kohlenstoffatomen verstanden, die mit einem aromatischen Ringsystem mit 6 oder 10 Kohlenstöffatomen substituiert sind. Beispielsweise werden hierfür genannt: Benzyl, 1- oder 2-Phenylethyl oder 1- oder 2-Naphthylethyl. Soweit nicht anders beschreiben, können die Aromaten substituiert sein mit einem oder mehreren Resten ausgewählt aus der Gruppe bestehend aus Methyl, Ethyl, *iso*-Propyl, *tert*-Butyl, Hydroxy, Fluor, Chlor, Brom und Jod.

Unter dem Begriff "Heteroaryl-C₁₋₆-alkylen" (auch soweit sie Bestandteil anderer Reste sind) werden - obwohl auch bereits unter "Aryl-C₁₋₆-alkylen umfasst - verzweigte und unverzweigte Alkylengruppen mit 1 bis 6 Kohlenstoffatomen verstanden, die mit einem Heteroaryl substituiert sind.

Ein solches Heteroaryl umfasst fünf oder sechsgliedrige heterocyclische Aromaten oder 5-10 gliedrige, bicyclische Heteroarylringe die ein, zwei oder drei Heteroatome, ausgewählt aus der Gruppe Sauerstoff. Schwefel und Stickstoff enthalten können und so viele konjugierte Doppelbindungen enthalten, dass ein aromatisches Systeme gebildet wird. Als Beispiele für fünf- oder sechsgliedrige heterocyclische Aromaten, werden genannt:

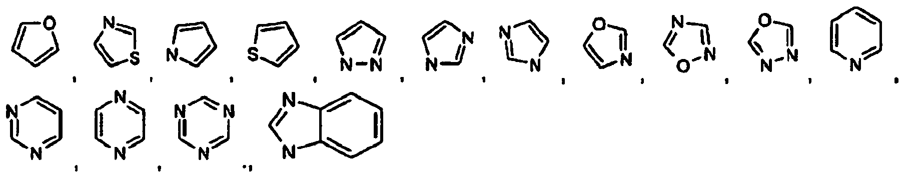

Soweit nicht anders beschreiben, können diese Heteroaryle substituiert sein mit einem oder mehreren Resten ausgewählt aus der Gruppe bestehend aus Methyl, Ethyl, *iso*-Propyl, *tert*-Butyl, Hydroxy, Fluor, Chlor, Brom und Jod.

Für die Heteroaryl-C₁₋₆-alkylene werden die folgenden Beispiele genannt:

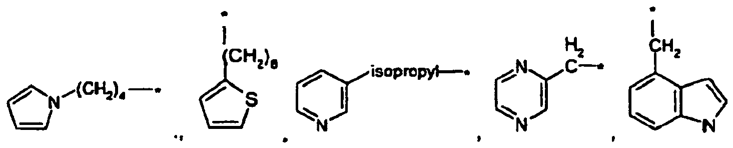

Unter dem Begriff "C₁₋₆-Haloalkyl" (auch soweit sie Bestandteil anderer Reste sind) werden verzweigte und unverzweigte Alkylgruppen mit 1 bis 6 Kohlenstoffatomen verstanden, die mit einem oder mehreren Halogenatomen substituiert sind. Unter dem Begriff "C₁₋₄-Alkyl" verzweigte und unverzweigte Alkylgruppen mit 1 bis 4 Kohlenstoffatomen verstanden, die mit einem oder mehreren Halogenatomen substituiert sind. Bevorzugt sind Alkylgruppen mit 1 bis 4 Kohlenstoffatome. Beispielsweise werden hierfür genannt: CF₃, CHF₂, CH₂F, CH₂CF₃.

Unter dem Begriff "C₃₋₇-Cycloalkyl" (auch soweit sie Bestandteil anderer Reste sind) werden cyclische Alkylgruppen mit.3 bis.7 Kohlenstoffatomen verstanden. Beispielsweise werden hierfür genannt: Cyclopropyl, Cyclobutyl, Cyclopentyl, Cyclohexyl oder Cycloheptyl. Soweit nicht anders beschrieben, können die cyclischen Alkylgruppen substituiert sein mit einem oder mehreren Resten ausgewählt aus der Gruppe bestehend aus Methyl, Ethyl, *iso*-Propyl, *tert*-Butyl, Hydroxy, Fluor, Chlor, Brom und Jod.

Unter dem Begriffe "C₃₋₁₀-Cycloalkyl" werden zudem monocyclische Alkylgruppen mit 3 bis 7 Kohlenstoffatomen und auch bicyclische Alkylgruppen mit 7 bis 10 Kohlenstoffatomen verstanden oder auch monocyclische Alkylgruppen, die durch mindestens eine C₁₋₃-Kohlenstoffbrücke überbrückt sind.

Unter dem Begriff "heterocyclische Ringe" oder auch "Heterocyclus" werden fünf-, sechs- oder siebengliedrige, gesättigte oder ungesättigte heterocyclische Ringe verstanden die ein, zwei oder drei Heteroatome, ausgewählt aus der Gruppe Sauerstoff, Schwefel und Stickstoff enthalten können, dabei kann der Ring über ein Kohlenstoffatom oder falls vorhanden über ein Stickstoffatom mit dem Molekül verknüpft sein. Obwohl unter dem Begriff "heterocyclische Ringe" oder "Heterocyclus" umfasst, definiert der Begriff "heterocyclische, nichtaromatische Ringe" fünf-, sechs- oder siebengliedrige ungesättigte Ringe. Als Beispiele werden genannt:

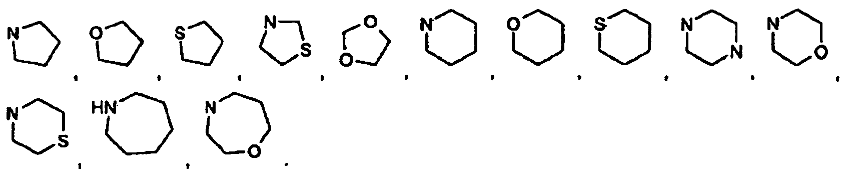

Obwohl unter dem Begriff "heterocyclische Ringe" oder "Heterocyclus" umfasst, definiert der Begriff "heterocyclische, aromatische Ringe" oder "Heteroaryl" fünf- oder sechsgliedrige heterocyclische Aromaten oder 5-10 gliedrige, bicyclische Heteroarylringe die ein, zwei, drei oder vier Heteroatome, ausgewählt aus der Gruppe Sauerstoff, Schwefel und Stickstoff, enthalten können und so viele konjugierte Doppelbindungen enthalten, dass ein aromatisches gebildet Systeme wird. Als Beispiele für fünf- oder sechsgliedrige heterocyclische Aromaten, werden genannt:

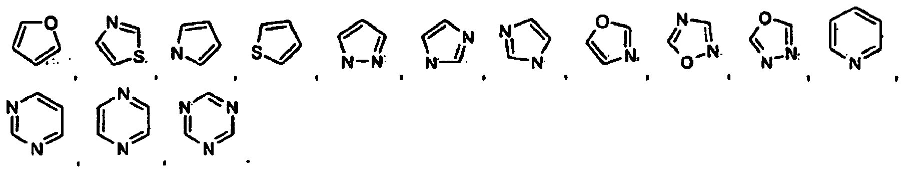

Soweit nicht anders erwährt, kann ein heterocyclischer Ring (oder "heterocyclus) mit einer Ketogruppe versehen sein. Als Beispiel hierfür werden genannt.

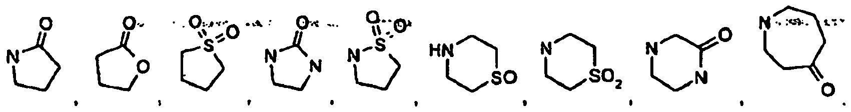

Obwohl unter "Cycloalkyl" bereits umfasst, werden unter dem Begriff "bicyclische Cycloalkyle" in der Regel acht-, neun- oder zehngliedrige, bicyclische Kohlenstoff-Ringe verstanden. Beispielhaft werden genannt:

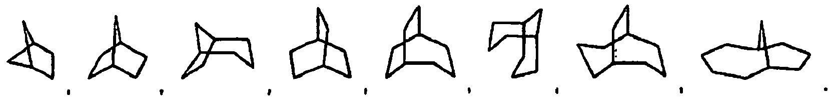

Obwohl unter "Heterocyclus" bereits umfasst, werden unter dem Begriff "bicyclische Heterocyclen" in der Regel acht-, neun- oder zehngliedrige, bicyclische Ringe verstanden, die ein oder mehrere Heteroatome, vorzugsweise 1-4, stärker bevorzugt, 1-3, noch stärker bevorzugt 1-2, insbesondere ein Heteroatom, ausgewählt aus der Gruppe Sauerstoff, Schwefel und Stickstoff enthalten können. Der Ring kann dabei über ein Kohlenstoffatom des Rings oder falls vorhanden über ein Stickstoffatom des Rings mit dem Molekül verknüpft sein. Beispielhaft werden genannt,

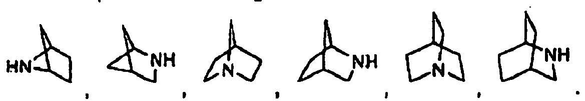

Obwohl unter "Aryl" bereits umfasst, wird unter einem "bicyclischen Aryl" ein 5.10 gliedriger, bicyclischer Arylring verstanden, der so viele konjugierte Doppelbindungen enthält, dass ein aromatisches System gebildet wird. Ein Beispiel für ein bicyclisches Aryl ist Naphthyl.

Obwohl unter "Heteroaryl" bereits umfasst, wird unter einem "bicyclischen Heteroaryl" ein 5-10 gliedriger, bicyclischer Heteroarylring verstanden, der ein, zwei, drei oder vier Heteroatome, ausgewählt aus der Gruppe Sauerstoff, Schwefel und Stickstoff, enthalten kann und so viele konjugierte Doppelbindungen enthält, dass ein aromatisches System gebildet wird.

Obwohl unter dem Begriff "bicyclische Cycloalkyle" oder "bicyclisches Aryl" umfasst, definiert der Begriff "kondensiertes Cycloalkyl" oder "kondensiertes Aryl" bicyclische Ringe, in denen die die Ringe trennende Brücke eine direkte Einfachbindung bedeutet. Als Beispiel für einen kondensiertes, bicyclisches Cycloalkyl werden genannt:

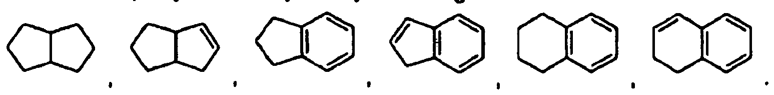

Obwohl unter dem Begriff "bicyclische Heterocyclen" oder "bicyclische Heteroaryle" umfasst, definiert der Begriff "kondensierte, bicyclische Heterocyclen "oder "kondensierte, bicyclische Heteroaryle"bicyclische 5-10 gliedrige Heteroringe die ein, zwei oder drei Heteroatome, ausgewählt aus der Gruppe Sauerstoff, Schwefel und Stickstoff enthalten und in denen die die Ringe trennende Brücke eine direkte Einfachbindung bedeutet. Die "kondensierten, bicyclischen Heteroaryle" enthalten zudem so viele konjugierte Doppelbindungen, dass ein aromatisches System gebildet wird. Beispielhaft werden genannt Pyrrolizin, Indol, Indolizin, Isoindol, Indazol, Purin, Chinolin, Isochinolin, Benzimidazol, Benzofuran, Benzopyran, Benzothiazol, Benzathiazol. Benzoisothiazol, Pyridopyrimidin, Pteridin, Pyrimidopyrimidin,

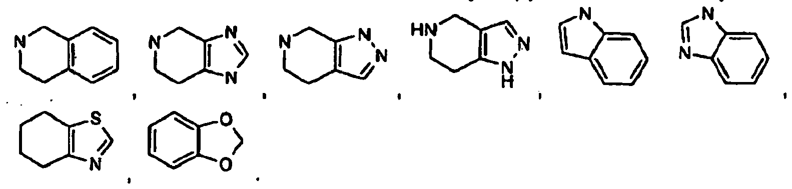

Unter dem Begriff "heterocyclische Spiroringe" (Spiro) werden 5-10 gliedrige, spirocyclische Ringe verstanden die gegebenenfalls ein, zwei oder drei Heteroatome, ausgewählt aus der Gruppe Sauerstoff, Schwefel und Stickstoff enthalten können, dabei kann der Ring über ein Kohlenstoffatom oder falls vorhanden über ein Stickstoffatom mit dem Molekül verknüpft sein. Soweit nicht anders erwähnt, kann ein spirocyclischer Ring mit einer Oxo-, Methyl- oder Ethylgruppe versehen sein. Als Beispiele hierfür werden genannt:

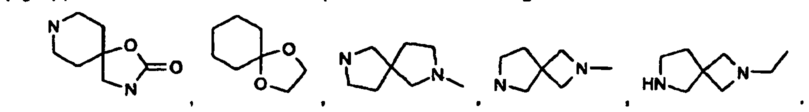

"Halogen" steht im Rahmen der vorliegenden Erfindung für Fluor, Chlor, Brom oder Jod. Sofern nicht gegenteilig angegeben, gelten Fluor, Chlor und Brom als bevorzugte Halogene.

Verbindungen der allgemeinen Formel 1 können Säuregruppen besitzen, hauptsächlich Carboxylgruppen, und/oder basische Gruppen wie z.B. Aminofunktionen. Verbindungen der allgemeinen Formel 1 können deshalb als innere Salze, als Salze mit pharmazeutisch verwendbaren anorganischen Säuren wie Salzsäure, Schwefelsäure, Phosphorsäure, Sulfonsäure oder organischen Säuren (wie beispielsweise Maleinsäure, Fumarsäure. Zitronensäure, Weinsäure oder Essigsäure) oder als Salze mit pharmazeutisch verwendbaren Basen wie Alkali- oder Erdalkalimetallhydroxiden oder Carbonaten, Zink- oder Ammoniumhydroxiden oder organischen Aminen wie z.B. Diethylamin, Triethylamin, Triethanolamin u.a. vorliegen.

Wie vorstehend genannt, können die Verbindungen der Formel **1** in ihre Salze, insbesondere für die pharmazeutische Anwendung, in ihre physiologisch und pharmakologisch verträglichen Salze überführt werden. Diese Salze können einerseits als physiologisch und pharmakologisch verträgliche Säureadditionssalze der Verbindungen der Formel **1** mit anorganischen oder organischen Säuren vorliegen. Andererseits kann die Verbindung der Formel **1** im Falle von R gleich Wasserstoff durch Umsetzung mit anorganischen Basen auch in physiologisch und pharmakologisch verträgliche Salze mit Alkali- oder Erdalkalimetallkationen als Gegenion überführt werden. Zur Darstellung der Säureadditionssalze kommen beispielsweise Salzsäure, Bromwasserstoffsäure, Schwefelsäure, Phosphorsäure, Methansulfonsäure, Essigsäure, Fumarsäure, Bernsteinsäure, Milchsäure, Zitronensäure, Weinsäure oder Maleinsäure in Betracht. Ferner können Mischungen der vorgenannten Säuren eingesetzt werden. Zur Darstellung der Alkali- und Erdalkalimetallsalze der Verbindung der Formel **1** in der R Wasserstoff bedeutet, kommen vorzugsweise die Alkali- und Erdalkalihydroxide und -hydride in Betracht, wobei die Hydroxide und Hydride der Alkalimetalle, besonders des Natriums und Kaliums bevorzugt, Natrium- und Kaliumhydroxid besonders bevorzugt sind.

Gegebenenfalls können die Verbindungen der allgemeinen Formel (1) in ihre Salze, insbesondere für die pharmazeutische Anwendung, in ihre pharmakologisch unbedenklichen Säureadditionssalze mit einer anorganischen oder organischen Säure, überführt werden. Als Säuren kommen hierfür beispielsweise Bernsteinsäure, Bromwasserstoffsäure, Essigsäure, Fumarsäure, Maleinsäure, Methansulfonsäure, Milchsäure, Phosphorsäure, Salzsäure, Schwefelsäure, Weinsäure oder Zitronensäure in Betracht. Ferner können Mischungen der vorgenannten Säuren eingesetzt werden.

Gegenstand der Erfindung sind die jeweiligen Verbindungen gegebenenfalls in Form der einzelnen optischen Isomeren, Mischungen der einzelnen Enantiomeren oder Racemate, in Form der Tautomere sowie in Form der freien Basen oder der entsprechenden Säureadditionssaize mit pharmakologisch unbedenklichen Säuren - wie beispielsweise Säureadditionssalze mit Halogenwasserstoffsäuren - beispielsweise Chlor oder Bromwasserstoffsäure - oder organische Säuren.- wie beispielsweise Oxal-, Fumar-, Diglycol- oder Methansulfonsäure.

Die erfindungsgemäßen Verbindungen können gegebenenfalls als Racemate vorliegen, sie können aber auch als reine Enantiomere, d.h. in (R)- oder (S)-Form gewonnen werden.

Gegenstand der Erfindung sind die jeweiligen Verbindungen, gegebenenfalls in Form der einzelnen optischen Isomeren, Mischungen der einzelnen Enantiomeren oder Racemate, in Form der Tautomere sowie in Form der freien Basen oder der entsprechenden Säureadditionssalze mit pharmakologisch unbedenklichen Säuren - wie beispielsweise Säureadditionssalze mit Halogenwasserstoffsäuren - beispielsweise Chlor- oder Bromwasserstoffsäure - oder organische Säuren - wie beispielsweise Oxal-, Fumar-, Diglycol- oder Methansulfonsäure.

Gegenstand der Erfindung sind die jeweiligen Verbindungen der Formel **1** in Form ihrer pharmakologisch verträglichen Salze wie vorstehend beschrieben. Diese pharmakologisch verträglichen Salze der Verbindungen der Formel **1** können ebenfalls in Form ihrer jeweiligen Hydrate (z.B. Monohydrate, Dihydrate etc.) sowie in Form Ihrer jeweiligen Solvate vorliegen. Unter einem Hydrat der Verbindung nach Formel **1** versteht man im Rahmen der Erfindung ein kristallines, kristallwasserhaltiges Salz der Verbindung nach Formel **1**.
Unter einem Solvat der Verbindung nach Formel **1** versteht man im Rahmen der Erfindung ein kristallines Salz der Verbindung nach Formel **1**, welches Lösungsmittelmoleküle-Moleküle (z.B. Ethanol, Methanol etc) im Kristallgitter enthalten.
Dem Fachmann sind Standardverfahren zur Gewinnung von Hydraten und Solvaten (z.B. das Umkristallisieren aus dem entsprechenden Lösungsmittel bei Solvaten bzw. aus Wasser bei Hydraten) bekannt.

### SYNTHESEVORSCHRIFTEN

Die Verbindungen der allgemeinen Formel (I) können nach folgendem allgemeinen Syntheseschema hergestellt werden, wobei die Substituenten der allgemeinen Formel (I) die zuvor genannten Bedeutungen haben. Diese Verfahren sind als Erläuterung der Erfindung zu verstehen ohne selbige auf deren Gegenstand zu beschränken.

### ALLGEMEINE SYNTHESE SCHEMA

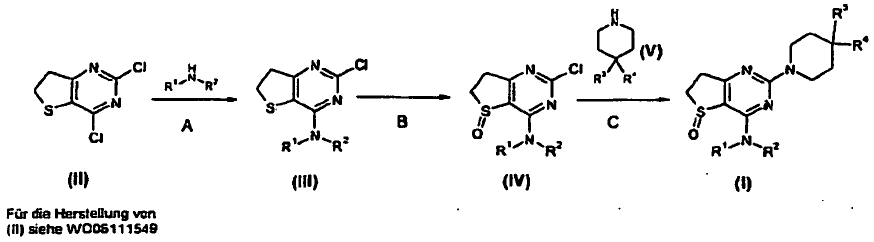

### 1. SYNTHESE VON (R)-2-{2-[4-(4-CHLORPHENYL)-PIPERIDIN-1-YL]-5-OXO-6,7-DIHYDRO-5H-5λ⁴-THIENO[3,2-d]PYRIMIDIN -4-YLAMIN}-3-METHYLBUTAN-1-OL (BEISPIEL 1)

### 1.1 (R)-2-(2-Chlor-6,7-dihydrothieno[3,2-d]pyrimidin-4-ylamino)-3-methylbutan-1-ol (III-1):

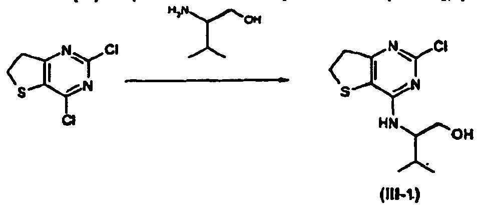

7,2 g 2,4-Dichlor-6,7-dihydrothieno[3,2-*d*]pyrimidin **(II)** werden in 36 ml Dioxan vorgelegt, erst werden 18 ml Diisopropylethylamin, dann 6,1 g (R)-(-)-2-Amino-3-methyl-1-butanol zugegeben. Das Reaktionsgemisch wird bei 1°00°C, bis keine weitere Umsetzung erfolgt, erhitzt und nach Abkühlen eingedampft. Der Rückstand wird mit Petrolether/Essigester (9:1) im Ultraschallbad behandelt und der Feststoff abgesaugt und getrocknet. 8,3 g **(III-1)** werden als Feststoff erhalten. Anatytische HPLG (Methode A): RT =2,75 min

### 1.2 (R)-2-(2-Chlor-5-oxo-6,7-dihydro-5H-5λ-thieno[3,2-d]pyrimidin-4-ylamino)-3-methylbutan-1-ol (IV-1):

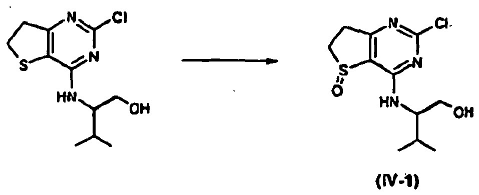

4,1 g S-(-)-1,1'-Bi-2-naphtol werden in 15 ml Chloroform unter Argon vorgelegt, dann 0,44 ml Titan(IV)-isopropylat und 0,54 ml Wasser zugegeben. Das Reaktionsgemisch wird 1 Stunde bei Raumtemperatur gerührt. Anschlieflend wird eine Suspension von 4,1 g (III-1) in 107 ml Dichlormethan zugegeben. Das Reaktionsgernisch wird auf -2°C abgekühlt und nach 30 Minuten werden 2,7 ml tert-Butylhydroperoxid 5-6 M in Decan zugetropft. Das Reaktionsgemisch wird bei -2 °C, bis keine weitere Umsetzung erfolgt, weitergerührt und mit NH₄OH basisch gestellt. Das Produkt wird mit Dichlormethan extrahiert und chromatographisch (Kieselgel, Essigester/Methanol 10010 bis 86114) gereinigt. 2,45 g (IV-1) werden als Feststoff erhalten. Analytische HPLC (Methode A): RT = 2,37 min

### 1.3 (R)-2-{2-[4-(4-Chlorphenyl)-piperidin-1-yl]-5-oxo-6,7-dihydro-5H-5λ⁴-thieno[3,2-d]pyrimidin -4-ylamino}-3-methylbutan-1-ol (Beispiel 1)

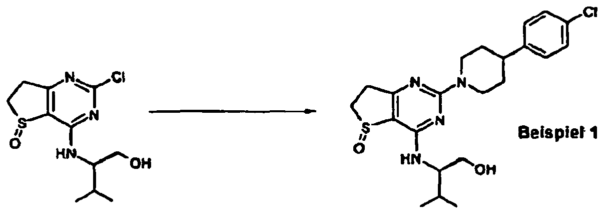

0,2 g (IV-1) wird in 3 ml Dioxan und 360 µl Diisopropylethylamin vorgelegt, mit 0,16 g 4-(4-Chlorphenyl)-piperidin versetzt und bei 120°C, bis keine weitere Umsetzung erfolgt, in der Mikrowelle erhitzt. Das Reaktionsgemisch wird mit Wasser versetzt, mit Dichlormethan extrahiert und das Produkt chromatographisch (Kieselgel, Dichlormethan/Methanol 100/0 bis 92/8) gereinigt. 0,33 g Beispiel 1 werden als Feststoff erhalten. Analytische HPLC-MS (Methode A): RT = 1,24 min.

### 2. SYNTHESE VON (1-{2-[4-(4-CHLORPHENYL)-PIPERIDIN-1-YL]-5-OXO-6,7-DIHYDRO-5H-5λ⁴-THIENO[3,2-d]PYRIMIDIN-4-YLAMINO}-CYCLOPROPYL)-METHANOL (BEISPIEL 2)

### 2.1 (1-Hydroxymethylcyclopropyl)-carbamidsäure tert-butylester:

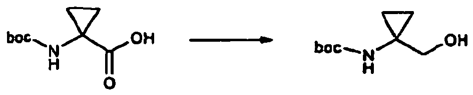

1 g 1-(BOC-amino)-cyclopropancarbonsäure wird in 20 ml Dimethoxyethan gelöst und auf - 70°C abgekühlt. Dann werden 0,65 ml N-Methylmorpholin zugegeben und 0,71 ml Isobutylchloroformiat in 5 ml Dimethoxyethan zugetropft. Das Reaktionsgemisch wird auf - 5°C erwärmt. Der Niederschlag wird abgesaugt. Das Eluat wird auf -15°C abgekühlt und 0,303 g Natriumborhydrid werden langsam zugegeben. Das Reaktionsgemisch wird anschliessend 30 Minuten bei Raumtemperatur gerührt, mit Wasser versetzt und das Produkt mit Dichlormethan extrahiert. Die organische Phase wird getrocknet und zur Trockne eingedampft. 1,04 g Produkt werden als Feststoff erhalten. ¹H NMR (400 MHz, DMSO): 1,36 (9H, s); 0,61 (2H, t); 0,52 (2H, t).

### 2.2 1-Aminocyclopropanmethanol:

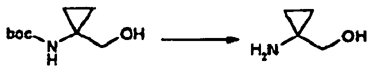

1,04 g (1-Hydroxymethylcyclopropyl)-carbamidsäure tert-butylester werden in 5 ml Dioxan vorgelegt. 2,5 ml HCl in Dioxan (4 mol/l) werden zugetropft. Das Reaktionsgemisch wird bei Raumtemperatur 15 h gerührt. Das Lösungsmittel wird zur Hälfte eingedampft und der ausgefallene Feststoff abgesaugt. 0,5 g Produkt werden als Hydrochlorid erhalten. ¹H NMR (400 MHz, DMSO): 5,27 (1H, t); 0,91 (2H, t); 0,71 (2H, t).

### 2.3 [1-(2-Chlor-6,7-dihydrothieno[3,2-d]pyrimidin-4-ylamino)-cyclopropyl]-methanol (III-2):

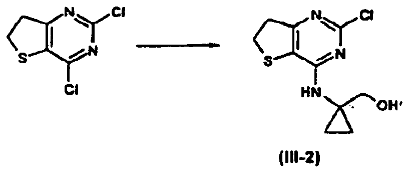

1,4 g (II) werden in 10 ml Dioxan vorgelegt, anschliessend werden 3,6 ml Diisopropylethylamin und dann 1 g 1-Aminocyclopropanmethanol (siehe 2.2) zugegeben. Das Reaktionsgemisch wird bei 160°C, bis keine weitere Umsetzung erfolgt, erhitzt und nach Abkühlen eingedampft. Der Rückstand wird mit Cyclohexan/Essigester (4:1) im Ultraschallbad behandelt, der Feststoff abgesaugt und getrocknet. 1,24 g (III-2) werden als Feststoff erhalten. Analytische HPLC-MS (Methode A): RT = 1,01 min.

### 2.4 [1-(2-Chlor-5-oxo-6,7-dihydro-5H-5λ⁴-thieno[3,2-d]pyrimidin-4-ylamino)-cyclopropyl]-methanol (IV-2):

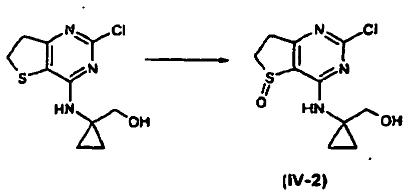

0,28 g S-(-)-1,1'-Bi-2-naphtol werden in 20 ml Chloroform unter Argon vorgelegt, dann 0,14 ml Titan(IV)-isopropylat und 0,17 ml Wasser zugegeben. Das Reaktionsgemisch wird 1 Stunde bei Raumtemperatur gerührt. Anschließend wird eine Suspension von 1,2 g (III-2) in 40 ml Dichlormethan und 2 ml Methanol zugegeben. Das Reaktionsgemisch wird auf -5°C abgekühlt und nach 30 Minuten werden 0,91 ml tert-Butylhydroperoxid 5-6 M in Decan zugetropft. Das Reaktionsgemisch wird bei -5 °C, bis keine weitere Umsetzung erfolgt, weitergerührt und mit NH₄OH basisch gestellt. Die Wasserphase wird mit Dichlormethan gewaschen und gefriergetrocknet. 1 g (IV-2) wird als Feststoff erhalten. Analytische HPLC-MS (Methode A) RT = 0,85 min

### 2.5 (1-{2-[4-(4-Chlorphenyl)-piperidin-1-yl]-5-oxo-6,7-dihydro-5H-5λ⁴-thieno[3,2-d]pyrimidin-4-ylamino}-cyclopropyl)-methanol (Beispiel 2)

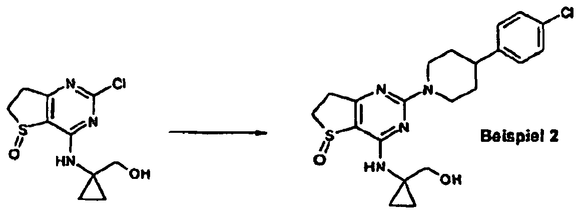

Ausgehend von 0,17 g (IV-2) und 0,15 g 4-(4-Chlorphenyl)-piperidin werden 0,14 g Beispiel 2 analog zu Beispiel 1 (siehe 1.3) hergestellt und aufgereinigt. Analytische HPLC-MS (Methode B): RT = 1,32 min.

### 3. SYNTHESE VON (R)-2-{2-[4-(4-CHLORPHENYL)-PIPERIDIN-1-YL]-5-OXO-6,7-DIHYDRO-5H-5λ⁴-THIENO[3,2-d]PYRIMIDIN-4-YLAMINO}-PENTAN-1-OL (BEISPIEL 3)

### 3.1 (R)-2-(2-Chlor-6,7-dihydrothieno[3,2-d]pyrimidin-4-ylamino)-pentan-1-ol (III-3):

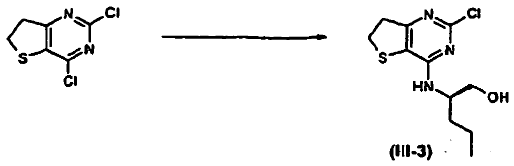

1,4 g 2,4-Dichlor-6,7-dihydrothieno[3,2-*d*]pyrimidin (II) werden in 9 ml Dioxan vorgelegt, erst werden 3,5 ml Diisopropylethylamin, dann 0,9 g D-norvalinol zugegeben. Das Reaktionsgemisch wird bei 120°C, bis keine weitere Umsetzung erfolgt, in der Mikrowelle erhitzt und nach Abkühlen eingedampft. Der Rückstand wird mit Petrolether/Essigester 9:1 im Ultraschallbad behandelt, der Feststoff wird abgesaugt und getrocknet 1,5 g (III-3) werden als Feststoff erhalten. ¹H NMR (400 MHz, DMSO): 4,67 (1H, t); 0,86 (3H, t).

### 3.2 (R)-2-(2-Chlor-5-oxo-6,7-dihydro-5H-5λ⁴-thieno[3,2-d]pyrimidin-4-ylamino)-pentan-1-ol (IV-3):

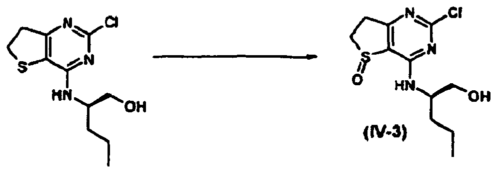

0,3 g S-(-)-1,1'-Bi-2-naphtol werden in 5 ml Chloroform unter Argon vorgelegt, dann 0,15 ml Titan(IV)-isopropylat und 0,19 ml Wasser zugegeben. Das Reaktionsgemisch wird 1 Stunde bei Raumtemperatur gerührt. Anschließend wird eine Suspension von 1,4 g (III-3) in 20 ml Dichlormethan zugegeben. Das Reaktionsgemisch wird auf -5°C abgekühlt und nach 30 Minuten werden 0.95 ml tert-Butylhydroperoxid 5-6 M in Decan zugetropft. Das Reaktionsgemisch wird bei -5 °C, bis keine weitere Umsetzung erfolgt, weitergerührt und mit NH₄OH basisch gestellt. Das Produkt wird mit Dichlormethan extrahiert und chromatographisch (Essigester/Methanol 100/0 bis 80/20) gereinigt. 1,17 g (IV-3) werden als Feststoff erhalten. Analytische HPLC (Methode A): RT = 2,41 min

### 3.3 (R)-2-{2-[4-(4-Chlorphenyl)-piperidin-1-yl]-5-oxo-6,7-dihydro-5H-5λ⁴-thieno[3,2-d]pyrimidin-4-ylamino}-pentan-1-ol (Beispiel 3)

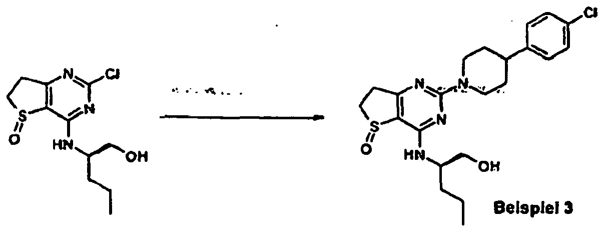

0,2 g (IV-3) werden in 4 ml Dioxan und 237 µl Diisopropylethylamin vorgelegt, mit 0,149 g 4-(4-Chlorphenyl)-piperidin versetzt und 30 min bei 130°C in der Mikrowelle erhitzt. Das Reaktionsgemisch wird mit Wasser versetzt und das Produkt mit Dichlormethan extrahiert. Der Rückstand wird mit Acetonitril im Ultraschallbad behandelt und der Feststoff abgesaugt. 0,104 g **Beispiel 3** werden als Feststoff erhalten. Analytische HPLC-MS (Methode A): RT = 1,29 min.

### 4. SYNTHESE VON (R)-1-{2-[4-(4-CHLORPHENYL)-PIPERIDIN-1-YL]-5-OXO-6,7-DIHYDRO-5H-5λ⁴-THIENO[3,2-d]PYRIMIDIN-4-YLAMINO}-1-(4-FLUORPHENYL)-2-METHYLPROPAN-2-OL (BEISPIEL 4)

### 4.1 (R)-Amino-(4-fluorphenyl)-essigsäuremethylester

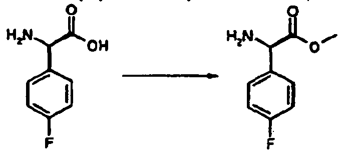

4 g (R)-4-fluorphenylglycin werden in 80 ml Methanol suspendiert. Unter Eisbadkühlung werden 3,28 ml Thionylchlorid langsam zugetropft, so dass die Temperatur zwischen 15°C und 20°C gehalten wird. Das Reaktionsgemisch wird 12 Stunden bei Raumtemperatur gerührt und anschließend zur Trockne eingedampft. 5,1 g des Produkts werden als Hydrochlorid erhalten. Analytische HPLC-MS (Methode A): RT = 0,8 min.

### 4.2 (R)-(4-Fluorphenyl)-(2,2,2-trifluoracetylamino)-essigsäuremethylester:

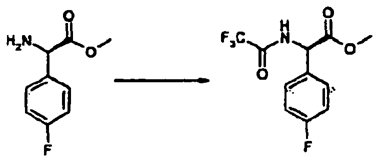

5,1 g (R)-Amino-(4-fluorphenyl)-essigsäuremethylester werden in 36.5 ml abs. Tetrahydrofuran vorgelegt, dann 3,9 ml Triethylamin zugegeben. Das Reaktionsgemisch wird auf -70°C abgekühlt. 3,9 ml Trifluoresslganhydrid werden dann langsam zugetropft, so dass die Temperatur nicht -60°C übersteigt. Das Reaktionsgemisch wird 12 Stunden bei Raumtemperatur gerührt und anschließend mit Wasser versetzt. Dann wird. Kaliumhydrogencarbonat, bis keine Schaumbildung mehr zu beobachten ist, zugegeben und das Produkt mit Essigester extrahiert. 6,2 g des Produkts werden als Öl erhalten. Analytische HPLC-MS (Methode A): RT = 1,28 min.

### 4.3 2,2,2-Trifluor-N-[(R)-1-(4-fluorphenyl)-2-hydroxy-2-methylpropyl]-acetamid:

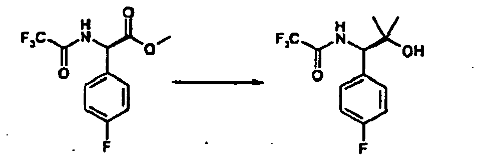

6,2 g (R)-(4-Fluorphenyl)-(2,2,2-trifluoracetylamino)-essigsäuremethylester werden in 195 ml abs. Tetrahydrofuran vorgelegt und das Reaktionsgemisch auf +3°C gekühlt. 37,2 ml einer Methylmagnesiumiodid Lösung (3 M) werden langsam zugetropft, so dass die Temperatur nicht +10°C übersteigt. Das Reaktionsgemisch wird 12 Stunden bei Raumtemperatur gerührt und anschließend in Eiswasser gerührt. Ammoniumchlorid wird, bis der Niederschlag gelöst ist, zugegeben und das Produkt mit Essigester extrahiert. 5,6 g des Produkts werden als Öl erhalten. Analytische HPLC-MS (Methode A): RT = 1,19 min

### 4.4 (R)-1-Amino-1-(4-fluorphenyl)-2-methylpropan-2-ol:

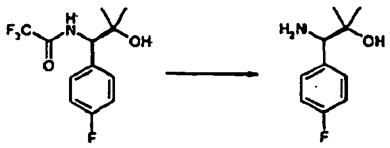

5,6 g 2,2,2-Trifluor-*N*-[(R)-1-(4-fluorphenyl)-2-hydroxy-2-methylpropyl]-acetamid und 2,27 g KOH werden in 60 ml Methanol suspendiert. Das Reaktionsgemisch wird 20 Stunden bei 60°C gerührt, anschließend mit Wasser versetzt und das Produkt mit Dichlormethan extrahiert. 3,2 g Produkt werden als Öl erhalten. Analytische HPLC-MS (Methode A): RT = 0,79 min.

### 4.5 (R)-1-(2-Chlor-6,7-dihydrothieno[3,2-d]pyrimidin-4-ylamino)-1-(4-fluorphenyl)-2-methylpropan-2-ol (III-4):

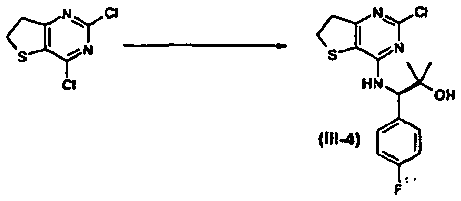

0.533 g (II), 0,850 g (R)-1-Amino-1-(4-fluorphenyl)-2-methylpropan-2-ol und 1,3 ml Diisopropylethylamin werden in 9,8 ml Dioxan suspendiert. Das Reaktionsgemisch wird in der Mikrowelle 2 Stunden bei 80°C erhitzt und anschießend zur Trockne eingedampft. Der Rückstand wird mit Wasser versetzt. Der ausgefallene Niederschlag wird abgesaugt und chromatographisch (Kieselgel, Petrolether/Essigester 100/0 bis 60/40) gereinigt. 0,260 g (III-4) werden als Feststoff erhalten. Analytische HPLC-MS (Methode A): 1,39 min.

### 4.6 (R)-1-(2-Chlor-5-oxo-6,7-dihydro-5H-5λ⁴-thieno[3,2-d]pyrimidin-4-ylamino)-1-(4-fluorphenyl)-2-methylpropan-2-ol (IV-4):

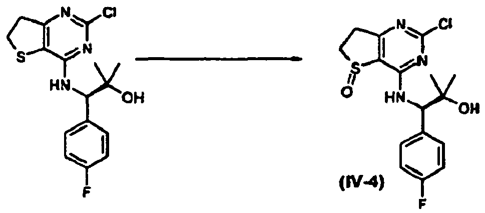

0,24 g S-(-)-1,1'-Bi-2-naphtol werden in 4 ml Chloroform unter Argon vorgelegt, dann 0,125 ml Titan(IV)-isopropylat und 0,15 ml Wasser zugegeben. Das Reaktionsgemisch wird 1 Stunde bei Raumtemperatur gerührt. Anschließend wird eine Suspension von 1,51 g (III-4) in 26 ml Chloroform zugegeben. Das Reaktionsgemisch wird auf -6°C abgekühlt und nach 30 Minuten werden 0,78 ml tert-Butylhydroperoxid 5-6 M in Decan zugetropft. Das Reaktionsgemisch wird bei -6 °C, bis keine weitere Umsetzung erfolgt, weitergerührt und mit NH₄OH basisch gestellt. Das Produkt wird mit Dichlormethan extrahiert und chromatographisch (Dichlormethan/Methanol 100/0 bis 95/5) gereinigt. 0,62 g (IV-4) werden als Feststoff erhalten. Analytische HPLC-MS (Methode A): RT = 1,19 min.

### 4.7 (R)-1-{2-[4-(4-Chlorphenyl)-piperidin-1-yl]-5-oxo-6,7-dihydro-5H-5λ⁴-thieno[3,2-d]pyrimidin-4-ylamino}-1-(4-fluorphenyl)-2-methylpropan-2-ol (Beispiel 4)

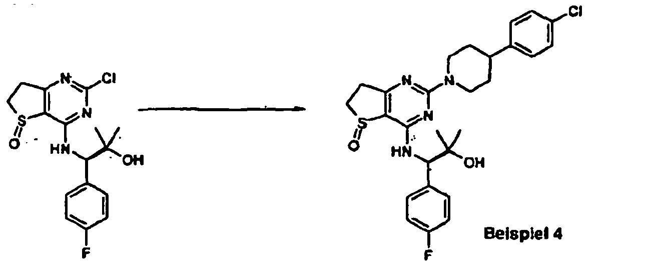

Ausgehend von 0,24 g (IV-4) und 0,15 g 4-(4-Chlorphenyl)-piperidin werden 0,19 g Beispiel 4 analog zu Beispiel 1 (siehe 1.3) hergestellt. Das Produkt wird chromatographisch (Dichlormethan/Methanol 100/0 bis 96/4) gereinigt. Analytische HPLC-MS (Methode A): RT = 1,36 min.

### 5. SYNTHESE VON (S)-5-{2-[4-(4-CHLORPHENYL)-PIPERIDIN-1-YL)-5-OXO-6,7-DIHYDRO-5H-5λ⁴-THIENO[3,2-d]PYRIMIDIN-4-YLAMINO}-1-METHYLPIPERIDIN-2-ON (BEISPIEL 5)

### 5.1 (S)-5-Dibenzylaminopiperidin-2-on:

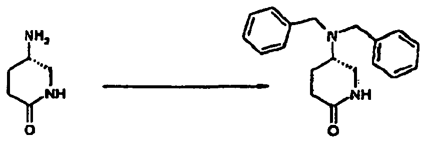

0,600 g 4-(S)-Amino-delta-valerolactam Hydrochlorid. 0,970 ml Benzylbromid und 1,5 g Natriumhydrogencarbonat werden in 30 ml Ethanol suspendiert. Das Reaktionsgemisch wird dann 8 Stunden bei 80°C gerührt und anschließend zur Trockne eingedampft. Der Rückstand wird in Wasser suspendiert und das Produkt mit Dichlormethan extrahiert und chromatographisch (Kieselgel, Dichlormethan/Methanol 100/0 bis 95/5) gereinigt. 0,500 g Produkt werden als Öl erhalten. Analytische HPLC-MS (Methode A): RT = 1,01 min.

### 5.2 (S)-5-Dibenzylamino-1-methylpiperidin-2-on:

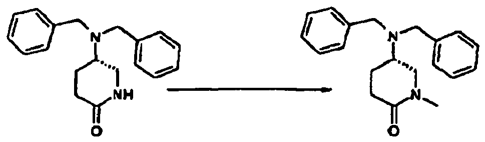

0,500 g (S)-5-Dibenzylaminopiperidin-2-on werden in 15 ml Tetrahydrofuran suspendiert. Unter Eisbadkühlung werden 0,175 g Kalium-*tert*-butylat zugegeben. Das Reaktionsgemisch wird dann 30 Minuten bei Raumtemperatur gerührt. Unter Eisbadkühlung werden 0,095 ml Methyliodid zugegeben. Das Reaktionsgemisch wird dann 48 Stunden bei Raumtemperatur gerührt und anschließend mit einer gesättigten NaCl Lösung versetzt. Das Produkt wird mit Essigester extrahiert. 0,450 g Produkt werden als Öl erhalten. Analytische HPLC-MS (Methode A): RT = 1,07 min.

### 5.3 (S)-5-Amino-1-methylpiperidin-2-on:

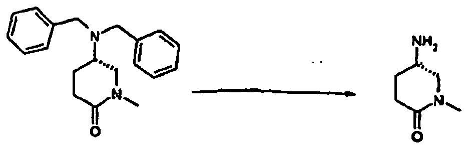

0.450 g (S)-5-Dibenzylamino-1-methylpiperidin-2-on werden in 25 ml Methanol suspendiert und mit 0.150 g Pd/C 10% bei einem Druck von 3 bar und einer Temperatur von 60 °C hydriert. Nach 16 Stunden wird der Katalysator abgesaugt und das Filtrat zur Trockne eingedampft. 0,190 g des Produktes werden als Öl erhalten. ¹H NMR (400 MHz, DMSO): 2,76 (3H, s).

### 5.4 (S)-5-(2-Chlor-6,7-dihydrothieno[3,2-d]pyrimidin-4-ylamino)-1-methylpiperidin-2-on (III-5):

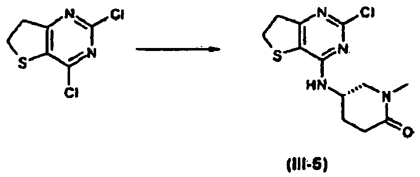

0,27 g (II) werden in 3 ml Dioxan vorgelegt, erst werden 0,45 ml Diisopropylethylamin, dann 0,25 g (S)-5-Amino-1-methylpiperidin-2-on zugegeben. Das Reaktionsgemisch wird bei 130°C, bis keine weitere Umsetzung erfolgt, erhitzt und nach Abkühlen eingedampft. Das Produkt wird mit Dichlormethan extrahiert und chromatographisch (präparative HPLC, Methode A) gereinigt. 0,26 g (III-5) werden als Feststoff erhalten. Analytische HPLC-MS (Methode A): RT = 1,06 min.

### 5.5 (S)-5-(2-Chlor-5-oxo-6,7-dihydro-5H-5λ⁴-thieno[3,2-d]pyrimidin-4-ylamino)-1-methylpiperidin-2-on (IV-5):

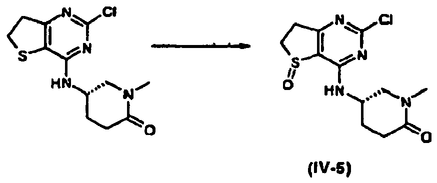

0,04 g S-(-)-1,1'-Bi-2-naphtol werden in 5 ml Chloroform unter Argon vorgelegt, dann 0,02 ml Titan(IV)-isopropylat und 0,025 ml Wasser zugegeben. Das Reaktionsgemisch wird 1 Stunde bei Raumtemperatur gerührt. Anschließend wird eine Suspension von 0,2 g (III-5) in 4 ml Dichlormethan zugegeben. Das Reaktionsgemisch wird auf -5°C abgekühlt und nach 20 Minuten werden 0,12 ml tert-Butylhydroperoxid 5-6 M in Decan zugetropft. Das Reaktionsgemisch wird bei -5 °C, bis keine weitere Umsetzung erfolgt, weitergerührt und mit NH₄OH basisch gestellt. Das Produkt wird chromatographisch (Kieselgel, Essigester/Methanol 100/0 bis 60/40) gereinigt. 0,09 g (IV-5) werden als Feststoff erhalten. Analytische HPLC-MS (Methode A): RT = 0,83 min.

### 5.6 (S)-5-{2-[4-(4-Chlorphenyl)-piperidin-1-yl]-5-oxo-6,7-dihydro-5H-5λ⁴-thieno[3,2-d]pyrimidin-4-ylamino}-1-methylpiperidin-2-on (Beispiel 5)

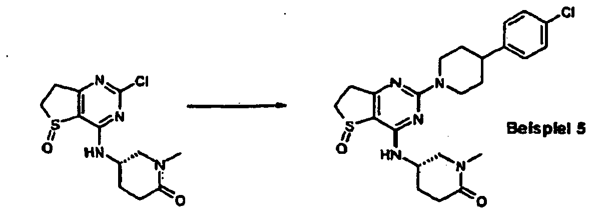

Ausgehend von 0,2 g (IV-5) und 0,18 g 4-(4Chlorphenyl)-piperidin werden 0,17 g Beispiel 5 analog zu Beispiel 1 (siehe 1.3) hergestellt. Das Produkt wird chromatographisch (präparative HPLC, Methode A) gereinigt. Die Produktfraktionen werden mit Ammoniak basisch gestellt und gefriergetrocknet. Analytische HPLC-MS (Methode A): RT = 1,18 min

### 6. SYNTHESE VON {2-[4-(4-CHLORPHENYL)-PIPERIDIN-1-YL]-5-OXO-6,7-DIHYDRO-5H-5λ⁴-THIENO[3,2-d]PYRIMIDIN-4-YL)-(TETRAHYDROPYRAN-4-YL)-AMIN (BEISPIEL 6)

### 6.1 (2-Chlor-6,7-dihydrothieno[3,2-d]pyrimimn-4-yl)-(tetrahydropyran-4-yl)-amin (III-6):

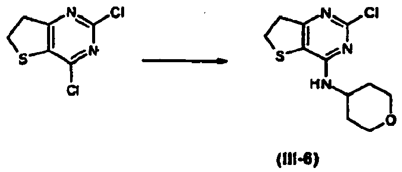

0,68 g (II) werden in 6 ml Dioxan vorgelegt, erst werden 1,72 ml Diisopropylethylamin, dann 0,6 g 4-Aminotetrahydropyran zugegeben. Das Reaktionsgemisch wird bei 130°C, bis keine weitere Umsetzung erfolgt, erhitzt und nach Abkühlen eingedampft. Das Produkt wird mit Wasser im Ultraschallbad behandelt, dann abgesaugt und getrocknet. 0,66 g (III-6) werden als Feststoff erhalten. Analytische HPLC-MS (Methode C): RT = 1,08 min.

### 6.2 (2-Chlor-5-oxo-6,7-dihydro-5H-5λ⁴-thieno[3,2-d]pyrimidin-4-yl)-(tetrahydropyran-4-yl)-amin (IV-6):

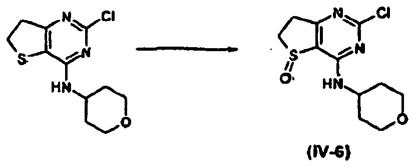

0,14 g S-(-)-1,1'-Bi-2-naphtol werden in 5 ml Chloroform unter Argon vorgelegt, dann 0,072 ml Titan(IV)-isopropylat und 0,087 ml Wasser zugegeben. Das Reaktionsgemisch wird 45 Minuten bei Raumtemperatur gerührt. Anschließend wird eine Suspension von 0,66 g (III-6) in 25 ml Chloroform zugegeben. Das Reaktionsgemisch wird auf -10°C abgefühlt und nach 60 Minuten werden 0,444 ml tert-Butylhydroperoxid 5-6 M in Decan zugetropft. Das Reaktionsgemisch wird bei -10 bis -4°C, bis keine weitere Umsetzung erfolgt, weitergerührt und mit Wasser versetzt. Das Produkt wird mit Dichlormethan extrahiert und chromatographisch (Kieselgel, Essigester/Methanol 100/0 bis 80/20) gereinigt. 0,42 g (IV-6) werden als Feststoff erhalten. Analytische HPLC-MS (Methode A): RT = 0,94 min.

### 6.3 {2-[4-(4-Chlorphenyl)-piperidin-1-yl]-5-oxo-6,7-dihydro-5H-5λ⁴-thieno[3,2-d]pyrimidin-4-yl}-(tetrahydropyran-4-yl)-amin (Beispiel 6)

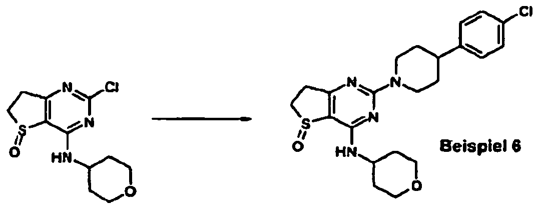

Ausgehend von 0,18 g (IV-6) und 0,17 g 4-(4-Chlorphenyl)-piperidin werden 0,23 g Beispiel 6 analog zu Beispiel 1 (siehe 1.3) hergestellt. Das Produkt wird mit Wasser im Ultraschallbad behandelt und der Feststoff abgesaugt. Analytische HPLC-MS (Methode A): RT = 1,24 min

### 7 SYNTHESE VON (R)-1-(4-(1-HYDROXY-3-METHYLBUTAN-2-YLAMINO)-5-OXO-6,7-DIHYDRO-5H-5λ⁴-THIENO[3,2-d]PYRIMIDIN-2-YL)-3'-METHYL-1'H-SPIRO[PIPERIDIN-4.4'-CHINAZOLIN]-2'(3'H)-ON (BEISPIEL 14)

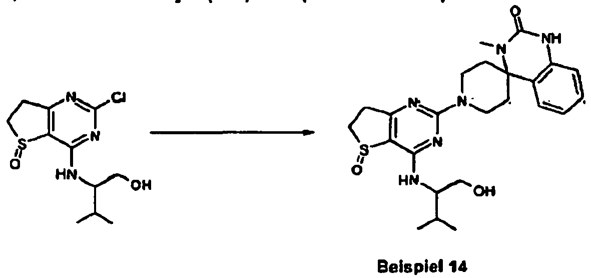

(**IV-1**) (siehe 1.2, 0,1 mmol) wird in 750 µl N-Methyl-2-pyrrolidon (NMP) und 50 µl Diisopropyltethylamin vorgelegt mit einer Lösung von 3'-methyl-1'*H*-spiro[piperidin-4,4'-chinazolin]-2'(3'*H*)-on *(*Chem. Pharm. Bull. 1988, 4659) (0,1 mmol) in 400µl NMP versetzt und 30 min bei 120°C in der Mikrowelle erhitzt. Anschliessend werden 600 µL DMF zugesetzt, die Reaktionslösung über präparative HPLC-MS (Methode A) aufgereinigt und die Produktfraktionen gefriergetrocknet. Analytische HPLC-MS (Methode C): RT = 1,58 min.

### 8. SYNTHESE VON (R)-2-[2-(4-BENZO[d]ISOXAZOL-3-YL-PIPERIDIN-1-YL)-5-OXO-6,7-DIHYDRO-5H-5λ⁴-THIENO[3,2-d]PYRIMIDIN-4-YLAMINO]-3-METHYLBUTAN-1-OL (BEISPIEL 16)

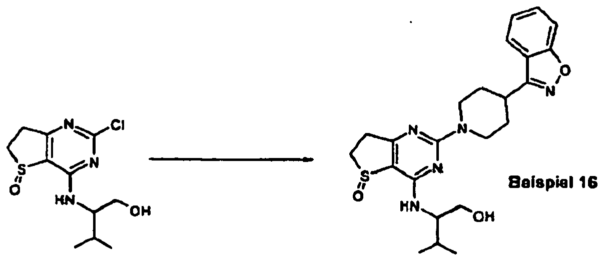

Ausgehend von (**IV-1**) (siehe 1.2) und 3-Piperidin-4-yl-benzo[d]isoxazol kann Beispiel 16 analog zu Beispiel 14 (siehe 7.) hergestellt und aufgereinigt werden. Analytische HPLC-MS (Methode C): RT = 1,74 min.

### 9. SYNTHESE VON (R)-3-METHYL-2-[5-OXO-2-(3,4,5,6-TETRAHYDRO-2H [4,4']BIPYRIDINYL-1-YL)-6,7-DIHYDRO-5H-5λ⁴-THIENO[3,2-d]PYRIMIDIN-4-YLAMINO]-BUTAN-1-OL (BEISPIEL 19)

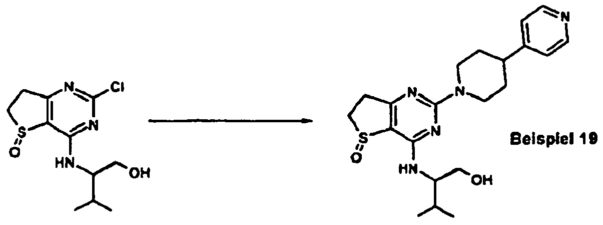

Ausgehend von (IV-1) (siehe 1.2) und 4-Piperidin-4-yl-pyridin kann Beispiel **19** analog zu Beispiel 14 (siehe 7) hergestellt und aufgereinigt werden. Analytische HPLC-MS (Methode C): RT = 1,33 min.

### 10. SYNTHESE VON (R)-2-{2-[4-(2-ETHYL-5-FLUOR-1H-INDOL-3-YL)-PIPERIDIN-1-YL]-5-OXO-6,7-D1HYDRO-5H-5λ⁴-THIENO[3,2-d]PYRIMIDIN-4-YLAMINO}-3-METHYLBUTAN-1-OL (BEISPIEL 22)

### 10.1 2-But-1-ynyl-4-fluorphenylamin

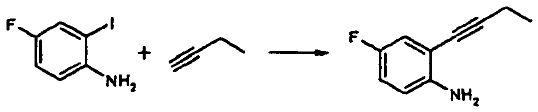

80 ml Tetrahydrofuran wird unter Argon vorgelegt. 5 g 4-Fluor-2-iodphenylamin, 0,74 g Dichlorbis(triphenylphosphin) palladium(II), 0,2 g Kupferiodid und 8,8 ml Triethylamin werden zugegeben. 4 g gasförmiges 1-butin werden durch die Suspension geleitet. Das Reaktionsgemisch wird unter Argon 15 Stunden bei Raumtemperatur gerührt, dann über Celite filtriert und zur Trockne eingedampft. 3,4 g Produkt werden als Feststoff erhalten. ¹H NMR (400 MHz, DMSO): 2,45 (2H, q); 1,18 (3H, t).

### 10.2 2-Ethyl-5-fluor-1H-indol

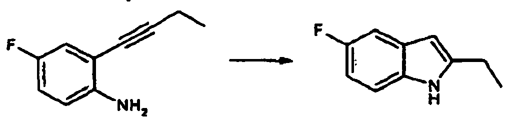

Unter Argon werden 4,9 g Kalium-*tert*-butylat in 25 ml N-Methyl-2-pyrrolidinon suspendiert und eine Suspension von 3,4 g 2-But-1-ynyl-4-fluorphenylamin in 25 ml N-Methyl-2-pyrrolidinon dazu getropft. Das Reaktionsgemisch wird 3 Stunden bei Raumtemperatur gerührt und mit Wasser versetzt. Das Produkt wird mit Diethylether extrahiert und chromatographisch (Kieselgel, Cyclohexan/Essigester 100/0- 90/10) gereinigt. 2,83 g Produkt werden als Feststoff erhalten. ¹H NMR (400 MHz, DMSO): 2,72 (2H, q); 1,27 (3H, t).

### 10.3 2-Ethyl-5-fluor-3-(1,2,3,6-tetrahydropyridin-4-yl)-1H-indol

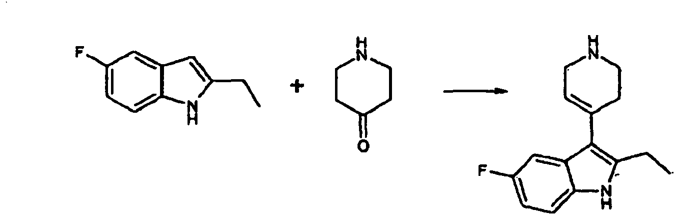

2,83 g 2-Ethyl-5-fluor-1*H*-indol werden in 50 ml Essigsäure suspendiert und auf 90°C erwärmt. Eine Suspension von 6,66 g 4-Piperidon in 15 ml Phosphorsäure 2N wird zugegeben. Das Reaktionsgemisch wird 3 Stunden bei 90°C gerührt, mit Natronlauge versetzt und das Produkt mit Essigester extrahiert. 2,85 g Produkt werden als Feststoff erhalten. ¹H NMR (400 MHz, DMSO): 5,63 (1H, s); 2,73 (2H, q); 1,23 (3H, t).

### 10.4 2-Ethyl-5-fluor-3-piperidin-4-yl-1H-indol (V-1)

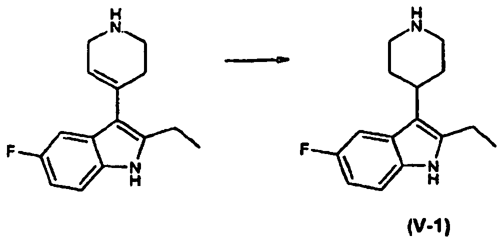

2,83 g 2-Ethyl-5-fluor-3-(1,2,3,6-tetrahydropyridin-4-yl)-1*H*-indol werden in 50 ml Methanol suspendiert und mit 0,3 g Pd/C 10% bei Normaldruck und Raumtemperatur hydriert. Der Katalysator wird abgesaugt und das Filtrat zur Trockne eingedampft. 2,3 g (V-1) werden als Feststoff erhalten. ¹H NMR (400 MHz, DMSO): 2,70 (2H, q); 1,19 (3H, t).

### 10.5 (R)-2-{2-[4-(2-Ethyl-5-fluor-1H-indol-3-yl)-piperidin-1-yl]-5-oxo-6,7-dihydro-5H-5λ⁴-thieno[3,2-d]pyrimidin-4-ylamino}-3-methylbutan-1-ol (Beispiel 22)

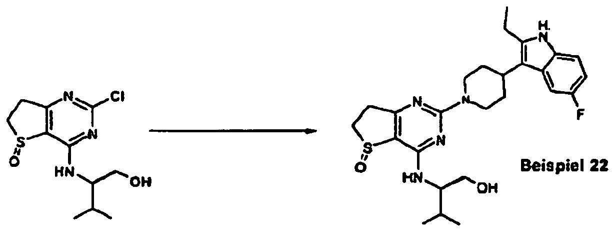

Ausgehend von (**IV-1**) (siehe 1.2) und (**V-1**) kann **Beispiel 22** analog zu Beispiel 14 (siehe 7.) hergestellt und aufgereinigt werden. Analytische HPLC-MS (Methode C): RT = 1,83 min.

### 11. SYNTHESE VON 1-(4-(1-HYDROXYMETHYLCYCLOPROPYLAMINO)-5-OXO-6,7-DIHYDRO-5H-5λ⁴-THIENO[3,2-d]PYRIMIDIN-2-YL)-3'-METHYL-1'H-SPIRO[PIPERIDIN-4,4'-CHINAZOLIN]-2'(3'H)-ON (BEISPIEL 28)

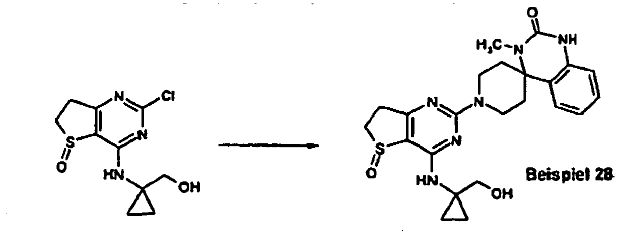

Ausgehend von (IV-2) (siehe 2.4) und 3'-methyl-1'H-spiro[piperidin-4,4'-chinazolin]-2'(3'*H*)-on *(*Chem. Pharm. Bull. 1988, 4659) kann **Beispiel 28** analog zu Beispiel 14 (siehe 7.) hergestellt und aufgereinigt werden. Analytische HPLC-MS (Methode C): RT = 1,52 min.

### 12. SYNTHESE VON 3-{1-[4-(1-HYDROXYMETHYLCYCLOPROPYLAMINO)-5-OXO-6,7-DIHYDRO-5H-5λ⁴-THIENO[3,2-d]PYRIMIDIN-2-YL}-PIPERIDIN-4-YL}-1H-INDOL-6-CARBONSÄUREETHYLESTER (BEISPIEL 29)

### 12.1 3-(1-Benzyl-1,2,3,6-tetrahydropyridin-4-yl)-1H-indol-6-carbonsäure

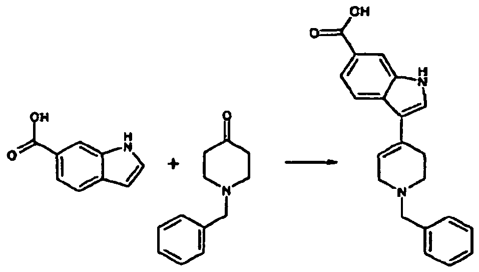

2,6 g Kaliumhydroxid werden in 25 ml Methanol suspendiert und 2,5 g 1*H*-Indol-6-carbonsäure und 5,5 g 1-Benzyl-piperidin-4-on zugefügt. Das Reaktionsgemisch wird 15 Stunden unter Rückfluß gerührt und dann zur Trockne eingedampft. Der Rückstand wird mit Salzäure (1 M) versetzt und zur Trockne eingedampft. Der Rückstand wird mit Methanol und Diethylether behandelt und der Feststoff abgesaugt. 12,4 g Produkt werden als Feststoff erhalten. ¹H NMR (400 MHz, DMSO): 6,2 (1H, s).

### 12.2 3-(1-Benzyl-1,2,3,6-tetrahydropyridin-4-yl)-1H-indol-6-carbonsäureethylester

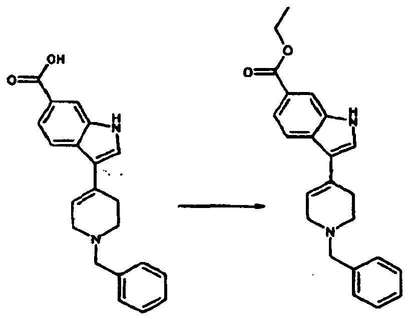

12,4 g 3-(1-Benzyl-1,2,3,6-tetrahydropyridin-4-yl)-1*H*-indol-6-carbonsäure werden in 80 ml Ethanol suspendiert und 1,6 ml konz. Schwefelsäure zugegeben. Das Reaktionsgemisch wird 96 Stunden unter Rückfluß geführt. Der Feststoff wird abgesaugt, in Ethanol gelöst und mit Natronlauge basisch gestellt. 5,3 g Produkt werden als Feststoff erhalten. ¹H NMR (400 MHz, DMSO): 6,2 (1H, s); 4,3 (2H, q); 1,35 (3H, s).

### 12.3 3-(1,2,3,6-Tetrahydropyridin-4-yl)-1H-indol-6-carbonsäureethylester (V-2)

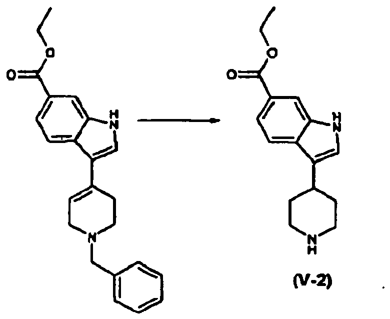

5 g 3-(1-Benzyl-1,2,3,6-tetrahydropyridin-4-yl)-1*H*-indol-6-carbonsäureethylester und 2,3 g Palladiumhydroxid werden in 180 ml Methanol suspendiert und bei 50 psi 2 Stunden bei Raumtemperatur hydriert. Der Katalysator wird abgesaugt und die Mutterlauge zur Trockne eingedampft. 3,6 g (V-2) werden als Feststoff erhalten. ¹H NMR (400 MHz, DMSO): 4,3 (2H, q); 2,95 - 2,80 (1H, m); 1,35 (3H, s).

### 12.4 3-{1-[4-(1-Hydroxymethylcyclopropylamino)-5-oxo-6,7-dihydro-5H-5λ⁴-thieno[3,2-d]pyrimidin-2-yl}-piperidin-4-yl}-1H-indol-6-carbonsäureethylester (Beispiel 29)

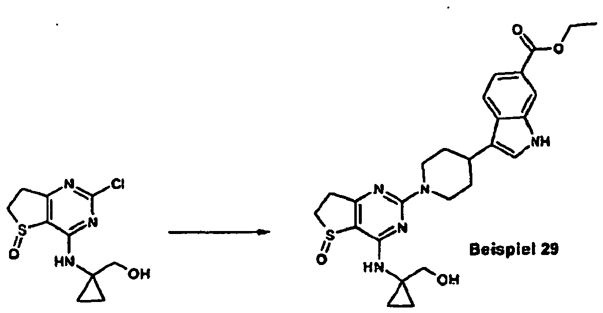

Ausgehend von **(IV-2)** (siehe 2.4) und **(V-2)** (siehe 12.3) kann **Beispiel 29** analog zu Beispiel 14 (siehe 7.) hergestellt und aufgereinigt werden. Analytische HPLC-MS (Methode C): RT = 1,77 min.

### 13. SYNTHESE VON (1-{2-[4-(2-ETHYL-5-FLUOR-1H-INDOL-3-YL)-PIPERIDIN-1-YL]-5-OXO-6,7-DIHYDRO-5H-5λ⁴-THIENO[3,2-d]PYRIMIDIN-4-YLAMINO}-CYCLOPROPYL)-METHANOL (BEISPIEL 37)

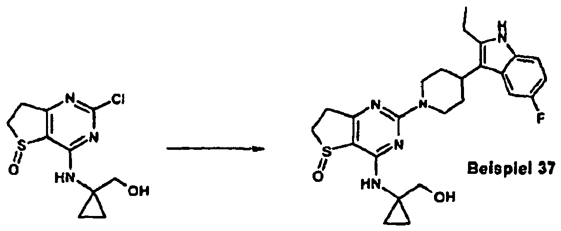

Ausgehend von (**IV-2**) (siehe 2.4) und (**V-1**) (siehe 10.4) kann **Beispiel 37** analog zu Beispiel 14 (siehe 7.) hergestellt und aufgereinigt werden. Analytische HPLC-MS (Methode C): RT = 1,78 min.

### 14_{.} SYNTHESE VON (S)-3'-METHYL-1-(4-(1-METHYL-6-OXOPIPERIDIN-3-YLAMINO)-5-OXO-6,7-DIHYDRO-5H-5λ⁴-THIENO[3,2-d]PYRIMIDIN-2-YL)-3'-METHYL-1'H-SPIRO[PIPERIDIN-4,4'-CHINAZOLIN]-2'(3'H)-ON (BEISPIEL 43)

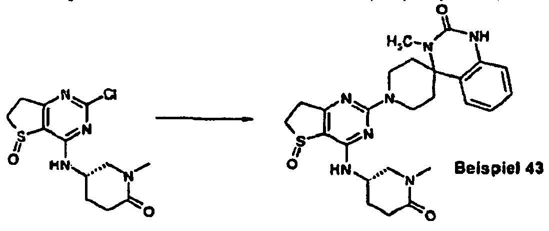

Ausgehend von (IV-5) (siehe 5.5) und 3'-methyl-1'*H*-spiro[piperidin-4,4'-chinazolin]-2'(3'H)-on (Chem. Pharm. Bull. 1988, 4659) kann **Beispiel 43** analog zu Beispiel 14 (siehe 7.) hergestellt und aufgereinigt werden. Analytische HPLC-MS (Methode C): RT = 1,49 min.

### 15. SYNTHESE VON 1-[4-((S)-1-METHYL-6-OXOPIPERIDIN-3-YLAMINO)-5-OXO-6,7-DIHYDRO-5H-5λ⁴-THIENO[3,2-d]PYRIMIDIN-2-YL]-4-PHENYLPIPERIDIN-4-CARBONITRIL (BEISPIEL 55)

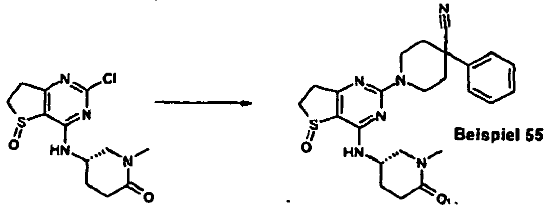

Ausgehend von **(IV-5)** (siehe 5.5) und 4-Phenylpiperidin-4-carbonitril
kann **Beispiel 55** analog zu Beispiel 14 (siehe 7) hergestellt und aufgereinigt werden. Analytische HPLC-MS (Methode C): RT = 1,71 min.

### 16. SYNTHESE VON 3'-METHYL-1-(4-(TETRAHYDRO-2H-PYRAN-4-YLAMINO)-5-OXO-6,7-DIHYDRO-5H-5λ⁴-THIENO[3,2-d]PYRIMIDIN-2-YL)-1'H-SPIRO[PIPERIDIN-4,4'-CHINAZOLIN]-2'(3'H)-ON (BEISPIEL 58)

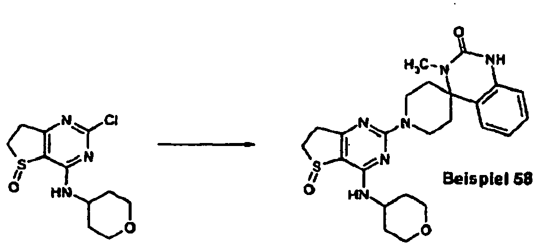

Ausgehend von **(IV-6)** (siehe 6.2) und 3'-methyl-1'*H*-spiro[piperidin-4,4'-chinazolin]-2'(3'H)-on *(*Chem. Pharm. Bull. 1988, 4659) kann **Beispiel 58** analog zu Beispiel 14 (siehe 7.) hergestellt und aufgereinigt werden. Analytische HPLC-MS (Methode C): RT =1,56 min.

### 17. SYNTHESE VON 1-(4-(3-FLUORPHENYLAMINO)-5-OXO-6,7-DIHYDRO-5H-5λ⁴-THIENO[3,2-d]PYRIMIDIN-2-YL)-3'-METHYL-1'H-SPIRO[PIPERIDIN-4,4'-CHINAZOLIN]-2'(3'H)-ON (BEISPIEL 73)

### 17.1 (2-Chlor-6,7-dihydrothieno[3,2-d]pyimidin-4-yl)-(3-fluorphenyl)-amin (III-7):

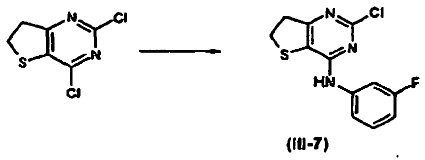

4 g (II) werden in 15 ml Dimethylformamid vorgelegt, anschliessend werden 4,5 ml Diisopropylethylamin und dann 2,5 ml 3-Fluorphenylamin zugegeben. Das Reaktionsgemisch wird bei 120°C, bis keine weitere Umsetzung erfolgt, erhitzt und nach Abkühlen eingedampft. Der Rückstand wird mit Wasser versetzt. Das Produkt wird mit Dichlormethan extrahiert und chromatographisch (Kieselgel, Petrolether/Essigester 80/20 bis 60/40) gereinigt. 2,6 g (III-7) werden als Feststoff erhalten. Analytische HPLC (Methode A): RT = 3,27 min

### 17.2 2-Chlor-5-oxo-6,7-dihydro-5H-5λ⁴-thieno[3,2-d]pyrimidin-4-yl)-(3-fluorphenyl)-amin (IV-7):

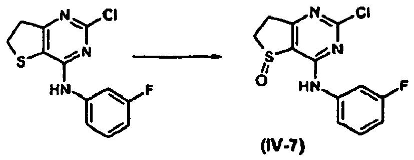

0,102 g S-(-)-1,1'-Bi-2-naphtol werden in 0,5 ml Chloroform unter Argon vorgelegt, dann 0,052 ml Titan(IV)-isopropylat und 0,064 ml Wasser zugegeben. Das Reaktionsgemisch wird 45 Minuten bei Raumtemperatur gerührt. Anschließend wird eine Suspension von 0,5 g (III-7) in 25 ml Chloroform zugegeben. Das Reaktionsgemisch wird auf -2°/-4°C abgekühlt und nach 20 Minuten werden 0,323 ml tert-Butylhydroperoxid 5-6 M in Decan zugetropft. Das Reaktionsgemisch wird bei -2/-4°C, bis keine weitere Umsetzung erfolgt, weitergerührt und mit Wasser versetzt. Das Produkt wird mit Dichlormethan extrahiert und chromatographisch (Kieselgel, Dichlormethan/Methanol 100/0 bis 95/5) gereinigt. 0,47 g (IV-7) werden als Feststoff erhalten. Analytische HPLC-MS (Methode A): RT = 1,15 min.

### 17.3 1-(4-(3-fluorphenylamino)-5-oxo-6,7-dihydro-5H-5λ⁴-thieno [3,2-d]pyrimidin-2-yl)-3'-methyl-1'H-spiro[piperidin-4,4'-chinazolin]-2'(3'H)-on (Beispiel 73)

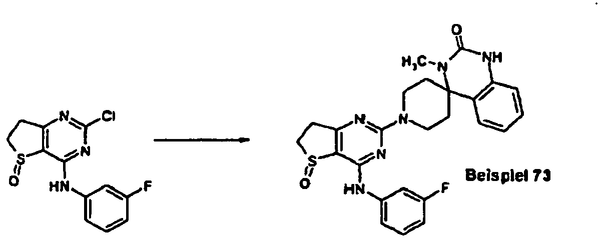

Ausgehend von **(IV-7)** (siehe 17.2) und 3'-methyl-1'*H*-spiro[piperidin-4,4'-chinazolin]-2'(3'H)-on *(*Chem. Pharm. Bull. 1988, 4659) kann **Beispiel 73** analog zu Beispiel 14 (siehe 7.) hergestellt und aufgereinigt werden. Analytische HPLC-MS (Methode C): RT = 1,81 min.

### 18. SYNTHESE VON [2-(4-BENZO[d]ISOXAZOL-3-YL-PIPERIDIN-1-YL)-5-OXO-6,7-6,7-DIHYDRO-5H-5λ⁴-THIENO[3,2-d]PYRIMIDIN-4-YL]-(3-FLUORPHENYL)-AMIN (BEISPIEL 75)

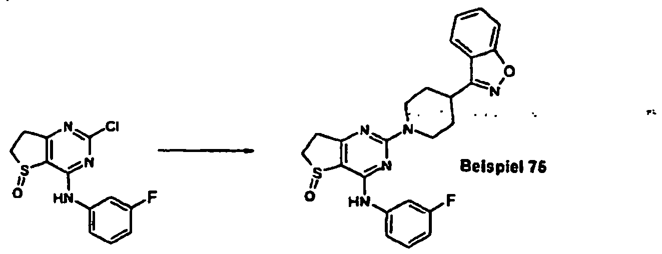

Ausgehend von (IV-7) (siehe 17.2) und 3-Piperidin-4-yl-benzo[*d*]isoxazol kann **Beispiel 75** analog zu Beispiel 14 (siehe 7.) hergestellt und aufgereinigt werden. Analytische HPLC-MS (Methode C): RT = 2,11 min.

### 19. SYNTHESE VON (3-FLUORPHENYL)-[5-OXO-2-(3,4,5,6-TETRAHYDRO-2H-[4,4']BIPYRIDINYL-1-YL)-6,7-DIHYDRO-5H-5λ⁴-THIENO[3,2-d]PYRIMIDIN-4-YL]-AMIN TRIFLUORACETAT (BEISPIEL 78)

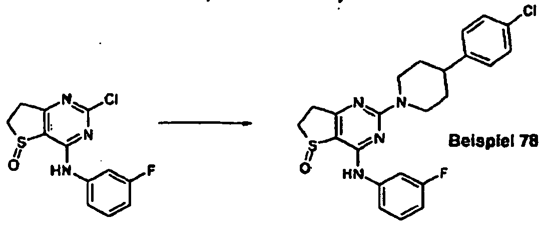

Ausgehend von (IV-7) (siehe 17.2) und 4-(4-Chlorphenyl)-piperidin kann **Beispiel 78** als Trifluoracetat analog zu Beispiel 14 (siehe 7.) hergestellt und aufgereinigt werden. Analytische HPLC-MS (Methode C): RT = 1,55 min.

### 20. SYNTHESE VON {2-[4-(2-ETHYL-5-FLUOR-1H-INDOL-3-YL)-PIPERIDIN-1-YL]-5-OXO-6,7-DIHYDRO-5H-5λ⁴-THIENO[3,2-d]PYRIMIDIN-4-YL}-(3-FLUORPHENYL)-AMIN (BEISPIEL 82)

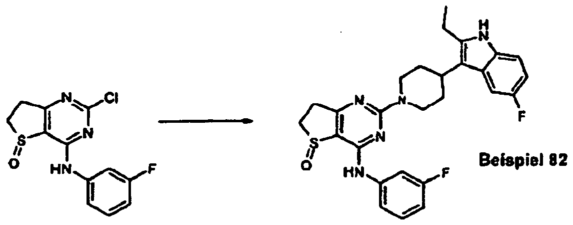

Ausgehend von **(IV-7)** (siehe 17.2) und **(V-1)** (siehe 10.4) kann **Beispiel 82** analog zu Beispiel 14 (siehe 7.) hergestellt und aufgereinigt werden. Analytische HPLC-MS (Methode C): RT = 2,12 min.

### 21. SYNTHESE VON (1-{2-[4-(2,4-DIFLUORPHENYL)-PIPERIDIN-1-YL]-5-OXO-6,7-DIHYDRO-5H-5λ⁴-THIENO[3,2-d]PYRIMIDIN-4-YLAMINO)-CYCLOPROPYL)-METHANOL (BEISPIEL 89)

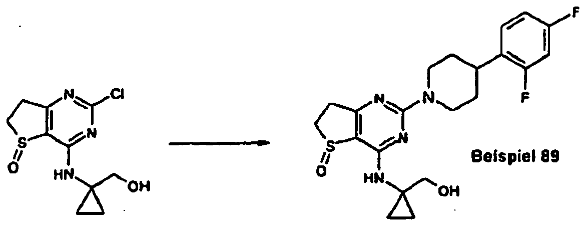

Ausgehend von **(IV-2)** (siehe 2.4) und 4-(2,4-Difluorphenyl)-piperidin kann **Beispiel 89** analog zu Beispiel 14 (siehe 7.) hergestellt werden. Das Produkt kann chromatographisch (präparative HPLC, Methode B) gereinigt werden. Analytische HPLC-MS (Methode D): RT = 1,18 min.

### 22. SYNTHESE VON {2-[4-(2,4-DIFLUORPHENYL)-PIPERIDIN-1-YL]-5-OXO-6,7-DIHYDRO-5H-5λ⁴-THIENO[3,2-d]PYRIMIDIN-4-YL}-(TETRAHYOROPYRAN-4-YL)-AMIN (BEISPIEL 90)

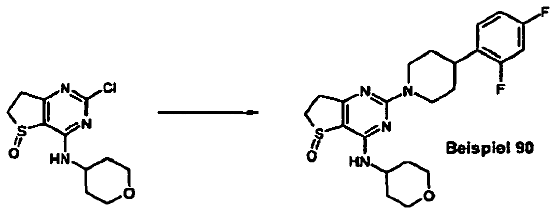

Ausgehend von **(IV-6)** (siehe 6.2) und 4-(2,4-Difluorphenyl)-piperidin kann **Beispiel 90** analog zu Beispiel 89 (siehe 21.) hergestellt und aufgereinigt werden. Analytische HPLC-MS (Methode D): RT = 1,23 min.

### 23. SYNTHESE VON (1-{2-[4-(3,5-DICHLORPHENYL)-PIPERIDIN-1-YL]-5-OXO-6,7-DIHYDRO-5H-5λ⁴-THIENO[3,2-d]PYRIMIDIN-4-YLAMINO}-CYCLOPROPYL)-METHANOL (BEISPIEL 91)

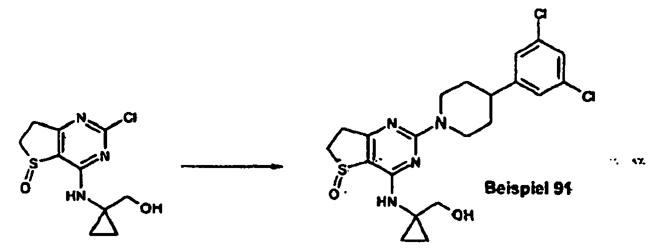

Ausgehend von (**IV-2**) (siehe 2.4) und 4-(3,5-Dichlorphenyl)-piperidin kann **Beispiel 91** analog zu Beispiel 89 (siehe 21.) hergestellt und aufgereinigt werden. Analytische HPLC-MS (Methode D): RT = 1,30 min.

### 24. SYNTHESE VON (1-{2-[4-(4-BROMPHENYL)-PIPERIDIN-1-YL]-5-OXO-6,7-DIHYDRO-5H-5λ⁴-THIENO[3,2-d]PYRIMIDIN-4-YLAMINO}-CYCLOPROPYL)-METHANOL (BEISPIEL 92)

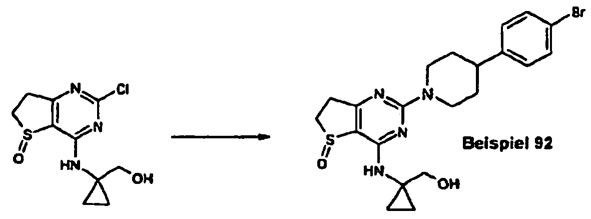

Ausgehend von (**IV-2**) (siehe 2.4) und 4-(4-Bromphenyl)-piperidin kann **Beispiel 92** analog zu Beispiel 89 (siehe 21.) hergestellt und aufgereinigt werden. Analytische HPLC-MS (Methode D): RT = 1,23 min.

### 25. SYNTHESE VON {2-[4-(4-BROMPHENYL)-PIPERIDIN-1-YL]-5-OXO-6,7-DIHYDRO-5H-5λ⁴-THIENO[3,2-d]PYRIMIDIN-4-YL}-(TETRAHYDROPYRAN-4-YLAMIN (BEISPIEL 93)

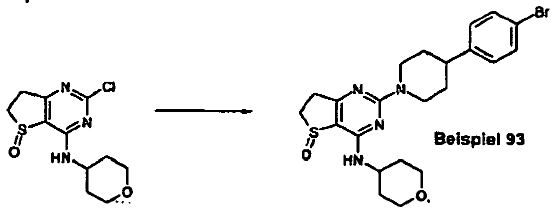

Ausgehend von **(IV-6)** (siehe 6.2) und 4-(4-Bromphenyl)-piperidin kann **Beispiel 93** analog zu Beispiel 89 (siehe 21) hergestellt und aufgereinigt werden. Analytische HPLC-MS (Methode D): RT = 1,28 min.

### 26. SYNTHESE VON (1-{5-OXO-2-[4-(4-TRIFLUORMETHYLPHENYL)-PIPERIDIN-1-YL]-6,7-DIHYDRO-5H-5λ⁴-THIENO[3,2-d]PYRIMIDIN-4-YLAMINO}-CYCLOPROPYL)-METHANOL (BEISPIEL 95)

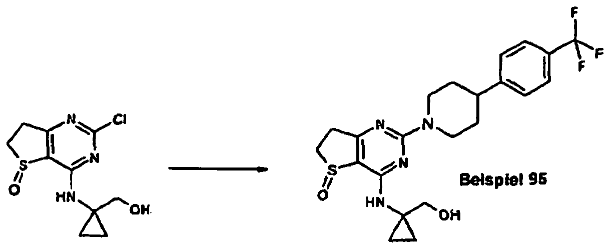

Ausgehend von **(IV-2)** (siehe 2.4) und 4-(4-Trifluormethylphenyl)-piperidin kann **Beispiel 95** analog zu Beispiel 89 (siehe 21.) hergestellt und aufgereinigt werden. Analytische HPLC-MS (Methode D): RT = 1,25 min.

### 27. SYNTHESE VON {5-OXO-2-[4-(4-TRIFLUORMETHYLPHENYL)-PIPERIDIN-1-YL]-6,7-DIHYDRO-5H-5λ⁴-THIENO[3,2-d]PYRIMIDIN-4-YL}-(TETRAHYDROPYRAN-4-YL)-AMIN (BEISPIEL 96)

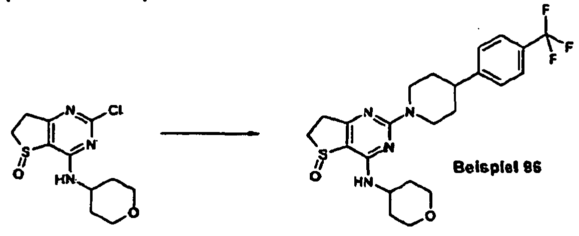

Ausgehend von **(IV-6)** (siehe 6.2) und 4-(4-Trifluormethylphenyl)-piperidin kann **Beispiel 96** analog zu Beispiel 89 (siehe 21.) hergestellt und aufgereinigt werden. Analytische HPLC-MS (Methode D): RT = 1,29 min.

### 28. SYNTHESE VON {2-[4-(3,5-DICHLORPHENYL)-PIPERIDIN-1-YL]-5-OXO-6,7-DIHYDRO-5H-5λ⁴-THIENO[3,2-d]PYRIMIDIN-4-YL}-(TETRAHYDROPYRAN-4-YL)-AMIN (BEISPIEL 97)

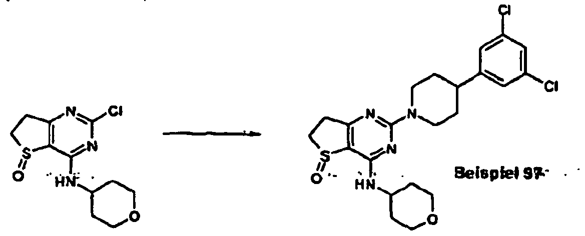

Ausgehend von **(IV-6)** (siehe 6.2) und 4-(3,5-Dichlorphenyl)-piperidin kann **Beispiel 97** analog zu Beispiel 89 (siehe 21.) hergestellt und aufgereinigt werden. Analytische HPLC-MS (Methode A): RT = 1,29 min.

### 29. SYNTHESE VON {1-[2-(4-BENZOXAZOL-2-YL-PIPERIDIN-1-YL)-5-OXO-6,7-DIHYDRO-5H-5λ⁴-THIENO[3,2-d]PYRIMIDIN-4-YLAMINO]-CYCLOPROPYL}-METHANOL (BEISPIEL 98)

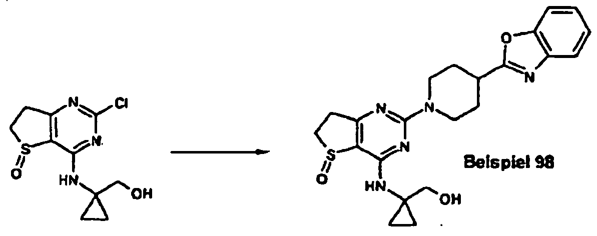

Ausgehend von **(IV-2)** (siehe 2.4) und 2-Piperidin-4-yl-Benzoxazol kann **Beispiel 98** analog zu Beispiel 89 (siehe 21) hergestellt und aufgereinigt werden . Analytische HPLC-MS (Methode B): RT = 1,22 min.

### 30. SYNTHESE VON [2-(4-BENZOXAZOL-2-YL-PIPERIDIN-1-YL)-5-OXO-6,7-DIHYDRO-5H-5λ⁴-THIENO[3,2-d]PYRIMIDIN-4-YL]-(TETRAHYDROPYRAN-4-YL)-AMIN (BEISPIEL 99)

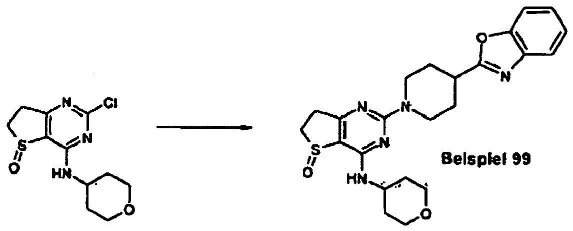

Ausgehend von **(IV-6)** (siehe 6.2) und 2-Piperidin-4-yl-benzoxazol kann **Beispiel 99** analog zu Beispiel 89 (siehe 21.) hergestellt und aufgereinigt werden. Analytische HPLC-MS (Methode B): RT = 1,23 min.

### 31. SYNTHESE VON (S)-5-[2-(4-BENZOXAZOL-2-YL-PIPERIDIN-1-YL)-5-OXO-6,7-DIHYDRO-5H-5λ⁴-THIENO[3,2-d]PYRIMIDIN-4-YLAMINO]-1-METHYLPIPERIDIN-2-ON (BEISPIEL 100)

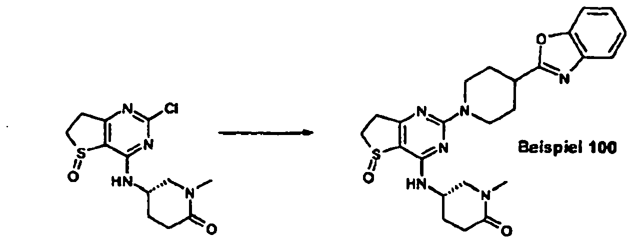

Ausgehend von **(IV-5)** (siehe 5.5) und 2-Piperidin-4-yl-benzoxazol kann **Beispiel 100** analog zu Beispiel 89 (siehe 21.) hergestellt und aufgereinigt werden. Analytische HPLC-MS (Methode B): RT = 1,18 min.

### 32. SYNTHESE VON (3-FLUORPHENYL)-{2-[4-(5-FURAN-2-YL-2H-PYRAZOL-3-YL)-PIPERIDIN-1-YL]-5-OXO-6,7-DIHYDRO-5H-5λ⁴-THIENO[3,2-d]PYRIMIDIN-4-YL}-AMIN TRIFLUORACETAT (BEISPIEL 145)

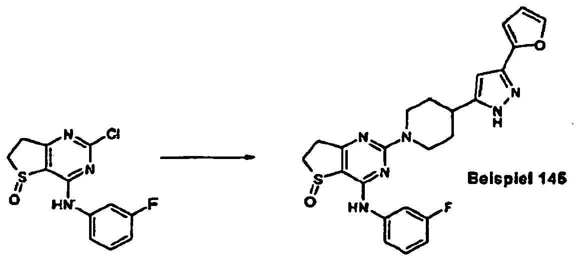

Ausgehend von **(IV-7)** (siehe 17.2) und 4-(5-Furan-2-yl-2*H*-pyrazol-3-yl)-piperidin kann **Beispiel 145** als Trifluoracetat analog zu Beispiel 14 (siehe 7.) hergestellt und aufgereinigt werden. Analytische HPLC-MS (Methode C): RT = 1,89 min.

### 33. SYNTHESE VON (3-FLUORPHENYL)-{5-OXO-2-[4-(3-PYRIDIN-4-YL-[1,2,4]OXADIAZOL-5-YL)-PIPERIDIN-1-YL]-6,7-DIHYDRO-5H-5λ⁴-THIENO[3,2-d]PYRIMIDIN-4-YL}-AMIN TRIFLUORACETAT (BEISPIEL 147)

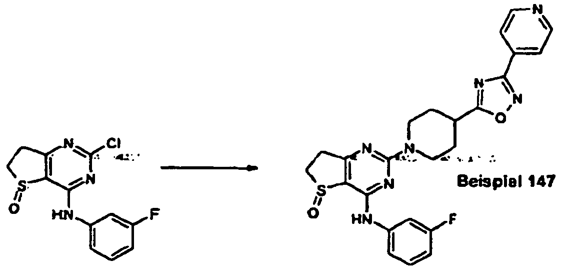

Ausgehend von **(IV-7)** (siehe 17.2) und 4-(5-Piperidin-4-yl-[1,2,4]oxadiazol-3-yl)-pyridin kann **Beispiel 147** als Trifluoracetat analog zu Beispiel 14 (siehe 7.) hergestellt und aufgereinigt werden. Analytische HPLC-MS (Methode C): RT = 1,72 min.

### 34. SYNTHESE VON (R)-2-{2-[4-(5-FURAN-2-YL-2H-PYRAZOL-3-YL)-PIPERIDIN-1-YL]-5-OXO-6,7-DIHYDRO-5H-5λ⁴-THIENO[3,2-d]PYRIMIDIN-4-YLAMINO}-3-METHYLBUTAN-1-OL TRIFLUORACETAT (BEISPIEL 161)

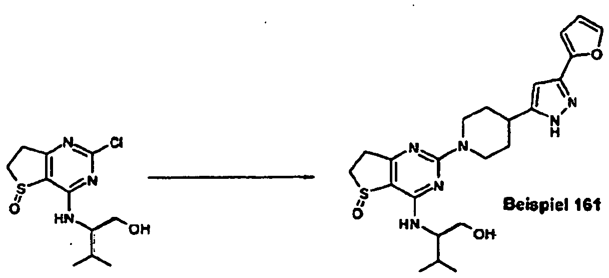

Ausgehend von **(IV-1)** (siehe 1.2) und 4-(5-Furan-2-yl-2*H*-pyrazol-3-yl)-piperidin kann **Beispiel 161** als Trifluoracetat analog zu Beispiel 14 (siehe 7.) hergestellt und aufgereinigt werden. Analytische HPLC-MS (Methode C): RT = 1,67 min.

### 35. SYNTHESE VON (R)-3-METHYL-2-{5-OXO-2-[4-(3-PYRIDIN-4-YL-[1,2,4]OXADIAZOL-5-YL)-PIPERIDIN-1-YL]-6,7-DIHYDRO-5H-5λ⁴-THIENO[3,2-d]PYRlMIDIN-4-YLAMINO}-BUTAN-1-OL TRIFLUORACETAT (BEISPIEL 163)

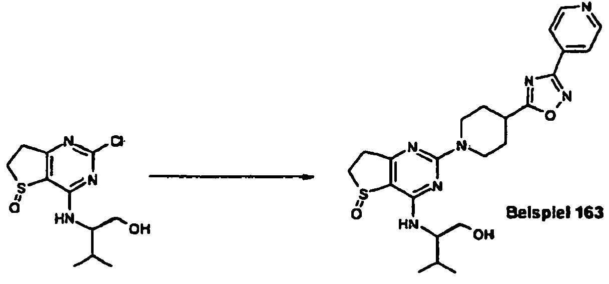

Ausgehend von **(IV-1)** (siehe 1.2) und 4-(5-Piperidin-4-yl-[1,2,4]oxadiazol-3-yl)-pyridin kann **Beispiel 163** als Trifluoracetat analog zu Beispiel 14 (siehe 7.) hergestellt und aufgereinigt werden. Analytische HPLC-MS (Methode,C): RT = 1,48 min.

### 36. SYNTHESE VON: (2-{4-[4-(2-DIETHYLAMINOETHOXY)PHENOXY]-PIPERID1N-1-Yl}-5-OXO-6,7-DIHYDRO-5H-5λ⁴-THIENO[3,2-d]PYRIMIDIN-4-YL)-(TETRAHYDRO PYRAN-4-YL)-AMIN (BEISPIEL 178)

### 36.1 4-[4-(2-Diethylaminoethoxy)-phenoxy]-piperidin-1-carbonsäure-tert-butylester.

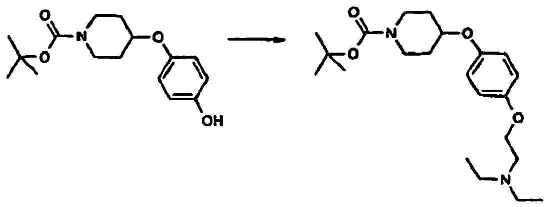

7,9 g 4-(4-Hydroxyphenoxy)-piperidin-1-carbonsäure-*tert*-butylester (siehe WO2006/64218), 5,2 g (2-Chlorethyl)-diethylamin Hydrochlorid und 16,6 g Kaliumcarbonat werden in 250 ml Aceton vorgelegt. Das Reaktionsgemisch wird unter Rückfluß gerührt. Nach 4 Stunden werden die anorganischen Satze abgesaugt und die Mischung zur Trockne eingedampft. Der Rückstand wird mit Essigester versetzt. Die organische Phase wird mit einer gesättigten NaHCO₃ Lösung gewaschen, getrocknet und zur Trockne eingedampft. 10,1 g des Produkts werden als Öl erhalten.

### 36.2 Diethyl-{2-[4-(piperidin-4-yloxy)-phenoxy]-ethyt}-amin (V-3):

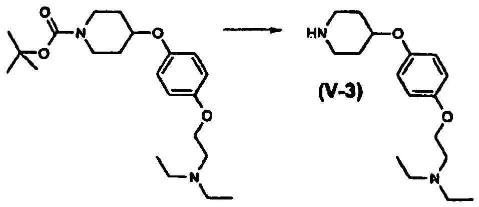

10,1 g 4-[4-(2-Diethylaminoethoxy)-phenoxy]-piperidin-1-carbonsäure-*tert*-butylester werben in 20 ml. Dichlormethan vorgelegt und unter Kühlung mit 30 ml Trifluoressigsäure versetzt. Das Reaktionsgemisch wird bei Raumtemperatur gerührt. Nach 2 Stunden wird das Reaktionsgemisch zur Trockne eingedampft. Der Rückstand wird mit einer NaOH Lösung (1 M) versetzt und das Produkt mit Dichlormethan extrahiert. 5,6 g **(V-3)** werden erhalten.

### 36.3 (2-{4-[4-(2-Diethylamino-ethoxy)-phenoxy]-piperidin-1-yl}-5-oxo-6,7-dihydro-5H-5λ⁴-thieno[3,2-d]pyrimidin-4-yl)-(tetrahydropyran-4-yl)-amin (Beispiel178)

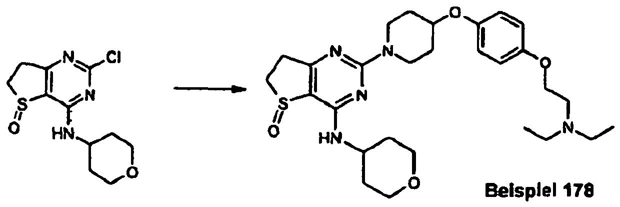

Ausgehend von **(IV-6)** (siehe 6.2) und **(V-3)** kann **Beispiel 178** analog zu Beispiel 89 (siehe 21.) hergestellt und aufgereinigt werden. Analytisch HPLC-MS (Methode A): RT = 1,01 min.

### 37. SYNTHESE VON: (2-{4-[4-(4,5-DIHYDROOXAZOL-2-YL)-PHENOXY]-PIPERIDIN-1-YL}-5-OXO-6,7-DIHYDRO-5H-5λ⁴-THIENO[3,2-d]PYRIMIDIN-4-YL)-(TETRAHYDRO PYRAN-4-YL)-AMIN (BEISPIEL 180)

### 37.1 4-(Toluol-4-sulfonyloxy)-piperidin-1-carbonsäure-tert-butylester.

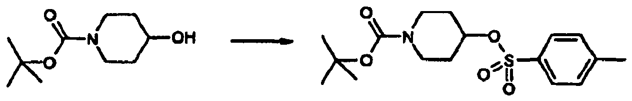

5 g 4-Hydroxypiperidin-1-carbonsäure-*tert*-butylester werden in 15 ml Pyridin vorgelegt, dann 4,7 g *p*-Toluolsulfonylchlorid portionsweise zugegeben. Das Reaktionsgemisch wird bei Raumtemperatur gerührt, nach 12 Stunden auf Eiswasser gegossen und die erhaltene Mischung eine weitere Stunde bei Raumtemperatur gerührt. Der ausgefallene Feststoff wird abgesaugt und getrocknet. 7,5 g Produkt werden erhalten.

### 37.2 4-[4-(4,5-Dihydrooxazol-2-yl)-phenoxy]-piperidin-1-carbonsäure-tert-butylester

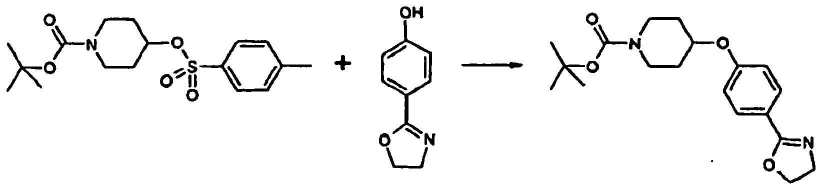

2,0 g 4-(4,5-Dihydrooxazol-2-yl)-phenol (siehe US5491201) werden in 30, ml Dimethylformamid vorgelegt, dann 3,3 g Kaliumcarbonat und 4,2 g 4-(Toluol-4-sulfonyloxy)-piperidin-1-carbonsäure-*tert*-butylester zugegeben. Das Reaktionsgemisch wird bei 75°C gerührt, nach 12 Stunden mit Wasser versetzt und der ausgefallene Feststoff abgesaugt und getrocknet 2,8 g Produkt werden erhalten.

### 37.3 4-[4-(4,5-Dihydrooxazol-2-yl)-phenoxy]-peperidin (V-4):

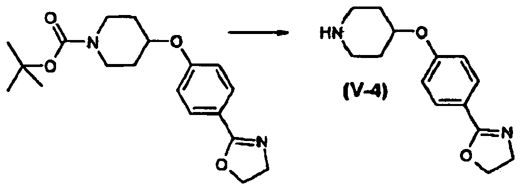

50 mg 4-[4-(4,5-Dihydrooxazol-2-yl)-phenoxy]-piperidin-1-carbonsäure-*tert*-butylester werden vorgelegt und mit 6 ml einer (5/1) Dichlormethan/Trifluoressigsäure Mischung versetzt. Das Reaktionsgemisch wird bei Raumtemperatur gerührt und nach 15 min vorsichtig mit einer gesättigten NaHCO₃ Lösung versetzt. Die organische Phase wird getrocknet und zur Trockne eingedampft. 20 mg **(V-4)** werden erhalten.

### 37.4 (2-{4-[4-(4,5-Dihydrooxazol-2-yl)-phenoxy]-piperidin-1-y}-5-oxo-6,7-dihydro-5H-5λ⁴-thieno[3,2-d]pyrimidin-4-yl)-(tetrahydropyran-4-yl)-amin (Beispiel 180)

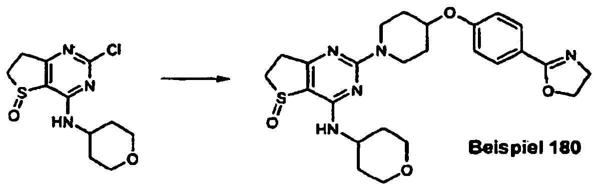

Ausgehend von **(IV-6)** (siehe 6.2) und **(V-4)** kann **Beispiel 180** analog zu Beispiel 89 (siehe 21.) hergestellt und aufgereinigt werden. Analytische HPLC-MS (Methode A): RT = 0,99 min.

### 38. SYNTHESE VON: 2,2,2-TRIFLUOR-1-(7-{1-[5-OXO-4-(TETRAHYDROPYRAN-4-YLAMINO)-6,7-DIHYDRO-5H-5λ⁴-THIENO[3,2-d]PYRIMIDIN-2-YL]-PIPERIDIN-4-YLOXY}-1,2.4,5-TETRAHYDROBENZO[d]AZEPIN-3-YL)-ETHANON (BEISPIEL 182)

### 38.1 2,2,2-Trifluor-1-(7-hydroxy-1,2,4,5-tetrahydrobenzo[d]azepin-3-yl)-ethanon,

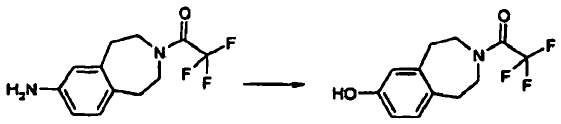

80 g 1-(7-Amino-1,2,4,5-tetrahydrobenzo[d]azepin-3-yl)-2,2,2-trifluor-ethanon (siehe US2005/137186) und 80 ml konz. Schwefelsäure werden in 672 ml Wasser vorgelegt. Das Reaktionsgemisch wird auf 0°C gekühlt, dann eine Mischung von 21,6 g Natriumnitrit in 128 ml Wasser innerhalb 10 min. zugetropft. Das Reaktionsgemisch wird 10 min. bei 0°, dann 2 Stunden bei Rückfluß gerührt, abgekühlt und auf 4 Liter Eiswasser gegossen. Der ausgefallene Feststoff wird abgesaugt und getrocknet. 71,9 g Produkt werden erhalten.

### 38.2 2,2,2-Trifluor-1-[7-(piperidin-4-yloxy)-1,2,4,5-tetrahydrobenzo[d]azepin-3-yl]-ethanon (V-5):

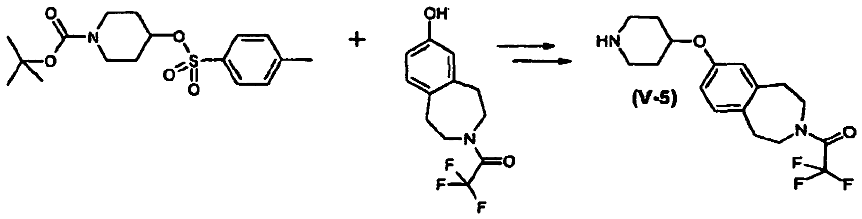

Ausgehend von 4-(Toluol-4-sulfonyloxy)-piperidin-1-carbonsäure*-tert*-butylester (siehe 37.1) und 2,2,2-Trifluor-1-(7-hydroxy-1,2,4,5-tetrahydrobenzo[d]azepin-3-yl)-ethanon (siehe 38.1) kann **(V-5)** analog zu (V-4) (siehe 37.2 und 37.3) hergestellt werden.

### 38.3 (2-{4-[4-(4,5-Dihydrooxazol-2-yl)-phenoxy]-piperidin-1-yl}-5-oxo-6,7-dihydro-5H-5λ⁴-thieno[3,2-d]pyrimidin-4-yl)-(tetrahydropyran-4-yl)-amin (Beispiel 182)

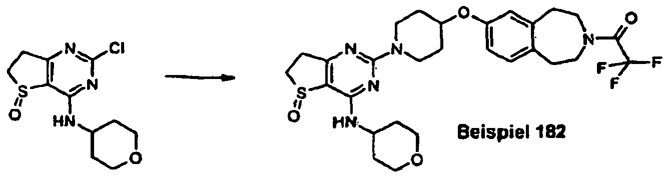

Ausgehend von **(IV-6)** (siehe 6.2) und **(V-5)** kann **Beispiel 182** analog zu Beispiel 89 (siehe 21.) hergestellt und aufgereinigt werden. Analytische HPLC-MS (Methode A): RT = 1,27 min.

### 39. SYNTHESE VON: {5-OXO-2-[4-(2,3,4,5-TETRAHYDRO-1H-BENZO[D]AZEPIN-7-YLOXY)-PIPERIDIN-1-YL]-6,7-DIHYDRO-5H-5λ⁴-THIENO[3,2-d]-PYRIMIDIN-4-YL} (TETRAHYDROPYRAN-4-YL)-AMIN (BEISPIEL 183)

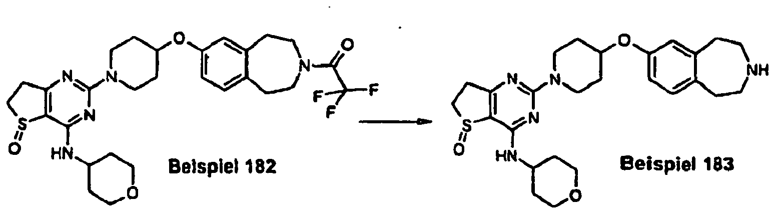

160 mg von **Beispiel 182** (siehe 38.3) werden in 5 ml Methanol vorgelegt, dann wird eine Mischung von 45 mg Kaliumcarbonat in 1 ml Wasser zugegeben. Das Reaktionsgemisch wird bei Raumtemperatur gerührt. Nach 24 Stunden wird das Methanol abrotiert. Der Rückstand wird mit Dichlormethan und Wasser versetzt. Die organische Phase wird getrocknet und zur Trockne eingedampft. 130 mg Beispiel 183 werden als Feststoff erhalten. Analytische HPLC-MS (Methode A): RT = 0,99 min.

### 40. SYNTHESE VON: 4-{1-[5-OXO-4-(TETRAHYDROPYRAN-4-YLAMINO)-6,7-DIHYDRO-5H-5λ⁴-THIENO[3,2-d]PYRIMIDIN-2-YL]-PIPERIDIN-4-YLOXY}-BENZOESÄURE (BEISPIEL 184)

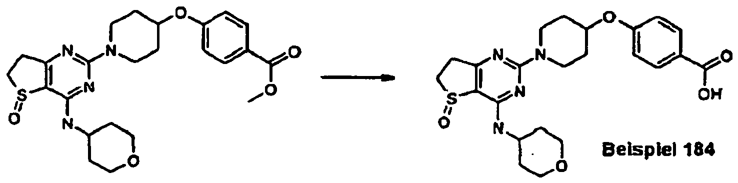

80 mg von **Beispiel 176 (siehe Tabelle D)** werden in 1,5 ml Methanol vorgelegt, dann 560 µl einer 1N NaOH Lösung zugegebenDas Reaktionsgemisch wird bei 50°C, bis keine weitere Umsetzung erfolgt, gerührt, dann mit einer 1 M HCl Lösung versetzt. Das Produkt wird mit Dichlormethan extrahiert. 77 mg **B**eispiel 184 werden als Feststoff erhalten. Analytische HPLC-MS (Methode B): RT = 1,19 min.

### 41. SYNTHESE VON 2-(1-{2-[4-(4-CHLORPHENYL)-PIPERIDIN-1-YL]-5-OXO-6,7-DIHYDRO-5H-5λ⁴-THIENO[3,2-d]PYRIMIDIN-4-YLAMINO}-CYCLOPROPYL)-PROPAN-2-OL (BEISPIEL 185)

### 41.1 2-[1-(2-Chlor-6,7-dihydrothieno[3,2-d]pyrimidin-4-ylamino)-cyclopropyl]-propan-2-ol (III-8):

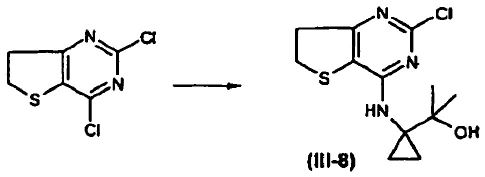

2,7 g (II) werden in 30 ml Dioxan vorgelegt, dann 6,8 ml Diisopropyl-ethylamin und 1,8 g 2-(1-Aminocyclopropyl)-propan-2-ol (siehe Liebigs Ann. Chem. 1978,1194) zugegeben. Das Reaktionsgemisch wird, bis keine weitere Umsetzung erfolgt, bei 160°C erhitzt und nach Abkühlen zur Trockne eingedampft. Der Rückstand wird mit Eiswasser versetzt. Das Produkt wird mit Dichlormethan extrahiert und chromatographisch gereinigt. 125 mg (III-8) werden als Feststoff erhalten. Analytische HPLC-MS (Methode A): RT = 1,08 min.

### 41.2 2-[1-(2-Chlor-5-oxo-6,7-dihydro-5H-5λ⁴-thieno[3,2-d]pyrimidin-4-ylamino)-cyclopropyl]-propan-2-ol (IV-8):

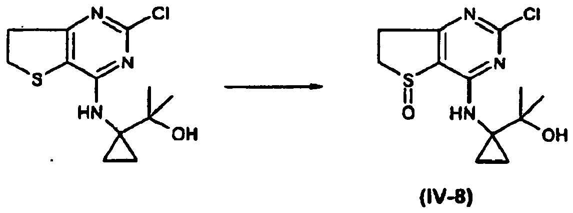

21,6 mg S-(-)-1,1'-Bi-2-naphtol werden in 1 ml Chloroform unter Argon vorgelegt, dann 11 µl Titan(IV)-isopropylat und 14 µl Wasser zugegeben. Das Reaktionsgemisch wird 1 Stunde bei Raumtemperatur gerührt. Anschließend wird eine Mischung von 120 mg (III-8) in 4 ml Dichlormethan zugegeben. Das Reaktionsgemisch wird auf -5°C abgekühlt und nach 30 Minuten werden 69,5 µl *tert*-Butylhydroperoxid 5-6 M in Decan zugetropft. Das Reaktionsgemisch wird bei -5 °C gerührt. Nach 2 Tage werden nochmal die gleichen Mengen von S-(-)-1,1'-Bi-2-naphtol, Ttan(IV)-isopropylat, Wasser und *tert*-Butylhydroperoxid zugegeben. Das Reaktionsgemisch wird bei -5°C bis 5°C weitergerührt bis keine weitere Umsetzung erfolgt, mit Wasser versetzt und mit NH₄OH basisch gestellt. Die organische Phase wird zur Trockne eingedampft und das Produkt chromatographisch (präparative HPLC, Methode B) gereinigt. 105 mg **(IV-8)** werden als erhalten. Analytische HPLC-MS (Methode A): RT = 0,96 min.

### 41.3 2-(1-{2-[4-(4-Chlorphenyl)-pipendin-1-yl]-5-oxo-6,7-dihydro-5H-5λ⁴-thieno[3,2-d]pyrimidin-4-ylamino}cyclopropyl)-propan-2-ol (Beispiel 185)

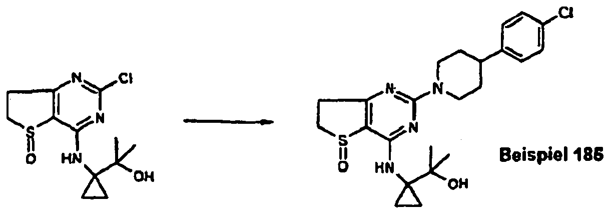

Ausgehend von **(IV-8)** und 4-(4-Chlorphenyl)-piperidin Hydrochlorid kann **Beispiel 185** analog zu Beispiel 89 (siehe 21.) hergestellt und aufgereinigt werden. Analytische HPLC-MS (Methode B): RT= 1,37 min.

### 42. SYNTHESE VON: {2-[4-(3-METHYL-2,3,4,5-TETRAHYDRO-1H-BENZO[d]AZEPIN-7-YLOXY)-PIPERIDIN-1-YL]-5-OXO-6,7-DIHYDRO-5H-5λ⁴-THIENO[3,2-d]PYRIMIDIN-4-YL}-(TETRAHYDROPYRAN-4-YL)-AMIN (BEISPIEL 186)

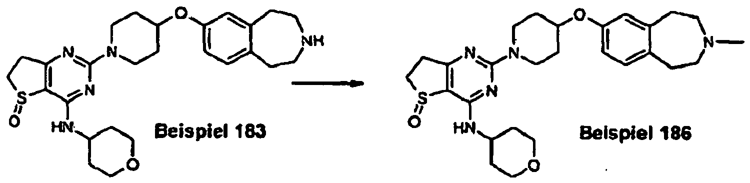

100 mg von **Beispiel 183** (siehe 39.) werden in 2 ml Methanol vorgelegt. Das pH der Mischung wird mit Essigsäure auf 6 gestellt. 34 µl einer wässerigen Formalinlösung werden dann zugegeben. Das Reaktionsgemisch wird 20 min bei Raumtemperatur gerührt, dann werden 50 mg Natriumtriacetoxyborhydrid langsam zugegeben. Das Reaktionsgemisch wird eine weitere Stunde bei Raumtemperatur gerührt, dann mit einer NaHCO₃ Lösung versetzt. Das Produkt wird mit Dichlormethan extrahiert und chromatographisch gereinigt. 56 mg **Beispiel 186** werden erhalten. Analytische HPLC-MS (Methode B): RT = 1,10 min.

### 43. SYNTHESE VON: {2-[4-(5-tert-BUTYL-1-METHYL-1H-INDOL-3-YL)-PIPERIDIN-1-YL]-5-OXO-6,7-DIHYDRO-5H-5λ⁴-THIENO[3,2-d]PYRIMIDIN-4-YL}-(TETRAHYDRO PYRAN-4-YL)-AMIN (BEISPIEL 192)

### 43.1 4-(1H-Indol-3-yl)-piperidin-1-carbonsäure-tert-butylester.

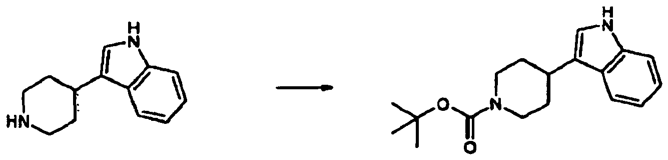

10 g 3-Piperidin-4-yl-1H-indol werden in 300 mL THF vorgelegt und 10,9 g Di-tert-butyl-dicarbonat zugegeben. Das Reaktionsgemisch wird über Nacht bei Raumtemperatur gerührt und zur Trockne eingedampft. Der Rückstand wird mit Wasser versetzt und das Produkt mit Diethylether extrahiert und chromatographisch gereinigt. 9 g des Produkts werden als Feststoff erhalten.

### 43.2 4-(1-Methyl-1H-indol-3-yl)-piperidin-1-carbonsäure-tert-butylester:

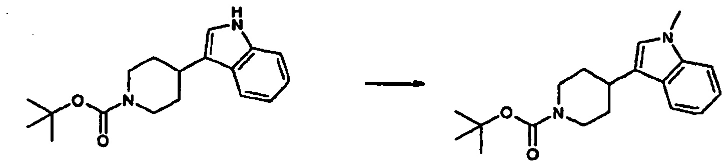

500 mg 4-(1H-Indol-3-yl)-piperidin-1-carbonsäure-*tert*-butylester werden in 8 ml Dimethylformamid vorgelegt und 73,3 mg Natriumhydrid. (60% in Mineralöl) zugegeben. Nach 15 min werden 175 µl Methyliodid zugegeben. Das Reaktionsgemisch wird bei Raumtemperatur gerührt. Nach vollständiger Umsetzung wird das Produkt direkt über präparative HPLC (Methode C) gereinigt. 302 mg des Produkts werden als Öl erhalten. Analytische HPLC-MS (Methode A): RT = 1,65 min.

### 43.3 5-tert-Butyl-1-methyl-3-piperidin-4-yl-1H-indol (V-6)

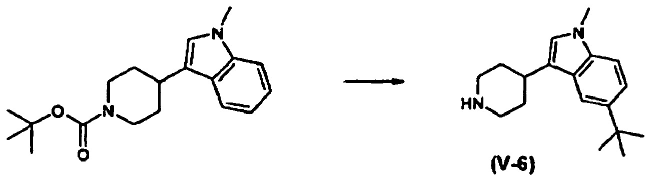

365 mg 4-(1-Methyl-1H-indol-3-yl)-piperidin-1-carbonsäure-*tert*-butylester werden in 1 ml Dichlormethan vorgelegt und mit 1,03 ml Trifluoressigsäure versetzt. Das Reaktionsgemisch wird bei Raumtemperatur gerührt. Nach 12 und 16 h werden nochmal 1,03 ml Trifluoressigsäure zugegeben. Nach weiteren 12 h wird das Reaktionsgemisch zur Trockne eingedampft. Der Rückstand wird mit Toluol versetzt und zur Trockne eingedampft. Der Rückstand wird mit Diethylether verrieben, der Niederschlag abgesaugt und getrocknet. 154 mg **(V-6)** werden als Feststoff erhalten. Analytische HPLC-MS (Methode A): RT = 1,34 min.

### 43.4 {2-[4-(5-tert-Butyl-1-methyl-1H-indol-3-yl)-piperidin-1-yl]-5-oxo-6,7-dihydro-5H-5λ⁴-thieno[3,2-d]pyrimidin-4-yl}-(tetrahydropyran-4-yl)-amin (Beispiel 192)

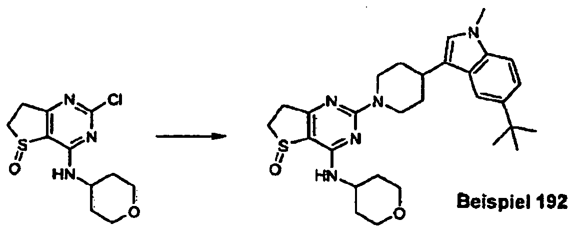

Ausgehend von **(IV-6)** (siehe 6.2) und **(V-6)** kann **Beispiel 192** analog zu Beispiel 89 (siehe 21.) hergestellt und aufgereinigt werden. Analytische HPLC-MS (Methode B): RT = 1,16 min.

### 44. SYNTHESE VON: 5-{2-[4-(6-CHLORBENZOXAZOL-2-YL)-PIPERIDIN-1-YL]-5-OXO-6,7-DIHYDRO-5H-5λ⁴-THIENO[3,2-d]PYRIMIDIN-4-YLAMINO}-1-METHYLPIPERIDIN-2-N (BEISPIEL 194)

### 44.1 6-Chlor-2-piperidin-4-yl-benzoxazol (V-7):

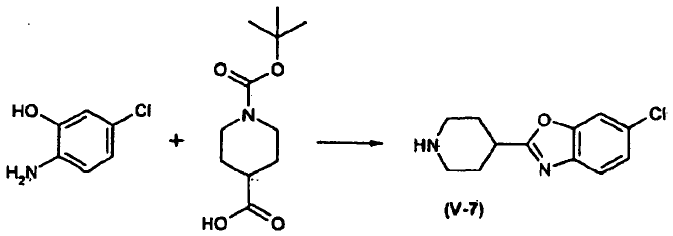

500 mg 2-Amino-5-chlorphenol und 800 mg Piperidin-1,4-dicarbonsäure-mono-tert-butylester werden in 4 ml Polyphosphorsäure bei 200°C für 4 h erhitzt. Nach dem Abkühlen wird das Reaktionsgemisch mit Eiswasser versetzt und 30 min gerührt. Der Niederschlag wird abgesaugt, mit Wasser gewaschen und getrocknet. 850 mg Produkt werden als Phosphat erhalten. Analytische HPLC-MS (Methode A): RT = 1,05 min.

### 44.2 5-{2-[4-(6-Chlorbenzoxazol-2-yl)-piperidin-1-yl]-5-oxo-6,7-dihydro-5H-5λ⁴-thieno[3,2-d]pyrimidin-4-ylamino}-1-methylpiperidin-2-on (Beispiel 194)

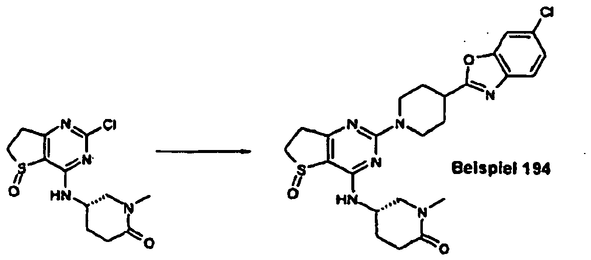

Ausgehend von **(IV-5)** (siehe 5.5) und **(V-7)** kann **Beispiel 194** analog zu Beispiel 89 (siehe 21.) hergestellt und aufgereinigt werden. Analytische HPLC-MS (Methode B): RT = 1,27 min.

### 45. SYNTHESE VON: 5-{2-[4-(5-FLUORBENZOXAZOL-2-YL)-PIPERIDIN-1-YL]-5-OXO-6,7-DIHYDRO-5H-5λ⁴-THIENO[3,2-d]PYRIMIDIN-4-YLAMINO}-1-METHYLPIPERIDIN-2-ON (BEISPIEL 195)

### 45.1 5-Fluor-2-piperidin-4-yl-benzoxazol (V-8):

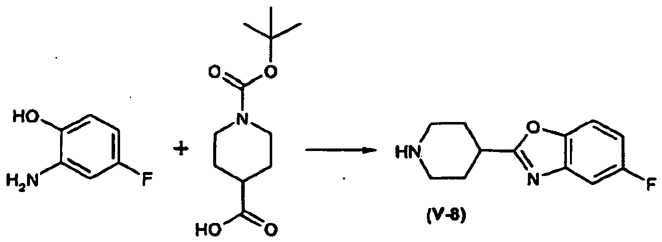

500 mg 2-Amino-4-fluorphenol und 900 mg Piperidin-1,4-dicarbonsäure-mono-*tert*-butylester werden in 5 g Polyphosphorsäure bei 200°C für 4 h erhitzt. Nach dem Abkühlen wird das Reaktionsgemisch mit Eiswasser versetzt und mit NaOH Lösung 50%ig basisch gestellt. Der Niederschlag wird abgesaugt, mit Wasser gewaschen und getrocknet 290 mg **(V.8)** werden als Feststoff erhalten.

### 45.2 5-{2-[4-(5-Fluorbenzoxazol-2-yl)-piperidin-1-yl]-5-oxo-6,7-dihydro-5H-5λ⁴-thieno[3,2-d]pyrimidin-4-ylamino}-1-methylpiperidin-2-on (Beispiel 195)

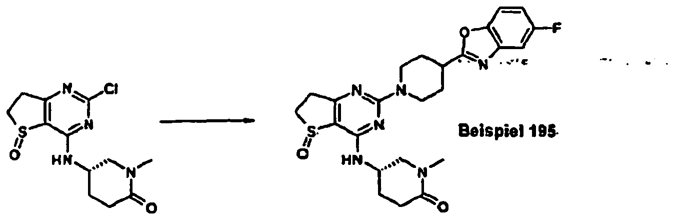

Ausgehend von **(IV-5)** (siehe 5.5) und **(V-8)** kann **Beispiel 195** analog zu Beispiel 89 (siehe 21.) hergestellt und aufgereinigt werden. Analytische HPLC-MS (Methode B): RT = 1,21 min.

### 46. SYNTHESE VON: 5-{2-[4-(5-FLUORBENZOXAZOL-2-YL)-4-METHYLPIPERIDIN-1-YL]-5-OXO-6,7-DIHYDRO-5H-5λ⁴-THIENO[3,2-d]PYRIMIDIN-4-YLAMINO}-1-METHYL PIPERIDIN-2-ON (BEISPIEL 197)

### 46.1 5-Fluor-2-(4-methylpiperidin-4-yl)-benzoxazol (V-9):

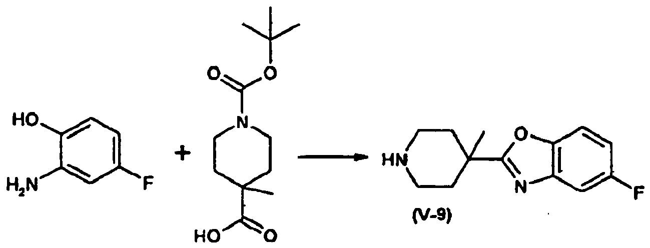

Ausgehend von 2-Amino-4-fluorphenol und 4-Methylpiperidin-1,4-dicarbonsäure-mono-*tert-*butylester kann **(V-9)** analog zu **(V-8)** (siehe 45.1) hergestellt und aufgereinigt werden.

### 46.2 5-{2-[4-(5-Fluorbenzoxazol-2-yl)-4-methylpiperidin-1-yl]-5-oxo-6,7-dihydro-5H-5λ⁴-thieno[3,2-d]pyrimidin-4-ylamino}-1-methylpiperidin-2-on (Beispiel 197)

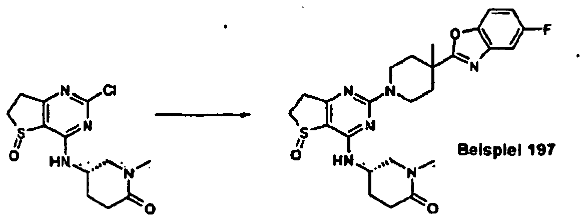

Ausgehend von **(IV-5)** (siehe 5.5) und **(V-9)** kann **Beispiel 197** analog zu Beispiel 89 (siehe 21.) hergestellt und aufgereinigt werden. Analytische HPLC-MS (Methode B): RT = 1,26 min.

### 47. SYNTHESE VON: {2-[4-(5-FURAN-2-YL-1-METHYL-1H-PYRAZOL-3-YL)-PIPERIDIN-1-YL]-5-OXO-6,7-DIHYDRO-5H-5λ⁴-THIENO[3,2-d]PYRYMIDIN-4-YL}-(TETRAHYDROPYRAN-4-YL)-AMIN (BEISPIEL 198)

### 47.1 14-(5-Furan-2-yl-2H-pyrazol-3-yl)-piperidin-1-carbonsäure-tert-butylester:

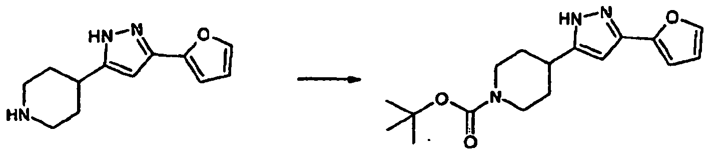

200 mg 4-(5-Furan-2-yl-2H-pyrazol-3-yl)-piperidin werden in 2 ml Dioxan vorgelegt Anschließend werden 0,34 ml Wasser und 155 mg Natriumcarbonat zugegeben. Das Reaktionsgemisch wird beim Raumtemperatur gerührt. Nach 5 min werden 204 mg Di-tert-butyl-dicarbonat zugegeben. Nach 3 h wird das Reaktionsgemisch mit Wasser versetzt und das Produkt mit Dichlormethan extrahiert. 300 mg Produkt werden als Öl erhalten. Analytisch HPLC-MS (Methode B): RT = 1,54 min.

### 47.2 4-(5-Furan-2-yl-2-methyl-2H-pyrazol-3-yl)-piperidin-1-carbonsäure-tert-butylester und 4-(5-Furan-2-yl-1-methyl-1H-pyrazol-3-yl)-piperidin-1-carbonsäure-tert-butytester.

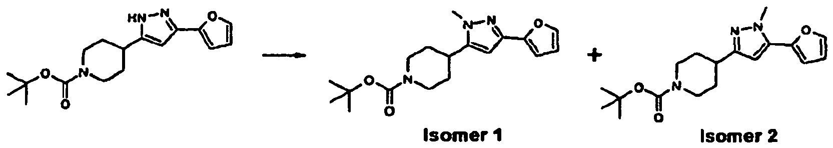

250 mg 14-(5-Furan-2-yl-2H-pyrazol-3-yl)-piperidin-1-carbonsäure-*tert*-butylester werden in 1,5 ml Dimethylformamid vorgelegt. Das Reaktionsgemisch wird im Eisbad abgekühlt und 40 mg Natriumhydrid (60% in Mineralöl) werden zugegeben. Nach 10 min werden 60 µl Methyliodid zugegeben. Das Reaktionsgemisch wird 30 min bei 5°C und anschliessend 4 h bei Raumtemperatur gerührt. Das Produkt wird dann direkt über präparative HPLC (Methode D) aufgereinigt. 90 mg **Isomer 1** und 50 mg **Isomer 2** werden als Feststoff erhalten. Analytische HPLC-MS (Methode D): RT = 1.33 min (Isomer 1); RT = 1,28 (Isomer 2).

### 47.3 4-(5-Furan-2-yl-1-methyl-1H-pyrazol-3-yl)-piperidin (V-10):

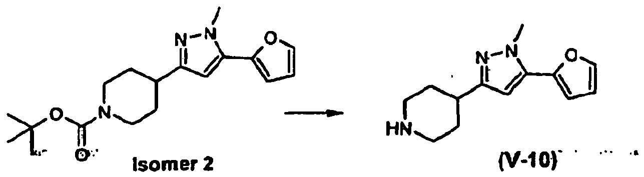

47 mg **Isomer 2** werden in 1 ml Dichlormethan vorgelegt und 120 µl Trifluoressigsäure zugegeben. Das Reaktionsgemisch wird 2h bei Raumtemperatur gerührt, dann zur Trockne eingedampft. Der Rückstand wird mit Toluol versetzt und zur Trockne eingedampft. Der Rückstand wird mit Wasser versetzt, mit konz. Ammoniak basisch gestellt und das Produkt mit Dichlormethan extrahiert. 23 mg **(V-10)** werden als Feststoff erhalten. Analytische HPLC-MS (Methode B): RT = 0,85 min

### 47.4 {2-[4-(5-Furan-2-yl-1-methyl-1H-pyrazol-3-yl)-piperidin-1-yl]-5-oxo-6,7-dihydro-5H-5λ⁴-thieno[3,2-d]pyrimidin-4-yl}-(tetrahydropyran-4-yl)-amin (Beispiel 198)

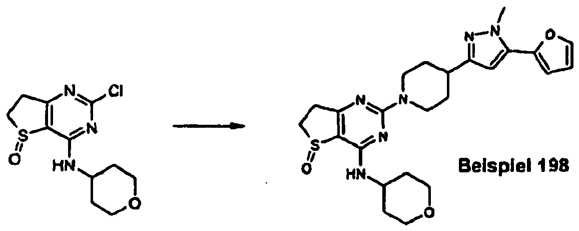

Ausgehend von **(IV-6)** (siehe 6.2) und **(V-10)** kann **Beispiel 198** analog zu Beispiel 89 (siehe 21) hergestellt und aufgereinigtwerden. Analytische HPLC-MS (Methode B): RT = 1,21 min.

### 48. SYNTHESE VON: {2-[4-(5-FURAN-2-YL-2-METHYL-2H-PYRAZOL-3-YL)-PIPERIDIN-1-YL]-5-OXO-6,7-DIHYDRO-5H-5λ⁴-THIENO[3,2-d]PYRIMIDIN-4-YL}-(TETRAHYDROPYRAN-4-YL)-AMIN (BEISPIEL 200)

### 48.1 4-(5-Furan-2-yl-2-methyl-2H-pyrazol-3-yl)-piperidin (V-11):

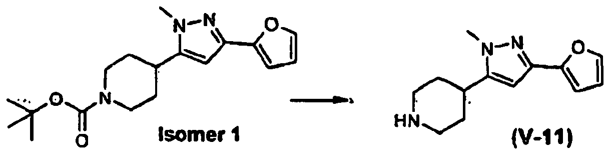

Ausgehend von **Isomer 1** (siehe 47.2), kann **(V-11)** analog zu **(V-10)** (siehe 47.3) hergestellt werden. Analytische HPLC-MS (Methode D): RT = 0,89 min.

### 48. 2 Synthese von: {2-[4-(5-Furan-2-yl-2-methyl-2H-pyrazol-3-yl)-piperidin-1-yl]-5-oxo-6,7-dihydro-5H-5λ⁴-thieno[3,2-d]pyrimidin-4-yl}-(tetrahydropyran-4-yl)-amin (Beispiel 200)

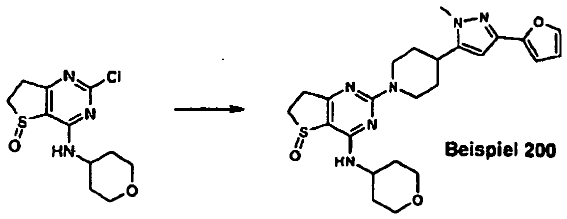

Ausgehend von **(IV-6)** (siehe 6.2) und **(V-11)** kann **Beispiel 200** analog zu Beispiel 89 (siehe 21) hergestellt und aufgereinigt werden. Analytische HPLC-MS (Methode B): RT = 1,26 min.

### 49. SYNTHESE VON 5-{2-[4-(4-CHLORPHENYL)-PIPERIDIN-1-YL]-5-OXO-6,7-DIHYDRO-5H-5λ⁴-THIENO[3,2-d]PYRIMIDIN-4-YLAMINO}-1-PROPYLPIPERIDIN-2-ON (BEISPIEL 201)

### 49.1 (S)-5-Dibenzylamino-1-propylpiperidin-2-on:

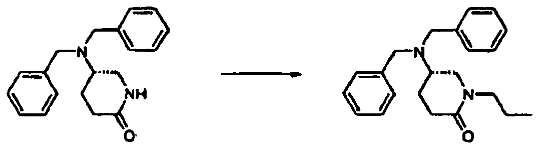

0,51 g (S)-5-Dibenzylaminopiperidin-2-on (siehe 5.1) werden in 5 mL Dimethylformamid vorgelegt. Unter Eisbadkühlung werden 120 mg Natriumhydrid (60% in Mineralöl) zugegeben. Das Reaktionsgemisch wird dann 30 Minuten bei Raumtemperatur gerührt. Unter Eisbadkühlung werden 0,289 ml 1-lodpropan zugegeben. Das Reaktionsgemisch wird dann über Nacht bei Raumtemperatur gerührt, anschließend mit einer gesättigten NaCl Lösung versetzt. Das Produkt wird mit Essigester extrahiert. 0,569 g Produkt werden als Öl erhalten. Analytische HPLC-MS (Methode A): RT = 1,13 min.

### 49.2 (S)-5-Amino-1-propylpiperidin-2-on:

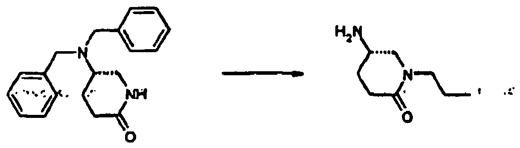

0,569 g (S)-5-Dibenzylamino-1-propylpiperidin-2-on werden in 25 ml Methanol vorgelegt und mit 0,150 g Pd/C 10% bei einem Druck von 3 bar und einer Temperatur von 60 °C hydriert. Nach 19 Stunden wird der Katalysator abgesaugt und das Filtrat zur Trockne eingedampft. 0,217 g des Produktes werden als Öl erhalten.

### 49.3 5-{2-[4-(4-Chlorphenyl)-piperidin-1-yl]-5-oxo-6,7-dihydro-5H-5λ⁴-thieno[3,2-d]pyrimidin-4-ylamino}-1-propylpiperidin-2-on (Beispiel 201)

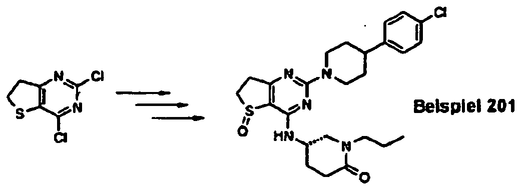

Ausgehend von (II), (S)-5-Amino-1-propylpiperidin-2-on (siehe 49.2) und 4-(4-Chlorphenyl)-piperidin Hydrochlorid kann **Beispiel 201** analog zu Beispiel 5 (siehe 5.4 bis 5.6) hergestellt werden. Das Produkt kann über präparative HPLC (Methode B) aufgereinigt werden. Analytische HPLC-MS (Methode B): RT = 1,36 min.

### 50. SYNTHESE VON: 2-METHOXY-N-{1-[4-(1-METHYL-6-OXOPIPERIDIN-3-YLAMINO)-5-OXO-6,7-DIHYDRO-5H-5λ⁴THIENO[3,2-d]PYRIMIDIN-2-YL]-4-PHENYL PIPERIDIN-4-YLMETHYL}-ACETAMID (BEISPIEL 202)

### 50.1 4-[(2-Methoxyacetylamino)-methyl]-4-phenylpiperidin-1-carbonsäure-tert-butylester.

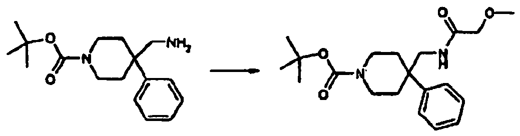

3,7 g des käuflichen 4-Aminomethyl-4-phenyl-piperidin-1-carbonsäure-*tert*-butylester und 3 ml Diisopropylethylamin werden in 30 ml Dichlormethan vorgelegt. Anschließend werden 2,25 ml Methoxyacetylchlorid langsam zugegeben. Das Reaktionsgemisch wird bei Raumtemperatur, bis keine weitere Umsetzung erfolgt, gerührt, dann mit Wasser versetzt. Die organische Phase wird zur Trockne eingedampft. 4,7 g Produkt werden als Öl erhalten.

### 50.2 2-Methoxy-N-(4-phenylpiperidin-4-ylmethyl)-acetamid (V-12):

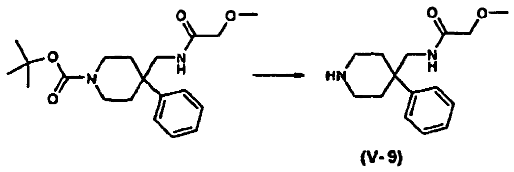

1 g 4-[(2-Methoxyacetylamino)-methyl]-4-phenylpiperidin-1-carbonsäure-*tert*-butylester werden in 4 ml Dichlormethan vorgelegt. Anschließend werden 1,7 ml Trifluoressigsäure zugegeben und über Nacht bei Raumtemperatur gerührt. Das Reaktionsgemisch wird mit Kaliumcarbonat basisch gestellt und die organische Phase zur Trockne eingedampft. 610 mg **(V-12)** werden als Öl erhalten.

### 50. 3 Synthese von: 2-Methoxy-N-{1-[4-(1-methyl-6-oxopiperidin-3-ylamino)-5-oxo-6,7-dihydro-5H-5λ⁴-thieno[3,2-d]pyrimidin-2-yl]-4-phenylpiperidin-4-ylmethyl}-acetamid (Beispiel 202)

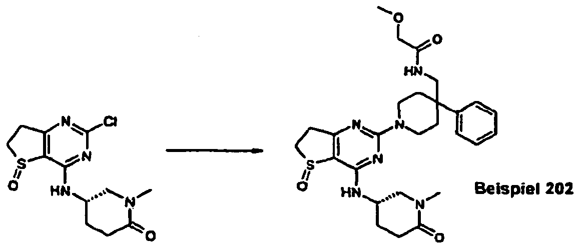

Ausgehend von **(IV-5)** (siehe 5.5) und **(V-12)** kann **Beispiel 202** analog zu Beispiel 89 (siehe 21.) hergestellt und aufgereinigt werden. Analytische HPLC-MS (Methode B): RT = 1.16 min.

### 51. SYNTHESE VON: 5-{2-[4-(4-FLUORBENZOYL)-4-(4-FLUORPHENYL)-PIPERIDIN-1-YL]-5-OXO-6,7-DIHYDRO-5H-5λ⁴-THIENO[3,2-d]PYRIMIDIN-4-YLAMINO}-1-METHYL PIPERIDIN-2-ON (BEISPIEL 203)

### 51.1 1-Benzyl-4-(4-fluorphenyl)-piperidin-4-carbonitril:

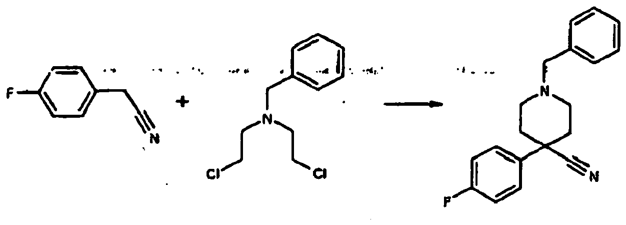

Unter Argon werden 16 ml 4-Fluorbenzylcyanid und 35,1g N-Benzyl-N,N-Di-(2-Chlorethyl)amin Hydrochlorid in 500 ml NMP vorgelegt und auf 5°C abgekühlt. Anschließend werden 18,9 g Natriumhydrid (55%ig in Mineralöl) portionsweise Innerhalb von 30 min zugegeben. Das Reaktionsgemisch wird 30 min bei 5-10°C und 6 h bei Raumtemperatur gerührt und anschliessend auf Eiswasser gegossen. Das Produkt wird mit Essigester extrahiert und chromatographisch gereinigt (Kieselgel, Dichlormethan/Ethanol 100:1 bis 50:1). 33 g Produkt werden als Öl erhalten.

### 51.2 [1-Benzyl-4-(4-fluorphenyl)-piperidin-4-yl]-(4-fluorphenyl)-methanon:

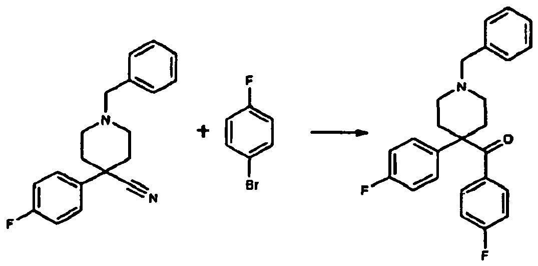

Unter Argon werden 11,86 g Magnesiumspäne in 50 ml wasserfreien Diethylether vorgelegt, dann wird eine Lösung von 85,4 g 4-Bromfluorbenzol in 200 ml wasserfreien Diethylether langsam zugetropft. Das Reaktionsgemisch wird 2 h unter Rückfluß gerührt und danach auf Raumtemperatur abgefühlt. Eine Lösung von 45,4 g 1-Benzyl-4-(4-fluor-phenyl)-piperidin-4-carbonitril in 100 ml wasserfreien Toluol wird zugetropft Der Diethylether wird abdestilliert und das restliche Reaktionsgemisch über Nacht bei 80°C gerührt. Nach dem Abkühlen wird das Reaktionsgemisch mit 500 ml Eiswasser und 100 g NH₄Cl versetzt und das Produkt mit Essigester extrahiert. Die organische Phase wird mit Wasser und ges. NaCl Lösung gewaschen, getrocknet und zur Trockne eingedampft. Zu dem Rückstand werden 70 ml Eisessig und 20 ml Schwefelsäure 33%ig gegeben. Das Reaktionsgemisch wird auf 100°C erhitzt, dann abgekühlt, mit Eiswasser versetzt und mit 4N NaOH auf pH ~ 9 gestellt Anschließend werden 250 ml Diisopropylether zugegeben. Das Reaktionsgemisch wird dann über Nacht bei Raumtemperatur gerührt. Der ausgefallene Niederschlag wird abgesaugt, mit Diisopropylether und Wasser gewaschen und getrocknet. 42,7 g Produkt werden als Feststoff erhalten. Smp: 136-138,5°C.

### 51.3 (4-Fluorphenyl)-[4-(4-fluorphenyl)-piperidin-4-yl]-methanon (V-13):

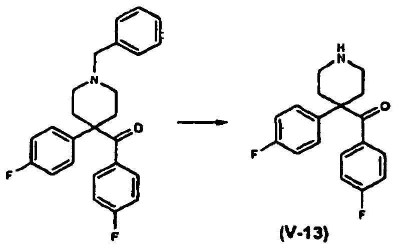

42,6 g [1-Benzyl-4-(4-fluor-phenyl)-piperidin-4-yl]-(4-fluor-phenyl)-methanon werden in 400 ml Methanol und 15 ml etherischer Salzsäure 10 mol/l vorgelegt und mit 8 g Pd/C 5% bei 30°C und 50 psi Wasserstoffdruck über Nacht hydriert. Der Katalysator wird abfiltriert und das Filtrat zur Trockne eingedampft. Der Rückstand wird mit tert-Butylmethylether verrührt, der Feststoff abgesaugt, mit *tert*-Butylmethylether gewaschen und getrocknet. 35,08 g **(V-13)** werden als Hydrochlorid erhalten. Smp: 149-151°C.

### 51.4 5-{2-[4-(4-Fluorbenzoyl)-4-(4-fluorphenyl)-piperidin-1-yl]-5-oxo-6,7-dihydro-5H-5λ⁴-thieno[3,2-d]pyrimidin-4-ylamino}-1-methylpiperidin-2-on (Beispiel 203)

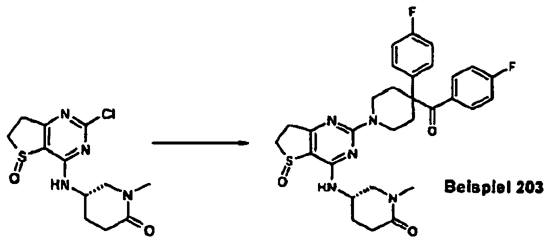

Ausgehend von **(IV-5)** (siehe 5.5) und **(V-13)** kann **Beispiel 203** analog zu Beispiel 89 (siehe 21.) hergestellt und aufgereinigt werden. Analytische HPLC-MS (Methode B): RT = 1,38 min.

### 52. SYNTHESE VON: N-{1-[4-(1-METHYL-6-OXOPIPERIDIN-3-YLAMINO)-5-OXO-6,7-DIHYDRO-5H-5λ⁴-THIENO[3,2-d]PYRIMIDIN-2-YL]-4-PHENYLPIPERIDIN-4-YLMETHYL}-ACETAMID (Beispiel 204)

### 52.1 4-(Acetylaminomethyl)-4-phenylpiperidin-1-carbonsäure-tert-butylester:

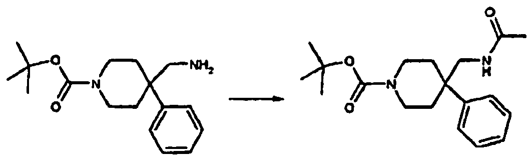

8 g 4-Aminomethyl-4-phenylpiperidin-1-carbonsäure-*tert*-butylester und 2,9 ml Essigsäureanhydrid werden in 80 ml Ethanol über Nacht bei Raumtemperatur gerührt und anschließend zur Trockne eingedampft. 10,4 g Produkt werden erhalten.

### 52.2 N-(4-Phenylpiperidin-4-ylmethyl)-acetamid (V-14):

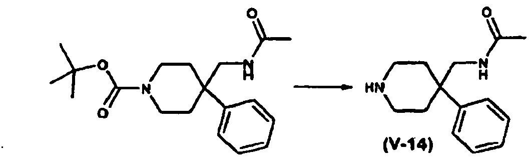

11,5 g 4-(Acetylaminomethyl)-4-phenylpiperidin-1-carbonsäure-*tert*-butylester und 25 ml Trifluoressigsäure werden in 200 ml Dichlormethan über Nacht bei Raumtemperatur gerührt. Das Reaktionsgemisch wird zur Trockne eingedampft und der Rückstand mit Diethylether/Diisopropylether verrieben. Der ausgefallene Feststoff wird abgesaugt und mit Diethylether gewaschen. 10 g **(V-14)** werden als Trifluoracetat erhalten.

### 52.3 N-{1-[4-(1-Methyl-6-oxopiperidin-3-ylamino)-5-oxo-6,7-dihydro-5H-5λ⁴-thieno[3,2-d]pyrimidin-2-yl]-4-phenylpiperidin-4-ylmethyl}-acetamid (Beispiel 204)

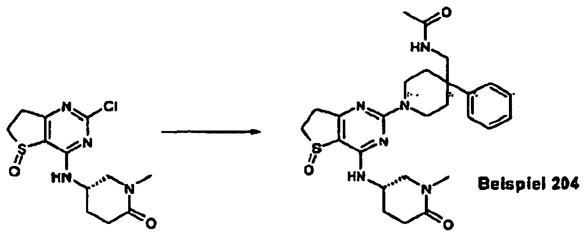

Ausgehend von **(IV-5)** (siehe 5.5) und **(V-14)** kann **Beispiel 204** analog zu Beispiel 89 (siehe 21.) hergestellt und aufgereinigt werden. Analytische HPLC-MS (Methode B): RT = 1,11 min.

### 53. SYNTHESE VON: 1-[4-(1-METHYL-6-OXOPIPERIDIN-3-YLAMINO)-5-OXO-6,7-DIHYDRO-5H-5λ⁴-THIENO[3,2-d]PYRIMIDIN-2-YL]-4-PHENYLPIPERIDIN-4-CARBONSÄUREMETHYLESTER (BEISPIEL 205)

### 53.1 4-Phenylpiperidin-4-carbonsäuremethylester (V-15):

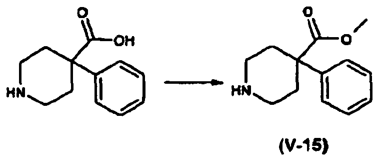

270 ml Methanol werden vorgelegt. Unter Rühren werden 10,6 ml Schwefelsäure und 25 g 4-Phenyl-4-piperidin-carbonsäure *p*-Toluolsulfonsäure zugegeben. Das Reaktionsgemisch wird 9 h unter Rückfluss gekocht, abgekühlt und vorsichtig auf eine Mischung aus Eiswasser und 10 M NaOH zugegeben. Der ausgefallene Feststoff wird abgesaugt, mit Wasser gewaschen und getrocknet. 11,2 g **(V-15)** werden als Feststoff erhalten.

### 53. 2 1-[4-(1-Methyl-6-oxopiperidin-3-ylamino)-5-oxo-6,7-dihydro-5H-5λ⁴-thieno[3,2-d]pyrimidin-2-yl]-4-phenylpiperidin-4-carbonsäuremethylester (Beispiel 205)

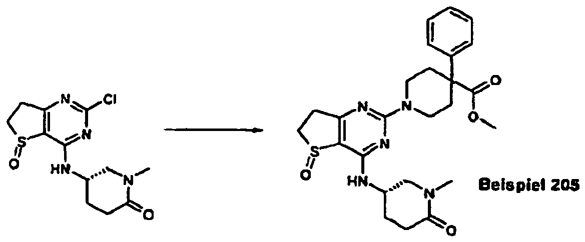

Ausgehend von **(IV-5)** (siehe 5.5) und **(V-15)** kann **Beispiel 205** analog zu Beispiel 89 (siehe 21.) hergestellt und aufgereinigt werden. Analytische HPLC-MS (Methode B): RT = 1,23 min.

### 54. SYNTHESE VON: 2-DIMETHYLAMINO-N-{1-[4-(1-METHYL-6-OXOPIPERIDIN-3-YLAMINO)-5-OXO-6,7-DIHYDRO-5H-5λ⁴-THIENO[3,2-d]PYRIMIDIN-2-YL]-4-PHENYL PIPERIDIN-4-YLMETHYL}-ACETAMID (BEISPIEL 206)

### 54.1 2-Dimethylamino-N-[4-(1-propenylbuta-1,3-dienyl)-piperidin-4-ylmethyl]-acetamid (V-16):

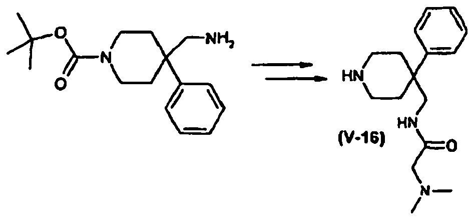

Ausgehend von den käuflichen 4-Aminomethyl-4-phenyl-piperidin-1-carbonsäure-*tert-*butylester und Dimethylaminoacetylchlorid Hydrochlorid kann **(V-16)** analog zu **(V-12)** (siehe 50.1 und 50:2) hergestellt werden.

### 48.2 2-Dimethylamino-N-{1-[4-(1-methyl-6-oxopiperidin-3-ylamino)-5-oxo-6,7-dihydro-5H-5λ⁴-thieno[3,2-d]pyrimidin-2-yl]-4-phenylpiperidin-4-ylmethyl}-acetamid (Beispiel 206)

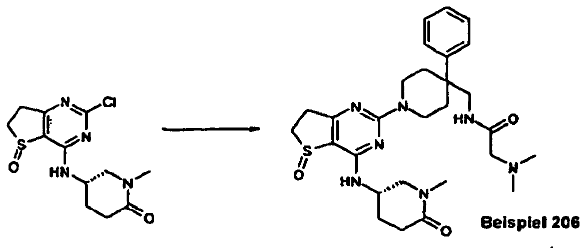

Ausgehend von **(IV-5)** (siehe 5.5) und **(V-16)** kann **Beispiel 206** analog zu Beispiel 89 (siehe 21.) hergestellt und aufgereinigt werden. Analytische HPLC-MS (Methode B): RT = 1,08 min.

### 55. SYNTHESE VON: {2-[4-(1-METHYL-1H-IMIDAZO[4,5-c]PYRIDIN-2-YL)-PIPERIDIN-1-YL]-5-OXO-6,7-DIHYDRO-5H-5λ⁴-THIENO[3,2-d]PYRIMIDIN-4-YL}-(TETRAHYDRO PYRAN-4-YL)-AMIN (BEISPIEL 210)

### 55.1 Methyl-(3-nitropyridin-4-yl)-amin:

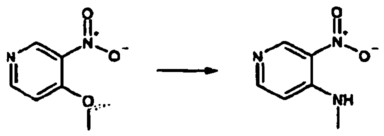

2,36 g 4-Methoxy-3-nitro-pyridin und 2,33 ml Methylamin (40%ig in Wasser) werden in 25 ml Ethanol 3 h unter Rückfluss gekocht. Anschließend wird das Reaktionsgemisch zur Trockne eingedampft. 2,3 g Produkt werden als Feststoff erhalten.

### 55.2 N⁴-Methylpyridin-3,4-diamin:

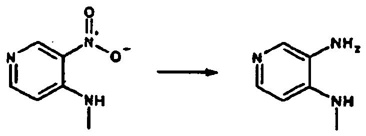

2,3 g Methyl-(3-nitropyridin-4-yl)-amin werden in 50 ml Methanol für und mit 0,8 g Raney-Nickel 2,5 h bei 50°C und 50 psi Wasserstoffdruck hydriert. Der Katalysator wird abfiltriert und das Filtrat zur Trockne eingedampft. Das Produkt wird chromatographisch (Alox, Dichlormethan/methanol von 99/1 bis 19/1) gereinigt. 1,55 g Produkt werden als Feststoff erhalten. Smp: 163-165°C.

### 55.3 1-Methyl-2-piperidin-4-yl-1H-imidazo[4,5-c]pyridin (V-17):

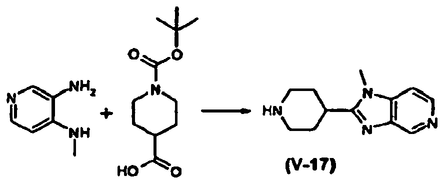

450 mg *N*⁴-Methylpyridin-3,4-diamin und 838 mg Piperidin-1,4-Dicarbonsäure-mono-*tert-*butylester werden in 8,6 g Polyphosphorsäure für 4 h bei 200°C erhitzt. Nach dem Abkühlen wird mit 4 N Na.OH basisch gestellt und mit Trifluoressigsäure angesäuert. Das Gemisch wird über (präparative HPLC (Methode C) aufgereinigt. 3,37 g (50%ig) **(V-17)** werden als Trifluoracetat erhalten. Analytische HPLC-MS (Methode B): RT = 0,30 min.

### 55.4 {2-[4-(1-Methyl-1H-imidazo[4,5-c]pyridin-2-yl)-piperidin-1-yl]-5-oxo-6,7-dihydro-5H-5λ⁴-thieno[3,2-d]pyrimidin-4-yl}-(tetrahydropyran-4-yl)-amin (Beispiel 210)

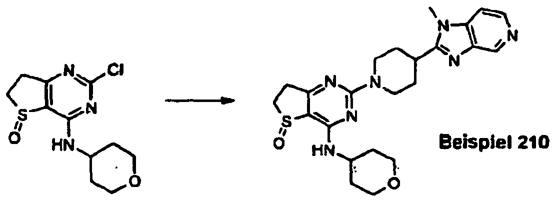

Ausgehend von **(N-6)** (siehe 6.2) und **(V-17)** kann **Beispiel 210** analog zu Beispiel 89 (siehe 21) hergestellt und aufgereinigt werden. Analytische HPLC-MS (Methode D): RT = 0,86 min.

### 56. SYNTHESE VON 5-{2-[4-(4-CHLORPHENYL)-PIPERIDIN-1-YL]-5-OXO-6,7-DIHYDRO-5H-5λ⁴-THIENO[3,2-d]PYRIMIDIN-4-YLAMINO}-1-(4-FLUORBENZYL)-PIPERIDIN-2-ON(BEISPIEL 211)

### 56.1 (S)-5-Dibenzylamino-1-(4-fluorbenzyl)-piperidin-2-on:

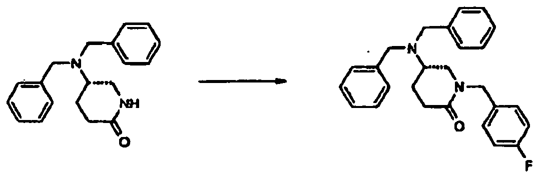

0,8 g (S)-5-Dibenzylaminopiperidin-2-on (siehe 5.1) werden in 8 ml Dimethylformamid vorgelegt, dann 200 mg Natriumhydrid (60% in Mineralöl) und 0,4 ml 4-Fluorbenzylbromid zugegeben. Das Reaktionsgemisch wird über Nacht bei 70°C gerührt und anschließend mit Eiswasser versetzt. Der Niederschlag wird abfiltriert und mit Wasser gewaschen. Das Produkt wird chromatographisch (Kieselgel, Petrolether/Essigester und Essigester/Methanol) gereinigt. 0,5 g Produkt werden als Öl erhalten. Analytische HPLC-MS (Methode A): RT = 1,21 min.

### 56.2 (S)-5-Amino-1-(4-fluor-benzyl)-piperidin-2-on:

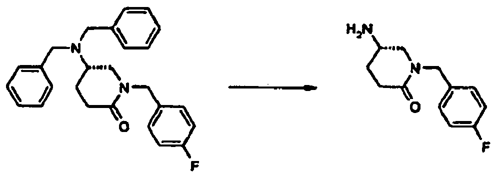

0;5 g (S)-5-Dibenzylamine-1-(4-fluorbenzyl)-piperidin-2-on werden in 20 ml Methanol vorgelegt und mit 0,150 g Pd/C 10% bei einem Druck von 3 bar und einer Temperatur von 60 °C hydriert. Nach 5 Stunden wird der Katalysator abgesaugt und das Filtrat zur Trockne eingedampft. 0,21 g des Produktes werden als Öl erhalten. Analytische HPLC-MS (Methode A): RT = 0,68 min.

### 56.3 5-{2-[4-(4-Chlorphenyl)-piperidin-1-yl]-5-oxo-6,7-dihydro-5H-5λ⁴-thieno[3,2-d]pyrimidin-4-ylamino}-1-(4-fluorbenzyl)-piperidin-2-on (Beispiel 211)

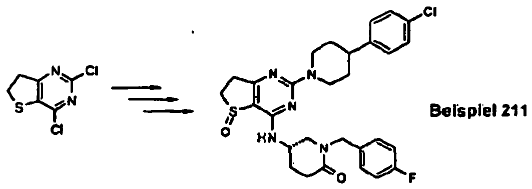

Ausgehend von **(II)**, (S)-5-Amino-1-(4-fluorbenzyl)-piperidin-2-on (siehe 56.2) und 4-(4-Chlorphenyl)-piperidin Hydrochlorid kann **Beispiel 211** analog zu Beispiel 5 (siehe 5.4 bis 5.6) hergestellt werden. Das Produkt kann über präparative HPLC (Methode A) aufgereinigt werden. Analytische HPLC-MS (Methode B): RT = 1,45 min.

### 57. SYNTHESE VON: CYCLOPROPYL-(7-{1-[5-OXO-4-(TETRAHYDROPYRAN-4-YLAMINO)-6,7-DIHYDRO-5H-5λ⁴-THIENO[3,2-d]PYRIMIDIN-2-YL]PIPERIDIN-4-YLOXY}-1,2,4,5-TETRAHYDROBENZO[d]AZEPIN-3-YL)-METHANON (BEISPIEL 214)

### 57.1 4-(2,3,4,5-Tetrahydro-1H-benzo[d]azepin-7-yloxy)-piperidin-1-carbonsäure-tert-butylester:

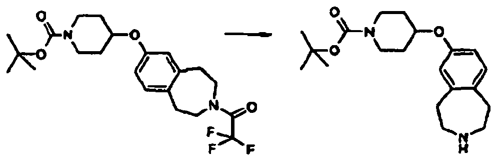

400 mg 4-[3-(2,2,2-Trifluoracetyl)-2,3,4,5-tetrahydro-1H-benzo[d]azepin-7-yloxy]-piperidin-1-carbonsäure-*tert*-butylester (siehe 38.2) werden in 17 ml Methanol vorgelegt, dann wird eine Mischung von 151,2 mg Kaliumcarbonat in 3,3 ml Wasser zugegeben. Das Reaktionsgemisch wird bei Raumtemperatur, bis keine weitere Reaktion erfolgt, gerührt. Das Methanol wird dann abrotiert. Der Rückstand wird mit Dichlormethan und Wasser versetzt. Die organische Phase wird getrocknet und zur Trockne eingedampft. 310 mg werden als Öl erhalten. Analytische HPLC-MS (Methode D): RT =1,25 min.

### 57.2 4-(3-Cyclopropancarbonyl-2,3,4,5-tetrahydro-1H-benzo[d]azepin-7-yloxy)-piperidin-1-carbonsäure-tert-butylester.

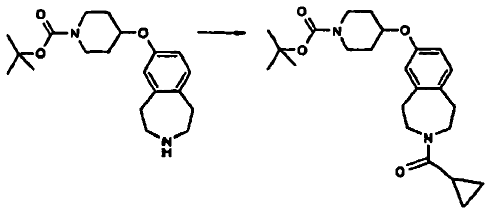

16 µl Cyclopropylcarbonsäure werden in 3 ml Dimethylformamid vorgelegt, dann 174 µl Diisopropylethylamin und 93,1 mg O-(7-Azabenzotriazol-1-yl-)-N,N,N'nN'-tetramethyluroniumhexafluorphosphat (HATU) zugegeben. Nach 15 min werden 77,5 mg 4-(2,3,4,5-Tetrahydro-1H-benzo[d]azepin-7-yloxy)-piperidin-1-carbonsäure-tert-butylester zugegeben. Das Reaktionsgemisch wird bei Raumtemperatur, bis keine weitere Reaktion erfolgt, gerührt und das Produkt direkt über präparative HPLC (Methode B) gereinigt. 70 mg des Produkts werden als Feststoff erhalten. Analytische HPLC-MS (Methode D): RT = 1,37 min.

### 57.3 Cyclopropyl-[7-(piperidin-4-yloxy)-1,2,4,5-tetrahydro-benzo[d]azepin-3-yl]-methanon (V-18):

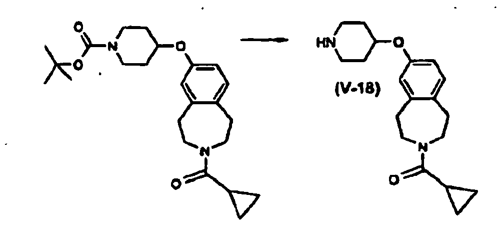

70 mg 4-(3-Cyclopropancarbonyl-2,3,4,5-tetrahydro-1H-benzo[d]azepin-7-yloxy)-pipendin-1-carbonsäure-tert-butylester werden in 1,4 ml Dichlormethan vorgelegt und mit 224 µl Trifluoressigsäure versetzt. Das Reaktionsgemisch wird 3 Stunden bei Raumtemperatur gerührt, dann zur Trockne eingedampft. Der Rückstand wird mit Toluol versetzt und zur Trockne eingedampft. 77 mg **(V-18)** werden als Öl erhalten. Analytische HPLC-MS (Methode B): RT = 1,18 min.

### 57.4 Cyclopropyl-(7-{1-[5-oxo-4-(tetrahydropyran-4-ylamino)-6,7-dihydro-5H-5λ⁴-thieno[3,2-d]pyrimidin-2-yl]-piperidin-4-yloxy}-1,2,4,5-tetrahydrobenzo[d]azepin-3-yl)-methanon (Beispiel 214)

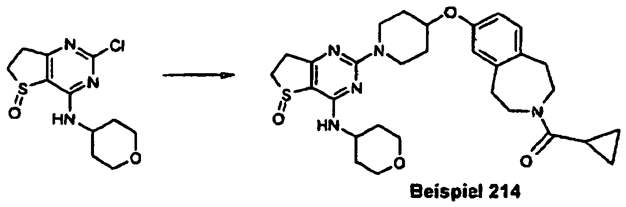

Ausgehend von **(IV-6)** (siehe 6.2) und **(V-18)** kann **Beispiel 214** analog zu Beispiel 89 (siehe 21.) hergestellt und aufgereinigt werden. Analytische HPLC-MS (Methode D): RT = 1,10 min.

### 58. SYNTHESE VON (2-{2-[4-(4-CHLORPHENYL)-PIPERIDIN-1-YL]-5-OXO-6,7-DIHYDRO-5H-5λ⁴-THIENO[3,2-d]PYRIMIDIN-4-YLAMINO}-CYCLOPROPYL)-CARBAMIDSÄURE-tert-BUTYLESTER (BEISPIEL 222)

### 58.1 Dihydrazid cis-1,2-cyclopropandicarbonsäure:

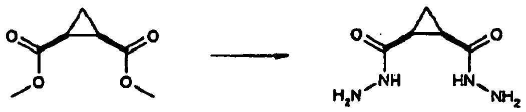

10 g Dimethyl *cis*-1,2-cyclopropandicarboxylat werden in 100 ml Ethanol vorgelegt und 12,7 ml Hydrazinmonohydrat zugegeben. Das Reaktionsgemisch wird 12 h unter Rückfluß gerührt. Nach dem Abkühlen wird der ausgefallene Feststoff filtriert, mit Petrolether und Diethylether gewaschen und getrocknet. 8 g (80%ig) Produkt werden als Feststoff erhalten.

### 58.2 cis-1,2-Cyclopropandiamin:

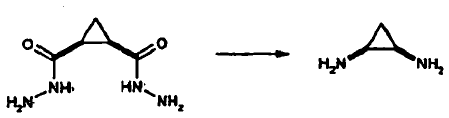

2 g Dihydrazid *cis*-1,2-cyclopropandicarbonsäure werden in 35 ml Diethylether vorgelegt, dann 14,2 ml konz. Salzsäure in 28 g Eis zugegeben. Das Reaktionsgemisch wird auf 0-5°C abgekühlt und anschliessend eine Lösung von 5,45 g Natriumnitrit in Wasser langsam zugetropft. Nach 20 min wird die organische Phase abgetrennt und getrocknet. 50 ml Toluol werden zugegeben und der Ether abdestilliert. Die verbliebene Toluollösung wird solange bei 80-90°C erhitzt bis die Stickstoff Entwicklung aufhört. Die heiße Toluollösung wird vorsichtig auf heiße (60°C) konz. Salzsäure gegossen und das Toluol abdestilliert. Wasserfreies Ethanol wird zugegeben und wieder abdistilliert bis ein Feststoff erhalten wird. Der Feststoff wird mit kaltem Ethanol versetzt und abfiltriert. 1,25 g Produkt werden als Dihydrochlorid erhalten. Smp: 225°C (Zersetzung).

### 58.3 cis-(2-tert-Butoxycarbonylaminocyclopropyl)-carbamidsäure-tert-butylester:

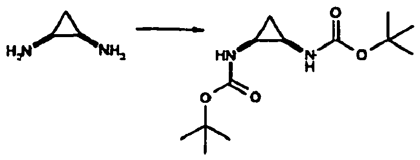

5 g *cis*-1,2-Cyclopropandiamin Dihydrochlorid wird in 50 ml Dioxan vorgelegt, auf 0°C gekühlt und anschließend mit 13,8 g 5 N Natronlauge und 22,55 g Di-*tert*-butyl-dicarbonat versetzt. Das Reaktionsgemisch wird 3 h bei Raumtemperatur gerührt und das Produkt mit Dichlormethan extrahiert. 6,3 g Produkt werden als Feststoff erhalten. Smp: 131-132°C.

### 58.4 cis-N-tert-Butyloxycarbonyl-1,2-cyclopropandiamin:

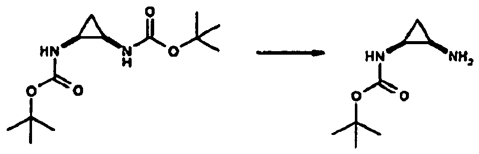

5 g *cis*-(2-*tert*-Butoxycarbonylaminocyclopropyl)-carbamidsäure-tert-butylester werden in 50 ml Essigester vorgelegt und auf 0°C abgekühlt. Eine Lösung von 0,87 g Salzsäure in 9,5 ml Essigester wird zugetropft. Anschließend wird das Reaktionsgemisch über Nacht bei Raumtemperatur gerührt. Der ausgefallene Feststoff wird abfiltriert und mit Essigester gewaschen. 0,76 g Produkt werden als Hydrochlorid erhalten. Smp: 208-209°C.

### 58.5 [2-(2-Chlor-6,7-dihydrothieno[3,2-d]pyrimidin-4-ylamino)-cyclopropyl]-carbamidsäure-tert-butylester (III-9):

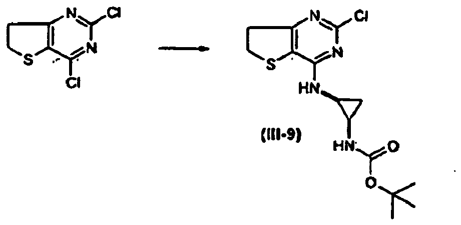

0,55 g (II) werden in 9 ml Dioxan vorgelegt, anschließend werden 1,4 ml Diisopropyl ethylamin und 0,6 g *cis*-N-*tert*-Butyloxycarbonyl-1,2-cyclopropandiamin Hydrochlorid (siehe 58.4) zugegeben. Das Reaktionsgemisch wird bei 110°C in der Mikrowelle erhitzt, bis keine weitere Umsetzung erfolgt und nach dem Abkühlen zur Trockne eingedampft. Der Rückstand wird mit Wasser im Ultraschallbad behandelt, der Niederschlag abgesaugt und mit Wasser gewaschen. Der Feststoff wird mit 10 ml Petrolether/Essigester = 7/3 behandelt und abgesaugt. 520 mg **(III-9)** werden als Feststoff erhalten. Analytische HPLC-MS (Methode B): RT = 1,42 min.

### 58.6 [2-(2-Chlor-5-oxo-6,7-dihydro-5H-5λ⁴-thieno[3,2-d]pyrimidin-4-ylamino)-cyclopropyl]-carbamidsäure-tert-butylester (IV-9):

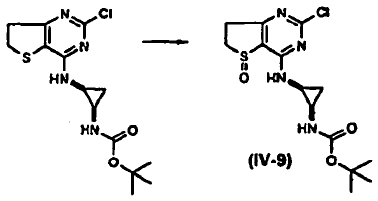

73 mg S-(-)-1,1'-Bi-2-naphtol werden in 2 ml Chloroform unter Argon vorgelegt, dann 38 µl Titan(IV)-isopropylat und 47 µl Wasser zugegeben. Das Reaktionsgemisch wird 1 Stunde bei Raumtemperatur gerührt. Anschließend wird eine Mischung von 480 mg **(III-9)** in 6 ml Chloroform zugegeben. Das Reaktionsgemisch wird auf -5°C abgekühlt und nach 60 Minuten werden 232 µl tert-Butylhydroperoxid 5-6 M in Decan zugetropft. Das Reaktionsgemisch wird 24 h bei -5 °C gerührt und anschliessend mit Wasser versetzt und mit NH₄OH basisch gestellt. Die organische Phase wird zur Trockne eingedampft und das Produkt chromatographisch (Kieselgel, Essigester/Methanol + 1% NH40H) gereinigt. 460 mg (IV-9) werden als Diastereomerengemisch erhalten. Analytische HPLC-MS (Methode B): RT = 1,23 und 1,24 min.

### 58.7 Synthese von (2-{2-[4-(4-Chlorphenyl)-piperidin-1-yl]-5-oxo-6,7-dihydro-5H-5λ⁴-thieno[3,2-d]pyrimidin-4-ylamino}-cyclopropyl)-carbamidsäure-tert-butylester (Beispiel 222)

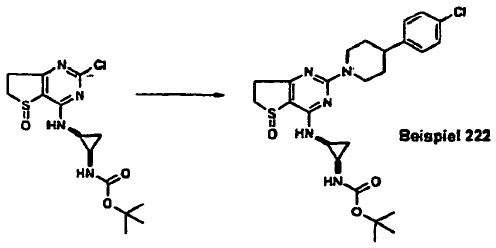

380 mg **(IV-9)** und 266 mg 4-(4-Chlorphenyl)-piperidin Hydrochlorid werden in 3 ml Dioxan vorgelegt, mit 570-µl-Diisopropylethylamin versetz und 25 min bei 120°C in der Mikrowelle erhitzt. Das Reaktionsgemisch wird mit Eiswasser versetzt und das Produkt mit Dichlormethan extrahiert. Die organische Phase wird zur Trockne eingedampft und der Rückstand mit Wasser im Ultraschallbad behandelt. Der ausgefallene Feststoff wird abgesaugt, mit Wasser gewaschen und getrocknet. 485 mg Produkt werden als Diastereomerengemisch erhalten. Analytische HPLC-MS (Methode B): RT = 1,46 min. Chirale HPLC (Säule: Diacel ADS-H, 250 x 4.6 mm, 5 µm, Fließmittel: (Hexan+Diethylamin (0,2%)/Isopropanol (75/25), 10°C, Flußrate: 1 ml/min): RT = 11,5 min und RT = 13,7 min.

### 59. SYNTHESE VON: N-CYCLOPROPYL-N-METHYL-4-{1-[5-OXO-4-(TETRAHYDRO PYRAN-4-YLAMINO)-6,7-DIHYDRO-5H-5λ⁴-THIENO[3,2-d]PYRIMIDIN-2-YL]-PIPERIDIN-4-YL}-BENZAMID (BEISPIEL 229)

### 59.1 4-[4-(Cyclopropylmethylcarbamoyl)-phenyl]-piperidin-1-carbonsäure-tert-butylester:

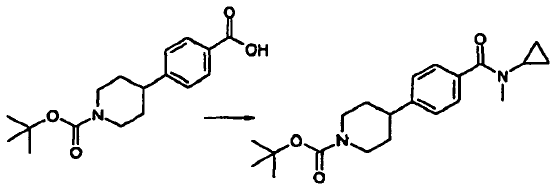

500 mg 4-(4-Carboxyphenyl)-piperidin-1-carbonsäure-*tert*-butylester werden in 28 ml Dimethylformamid vorgelegt, dann 1,14 ml Diisopropylethylamin und 747 mg HATU zugegeben. Das Reaktionsgemisch wird 15 min bei Raumtemperatur gerührt, dann werden 194 mg Cyclopropylmethylamin Hydrochlorid zugegeben. Das Reaktionsgemisch wird über Nacht bei Raumtemperatur gerührt. Anschließend wird das Produkt über präparative HPLC (Methode A) gereinigt. 480 mg Produkt werden als Öl erhalten. Analytische HPLC-MS (Methode B): RT = 1,64 min.

### 59.2 N-Cyclopropyl-N-methyl-4-piperidin-4-yl-benzamid (V-19):

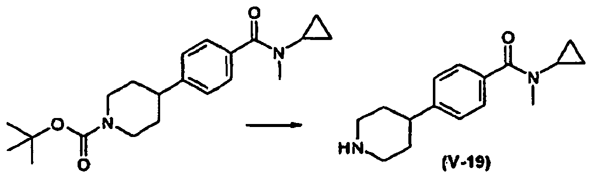

480 mg 4-[4-(Cyclopropylmethylcarbamoyl)-phenyl]-piperidin-1-carbonsäure-*tert*-butylester werden in 7,8 ml Dichlormethan vorgelegt und mit 1,09 ml Trifluoressigsäure versetzt. Das Reaktionsgemisch wird 1,5 h bei Raumtemperatur gerührt und anschließend zur Trockne eingedampft. Der Rückstand wird mit Toluol versetzt und wieder zur Trockne eingedampft. 444 mg **(V-19)** werden als Trifluoracetat erhalten. Analytische HPLC-MS (Methode B): RT = 1,11 min.

### 59.3 N-Cyclopropyl-N-methyl-4-{1-[5-oxo-4-(tetrahydropyran-4-ylamino)-6,7-dihydro-5H-5λ⁴-thieno[3,2-d]pyrimidin-2-yl]-piperidin-4-yl}-benzamid (Beispiel 229)

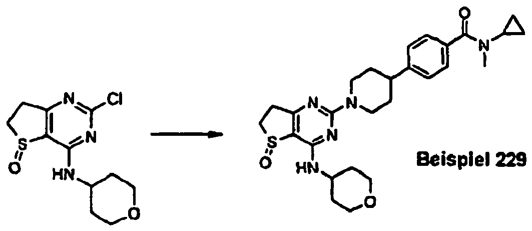

Ausgehend von **(IV-6)** (siehe 6.2) und **(V-19)** kann **Beispiel 229** analog zu Beispiel 89 (siehe 21) hergestellt und aufgereinigt werden. Analytische HPLC-MS (Methode D): RT = 1,05 min.

### 60. SYNTHESE VON (2-{2-[4-(4-CHLORPHENYL)-PIPERIDIN-1-YL]-5-OXO-6,7-DIHYDRO-5H-5λ⁴-THIENO[3,2-d]PYRIMIDIN-4-YLAMINO}-CYCLOPROPYL)-CARBAMIDSÄURE tert-BUTYLESTER (BEISPIEL 231)

### 60.1 trans-N-tert-Butyloxycarbonyl-1,2-cyclopropandiamin:

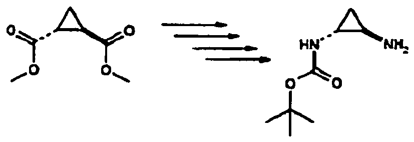

Ausgehend von Dimethyl *trans*-1,2-cyclopropandicarboxylat kann *trans*-N-tert-Butyloxycarbonyl-1,2-cyclopropandiamin Hydrochlorid analog zu *cis*-N-*tert*-Butyloxycarbonyl-1,2-cyclopropandiamin Hydrochlorid (siehe 58.4) hergestellt und aufgereinigt werden. Smp: 200-202°C.

### 60.2 [2-(2-Chlor-6,7-dihydrothieno[3,2-d]pyrimidin-4-ylamino)-cyclopropyl]carbamidsäure-tert-butylester (III-10):

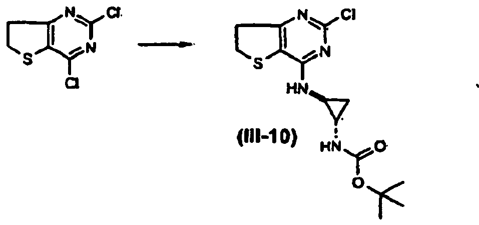

Ausgehend von (II) und *trans*-N-*tert*-Butyloxycarbonyl-1,2-cyclopropandiamin Hydrochlorid kann **(III-10)** analog zu Beispiel **(III-9)** (siehe 58.5) hergestellt werden. Analytische HPLC-MS (Methode B): RT = 1,46 min.

### 60.3 2-(2-Chlor-5-oxo-6,7-dihydro-5H-5λ⁴-thieno[3,2-d]pyrimidin-4-ylamino)-cyclopropyl]-carbamidsäure-tert-butylester (IV-10):

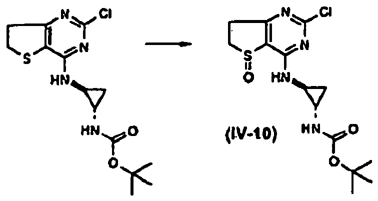

Ausgehend von **(III-10)** wird **(IV-10)** als Diastereomerengemisch analog zu Beispiel **(IV-9)** hergestellt und aufgereinigt. Analytische HPLC-MS (Methode B): RT = 1,27 min. Chirale HPLC (Säule: Diacel ADS-H, 250 x 4.6 mm, 5 µm, Fließmittel: ((9/1) Hexan+Diethylamin (0,2%)/Methanol/Ethanol (1/1), 10°C, Flußrate: 1 ml/min): RT = 6,7 min und RT = 8,3 min.

### 60.4 (2-{2-[4-(4-Chlorphenyl)-piperidin-1-yl]-5-oxo-6,7-dihydro-5H-5λ⁴-thieno[3,2-d]pyrimidin-4-ylamino}-cyclopropyl)-carbamidsäure-tert-butylester (Beispiel 231)

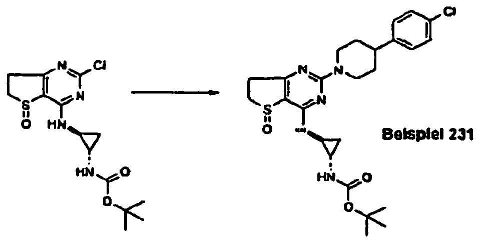

Ausgehend von **(IV-10)** wird **Beispiel 231** als Diastereoisomerengemisch analog zu **Beispiel 222** (siehe 58.7) hergestellt und aufgereinigt. Analytische HPLC-MS (Methode B): RT = 1,48 min. Chirale HPLC (Säule: Diacel ADS-H, 250 x 4.6 mm, 5 µm, Fließmittel:
(Hexan+Diethylamin (0.2%)/Isopropanol (8/2), 10°C, Flußrate: 1 ml/min): RT = 15,17 min und RT = 18,1 min.

### 61. SYNTHESE VON: N-{2-[4-(4-CHLORPHENYL)-PIPERIDIN-1-YL]-5-OXO-6,7-DIHYDRO-5H-5λ⁴-THIENO[3,2-d]PYRIMIDIN-4-YL}-CYCLOPROPAN-1,2-DIAMIN (BEISPIELE 232 UND 245)

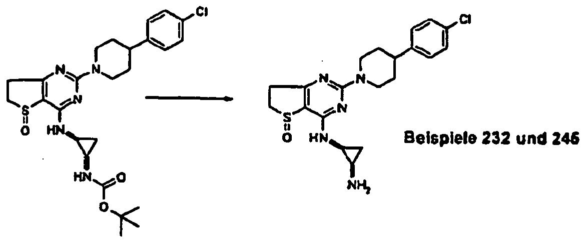

150 mg von **Beispiel 222** (siehe 58.7) werden in 0,5 ml Dichlormethan vorgelegt und 0,25 ml Trifluoressigsäure zugegeben. Das Reaktionsgemisch wird 1 h im Eisbad und 2 h bei Raumtemperatur gerührt, dann im Eisbad abgekühlt, mit Wasser versetzt und mit konz. Ammoniak basisch gestellt. Das Produkt wird mit Dichlormethan extrahiert und chromatographisch (präparative HPLC, Methode B) gereinigt. 57 mg von **Beispiel 232** und 27 mg von **Beispiel 245** werden erhalten. Analytische HPLC-MS (Methode E): RT = 2,73 min **(Beispiel 232)**; RT = 2,85 min **(Beispiel 245)**.

### 62. SYNTHESE VON: N-{2-[4-(4-CHLORPHENYL)-PIPERIDIN-1-YL]-5-OXO-6,7-DIHYDRO-5H-5λ⁴-THIENO[3,2-d]PYRIMIDIN-4-YL}-CYCLOPROPAN-1,2-DIAMIN (BEISPIEL 233)

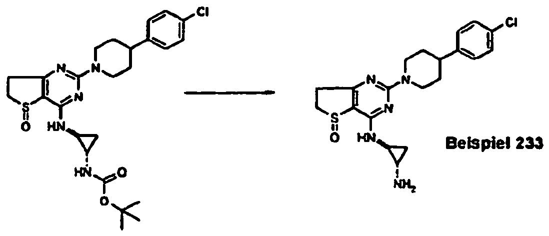

Ausgehend von **Beispiel 231** (siehe 60.4) wird **Beispiel 233** als Diastereomerengemisch analog zu **Beispiele 232/245** (siehe 61.) hergestellt und aufgereinigt. Analytische HPLC-MS (Methode B): RT = 1,24 min.

### 62. SYNTHESE VON: N-CYCLOPROPYL-N-METHYL-4-{1-[5-OXO-4-(TETRAHYDROPYRAN-4-YLAMINO)-6,7-DIHYDRO-5H-5λ⁴-THIENO[3,2-d]PYRIMIDIN-2-YL]-PIPERIDIN-4-YLOXY}-BENZAMID (BEISPIEL 242)

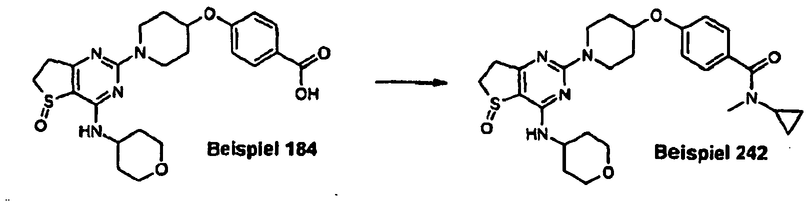

55 mg von **Beispiel 184** (siehe 40.) werden in 2 ml Dimethylformamid vorgelegt, dann. 81 µl Diisopropylethylamin und 53,1 mg O-(7-Azabenzotriazol-1-yl-)-N,N,N',N'-tetramethyluroniumhexafluorphosphat (HATU) zugegeben. Nach 15 min werden 13,8 mg cyclopropylmethylamin Hydrochlorid zugegeben. Das Reaktionsgemisch wird bei Raumtemperatur, bis keine weitere Reaktion erfolgt, gerührt und das Produkt direkt über präparative HPLC (Methode B) gereinigt. 30 mg **Beispiel 242** werden als Feststoff erhalten. Analytische HPLC-MS (Methode D): RT = 1,03 min.

### 63. SYNTHESE VON: 5-{2-[4-(4-CHLORPHENYL)-4-HYDROXYMETHYLPIPERIDIN-1-YL]-5-OXO-6,7-DIHYDRO-5H-5λ⁴-THIENO[3,2-d]PYRIMIDIN-4-YLAMINO}-1-METHYL PIPERIDIN-2-ON (BEISPIEL 246)

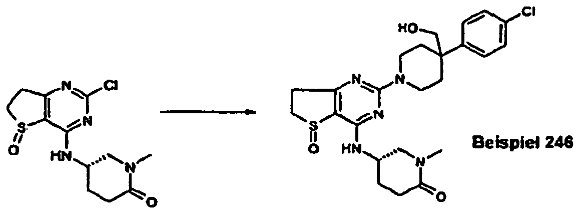

Ausgehend von **(IV-5)** (siehe 5.5) und [4-(4-Chlorphenyl)-piperidin-4-yl]-methanol (siehe J. Med. Chem. 2004, 497) kann **Beispiel 246** analog zu **Beispiel 89** (siehe 21.) hergestellt und aufgereinigt werden. Analytische HPLC-MS (Methode B): RT = 1,21 min.

### 64. SYNTHESE VON: N-CYCLOPROPYL-N-METHYL-3-{1-[5-OXO-4-(TETRAHYDRO PYRAN-4-YLAMINO)-6,7-DIHYDRO-5H-5λ⁴-THIENO[3,2-d]PYRIMIDIN-2-YL]-PIPERIDIN-4-YL}-BENZAMID (BEISPIEL 249)

### 64.1 3-{1-[5-Oxo-4-(tetrahydropyran-4-ylamino)-6,7-dihydro-5H-5λ⁴-thieno[3,2-d]pyrimidin-2-yl]-piperidin-4-yl}benzoesäuremethylester:

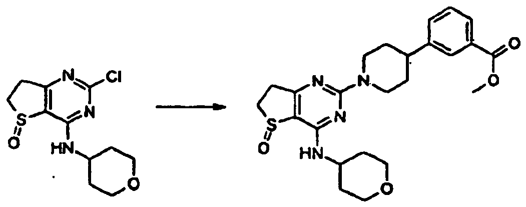

Ausgehend von **(IV-6)** (siehe 6.2) und 3-Piperidin-4-yl-benzoesäuremethylester Hydrochlorid kann das Produkt analog zu Beispiel 89 (siehe 21.) hergestellt und aufgereinigt werden. Analytische HPLC-MS (Methode D): RT = 1,15 min.

### 64.2 3-{1-[5-Oxo-4-(tetrahydropyran-4-ylamino)-6,7-dihydro-5H-5λ⁴-thieno[3,2-d]pyrimidin-2-yl]-piperidin-4-yl}-benzoesäure:

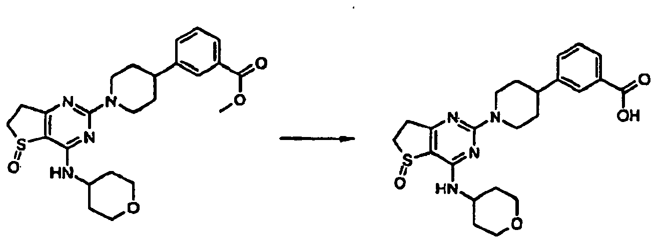

1,3 g 3-{1-[5-0xo-4-(tetrahydrvpyran-4-ylamino)-6,7-dihydro-5H-5λ⁴-thieno[3,2-*d*]pyrimidin-2-yl]-pipetidin-4-yl}-benzoesäuremethylester werden in 24,6 ml Methanol vorgelegt, dann 9,2 ml einer 1 N NaOH Lösung zugegeben. Das Reaktionsgemisch wird bei Raumtemperatur, bis keine weitere Realtion erfolgt, gerührt, dann mit einer 1 N HCl Lösung versetzt. Das Methanol wird abrotiert und der ausgefallene Feststoff abgesaugt. Das Produkt wird über präparative HPLC (Methode B) gereinigt. 760 mg Produkt werden als Feststoff erhalten. Analytische HPLC-MS (Methode D): RT = 0,80 min.

### 64.3 N-Cyclopropyl-N-methyl-3-{1-[5-oxo-4-(tetrahydropyran-4-ylamino)-6,7-dihydro-5H-5λ⁴-thieno[3,2-d]pyrimidin-2-yl]-piperidin-4-yl}-benzamid (Beispiel 249)

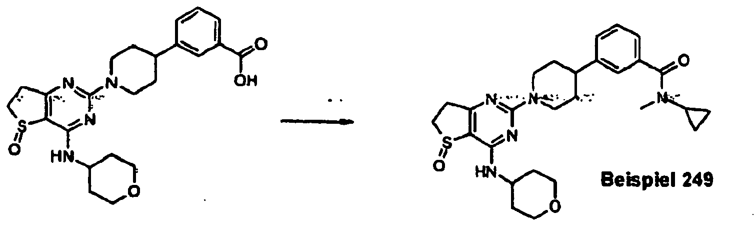

59,8 mg 3-{1-[5-Oxo-4-(tetrahydropyran-4-ylamino)-6,7-dihydro-5H-5λ⁴-thieno[3,2-*d*]pyrimidin -2-yl]-piperidin-4-yl}-benzoesäure werden in 2,3 ml Dimethylformamid vorgelegt, dann 91 µl Diisopropylethylamin und 60 mg O-(7-Azabenzotriazol-1-yl-)-N,N,N',N'-tetramethyluronium hexafluorphosphat (HATU) zugegeben. Nach 15 min wird eine Mischung von 15,5 mg cyclopropylmethylamin Hydrochlorid in 300 µl Dimethylformamid zugegeben. Das Reaktionsgemisch wird bei Raumtemperatur, bis keine weitere Reaktion erfolgt, gerührt und das Produkt direkt über präparative HPLC (Methode B) gereinigt. 50 mg **Beispiel 249** werden als Feststoff erhalten. Analytische HPLC-MS (Methode D): RT = 1,05 min.

### 65. SYNTHESE VON: 1-METHYL-5-{2-[4-(MORPHOLIN-4-CARBONYL)-4-PHENYL PIPERIDIN-1-YL]-5-OXO-6,7-DIHYDRO-5H-5λ⁴-THIENO[3,2-d]PYRIMIDIN-4-YLAMINO}-PIPERIDIN-2-ON (BEISPIEL 252)

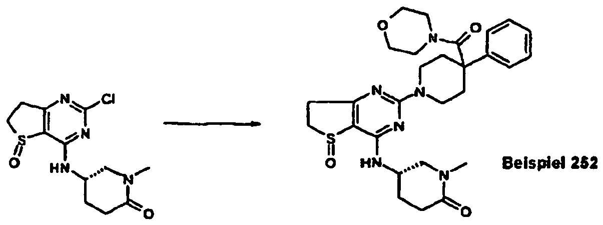

Ausgehend von **(IV-5)** (siehe 5.5) und Morpholin-4-yl-(4-phenylpiperidin-4-yl)-methanon (siehe Bloorg. Med. Chem. Lett. 1997, 2531) kann **Beispiel 252** analog zu Beispiel 89 (siehe 21.) hergestellt und aufgereinigt werden. Analytische HPLC-MS (Methode B): RT= 1,18 min.

### 66. SYNTHESE VON: N-{1-[4-(1-METHYL-6-OXO-PIPERIDlN-3-YLAMINO)-5-OXO-6,7-DIHYDRO-5H-5λ⁴-THIENO[3,2-d]PYRIMIDIN-2-YL]-4-PHENYLPIPERIDIN-4-YLMETHYL}-METHANESULFONAMID (BEISPIEL 253)

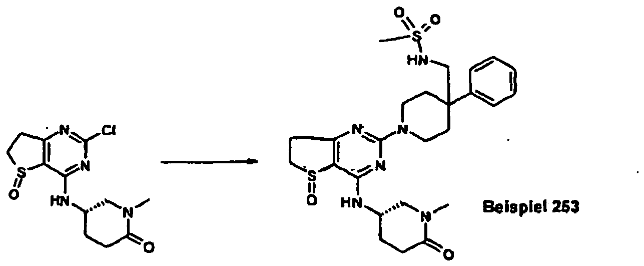

Ausgehend von **(IV-5)** (siehe 5.5) und N-(4-Phenylpipendin-4-ylmethyl)-methansulfonamid (siehe Bioorg. Med. Chem. Lett., 1998, 1851) kann **Beispiel 253** analog zu Beispiel 89 (siehe 21.) hergestellt und aufgereinigt werden. Analytische HPLC-MS (Methode B): RT = 1,15 min.

### 67. SYNTHESE VON: 5-{2-[4-(4-CHLORPHENYL)-4-METHOXYMETHYLPIPERIDIN-1-YL]-5-OXO-6,7-DIHYDRO-5H-5λ⁴-THIENO[3,2-d]PYRIMIDIN-4-YLAMINO}-1-METHYL PIPERIDIN-2-ON (BEISPIEL 260)

### 67.1 4-(4-Chlorphenyl)-4-hydroxymethylpiperidin-1-carbonsäure-tert-butylester:

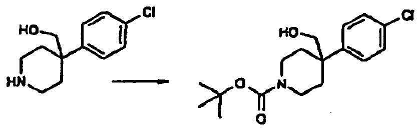

300 mg [4-(4-Chlorphenyl)-piperidin-4-yl]-methanol (siehe J. Med. Chem. 2004, 497) werden in 3 ml Dioxan vorgelegt, dann 0,5 ml Wasser und 0.224 g Natriumcarbonat zugegeben. Nach 5 min werden 300 mg Di-*tert*-butyl-dicarbonat zugegeben. Das Reaktionsgemisch wird 3 h bei Raumtemperatur gerührt, anschließend mit Wasser versetzt und das Produkt mit Dichlormethan extrahiert.. 440 mg Produkt werden als Öl erhalten. Analytische HPLC-MS (Methode B): RT = 1,65 min.

### 67.2 4-(4-Chlorphenyl)-4-methoxymethylpiperidin-1-carbonsäure-tert-butylester:

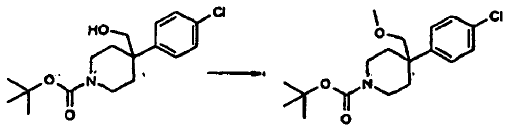

440 mg 4-(4-Chlorphenyl)-4-hydroxymethylpiperidin-1-carbonsäure-*tert*-butylester werden in 2,5 ml Dimethylformamid vorgelegt und 92 mg Natriumhydrid (60% in Mineralöl) zugegeben. Das Reaktionsgemisch wird 30 min bei Raumtemperatur gerührt, dann 95 µl Methyliodid zugegeben. Nach 1 h wird das Reaktionsgemisch auf Eis gegossen und das Produkt mit Diethylether extrahiert. 370 mg Produkt werden als Öl erhalten. Analytische HPLC-MS (Methode B): RT = 1,87 min.

### 67.3 4-(4-Chlorphenyl)-4-methoxymethylpiperidin (V-20):

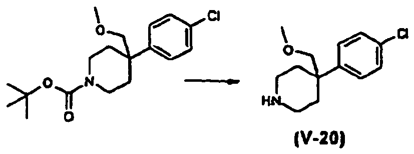

370 mg 4-(4-Chlorphenyl)-4-methoxymethylpiperidin-1-carbonsäure-*tert*-butylester werden in 1,5 ml Dichlormethan vorgelegt, dann 0,8 ml Trifluoressigsäure zugegeben. Das Reaktionsgemisch wird über Nacht bei Raumtemperatur gerührt und zur Trockne eingedampft. Der Rückstand wird mit Toluol versetzt und wieder zur Trockne eingedampft. Der Rückstand wird mit Diethylether verrieben und der Feststoff abgesaugt. 284 mg **(V-20)** werden als Trifluoracetat erhalten. Analytische HPLC-MS (Methode B): RT = 1,23 min.

### 67.4 5-{2-[4-(4-Chlorphenyl)-4-methoxymethylpiperidin-1-yl]-5-oxo-6,7-dihydro-5H-5λ⁴-thieno[3,2-d]pyrimidin-4-ylamino}-1-methylpiperidin-2-on (Beispiel 260)

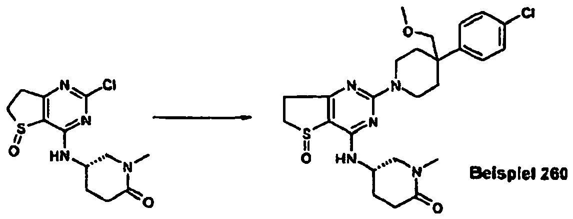

Ausgehend von **(IV-5)** (siehe 5.5) und **(V-20)** kann **Beispiel 260** analog zu **Beispiel 89** (siehe 21.) hergestellt und aufgereinigt werden. Analytische HPLC-MS (Methode B): RT = 1,30 min.

### 68. SYNTHESE VON: 5-{2-[4-(4-CHLORPHENYL)-4-METHOXYPIPERIDIN-1-YL]-5-OXO-6,7-DIHYDRO-5H-5λ⁴-THIENO[3,2-d]PYRIMIDIN-4-YLAMINO}-1-METHYLPIPERIDIN-2-ON (BEISPIEL 261)

### 68.1 4-(4-Chlorphenyl)-4-methoxypiperidin (V-21):

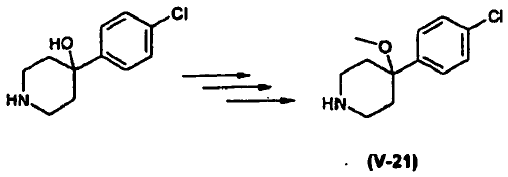

Ausgehend von 4-(4-Chlorphenyl)-piperidin-4-ol kann **(V-21)** analog zu **(V-20)** (siehe 67.1 bis 67.3) hergestellt werden. Analytische HPLC-MS (Methode B): RT = 1,22 min.

### 68.2 5-{2-[4-(4-Chlorphenyl)-4-methoxypiperidin-1-yl]-5-oxo-6,7-dihydro-5H-5λ⁴-thieno[3,2-d]pyrimidin-4-ylamino}-1-methylpiperidin-2-on (Beispiel 261)

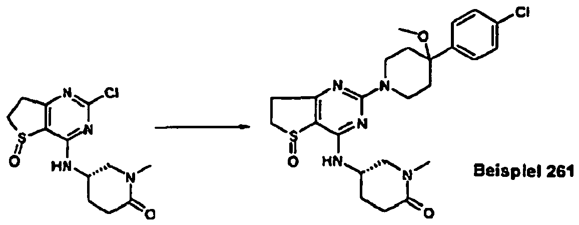

Ausgehend von **(IV-5)** (siehe 5.5) und **(V-21)** kann **Beispiel 261** analog zu Beispiel 89 (siehe 21.) hergestellt und aufgereinigt werden. Analytische HPLC-MS (Methode B): RT = 1,30 min.

### 69. SYNTHESE VON: N-METHYL-N-{1-[4-(1-METHYL-6-OXO-PIPERIDIN-3-YLAMINO)-5-OXO-6,7-DIHYDRO-5H-5λ⁴-THIENO[3,2-d]PYRIMIDIN-2-YL]-4-PHENYLPIPERIDIN-4-YLMETHYL}-METHANSULFONAMID (BEISPIEL 270)

### 69.1 N-Methyl-N-(4-phenylpiperidin-4-ylmethyl)-methanesulfonamid (V-22):

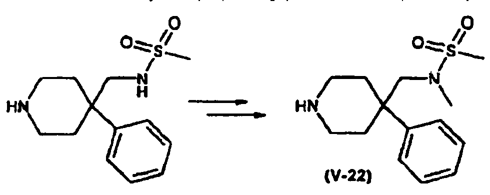

Ausgehend von N-(4-Phenylpiperidin-4-ylmethyl)-methansulfonamid (siehe Bioorg. Med. Chem. Lett., 1998, 1851) kann **(V-22)** analog zu **(V-20)** (siehe 67.1 bis 67.3) hergestellt werden. Analytische HPLC-MS (Methode B): RT = 1,10 min.

### 69.2 N-Methyl-N-{1-[4-(1-methyl-6-oxo-piperidin-3-ylamino)-5-oxo-6,7-dihydro-5H-5λ⁴-thieno[3,2-d]pyrimidin-2-yl]-4-phenylpiperidin-4-ylmethyl}-methansulfonamid (Beispiel 270)

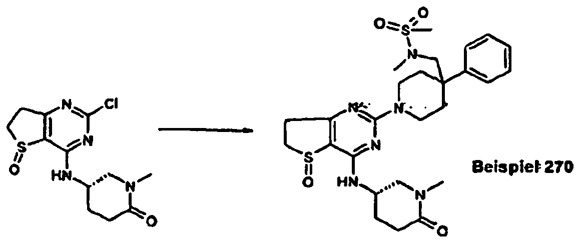

Ausgehend von **(IV-5)** (siehe 5.5) und **(V-22)** kann **Beispiel 270** analog zu Beispiel 89 (siehe 21.) hergestellt und aufgereinigt werden. Analytische HPLC-MS (Methode B): RT = 1,21 min.

### 70. SYNTHESE VON: 5-{2-[4-(3,5-DIFLUORPHENYL)-4-METHOXYPIPERIDIN-1-YL]-5-OXO-6,7-DIHYDRO-5H-5λ⁴-THIENO[3,2-d]PYRIMIDIN-4-YLAMINO}-1-METHYL PIPERIDIN-2-ON (BEISPIEL 273)

### 70.1 4-(3,5-Difluorphenyl)-4-methoxypiperidin (V-23):

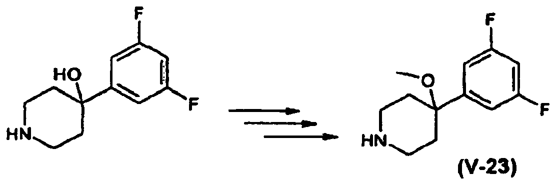

Ausgehend von 4-(3,5-Difluorphenyl)-piperidin-4-ol Hydrochlorid kann **(V-23)** analog zu (V-20) (siehe 67.1 bis 67.3) hergestellt werden. Analytische HPLC-MS (Methode B): RT = 1,10 min.

### 70.2 5-{2-[4-(3,5-Difluorphenyl)-4-methoxypiperidin-1-yl]-5-oxo-6,7-dihydro-5H-5λ⁴-thieno[3,2-d]pyrimidin-4-ylamino}-1-methylpiperidin-2-on (Beispiel 273)

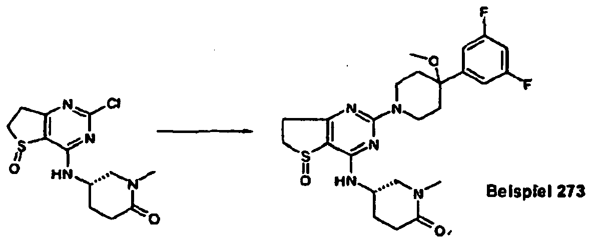

Ausgehend von **(IV-5)** (siehe 5.5) und **(V-23)** kann **Beispiel 273** analog zu Beispiel 89 (siehe 21.) hergestellt und aufgereinigt werden. Analytische HPLC-MS (Methode B): RT = 1,23 min.

### 71. SYNTHESE VON: N-METHYL-N-{1-[4-(1-METHYL-6-OXO-PIPERIDIN-3-YLAMINO)-5-OXO-6,7-DIHYDRO-5H-5λ⁴-THIENO[3,2-d]PYRIMIDIN-2-YL]-4-PHENYLPIPERIDIN-4-YLMETHYL}-ACETAMID (BEISPIEL 274)

### 71.1 N-Methyl-N-(4-phenylpiperidin-4-ylmethyl)-acetamid (V-24):

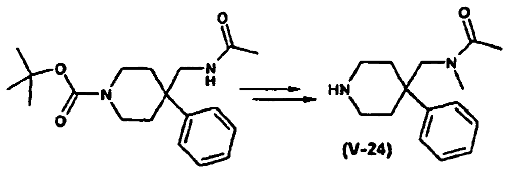

Ausgehend von 4-(Acetylaminomethyl)-4-phenylpiperidin-1-carbonsäure-*tert*-butylester (siehe 52.1) kann **(V-24)** analog zu (V-20) (siehe 67.2 und 67.3) hergestellt werden. Analytische HPLC-MS (Methode B): RT = 1,12 min.

### 71.2 N-Methyl-N-{1-[4-(1-methyl-6-oxo-piperidin-3-ylamino)-5-oxo-6,7-dihydro-5H-5λ⁴-thieno[3,2-d]pyrimidin-2-yl]-4-phenylpiperidin-4-ylmethyl}-acetamid (BEISPIEL 274)

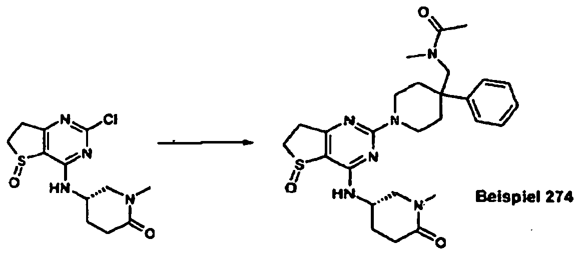

Ausgehend von **(IV-5)** (siehe 5.5) und **(V-24)** kann **Beispiel 274** analog zu Beispiel 89 (siehe 21.) hergestellt und aufgereinigt werden. Analytische HPLC-MS (Methode D): RT = 1,19 min.

### 72. SYNTHESE VON: 1-METHYL-5-{2-[4-(5-METHYL-4-PHENYL-OXAZOL-2-YL)-PIPERIDIN-1-YL]-5-OXO-6,7-DIHYDRO-5H-5λ⁴-THIENO[3,2-d]PYRIMIDIN-4-YLAMINO}-PIPERIDIN-2-ON (BEISPIEL 275)

### 72.1 4-(5-Methyl-4-phenyloxazol-2-yl)-piperidin (V-25):

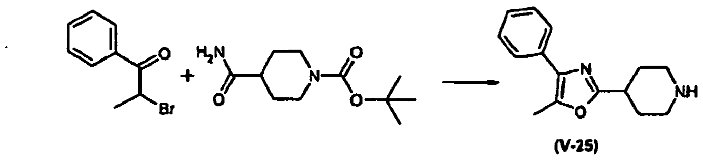

1,75 g 2-Brom-1-phenylpropan-1-on und 1,87 g 4-Carbamoylpiperidin-1-carbonsäure-*tert-*butylester werden in 0,5 ml NMP vorgelegt. Das Reaktionsgemisch wird bei 160°C für 20 min in der Mikrowelle und 35 min im Ölbad erhitzt, dann nach dem Abkühlen in Methanol aufgenommen und zur Trockne eingedampft. Der Rückstand wird mit Wasser versetzt, im Ultraschallbad behandelt und das unlösliche Öl abgesaugt. Die Mutterlauge wird über präparative HPLC (Methode C) gereinigt. 160 mg **(V-25)** werden als Trifluoracetat erhalten. Analytische HPLC-MS (Methode B): RT = 1,24 min.

### 72.2 1-Methyl-5-{2-[4-(-5-methyl-4-phenyloxazol-2-yl)-piperidin-1-yl]-5-oxo-6,7-dihydro-5H-5λ⁴-thieno[3,2-d]pyrimidin-4-ylamino}-piperidin-2-on (Beispiel 275)

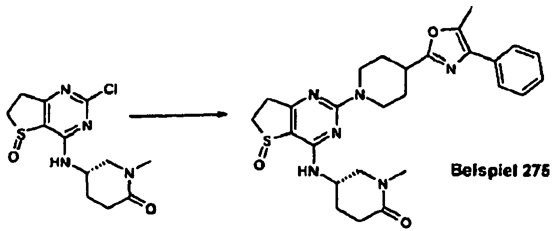

Ausgehend von **(IV-5)** (siehe 5.5) und **(V-21)** kann **Beispiel 275** analog zu Beispiel 89 (siehe 21.) hergestellt und aufgereinigt werden. Analytische HPLC-MS (Methode D): RT = 1,08 min.

### 73. SYNTHESE VON: 5-{2-[4-(4,5-DIPHENYLOXAZOL-2-YL)-PIPERIDIN-1-YL]-5-OXO-6,7-DIHYDRO-5H-5λ⁴-THIENO[3,2-d]PYRIMIDIN-4-YLAMINO}-1-METHYLPIPERIDIN-2-ON (BEISPIEL 278)

### 73.1 4-(4,5-Diphenyloxazol-2-yl)-piperidin-1-carbonsäure-tert-butylester

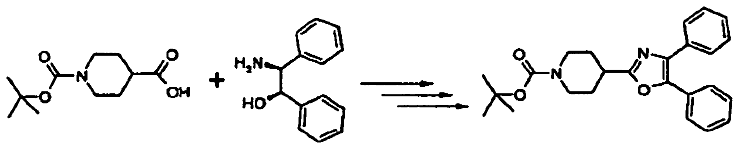

Ausgehend von 1,08 g Piperidin-1,4-dicarbonsäure-mono-*tert*-butylester und 1 g 2-Amino-1,2-diphenyl-ethanol kann das Produkt wie in der Literatur beschrieben (siehe Tet. 2001, 4867) hergestellt werden. Das Produkt wird chromatographisch (Methode B) gereinigt. 560 mg werden als Öl erhalten. Analytische HPLC-MS (Methode A): RT = 1,72 min.

### 73.2 4-(4,5-Diphenyloxazol-2-yl)-piperidin (V-26)

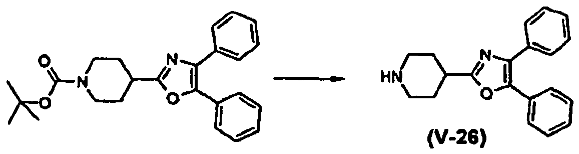

560 mg 4-(4,5-Diphenyloxazol-2-yl)-piperidin-1-carbonsäure-*tert*-butylester werden in 2 ml Dichlormethan vorgelegt, dann 1,1 ml Trifluoressigsäure zugegeben. Das Reaktionsgemisch wird 15 Stunden bei Raumtemperatur gerührt, dann zur Trockne eingedampft. Der Rückstand wird mit Toluol versetzt und wieder zur Trockne eingedampft. Der Rückstand wird mit Diethylether versetzt und der ausgefallene Feststoff abgesaugt und getrocknet. 510 mg **(V-26)** werden erhalten. Analytische HPLC-MS (Methode B): RT = 1,38 min.

### 73.3 5-{2-[4-(4,5-Diphenyloxazol-2-yl)-piperidin-1-yl]-5-oxo-6,7-dihydro-5H-5λ⁴-thieno[3,2-d]pyrimidin-4-ylamino}-1-methylpiperidin-2-on (Beispiel 278)

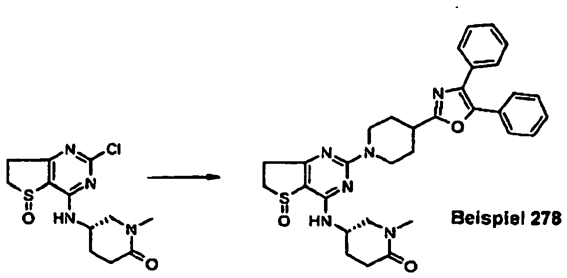

Ausgehend von **(IV-5)** (siehe 5.5) und **(V-26)** kann **Beispiel 278** analog zu Beispiel 89 (siehe 21.) hergestellt und aufgereinigt werden. Analytische HPLC-MS (Methode B): RT = 1,40 min.

### 74. SYNTHESE VON: [1-(2-{4-[5-(4-CHLORPHENYL)-4-METHYLOXAZOL-2-YL]-PIPERIDIN-1-YL}-5-OXO-6,7-DIHYORO-5H-5λ⁴-THIENO[3,2-d]PYRIMIDIN-4-YLAMINO)-CYCLOPROPYL]-METHANOL (BEISPIEL 283)

### 74.1 4-[5-(4-Chlorphenyl)-4-methyloxazol-2-yl]-piperidin (V-27):

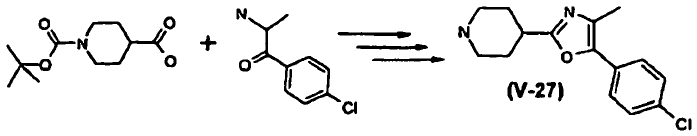

Ausgehend von Piperidin-1,4-dicarbonsäure-mono-*tert*-butylester und 2-Amino-1-(4-chlorphenyl)-propan-1-on (siehe J. Med. Chem. 1974, 416) kann **(V-27)** analog zu (V-26) (siehe 73.1 und 73.2) hergestellt werden. Analytische HPLC-MS (Methode B): RT = 1,30 min.

### 74.2 [1-(2-{4-[5-(4-Chlorpherlyl)-4-methyloxazol-2-yl]piperidin-1-yl}-5-oxo-6,7-dihydro-5H-5λ⁴-thieno[3,2-d]pyrimidin-4-ylamino)-cyclopropyl]-methanol (Beispiel 283)

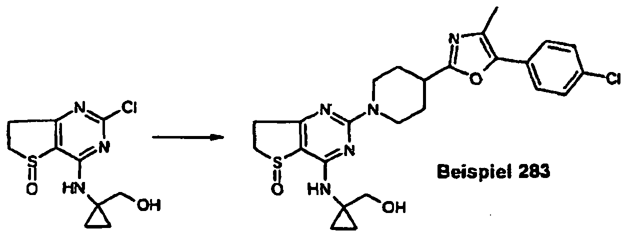

Ausgehend von **(IV-2)** (siehe 2.4) und **(V-27)** kann **Beispiel 283** analog zu Beispiel 89 (siehe 21.) hergestellt und aufgereinigt werden. Analytische HPLC-MS (Methode B): RT = 1,37 min.

### 75. SYNTHESE VON: {2-[4-BENZYLOXYMETHYL-4-(4-CHLORPHENYL)-PIPERIDIN-1-YL]-5-OXO-6,7-DIHYDRO-5H-5λ⁴-THIENO[3,2-d]PYRIMIDIN-4-YL}-(3-FLUORPHENYL)-AMIN (BEISPIEL 306)

### 75.1 4-Benzyloxymethyl-4-(4-chlorphenyl)-piperidin (V-28):

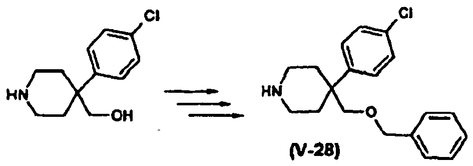

Ausgehend von [4-(4-Chlorphenyl)-piperidin-4-yl]-methanol (siehe J. Med. Chem. 2004, 497) kann **(V-28)** analog zu (V-20) (siehe 67.1 bis 67.3) hergestellt werden. Analytische HPLC-MS (Methode B): RT = 1,43 min

### 75.2 {2-[4-Benzyloxymethyl-4-(4-chlorphenyl)-piperidin-1-yl]-5-oxo-6,7-dihydro-5H-5λ⁴-thieno[3,2-d]pyrimidin-4-yl}-(3-fluorphenyl)-amin (Beispiel 306)

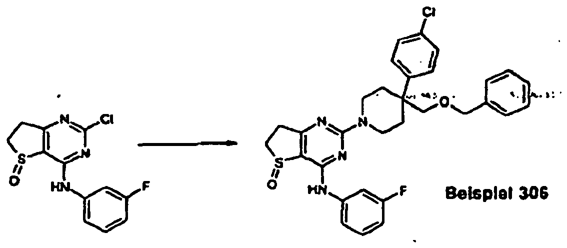

Ausgehend von **(IV-7)** (siehe 17.2) und **(V-28)** kann **Beispiel 306** analog zu Beispiel 89 (siehe 21.) hergestellt und aufgereinigt werden. Analytische HPLC-MS (Methode B): RT = 1,75 min.

### 76. SYNTHESE VON: 2-METHOXY-N-METHYL-N-{1-[5-OXO-4-(TETRAHYDROPYRAN-4-YLAMINO)-6,7-DIHYDRO-5H-5λ⁴-THIENO[3,2-d]PYRIMIDIN-2-YL]-4-PHENYLPIPERIDIN-4-YLMETHYL}-ACETAMID (BEISPIEL 323)

### 76.1 4-Benzyloxymethyl-4-(4-chlorphenyl)-piperidin (V-29):

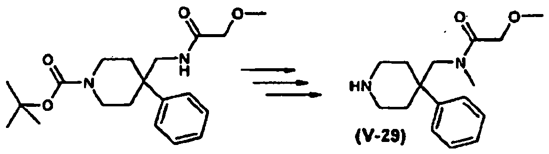

Ausgehend von 4-[(2-Methoxyacetylamino)-methyl]-4-phenylpiperidin-1-carbonsäure-*tert-*butylester (siehe 50.1) kann **(V-28)** analog zu (V-20) (siehe 67.2 bis 67.3) hergestellt werden.

### 76.2 2-Methoxy-N-methyl-N-{1-[5-oxo-4-(tetrahydropyran-4-ylamino)-6,7-dihydro-5H-5λ⁴-thieno[3,2-d]pyrimidin-2-yl]-4-phenylpiperidin-4-ylmethyl}-acetamid (Beispiel 323)

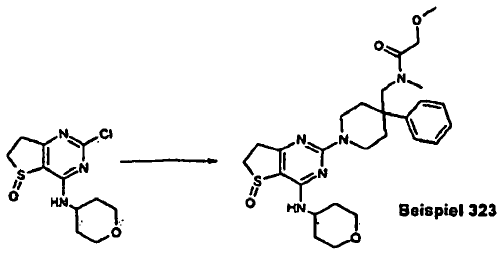

Ausgehend von **(IV-6)** (siehe 6.2) und **(V-29)** kann **Beispiel 323** analog zu Beispiel 89 (siehe 21.) hergestellt und aufgereinigt werden. Analytische HPLC-MS (Methode B): RT = 1,24 min.

### 77. SYNTHESE VON: 5-OXO-2-[4-(4,5,6,7-TETRAHYDROBENZOXAZOL-2-YL)-PIPERIDIM-1-YL]-6,7-DIHYDRO-5H-5λ⁴-THIENO[3,2-d]PYRIMIDIN-4-YL}-(TETRAHYDRO PYRAN-4-YL)-AMIN (BEISPIEL 329)

### 77.1 2-(1-Benzylpiperidin-4-yl)-4,5,6,7-tetrahydrobenzoxazol:

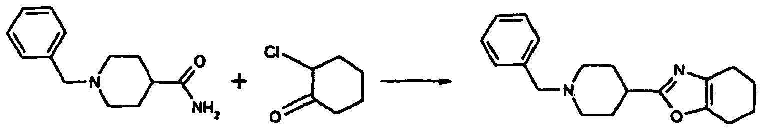

Ein Gemisch von 2,43 g 2-Chlorcyclohexanon und 1g 1-Benzylpiperidin-4-carbonsäureamid (siehe WO2005/61483) wird bei 160°C in der Mikrowelle, bis keine weitere Umsetzung erfolg, erhitzt. Das Produkt wird chromatographisch gereinigt. 963 mg des Produkts werden erhalten. Analytische HPLC-MS (Methode B): RT = 1,28 min.

### 77.2 2-Piperidin-4-yl-4,5,6,7-tetrahydrobenzoxazol (V-30):

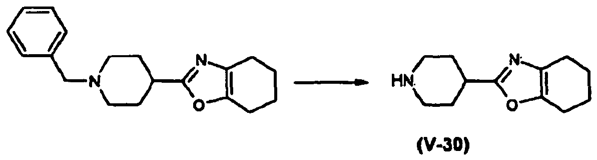

903 mg 2-(1-Benzyl-piperidin-4-yl)-4,5,6,7-tetrahydrobenzoxazol werden in 20 ml Methanol vorgelegt und mit 450 mg Pd/C 10% bei einem Druck von 3 bar und bei Raumtemperatur hydriert. Nach 12 Stunden wird der Katalysator abgesaugt und das Filtrat zur Trockne eingedampft. Das Produkt wird chromatographisch gereinigt. 469 mg **(V-30)** werden als Trifluoracetat erhalten. Analytische HPLC-MS (Methode B): RT = 1,09 min.

### 77.3 5-Oxo-2-[4-(4,5,6,7-tetrahydrobenzoxazol-2-yl)-piperidin-1-yl]-6,7-dihydro-5H-5λ⁴-thieno[3,2-d]pyrimidin-4-yl}-(tetrahydropyran-4-yl)-amin (Beispiel 329)

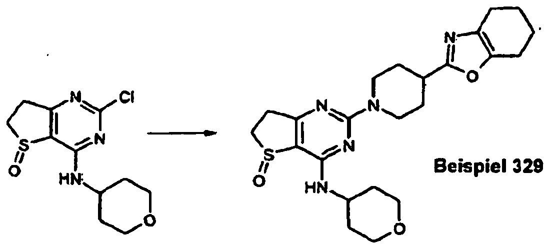

Ausgehenden **(IV-2)** (siehe 2.4) und **(V-30)** kann **Beispiel 329** analog zu Beispiel 89 (siehe 21.) hergestellt und aufgereinigt werden. Analytische HPLC-MS (Methode B): RT = 1,23 min.

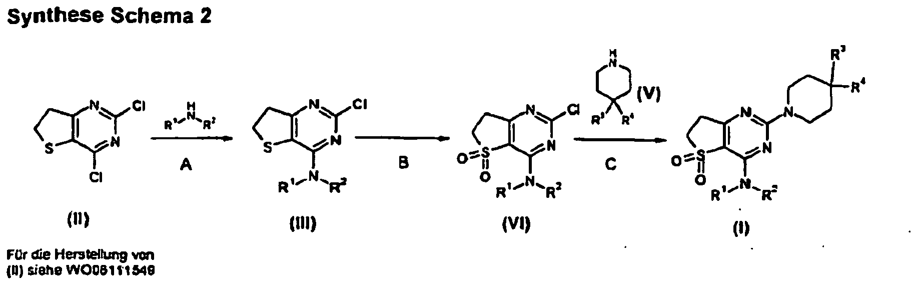

### 78. SYNTHESE VON: (1-{2-[4-(4-CHLORPHENYL)-PIPERlDIN-1-YL]-5,5-DIOXO-6,7-DIHYDRO-5H-5λ⁶-THIENO[3,2-d]PYRIMIDIN-4-YLAMINO}-CYCLOPROPYL)-METHANOL (Beispiel 333)

### 78.1 [1-(2-Chior-5,5-dioxo-6,7-dihydro-5H-5λ⁶-thieno[3,2-d]pyrimidin-4-ylamino)-cyclopropyl]-methanol (VI-1):

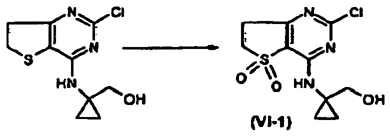

200 mg **(III-2)** (siehe 2.2) werden in 3 ml Trifluoressigsäure vorgelegt, dann 180 µl Wasserstoffperoxid (35%) langsam zugetropft. Eine exotherme Reaktion findet statt. Das Reaktionsgemisch wird 12 Stunden bei Raumtemperatur gerührt, dann mit Eiswasser versetzt und mit NH₄OH basisch gestellt. Das ausgefallene Feststoff wird abgesaugt und getrocknet. 80 mg **(VI-1)** werden erhalten. Analytische HPLC-MS (Methode B): RT = 1,1 min.

### 78.2 (1-{2-[4-(4-Chlorphenyl)-piperidin-1-yl]-5,5-dioxo-6,7-dihydro-5H-5λ⁶-thieno[3,2-d]pyrimidin-4-ylamino}-cyclopropyl)-methanol (Beispiel 333)

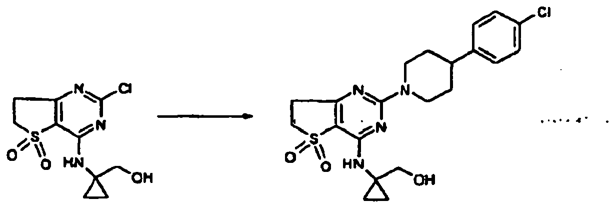

Ausgehend von **(VI-1)** und 4-(4-Chlorphenyl)-piperidin Hydrochlorid kann **Beispiel 333** analog zu **Beispiel 89** (siehe 21.) hergestellt und aufgereinigt werden. Analytische HPLC-MS (Methode B): RT = 1,49 min.

### 79. SYNTHESE VON: {2-[4-(4-CHLORPHENYL)-PIPERIDIN-1-YL]-5,5-DIOXO-6,7-DIHYDRO-5H-5λ⁶-THIENO[3,2-d]PYRIMIDIN -4-YL}-(TETRAHYDROPYRAN-4-YL)-AMIN (Beispiel 334)

### 79.1 (2-Chlor-5,5-dioxo-6,7-dihydro-5H-5λ⁶-thieno[3,2-d]pyrimidin-4-yl)-(tetrahydropyran-4-yl)-amin (VI-2):

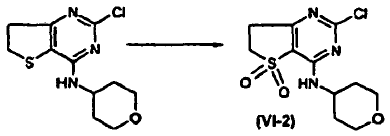

200 mg **(III-2)** (siehe 2.2) werden in 3 ml Trifluoressigsäure vorgelegt, dann 180 µl Wasserstoffperoxid (35%) langsam zugetropft. Eine exotherme Reaktion findet statt. Das Reaktionsgemisch wird 12 Stunden bei Raumtemperatur gerührt, dann mit Eiswasser versetzt und mit NH₄OH basisch gestellt. Das ausgefallene Feststoff wird abgesaugt und getrocknet. 170 mg (VI-2) werden als Feststoff erhalten.

### 79.2 {2-[4-(4-Chlorphenyl)-piperidin-1-yl]-5,5-dioxo-6,7-dihydro-5H-5λ⁶-thieno[3,2-d]pyrimidin-4-yl}-(tetrahydropyran-4-yl)-amin (Beispiel 334)

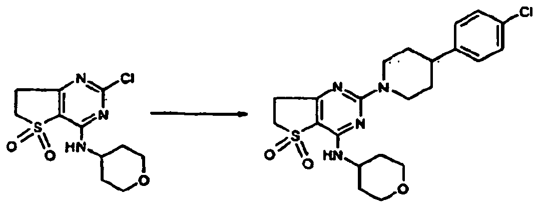

Ausgehend von **(VI-2)** und 4-(4-Chlorphenyl)-piperidin Hydrochlorid kann **Beispiel 334** analog zu Beispiel 89 (siehe 21.) hergestellt und aufgereinigt werden. Analytische HPLC-MS (Methode B): RT=1,55 min.

### 80. SYNTHESE VON: 5-{2-[4-(4-CHLORPHENYL)PIPERIDIN-1-YL]-5,5-DIOXO-6,7-DIHYDRO-5H-5λ⁶-THIENO[3,2-d]PYRIMIDIN-4-YLAMINO}-1-METHYLPIPERIDIN-2-ON (Beispiel 335)

### 80.1 5-(2-Chlo-5,5-dioxo-6,7-dihydro-5H-5λ⁶-thieno[3,2-d]pyrimidin-4-ylamino)-1-methylpiperidin-2-on (VI-3):

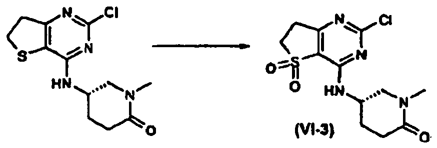

200 mg **(III-5)** (siehe 5.4) werden in 3 ml Trifluoressigsäure vorgelegt, dann 165 µl Wasserstoffperoxid (35%) langsam zugetropft. Eine exotherme Reaktion findet statt. Das Reaktionsgemisch wird 12 Stunden bei Raumtemperatur gerührt, dann mit Eiswasser versetzt und mit NH₄OH basisch gestellt. Das Produkt wird mit Dichlormethan extrahiert. 150 mg **(VI-3)** werden als Feststoff erhalten.

### 80.2 5-{2-[4-(4-Chlorphenyl)-piperidin-1-yl]-5,5-dioxo-6,7-dihydro-5H-5λ⁶-thieno[3,2-d]pyrimidin-4-ylamino}-1-methylpiperidin-2-on (Beispiel 335)

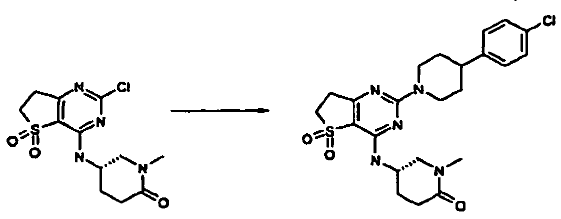

Ausgehend von **(VI-3)** und 4-(4-Chlorphenyl)-piperidin Hydrochlorid kann **Beispiel 335** analog zu Beispiel 89 (siehe 21.) hergestellt und aufgereinigt werden. Analytische HPLC-MS (Methode B): RT = 1.48 min.

### CHROMATOGRAPHIE METHODEN

Die nach den obigen Synthese-Schema hergestellten Beispielverbindungen wurden durch folgende chromatographische Methoden, die - sofern sie durchgeführt wurden - im einzelnen in der Tabellen B, D und E angegeben sind, charakterisiert.

### Analytische HPLC-MS, Methode A

Waters ZMD Masse Spektrometer (positive Ionisation (ESI+)), Alliance 2690/2695 HPLC (Diodenarraydetektor, Wellenlängenbereich: 210 to 500 nm), Waters 2700 Autosampler, Waters 996/2996.
A: Wasser mit 0.10% TFA
B: Acetonitril mit 0.10% TFA

| Zeit in min | %A | %B | Flussrate in ml/min |
|---|---|---|---|
| 0.00 | 95 | 5 | 2.50 |
| 0.20 | 95 | 5 | 2.50 |
| 1.50 | 2 | 98 | 2.50 |
| 1.70 | 2 | 98 | 2.50 |
| 1.90 | 95 | 5 | 2.50 |
| 2.20 | 95 | 5 | 2.50 |

Als stationäre Phase dient eine Säule Merck Chromolith^{™} Flash RP-18e, 4.6 mm x 25 mm (Säulentemperatun konstant bei 25°C).

### Analytische HPLC-MS, Methode B

Waters ZMD Masse Spektrometer (positive Ionisation (ESI+)), Alliance 2690/2695 HPLC (Diodenarraydetektor, Wellenlängenbereich: 210 to 500 nm), Waters 2700 Autosampler, Waters 996/2996.
A: Wasser mit 0.10% TFA
B: Acetonitril mit 0.10% TFA

| Zeit in min | %A | %B | Flussrate in ml/min |
|---|---|---|---|
| 0.00 | 95 | 5 | 2.80 |
| 0.30 | 95 | 5 | 2.80 |
| 1.60 | 2 | 98 | 2.80 |
| 1.90 | 2 | 98 | 2.80 |
| 2.00 | 95 | 5 | 2.50 |

Als stationäre Phase dient eine Säule Merck Chromolith^{™} Flash RP-18e, 3 mm x 100 mm (Säulentemperatur: konstant bei 25°C).

### Analytische HPLC-MS, Methode C

Waters ZQ2000 Massenspektrometer (positive Ionisation (ESI+)). HP1100 HPLC (DAD, Wellenlängenbereich: 210 to 500 nm), and Gilson 215 Autosampler.
A: Wasser mit 0.10% TFA
B: Acetonitril mit 0.10% TFA

| Zeit in min | %A | %B | Flussrate in ml/min |
|---|---|---|---|
| 0.00 | 95 | 5 | 1.50 |
| 2.00 | 0 | 100 | 1.50 |
| 2.50 | 0 | 100 | 1.50 |
| 2.60 | 95 | 5 | 1.50 |

Als stationäre Phase dient eine Säule Sunfire C18, 4,6 X 50mm, 3,5 µm, Säulentemperatur 40°C.

### Analytische HPLC-MS, Methode D

Waters ZMD Masse Spektrometer (positive Ionisation (ESI+)), Alliance 2690/2695 HPLC (Diodenarraydetektor, Wellenlängenberelch: 210 to 500 nm), Waters 2700 Autosampler, Waters 996/2996.
A: Wasser mit 0.10% NH₃
B: Acetonitril mit 0.10% NH₃

| Zeit in min | %A | %B | Flussrate in ml/min |
|---|---|---|---|
| 0.00 | 95 | 5 | 3.00 |
| 0.20 | 95 | 5 | 3.00 |
| 1.50 | 2 | 98 | 3.00 |
| 1.90 | 2 | 98 | 3.00 |
| 2.00 | 2 | 98 | 3.00 |

Als stationäre Phase dient Waters, X-Bridge, C18, 3,5 nm, 4,6 X 20 mm. Raumtemperatur.

### Analytische HPLC-MS, Methode E

Waters ZMD Masse Spektrometer (positive Ionisation (ESI+)), Alliance 2690/2695 HPLC (Diodenarraydetektor, Wellenlängenbereich: 210 to 500 nm), Waters 2700 Autosampler, Waters 996/2996.
A: Wasser mit 0.10% TFA
B: Acetonitril mit 0.10% TFA

| Zeit in min | %A | %B | Flussrate in ml/min |
|---|---|---|---|
| 0.00 | 95 | 5 | 1.20 |
| 0.30 | 95 | 5 | 1.20 |
| 9.00 | 2 | 98 | 1.20 |
| 9.40 | 2 | 98 | 1.20 |
| 9.50 | 95 | 5 | 2.80 |
| 9.90 | 95 | 5 | 2.80 |
| 10.00 | 95 | 5 | 0.20 |

Als stationäre Phase dient eine Säule Merck Chromolith^{™} Flash RP-18e, 4.6 mm x 25 mm (Säulentemperatur: konstant bei 25°C).

### Analytische HPLC, Methode A

Agilent 1100 (Diodenarraydetektion, Wellenlängenberelch: 210-380 nm).
A: Wasser mit 0.10% TFA
B: Acetonitril mit 0.13% TFA

| Zeit in min | %A | %B | Flussrate in ml/min |
|---|---|---|---|
| 0.00 | 95 | 5 | 1.50 |
| 0.60 | 95 | 5 | 1.50 |
| 3.40 | 2 | 98 | 1.50 |
| 3.90 | 2 | 98 | 1.50 |
| 4.20 | 95 | 5 | 1.50 |
| 4.90 | 95 | 5 | 1.50 |

Als stationäre Phase dient eine Säule Varian Microsorb, RP C18, 3 µm, 100 A, Raumtemperatur.

### Präparative HPLC-MS, Methode A

Waters ZQ2000 Masse Spektrometer (positive Ionisation (ESI+)), HP1100 HPLC (DAD, Wellenlängenbereich: 210 - 500 nm), and Gilson 215 Autosampler.
A: Wasser mit 0.10% TFA
B: Acetonitril

| Zeit in min | %A | %B | Flussrate in ml/min |
|---|---|---|---|
| 0.00 | 90 | 10 | 50 |
| 1.50 | 90 | 10 | 50 |
| 8.00 | 40 | 60 | 50 |
| 10.00 | 40 | 60 | 50 |
| 11.00 | 90 | 10 | 50 |

Als stationäre Phase dient eine Säule Sunfire C18, 30 X 100 mm, 5 µm, Raumtemperatur.

### Präparative HPLC, Methode A

Gilson HPLC mit Gilson UV-VIS-155 Detektor, Sampling Injektor 231 XL.
Als Wellenlänge wird das substanzspezifische UV-Maximum angegeben.
A: Wasser mit 0.13% TFA
B: Acetonitril mit 0.1% TFA

| Zeit in min | %A | %B | Flussrate in ml/min |
|---|---|---|---|
| 0.00 | 95 | 5 | 165 |
| 1.30 | 95 | 5 | 165 |
| 8.90 | 2 | 98 | 165 |
| 10.00 | 2 | 98 | 165 |
| 10.50 | 95 | 5 | 165 |
| 11.60 | 95 | 5 | 165 |

Als stationäre Phase dient eine Säule Microsorb RP 18, 8 µm, 50 X 65 mm, Raumtemperatur.

### Präparative HPLC, Methode B

Gilson HPLC mit Gilson UV-VIS-155 Detektor, Sampling Injektor 231 XL.
Als Wellenlänge wird das substanzspezifische UV-Maximum angegeben.
A: Wasser mit 0.1 % Ammoniak 35%ig
B: Acetonitril

| Zeit in min | %A | %B | Flussrate in ml/min |
|---|---|---|---|
| 0.00 | 95 | 5 | 180 |
| 1.40 | 95 | 5 | 180 |
| 17.00 | 2 | 98 | 180 |
| 18.50 | 2 | 98 | 180 |
| 18.70 | 95 | 5 | 180 |
| 20.-50 | 95 | 5 | 180 |

Als stationäre Phase dient eine Säule Pursuit XRS RP 18, 10 µm, 50 X 150 mm, Raumtemperatur.

### Präparative HPLC, Methode C

Gilson HPLC mit Gilson UV-VIS-155 Detektor, Sampling Injektor 231 XL.
Als Wellenlänge wird das substanzspezifische UV-Maximum angegeben.
A: Wasser mit 0.13% TFA
B: Acetonitril mit 0,1% TFA

| Zeit in min | %A | %B | Flussrate in ml/min |
|---|---|---|---|
| 0.00 | 95 | 5 | 180 |
| 1.40 | 95 | 5 | 180 |
| 17.00 | 2 | 98 | 180 |
| 18.50 | 2 | 98 | 180 |
| 18.70 | 95 | 5 | 180 |
| 20.50 | 95 | 5 | 180 |

Als stationäre Phase dient eine Säule Microsorb RP 18, 8 µm, 50 X 150 mm, Raumtemperatur.

### Präparative HPLC, Methode D

Gilson HPLC mit Gilson UV-VIS-155 Detektor, Sampling Injektor 231 XL.
Als Wellenlänge wird das substanzspezifische UV-Maximum angegeben.
A: Wasser mit 0.1% Ammoniak 35%ig
B: Acetonitril

| Zeit in min | %A | %B | Flussrate in ml/min |
|---|---|---|---|
| 0.00 | 93 | 5 | 180 |
| 1.10 | 95 | 5 | 180 |
| 9.00 | 2 | 98 | 180 |
| 10.00 | 2 | 98 | 180 |
| 10.50 | 95 | 5 | 180 |
| 12.00 | 95 | 5 | 180 |

Als stationäre Phase dient eine Säule X-Bridge C18, 5 µm, 50 X 65 mm, Raumtemperatur.

### BEISPIELE

Die folgenden Beispiele wurden analog den oben gezeigten Synthesevorschriften herstellt (je nach Kennzeichnung in der Tabelle). Diese Verbindungen sind als PDE4-Inhibitoren geeignet und besitzen IC₅₀-Werte kleiner oder gleich 1 µmol. Die Inhibitionen (in %) bei 1 µM der einzelnen Beispiel-Substanzen sind in der nachfolgenden Beispiel-Tabelle eingefügt und wurden wie folgt bestimmt:
Bei der Durchführung des Scintilation Proximity (SPA) Assays (GE Healthcare, Nr. TRKQ7090) werden die unterschiedlichen Affinitäten des cyklischen 3'-5'-Adenosinmonophosphates (cAMP, niedrige Affinität) und des linearen 5'-Adenosinmonophosphates (AMP, hohe Affinität) an Yttrium-Silicat-Scintillatorbeads ausgenutzt. Die cAMP spezifische Phosphodiesterase (PDE) PDE4B spaltet die 3'-Phosphoesterbindung des Tritiummarkierten [H3]-cAMP zum [H3]-5'-AMP. Dieses [H3]-AMP lagert sich aufgrund der höheren Affinität zu den Scintillatorbeads an diese an und verursacht Scintillationsereignisse (Lichtblitze) welche in einem *Wallac Microbeta Scintillation Counter* gemessen werden.

Der Versuch startet mit einer einstündigen Inkubation von [H3]-cAMP mit dem PDE4B Enzym in Assaypuffer bei 30°C, jeweils einmal mit der zu testenden Beispiel-Substanz (in einer Konzentation von 1 µM) und einmal ohne die zu testende Beispiel-Substanz. Nach dieser Inkubation wird die Reaktion durch Zugabe der Beads gestoppt. Die Beads erhalten in den folgenden 45 Minuten Gelegenheit, sich abzusetzen, danach wird im Scintillation Counter gemessen. Ist die Substanz in der Lage, die enzymatische Aktivität der PDE4B zu hemmen, so entsteht während der Inkubationsphase weniger [H3]-AMP und es sind weniger Scintillationsereignisse messbar. Ausgedrückt werden diese Ergebnisse als Prozent Inhibition bei einer Konzentration der Test-Substanz von 1µM.

Bei den Beispielen handelt es sich um Verbindungen der folgenden Formel 1, 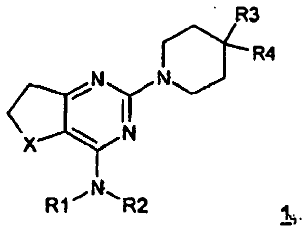 mit den in den folgenden Tabellen A und B bezeichneten Eigenschaften:

**Tabelle A: Chemische Strukturen der Beispiel-Substanzen 1 - 163**

| # | Struktur | R¹ | R² | R³ | R⁴ | % Inhibition PDE4B @ 1µM |
|---|---|---|---|---|---|---|
| 1 | 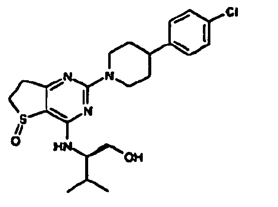 | H | 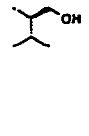 | 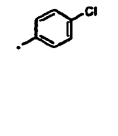 | H | 93 |
| 2 | 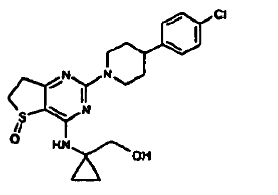 | H | 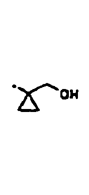 | 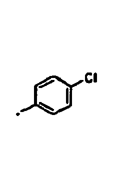 | H | 94 |
| 3 | 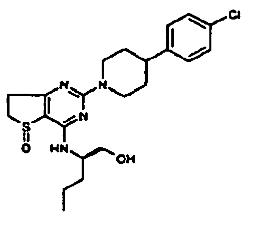 | H | 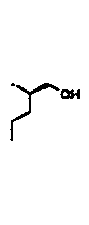 | 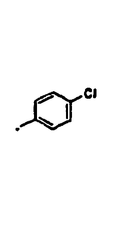 | H | 94 |
| 4 | 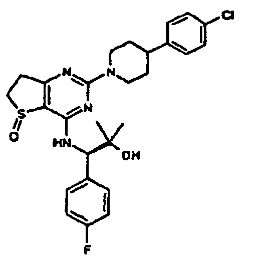 | H | 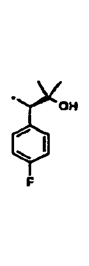 | 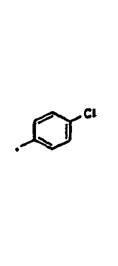 | H | 91 |
| 5 | 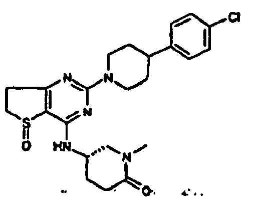 | H | 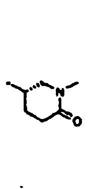 | 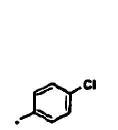 | H | 94 |
| 6 | 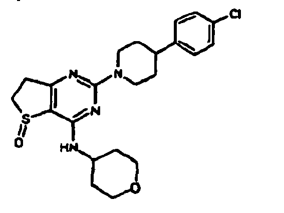 | H | 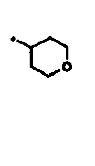 | 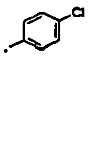 | H | 94 |
| 7 | 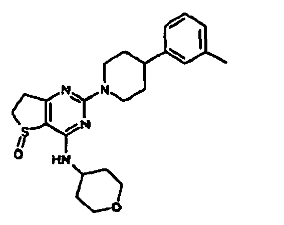 | H | 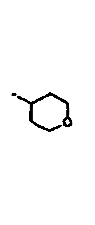 | 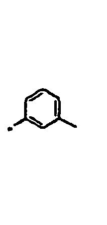 | H | 93 |
| 8 | 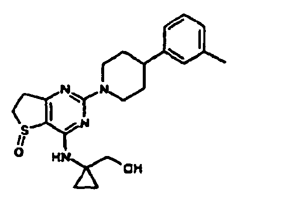 | H | 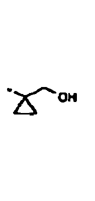 | 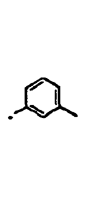 | H | 93 |
| 9 | 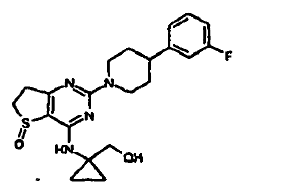 | H | 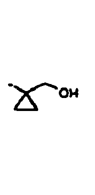 | 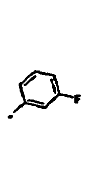 | H | 95 |
| 10 | 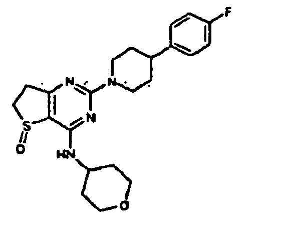 | H | 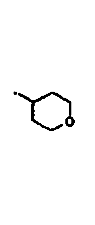 | 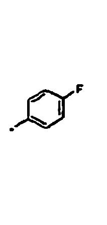 | H | 93 |
| 11 | 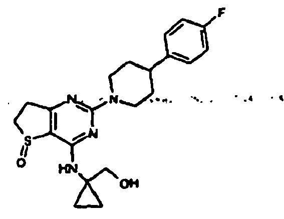 | H | 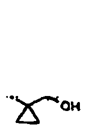 | 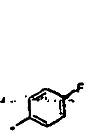 | H | 94 |
| 12 | 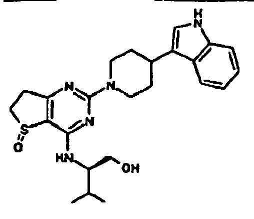 | H | 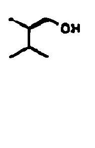 | 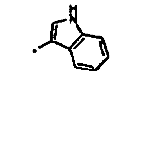 | H | 94 |
| 13 | 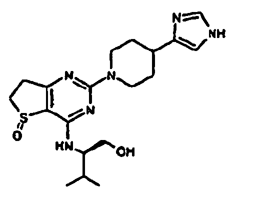 | H | 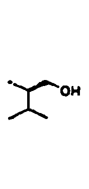 | 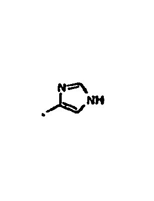 | H | 71 |
| 14 | 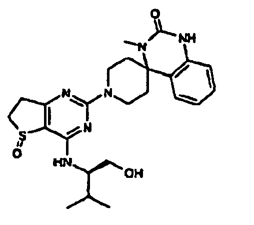 | H | 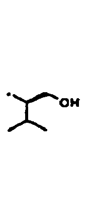 | 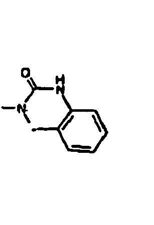 | | 60 |
| 15 | 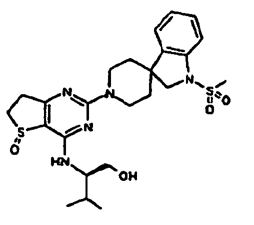 | H | 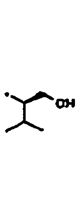 | 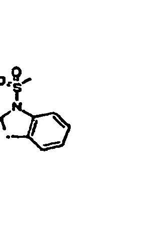 | | 67 |
| 16 | 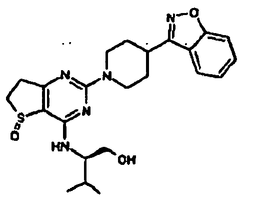 | H | 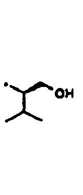 | 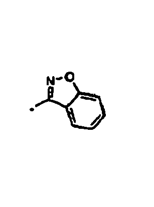 | H | 96 |
| 17 | 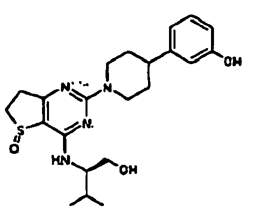 | H | 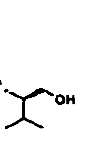 | 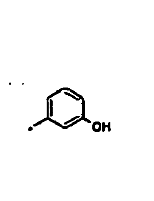 | H | 93 |
| 18 | 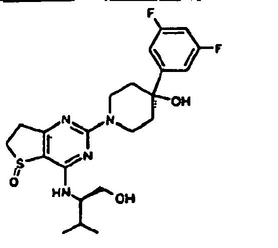 | H | 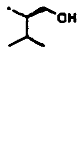 | 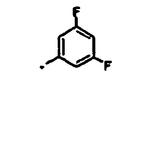 | OH | 92 |
| 19 | 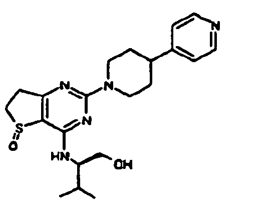 | H | 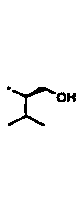 | 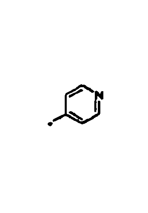 | H | 83 |
| 20 | 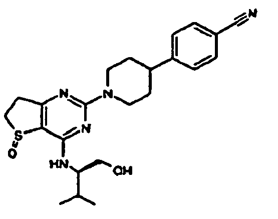 | H | 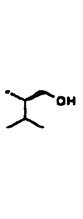 | 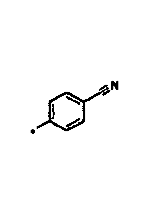 | H | 94 |
| 21 | 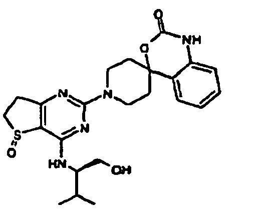 | H | 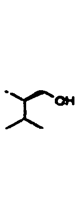 | 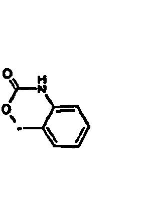 | | 83 |
| 22 | 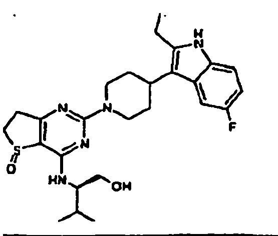 | H | 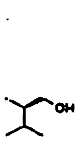 | 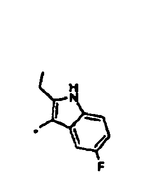 | H | 86 |
| 23 | 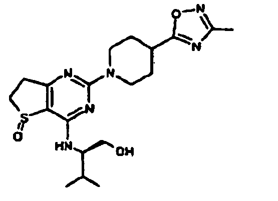 | H | 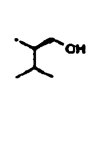 | 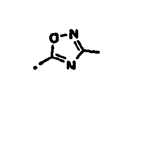 | H | 85 |
| 24 | 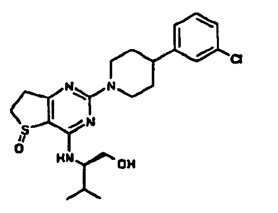 | H | 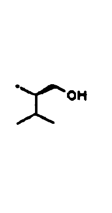 | 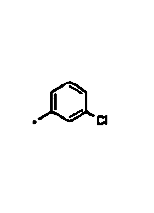 | H | 94 |
| 25 | 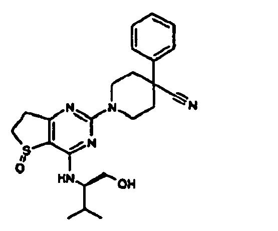 | H | 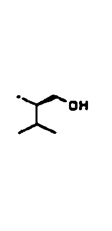 | 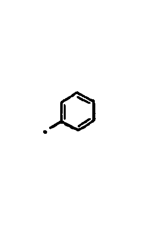 | CN | 90 |
| 26 | 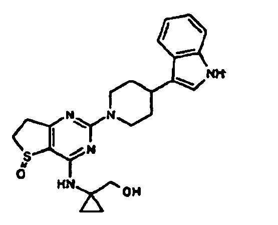 | H | 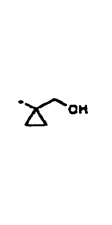 | 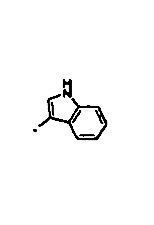 | H | 96 |
| 27 | 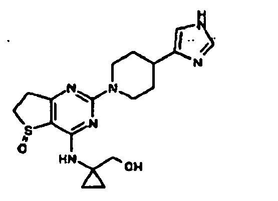 | H | 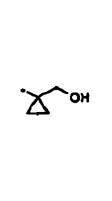 | 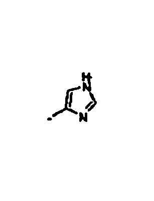 | H | 87 |
| 28 | 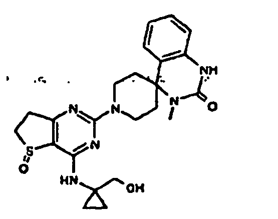 | H | 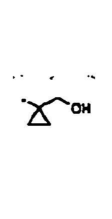 | 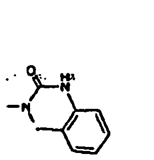 | | 85 |
| 29 | 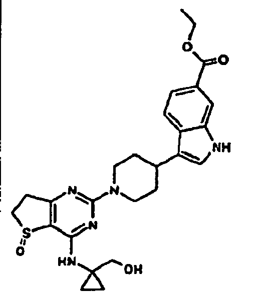 | H | 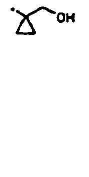 | 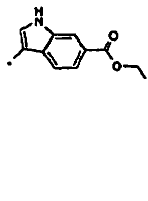 | H | 97 |
| 30 | 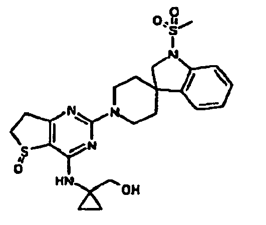 | H | 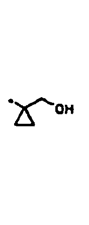 | 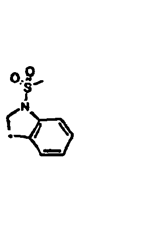 | | 89 |
| 31 | 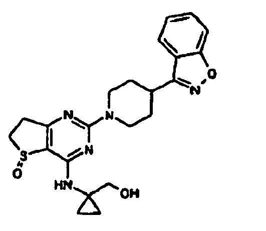 | H | 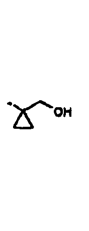 | 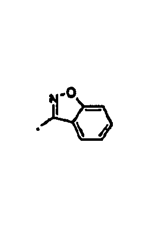 | H | 97 |
| 32 | 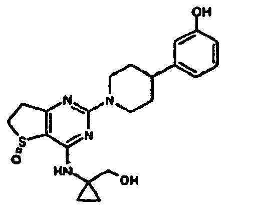 | H | 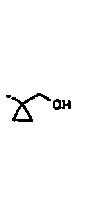 | 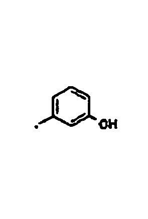 | H | 96 |
| 33 | 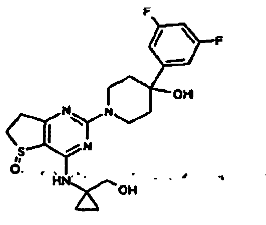 | H | 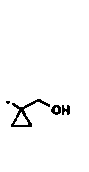 | 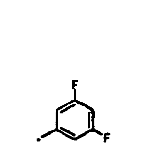 | OH | 95 |
| 34 | 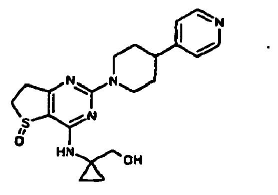 | H | 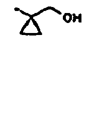 | 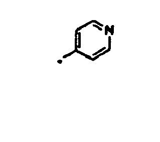 | H | 92 |
| 35 | 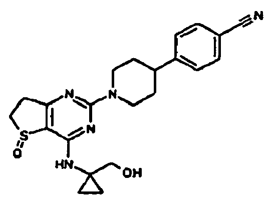 | H | 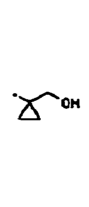 | 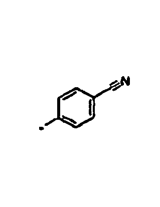 | H | 95 |
| 36 | 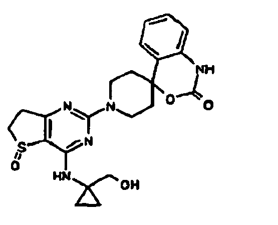 | H | 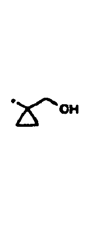 | 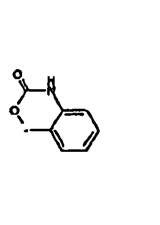 | | 92 |
| 37 | 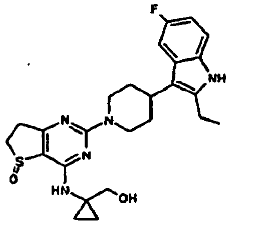 | H | 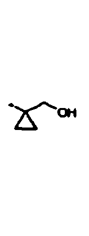 | 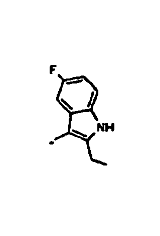 | H | 92 |
| 38 | 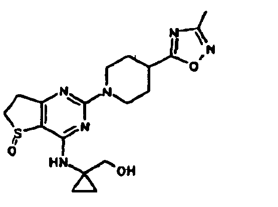 | H | 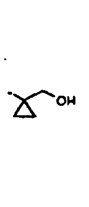 | 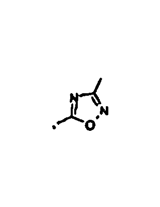 | H | 92 |
| 39 | 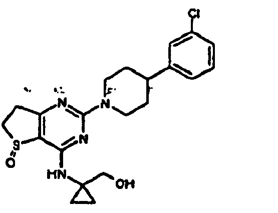 | H | 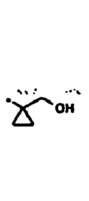 | 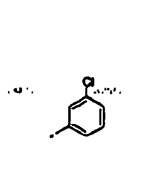 | H | 96 |
| 40 | 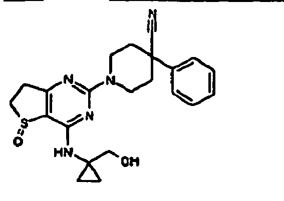 | H | 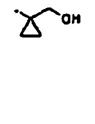 | 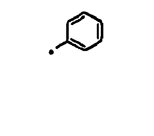 | CN | 94 |
| 41 | 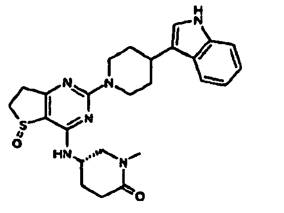 | H | 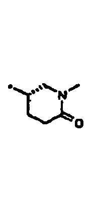 | 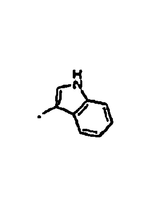 | H | 97 |
| 42 | 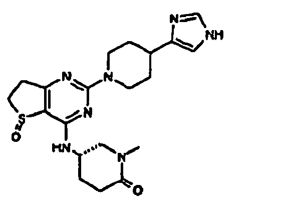 | H | 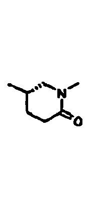 | 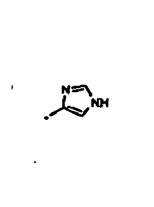 | H | 95 |
| 43 | 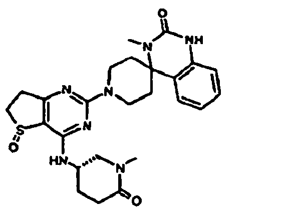 | H | 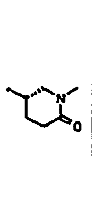 | 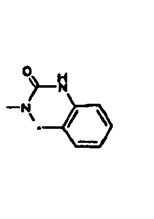 | | 95 |
| 44 | 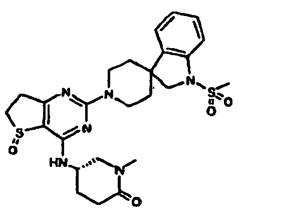 | H | 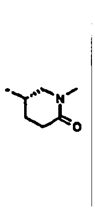 | 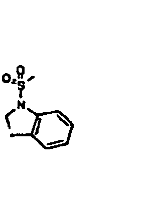 | | 95 |
| 45 | 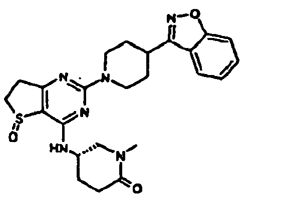 | H | 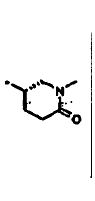 | 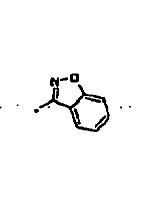 | H | 97 |
| 46 | 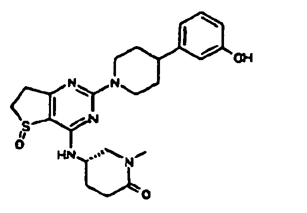 | H | 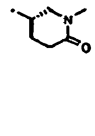 | 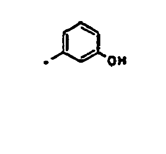 | H | 97 |
| 47 | 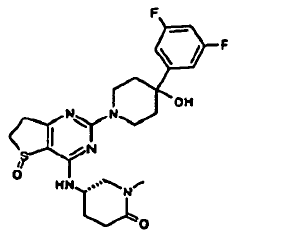 | H | 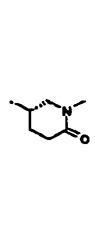 | 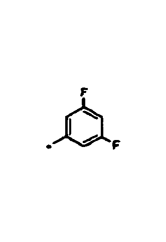 | OH | 97 |
| 48 | 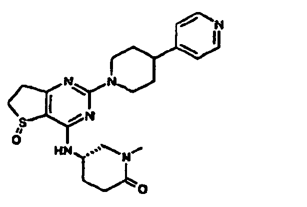 | H | 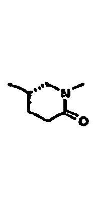 | 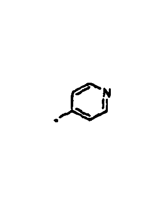 | H | 96 |
| 49 | 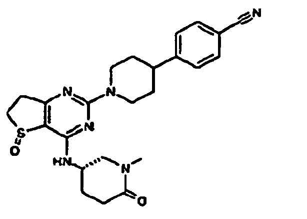 | H | 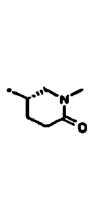 | 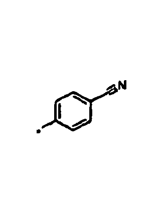 | H | 96 |
| 50 | 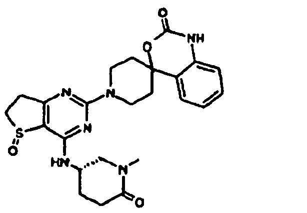 | H | 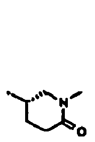 | 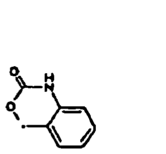 | | 95 |
| 51 | 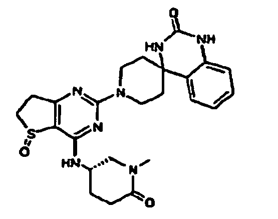 | H | 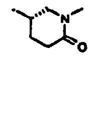 | 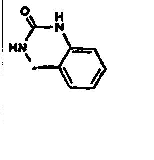 | | 83 |
| 52 | 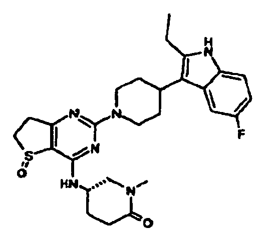 | H | 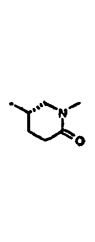 | 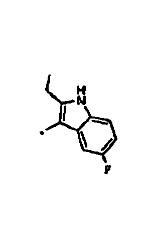 | H | 96 |
| 53 | 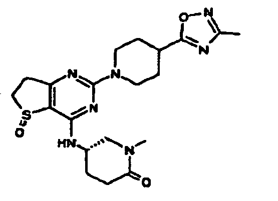 | H | 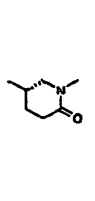 | 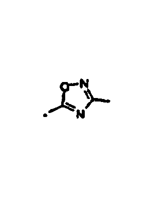 | H | 96 |
| 54 | 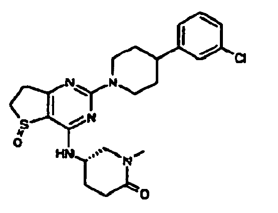 | H | 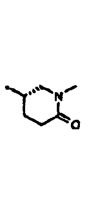 | 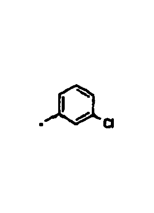 | H | 97 |
| 55 | 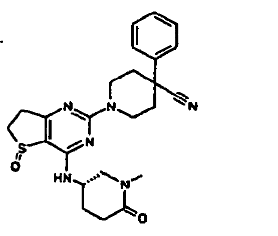 | H | 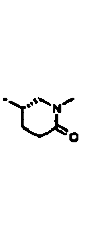 | 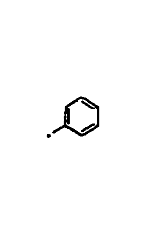 | CN | 96 |
| 56 | 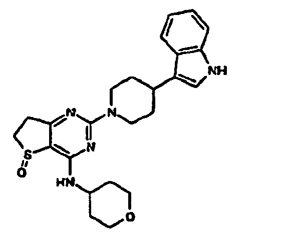 | H | 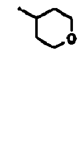 | 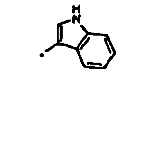 | H | 96 |
| 57 | 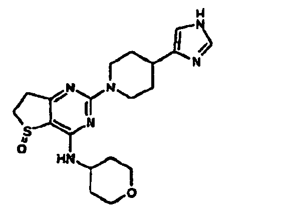 | H | 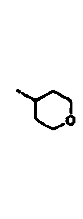 | 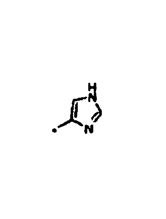 | H | 86 |
| 58 | 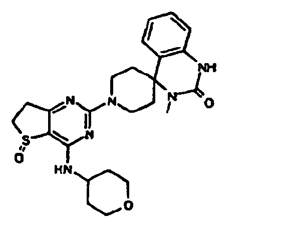 | H | 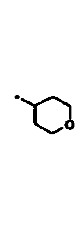 | 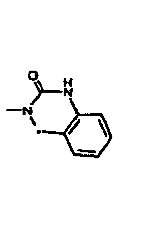 | | 88 |
| 59 | 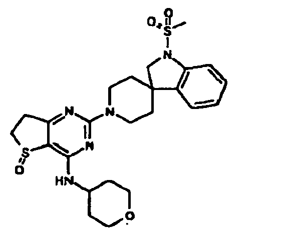 | H | 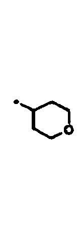 | 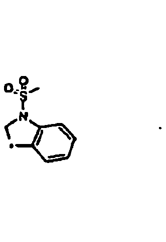 | | 90 |
| 60 | 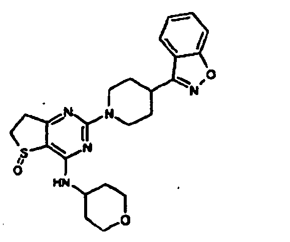 | H | 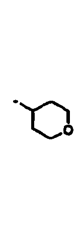 | 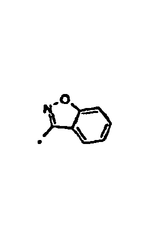 | H | 97 |
| 61 | 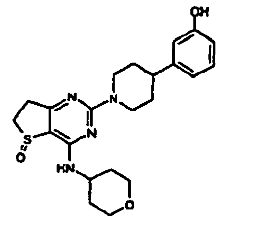 | H | 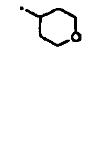 | 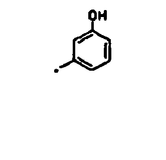 | H | 95 |
| 62 | 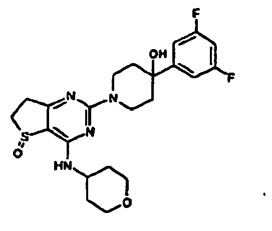 | H | 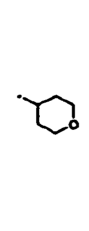 | 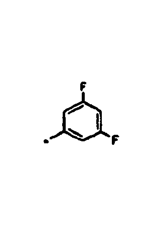 | OH | 96 |
| 63 | 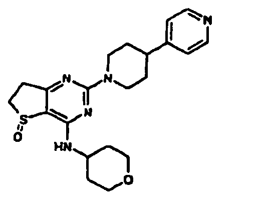 | H | 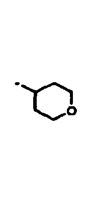 | 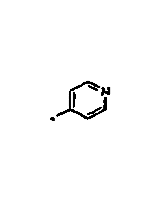 | H | 92 |
| 64 | 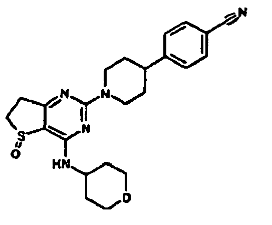 | H | 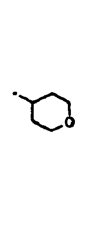 | 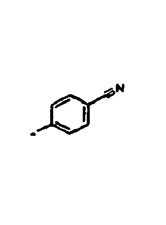 | H | 96 |
| 65 | 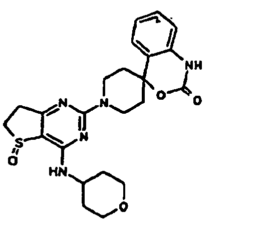 | H | 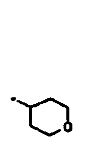 | 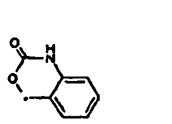 | | 94 |
| 66 | 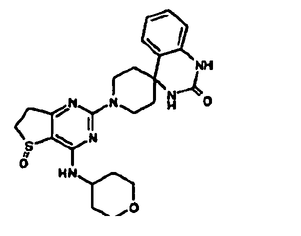 | H | 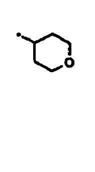 | 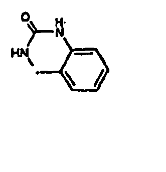 | | 62 |
| 67 | 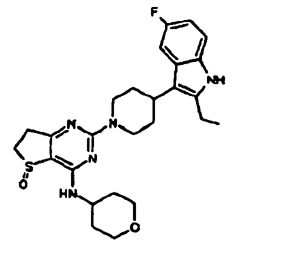 | H | 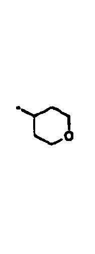 | 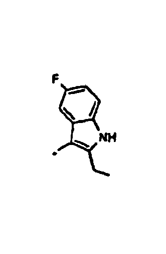 | H | 93 |
| 68 | 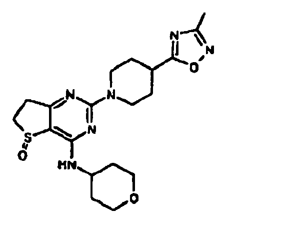 | H | 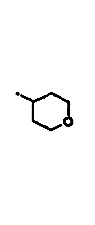 | 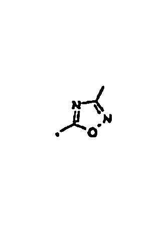 | H | 93 |
| 69 | 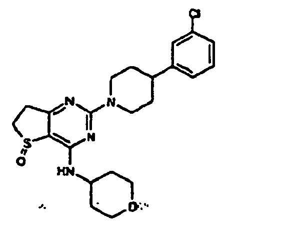 | H | 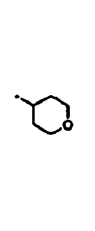 | 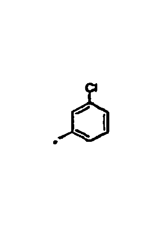 | H | 93 |
| 70 | 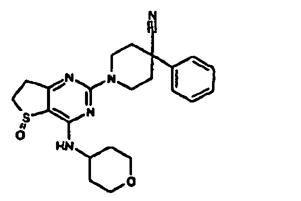 | H | 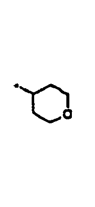 | 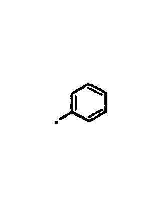 | CN | 95 |
| 71 | 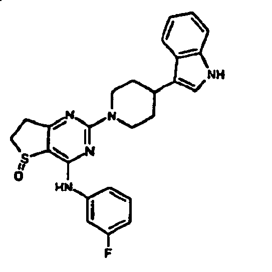 | H | 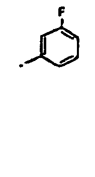 | 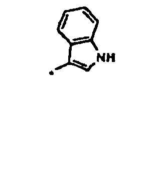 | H | 96 |
| 72 | 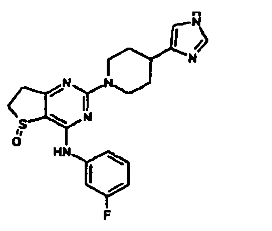 | H | 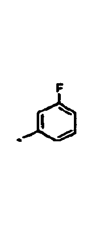 | 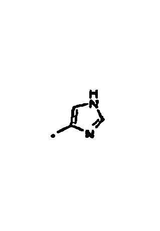 | H | 95 |
| 73 | 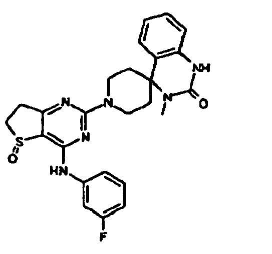 | H | 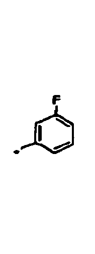 | 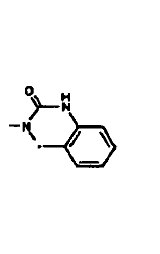 | | 94 |
| 74 | 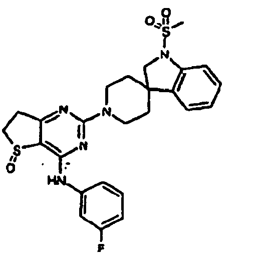 | H | 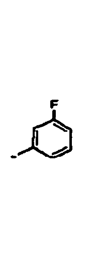 | 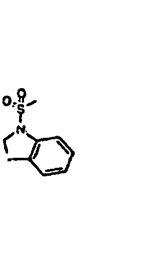 | | 95 |
| 75 | 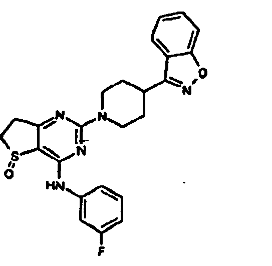 | H | 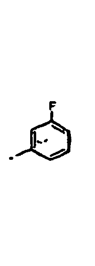 | 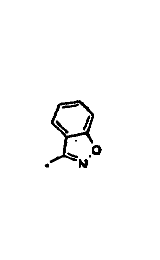 | H | 97 |
| 76 | 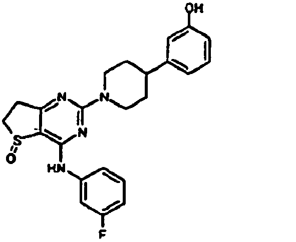 | H | 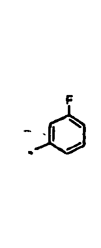 | 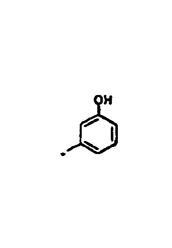 | H | 97 |
| 77 | 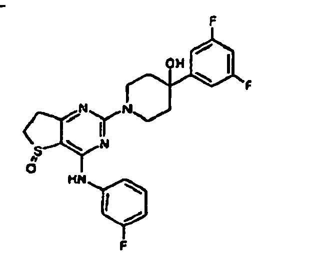 | H | 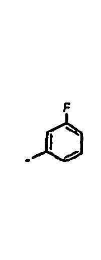 | 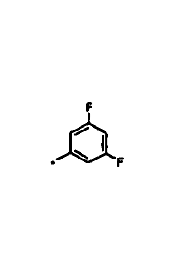 | OH | 96 |
| 78 | 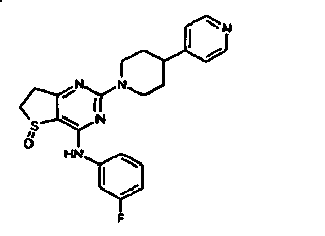 | H | 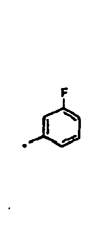 | 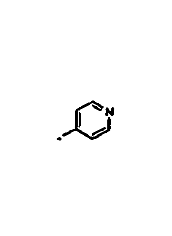 | H | 96 |
| 79 | 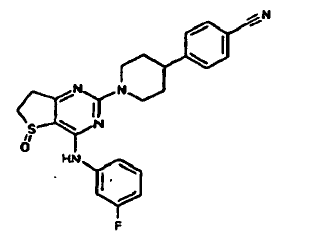 | H | 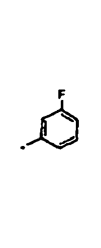 | 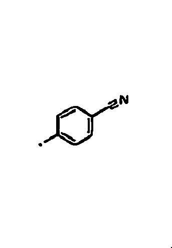 | H | 96 |
| 80 | 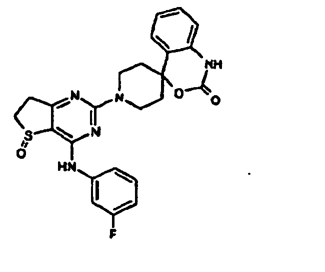 | H | 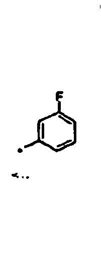 | 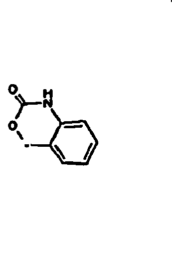 | | 94 |
| 81 | 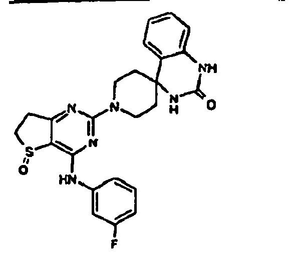 | H | 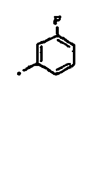 | 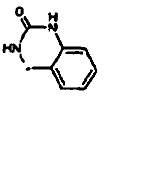 | | 83 |
| 82 | 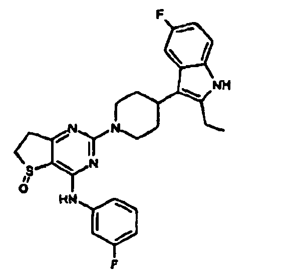 | H | 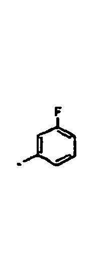 | 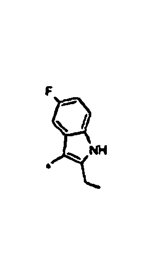 | H | 92 |
| 83 | 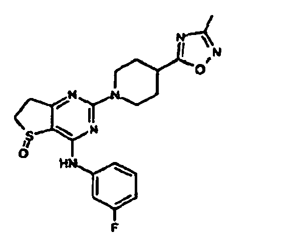 | H | 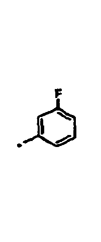 | 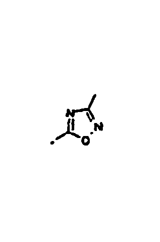 | H | 96 |
| 84 | 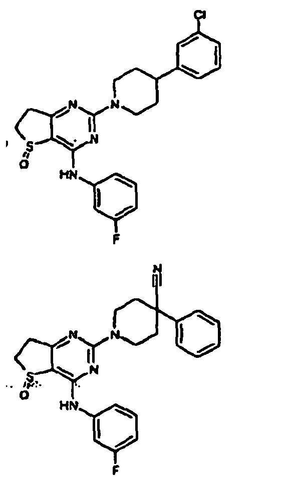 | H | 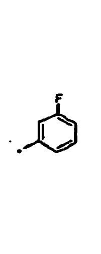 | 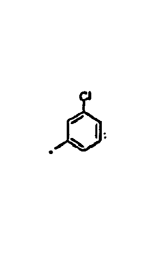 | H | 95 |
| 85 | 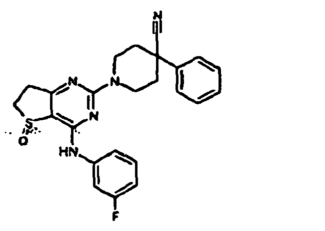 | H | 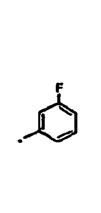 | 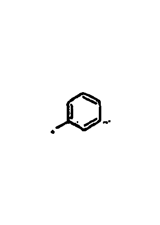 | CN | 96 |
| 86 | 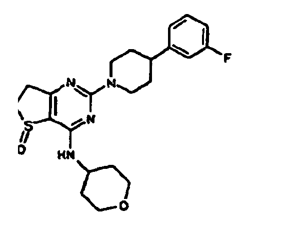 | H | 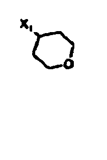 | 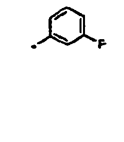 | H | 94 |
| 87 | 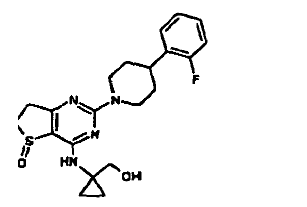 | H | 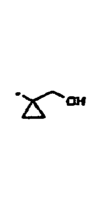 | 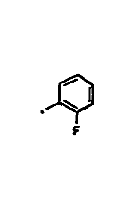 | H | 92 |
| 88 | 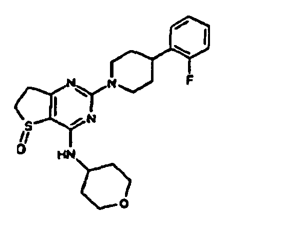 | H | 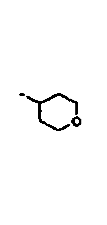 | 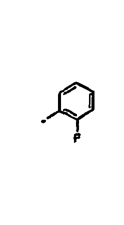 | H | 92 |
| 89 | 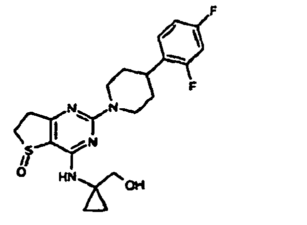 | H | 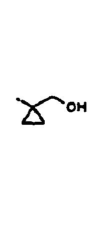 | 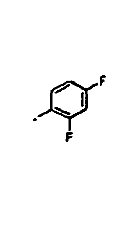 | H | 93 |
| 90 | 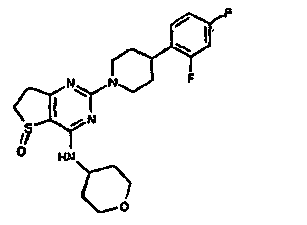 | H | 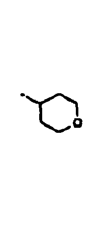 | 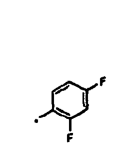 | H | 92 |
| 91 | 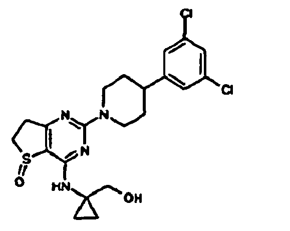 | H | 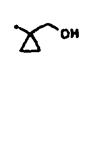 | 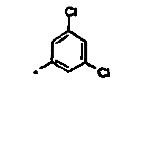 | H | 94 |
| 92 | 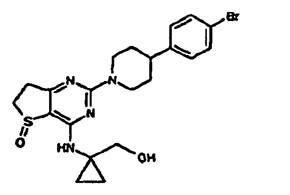 | H | 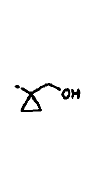 | 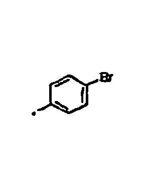 | H | 94 |
| 93 | 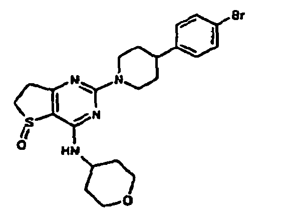 | H | 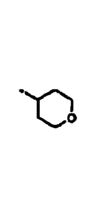 | 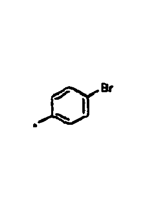 | H | 94 |
| 94 | 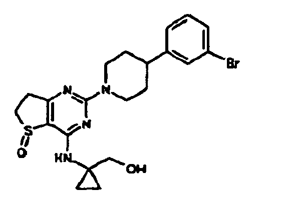 | H | 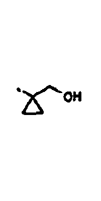 | 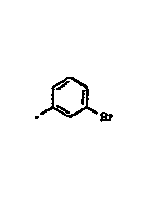 | H | 94 |
| 95 | 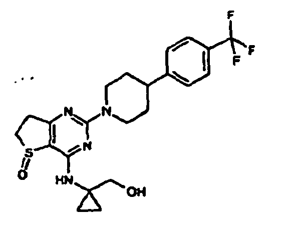 | H | 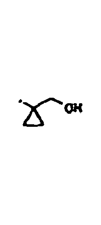 | 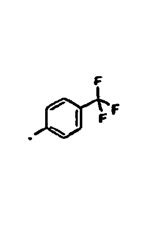 | H | 93 |
| 96 | 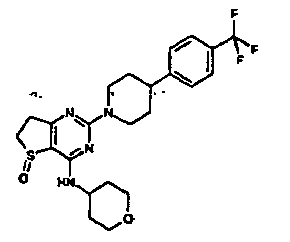 | H | 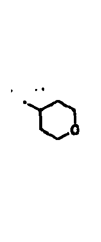 | 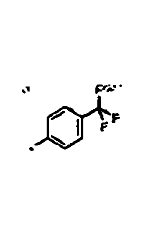 | H | 94 |
| 97 | 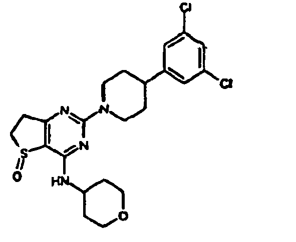 | H | 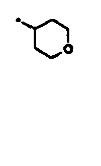 | 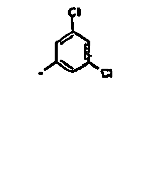 | H | 93 |
| 98 | 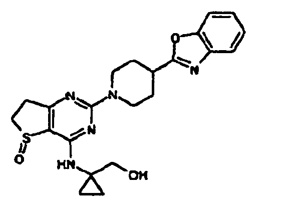 | H | 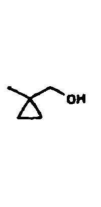 | 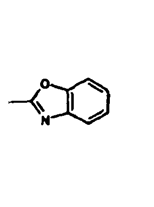 | H | 97 |
| 99 | 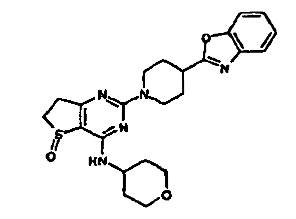 | H | 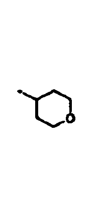 | 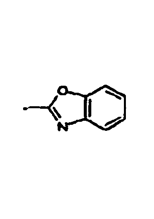 | H | 97 |
| 100 | 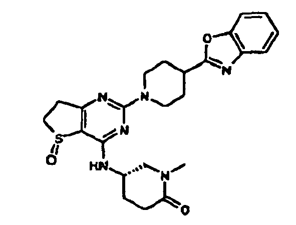 | H | 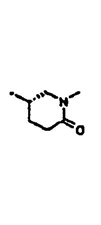 | 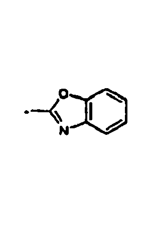 | H | 97 |
| 101 | 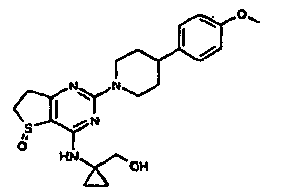 | H | 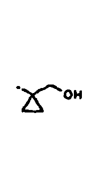 | 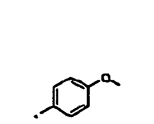 | H | 94 |
| 102 | 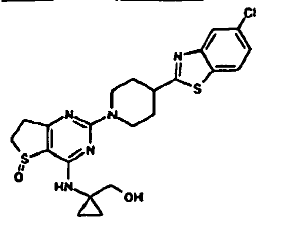 | H | 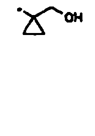 | 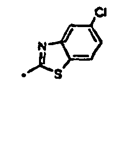 | H | 95 |
| 103 | 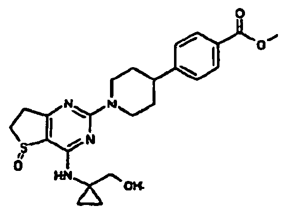 | H | 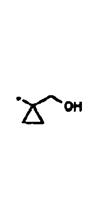 | 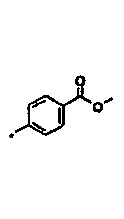 | H | 95 |
| 104 | 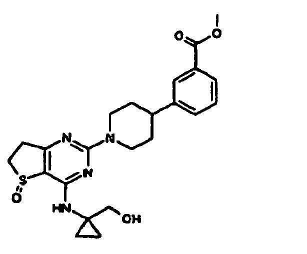 | H | 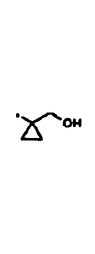 | 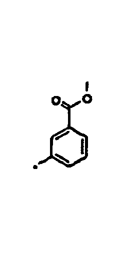 | H | 95 |
| 105 | 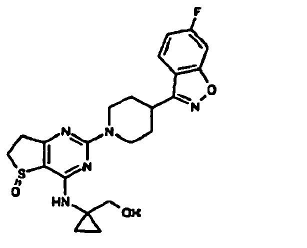 | H | 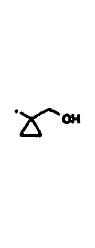 | 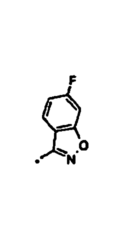 | H | 95 |
| 106 | 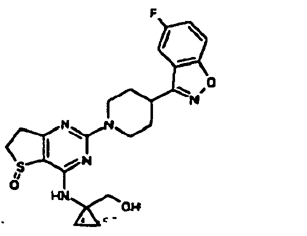 | H | 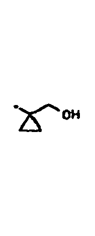 | 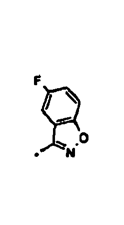 | H | 95 |
| 107 | 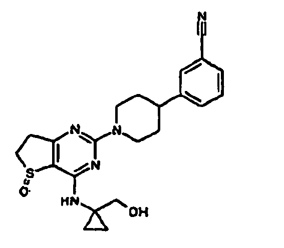 | H | 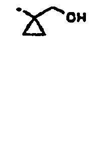 | 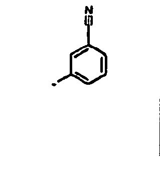 | H | 94 |
| 108 | 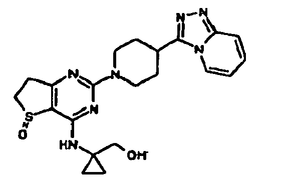 | H | 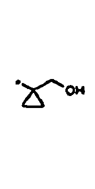 | 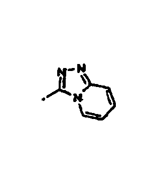 | H | 88 |
| 109 | 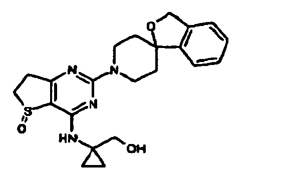 | H | 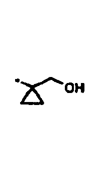 | 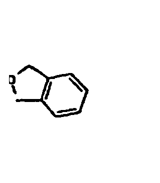 | | 75 |
| 110 | 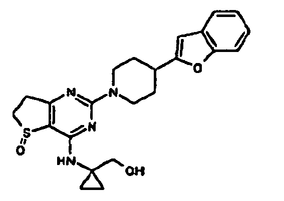 | H | 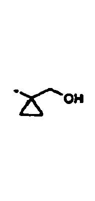 | 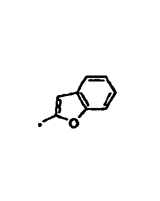 | H | 97 |
| 111 | 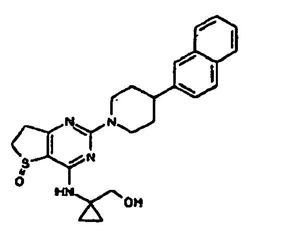 | H | 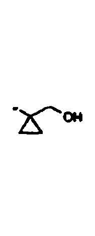 | 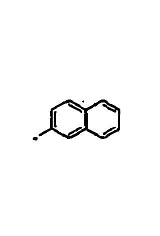 | H | 96 |
| 112 | 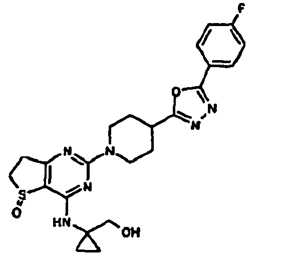 | H | 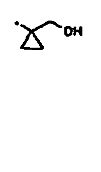 | 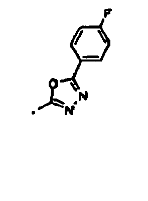 | H | 95 |
| 113 | 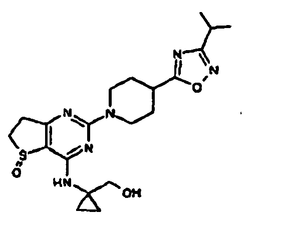 | H | 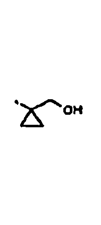 | 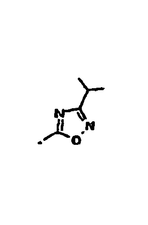 | H | 92 |
| 114 | 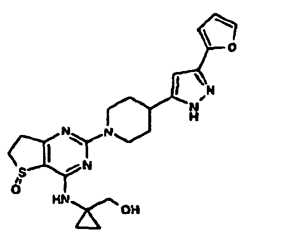 | H | 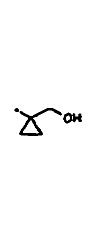 | 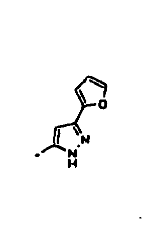 | H | 96 |
| 115 | 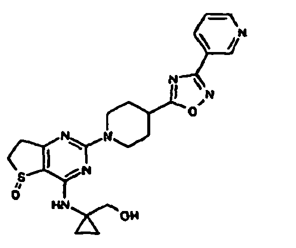 | H | 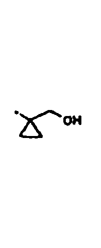 | 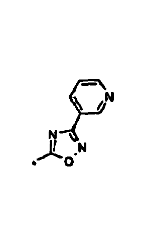 | H | 94 |
| 116 | 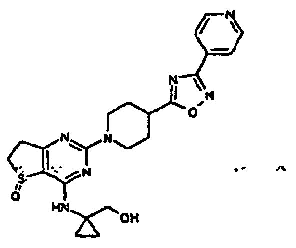 | H | 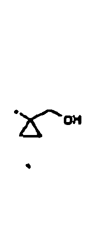 | 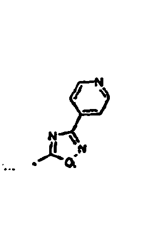 | H | 94 |
| 117 | 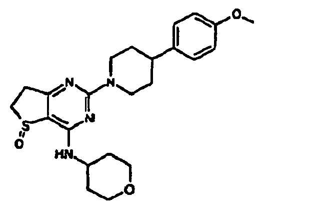 | H | 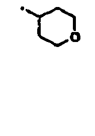 | 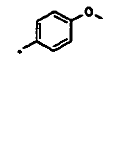 | H | 94 |
| 118 | 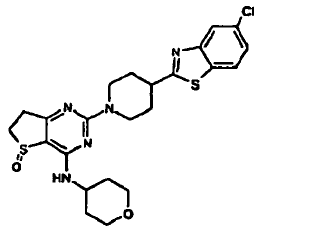 | H | 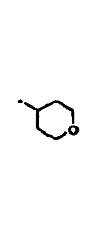 | 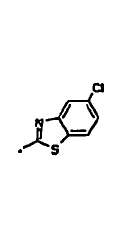 | H | 96 |
| 119 | 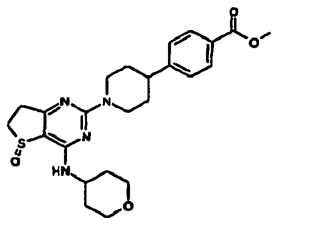 | H | 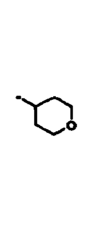 | 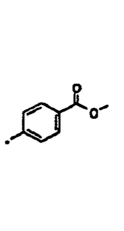 | H | 95 |
| 120 | 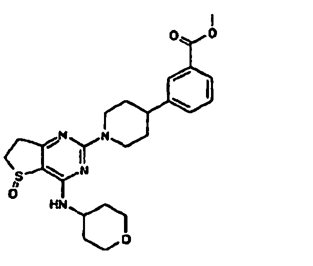 | H | 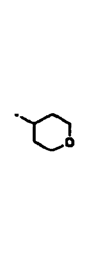 | 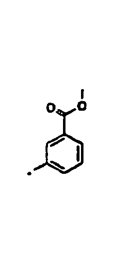 | H | 95 |
| 121 | 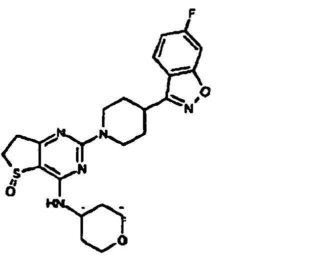 | H | 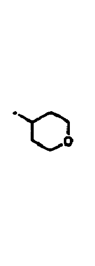 | 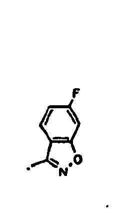 | H | 96 |
| 122 | 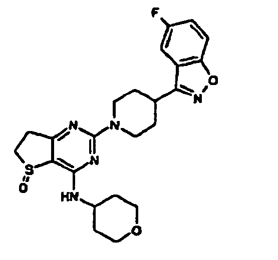 | H | 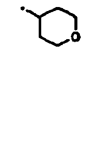 | 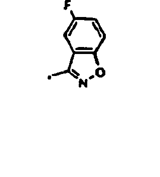 | H | 96 |
| 123 | 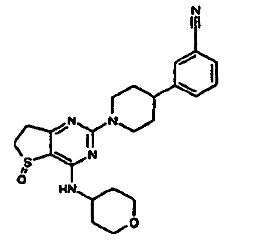 | H | 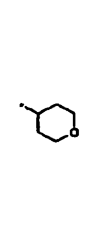 | 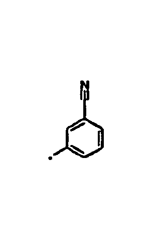 | H | 94 |
| 124 | 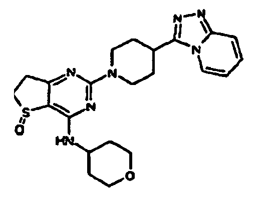 | H | 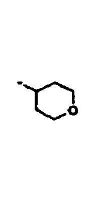 | 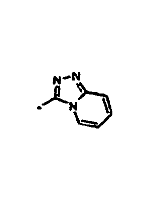 | H | 89 |
| 125 | 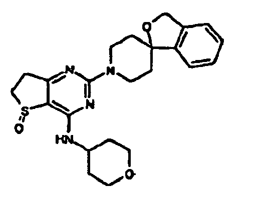 | H | 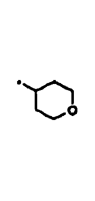 | 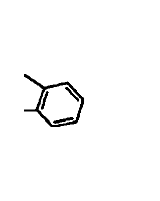 | | 74 |
| 126 | 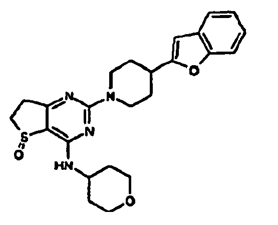 | H | 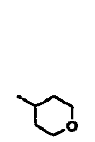 | 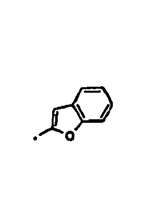 | H | 96 |
| 127 | 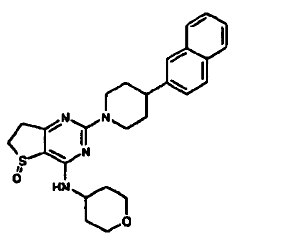 | H | 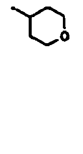 | 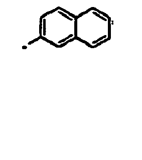 | H | 96 |
| 128 | 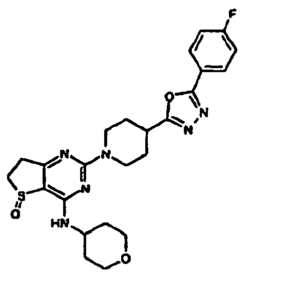 | H | 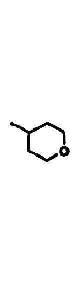 | 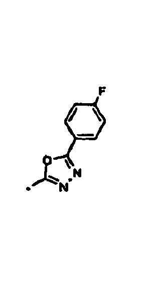 | H | 95 |
| 129 | 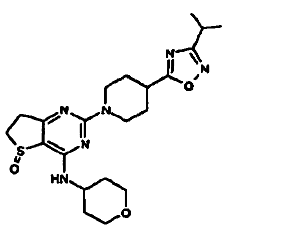 | H | 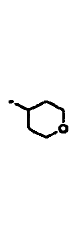 | 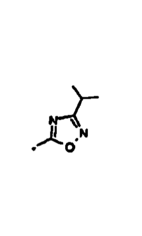 | H | 93 |
| 130 | 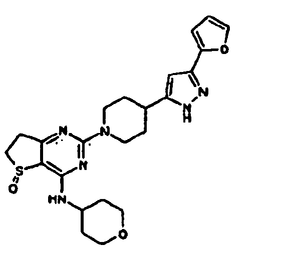 | H | 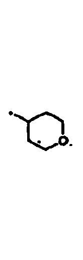 | 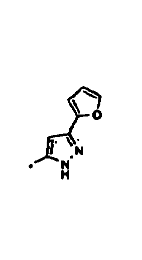 | H | 96 |
| 131 | 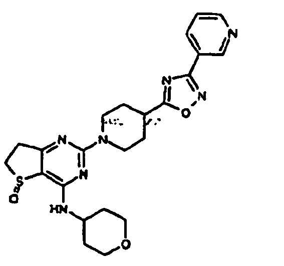 | H | 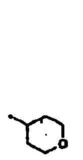 | 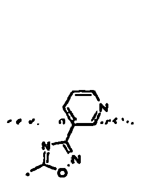 | H | 94 |
| 132 | 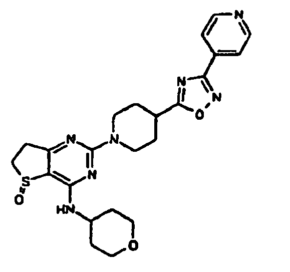 | H | 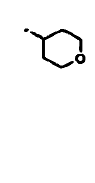 | 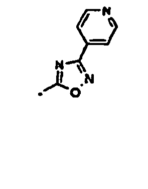 | H | 95 |
| 133 | 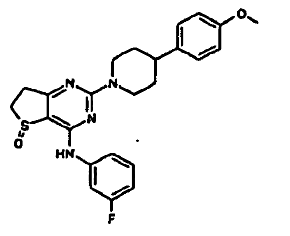 | H | 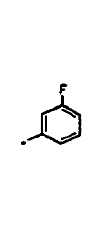 | 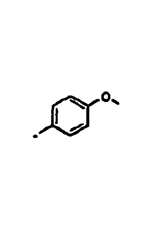 | H | 95 |
| 134 | 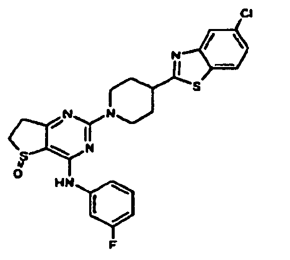 | H | 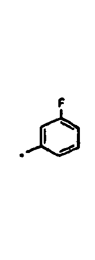 | 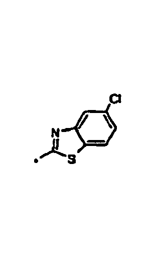 | H | 95 |
| 135 | 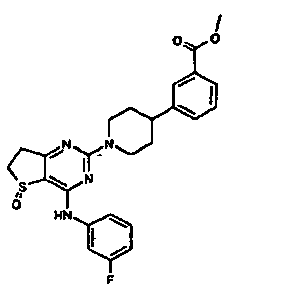 | H | 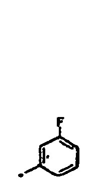 | 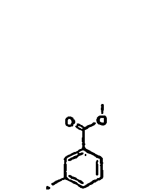 | H | 96 |
| 136 | 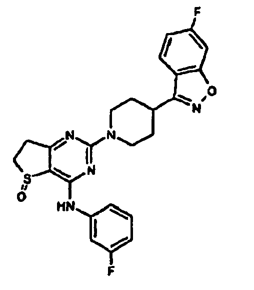 | H | 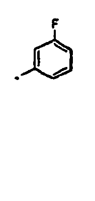 | 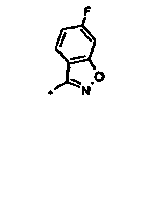 | H | 95 |
| 137 | 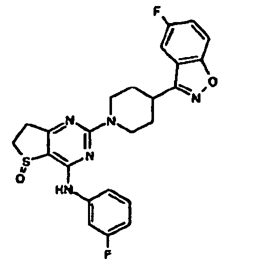 | H | 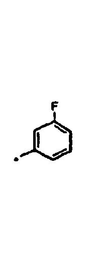 | 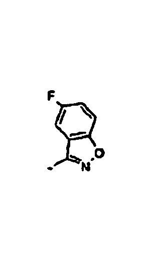 | H | 95 |
| 138 | 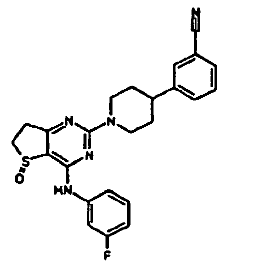 | H | 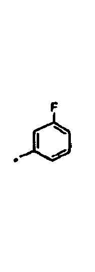 | 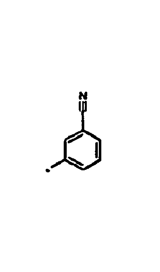 | H | 96 |
| 139 | 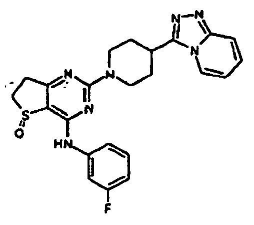 | H | 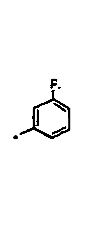 | 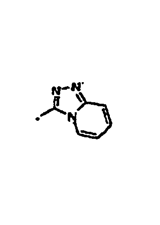 | H | 94 |
| 140 | 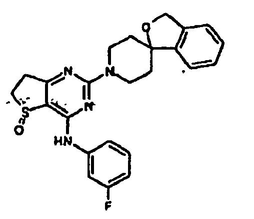 | H | 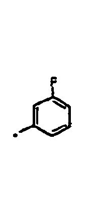 | 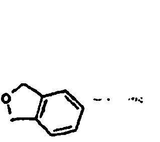 | | 83 |
| 141 | 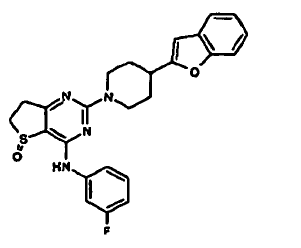 | H | 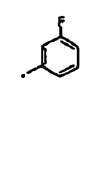 | 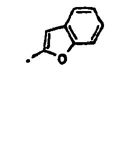 | | 97 |
| 142 | 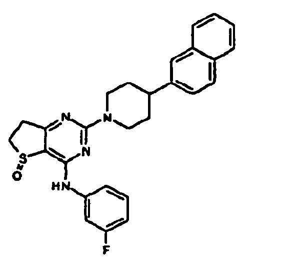 | H | 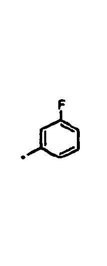 | 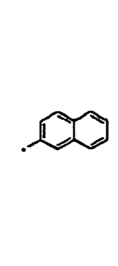 | | 94 |
| 143 | 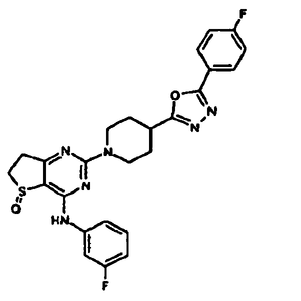 | H | 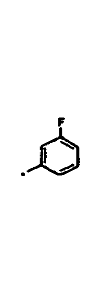 | 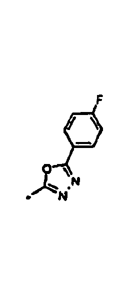 | H | 95 |
| 144 | 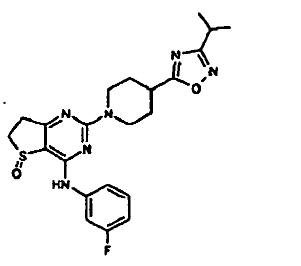 | H | 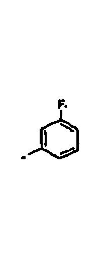 | 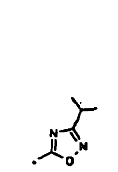 | H | 94 |
| 145 | 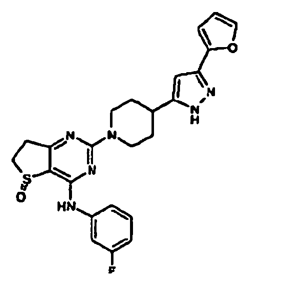 | H | 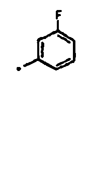 | 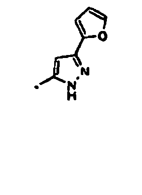 | H | 96 |
| 146 | 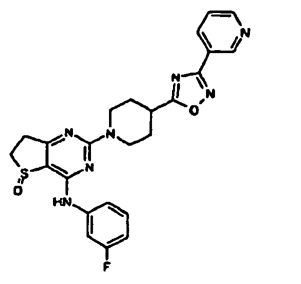 | H | 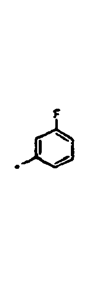 | 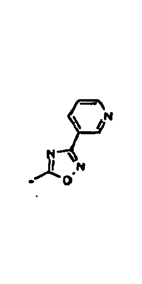 | H | 96 |
| 147 | 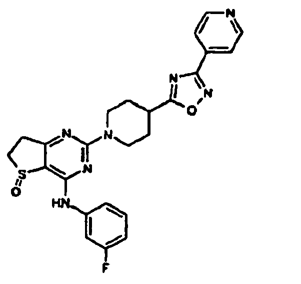 | H | 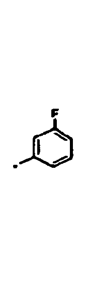 | 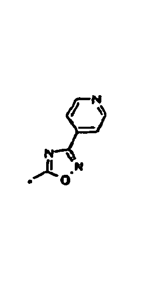 | H | 95 |
| 148 | 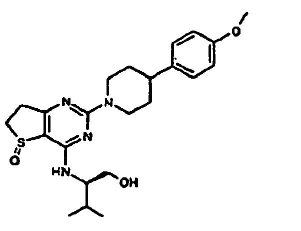 | H | 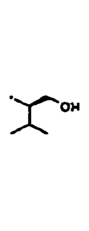 | 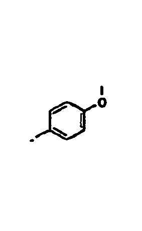 | H | 93 |
| 149 | 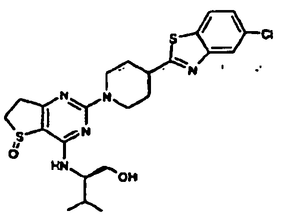 | H | 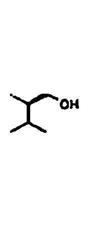 | 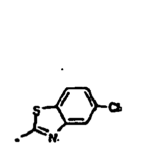 | H | 94 |
| 150 | 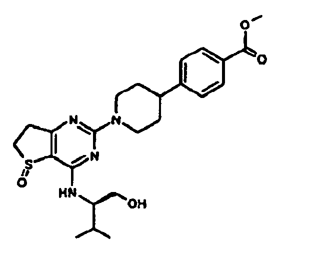 | H | 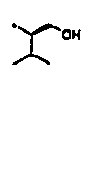 | 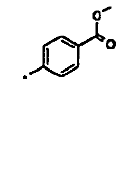 | H | 94 |
| 151 | 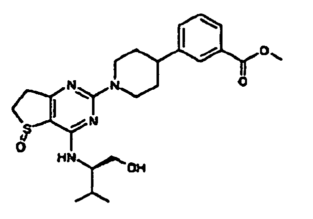 | H | 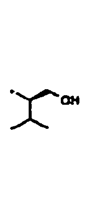 | 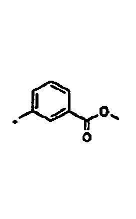 | H | 94 |
| 152 | 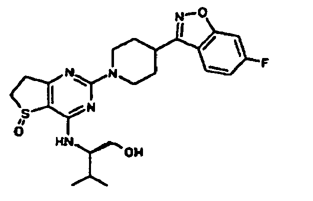 | H | 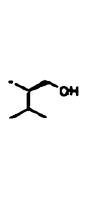 | 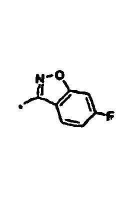 | H | 95 |
| 153 | 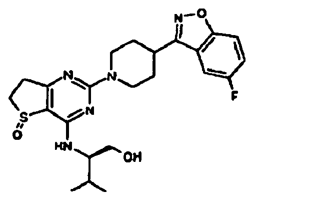 | H | 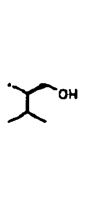 | 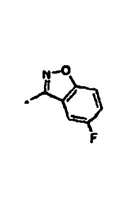 | H | 95 |
| 154 | 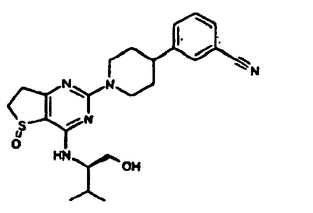 | H | 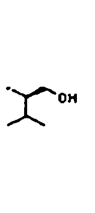 | 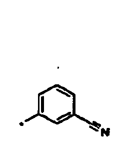 | H | 92 |
| 155 | 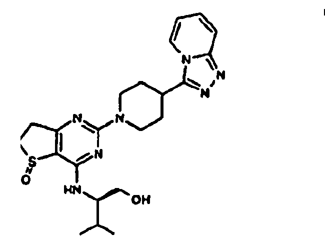 | H | 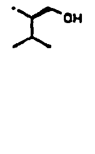 | 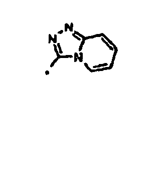 | H | 83 |
| 156 | 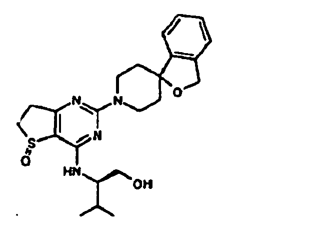 | H | 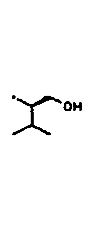 | 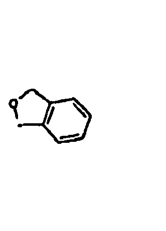 | | 64 |
| 157 | 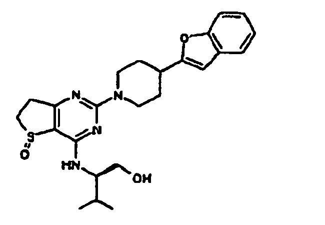 | H | 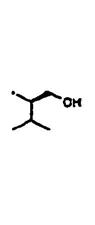 | 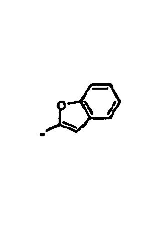 | H | 96 |
| 158 | 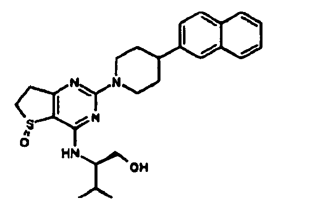 | H | 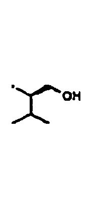 | 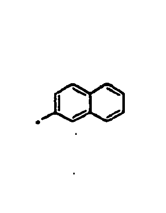 | H | 94 |
| 159 | 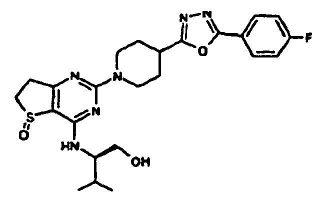 | H | 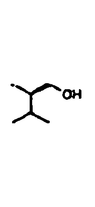 | 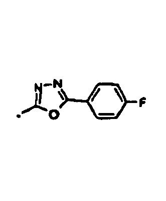 | H | 94 |
| 160 | 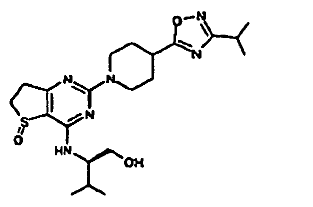 | H | 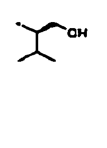 | 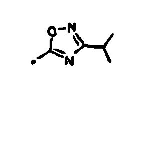 | H | 86 |
| 161 | 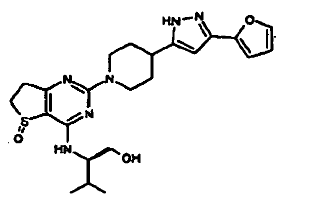 | H | 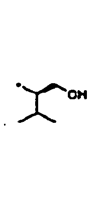 | 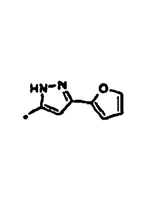 | H | 94 |
| 162 | 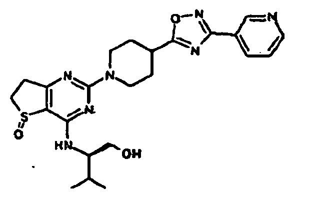 | H | 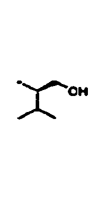 | 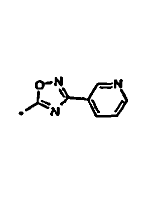 | H | 92 |
| 163 | 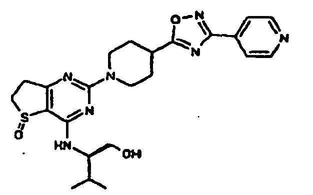 | H | 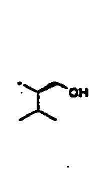 | 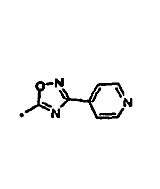 | H | 92 |

In der folgenden Tabelle B werden Detail-Informationen zu den chemischen Synthesen und der Analytik der einzelnen Beispiel-Substanzen 1-163 zusammengefasst.

**Tabelle B: Detail-Informationen zu den Herstellungen der einzelnen Beispiel-Substanzen 1-163**

| # | Herstellung analog zu #* | nicht käuflicher Arylpiperidin-Baustein (V) | Literatur zur Herstellung des nicht käuflichen Arylpiperidin-Bausteins (V) | Analytische HPLC-MS, RT [min], Methode |
|---|---|---|---|---|
| 1 | siehe experim. Teil | | | 1,24 Methode A |
| 2 | siehe experim. Teil | | | 1,32 Methode B |
| 3 | siehe experim. Teil | | | 1,29 Methode A |
| 4 | siehe experim. Teil | | | 1,36 Methode A |
| 5 | siehe experim. Teil | | | 1,18 Methode A |
| 6 | siehe experim. Teil | | | 1,24 Methode A |
| 7 | 6 | | | 1,25 Methode A |
| 8 | 2 | | | 1,21 Methode A |
| 9 | 2 | | | 1,15 Methode D |
| 10 | 6 | | | 1,20 Methode D |
| 11 | 2 | | | 1,14 Methode D |
| 12 | 14 | | | 1,77 Methode C |
| 13 | 14 | | | 1,32 Methode C |
| 14 | siehe experim. Teil | | | 1,58 Methode C |
| 15 | 14 | | | 1,74 Methode C |
| 16 | siehe experim. Teil | | | 1,74 Methode C |
| 17 | 14 | | | 1,65 Methode C |
| 18 | 14 | | | 1,64 Methode C |
| 19 | siehe experim. Teil | | | 1,33 Methode C |
| 20 | 14 | | | 1,73 Methode C |
| 21 | 14 | 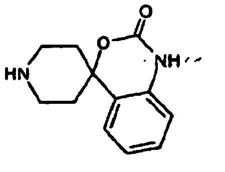 | J. Med. Chem. 1983, 657 | 1,6 Methode C |
| 22 | siehe experim. Teil | | | 1,83 Methode C |
| 23 | 14 | | | 1,55 Methode C |
| 24 | 14 | | | 1,87 Methode C |
| 25 | 14 | | | 1,78 Methode C |
| 26 | 28 | | | 1,72 Methode C |
| 27 | 28 | | | 0,55 Methode C |
| 28 | siehe experim. Teit | | | 1,52 Methode C |
| 29 | siehe experim. Teil | | | 1,77 Methode C |
| 30 | 28 | | | 1,69 Methode C |
| 31 | 28 | | | 1,7 Methode C |
| 32 | 28 | | | 1,59 Methode C |
| 33 | 28 | | | 1,58 Methode C |
| 34 | 28 | | | 0,56 Methode C |
| 35 | 28 | | | 1,68 Methode C |
| 36 | 28 | 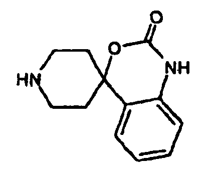 | J. Med. Chem. 1983, 657 | 1,54 Methode C |
| 37 | siehe experim. Teil | | | 1,78 Methode C |
| 38 | 28 | | | 1,48 Methode C |
| 39 | 28 | | | 1,21 Methode D |
| 40 | 28 | | | 1,74 Methode C |
| 41 | 43 | | | 1,68 Methode C |
| 42 | 43 | | | 0,55 Methode C |
| 43 | siehe experim. Teil | | | 1,49 Methode C |
| 44 | 43 | | | 1,66 Methode C |
| 45 | 43 | | | 1,66 Methode C |
| 46 | 43 | | | 1,55 Methode C |
| 47 | 43 | | | 1,54 Methode C |
| 48 | 43 | | | 0,56 Methode C |
| 49 | 43 | | | 1,64 Methode C |
| 50 | 43 | 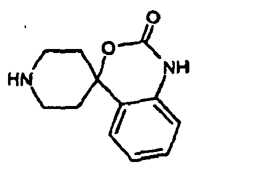 | J. Med. Chem. 1983, 657 | 1,5 Methode C |
| 51 | 43 | 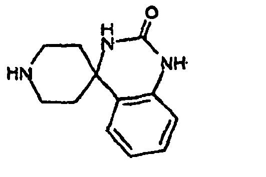 | WO2003/104236 | 1,49 Methode C |
| 52 | 43 | 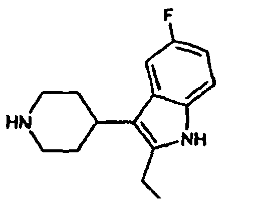 | siehe experim. Teil § 10.4, Baustein (V-1) | 1,73 Methode C |
| 53 | 43 | | | 1,45 Methode C |
| 54 | 43 | | | 1,77 Methode C |
| 55 | siehe experim. Teil | | | 1,71 Methode C |
| 56 | 58 | | | 1,76 Methode C |
| 57 | 58 | | | 1,3 Methode C |
| 58 | siehe experim. Teil | | | 1,56 |
| 59 | 58 | | | 1,72 Methode C |
| 60 | 58 | | | 1,73 Methode C |
| 61 | 58 | | | 1,63 Methode C |
| 62 | 58 | | | 1,61 Methode C |
| 63 | 58 | | | 1,3 Methode C |
| 64 | 58 | | | 1,71 Methode C |
| 65 | 58 | 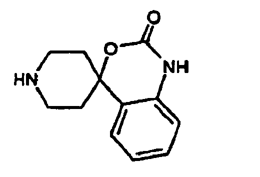 | J. Med. Chem. 1983, 657 | 1,56 Methode C |
| 66 | 58 | 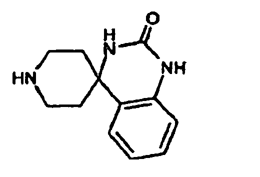 | WO2003/104238 | 1,55 Methode C |
| 67 | 58 | 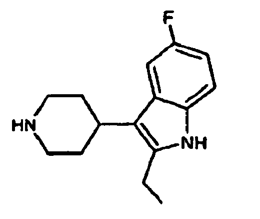 | siehe experim. Teil § 10.4, Baustein (V-1) | 1,81 Methode C |
| 68 | 58 | | | 1,52 Methode C |
| 69 | 58 | | | 1,25 Methode D |
| 70 | 58 | | | 1,78 Methode C |
| 71 | 73 | | | 2,07 Methode C |
| 72 | 73 | | | 1,53 Methode C |
| 73 | siehe experim. Teil | | | 1,81 Methode C |
| 74 | 73 | | | 2,07 Methode C |
| 75 | siehe experim. Teil | | | 2,11 Methode C |
| 76 | 73 | | | 1,92 Methode C |
| 77 | 73 | | | 1,91 Methode C |
| 78 | siehe experim. Teil | | | 1,55 Methode C |
| 79 | 73 | | | 2,09 Methode C |
| 80 | 73 | 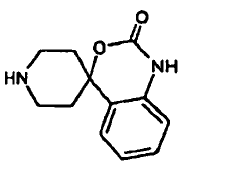 | J. Med. Chem. 1983, 657 | 1,86 Methode C |
| 81 | 73 | 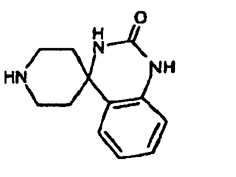 | WO2003/104236 | 1.81 Methode C |
| 82 | siehe experim. Teil | | | 2,12 Methode C |
| 83 | 73 | | | 1,87 Methode C |
| 84 | 73 | | | 2,29 Methode C |
| 85 | 73 | | | 2,24 Methode C |
| 86 | 58 | | | 1,20 Methode D |
| 87 | 28 | | | 1,15 Methode D |
| 88 | 58 | | | 1,20 Methode D |
| 89 | siehe experim. Teil | | | 1,18 Methode D |
| 90 | siehe experim. Teil | | | 1,23 Methode D |
| 91 | siehe experim. Teil | | | 1.30 Methode D |
| 92 | siehe experim. Teil | | | 1,23 Methode D |
| 93 | siehe experim. Teil | | | 1,28 Methode D |
| 94 | 28 | | | 1,22 Methode D |
| 95 | siehe experim. Teil | | | 1,25 Methode D |
| 96 | siehe experim. Teil | | | 1,29 Methode D |
| 97 | siehe experim. Teil | | | 1,29 Methode A |
| 98 | siehe experim. Teil | | | 1,22 Methode B |
| 99 | siehe experim. Teil | | | 1,23 Methode B |
| 100 | siehe experim. Teil | | | 1.18 Methode B |
| 101 | 28 | | | 1,74 Methode C |
| 102 | 28 | | | 1,86 Methode C |
| 103 | 28 | | | 1,73 Methode C |
| 104 | 28 | | | 1,73 Methode C |
| 105 | 28 | | | 1,76 Methode C |
| 106 | 28 | | | 1,74 Methode C |
| 107 | 28 | | | 1,71 Methode C |
| 108 | 28 | | | 1,33 Methode C |
| 109 | 28 | | | 1,71 Methode C |
| 110 | 28 | | | 1,83 Methode C |
| 111 | 28 | | | 1,89 Methode C |
| 112 | 28 | | | 1,69 Methode C |
| 113 | 28 | | | 1,66 Methode C |
| 114 | 28 | | | 1,61 Methode C |
| 115 | 28 | | | 1,46 Methode C |
| 116 | 28 | | | 1,43 Methode C |
| 117 | 58 | | | 1,77 Methode C |
| 118 | 58 | | | 1,91 Methode C |
| 119 | 58 | | | 1,77 Methode C |
| 120 | 58 | | | 1,78 Methode C |
| 121 | 58 | | | 1,79 Methode C |
| 122 | 58 | | | 1,78 Methode C |
| 123 | 58 | | | 1,74 Methode C |
| 124 | 58 | | | 1,36 Methode C |
| 125 | 58 | | | 1,74 Methode C |
| 126 | 58 | | | 1,88 Methode C |
| 127 | 58 | | | 1,92 Methode C |
| 128 | 58 | | | 1,73 Methode C |
| t29 | 58 | | | 1,69 Methode C |
| 130 | 58 | | | 1,64 Methode C |
| 131 | 58 | | | 1,5 Methode C |
| 132 | 58 | | | 1,45 Methode C |
| 133 | 73 | | | 2,14 Methode C |
| 134 | 73 | | | 2,44 Methode C |
| 135 | 73 | | | 2,14 Methode C |
| 136 | 73 | | | 2,17 Methode C |
| 137 | 73 | | | 2,16 Methode C |
| 138 | 73 | | | 2,09 Methode C |
| 139 | 73 | | | 1,6 Methode C |
| 140 | 73 | | | 2,12 Methode C |
| 141 | 73 | | | 2,31 Methode C |
| 142 | 73 | | | 2,34 Methode C |
| 143 | 73 | | | 2,07 Methode C |
| 144 | 73 | | | 2,08 Methode C |
| 145 | siehe experim. Teil | | | 1,89 Methode C |
| 146 | 73 | | | 1,79 Methode C |
| 147 | siehe experim. Teil | | | 1,72 Methode C |
| 148 | 14 | | | 1,8 Methode C |
| 149 | 14 | | | 1,9 Methode C |
| 150 | 14 | | | 1,78 Methode C |
| 151 | 14 | | | 1,79 Methode C |
| 152 | 14 | | | 1,81 Methode C |
| 153 | 14 | | | 1,8 Methode C |
| 154 | 14 | | | 1,76 Methode C |
| 155 | 14 | | | 1,39 Methode C |
| 156 | 14 | | | 1,76 Methode C |
| 157 | 14 | | | 1,88 Methode C |
| 158 | 14 | | | 1,94 Methode C |
| 159 | 14 | | | 1,74 Methode C |
| 160 | 14 | | | 1,71 Methode C |
| 161 | siehe experim. Teil | | | 1,67 Methode C |
| 162 | 14 | | | 1,52 Methode C |
| 163 | siehe experim. Teil | | | 1,48 Methode C |

| | | | | |
|---|---|---|---|---|
| * Das Beispiel kann analog hergestellt und aufgereinigt werden. | | | | |

**Tabelle C: Chemische Strukturen der Beispiel-Substanzen 164 -332**

| Bsp. | Struktur | R¹ | R² | R³ | R⁴ | % Inhibition PDE4B @ 1 µM |
|---|---|---|---|---|---|---|
| 164 | 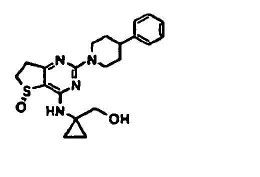 | H | 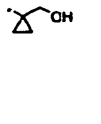 | 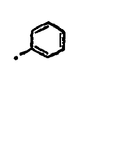 | H | 92 |
| 165 | 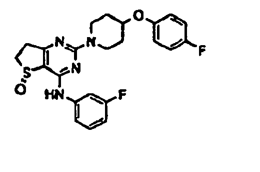 | H | 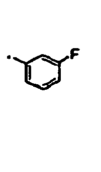 | 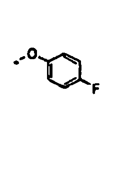 | H | 92 |
| 166 | 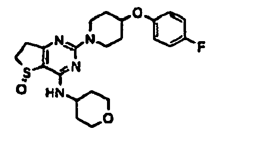 | H | 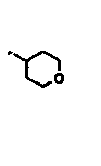 | 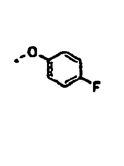 | H | 92 |
| 167 | 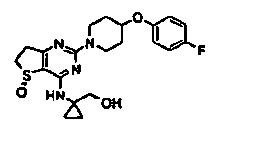 | H | 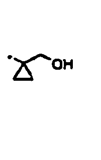 | 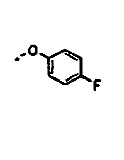 | H | 91 |
| 168 | 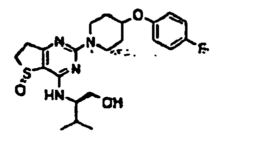 | H | 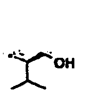 | 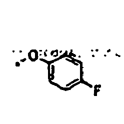 | H | 89 |
| 169 | 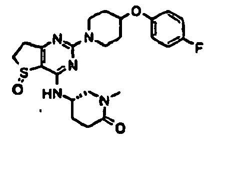 | H | 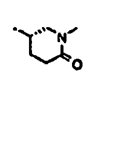 | 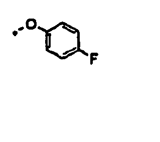 | H | 92 |
| 170 | 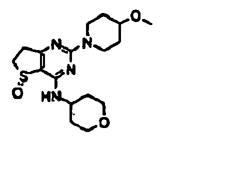 | H | 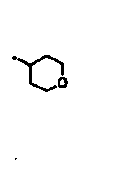 | 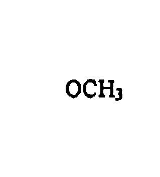 | H | 77 |
| 172 | 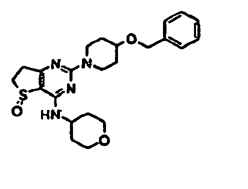 | H | 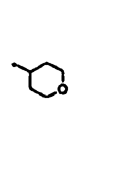 | 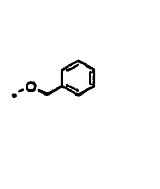 | H | 91 |
| 173 | 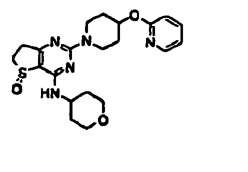 | H | 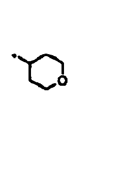 | 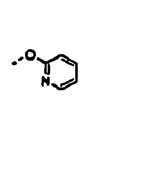 | H | 95 |
| 174 | 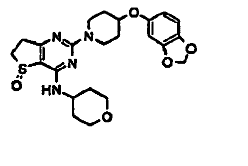 | H | 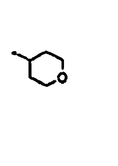 | 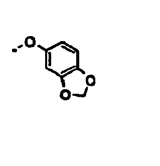 | H | 92 |
| 175 | 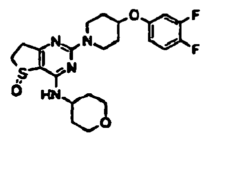 | H | 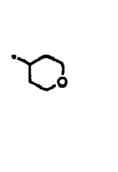 | 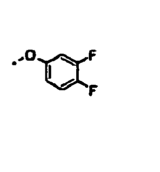 | H | 89 |
| 176 | 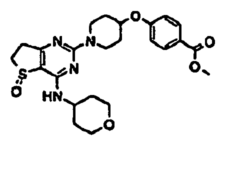 | H | 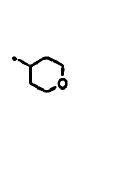 | 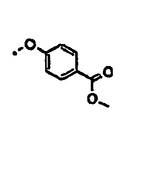 | H | 93 |
| 177 | 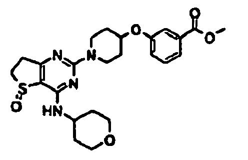 | H | 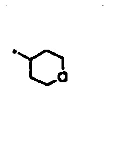 | 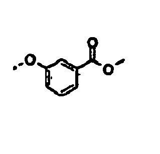 | H | 93 |
| 178 | 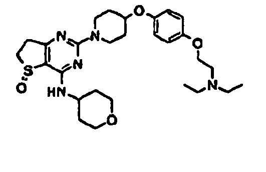 | H | 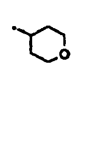 | 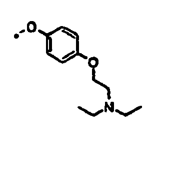 | H | 92 |
| 179 | 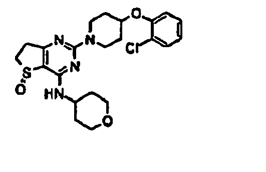 | H | 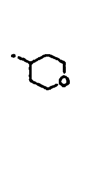 | 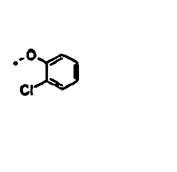 | H | 75 |
| 180 | 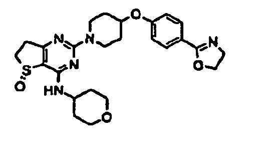 | H | 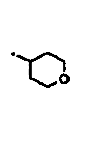 | 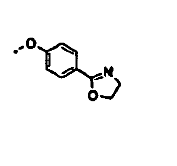 | H | 93 |
| 181 | 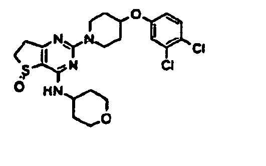 | H | 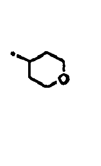 | 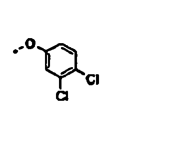 | H | 92 |
| 182 | 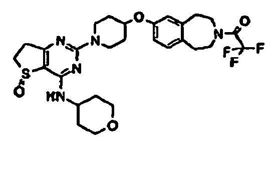 | H | 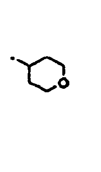 | 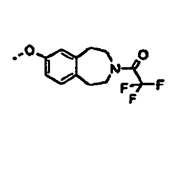 | H | 82 |
| 183 | 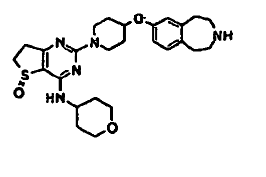 | H | 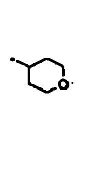 | 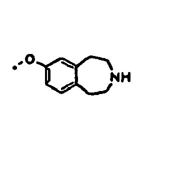 | H | 91 |
| 184 | 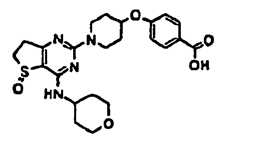 | H | 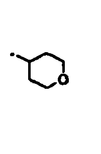 | 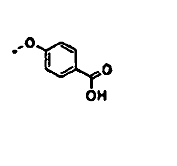 | H | 92 |
| 185 | 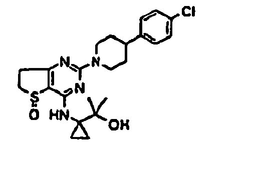 | H | 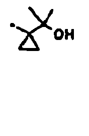 | 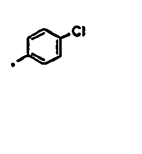 | H | 94 |
| 186 | 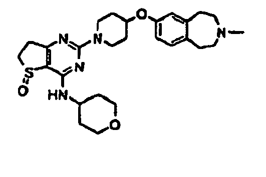 | H | 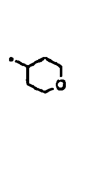 | 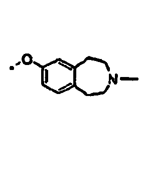 | H | 96 |
| 187 | 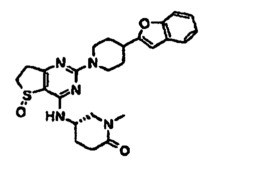 | H | 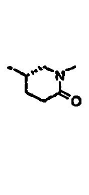 | 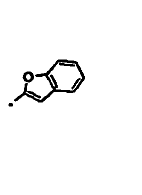 | H | 98 |
| 188 | 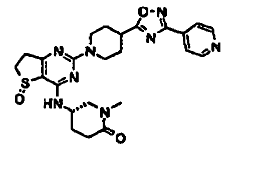 | H | 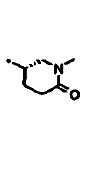 | 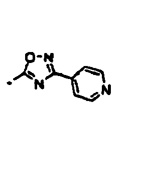 | H | 97 |
| 189 | 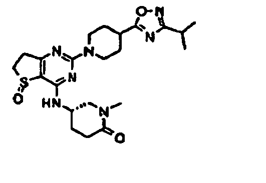 | H | 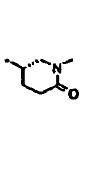 | 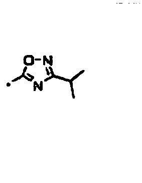 | H | 97 |
| 190 | 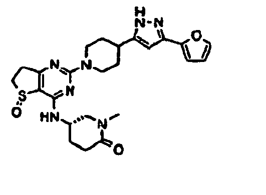 | H | 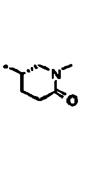 | 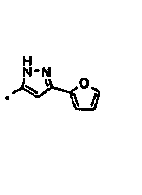 | H | 97 |
| 191 | 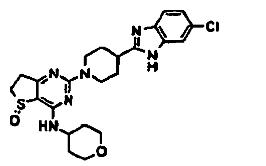 | H | 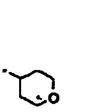 | 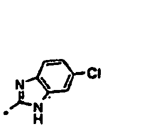 | H | 96 |
| 192 | 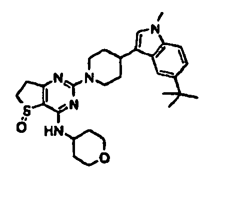 | H | 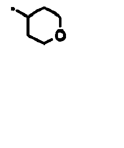 | 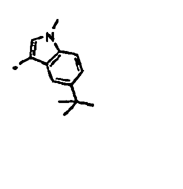 | H | 95 |
| 193 | 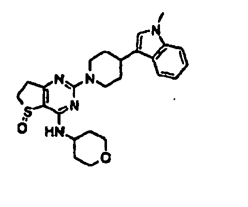 | H | 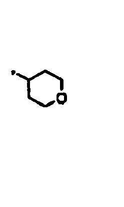 | 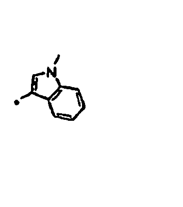 | H | 96 |
| 194 | 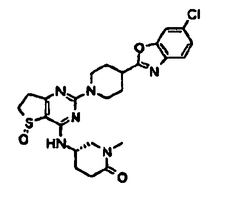 | H | 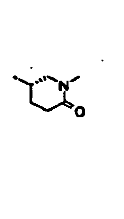 | 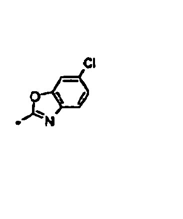 | H | 97 |
| 195 | 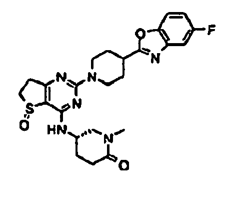 | H | 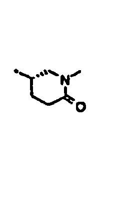 | 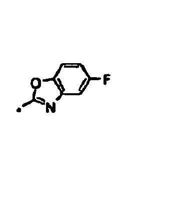 | H | 96 |
| 196 | 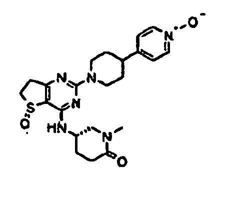 | H | 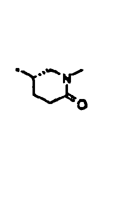 | 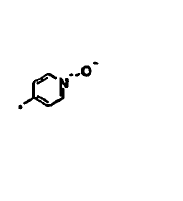 | H | 94 |
| 197 | 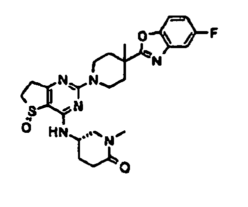 | H | 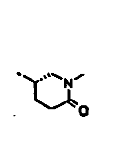 | 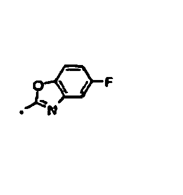 | CH₃ | 96 |
| 198 | 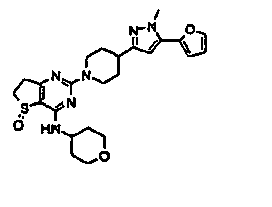 | H | 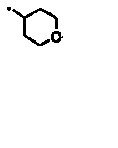 | 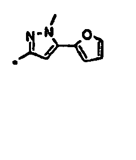 | H | 94 |
| 199 | 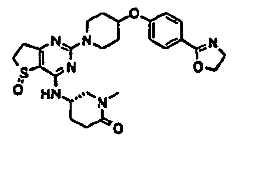 | H | 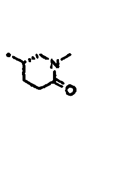 | 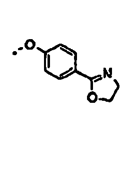 | H | 96 |
| 200 | 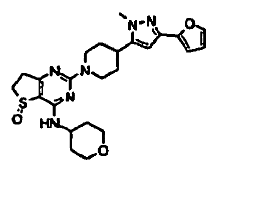 | H | 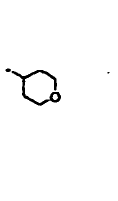 | 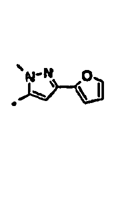 | H | 97 |
| 201 | 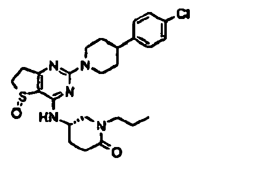 | H | 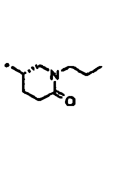 | 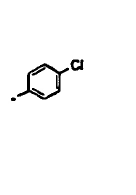 | H | 95 |
| 202 | 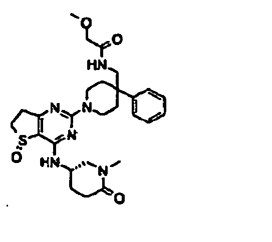 | H | 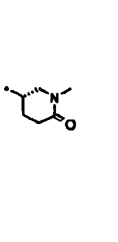 | 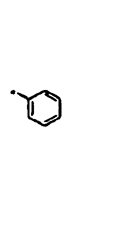 | 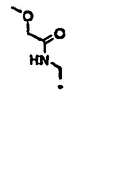 | 96 |
| 203 | 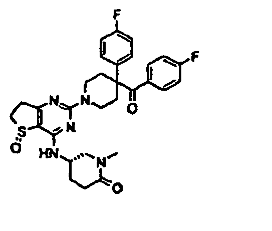 | H | 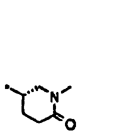 | 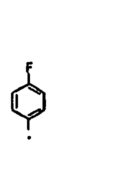 | 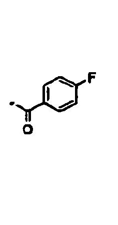 | 96 |
| 204 | 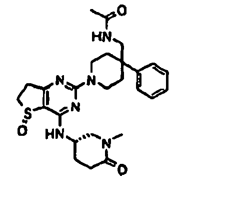 | H | 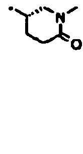 | 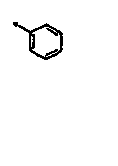 | 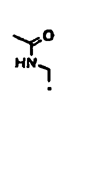 | 96 |
| 205 | 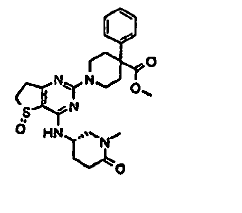 | H | 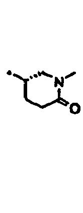 | 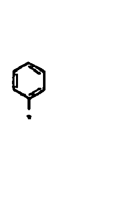 | 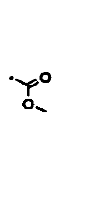 | 96 |
| 206 | 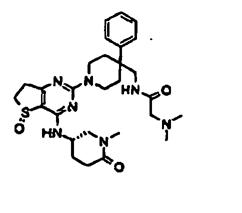 | H | 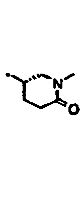 | 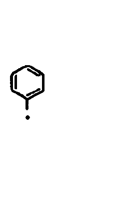 | 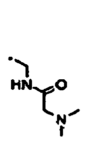 | 95 |
| 207 | 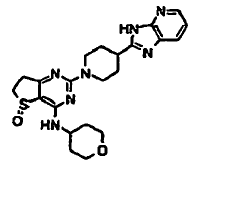 | H | 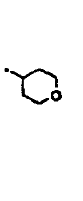 | 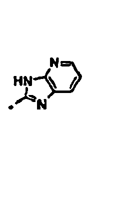 | H | 95 |
| 208 | 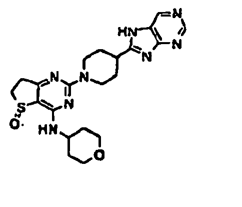 | H | 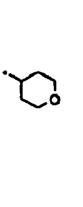 | 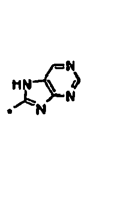 | H | 82 |
| 209 | 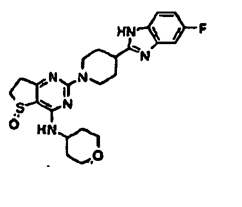 | H | 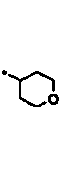 | 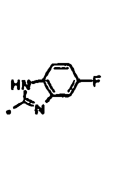 | H | 95 |
| 210 | 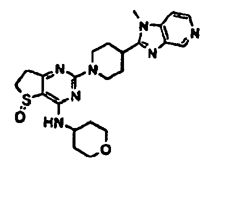 | H | 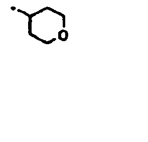 | 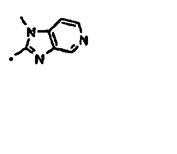 | H | 85 |
| 211 | 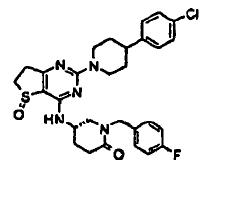 | H | 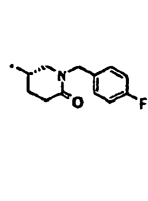 | 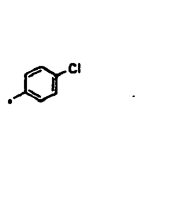 | H | 94 |
| 212 | 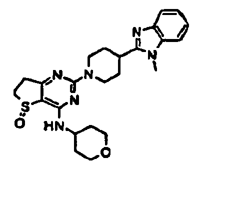 | H | 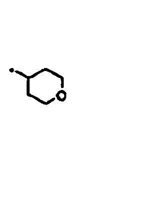 | 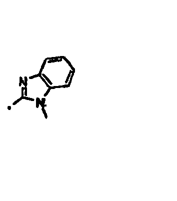 | H | 93 |
| 213 | 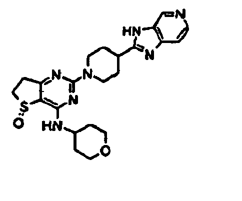 | H | 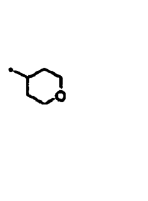 | 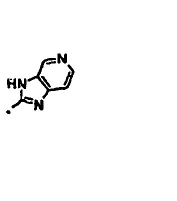 | H | 88 |
| 214 | 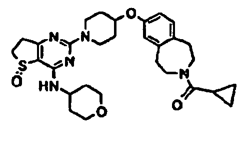 | H | 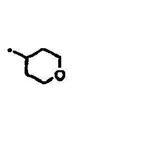 | 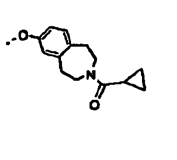 | H | 94 |
| 215 | 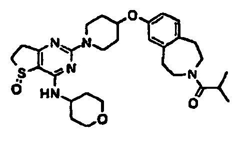 | H | 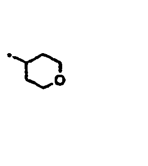 | 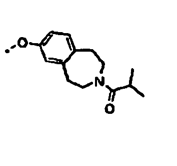 | H | 79 |
| 216 | 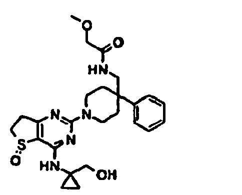 | H | 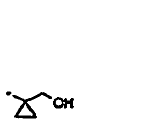 | 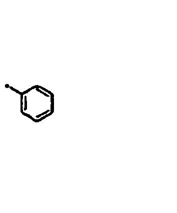 | 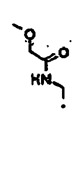 | 80 |
| 217 | 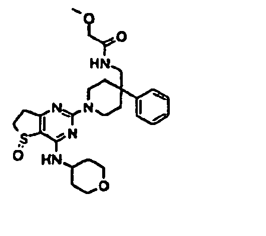 | H | 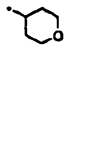 | 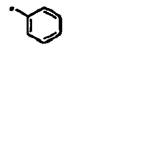 | 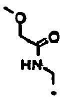 | 88 |
| 218 | 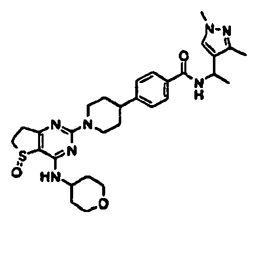 | H | 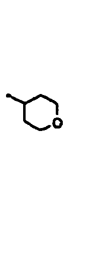 | 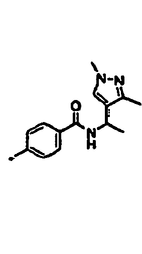 | H | 94 |
| 219 | 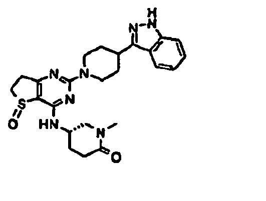 | H | 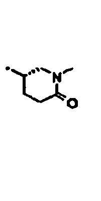 | 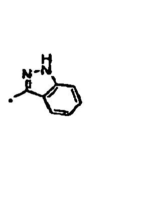 | H | 82 |
| 220 | 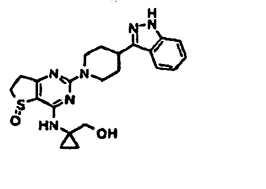 | H | 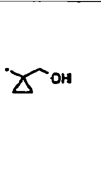 | 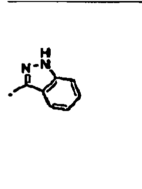 | H | 93 |
| 221 | 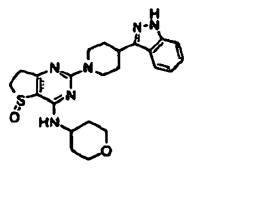 | H | 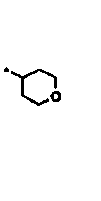 | 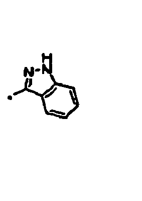 | H | 94 |
| 222 | 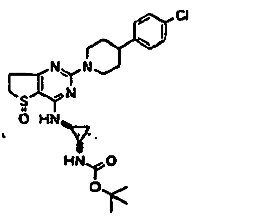 | H | 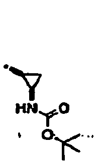 | 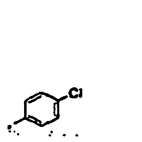 | H | 89 |
| 223 | 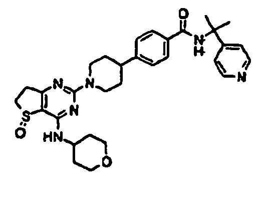 | H | 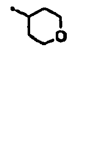 | 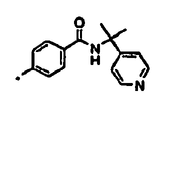 | H | 80 |
| 224 | 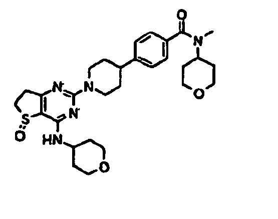 | H | 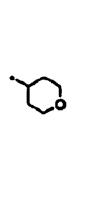 | 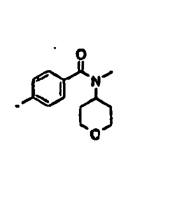 | H | 93 |
| 225 | 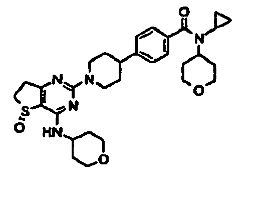 | H | 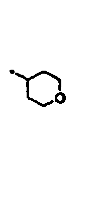 | 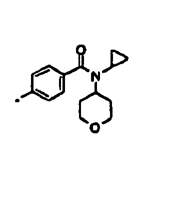 | H | 89 |
| 226 | 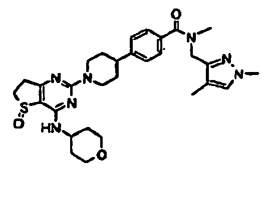 | H | 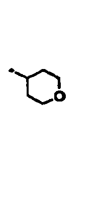 | 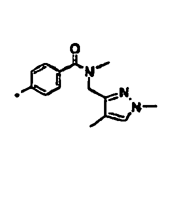 | H | 95 |
| 227 | 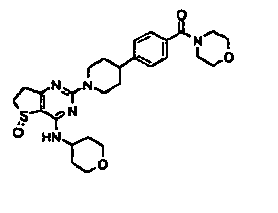 | H | 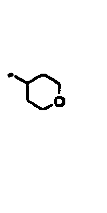 | 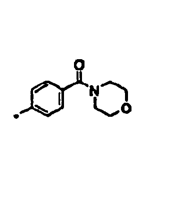 | H | 83 |
| 228 | 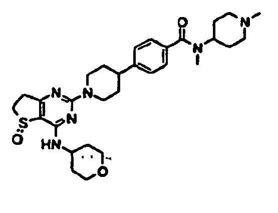 | H | 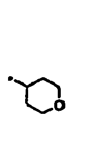 | 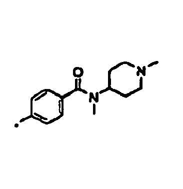 | H | 94 |
| 229 | 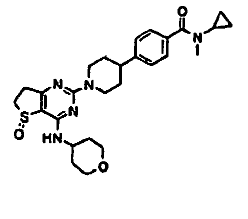 | H | 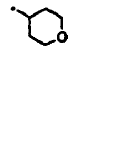 | 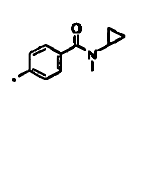 | H | 94 |
| 230 | 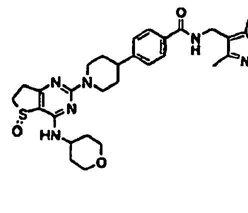 | H | 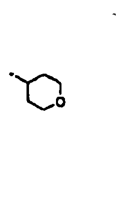 | 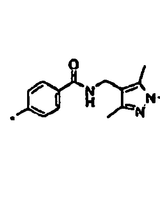 | H | 81 |
| 231 | 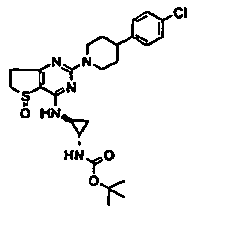 | H | 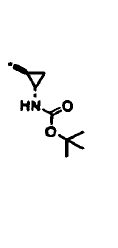 | 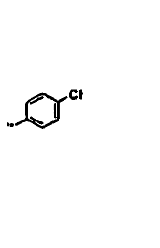 | H | 94 |
| 232¹⁾ | 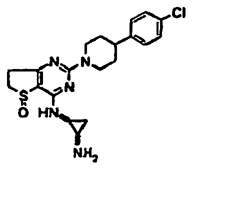 | H | 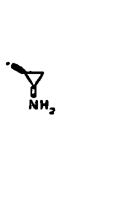 | 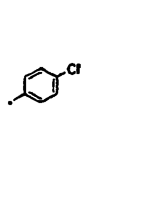 | H | 84 |
| 233 | 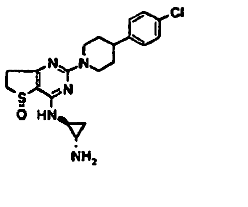 | H | 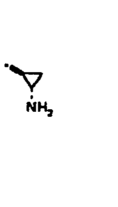 | 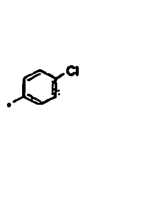 | H | 93 |
| 234 | 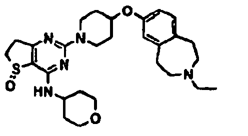 | H | 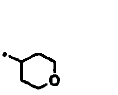 | 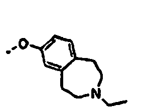 | H | 95 |
| 235 | 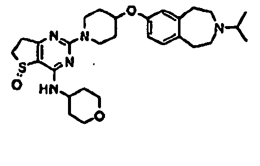 | H | 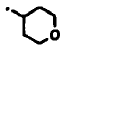 | 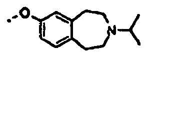 | H | 87 |
| 236 | 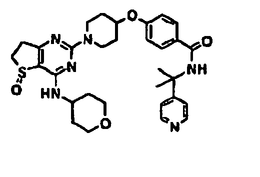 | H | 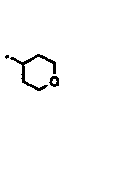 | 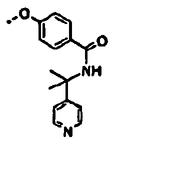 | H | 95 |
| 237 | 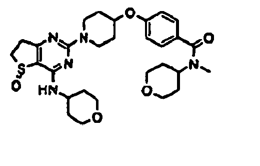 | H | 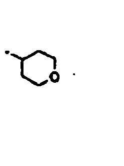 | 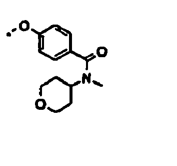 | H | 95 |
| 238 | 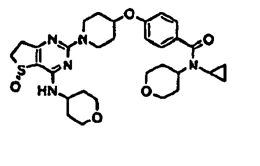 | H | 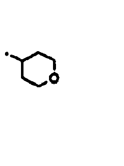 | 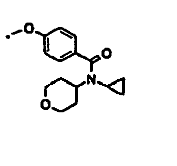 | H | 95 |
| 239 | 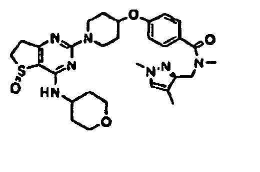 | H | 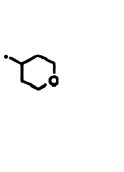 | 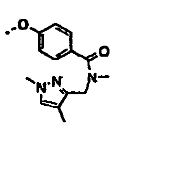 | H | 94 |
| 240 | 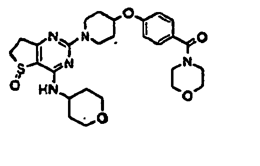 | H | 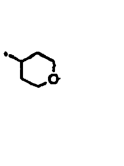 | 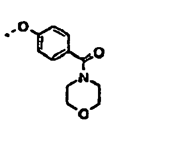 | H | 94 |
| 241 | 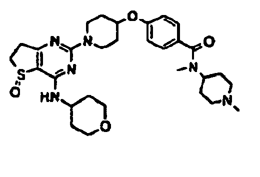 | H | 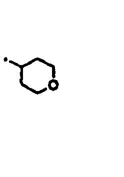 | 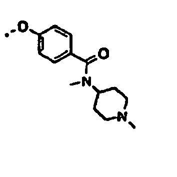 | H | 93 |
| 242 | 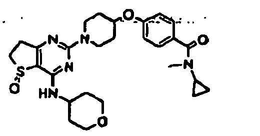 | H | 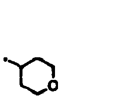 | 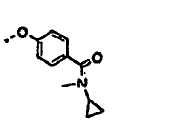 | H | 94 |
| 243 | 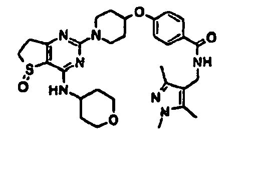 | H | 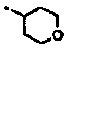 | 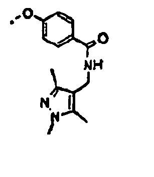 | H | 94 |
| 244 | 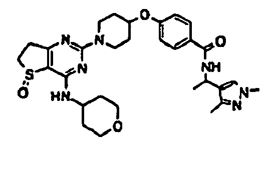 | H | 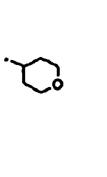 | 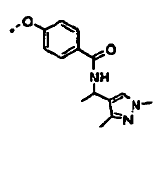 | H | 95 |
| 245²⁾ | 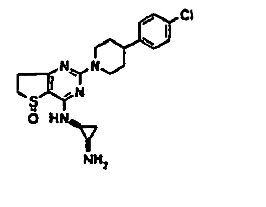 | H | 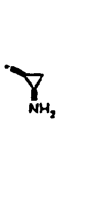 | 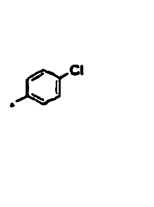 | H | 68 |
| 246 | 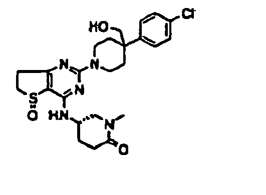 | H | 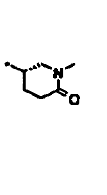 | 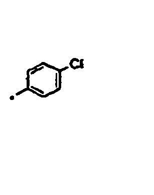 | 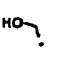 | 93 |
| 247 | 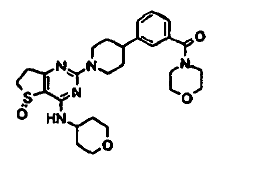 | H | 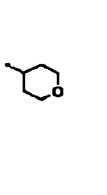 | 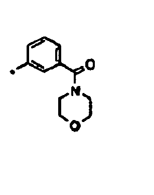 | H | 90 |
| 248 | 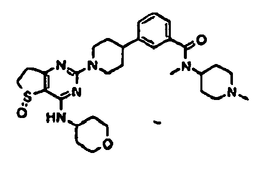 | H | 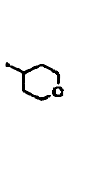 | 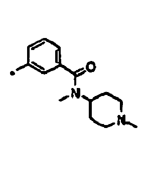 | H | 88 |
| 249 | 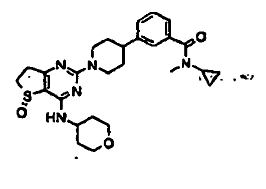 | H | 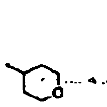 | 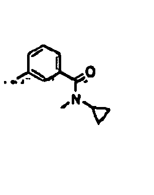 | H | 93 |
| 250 | 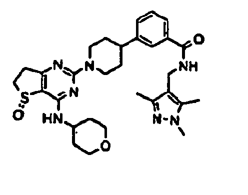 | H | 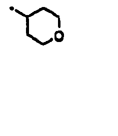 | 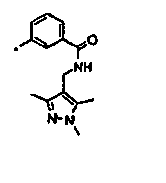 | H | 92 |
| 251 | 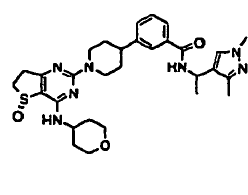 | H | 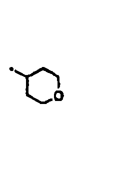 | 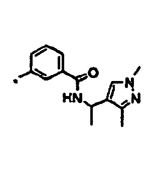 | H. | 90 |
| 252 | 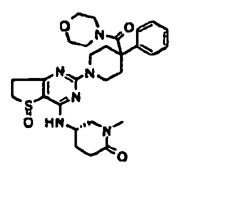 | H | 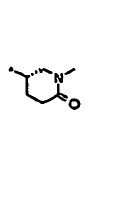 | 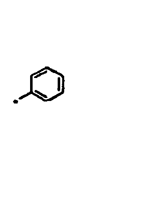 | 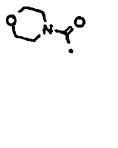 | 92 |
| 253 | 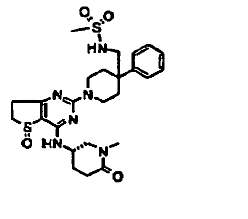 | H | 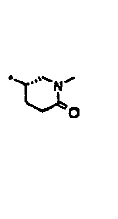 | 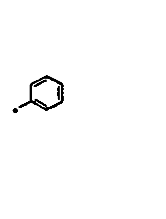 | 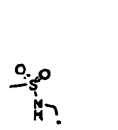 | 92 |
| 254 | 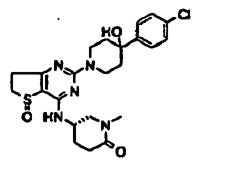 | H | 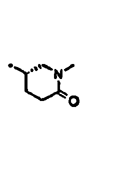 | 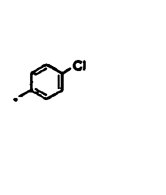 | OH | 92 |
| 255 | 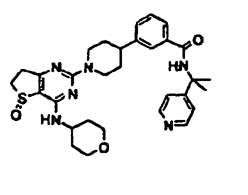 | H | 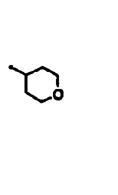 | 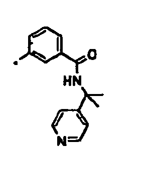 | H | 93 |
| 256 | 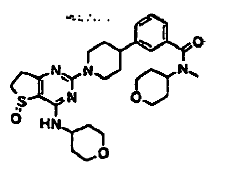 | H | 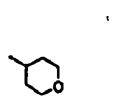 | 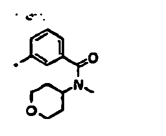 | H | 91 |
| 257 | 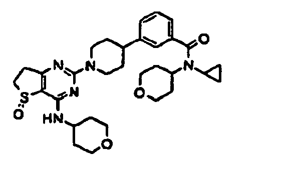 | H | 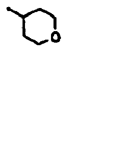 | 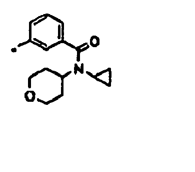 | H | 94 |
| 258 | 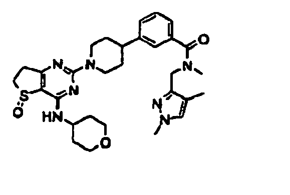 | H | 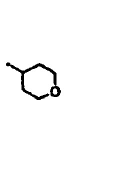 | 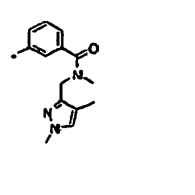 | H | 93 |
| 259 | 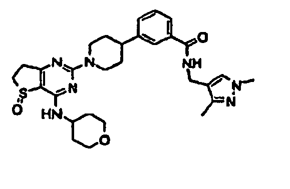 | H | 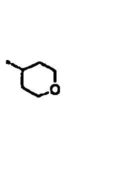 | 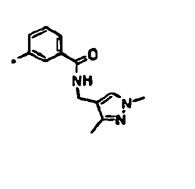 | H | 92 |
| 260 | 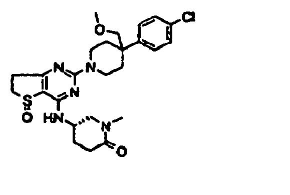 | H | 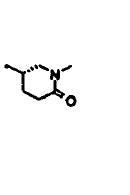 | 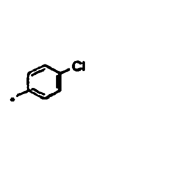 | 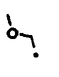 | 96 |
| 261 | 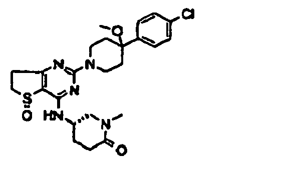 | H. | 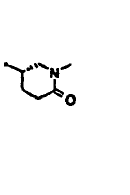 | 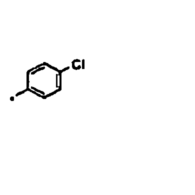 | OCH₃ | 95 |
| 262 | 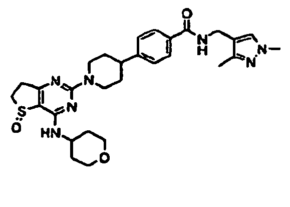 | H | 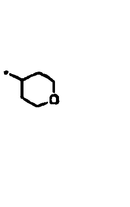 | 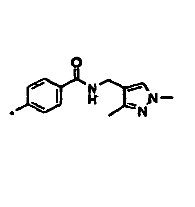 | H | 94 |
| 263 | 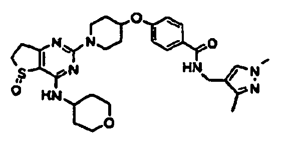 | H | 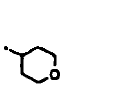 | 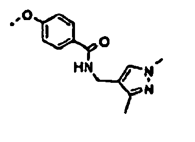 | H | 94 |
| 264 | 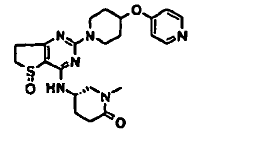 | H | 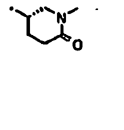 | 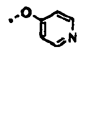 | H | 94 |
| 265 | 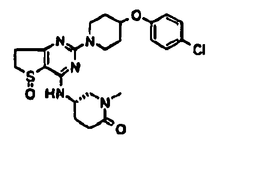 | H | 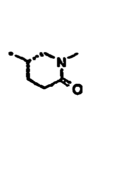 | 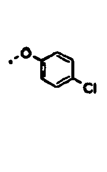 | H | 89 |
| 266 | 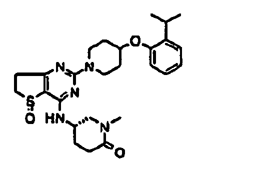 | H | 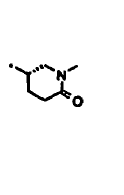 | 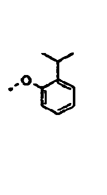 | H | 95 |
| 267 | 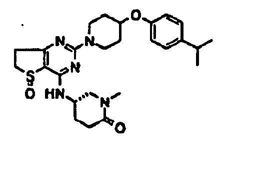 | H | 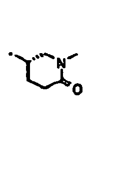 | 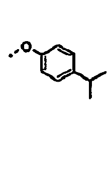 | H | 78 |
| 268 | 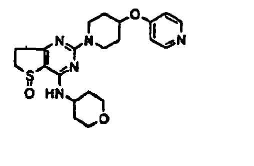 H | H | 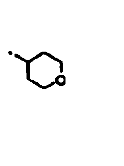 | 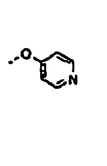 | H | 80 |
| 269 | 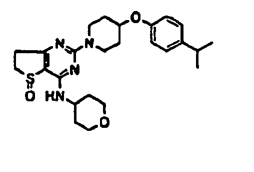 | H | 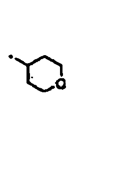 | 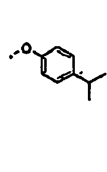 | H | 94 |
| 270 | 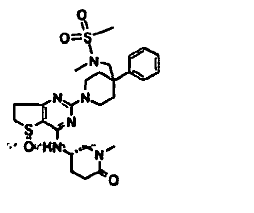 | H | 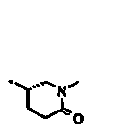 | 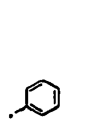 | 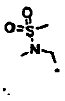 | 95 |
| 271 | 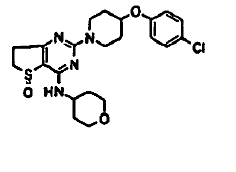 | H | 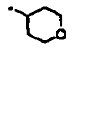 | 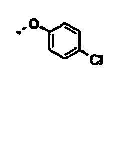 | H | 92 |
| 272 | 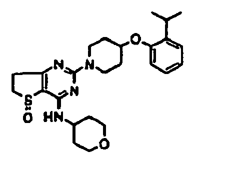 | H | 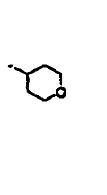 | 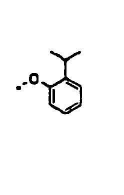 | H | 95 |
| 273 | 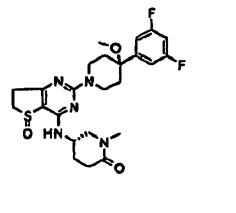 | H | 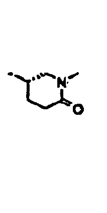 | 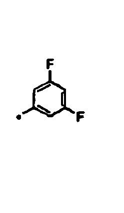 | OCH₃ | 94 |
| 274 | 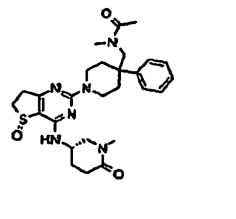 | H | 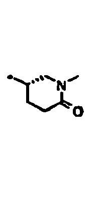 | 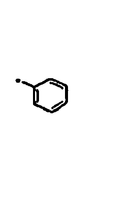 | 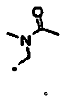 | 94 |
| 275 | 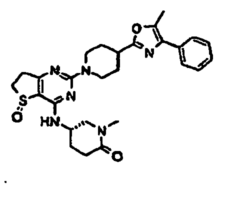 | H | 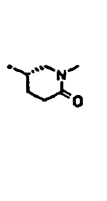 | 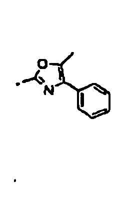 | H | 95 |
| 276 | 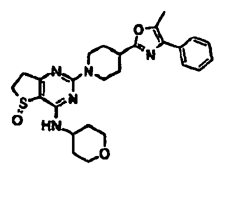 | H | 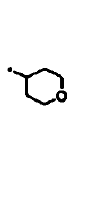 | 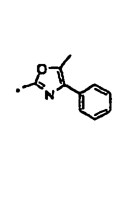 | H | 95 |
| 277 | 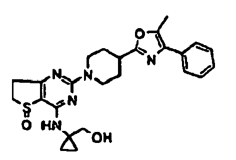 | H | 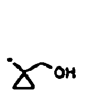 | 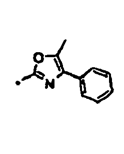 | H | 96 |
| 278 | 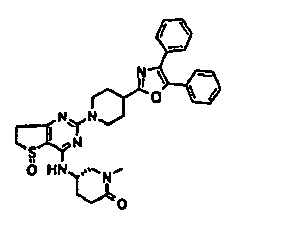 | H | 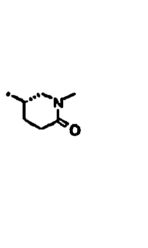 | 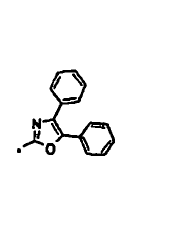 | H | 95 |
| 279 | 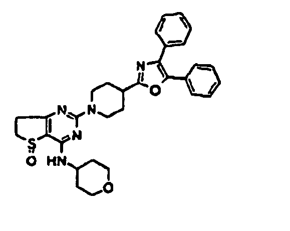 | H | 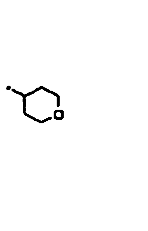 | 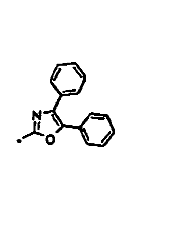 | H | 95 |
| 280 | 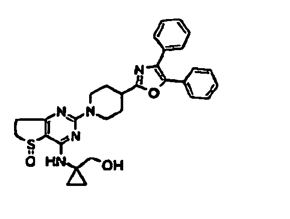 | H | 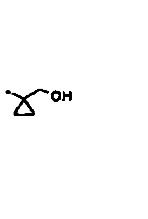 | 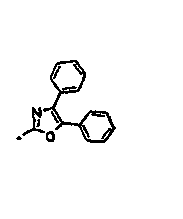 | H | 95 |
| 281 | 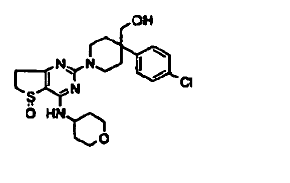 | H | 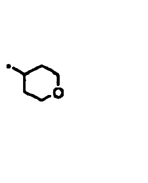 | 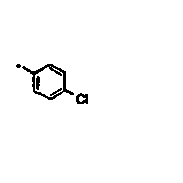 | 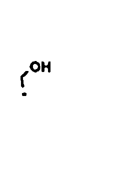 | 87 |
| 282 | 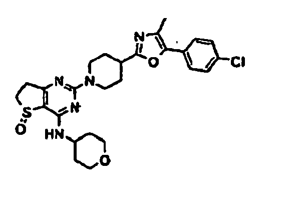 | H | 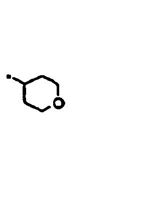 | 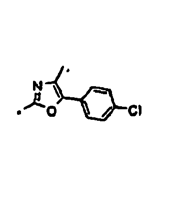 | H | 87 |
| 283 | 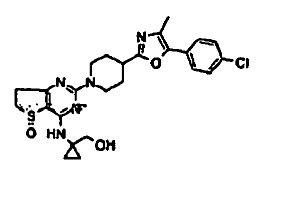 | H | 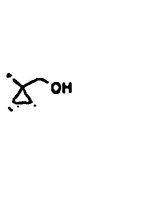 | 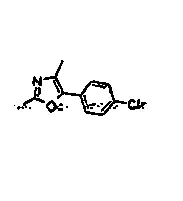 | H | 89 |
| 284 | 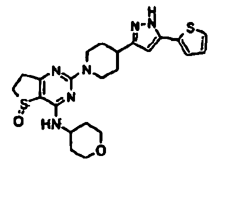 | H | 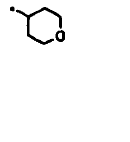 | 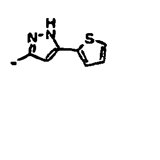 | H | 83 |
| 285 | 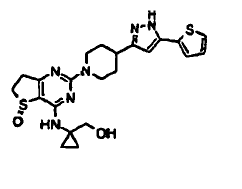 | H | 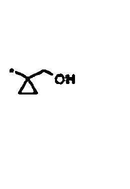 | 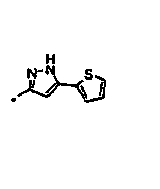 | H | 90 |
| 286 | 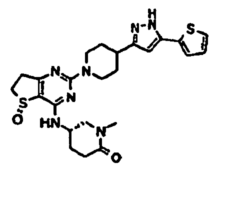 | H | 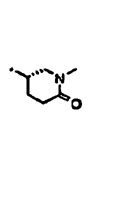 | 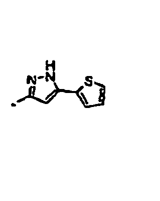 | H | 89 |
| 287 | 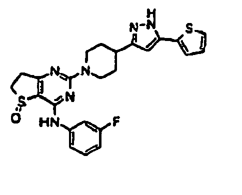 | H | 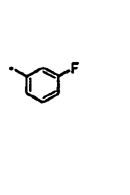 | 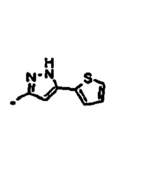 | H | 89 |
| 288 | 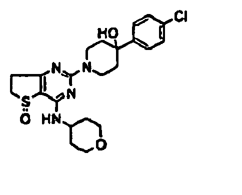 | H | 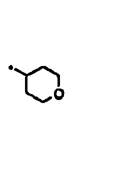 | 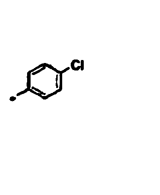 | OH | 89 |
| 289 | 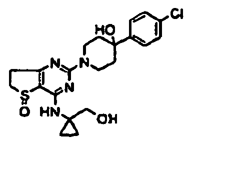 | H | 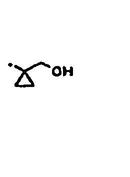 | 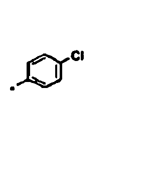 | OH | 81 |
| 290 | 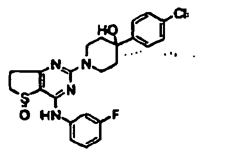 | H | 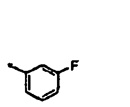 | 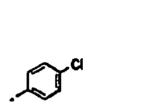 | OH | 86 |
| 291 | 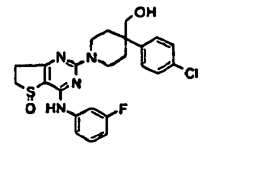 | H | 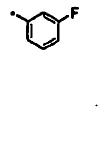 | 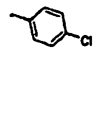 | 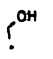 | 89 |
| 292 | 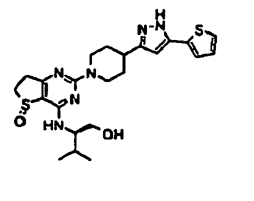 | H | 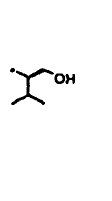 | 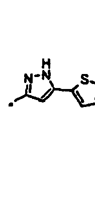 | H | 83 |
| 293 | 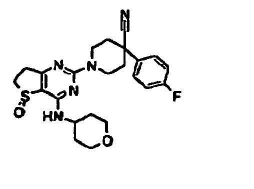 | H | 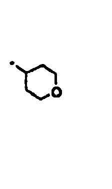 | 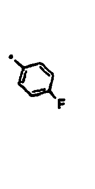 | CN | 86 |
| 294 | 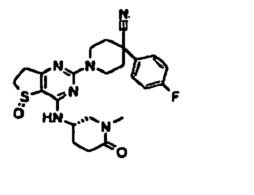 | H | 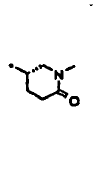 | 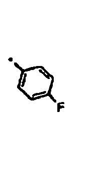 | CN | 88 |
| 295 | 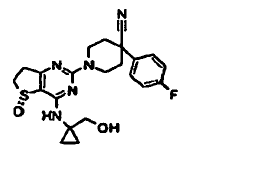 | H | 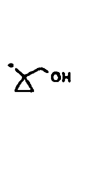 | 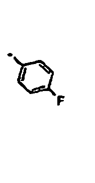 | CN | 93 |
| 296 | 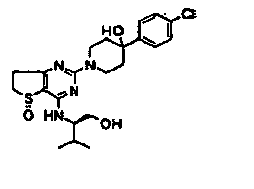 | H | 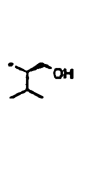 | 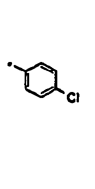 | OH | 81 |
| 297 | 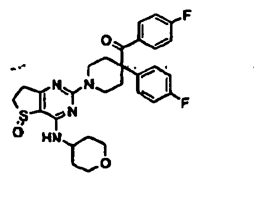 | H | 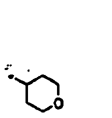 | 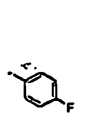 | 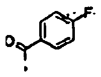 | 73 |
| 298 | 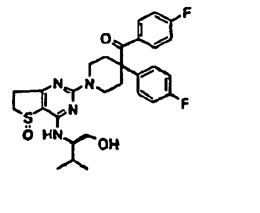 | H | 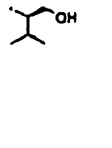 | 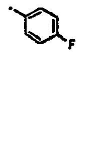 | 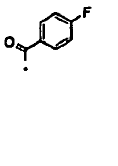 | 91 |
| 299 | 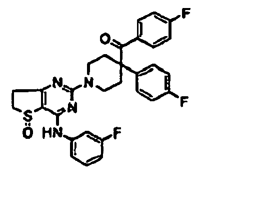 | H | 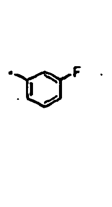 | 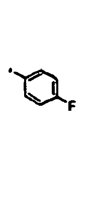 | 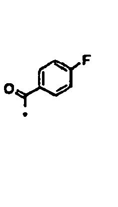 | 90 |
| 300 | 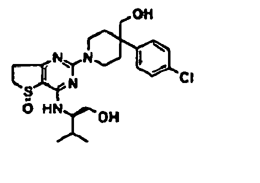 | H | 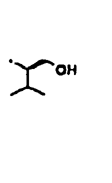 | 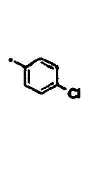 | 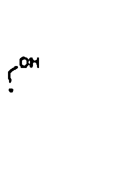 | 86 |
| 301 | 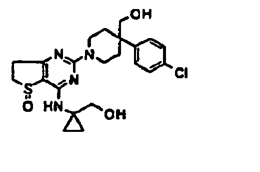 | H | 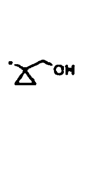 | 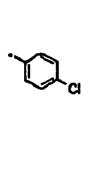 | 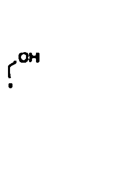 | 92 |
| 302 | 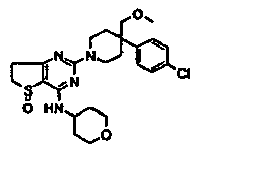 | H | 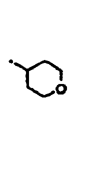 | 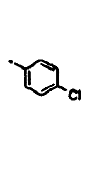 | 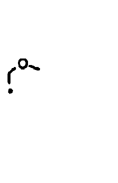 | 91 |
| 303 | 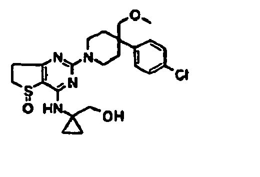 | H | 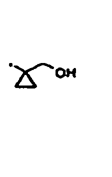 | 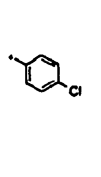 | 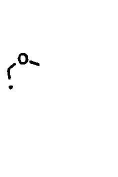 | 89 |
| 304 | 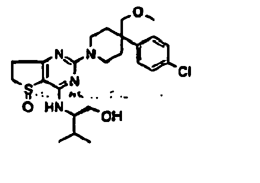 | H | 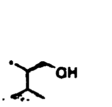 | 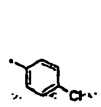 | 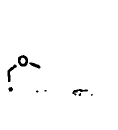 | 89 |
| 305 | 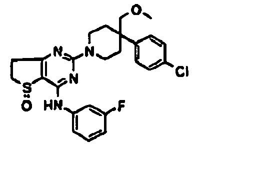 | H | 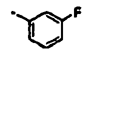 | 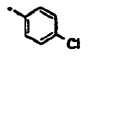 | 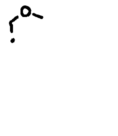 | 91 |
| 306 | 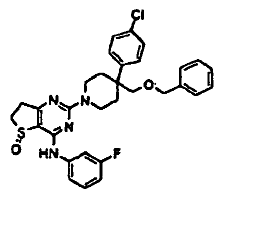 | H | 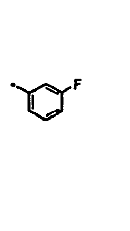 | 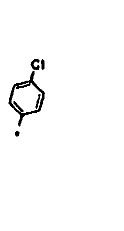 | 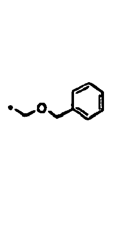 | 89 |
| 307 | 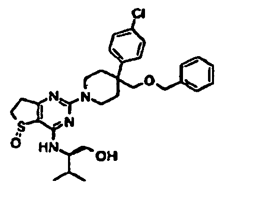 | H | 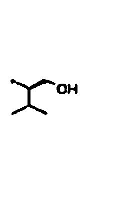 | 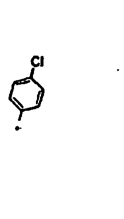 | 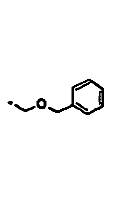 | 89 |
| 308 | 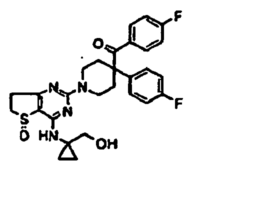 | H | 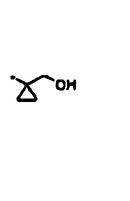 | 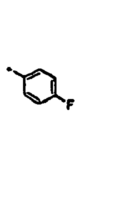 | 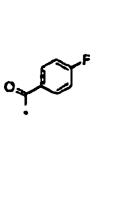 | 89 |
| 309 | 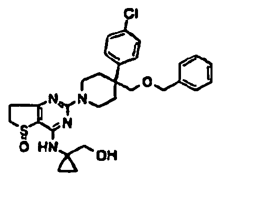 | H | 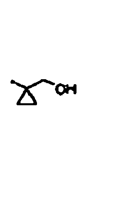 | 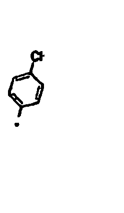 | 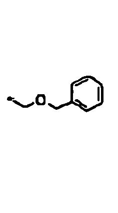 | 92 |
| 310 | 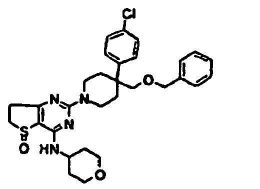 | H | 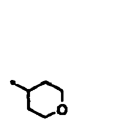 | 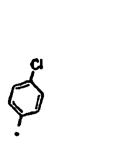 | 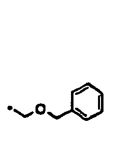 | 92 |
| 311 | 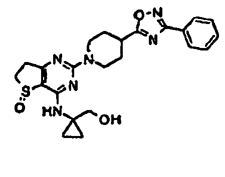 | H | 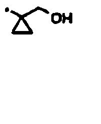 | 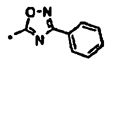 | H | 91 |
| 312 | 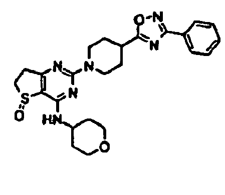 | H | 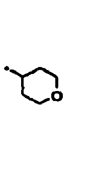 | 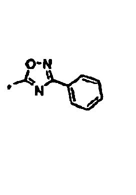 | H | 92 |
| 313 | 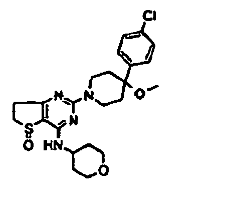 | H | 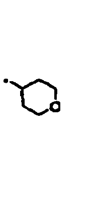 | 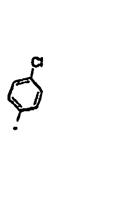 | OCH₃ | 89 |
| 314 | 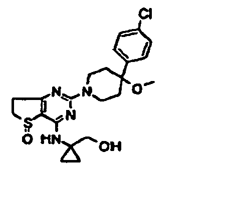 | H | 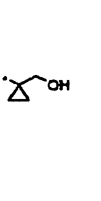 | 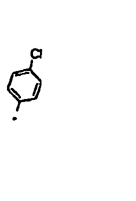 | OCH₃ | 88 |
| 315 | 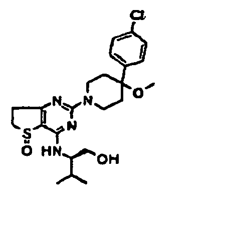 | H | 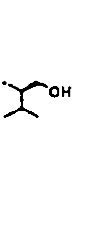 | 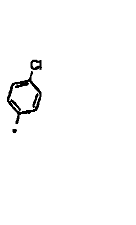 | OCH₃ | 89 |
| 316 | 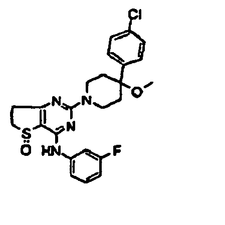 | H | 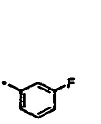 | 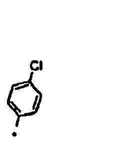 | OCH₃ | 92 |
| 317 | 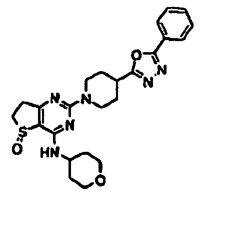 | H | 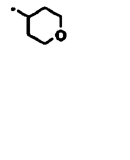 | 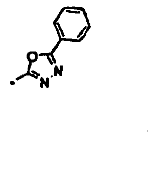 | H | 95 |
| 318 | 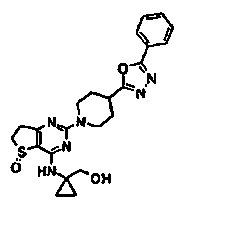 | H | 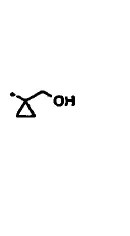 | 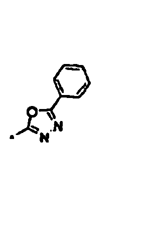 | H | 91 |
| 319 | 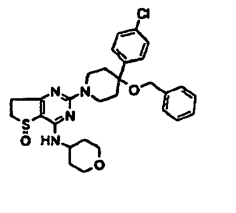 | H | 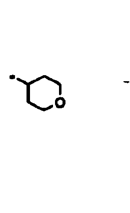 | 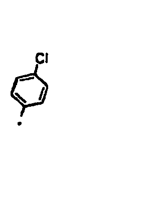 | 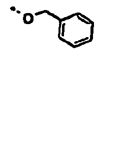 | 91 |
| 320 | 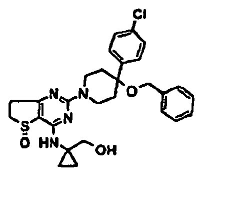 | H | 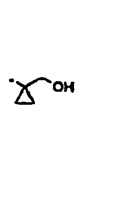 | 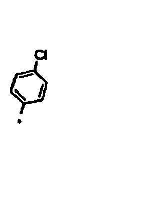 | 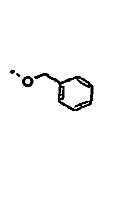 | 92 |
| 321 | 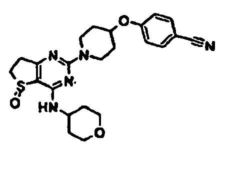 | H | 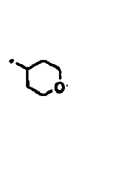 | 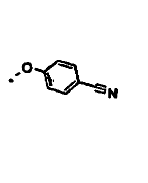 | H | 93 |
| 322 | 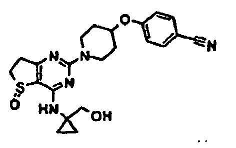 | H | 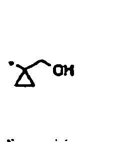 | 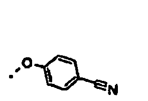 | H | 91 |
| 323 | 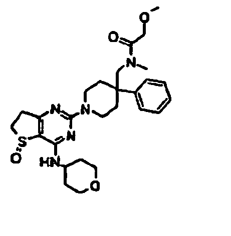 | H | 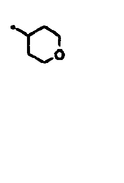 | 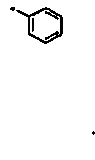 | 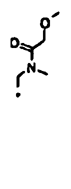 | 94 |
| 324 | 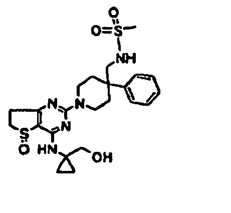 | H | 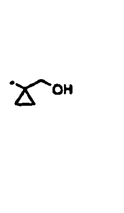 | 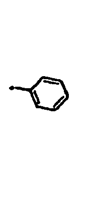 | 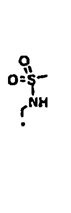 | 89 |
| 325 | 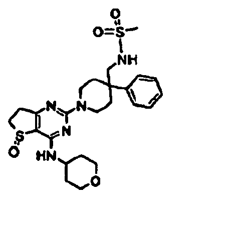 | H | 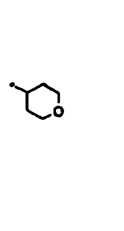 | 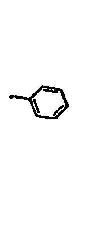 | 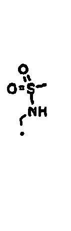 | 87 |
| 326 | 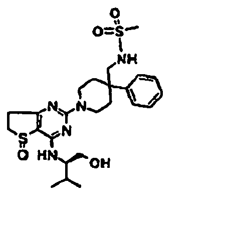 | H | 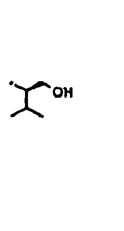 | 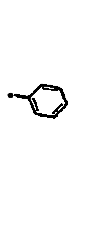 | 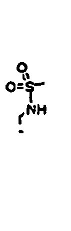 | 88 |
| 327 | 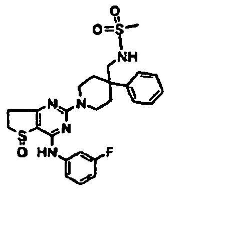 | H | 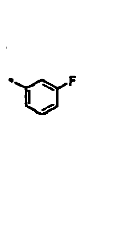 | 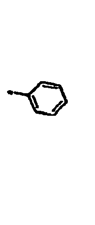 | 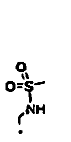 | 83 |
| 328 | 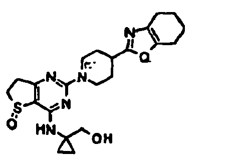 | H | 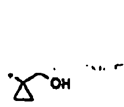 | 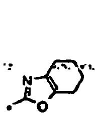 | H | 93 |
| 329 | 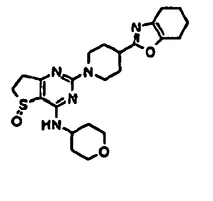 | H | 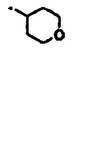 | 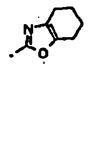 | H | 95 |
| 330 | 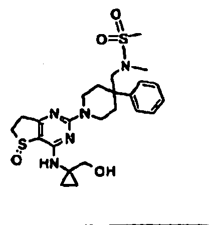 | H | 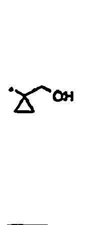 | 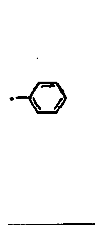 | 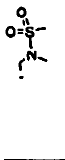 | 92 |
| 331 | 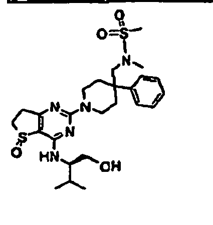 | H | 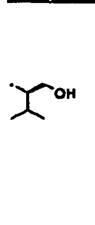 | 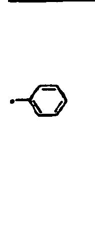 | 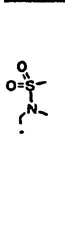 | 85 |
| 332 | 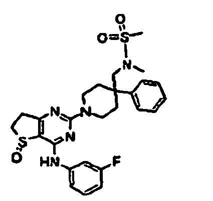 | H | 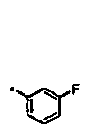 | 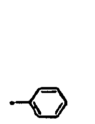 | 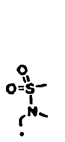 | 93 |

| | | | | | | |
|---|---|---|---|---|---|---|
| ¹⁾ Dieses Beispiel ist diastereomer zu Beispiel 245 (siehe experim. Teil). ²⁾ Dieses Beispiel ist diastereomer zu Beispiel 232 (siehe experim. Teil) | | | | | | |

In der folgenden Tabelle D werden Detail-Informationen zu den chemischen Synthesen und der Analytik der einzelnen Beispiel-Substanzen 164-332 zusammengefasst.

**Tabelle D: Detail-Informationen zu den Herstellungen der einzelnen Beispiel-Substanzen 164-332**

| # | Herstellung analog zu #* | nicht käufliche Arylpiperidin-Baustein (V) | Literatur zur Herstellung des Bausteins (V) | Analytische HPLC-MS, RT [min], Methode |
|---|---|---|---|---|
| 164 | 89 | | | 1,13 Methode A |
| 165 | 73 | | | 1,39 Methode A |
| 166 | 90 | | | 1,20 Methode A |
| 167 | 89 | | | 1,16 Methode A |
| 168 | 14 | | | 1,17 Methode A |
| 169 | 100 | | | 1,15 Methode A |
| 170 | 90 | | | 0,94 Methode, A |
| 172 | 90 | | | 1,15 Methode A |
| 173 | 90 | | | 0,97 Methode A |
| 174 | 90 | | | 1,15 Methode A |
| 175 | 90 | | | 1,21 Methode A |
| 176 | 90 | 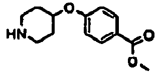 | J. Med. Chem. 2002, 3406 | 1,17 Methode A |
| 177 | 90 | | | 1,18 Methode A |
| 178 | siehe experim. Teil | | | 1,01 Methode A |
| 179 | 90 | | | 1,22 Methode A |
| 180 | siehe experim. Teil | | | 0,99 Methode A |
| 181 | 90 | | | 1,30 Methode A |
| 182 | siehe experim. Teil | | | 1,27 Methode A |
| 183 | siehe experim. Teil | | | 0,99 Methode A |
| 184 | siehe experim. Teil | | | 1,19 Methode B |
| 185 | siehe experim. Teil | | | 1,37 Methode B |
| 186 | siehe experim. Teil | | | 1,10 Methode B |
| 187 | 100 | | | 1,33 Methode B |
| 188 | 100 | | | 1,03 Methode B |
| 189 | 100 | | | 1,18 Methode B |
| 190 | 100 | | | 1,14 Methode B |
| 191 | 90 | | | 1,18 Methode B |
| 192 | siehe experim. Teil | | | 1,16 Methode B |
| 193 | 90 | 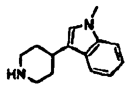 | WO2004/006922 | 1,23 Methode B |
| 194 | siehe experim. Teil | | | 1,27 Methode B |
| 195 | siehe experim. Teil | | | 1,21 Methode B |
| 196 | 100 | 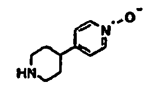 | WO03051868 | 0,96 Methode B |
| 197 | siehe experim. Teil | | | 1,26 Methode B |
| 198 | siehe experim. Teil | | | 1,21 Methode B |
| 199 | 100 | 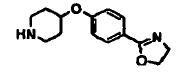 | (V-4) (siehe experim. Teil, 37.3) | 1,07 Methode B |
| 200 | siehe experim. Teil | | | 1,26 Methode B |
| 201 | siehe experim. Teil | | | 1,36 Methode B |
| 202 | siehe experim. Teil | | | 1,16 Methode B |
| 203 | siehe experim. Teil | | | 1,38 Methode B |
| 204 | siehe experim. Teil | | | 1,11 Methode B |
| 205 | siehe experim. Teil | | | 1,23 Methode B |
| 206 | siehe experim. Teil | | | 1,08 Methode B |
| 207 | 90 | | | 0,83 Methode D |
| 208 | 90 | 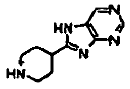 | Bioorg. Med. Chem. Lett. 2004, 695 | 0,76 Methode D |
| 209 | 90 | | | 0,95 Methode D |
| 210 | siehe experim. Teil | | | 0,86 Methode D |
| 211 | siehe experim. Teil | | | 1,45 Methode B |
| 212 | 90 | | | 0,99 Methode D |
| 213 | 90 | | | 1,00 Methode B |
| 214 | siehe experim. Teil | | | 1,10 Methode D |
| 215 | 90 | 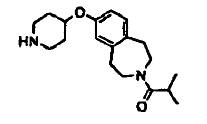 | Herstellung analog zu (V-18) (siehe experim. Teil 57,3)* | 1,11 Methode D |
| 216 | 89 | 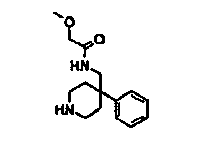 | (V-12) (siehe experim. Teil 50.2) | 1,20 Methode B |
| 217 | 90 | 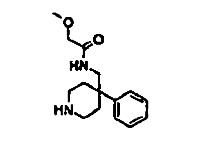 | **(V-12)** (siehe experim. Teil 50.2) | 1,21 Methode B |
| 218 | 90 | 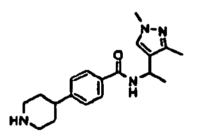 | Herstellung analog zu **(V-19)** (siehe experim. Teil 59.2)* | 1,17 Methode B |
| 219 | 100 | | | 1,18 Methode B |
| 220 | 89 | | | 1,19 Methode B |
| 221 | 90 | 90 | 90 | 1,22 Methode B |
| 222 | siehe experim. Teil | | | 1,46 Methode B |
| 223 | 90 | 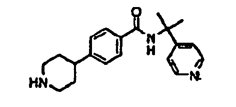 | Herstellung analog zu (V-19) (siehe experim. Teil 59.2)* | 1,03 Methode D |
| 224 | 90 | 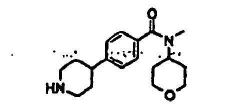 | Herstellung analog zu **(V-19)** (siehe experim. Teil 59.2)* | 0,99 Methode D |
| 225 | 90 | 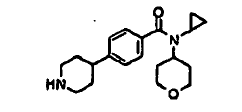 | Herstellung analog zu (V-19) (siehe experim. Teil 59.2)* | 1,06 Methode D |
| 226 | 90 | 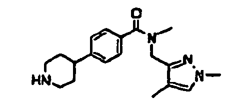 | Herstellung analog zu (V-19) (siehe experim. Teil 59.2)* | 0,98 Methode D |
| 227 | 90 | 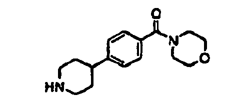 | Herstellung analog zu (V-19) (siehe experim. Teil 59.2)* | 0,98 Methode D |
| 228 | 90 | 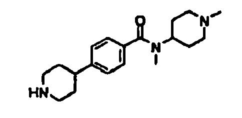 | Herstellung analog zu (V-19) (siehe experim. Teil 59.2)* | 0,96 Methode D |
| 229 | siehe experim. Teil | | | 1,05 Methode D |
| 230 | 90 | 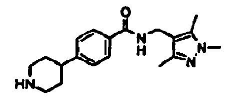 | Herstellung analog zu (V-19) (siehe experim. Teil 59.2)* | 0,99 Methode D |
| 231 | siehe experim. Teil | | | 1,48 Methode B |
| 232¹⁾ | siehe experim. Teil | | | 2,73 Methode E |
| 233 | siehe experim. Teil | | | 1,24 Methode B |
| 234 | 186 | | | 1,14 Methode D |
| 235 | 186 | | | 1,21 Methode D |
| 236 | 242 | | | 1,02 Methode D |
| 237 | 242 | | | 0,98 Methode D |
| 238 | 242 | | | 1,05 Methode D |
| 239 | 242 | | | 0,97 Methode D |
| 240 | 242 | | | 1,23 Methode B |
| 241 | 242 | | | 1,09 Methode. B |
| 242 | siehe experim. Teil | | | 1,03 Methode D |
| 243 | 242 | | | 0,97 Methode D |
| 244 | 242 | | | 0.99 Methode D |
| 245²⁾ | siehe experim. Teil | | | 2,85 Methode E |
| 246 | siehe experim. Teil | | | 1,21 Methode B |
| 247 | 90 | | | 1,00 Methode D |
| 248 | 90 | | | 0,99 Methode D |
| 249 | siehe experim. Teil | | | 1,05 Methode D |
| 250 | 249 | | | 1,01 Methode D |
| 251 | 249 | | | 1,14 Methode D |
| 252 | siehe experim. Teil | | | t,18 Methode B |
| 253 | siehe experim. Teil | | | 1,15 Methode B |
| 254 | 100 | | | 1,23 Methode B |
| 255 | 249 | | | 1,04 Methode D |
| 256 | 249 | | | 1,00 Methode D |
| 257 | 249 | | | 1,07 Methode D |
| 258 | 249 | | | 1,00 Methode D |
| 259 | 249 | | | 0,98 Methode D |
| 260 | siehe experim. Teil | | | 1,30 Methode B |
| 261 | siehe experim. Teil | | | 1,30 Methode B |
| 262 | 90 | 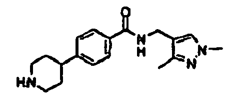 | Herstellung analog zu (V-19) (siehe experim. Teil 59.2)* | 1,15 Methode B |
| 263 | 242 | | | 1,15 Methode B |
| 264 | 100 | | | 0,94 Methode B |
| 265 | 100 | | | 1,32 Methode B |
| 266 | 100 | | | 1,39 Methode B |
| 267 | 100 | | | 1,39 Methode B |
| 268 | 90 | | | 0,99 Methode B |
| 269 | 90 | | | 1,45 Methode B |
| 270 | siehe experim. Teil | | | 1,21 Methode B |
| 271 | 90 | | | 1,39 Methode B |
| 272 | 90 | | | 1,45 Methode B |
| 273 | siehe experim. Teil | | | 1,23 Methode B |
| 274 | siehe experim. Teil | | | 1,19 Methode D |
| 275 | siehe experim. Teil | | | 1,08 Methode D |
| 276 | 90 | 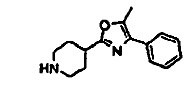 | (V-25) (siehe experim. Teil 72.1) | 1,18 Methode D |
| 277 | 89 | 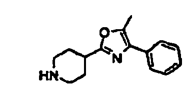 | (V-25) (siehe experim. Teil 72.1) | 1,33 Methode B |
| 278 | siehe experim. Teil | | | 1,40 Methode B |
| 279 | 90 | 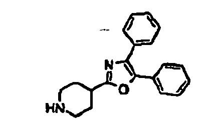 | (V-26) (siehe experim. Teil 73.2) | 1,50 Methode B |
| 280 | 89 | 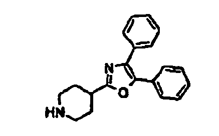 | (V-26) (siehe experim. Teil 73.2) | 1,43 Methode B |
| 281 | 90 | 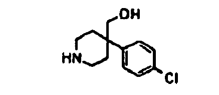 | J. Med. Chem. 2004, 497 | 1,24 Methode B |
| 282 | 90 | 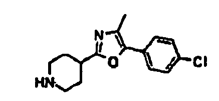 | (V-27) (siehe experim. Teil 74.1) | 1,24 Methode B |
| 283 | siehe experim. Teil | | | 1,37 Methode B |
| 284 | 90 | | | 1,23 Methode B |
| 285 | 89 | | | 1,0 Methode D |
| 286 | 100 | | | 1,19 Methode B |
| 287 | 73 | | | 1,17 Methode D |
| 288 | 90 | | | 1,25 Methode B |
| 289 | 89 | | | 1,23 Methode B |
| 290 | 73 | | | 1,42 Methode B |
| 291 | 73 | 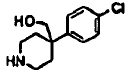 | J. Med. Chem. 2004, 497 | 1,41 Methode B |
| 292 | 14 | | | 1,03 Methode D |
| 293 | 90 | 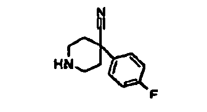 | J. Med. Chem. 1999, 4778 | 1,11 Methode D |
| 294 | 100 | 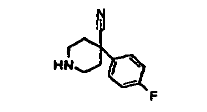 | J. Med. Chem. 1999, 4778 | 1.03 Methode D |
| 295 | 89 | 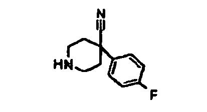 | J. Med. Chem. 1999, 4778 | 1.08 Methode D |
| 296 | 14 | | | 1,27 Methode B |
| 297 | 90 | 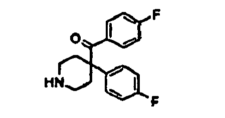 | (V-13) (siehe experim. Teil 51.3) | 1,43 Methode B |
| 298 | 14 | 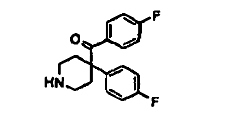 | (V-13) (siehe experim. Teil 51.3) | 1,45 Methode B |
| 299 | 73 | 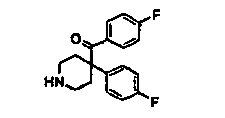 | (V-13) (siehe experim. Teil 51.3) | 1,66 Methode B |
| 300 | 14 | 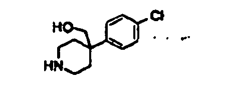 | J. Med. Chem. 2004, 497 | 1,29 Methode B |
| 301 | 89 | 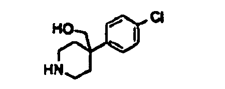 | J. Med. Chem. 2004, 497 | 1,23 Methode B |
| 302 | 90 | 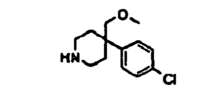 | (V-20) (siehe experim. Teil 67.3) | 1,48 Methode B |
| 303 | 89 | 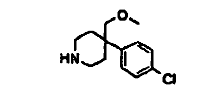 | (V-20) (siehe experim. Teil 67.3) | 1,32 Methode B |
| 304 | 14 | 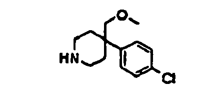 | (V-20) (siehe experim. Teil 67.3) | 1,37 Methode B |
| 305 | 73 | 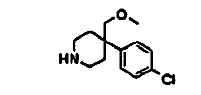 | (V-20) (siehe experim. Teil 67.3) | 1,58 Methode B |
| 306 | siehe experim. Teil | | | 1,75 Methode B |
| 307 | 14 | 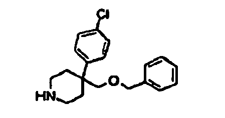 | (V-28) (siehe experim. Teil 75.1) | 1,50 Methode B |
| 308 | 89 | 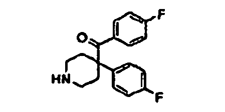 | (V-13) (siehe experim. Teil 51.3) | 1,40 Methode B |
| 309 | 89 | 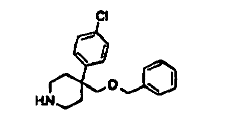 | (V-28) (siehe experim. Teil 75.1) | 1,48 Methode B |
| 310 | 90 | 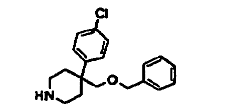 | (V-28) (siehe experim. Teil 75.1) | 1,51 Methode B |
| 311 | 89 | | | 1,29 Methode B |
| 312 | 90 | | | 1,33 Methode B |
| 313 | 90 | 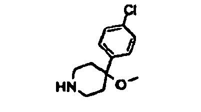 | (V-21) (siehe experim. Teil 68.1) | 1,39 Methode B |
| 314 | 89 | 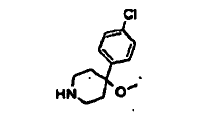 | (V-21) (siehe experim. Teil 68.1) | 1,35 Methode B. |
| 315 | 14 | 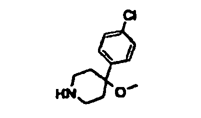 | (V-21) (siehe experim. Teil 68.1) | 1,39 Methode B |
| 316 | 73 | 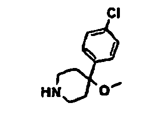 | (V-21) (siehe experim. Teil 68.1) | 1,61 Methode B |
| 317 | 90 | | | 1,25 Methode B |
| 318 | 89 | | | 1,22 Methode B |
| 319 | 90 | 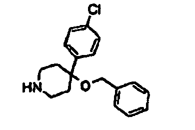 | (V-28) (siehe experim. Teil 75.1) | 1,54 Methode B |
| 320 | 89 | 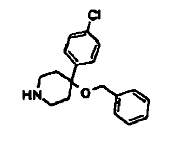 | (V-28) (siehe experim. Teil 75.1) | 1,51 Methode B |
| 321 | 90 | 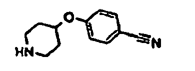 | WO2007/106705 | 1,29 Methode B |
| 322 | 89 | 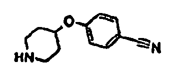 | WO2007/106705 | 1,24 Methode B |
| 323 | siehe experim. Teil | | | 1,24 Methode B |
| 324 | 89 | 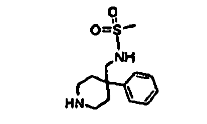 | Bioorg. Med. Chem. Lett. 1998.1851 | 1,21 Methode B |
| 325 | 90 | 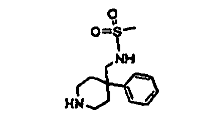 | Bioorg. Med. Chem. Lett. 1998, 1851 | 1,21 Methode B |
| 326 | 14 | 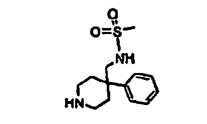 | Bioorg. Med. Chem. Lett. 1998, 1851 | 1,24 Methode B |
| 327 | 73 | 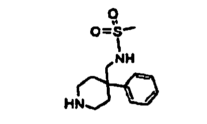 | Bioorg. Med. Chem. Lett. 1998, 1851 | 1,38 Methode B |
| 328 | 89 | 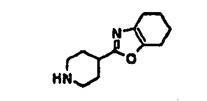 | (V-30) (siehe experim. Teil 77.2) | 1,23 Methode B |
| 329 | siehe experim. Teil | | | 1,23 Methode B |
| 330 | 89 | 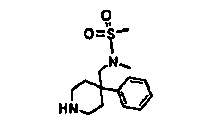 | (V-22) (siehe experim. Teil 69.1) | 1,24 Methode B |
| 331 | 14 | 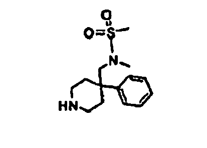 | (V-22) (siehe experim. Teil 69.1) | 1,30 Methode B |
| 332 | 73 | 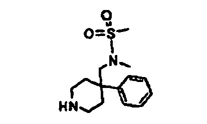 | (V-22) (siehe experim. Teil 69.1) | 1,44 Methode B |

| | | | | |
|---|---|---|---|---|
| * Das Beispiel kann analog hergestellt und aufgereinigt werden. ¹⁾ Dieses Beispiel ist diastereomer zu Beispiel 245 (siehe experim. Teil). ²⁾ Dieses Beispiel ist diastereomer zu Beispiel 232 (siehe experim. Teil) | | | | |

**Tabelle E: Chemische Strukturen und Herstellungen der Beispiel-Substanzen 333 -335**

| # | Struktur | R1 | R2 | R3 | R4 | Herstellung | Analytische HPLC-MS, RT [min], Methode | % Inhibition PDE4B @ 1 µM |
|---|---|---|---|---|---|---|---|---|
| 333 | 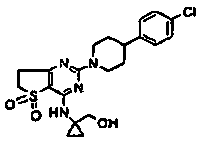 | H | 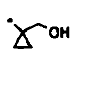 | 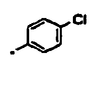 | H | siehe experim. Teil | 1,49 Methode B | 56 |
| 334 | 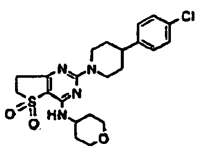 | H | 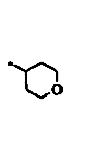 | 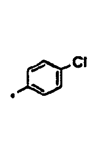 | H | siehe experim. Teil | 1,55 Methode B | 65 |
| 335 | 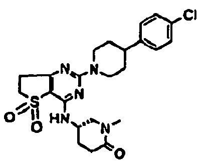 | H | 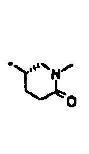 | 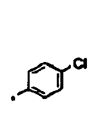 | H | siehe experim. Teil | 1,48 Methode B | 85 |

### INDIKATIONSGEBIETE

Wie gefunden wurde, zeichnen sich die Verbindungen der Formel 1 durch vielfältige Anwendungsmöglichkeiten auf therapeutischem Gebiet aus. Hervorzuheben sind solche Anwendungsmöglichkeiten, für welche die erfindungsgemäßen Verbindungen der Formel 1 aufgrund ihrer pharmazeutischen Wirksamkeit als PDE4-Inhibitor bevorzugt zur Anwendung gelangen können. Beispielhaft genannt seinen Atemwegs- oder gastrointestinalen Erkrankungen oder Beschwerden, entzündliche Erkrankungen der Gelenke, der Haut oder der Augen, Krebserkrankungen, sowie Erkrankungen des periphären oder zentralen Nervensystems.

Hierbei bevorzugt genannt sei die Vorbeugung und Behandlung von Atemwegs- oder Lungenerkrankungen, welche mit einer erhöhten Schleimproduktion, Entzündungen und/oder obstruktiven Erkrankungen der Atemwege einher gehen. Beispielhaft hierfür seinen genannt, akute, allergische oder chronische Bronchitis, chronisch obstruktive Bronchitis (COPD), Husten, Lungenemphysem, allergische oder nicht-allergische Rhinitis oder Sinusitis, chronische Rhinitis oder Sinusitis, Asthma, Alveolitis, Farmers Krankheit, hyperreaktive Atemwege, infektiöse Bronchitis oder Pneumonitis, pediatrisches Asthma, Bronchiectasien, Lungenfibrose, ARDS (akutes Atemnotsyndrom des Erwachsenen), Bronchialödem, Lungenödem, Bronchitis, Pneumonie oder interstitielle Pneumonie ausgelöst durch verschiedene Ursachen wie Aspiration, Inhalation von toxischen Gasen oder Bronchitis, Pneumonie oder interstitielle Pneumonie ausgelöst durch Herzinsuffizienz, Bestrahlung, Chemotherapie zystische Fibrose oder Mukoviszidose, alpha1-Antitrypsin-Mangel.

Ebenfalls bevorzugt genannt sei die Behandlung von entzündlichen Erkrankungen des Gastrointestinaltraktes. Beispielhaft hierfür seinen genannt, akute oder chronische entzündliche Veränderungen bei Gallenblasenentzündung, Morbus Crohn, Colitis ulcerosa, entzündliche Pseudopolypen, juvenile Polypen, Colitis cystica profunda, Pneumatosis cystoides intestinales, Erkrankungen der Gallengänge und Gallenblase, z.B. Gallensteine und Konglomerate, zur Behandlung von entzündlichen Erkrankungen der Gelenke wie rheumatoide Arthritis oder entzündliche Erkrankungen der Haut und der Augen.

Ebenfalls bevorzugt genannt sei die Behandlung von Krebserkrankungen. Beispielhaft hierfür seinen genannt alle Formen von akuten und chronischen Leukämien wie, akute lymphatische und akute myeloische Leukämie, chronisch lymphatische und chronisch myeloische Leukämie, sowie Knochentumoren wie das Osteosarkom und sowie alle Arten von Gliomen wie Oligodendrogliom und Glioblastom.

Des Weiteren bevorzugt genannt sei die Vorbeugung und Behandlung von Erkrankungen des periphären oder zentralen Nervensystems. Beispielhaft hierfür seien genannt Depression, bipolare oder manische Depression, akute und chronische Angstzustände, Schizophrenie, Alzheimer'sche Erkrankung, Parkinson'sche Erkrankung, akute und chronische Multiple Sklerose oder akute und chronische Schmerzzustände sowie Verletzungen des Gehirns hervorgerufen durch Schlaganfall, Hypoxie oder Schädel-Him-Trauma.

Besonders bevorzugt betrifft die vorliegende Erfindung die Verwendung von Verbindungen der Formel 1 zur Herstellung eines Arzneimittels zur Behandlung entzündlicher oder obstruktiver Erkrankungen der oberen und unteren Atmungsorgane einschließlich der Lunge wie beispielsweise allergische Rhinitis, chronische Rhinitis, Bronchiectasis, zystische Fibrose, idiopathische Lungenfibrose, fibrosierende Alveolitis, COPD, chronische Bronchitis, chronischer Sinusitis, Asthma, Morbus Crohn, Colitis ulcerosa, insbesondere COPD, chronische Bronchitis und Asthma.

Am meisten bevorzugt ist die Verwendung der Verbindungen der Formel 1 zur Behandlung von entzündlichen und obstruktiven Erkrankungen wie COPD, chronische Bronchitis, chronischer Sinusitis, Asthma, Morbus Crohn, Colitis ulcerosa, insbesondere COPD, chronische Bronchitis und Asthma.

Ebenfalls bevorzugt ist die Verwendung der Verbindungen der Formel 1 zur Behandlung von Erkrankungen des periphären oder zentralen Nervensystems wie Depression, bipolare oder manische Depression, akute und chronische Angstzustände, Schizophrenie, Alzheimer'sche Erkrankung, Parkinson'sche Erkrankung, akute und chronische Multiple Sklerose oder akute und chronische Schmerzzustände sowie Verletzungen des Gehirns hervorgerufen durch Schlaganfall, Hypoxie oder Schädel-Hirn-Trauma.

Ein herausragender Aspekt der vorliegende Erfindung ist das reduzierte Profil an Nebenwirkungen. Darunter wird im Rahmen der Erfindung verstanden, eine Dosis einer pharmazeutischen Zusammensetzung verabreichen zu können, ohne beim Patienten Erbrechen, bevorzugt Übelkeit, besonders bevorzugt Unwohlsein auszulösen. Höchst bevorzugt ist die Verabreichung einer therapeutisch wirksamen Substanzmengen, ohne Emesis oder Nausea auszulösen, in jedem Stadium des Krankheitsverlaufs.

### KOMBINATIONEN

Die Verbindungen der Formel 1 können allein oder in Kombination mit anderen erfindungsgemäßen Wirkstoffen der Formel 1 zur Anwendung gelangen. Gegebenenfalls können die Verbindungen der Formel 1 auch in Kombination mit weiteren pharmakologisch aktiven Wirkstoffen eingesetzt werden. Bevorzugt gelangen hierbei solche Wirkstoffe zur Anwendung, die beispielsweise ausgewählt sind aus der Gruppe bestehend aus Betamimetika, Anticholinergika, Corticosteroiden, weiteren PDE4-Inhibitoren, LTD4-Antagonisten, EGFR-Hemmern, MRP4-Inhibitoren, Dopamin-Agonisten, H1-Antihistaminika, PAF-Antagonisten und PI3-Kinase Inhibitoren oder zwei- oder dreifach Kombinationen davon, wie beispielsweise Kombinationen von Verbindungen der Formel 1 mit ein oder zwei Verbindungen aus der Gruppe bestehend aus
• Betamimetika, Corticosteroiden, PDE4-Inhibitoren, EGFR-Hemmer und LTD4-Antagonisten,
• Anticholinergika, Betamimetika, Corticosteroiden, PDE4-Inhibitoren, EGFR-Hemmem und LTD4-Antagonisten,
• PDE4-Inhibitoren, Corticosteroiden, EGFR-Hemmem und LTD4-Antagonisten
• EGFR-Hemmern, PDE4-inhibitoren und LTD4-Antagonisten
• EGFR-Hemmem und LTD4-Antagonisten
• CCR3-Inhibitoren, iNOS-Inhibitoren (inducible nitric oxide synthase-Inhibitoren), (6R)-L-erythro-5,6,7,8-tetrahydrobiopterin (im folgenden "BH4" genannt) und dessen Derivate wie in WO 2006/120176 genannt und SYK-Inhbitoren (spleen tyrosine kinase-Inhibitoren)
• Anticholinergika, Betamimetika, Corticosteroiden, PDE4-Inhibitoren und MRP4-Inhibitoren.
Auch die Kombinationen dreier Wirkstoffe je einer der o.g. Verbindungsklassen ist Bestandteil der Erfindung.

Als Betamimetika gelangen hierbei vorzugsweise Verbindungen zur Anwendung, die ausgewählt sind aus der Gruppe bestehend aus Albuterol, Bambuterol, Bitolterol, Broxaterol, Carbuterol, Clenbuterol, Fenoterol, Formoterol, Arformoterol, Zinterol, Hexoprenaline, Ibuterol, Isoetharine, Isoprenaline, Levosalbutamol, Mabuterol, Meluadrine, Metaproterenol, Orciprenaline, Pirbuterol, Procaterol, Reproterol, Rimiterol, Ritodrine, Salmeterol, Salmefamol, Soterenol, Sulphonterol, Tiaramide, Terbutaline, Tolubuterol, CHF-1035, HOKU-81, KUL-1248, 3-(4-{6-[2-Hydroxy-2-(4-hydroxy-3-hydroxymethyl-phenyl)-ethylamino]-hexyloxy}-butyl)-benzyl-sulfonamid, 5-[2-(5,6-Diethyl-indan-2-ylamino)-1-hydroxy-ethyl]-8-hydroxy-1H-quinolin-2-on, 4-Hydroxy-7-[2-{[2-{[3-(2-phenylethoxy)propyl]sulphonyl)ethyl]-amino}ethyl]-2(3H)-benzothiazolon, 1-(2-Fluoro-4-hydroxyphenyl)-2-[4-(1-benzimidazolyl)-2-methyl-2-butylamino]ethanol, 1-[3-(4-Methoxybenzyl-amino)-4-hydroxyphenyl]-2-[4-(1-benzimidazolyl)-2-methyl-2-butylamino]ethanol, 1-[2H-5-hydroxy-3-oxo-4H-1,4-benzoxazin-8-yl]-2-[3-(4-N,N-dimethylaminophenyl)-2-methyl-2-propylamino]ethanol, 1-[2H-5-hydroxy-3-oxo-4H-1,4-benzoxazin-8-yl]-2-[3-(4-methoxyphenyl)-2-methyl-2-propylamino]ethanol, 1-[2H-5-hydroxy-3-oxo-4H-1,4-benzoxazin-8-yl]-2-[3-(4-n-butyloxyphenyl)-2-methyl-2-propylamino]ethanol, 1-[2H-5-hydroxy-3-oxo-4H-1,4-benzoxazin-8-yl]-2-{4-[3-(4-methoxyphenyl)-1,2,4-triazol-3-yl]-2-Methyl-2-butylamino)ethanol, 5-Hydroxy-8-(1-hydroxy-2-isopropylaminobutyl)-2H-1,4-benzoxazin-3-(4H)-on, 1-(4-Amino-3-chloro-5-trifluormethylphenyl)-2-tert.-butylamino)ethanol, 6-Hydroxy-8-{1-hydroxy-2-[2-(4-methoxyphenyl)-1,1-dimethyl-ethylamino]-ethyl}-4H-benzo[1,4]oxazin-3-on, 6-Hydroxy-8-{1-hydroxy-2-[2-(4-phenoxy-essigsäureethylester)-1,1-dimethyl-ethylamino]-ethyl}-4H-benzo[1,4]oxazin-3-on, 6-Hydroxy-8-{1-hydroxy-2-[2-(4-phenoxy-essigsäure)-1,1-dimethyl-ethylamino]-ethyl}-4H-benzo[1,4]oxazin-3-on, 8-{2-[1,1-Dimethyl-2-(2,4,6-trimethylphenyl)-ethylamino]-1-hydroxy-ethyl}-6-hydroxy-4H-benzo[1,4]oxazin-3-on, 6-Hydroxy-8-{1-hydroxy-2-[2-(4-hydroxy-phenyl)-1,1-dimethyl-ethylamino]-ethyl}-4H-benzo[1,4]oxazin-3-on, 6-Hydroxy-8-{1-hydroxy-2-[2-(4-isopropyl-phenyl)-1,1dimethyl-ethylamino]-ethyl}-4H-benzo[1,4]oxazin-3-on, 8-{2-[2-(4-Ethyl-phenyl)-1,1-dimethyl-ethylamino]-1-hydroxy-ethyl}-6-hydroxy-4H-benzo[1,4]oxazin-3-on, 8-{2-[2-(4-Ethoxy-phenyl)-1,1-dimethyl-ethylamino]-1-hydroxy-ethyl}-6-hydroxy-4H-benzo[1,4]oxazin-3-on, 4-(4-{2-[2-Hydroxy-2-(6-hydroxy-3-oxo-3,4-dihydro-2H-benzo[1,4]oxazin-8-yl)-ethylamino]-2-methyl-propyl}-phenoxy)-buttersäure 8-{2-[2-(3,4-Difluor-phenyl)-1,1-dimethyl-ethylamino]-1-hydroxy-ethyl}-6-hydroxy-4H-benzo[1,4]oxazin-3-on und 1-(4-Ethoxy-carbonylamino-3-cyano-5-fluorophenyl)-2-(tert.-butylamino)ethanol, gegebenenfalls in Form ihrer Racemate, Enantiomere, Diasteromere und gegebenenfalls in Form ihrer pharmakologisch verträglichen Säureadditionssalze, Solvate oder Hydrate.

Bevorzugt sind die Betamimetika ausgewählt aus der Gruppe bestehend aus Bambuterol, Bitolterol, Carbuterol, Clenbuterol, Fenoterol, Formoterol, Hexoprenaline, Ibuterol, Pirbuterol, Procaterol, Reproterol, Salmeterol, Sulphonterol, Terbutaline, Tolubuterol, 3-(4-{6-[2-Hydroxy-2-(4-hydroxy-3-hydroxymethyl-phenyl)-ethylamino]-hexyloxy}-butyl)-benzenesulfoneamide, 5-{2-(5,6-Diethyl-indan-2-ylamino}-1-hydroxy-ethyl}-8-hydroxy-1H-quinolin-2-on , 4-hydroxy-7-[2-{[2-{[3-(2-phenylethoxy)propyl]sulphonyl}ethyl]-amino}ethyl]-2(3H)-benzothiazolone, 1-(2-fluoro-4-hydroxyphenyl)-2-[4-(1-benzimidazolyl)-2-methyl-2-butylamino]ethanol, 1-[3-(4-methoxybenzyl-amino)-4-hydroxyphenyl]-2-[4-(1-benzimidazolyl)-2-methyl-2-butylamino]ethanol, 1-[2H-5-hydroxy-3-oxo-4H-1,4-benzoxazin-8-yl]-2-[3-(4-N,N-dimethylaminophenyl)-2-methyl-2-propylamino]ethanol, 1-[2H-5-hydroxy-3-oxo-4H-1,4-benzoxazin-8-yl]-2-[3-(4-methoxyphenyl)-2-methyl-2-propylamino]ethanol, 1-[2H-5-hydroxy-3-oxo-4H-1,4-benzoxazin-8-yl]-2-[3-(4-n-butyloxyphenyl)-2-methyl-2-propylamino]ethanol, 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol, 5-hydroxy-8-(1-hydroxy-2-isopropylaminobutyl)-2H-1,4-benzoxazin-3-(4H)-on, 1-(4-amino-3-chloro-5-trifluormethylphenyl)-2-tert.-butylamino)ethanol, 6-Hydroxy-8-{1-hydroxy-2-[2-(4-methoxy-phenyl)-1,1-dimethyl-ethylamino]-ethyl}-4H-benzo[1,4]oxazin-3-on, 6-Hydroxy-8-(1-hydroxy-2-[2-(4-phenoxy-essigsäureethylester)-1,1-dimethyl-ethylamino]-ethyl}-4H-benzo[1,4]oxazin-3-on, 6-Hydroxy-8-[1-hydroxy-2-[2-(4-phenoxy-essigsäure)-1,1-dimethyl-ethylamino]-ethyl}-4H-benzo[1,4]oxazin-3-on, 8-{2-[1,1-Dimethyl-2-(2,4,6-trimethylphenyl)-ethylamino]-1-hydroxy-ethyl}-6-hydroxy-4H-benzo[1,4]oxazin-3-on, 6-Hydroxy-8-{1-hydroxy-2-[2-(4-hydroxy-phenyl)-1,1-dimethyl-ethylamino]-ethyl}-4H-benzo[1,4]oxazin-3-on, 6-Hydroxy-8-{1-hydroxy-2-[2-(4-isopropyl-phenyl)-1,1-dimethyl-ethylamino]-ethyl}-4H-benzo[1,4]oxazin-3-on, 8-{2-[2-(4-Ethyl-phenyl)-1,1-dimethyl-ethylamino]-1-hydroxy-ethyl}-6-hydroxy-4H-benzo[1,4]oxazin-3-on, 8-{2-[2-(4-Ethoxy-phenyl)-1,1-dimethyl-ethylamino]-1-hydroxy-ethyl}-6-hydroxy-4H-benzo[1,4]oxazin-3-on, 4-(4-{2-[2-Hydroxy-2-(6-hydroxy-3-oxo-3,4-dihydro-2H-benzo[1,4]oxazin-8-yl)-ethylamino]-2-methyl-propyl}-phenoxy)-buttersäure, 8-{2-[2-(3,4-Difluor-phenyl)-1,1-dimethyl-ethylamino]-1-hydroxy-ethyl}-6-hydroxy-4H-benzo[1,4]oxazin-3-on und 1-(4-ethoxycarbonylamino-3-cyano-5-fluorophenyl)-2-(tert.-butylamino)ethanol, gegebenenfalls in Form ihrer Racemate, Enantiomere, Diasteromere und gegebenenfalls in Form ihrer pharmakologisch verträglichen Säureadditionssalze, Solvate oder Hydrate.

Besonders bevorzugte Betamimetika sind ausgewählt aus der Gruppe bestehend aus Fenoterol, Formoterol, Salmeterol, 3-(4-{6-[2-Hydroxy-2-(4-hydroxy-3-hydroxymethyl-phenyl)-ethylamino]-hexyloxy}-butyl)-benzenesulfoneamid, 5-[2-(5,6-Diethyl-indan-2-ylamino)-1-hydroxy-ethyl]-6-hydroxy-1H-quinolin-2-on, 1-[3-(4-Methoxybenzyl-amino)-4-hydroxyphenyl]-2-[4-(1-benzimidazolyl)-2-methyl-2-butylamino]ethanol, 1-[2H-5-hydroxy-3-oxo-4H-1,4-benzoxazin-8-yl]-2-[3-(4-N,N-dimethylaminophenyl)-2-methyl-2-propylamino]ethanol, 1-[2H-5-Hydroxy-3-oxo-4H-1,4-benzoxazin-8-yl]-2-[3-(4-methoxyphenyl)-2-methyl-2-propylamino]ethanol, 1-[2H-5-Hydroxy-3-oxo-4H-1,4-benzoxazin-8-yl]-2-[3-(4-n-butyloxyphenyl)-2-methyl-2-propylamino]ethanol, 6-Hydroxy-8-{1-hydroxy-2-[2-(4-methoxyphenyl)-1,1-dimethyl-ethylamino]-ethyl}-4H-benzo[1,4]oxazin-3-on, 6-Hydroxy-8-{1-hydroxy-2-[2-(4-phenoxy-essigsäureethylester)-1,1-dimethyl-ethylamino]-ethyl}-4H-benzo[1,4]oxazin-3-on, 6-Hydroxy-8-{1-hydroxy-2-[2-(4-phenoxy-essigsäure)-1,1-dimethyl-ethylamino)-ethyl}-4H-benzo[1,4]oxazin-3-on, 8-{2-[1,1-Dimethyl-2-(2,4,6-trimethylphenyl)-ethylamino]-1-hydroxy-ethyl}-6-hydroxy-4H-benzo[1,4]oxazin-3-on, 6-Hydroxy-8-{1-hydroxy-2-[2-(4-hydroxy-phenyl)-1,1-dimethyl-ethylamino]-ethyl}-4H-benzo[1,4]oxazin-3-on, 6-Hydroxy-8-(1-hydroxy-2-[2-(4-isopropyl-phenyl)-1,1-dimethyl-ethylamino]-ethyl}-4H-benzo[1,4]oxazin-3-on, 8-{2-[2-(4-Ethyl-phenyl)-1,1-dimethyl-ethylamino]-1-hydroxy-ethyl}-6-hydroxy-4H-benzo[1,4]oxazin-3-on, 8-{2-[2-(4-Ethoxy-phenyl)-1,1-dimethyl-ethylamino]-1-hydroxy-ethyl}-6-hydroxy-4H-benzo[1,4]oxazin-3-on, 4-(4-{2-[2-Hydroxy-2-(6-hydroxy-3-oxo-3,4-dihydro-2H-benzo[1,4]oxazin-8-yl)-ethylamino]-2-methyl-propyl}-phenoxy)-buttersäure, 8-(2-[2-(3,4-Difluor-phenyl)-1,1-dimethyl-ethylamino]-1-hydroxy-ethyl}-6-hydroxy-4H-benzo[1,4]oxazin-3-on und 1-[2H-5-Hydroxy-3-oxo-4H-1,4-benzoxazin-8-yl]-2-{4-[3-(4-methoxyphenyl)-1,2,4-trlazol-3-yl]-2-methyl-2-butylamino}ethanol, gegebenenfalls in Form ihrer Racemate, Enantiomere. Diasteromere und gegebenenfalls in Form ihrer pharmakologisch verträglichen Säureadditionssalze, Solvate oder Hydrate.

Von diesen Betamimetika sind erfindungsgemäß besonders bevorzugt Formoterol, Salmeterol, 3-(4-{6-[2-Hydroxy-2-(4-hydroxy-3-hydroxymethyl-phenyl)-ethylamino]-hexyloxy)-butyl)-benzenesulfoneamide, 6-Hydroxy-8-{1-hydroxy-2-[2-(4-methoxy-phenyl)-1,1-dimethyl-ethylamino]-ethyl}-4H-benzo[1,4]oxazin-3-on, 6-Hydroxy-8-{1-hydroxy-1-[2-(4-phenoxy-essigsäureethylester)-1,1-dimethyl-ethylamino]-ethyl}-4H-benzo[1,4]oxazin-3-on, 6-Hydroxy-8-{1-hydroxy-2-[2-(4-phenoxy-essigsäure)-1,1-dimethyl-ethylamino]-ethyl}-4H-benzo[1,4]oxazin-3-on, 8-{2-[1,1-Dimethyl-2-(2,4,6-trimethylphenyl)-ethylamino]-1-hydroxyethyl}-6-hydroxy-4H-benzo[1,4]oxazin-3-on, 6-Hydroxy-8-{1-hydroxy-2-[2-(4-hydroxy-phenyl)-1,1-dimethyl-ethylamino]-ethyl}-4H-benzo[1,4]oxazin-3-on, 6-Hydroxy-8-{1-hydroxy-2-[2-(4-isopropyl-phenyl)-1,1dimethyl-ethylamino]-ethyl}-4H-benzo[1,4]oxazin-3-on, 8-{2-[2-(4-Ethyl-phenyl)-1,1-dimethyl-ethylamino]-1-hydroxy-ethyl}-6-hydroxy-4H-benzo[1,4]oxazin-3-on, 8-{2-[2-(4-Ethoxyphenyl)-1,1-dimethyl-ethylamino]-1-hydroxy-ethyl}-6-hydroxy-4H-benzo[1,4]oxazin-3-on, 4-(4-{2-[2-Hydroxy-2-(6-hydroxy-3-oxo-3,4-dihydro-2H-benzo[1,4]oxazin-8-yl)-ethylamino]-2-methyl-propyl}-phenoxy)-buttersäure, 8-{2-[2-(3,4-Difluor-phenyl)-1,1-dimethyl-ethylamino]-1-hydroxy-ethyl}-6-hydroxy-4H-benzo[1,4]oxazin-3-on und 5-[2-(5,6-Diethyl-indan-2-ylamino)-1-hydroxy-ethyl]-8-hydroxy-1H-quinolin-2-on, gegebenenfalls in Form ihrer Racemate, Enantiomere, Diasteromere und gegebenenfalls in Form ihrer pharmakologisch verträglichen Säureadditionssalze, Solvate oder Hydrate.

Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrobenzoat, Hydrocitrat, Hydrofumarat, Hydrotartrat, Hydrooxalat, Hydrosuccinat, Hydrobenzoat und Hydro-p-toluolsulfonat, bevorzugt Hydrochlorid, Hydrobromid, Hydrosulfat, Hydrophosphat, Hydrofumarat und Hydromethansulfonat verstanden. Von den vorstehend genannten Säureadditionssalzen sind die Salze der Chlorwasserstoffsäure, der Methansulfonsäure, der Benzoesäure und der Essigsäure erfindungsgemäß besonders bevorzugt.

Als Anticholinergika gelangen hierbei vorzugsweise Verbindungen zur Anwendung, die ausgewählt sind aus der Gruppe bestehend aus Tiotropiumsalzen, Oxitropiumsalzen, Flutropiumsalzen, Ipratropiumsalzen, Glycopyrroniumsalzen, Trospiumsalzen 2,2-Diphenylpropionsäuretropenolester-methobromid, 2,2-Diphenylpropionsäurescopinestermethobromid, 2-Fluor-2,2-Diphenylessigsäurescopinester-methobromid, 2-Fluor-2,2-Diphenylessigsäuretropenolester-methobromid, 3,3',4,4'-Tetrafluorbenzilsäuretropenolester-Methobromid, 3,3',4,4'-Tetrafluorbenzilsäurescopinester-Methobromid, 4,4'-Difluorbenzilsäuretropenolester-Methobromid, 4,4'-Difluorbenzilsäurescopinester-Methobromid, 3,3'-Difluorbenzilsäuretropenolester-Methobromid, 3,3'-Difluorbenzilsäurescopinester-Methobromid, 9-Hydroxy-fluoren-9-carbonsäureiropenolester -Methobromid, 9-Fluor-fluoren-9-carbonsäuretropenolester -Methobromid, 9-Hydroxy-fluoren-9-carbonsäurescopinester -Methobromid, 9-Fluor-fluoren-9-carbonsäurescopinester Methobromid, 9-Methyl-fluoren-9-carbonsäuretropenolester Methobromid, 9-Methyl-fluoren-9-carbonsäurescopinester Methobromid, Benzilsäurecyclopropyltropinester-Methobromid, 2,2-Diphenylpropionsäurecyclopropyltropinester -Methobromid, 9-Hydroxy-xanthen-9-carbonsäurecyclopropyltropinestermethobromid, 9-Methyl-fluoren-9-carbonsäurecyclopropyltropinester-Methobromid, 9-Methyl-xanthen-9-carbonsäurecyclopropyltropinester -Methobromid, 9-Hydroxy-fluoren-9-carbonsäurecyclopropyltropinester -Methobromid, 4,4'-Difluorbenzilsäuremethylestercyclopropyltropinester -Methobromid, 9-Hydroxy-xanthen-9-carbonsäuretropenolester -Methobromid, 9-Hydroxy-xanthen-9-carbonsäurescopinester Methobromid, 9-Methyl-xanthen-9-carbonsäuretropenolester -Methobromid, 9-Methyl-xanthen-9-carbonsäurescopinester -Methobromid, 9-Ethyl-xanthen-9-carbonsäuretropenolester Methobromid, 9-Difluormethyl-xanthen-9-carbonsäuretropenolester-Methobromid. 9-Hydroxymethyl-xanthen-9-carbonsäurescopinester -Methobromid, gegebenenfalls in Form ihrer Solvate oder Hydrate.

In den vorstehend genannten Salzen stellen die Kationen Tiotropium, Oxitropium, Flutropium, Ipratropium, Glycopyrronium und Trospium die pharmakologisch aktiven Bestandteile dar. Als Anionen können die vorstehend genannten Salze bevorzugt enthalten Chlorid, Bromid, Iodid, Sulfat, Phosphat, Methansulfonat, Nitrat, Maleat, Acetat, Citrat, Fumarat, Tartrat, Oxalat, Succinat, Benzoat oder p-Toluolsulfonat, wobei Chlorid, Bromid, Iodid, Sulfat, Methansulfonat oder p-Toluolsulfonat als Gegenionen bevorzugt sind. Von allen Salzen sind die Chloride. Bromide, Iodid und Methansulfonat besonders bevorzugt.

Von besonderer Bedeutung ist das Tiotropiumbromid. Im Falle des Tiotropiumbromids enthalten die erfindungsgemäßen Arzneimittelkombinationen dieses bevorzugt in Form des kristallinen Tiotropiumbromid Monohydrats, welches aus der WO 02/30928 bekannt ist. Wird das Tiotropiumbromid in den erfindungsgemäßen Arzneimittelkombinationen in wasserfreier Form eingesetzt, so gelangt bevorzugt das wasserfreie kristalline Tiotropiumbromid zur Anwendung, welches aus der WO 03/000265 bekannt ist.

Als Corticosteroide gelangen hierbei vorzugsweise Verbindungen zur Anwendung, die ausgewählt sind aus der Gruppe bestehend aus Prednisolon, Prednison, Butixocortpropionat, Flunisolid, Beclomethason, Triamcinolon, Budesonid, Fluticason, Mometason, Ciclesonid, Rofleponid, Dexamethason, Betamethason, Deflazacort, RPR-106541, NS-126, 6,9-Difluoro-17-[(2-furanylcarbonyl)oxy]-11-hydroxy-11-methyl-3-oxo-androsta-1,4-dien-17-carbothionsäure (S)-fluoromethylester und 6,9-Difluoro-11-hydroxy-16-methyl-3-oxo-17-propionyloxy-androsta-1,4-dien-17-carbothionsäure (S)-(2-oxo-tetrahydrofuran-3S-yl)ester, gegebenenfalls in Form ihrer Racemate, Enantiomere oder Diastereomere und gegebenenfalls in Form ihrer Salze und Derivate, ihrer Solvate und/oder Hydrate.

Besonders bevorzugt ist das Steroid ausgewählt aus der Gruppe bestehend aus Flunisolid, Beclomethason, Triamcinolon, Budesonid, Fluticason, Mometason, Ciclesonid, Rofleponid, Dexamethason, NS-126, 6,9-Difluoro-17-[(2-furanylcarbonyl)oxy]-11-hydroxy-16-methyl-3-oxo-androsta-1,4-dien-17-carbothionsäure (S)-fluoromethylester und 6,9-Difluoro-11-hydroxy-16-methyl-3-oxo-17-propionyloxy-androsta-1,4-dien-17-carbothionsäure (S)-(2-oxo-tetrahydro-furan-3S-yl)ester, gegebenenfalls in Form ihrer Racemate, Enantiomere oder Diastereomere und gegebenenfalls in Form ihrer Salze und Derivate, ihrer Solvate und/oder Hydrate.

Besonders bevorzugt ist das Steroid ausgewählt aus der Gruppe bestehend aus Budesonid, Fluticason, Mometason, Ciclesonid und 6,9-Difluoro-17-[(2-furanylcarbonyl)oxy]-11-hydroxy-16-methyl-3-oxo-androsta-1,4-dien-17-carbothionsäure (S)-fluoromethylester, gegebenenfalls in Form ihrer Racemate, Enantiomere oder Diastereomere und gegebenenfalls in Form ihrer Salze und Derivate, ihrer Solvate und/oder Hydrate.

Jede Bezugnahme auf Steroide schließt eine Bezugnahme auf deren gegebenenfalls existierende Salze oder Derivate, Hydrate oder Solvate mit ein. Beispiele möglicher Salze und Derivate der Steroide können sein: Alkalisalze, wie beispielsweise Natrium- oder Kaliumsalze, Sulfobenzoate, Phosphate, Isonicotinate, Acetate, Propionate, Dihydrogenphosphate, Palmitate, Pivalate oder auch Furoate.

Als weitere PDE4-Inhibitoren gelangen hierbei vorzugsweise Verbindungen zur Anwendung, die ausgewählt sind aus der Gruppe bestehend aus Enprofyllin, Theophyllin, Roflumilast, Ariflo (Cilomilast), Tofimilast, Pumafentrin, Lirimilast, Arofyllin, Atizoram, D-4396 (Sch-351591), AWD-12-281 (GW-842470), NCS-613, CDP-840, D-4418, PD-168787, T-440, T-2585, V-11294A, CI-1018, CDC-801, CDC-3052, D-22888, YM-58997, Z-15370,N-(3,5-Dichforo-1-oxo-pyridin-4-yl)-4-difluoromethoxy-3-cyclopropylmethoxybenzamid, (-)p-[(4aR*, 10bS*)-9-Ethoxy-1,2,3,4,4a,10b-hexahydro-8-methoxy-2-methylbenzo[s][1,6]naphthyridin-6-yl]-N,N-diisopropylbenzamid, (R)-(+)-1-(4-Bromobenzyl)-4-[(3-cyclopentyloxy)-4-methoxyphenyl]-2-pyrrolidon, 3-(Cyclopentytoxy-4-methoxyphenyl)-1-(4-N'-[N-2-cyano-S-methyl-isothioureido]benzyl)-2-pyrrolidon, cis[4-Cyano-4-(3-cyclopentyloxy-4-methoxyphenyl)cyclohexan-1-carbonsäure], 2-carbomethoxy-4-cyano-4-(3-cyclopropylmethoxy-4-difluorornethoxyphenyl)cyclohexan-1-on, cis[4-Cyano-4-(3-cyclopropylmethoxy-4-difluoromethoxyphenyl)cyclohexan-1-ol], (R)-(+)-Ethyl[4-(3-cyclopentyloxy-4-methoxyphenyl)pyrrolidin-2-yliden]acetat, (S)-(-)-Ethyl[4-(3-cyclopentyloxy-4-methoxyphenyl)pyrrolidin-2-yliden]acetat, 9-Cyclopentyl-5,6-dihydro-7-ethyl-3-(2-thienyl)-9H-pyrazolo[3,4-c]-1,2,4-triazolo[4,3-a]pyridin und 9-Cyclopentyl-5,6-dihydro-7-ethyl-3-(tert-butyl)-9H-pyrazolo[3,4-c]-1,2,4-triazolo[4,3-a]pyridin, gegebenenfalls in Form ihrer Racemate, Enantiomere oder Diastereomere und gegebenenfalls in Form ihrer pharmakologisch verträglichen Säureadditionssalze, Solvate und/oder Hydrate.

Besonders bevorzugt ist der PDE4-Inhibitor ausgewählt aus der Gruppe bestehend aus Enprofyllin, Roflumitast, Ariflo (Cilomilast), Arofyllin, Atizoram,AWD-12-281 (GW-842470), T-440, T-2585, PD-168787, V-11294A, CI-1018, CDC-801, D-22888, YM-58997, Z-15370, N-(3,5-Dichloro-1-oxo-pyridin-4-yl)-difluoromethoxy-3-cyclopropylmethoxybenzamid, Cis[4-Cyano-4-(3-cyclopentyloxy-4-methoxyphenyl)cyclohexan-1-carbonsäure], 2-Carbomethoxy-4-cyano-4-(3-cyclopropylmethoxy-4-difluoromethoxyphenyl)cyclohexan-1-on, Cis[4-cyano-4-(3-cyclopropylmethoxy-4-difluoromethoxyphenyl)cyclohexan-1-ol], 9-Cyclopentyl-5,6-dihydro-7-ethyl-3-(2-thienyl)-9H-pyrazolo[3,4-c]-1,2,4-triazolo[4,3-a]pyridin und 9-Cyclopentyl-5,6-dihydro-7-ethyl-3-(tert-butyl)-9H-pyrazolo[3,4-c]-1,2,4-triazolo[4,3-a]pyridin, gegebenenfalls in Form ihrer Racemate, Enantiomere oder Diastereomere und gegebenenfalls in Form ihrer pharmakologisch verträglichen Säureadditionssalze, Solvate und/oder Hydrate.

Besonders bevorzugt ist der PDE4-Inhibitor ausgewählt aus der Gruppe bestehend aus Roflumilast, Ariflo (Cilomilast), Arofyllin, AWD-12-281 (GW-842470), 2-Carbomethoxy-4-cyano-4-(3-cyclopropylmethoxy-4-difluoromethoxyphenyl)cyclohexan-1-on, Cis[4-cyano-4-(3-cyclopropylmethoxy-4-difluoromethoxyphenyl)cyclohexan-1-ol], Atizoram, Z-15370, 9-Cyclopentyl-5,6-dihydro-7-ethyl-3-(2-thienyl)-9H-pyrazolo[3,4-c]-1,2,4-Mazolo[4,3-a]pyridin und 9-Cyclopentyl-5,6-dihydro-7-ethyl-3-(tert-butyl)-9H-pyrazolo[3,4-c]-1,2,4-triazolo[4,3-a]pyridin gegebenenfalls in Form ihrer Racemate, Enantiomere oder Diastereomere und gegebenenfalls in Form ihrer pharmakologisch verträglichen Säureadditionssalze, Solvate und/oder Hydrate.

Unter Säureadditionssalzen mit pharmakologisch verträglichen Säuren zu deren Bildung die o.g. PDE4-inhibitoren gegebenenfalls in der Lage sind, werden beispielsweise Salze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrobenzoat, Hydrocitrat, Hydrofumarat, Hydrotartrat, Hydrooxalat, Hydrosuccinat, Hydrobenzoat und Hydro-p-toluolsulfonat, bevorzugt Hydrochlorid, Hydrobromid, Hydrosulfat, Hydrophosphat, Hydrofumarat und Hydromethansulfonat verstanden.

Als LTD4-Antagonisten gelangen hierbei vorzugsweise Verbindungen zur Anwendung die ausgewählt sind aus der Gruppe bestehend aus Montelukast, Pranlukast, Zafirlukast, MCC-847 (ZD-3523), MN-001, MEN-91507 (LM-1507), VUF-5078, VUF-K-8707, L-733321, 1-(((R)-(3-(2-(6,7-Difluoro-2-quinolinyl)ethenyl)phenyl)-3-(2-(2- hydroxy-2-propyl)phenyl)thio)-methylcyclopropan-essigsäure, 1-(((1(R)-3(3-(2-(2,3-Dichlorothieno[3,2-b]pyridin-5-yl)-(E)-ethenyl)phenyl)-3-(2-(1-hydroxy-1-methylethyl)phenyl)propyl)thio)methyl)cyclopropanessigsäure und [2-[[2-(4-tert-Butyl-2-thiazolyl)-5-benzofuranyl]oxymethyl]phenyl]essigsäure gegebenenfalls in Form ihrer Racemate, Enantiomere oder Diastereomere, gegebenenfalls in Form ihrer pharmakologisch verträglichen Säureadditionssalze sowie gegebenenfalls in Form ihrer Salze und Derivate, ihrer Solvate und/oder Hydrate.

Bevorzugt ist der LTD4-Antagonist ausgewählt aus der Gruppe bestehend aus Montelukast, Pranlukast, Zafirlukast, MCC-847 (ZD-3523), MN-001, MEN-91507 (LM-1507), VUF-5078, VUF-K-8707 und L-733321, gegebenenfalls in Form ihrer Racemate, Enantiomere oder Diastereomere, gegebenenfalls in Form ihrer pharmakologisch verträglichen Säureadditionssalze sowie gegebenenfalls in Form ihrer Salze und Derivate, ihrer Solvate und/oder Hydrate.

Besonders bevorzugt ist der LTD4-Antagonist ausgewählt aus der Gruppe bestehend aus Montelukast, Pranlukast, Zafirlukast, MCC-847 (ZD-3523), MN-001 und MEN-91507 (LM-1507) gegebenenfalls in Form ihrer Racemate, Enantiomere oder Diastereomere, gegebenenfalls in Form ihrer pharmakoligisch verträglichen Säureadditionssalze sowie gegebenenfalls in Form ihrer Salze und Derivate, ihrer Solvate und/oder Hydrate.

Unter Säureadditionssalzen mit pharmakologisch verträglichen Säuren zu deren Bildung die LTD4-Antagonisten gegebenenfalls in der Lage sind, werden beispielsweise Salze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrobenzoat, Hydrocitrat, Hydrofumarat, Hydrotartrat, Hydrooxalat, Hydrosuccinat, Hydrobenzoat und Hydro-p-toluolsulfonat, bevorzugt Hydrochlorid, Hydrobromid, Hydrosulfat, Hydrophosphat, Hydrofumarat und Hydromethansulfonat verstanden. Unter Salzen oder Derivaten zu deren Bildung die LTD4-antagonisten gegebenenfalls in der Lage sind, werden beispielsweise verstanden : Alkalisalze, wie beispielsweise Natrium- oder Kaliumsalze, Erdalkalisalze, Sulfobenzoate, Phosphate, Isonicotinate, Acetate, Propionate, Dihydrogenphosphate, Palmitate, Pivalate oder auch Furoate.

Als EGFR-Hemmer gelangen hierbei vorzugsweise Verbindungen zur Anwendung, die ausgewählt sind aus der Gruppe bestehend aus 4-[(3-Chlor-4-fluorphenyl)amino]-6-{[4-(morpholin-4-yl)-1-oxo-2-buten-1-yl]amino}-7-cyclopropylmethoxy-chinazolin, 4-[(3-Chlor-4-fluorphenyl)amino]-6-{[4-(N,N-diethylamino)-1-oxo-2-buten-1-yl]amino}-7-cyclopropylmethoxy-chinazolin, 4-[(3-Chlor-4-fluorphenyl)amino]-6-{[4-(N,N-dimethylamino)-1-oxo-2-buten-1-yl]amino}-7-cyclopropylmethoxy-chinazolin, 4-[(R)-(1-Phenyl-ethyl)amino]-6-{[4-(morpholin-4-yl)-1-oxo-2-buten-1-yl]amino}-7-cyclopentyloxy-chinazolin, 4-[(3-Chlor-4-fluor-phenyl)amino]-6-{[4-((R)-6-methyl-2-oxo-morpholin-4-yl)-1-oxo-2-buten-1-yl]amino}-7-cyclopropylmethoxy-chinazolin, 4-[(3-Chlor-4-fluor-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin, 4-[(3-Chlor-4-fluor-phenyl)amino]-6-{[4-((R)-2-methoxymethyl-5-oxo-morpholin-4-yl)-1-oxo-2-buten-1-yl]amino}-7-cyclopropylmethoxy-chinazolin, 4-[(3-Chlor-4-fluor-phenyl)amino]-6-[2-((S)-6-methyl-2-oxo-morpholin-4-yl)-ethoxy]-7-methoxy-chinazolin, 4-[(3-Chlor-4-fluorphenyl)amino]-6-({4-(N-(2-methoxy-ethyl)-N-methyl-amino)-1-oxo-2-buten-1-yl}amino)-7-cyclopropylmethoxy-chinazolin, 4-[(3-Chlor-4-fluorphenyl)amino]-6-{[4-(N,N-dimethylamino)-1-oxo-2-buten-1-yl]amino}-7-cyclopentyloxy-chinazolin, 4-[(R)-(1-Phenyl-ethyl)amino]-6-{[4-(N,N-bis-(2-methoxy-ethyl)-amino)-1-oxo-2-buten-1-yl]amino}-7-cyclopropylmethoxy-chinazolin, 4-[(R)-(1-Phenyl-ethyl)amino]-6-({4-[N-(2-methoxy-ethyl)-N-ethyl-amino]-1-oxo-2-buten-1-yl}amino)-7-cyclopropylmethoxy-chinazolin, 4-[(R)-(1-Phenyl-ethyl)amino]-6-({4-[N-(2-methoxy-ethyl)-N-methyl-amino]-1-oxo-2-buten-1-yl}amino)-7-cyclopropylmethoxy-chinazolin, 4-[(R)-(1-Phenyl-ethyl)amino]-6-({4-[N-(tetrahydropyran-4-yl)-N-methyl-amino]-1-oxo-2-buten-1-yl}amino)-7-cyclopropylmethoxy-chinazolin, 4-[(3-Chlor-4-fluorphenyl)amino]-6-{[4-(N-N-dimethylamino)-1-oxo-2-buten-1-yl]amino}-7-((R)-tetrahydrofuran-3-yloxy)-chinazolin, 4-[(3-Chlor-4-fluorphenyl)amino]-6-{[4-(N,N-dimethylamino)-1-oxo-2-buten-1-yl]amino}-7-((S)-tetrahydrofuran-3-yloxy)-chinazolin, 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin, 4-[(3-Chlor-4-fluorphenyl)amino]-6-{[4-(N-cyclopropyl-N-methyl-amino)-1-oxo-2-buten-1-yl]amino}-7-cyclopentyloxy-chinazolin, 4-[(3-Chlor-4-fluorphenyl)amino]-6-{[4-(N,N-dimethylamino)-1-oxo-2-buten-1-yl]amino}-7-[(R)-(tetrahydrofuran-2-yl)methoxy]-chinazolin, 4-[(3-Chlor-4-fluorphenyl)amino]-6-{[4-(N,N-dimethylamino)-1-oxo-2-buten-1-yl]amino)-7-[(S)-(tetrahydrofuran-2-yl)methoxy]-chinazolin, 4-[(3-Ethinyl-phenyl)amino]-6,7-bis-(2-methoxy-ethoxy)-chinazolin, 4-[(3-Chlor-4-fluorphenyl)amino]-7-[3-(morpholin-4-yl)-propyloxy]-6-[(vinylcarbonyl)amino]-chinazolin, 4-[(R)-(1-Phenyl-ethyl)amino]-6-(4-hydroxy-phenyl)-7H-pyrrolo[2,3-d]pyrimidin, 3-Cyano-4-[(3-chlor-4-fluorphenyl)amino]-{[4-(N,N-dimethylamino)-1-oxo-2-buten-1-yl]amino}-7-ethoxy-chinolin, 4-{[3-Chlor-4-(3-fluor-benzyloxy)-phenyl]amino}-6-(5-{[(2-methansulfonyl-ethyl)amino]methyl}-furan-2-yl)chinazolin, 4-[(R)-(1-Phenylethyl)amino]-6-{[4-((R)-6-methyl-2-oxo-morpholin-4-yl)-1-oxo-2-buten-1-yl]amino}-7-methoxy-chinazolin, 4-[(3-Chlor-4-fluorphenyl)amino]-6-{[4-(morpholin-4-yl)-1-xo-2-buten-1-yl]amino}-7-[(tetrahydrofuran-2-yl)methoxy]-chinazolin, 4-[(3-Chlor-4-fluorphenyl)amino]-6-({4-[N,N-bis-(2-methoxy-ethyl)-amino]-1-oxo-2-buten-1-yl}amino)-7-[(tetrahydrofuran-2-yl)methoxy]-chinazolin, 4-[(3-Ethinyl-phenyl)amino]-6-{[4-(5,5-dimethyl-2-oxo-morpholin-4-yl)-1-oxo-2-buten-1-yl]amino}-chinazolin, 4-[(3-Chlor-4-fluor-phenyl)amino]-6-[2-(2,2-dimethyl,-6-oxo-morpholin-4-yl)-ethoxy]-7-methoxy-chinazolin, 4-[(3-Chlor-4-fluor-phenyl)amino]-6-[2-(2,2-dimethyl-6-oxo-morpholin-4-yl)-ethoxy]-7-[(R)-(tetrahydrofuran-2-yl)methoxy]-chinazolin, 4-[(3-Chlor-4-fluor-phenyl)amino]-7-[2-(2,2-dimethyl-6-oxo-morpholin-4-yl)-ethoxy]-6-[(S)-(tetrahydrofuran-2-yl)methoxy]-chinazolin, 4-[(3-Chlor-4-fluor-phenyl)amino]-6-{2-[4-(2-oxo-morpholin-4-yl)-piperidin-1-yl]-ethoxy}-7-methoxy-chinazolin, 4-[(3-Chlor-4-fluor-phenyl)amino]-6-[1-(tert-butyloxycarbonyl)-piperidin-4-yloxyl-7-methoxy-chinazolin, 4-[(3-Chlor-4-fluor-phenyl)amino]-6-(trans-4-amino-cyclohexan-1-yloxy)-7-methoxy-chinazolin, 4-[(3-Chlor-4-fluor-phenyl)amino]-6-(trans-4-methansulfonylamino-cyclohexan-1-yloxy)-7-methoxy-chinazolin, 4-[(3-Chlor-4-fluor-phenyl)amino]-6-(tetrahydropyran-3-yloxy)-7-methoxy-chinazolin, 4-[(3-Chlor-4-fluor-phenyl)amino]-6-(1-methyl-piperidin-4-yloxy)-7-methoxy-chinazolin, 4-[(3-Chlor-4-fluor-phenyl)amino]-6-{1-[(morpholin-4-yl)carbonyl]-piperidin-4-yloxy}-7-methoxy-chinazolin, 4-[(3-Chlor-4-fluor-phenyl)amino]-6-{1-[(methoxymethyl)carbonyl]-piperidin-4-yloxy}-7-methoxy-chinazolin, 4-[(3-Chlor-4-fluor-phenyl)amino]-6-(piperidin-3-yloxy)-7-methoxy-chinazolin, 4-[(3-Chlor-4-fluor-phenyl)amino]-6-[1-(2-acetylamino-ethyl)-piperidin-4-yloxy]-7-methoxy-chinazolin, 4-[(3-Chlor-4-fluor-phenyl)amino]-6-(tetrahydropyran-4-yloxy)-7-ethoxy-chinazolin, 4-[(3-Chlor-4-fluor-phenyl)amino]-6-((S)-tetrahydrofuran-3-yloxy)-7-hydroxy-chinazolin, 4-[(3-Chlor-4-fluor-phenyl)amino]-6-(tetrahydropyran-4-yloxy)-7-(2-methoxy-ethoxy)-chinazolin, 4-[(3-Chlor-4-fluor-phenyl)amino]-6-{trans-4-[(dimethylamino)sulfonylamino]-cyclohexan-1-yloxy}-7-methoxy-chinazolin, 4-[(3-Chlor-4-fluor-phenyl)amino]-6-{trans-4-[(morpholin-4-yl)carbonylamino]-cyclohexan-1-yloxy}-7-methoxy-chinazolin, 4-[(3-Chlor-4-fluor-phenyl)amino]-6-{trans-4-[(morpholin-4-yl)sulfonylamino]-cyclohexan-1-yloxy}-7-methoxy-chinazolin, 4-[(3-Chlor-4-fluor-phenyl)amino]-6-(tetrahydropyran-4-yloxy)-7-(2-acetylamino-ethoxy)-chinazolin, 4-[(3-Chlor-4-fluor-phenyl)amino]-6-(tetrahydropyran-4-yloxy)-7-(2-methansulfonylamino-ethoxy)-chinazolin, 4-[(3-Chlor-4-fluor-phenyl)amino]-6-{1-[(piperidin-1-yl)carbonyl]-piperidin-4-yloxy}-7-methoxy-chinazolin, 4-[(3-Chlor-4-fluor-phenyl)amino]-6-(1-aminocarbonylmethyl-piperidin-4-yloxy)-7-methoxy-chinazolin, 4-[(3-Chlor-4-fluor-phenyl)amino]-6-(cis-4-(N-[(tetrahydropyran-4-yl)carbonyl]-N-methyl-amino)-cyclohexan-1-yloxy)-7-methoxy-chinazolin, 4-[(3-Chlor-4-fluor-phenyl)amino]-6-(cis-4-{N-[(morpholin-4-yl)carbonyl]-N-methyl-amino}-cyclohexan-1-yloxy)-7-methoxy-chinazolin, 4-[(3-Chlor-4-fluor-phenyl)amino]-6-(cis-4-{N-[(morpholin-4-yl)sulfonyl]-N-methyl-amino}-cyclohexan-1-yloxy)-7-methoxy- chinazolin, 4-[(3-Chlor-4-fluor-phenyl)amino]-6-(trans-4-ethansulfonylamino-cyclohexan-1-yloxy)-7-methoxy-chinazolin, 4-[(3-Chlor-4-fluor-phenyl)amino]-6-(1-methansulfonyl-piperidin-4-yloxy)-7-ethoxy-chinazolin, 4-[(3-Chlor-4-fluor-phenyl)amino]-6-(1-methansulfonyl-piperidin-4-yloxy)-7-(2-methoxy-ethoxy)-chinazolin, 4-[(3-Chlor-4-fluor-phenyl)amino]-6-[1-(2-methoxy-acetyl)-piperidin-4-yloxy]-7-(2-methoxy-ethoxy)-chinazolin, 4-[(3-Chlor-4-fluor-phenyl)amino]-6-(cis-4-acetylamino-cyclohexan-1-yloxy)-7-methoxy-chinazolin, 4-[(3-Ethinyl-phenyl)amino]-6-[1-(tert.-butyloxycarbonyl)-piperidin-4-yloxy]-7-methoxy-chinazolin, 4-[(3-Ethinyl-phenyl)amino]-6-(tetrahydropyran-4-yloxy]-7-methoxy-, chinazolin, 4-[(3-Chlor-4-fluor-phenyl)amino]-6-(cis-4-{N-[(piperidin-1-yl)carbonyl]-N-methyl-amino}-cyclohexan-1-yloxy)-7-methoxy-chinazolin, 4-[(3-Chlor-4-fluor-phenyl)amino]-6-(cis-4-{N-[(4-methyl-piperazin-1-yl)carbonyl]-N-methyl-amino}-cyclohexan-1-yloxy)-7-methoxy-chinazolin, 4-[(3-Chlor-4-fluor-phenyl)amino]-6-{cis-4-[(morpholin-4-yl)carbonylamino]-cyclohexan-1-yloxy}-7-methoxy-chinazolin, 4-[(3-Chlor-4-fluor-phenyl)amino]-6-{1-[2-(2-oxopyrrolidin-1-yl)ethyl]-piperidin-4-yloxy}-7-methoxy-chinazolin, 4-[(3-Chlor-4-fluor-phenyl)amino]-6-{1-[(morpholin-4-yl)carbonyl]-piperidin-4-yloxy}-7-(2-methoxy-ethoxy)-chinazolin, 4-[(3-Ethinyl-phenyl)amino]-6-(1-acetyl-piperidin-4-yloxy)-7-methoxy-chinazolin, 4-[(3-Ethinyl-phenyl)amino]-6-(1-methyl-piperidin-4-yloxy)-7-methoxy-chinazolin, 4-[(3-Ethinyl-phenyl)amino]-6-(1-methansulfonyl-piperidin-4-yloxy)-7-methoxy-chinazolin, 4-[(3-Chlor-4-fluor-phenyl)amino]-6-(1-methyl-piperidin-4-yloxy)-7(2-methoxy-ethoxy)-chinazolin, 4-[(3-Chlor-4-fluor-phenyl)amino]-6-(1-isopropyloxycarbonyl-piperidin-4-ytoxy)-7-methoxy-chinazolin, 4-[(3-Chlor-4-fluor-phenyl)amino]-6-(cis-4-methylamino-cyclohexan-1-yloxy)-7-methoxy-chinazolin, 4-[(3-Chlor-4-fluor-phenyl)amino]-6-{cis-4-[N-(2-methoxy-acetyl)-N-methyl-amino]-cyclohexan-1-yloxy}-7-methoxy-chinazolin, 4-[(3-Ethinyl-phenyl)amino]-6-(piperidin-4-yloxy)-7-methoxy-chinazolin, 4-[(3-Ethinyl-phenyl)amino]-6-[1-(2-methoxy-acetyl)-piperidin-4-yloxy]-7-methoxy-cchinazolin, 4-[(3-Ethinyl-phenyl)amino]-6-{1-[(morpholin-4-yl)carbonyl]-piperidin-4-yloxy}-7-methoxy-chinazolin, 4-[(3-Chlor-4-fluor-phenyl)amino]-6-{1-[(cis-2,6-dimethyl-morpholin-4-yl)carbonyl]-piperidin-4-yloxy}-7-methoxy-chinazolin, 4-[(3-Chlor-4-fluor-phenyl)amino]-6-{1-[(2-methyl-morpholin-4-yl)carbonyl]-piperidin-4-yloxy}-7-methoxy-chinazolin, 4-[(3-Chlor-4-fluor-phenyl)amino]-6-{1-[(S,S)-(2-oxa-5-aza-bicyclo[2.2.1]hept-5-yl)carbonyl]-piperidin-4-yloxy}-7-methoxy-chinazolin, 4-[(3-Chlor-4-fluor-phenyl)amino]-6-{1-[(N-methyl-N-2-methoxyethyl-amino)carbonyl]-piperidin-4-yloxy}-7-methoxy-chinazolin, 4-[(3-Chlor-4-fluor-phenyl)amino]-6-(1-ethyl-piperidin-4-yloxy)-7-methoxy-chinazolin, 4-[(3-Chlor-4-fluor-phenyl)amino]-6-{1-[(2-methoxyethyl)carbonyl]-piperidin-4-yloxy}-7-methoxy-chinazolin, 4-[(3-Chlor-4-fluor-phenyl)amino]-6-{1-[(3-methoxypropyl-amino)-carbonyl]-piperidin-4-yloxy}-7-methoxy-chinazolin, 4-[(3-Chlor-4-fluor-phenyl)amino]-6-[cis-4-(N-methansulfonyl-N-methyl-amino)-cyclohexan-1-yloxy]-7-methoxy-chinazolin, 4-[(3-Chlor-4-fluor-phenyl)amino]-6-[cis-4-(N-acetyl-N-methyl-amino)-cyclohexan-1-yloxy]-7-methoxy-chinazolin, 4-[(3-Chlor-4-fluor-phenyl)amino]-6-(trans-4-methylamino-cyclohexan-1-yloxy)-7-methoxy-chinazolin, 4-[(3-Chlor-4-fluor-phenyl)amino]-6-[trans-4-(N-methansulfonyl-N-methyl-amino)-cyclohexan-1-yloxy]-7-methoxy-chinazolin, 4-[(3-Chlor-4-fluor-phenyl)amino]-6-(trans-4-dimethylamino-cyclohexan-1-yloxy)-7-methoxy-chinazolin 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazolin, 4-[(3-Chlor-4-fluor-phenyl)amino]-6-[2-(2,2-dimethyl-6-oxo-morpholin-4-yl)-ethoxy]-7-[(S)-(tetrahydrofuran-2-yl)methoxy]-chinazolin, 4-[(3-Chlor-4-fluor-phenyl)amino]-6-(1-methansulfonyl-piperidin-4-yloxy)-7-methoxy-chinazolin, 4-[(3-Chlor-4-fluor-phenyl)amino]-6-(1-cyano-piperidin-4-yloxy)-7-methoxy-chinazolin, Cetuximab, Trastuzumab, ABX-EGF und Mab ICR-62, gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

Bevorzugte EGFR-Hemmer sind ausgewählt aus der Gruppe bestehend aus 4-[(3-Chlor-4-fluorphenyl)amino]-6-{[4-(morpholin-4-yl)-1-oxo-2-buten-1-yl]amino}-7-cyclopropylmethoxy-chinazolin, 4-[(3-Chlor-4-fluorphenyl)amino]-6-{[4-(N,N-diethylamino)-1-oxo-2-buten-1-yl]-amino}-7-cyclopropylmethoxy-chinazolin, 4-[(3-Chlor-4-fluorphenyl)amino]-6-{[4-(N,N-dimethylamino)-1-oxo-2-buten-1-yl]amino}-7-cyclopropylmethoxy-chinazolin, 4-[(R)-(1-Phenyl-ethyl)amino]-6-{[4-(morpholin-4-yl)-1-oxo-2-buten-1-yl]amino}-7-cyclopentyloxy-chinazolin, 4-[(3-Chlor-4-fluor-phenyl)amino]-6-{[4-((R)-6-methyl-2-oxo-morpholin-4-yl)-1-oxo-2-buten-1-yl]amino}-7-cyclopropylmethoxy-chinazolin, 4-[(3-Chlor-4-fluor-phenyl)amino]-6-{[4-((R)-6-methyl-2-oxo-morpholin-4-yl)-1-oxo-2-buten-1-yl]amino}-7-(S)-(tetrahydrofuran-3-yl)oxy]-chinazolin, 4-[(3-Chlor-4-fluor-phenyl)amino]-6-{[4-((R)-2-methoxymethyl-6-oxo-morpholin-4-yl)-1-oxo-2-buten-1-yl]amino}-7-cyclopropylmethoxy-chinazolin, 4-[(3-Chlor-4-fluor-phenyl)amino]-6-[2-((S)-6-methyl-2-oxo-morpholin-4-yl)-ethoxy]-7-methoxy-chinazolin, 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin, 4-[(3-Chlor-4-fluorphenyl)amino]-6-{[4-(N,N-dimethylamino)-1-oxo-2-buten-1-yl]amino}-7-cyclopentyloxy-chinazolin, 4-[(R)-(1-Phenyl-ethyl)amino]-6-{[4-(N,N-bis-(2-methoxy-ethyl)-amino)-1-oxo-2-buten-1-yl]amino}-7-cyclopropylmethoxy-chinazolin, 4-[(R)-(1-Phenyl-ethyl)amino]-6-({4-[N-(2-methoxy-ethyl)-N-ethyl-amino]-1-oxo-2-buten-1-yl}amino)-7-cyclopropylmethoxy-chinazolin, 4-[(R)-(1-Phenyl-ethyl)amino]-6-({4-[N-(2-methoxy-ethyl)-N-methyl-amino]-1-oxo-2-buten-1-yl}amino)-7-cyclopropylmethoxy-chinazolin, 4-[(R)-(1-Phenyl-ethyl)amino]-6-({4-[N-(tetrahydropyran-4-yl)-N-methyl-amino]-1-oxo-2-buten-1-yl}amino)-7-cyclopropylmethoxy-chinazolin, 4-[(3-Chlor-4-fluorphenyl)amino]-6-{[4-(N,N-dimethylamino)-1-oxo-2-buten-1-yl]amino}-7-((R)-tetrahydrofuran-3-yloxy)-chinazolin, 4-[(3-Chlor-4-fluorphenyl)amino]-6-{[4-(N,N-dimethylamino)-1-oxo-2-buten-1-yl]amino}-7-((S)-tetrahydrofuran-3-yloxy)-chinazolin, 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin, 4-[(3-Chlor-4-fluorphenyl)amino]-6-{[4-(N-cyclopropyl-N-methyl-amino)-oxo-2-buten-1-yl]amino}-7-cyclopentyloxy-chinazolin, 4-[(3-Chlor-4-fluorphenyl)amino]-6-{[4-(N,N-dimethylamino)-1-oxo-2-buten-1-yl]amino}-7-[(R)-(tetrahydrofuran-2-yl)methoxy]-chinazolin, 4-[(3-Chlor-4-fluorphenyl)amino]-6-{[4-(N,N-dimethylamino)-1-oxo-2-buten-1-yl]amino}-7-[(S)-(tetrahydrofuran-2-yl)methoxy]-chinazolin, 4-[(3-Ethinyl-phenyl)amino]-6,7-bis-(2-methoxy-ethoxy)-chinazolin, 4-[(3-Chlor-4-fluorphenyl)amino]-7-[3-(morpholin-4-yl)-propyloxy]-6-[(vinylcarbonyl)amino]-chinazolin, 4-[(R)-(1-Phenyl-ethyl)amino]-6-(4-hydroxy-phenyl)-7H-pyrrolo[2,3-d]pyrimidin, 3-Cyano-4-[(3-chlor-4-fluorphenyl)amino]-6-{[4-(N,N-dimethylamino)-1-oxo-2-buten-1-yl]amino}-7-ethoxy-chinolin, 4-{[3-Chlor-4-(3-fluor-benzyloxy)-phenyl]amino}-6-(5-{[(2-methansulfonyl-ethyl)amino]methyl}-furan-2-yl)chinazolin, 4-[(R)-(1-Phenyl-ethyl)amino]-6-{[4-((R)-6-methyl-2-oxo-morpholin-4-yl)-1-oxo-2-buten-1-yl]amino}-7-methoxy-chinazolin, 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin, 4-[(3-Chlor-4-fluorphenyl)amino]-6-({4-[N,N-bis-(2-methoxy-ethyl)-amino]-1-oxo-2-buten-1-yl}amino)-7-[(tetrahydrofuran-2-yl)methoxy]-chinazolin, 4-[(3-Ethinyl-phenyl)amino]-6-{[4-(5,5-dimethyl-2-oxo-morpholin-4-yl)-1-oxo-2-buten-1-yl]amino}-chinazolin, 4-[(3-Chlor-4-fluor-phenyl)amino]-6-[2-(2,2-dimethyl-6-oxo-morpholin-4-yl)-ethoxy]-7-methoxy-chinazolin, 4-[(3-Chlor-4-fluor-phenyl)amino]-6-[2-(2,2-dimethyl-6-oxo-morpholin-4-yl)-ethoxy]-7-[(R)-(tetrahydrofuran-2-yl)methoxy]-chinazolin, 4-[(3-Chlor-4-fluor-phenyl)amino]-7-[2-(2,2-dimethyl-6-oxo-morpholin-4-yl)-ethoxy]-6-[(S)-(tetrahydrofuran-2-yl)methoxy]-chinazolin, 4-[(3-Chlor-4-fluor-phenyl)amino]-6-{2-[4-(2-oxo-morpholin-4-yl)-piperidin-1-yl]-ethoxy}-7-methoxy-chinazolin, 4-[(3-Chlor-4-fluor-phenyl)amino]-6-[1-(tert.-butyloxycarbonyl)-piperidin-4-yloxy]-7-methoxy-chinazolin, 4-[(3-Chlor-4-fluor-phenyl)amino]-6-(trans-4-amino-cyclohexan-1-yloxy)-7-methoxy-chinazolin, 4-[(3-Chlor-4-fluorphenyl)amino]-6-(trans-4-methansulfonylamino-cyclohexan-1-yloxy)-7-methoxy-chinazolin, 4-[(3-Chlor-4-fluor-phenyl)amino]-6-(tetrahydropyran-3-yloxy)-7-methoxy-chinazolin, 4-[(3-Chlor-4-fluor-phenyl)amino]-6-(1-methyl-piperidin-4-yloxy)-7-methoxy-chinazolin, 4-[(3-Chlor-4-fluor-phenyl)amino]-6-{1-[(morpholin-4-yl)carbonyl]-piperidin-4-yloxy}-7-methoxy-chinazolin, 4-[(3-Chlor-4-fluor-phenyl)amino]-6-{1-[(methoxymethyl)carbonyl]-piperidin-4-yl-oxy}-7-methoxy-chinazolin, 4-[(3-Chlor-4-fluor-phenyl)amino]-6-(piperidin-3-yloxy)-7-methoxy-chinazolin, 4-[(3-Chlor-4-fluor-phenyl)amino]-6-[1-(2-acetylamino-ethyl)-piperidin-4-yloxy]-7-methoxy-chinazolin, 4-[(3-Chlor-4-fluor-phenyl)amino]-6-(tetrahydropyran-4-yloxy)-7-ethoxy-chinazolin, 4-[(3-Chlor-4-fluor-phenyl)amino]-6-((S)-tetrahydrofuran-3-yloxy)-7-hydroxy-chinazolin, 4-[(3-Chlor-4-fluor-phenyl)amino]-6-(tetrahydropyran-4-yloxy)-7-(2-methoxy-ethoxy)-chinazolin, 4-[(3-Chlor-4-fluor-phenyl)amino]-6-{trans-4-[(dimethylamino)sulfonylamino]-cyclohexan-1-yloxy}-7-methoxy-chinazolin, 4-[(3-Chlor-4-fluor-phenyl)amino]-6-{trans-4-[(morpholin-4-yl)carbonylamino]-cyclohexan-1-yloxy}-7-methoxy-chinazolin, 4-[(3-Chlor-4-fluor-phenyl)amino]-6-{trans-4-[(morpholin-4-yl)sulfonylamino]-cyclohexan-1-yloxy}-7-methoxy-chinazolin, 4-[(3-Chlor-4-fluor-phenyl)amino]-6-(tetrahydropyran-4-yloxy)-7-(2-acetylamino-ethoxy)-chinazolin, 4-[(3-Chlor-4-fluor-phenyl)amino]-6-(tetrahydropyran-4-yloxy)-7-(2-methansulfonylamino-ethoxy)-chinazolin, 4-[(3-Chlor-4-fluor-phenyl)amino]-6-{1-[(piperidin-1-yl)carbonyl]-piperidin-4-yloxy}-7-methoxy-chinazolin, 4-[(3-Chlor-4-fluor-phenyl)amino]-6-(1-aminocarbonylmethyl-piperidin-4-yloxy)-7-methoxy-chinazolin, 4-[(3-Chlor-4-fluor-phenyl)amino]-6-(cis-4-{N-[(tetrahydropyran-4-yl)carbonyl]-N-methyl-amino}-cyclohexan-1-yloxy)-7-methoxy-chinazolin, 4-[(3-Chlor-4-fluor-phenyl)amino]-6-(cis-4-{N-[(morpholin-4-yl)carbonyl]-N-methyl-amino}-cydohexan-1-yloxy)-7-methoxy-chinazolin, 4-[(3-Chlor-4-fluor-phenyl)amino]-6-(cis-4-{N-[(morpholin-4-yl)sulfonyl]-N-methyl-amino}-cyclohexan-1-yloxy)-7-methoxy-chinazolin, 4-[(3-Chlor-4-fluor-phenyl)amino]-6-(trans-4-ethansulfonylamino-cyclohexan-1-yloxy)-7-methoxy-chinazolin, 4-[(3-Chlor-4-fluor-phenyl)amino]-6-(1-methansulfonyl-piperidin-4-yloxy)-7-ethoxy-chinazolin, 4-[(3-Chlor-4-fluor-phenyl)amino]-6-(1-methansulfonyl-piperidin-4-yloxy)-7-(2-methoxy-ethoxy)-chinazolin, 4-[(3-Chlor-4-fluor-phenyl)amino]-6-[1-(2-methoxy-acetyl)-piperidin-4-yloxy]-7-(2-methoxy-ethoxy)-chinazolin, 4-[(3-Chlor-4-fluor-phenyl)amino]-6-(cis-4-acetylamino-cyclohexan-1-yloxy)-7-methoxy-chinazolirt, 4-[(3-Ethinyl-phenyl)amino]-6-[1-(tert.-butyloxycarbonyl)-piperidin-4-yloxy]-7-methoxy-chinazolin, 4-[(3-Ethinyl-phenyl)amino]-6-(tetrahydropyran-4-yloxy]-7-methoxy-chinazolin, 4-[(3-Chlor-4-fluor-phenyl)amino]-6-(cis-4-{N-[(piperidin-1-yl)carbonyl]-N-methyl-amino}-cyclohexan-1-yloxy)-7-methoxy-chinazolin, 4-[(3-Chlor-4-fluor-phenyl)amino]-6-(cis-4-{N-[(4-methyl-piperazin-1-yl)carbonyl]-N-methyl-amino}-cyclohexan-1-yloxy)-7-methoxy-chinazolin, 4-[(3-Chlor-4-fluor-phenyl)amino]-6-{cis-4-[(morpholin-4-yl)carbonylamino]-cyclohexan-1-yloxy}-7-methoxy-chinazolin, 4-[(3-Chlor-4-fluor-phenyl)amino]-6-{1-[2-(2-oxopyrrolidin-1-yl)ethyl]-piperidin-4-yloxy}-7-methoxy-chinazolin, 4-[(3-Chlor-4-fluo-phenyl)amino]-6-{1-[(morpholin-4-yl)carbonyl]-piperidin-4-yloxy}-7-(2-methoxy-ethoxy)-chinazolin, 4-[(3-Ethinyl-phenyl)amino]-6-(1-acetyl-piperidin-4-yloxy)-7-methoxy-chinazolin, 4-[(3-Ethinyl-phenyl)amino]-6-(1-methyl-piperidin-4-yloxy)-7-methoxy-chinazolin, 4-[(3-Ethinyl-phenyl)amino]-6-(1-methansulfonyl-piperidin-4-yloxy)-7-methoxy-chinazolin, 4-[(3-Chlor-4-fluor-phenyl)amino]-6-(1-methyl-piperidin-4-yloxy)-7(2-methoxy-ethoxy)-chinazolin, 4-[(3-Chlor-4-fluor-phenyl)amino]-6-(1-isopropyloxycarbonyl-piperidin-4-yloxy)-7-methoxy-chinazolin, 4-[(3-Chlor-4-fluor-phenyl)amino]-6-(cis-4-methylamino-cyclohexan-1-yloxy)-7-methoxy-chinazolin, 4-[(3-Chlor-4-fluor-phenyl)amino]-6-{cis-4-[N-(2-methoxy-acetyl)-N-methyl-amino]-cyclohexan-1-yloxy}-7-methoxy-chinazolin, 4-[(3-Ethinyl-phenyl)amino]-6-(piperidin-4-yloxy)-7-methoxy-chinazolin, 4-[(3-Ethinyl-phenyl)amino]-6-[1-(2-methoxy-acetyl)-piperidin-4-yloxy]-7-methoxy-chinazolin, 4-[(3-Ethinyl-phenyl)amino]-6-{1-[(morpholin-4-yl)carbonyl]-piperidin-4-yloxy}-7-methoxy-chinazolin, 4-[(3-Chlor-4-fluor-phenyl)amino]-6-{1-[(cis-2,6-dimethyl-morpholin-4-yl)carbonyl]-piperidin-4-yloxy}-7-methoxy-chinazolin, 4-[(3-Chlor-4-fluor-phenyl)amino]-6-{1-[(2-methyl-morpholin-4-yl)carbonyl]-piperidin-4-yloxy}-7-methoxy-chinazolin, 4-[(3-Chlor-4-fluor-phenyl)amino]-6-{1-[(S,S)-(2-oxa-5-aza-bicyclo[2.2.1]hept-5-yl)carbonyl]-piperidin-4-yloxy}-7-methoxy-chinazolin, 4-[(3-Chlor-4-fluor-phenyl)amino]-6-{1-[(N-methyl-N-2-methoxyethyl-amino)carbonyl]-piperidin-4-yloxy}-7-methoxy-chinazolin, 4-[(3-Chlor-4-fluor-phenyl)amino]-6-(1-ethyl-piperidin-4-yloxy)-7-methoxy-chinazolin, 4-[(3-Chlor-4-fluor-phenyl)amino]-6-{1-[(2-methoxyethyl)carbonyl]-piperidin-4-yloxy]-7-methoxy-chinazolin, 4-[(3-Chlor-4-fluor-phenyl)amino]-6-{1-[(3-methoxypropyl-amino)-carbonyl]-piperidin-4-yloxy}-7-methoxy-chinazolin, 4-[(3-Chlor-4-fluor-phenyl)amino]-6-[cis-4-(N-methansulfonyl-N-methyl-amino)-cyclohexan-1-yloxy]-7-methoxy-chinazolin, 4-[(3-Chlor-4-fluor-phenyl)amino]-6-[cis-4-(N-acetyl-N-methyl-amino)-cyclohexan-1-yloxy]-7-methoxy-chinazolin, 4-[(3-Chlor-4-fluor-phenyl)amino]-6-(trans-4-methylamino-cyclohexan-1-yloxy)-7-methoxy-chinazolin, 4-[(3-Chlor-4-fluor-phenyl)amino]-6-[trans-4-(N-methansulfonyl-N-methyl-amino)-cyclohexan-1-yloxy]-7-methoxy-chinazolin, 4-[(3-Chlor-4-fluor-phenyl)amino]-6-(trans-4-dimethylamino-cyclohexan-1-yloxy)-7-methoxy-chinazolin, 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazolin, 4-[(3-Chlor-4-fluor-phenyl)amino]-6-[2-(2,2-dimethyl-6-oxo-morpholin-4-yl)-ethoxy]-7-[(S)-(tetrahydrofuran-2-yl)methoxy]-chinazolin, 4-[(3-Chlor-4-fluor-phenyl)amino]-6-(1-methansulfonyl-piperidin-4-yloxy)-7-methoxy-chinazolin, 4-[(3-Chlor-4-fluor-phenyl)amino]-6-(1-cyano-piperidin-4-yloxy)-7-methoxy-chinazolin, und Cetuximab, gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

Besonders bevorzugt gelangen im Rahmen der vorliegenden Erfindung diejenigen EGFR-Hemmer zur Anwendung, die ausgewählt sind aus der Gruppe bestehend aus 4-[(3-Chlor-4-fluorphenyl)amino]-6-{[4-(morpholin-4-yl)-1-oxo-2-buten-1-yl]amino}-7-cyclopropylmethoxy-chinazolin, 4-[(R)-(1-Phenyl-ethyl)amino]-6-{[4-(morpholin-4-yl)-1-oxo-2-buten-1-yl]amino}-7-cyclopentyloxy-chinazolin, 4-[(3-Chlor-4-fluor-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin, 4-[(3-Chlor-4-fluor-phenyl)amino]-6-[2-((S)-6-methyl-2-oxo-morpholin-4-yl)-ethoxy]-7-methoxy-chinazolin, 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin, 4-[(R)-(1-Phenyl-ethyl)amino]-6-({4-[N-(tetrahydropyran-4-yl)-N-methyl-amino]-1-oxo-2-buten-1-yl}amino)-7-cyclopropylmethoxy-chinazolin, 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin 4-[(3-Chlor-4-fluorphenyl)amino]-6-{[4-(N,N-dimethylamino)-1-oxo-2-buten-1-yl]amino}-7-[(R)-(tetrahydrofuran-2-yl)methoxy]-chinazolin, 4-[(3-Ethinyl-phenyl)amino]-6,7-bis-(2-methoxy-ethoxy)-chinazolin, 4-[(R)-(1-Phenyl-ethyl)amino]-6-(4-hydroxy-phenyl)-7H-pyrrolo[2,3-d]pyrimidin, 3-Cyano-4-[(3-chlor-4-fluorphenyl)amino]-6-{[4-(N,N-dimethylamino)-1-oxo-2-buten-1-yl]amino}-7-ethoxy-chinolin, 4-[(R)-(1-Phenyl-ethyl)amino]-6-{[4-((R)-6-methyl-2-oxo-morpholin-4-yl)-1-oxo-2-buten-1-yl]amino}-7-methoxy-chinazolin, 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin, 4-[(3-Ethinyl-phenyl)amino]-6-{[4-(5,5-dimethyl-2-oxo-morpholin-4-yl)-1-oxo-2-buten-1-yl]amino}-chinazolin, 4-[(3-Chlor-4-fluor-phenyl)amino]-6-{2-[4-(2-oxo-morpholin-4-yl)-piperidin-1-yl]-ethoxy}-7-methoxy-chinazolin, 4-[(3-Chlor-4-fluorphenyl)amino]-6-(trans-4-amino-cyclohexan-1-yloxy)-7-methoxy-chinazolin, 4-[(3-Chlor-4-fluor-phenyl)amino]-6-(trans-4-methansulfonylamino-cyclohexan-1-yloxy)-7-methoxy-chinazolin, 4-[(3-Chlor-4-fluor-phenyl)amino]-6-(tetrahydropyran-3-yloxy-7-methoxy-chinazolin, 4-[(3-Chlor-4-fluor-phenyl)amino]-6-{1-[(morpholin-4-yl)carbonyl]-piperidin-4-yl-oxy}-7-methoxy-chinazolin, 4-[(3-Chlor-4-fluor-phenyl)amino]-6-(piperidin-3-yloxy)-7-methoxy-chinazolin, 4-[(3-Chlor-4-fluor-phenyl)amino]-6-[1-(2-acetylamino-ethyl)-piperidin-4-yloxy]-7-methoxy-chinazolin, 4-[(3-Chlor-4-fluor-phenyl)amino]-6-(tetrahydropyran-4-yloxy)-7-ethoxy-chinazolin, 4-[(3-Chlor4-fluor-phenyl)amino]-6-{trans-4-[(morpholin-4-yl)carbonylamino]-cyclohexan-1-yloxy}-7-methoxy-chinazolin, 4-[(3-Chlor-4-fluor-phenyl)amino]-6-{1-[(piperidin-1-yl)carbonyl]-piperidin-4-yloxy}-7-methoxy-chinazolin, 4-[(3-Chlor-4-fluor-phenyl)amino]-6-(cis-4-{N-[(morpholin-4-yl)carbonyl]-N-methyl-amino}-cyclohexan-1-yloxy)-7-methoxy-chinazolin, 4-[(3-Chlor-4-fluor-phenyl)amino]-6-(trans-4-ethansulfonylamino-cyclohexan-1-yloxy)-7-methoxy-chinazolin, 4-[(3-Chlor-4-fluor-phenyl)amino]-6-(1-methansulfonyl-piperidin-4-yloxy)-7-(2-methoxy-ethoxy)-chinazolin, 4-[(3-Chlor-4-fluor-phenyl)amino]-6-[1-(2-methoxy-acetyl)-piperidin-4-yloxy]-7-(2-methoxy-ethoxy)-chinazolin, 4-[(3-Ethinyl-phenyl)amino]-6-(tetrahydropyran-4-yloxy]-7-methoxy-chinazolin, 4-[(3-Chlor-4-fluor-phenyl)amino]-6-(cis-4-{N-[(piperidin-1-yl)carbonyl]-N-methyl-amino}-cyclohexan-1-yloxy)-7-methoxy-chinazolin, 4-[(3-Chlor-4-fluor-phenyl)amino]-6-{cis-4-[(morpholin-4-yl)carbonylamino]-cyclohexan-1-yloxy}-7-methoxy-chinazolin, 4-[(3-Chlor-4-fluor-phenyl)amino]-6-{1-[2-(2-oxopyrrolidin-1-yl)ethyl]-piperidin-4-yloxy}-7-methoxy-chinazolin, 4-[(3-Ethinyl-phenyl)amino]-6-(1-acetyl-piperidin-4-yloxy)-7-methoxy-chinazolin, 4-[(3-Ethinyl-phenyl)amino]-6-(1-methyl-piperidin-4-yloxy)-7-methoxy-chinazolin, 4-[(3-Ethinyl-phenyl)amino]-6-(1-methansulfonyl-piperidin-4-yloxy)-7-methoxy-chinazolin, 4-[(3-Chlor-4-fluor-phenyl)amino]-6-(1-methyl-piperidin-4-yloxy)-7(2-methoxy-ethoxy)-chinazolin, 4-[(3-Ethinyl-phenyl)amino]-6-{1-[(morpholin-4-yl)carbonyl]-piperidin-4-yloxy}-7-methoxy-chinazolin, 4-[(3-Chlor-4-fluor-phenyl)amino]-6-{1-[(N-methyl-N-2-methoxyethyl-amino)carbonyl]-piperidin-4-yloxy}-7-methoxy-chinazolin, 4-[(3-Chlor-4-fluor-phenyl)amino]-6-(1-ethyl-piperidin-4-yloxy)-7-methoxy-chinazolin, 4-[(3-Chlor-4-fluor-phenyl)amino]-6-[cis-4-(N-methansulfonyl-N-methyl-amino)-cyclohexan-1-yloxy]-7-methoxy-chinazolin, 4-[(3-Chlor-4-fluor-phenyl)amino]-6-[cis-4-(N-acetyl-N-methyl-amino)cyclohexan-1-yloxy]-7-methoxy-chinazolin, 4-[(3-Chlor-4-fluor-phenyl)amino]-6-(trans-4-methylamino-cyclohexan-1-yloxy)-7-methoxy-chinazolin, 4-[(3-Chlor-4-fluor-phenyl)amino]-6-[trans-4-(N-methansulfonyl-N-methyl-amino)-cyclohexan-1-yloxy]-7-methoxy-chinazolin, 4-[(3-Chlor-4-fluor-phenyl)amino]-6-(trans-4-dimethylamino-cyclohexan-1-yloxy)-7-methoxy-chinazolin, 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazolin, 4-[(3-Chlor-4-fluor-phenyl)amino]-6-[2-(2,2-dimethyl-6-oxo-morpholin-4-yl)-ethoxy]-7-[(S)-(tetrahydrofuran-2-yl)methoxy]-chinazolin, 4-[(3-Chlor-4-fluor-phenyl)amino]-6-(1-methansulfonyl-piperidin-4-yloxy)-7-methoxy-chinazolin, 4-[(3-Chlor-4-fluor-phenyl)amino]-6-(1-cyano-piperidin-4-yloxy)-7-methoxy-chinazolin, und 4-[(3-Chlor-4-fluor-phenyl)amino]-6-{1-[(2-methoxyethyl)carbonyl]-piperidin-4-yloxy}-7-methoxy-chinazolin, gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

Erfindungsgemäß besonders bevorzugt sind als EGFR-Hemmer diejenigen Verbindungen, die ausgewählt sind aus der Gruppe bestehend aus 4-[(3-Chfor-4-fluorphenyl)amino]-6-{[4-(morpholin-4-yl)-1-oxo-2-buten-1-yl]amino}-7-cyclopropylmethoxy-chinazolin, 4-[(3-Chlor-4-fluor-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin, 4-[(3-Chlor-4-fluor-phenyl)amino]-6-[2-((S)-6-methyl-2-oxo-morpholin-4-yl)-ethoxy]-7-methoxy-chinazolin, 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin, 4-[(3-Ethinyl-phenyl)amino]-6,7-bis-(2-methoxy-ethoxy)-chinazolin, 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin, 4-[(3-Ethinyl-phenyl)amino]-6-{[4-(5,5-dimethyl-2-oxo-Morpholin-4-yl)-1-oxo-2-buten-1-yl]amino}-chinazolin, 4-[(3-Chlor-4-fluor-phenyl)amino]-6-(trans-4-methansulfonylamino-cyclohexan-1-yloxy)-7-methoxy-chinazolin, 4-[(3-Chlor-4-fluor-phenyl)amino]-6-(tetrahydropyran-3-yloxy)-7-methoxy-chinazolin, 4-[(3-Chlor-4-fluor-phenyl)amino]-6-{1-[(morpholin-4-yl)carbonyl]-piperidin-4-yloxy}-7-methoxy-chinazolin, 4-[(3-Chlor-4-fluor-phenyl)amino]-6-{1-[2-(2-oxopyrrolidin-1-yl)ethyl]-piperidin-4-yloxy}-7-methoxy-chinazolin, 4-[(3-Ethinyl-phenyl)amino]-6-(1-acetyl-piperidin-4-yloxy)-7-methoxy-chinazolin, 4-[(3-Ethinyl-phenyl)amino]-6-(1-methyl-piperidin-4-yloxy)-7-methoxy-chinazolin, 4-[(3-Ethinyl-phenyl)amino]-6-(1-methansulfonyl-piperidin-4-yloxy)-7-methoxy-chinazolin, 4-[(3-Ethinyl-phenyl)amino]-6-{1-[(morpholin-4-yl)carbonyl]-piperidin-4-yloxy}-7-methoxy-chinazolin, 4-[(3-Chlor-4-fluor-phenyl)amino]-6-{1-[(2-methoxyethyl)carbonyl]-piperidin-4-yloxy}-7-methoxy-chinazolin, 4-[(3-Chlor-4-fluor-phenyl)amino]-6-[cis-4-(N-methansulfonyl-N-methyl-amino)-cyclohexan-1-yloxy]-7-methoxy-chinazolin, 4-[(3-Chlor-4-fluor-phenyl)amino]-6-[cis-4-(N-acetyl-N-methyl-amino)-cyclohexan-1-yloxy]-7-methoxy-chinazolin, 4-[(3-Chlor-4-fluor-phenyl)amino]-6-(trans-4-methylamino-cyclohexan-1-yloxy)-7-methoxy-chinazolin, 4-[(3-Chlor-4-fluor-phenyl)amino]-6-[trans-4-(N-methansulfonyl-N-methyl-amino)-cyclohexan-1-yloxy]-7-methoxy-chinazolin, 4-[(3-Chlor-4-fluor-phenyl)amino]-6-(trans-4-dimethylamino-cyclohexan-1-yloxy)-7-methoxy-chinazolin, 4-[(3-Chlor-4-fluor-phenyl)amino]-6-(trans-4-{N-[(morpholin-4-yl)carbonyl]-N-methyl-amino}cyclohexan-1-yloxy)-7-methoxy-chinazolin, 4-[(3-Chtor-4-fluor-phenyl)amino]-6-[2-(2,2-dimethyl-6-oxo-morpholin-4-yl)-ethoxy]-7-[(S)-(tetrahydrofuran-2-yl)methoxy]-chinazolin, 4-[(3-Chlor-4-fluor-phenyl)amino]-6-(1-methansulfonyl-piperidin-4-yloxy)-7-methoxy-chinazolin und 4-[(3-Chlor-4-fluor-phenyl)amino]-6-(1-cyano-piperidin-4-yloxy)-7-methoxy-chinazolin gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

Unter Säureadditionssalzen mit pharmakologisch verträglichen Säuren zu deren Bildung die EGFR-Hemmer gegebenenfalls in der Lage sind, werden beispielsweise Salze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrobenzoat, Hydrocitrat, Hydrofumarat, Hydrotartrat Hydrooxalat, Hydrosuccinat, Hydrobenzoat und Hydro-p-toluolsulfonat, bevorzugt Hydrochlorid, Hydrobromid, Hydrosulfat, Hydrophosphat, Hydrofumarat und Hydromethansulfonat verstanden.

Als Dopamin-Agonisten gelangen hierbei vorzugsweise Verbindungen zur Anwendung, die ausgewählt sind aus der Gruppe bestehend aus Bromocriptin, Cabergolin, Alpha-Dihydroergocryptin, Lisurid, Pergolid, Pramipexol, Roxindol, Ropinirol, Talipexol, Tergurid und Viozan. Eine Bezugnahme auf die vorstehend genannten Dopamin-Agonisten schließt im Rahmen der vorliegenden Erfindung eine Bezugnahme auf deren gegebenenfalls existierende pharmakologisch verträgliche Säureadditionssalze und gegebenenfalls deren Hydrate ein. Unter den physiologisch verträglichen Säureadditionssalzen, die von den vorstehend genannten Dopamin-Agonisten gebildet werden können, werden beilspielsweise pharmazeutisch verträgliche Salze verstanden, die ausgewählt aus den Salzen der Salzsäure, Bromwasserstoffsäure, Schwefelsäure, Phosphorsäure, Methansulfonsäure, Essigsäure, Fumarsäure, Bernsteinsäure, Milchsäure, Zitronensäure, Weinsäure und Maleinsäure sind.

Als H1-Antihistaminika gelangen hierbei vorzugsweise Verbindungen zur Anwendung, die ausgewählt sind aus der Gruppe bestehend aus Epinastin, Cetirizin, Azelastin, Fexofenadin, Levocabastin, Loratadin, Mizolastin, Ketotifen, Emedastin, Dimetinden, Clemastin, Bamipin, Cexchlorpheniramin, Pheniramin, Doxylamin, Chlorphenoxamin, Dimenhydrinat, Diphenhydramin, Promethazin, Ebastin, Desloratidin und Meclozin. Eine Bezugnahme auf die vorstehend genannten H1-Antihistaminika schließt im Rahmen der vorliegenden Erfindung eine Bezugnahme auf deren gegebenenfalls existierende pharmakologisch verträgliche Säureadditionssalze ein.

Als PAF-Antagonisten gelangen hierbei vorzugsweise Verbindungen zur Anwendung, die ausgewählt sind aus der Gruppe bestehend aus 4-(2-Chlorphenyl)-9-methyl-2-[3(4-morpholinyl)-3-propanon-1-yl]-6H-thieno-[3,2-f]-[1,2,4]triazolo[4,3-a][1,4]diazepin, 6-(2-Chlorphenyl)-8,9-dihydro-1-methyl-8-[(4-morpholinyl)carbonyl]-4H,7H-cyclo-penta-[4,5]thieno-[3,2-f][1,2,4]triazolo[4,3-a][1,4]diazepin.

Als MRP4-Inhibitoren gelangen hierbei vorzugsweise Verbindungen zur Anwendung, die ausgewählt sind aus der Gruppe bestehend aus N-Acetyl-dinitrophenyl-Cysteine, cGMP, Cholate, Diclofenac, Dehydroepiandrosterone 3-glucuronide, Dehydroepiandrosterone 3-sulphate, Dilazep, Dinitrophenyl-S-glutathione, Estradiol 17-□-glucuronide, Estradiol 3,17-disulphate, Estradiol 3-glucuronide, Estradiol 3-sulphate, Estrone 3-sulphate, Flurbiprofen, Folate, N5-formyl-tetrahydrofolate, Glycocholate, Glycolithocholic acid sulphate, Ibuprofen, Indomethacin, Indaprofen, Ketoprofen, Lithocholic acid sulphate, Methotrexate, MK571 ((*E*)-3-[[[3-[2-(7-Chloro-2-quinolinyl)ethenyl]phenyl]-[[3-dimethylamino)-3-oxopropyl]thio]methyl]thio]-propanoic acid), □-Naphthyl-□-D-glucuronide, Nitrobenzyl mercaptopurine riboside, Probenecid, PSC833, Sildenafil, Sulfinpyrazone, Taurochenodeoxycholate, Taurocholate, Taurodeoxycholate, Taurolithocholate, Taurolithocholic acid sulphate, Topotecan, Trequinsin und Zaprinast, Dipyridamol, gegebenenfalls in Form ihrer Racemate, Enantiomere, Diastereomere und ihrer pharmakologisch verträglichen Säure-Additionssalze und Hydrate.

Vorzugsweise bezieht sich die Erfindung auf die Verwendung von MRP4-Inhibitoren zur Herstellung einer pharmazeutischen Zusammensetzung zur Behandlung von Atemwegserkrankungen enthaltend die erfindungsgemäßen PDE4B-Inhibitoren und MRP4-Inhibitoren, wobei die MRP4-Inhibitoren vorzugsweise ausgewählt sind aus der Gruppe bestehend aus N-Acetyl-dinitrophenyl-Cysteine, Dehydroepiandrosterone 3-sulphate, Dilazep, Dinitrophenyl-S-glutathione, Estradiol 3,17-disulphate, Flurbiprofen, Glycocholate, Glycolithocholic acid sulphate, Ibuprofen, Indomethacin, Indoprofen, Lithocholic acid sulphate, MK571, PSC833, Sildenafil,
Taurochenodeoxycholate, Taurocholate, Taurolithocholate, Taurolithocholic acid sulphate, Trequinsin und Zaprinast, Dipyridamol, gegebenenfalls in Form ihrer Racemate, Enantiomere, Diastereomere und ihrer pharmakologisch verträglichen Säure-Additionssalze und Hydrate.

Stärker bevorzugt bezieht sich die Erfindung auf die Verwendung von MRP4-Inhibitoren zur Herstellung einer pharmazeutischen Zusammensetzung zur Behandlung von Atemwegserkrankungen enthaltend die erfindungsgemäßen PDE4-Inhibitoren und MRP4-Inhibitoren, wobei die MRP4-Inhibitoren vorzugsweise ausgewählt sind aus der Gruppe bestehend aus
Dehydroepiandrosterone 3-sulphate, Estradiol 3,17-disulphate, Flurbiprofen, Indomethacin, Indoprofen, MK571, Taurocholate, gegebenenfalls in Form ihrer Racemate, Enantiomere, Diastereomere und ihrer pharmakologisch verträglichen Säure-Additionssalze und Hydrate. Die Trennung von Enantiomeren aus den Racematen kann durch bekannte Verfahren nach dem Stand der Technik durchgeführt werden (z.B. durch Chromatographie an chiralen Phasen etc.).

Mit Säure-Additionssalzen mit pharmakologisch verträglichen Säuren sind z.B. Salze ausgewählt aus der Gruppe bestehend aus Hydrochloriden, Hydrobromiden, Hydroiodiden, Hydrosulphaten, Hydrophosphaten, Hydromethanesulphonaten, Hydronitraten, Hydromaleaten, Hydroacetaten, Hydrobenzoaten, Hydrocitraten, Hydrofumaraten, Hydrotartraten, Hydrooxalaten, Hydrosuccinaten, Hydrobenzoaten and Hydro-*p-*toluenesulphonaten, vorzugsweise Hydrochloride, Hydrobromide, Hydrosulphate, Hydrophosphate, Hydrofumarate and Hydromethanesulphonate gemeint.

Ein weiterer Gegenstand der Erfindung sind pharmazeutische Zubereitungen, die Dreifachkombinationen von den erfindungsgemäßen PDE4B-Inhibitoren, von MRP4-Inhibitoren und einer weiteren aktiven Substanz wie z.B. einem Anticholinergikum, einem Steroid, einem LTD4-Antagonist oder einem Betamimetikum enthalten, sowie deren Herstellung und deren Verwendung zur Behandlung von Atemwegserkrankungen.

Als iNOS-Inhibitoren gelangen hierbei vorzugsweise Verbindungen zur Anwendung, die ausgewählt sind aus der Gruppe bestehend aus: S-(2-Aminoethyl)isothioharnstoff, Aminoguanidin, 2-Aminomethylpyridin, AMT, L-Canavanin, 2-Iminopiperidin, S-Isopropylisothiohamstoff, S-Methylisothiohamstoff, S-Ethylisothiohamsfoff, S-Methyltiocitrullin, S-Ethylthiocitruilin, L-NA (N^{ω}-Nitro-L-arginin), L-NAME (N^{ω}-Nitro-L-argininmethylester), L-NMMA (N^{G}-Monomethyl-L-arginin), L-NIO (N^{ω}-Iminoethyl-L-omithin), L-NIL (N^{ω}-Iminoethyl-lysin), (S)-6-Acetirntdearnino-2-amino-hexanoic acid (1*H*-tetrazol-5-yl)-amid (SC-51) (J. Med. Chem. 2002, 45, 1686-1689), 1400W, (S)-4-(2-Acetimidoylamino-ethylsulfanyl)-2-amino-buttersäure (GW274150) (Bioorg. Med. Chem. Lett. 2000, 10, 597-600), 2-[2-(4-Methoxy-pyridin-2-yl)-ethyl]-3*H*-imidazo[4,5-*b*]pyridin (BYK191023) (Mol. Pharmacol. 2006, 69, 328-337), 2-((R)-3-Amino-1-phenyl-propoxy)-4-chlor-5-fluorbenzonitril (WO 01/62704), 2-((1R,3S)-3-Amino-4-hydroxy-1-thiazol-5-yl-butylsulfanyl}-6-trifluoromethyl-nicotinonitril (WO 2004/041794), 2-((1R,3S)-3-Amino-4-hydroxy-1-thiazol-5-yl-butylsutfanyl)-4-chlor-benzonitril (WO 2004/041794), 2-((1R,35)-3-Amino-4-hydroxy-1-thiazol-5-yl-butylsulfanyl)-5-chlor-benzonitril (WO 2004/041794), (2S,4R)-2-Amino-4-(2-chlor-5-trifluoromethyl-phenylsulfanyl)-4-thiazol-5-yl-butan-1-ol (WO 2004/041794), 2-((1R,3S)-3-Amino-4-hydroxy-1-thiazol-5-yl-butylsulfanyl)-5-chlor-nicotinonitril (WO 2004/041794), 4-((S)-3-Amino-4-hydroxy-1-phenyl-butylsulfanyl)-6-methoxy-nicotinonitril (WO 02/090332), substitulerte 3-Phenyl-3,4-dihydro-1-isoquinolinamin wie z.B. AR-C102222 (J. Med. Chem. 2003, 46, 913-916), (1S,5S,6R)-7-Chlor-5-methyl-2-aza-bicyclo[4,1,0]hept-2-en-3-ylamin (ONO-1 714) (Biochem. Biophys. Res. Commun. 2000, 270, 663-667), (4R,5R)-5-Ethyl-4-methyl-thiazolidin-2-ylideneamin (Bioorg. Med. Chem. 2004, 12, 4101), (4R,5R)-5-Ethyl-4-methyl-selenazolidin-2-ylideneamin *(*Bloorg. Med. Chem. Lett. 2005, 15,1361), 4-Aminotetrahydrobiopterin (Curr. Drug Metabol. 2002, 3, 119-121), (E)-3-(4-Chlor-phenyl)-*N-*(1-{2-oxo-2-[4-(6-trifluormethyl-pyrimidin-4-yloxy)-piperidin-1-yl]-ethylcarbamoyl}-2-pyridin-2-yl-ethyl)-acrylamid (FR260330) (Eur. J. Pharmacol. 2005, 509, 71-76), 3-(2,4-Difluor-phenyl)-6-[2-(4-imidazol-1-ylmethyl-phenoxy)-ethoxy]-2-phenyl-pyridin (PPA250) (J. Pharmacol. Exp. Ther. 2002, 303, 52-57), 3-{[(Benzo[1,3]dioxol-5-ylmethyl)-carbamoyl]-methyl}-4-(2-imidazol-1-yl-pyrimidin-4-yl)-piperazin-1-carbonsäuremethylester (BBS-1) (Drugs Future 2004, 29, 45-52), (R)-1-(2-Imidazol-1-yl-6-methyl-pyrimidin-4-yl)-pyrrolidin-2-carbonsäure (2-benzo[1,3]dioxol-5-yl-ethyl)-amid (BBS-2) (Drugs Future 2004, 29, 45-52) und deren pharmazeutischen Salze, Prodrugs oder Solvate.

Als iNOS-Inhibitoren im Rahmen der vorliegenden Erfindung können weiterhin antisense-Oligonucleotide, insbesondere solche antisense-Oligonucleotide, die iNOS-kodierende Nukleinsäuren binden, eingesetzt werden. Z.B. werden in WO 01/52902 antisense-Oligonucleotide, insbesondere antisense-Oligonucleotide, die iNOS kodierende Nukleinsäuren binden, zur Modulierung der Expression von iNOS beschrieben. Solche iNOS-antisense-Oligonucleotide wie insbesondere in WO 01/52902 beschrieben können daher auch aufgrund ihrer ähnlichen Wirkung wie die iNOS-Inhibitoren mit den PDE4-Inhibitoren der vorliegenden Erfindung kombiniert werden.

Als SYK-Inhibitoren gelangen hierbei vorzugsweise Verbindungen zur Anwendung, die ausgewählt sind aus der Gruppe bestehend aus:
2-[(2-aminoethyl)amino]-4-[(3-bromophenyl)amino]-5-Pyrimidinecarboxamide;
2-{[7-(3,4-dimethoxyphenyl)imidazo[1,2-c]pyrimidin-5-yl]amino}-3-Pyridinecarboxamide;
6-[[5-fluoro-2-(3,4,5-trimethoxyphenyl)amino]-4-pyrimidinyl]amino]-2,2-dimethyl-2H-Pyrido[3,2-b]-1,4-oxazin-3(4H)-one;
N-[3-bromo-7-(4-methoxyphenyl)-1,6-naphthyridin-5-yl]-1,3-Propanediamine 7-(4-methoxyphenyl)-N-methyl-1,6-Naphthyridin-5-amine;
N-[7-(4-methoxyphenyl)-1,6-naphthyridin-5-yl]-1,3-Propanediamine:
N-[7-(2-thienyl)-1,6-naphthyridin-5-yl-1,3-Propanediamine;
N-[7-[4-(dimethylamino)phenyl]-1,6-naphthyridin-5-yl]-1,2-Ethanediamine;
N-[7-(4-methoxyphenyl)-2-(trifluoromethyl)-1,6-naphthyridin-5-yl]-1,3-Propanediamine;
N-[7-(4-methoxyphenyl)-3-phenyl-1,8-naphthyridin-5-yl]-1,3-Propanediamine;
N-(7-phenyl-1,6-naphthyridin-5-yl)-1,3-Propanediamine;
N-[7-(3-fluorophenyl)-1,6-naphthyridin-5-yl]-1,3-Propanediamine;
N-[7-(3-chlorophenyl)-1,6-naphthyridin-5-yl]-1,3-Propanediamine;
N-[7-[3-(trifluoromethoxy)phenyl]-1,6-naphthyridin-5yl]-1,3-Propanediamine;
N-[7-(4-fluorophenyl)-1,6-naphthyridin-5-yl]-1,3-Propanediamine;
N-[7-(4-fluorophenyl)-1,6-naphthyridin-5-yl]-1,3-Propanediamine;
N-[7-(4-chlorophenyl)-1,6-naphthyridin-5-yl]-1,3-Propanediamine;
N-[7-(4'-methyl[1,1'-biphenyl]-4-yl)-1,6-naphthyridin-1,3-Propanediamine;
N-[7-[4-(dimethylamino)phenyl]-1,6-naphthyridin-5-yl]-1,3-Propanediamine;
N-[7-[4-(diethylamino)phenyl]-1,6-naphthyridin-5-yl]-1,3-Propanediamine;
N-[7-[4-(4-morpholinyl)phenyl]-1,6-naphthyridin-5-yl]-1,3-Propanediamine;
N-[7-[4-[[2-(dimethylamino)ethyl]methylamino]phenyl]-1,6-naphthyridin-5-yl]-1,3-Propanediamine;
N-[7-(4-bromophenyl)-1,6-naphthyridin-5-yl]-1,3-Propanediamine;
N-[7-(4-methylphenyl)-1,6-naphthyridin-5-yl]-1,3-Propanediamine;
N-[7-[4-(methylthio)phenyl]-1,6-naphthyridin-5-yl]-1,3-Propanediamine;
N-[7-[4-(1-methylethyl)phenyl]-1,6-naphthyridin-5-yl]-1,3-Propanediamine;
7-[4-(dimethylamino)phenyl]-N-methyl-1,6-Naphthyridin-5-amine;
7-[4-(dimethylamino)phenyl]-N,N-dimethyl-1,6-Naphthyridin-5-amine;
N-[7-[4-(dimethylamino)phenyl)-1,6-naphthyridin-5-yl]-1,4-Butanediamine;
N-[7-[4-(dimethylamino)phenyl]-1,6-naphthyridin-5-yl]-1,5-Pentanediamine;
3-[[7-(4-(dimethylamino)phenyl]-1,6-naphthyridin-5-yl]oxy]-1-Propanol;
4-[5-(4-aminobutoxy)-1,6-naphthyridin-7-yl]-N,N-dimethyl-Benzenamine;
4-[[7-[4-(dimethylamino)phenyl]-1,6-naphthyridin-5-yl]amino]-1-Butanol;
N-[7-[4-(dimethylamino)phenyl]-1,6-naphthyridin-5-yl]-N-methyl-1,3-Propanediamine;
N-[7-[4-(dimethylamino)phenyl]-1,6-naphthyridin-5-yl]-N'-methyl-1,3-Propanediamine;
N-[7-[4-(dimethylamino)phenl-1,6-naphthyridin-5-yl]-N,N'-dimethyl-1,3-Propanediamine;
1-amino-3-[[7-[4-(dimethylamino)phenyl]-1,6-naphthyridin-5-yl]amino]-2-Propanol;
N-[7-[4-(dimethylamino)phenyl]-1,6-naphthyridin-5-yl]-2,2-dimethyl-1,3-Propanediamine;
7-[4-(dimethylamino)phenyl]-N-(3-pyridinylmethyl)-1,6-Naphthyridin-5-amine;
N-[(2-aminophenyl)methyl]-7-[4-(dimethylamino)phenyl]-1,6-Naphthyridin-5-amine;
N-[7-[6-(dimethylamino)[1,1'-biphenyl]-3-yl]-1,6-naphthyridin-5-yl]-1,3-Propanediamine ;
N-[7-[3-chloro-4-(diethylamino)phenyl]-1,6-naphthyridin-5-yl]-1,3-Propanediamine;
N-[7-[4-(dimethylamino)-3-methoxyphenyl]-1,6-naphthyridin-5-yl]-1,3-Propanediamine;
N-[7-(4-(diethylamino)phenyl]-3-methyl-1,6-naphthyridin-5-yl)-1,3-Propanediamine;
N-[7-(3'-fluoro[1,1'-biphenyl]-3-yl)-1,6-naphthyridin-5-yl]-1,2-Ethanediamine,
N-[7-(4-methoxyphenyl)-1,6-naphthyridin-5-yl]-1,6-Naphthyridine-1,3-Propanediamine;
N,N'-bis(3-aminopropyl)-7-(4-methoxyphenyl)-2,5-diamine;
N-[7-(4-methoxyphenyl)-2-(phenylmethoxy)-1,6-naphthyridin-5-yl]-1,6-Naphthyridine-1,3-Propanediamine;
N5-(3-aminopropyl)-7-(4-methoxyphenyl)-N2-(phenylmethyl)-2,5-diamine;
N-[7-(2-naphthalenyl)-1,6-naphthyridin-5-yl]-1,3-Propanediamine;
N-[7(2'-fluoro[1,1'-biphenyl]-4-yl)-1,6-naphthyridin-5-yl]-1,3-Propanediamine;
N-[7-(3,4,5-trimethoxyphenyl)-1,6-naphthyridin-5-yl]-1,3-Propanediamine;
N-[7-(3,4-dimethylphenyl)-1,6-naphthyridin-5-yl]-1,3-Propanediamine;
1-amino-3-[[7-(2-naphthalenyl)-1,6-naphthyridin-5-yl]amino]-2-Propanol;
1-amino-3-[[7-(2'-fluoro[1,1'-biphenyl]-4-yl-1,6-naphthyridin-5-yl]amino]-2-Propanol;
1-amino-3-[[7-(4'-methoxy[1,1'-biphenyl]-4-yl)-1,6-naphthyridin-5-yl]amino}-2-Propanol;
1-amino-3-[[7-(3,4,5-trimethoxyphenyl)-1,6-naphthyridin-5-yl]amino]-2-Propanol;
1-amino-3-[[7-(4-bromophenyl)-1,6-naphthyridin-5-yl]amino]-2-Propanol;
N-[7-(4'-methoxy[1,1'-biphenyl]-4-yl)-1,6-naphthyridin-5-yl]-2,2-dimethyl-1,3-Propanediamine;
1-[[7-[4-dimethylamino)phenyl]-1,6-naphthyridin-5-yl]amino]-2-Propanol;
2-[[2-[[7-[4-(dimethylamino)phenyl]-1,6-naphthyridin-5-yl]amino]ethyl]thio]-Ethanol;
7-[4-(dimethylamino)phenyl]-N-(3-methyl-risoxazolyl)-1,6-Naphthyridin-5-amine;
7-[4-(dimethylamino)phenyl]-N-4-pyrimidinyl-1,6-Naphthyridin-5-amine;
N-[7-[4-(dimethylamino)phenyl]-1,6-naphthyridin-5-yl]-1,3-Cyclohexanediamine;
N,N-dimethyl-4-[5-(1-piperazinyl)-1,6-naphthyridin-7-yl)-Benzenamine;
4-[5-(2-methoxyethoxy)-1,6-naphthyridin-7-yl]-N,N-dimethyl-Benzenamine;
1-[7-{4-(dimethylamino)phenyl]-1,6-naphthyridin-5-yl}-4-Piperidinol;
1-[7-{4-(dimethylamino)phenyl]-1,6-naphthyridin-5-yl]-Pyrrolidinol;
7-[4-(dimethylamino)phenyl]-N-(2-furanylmethyl)-1,6-Naphthyridin-5-amine;
7-[4-(dimethylamino)phenyl]-N-[3-(1H-imidazol-1-yl)propyl]-1,6-Naphthyridin-5-amine;
1-[7-[4-dimethylamino)phenyl]1,6-naphthyridin-5-yl]-4-Piperidinecarboxamide;
1-[3-[[7-[4-(dimethylamino)phenyl]-1,6-naphthyridin-5-yl]amino]propyl-2-Pyrrolidinone;
N-[3'-[5-[(3-aminopropyl)amino]-1,6-naphthyridin-7-yl][1,1'-biphenyl]-3-yl]-Acetamide;
N-[7-(4'-fluoro[1,1'-biphenyl]-4-yl)-1,6-naphthyridin-5-yl]-1,3-Propanediamine;
N-[4'-[5-[(3-aminopropyl)amino]-1,6-naphthyridin-7-yl][1,1'-biphenyl]-3-yl]-Acetamide;
N-[7-[4-(1,3-benzodioxol-5-yl)phenyl]-1,6-naphthyridin-5-yl]-1,3-Propanediamine;
N-[7-[4-(2-thienyl)phenyl]-1,6-naphthyridin-5-yl]-1,3-Propanediamine;
N-[7-[4-fluoro-3-(trifluoromethyl)phenyl]-1,6-naphthyridin-5-yl]-1,3-Propanediamine;
N-[7-[4-(3-pyridinyl)phenyl]-1,6-naphthyridin-5-yl]-1,3-Propanediamine;
N-[7-(1,3-benzodioxol-5-yl)-1,6-naphthyridin-5-yl]-1,3-Propanediamine;
N-[7-(6-methoxy-2-naphthalenyl)-1,6-naphthyridin-5-yl]-1,3-Propanediamine;
7-[4-(dimethylamino)phenyl]-N-(4-pyridinylmethyl)-1,6-Naphthyridin-5-amine;
3-[[7-[4-(dimethylamino)phenyl]-1,6-naphthyridin-5-yl]methylamino]-Propanenitrile;
7-[4-(dimethylamino)phenyl]-N-(1-(phenylmethyl)-4-piperidinyl]-1,6-Naphthyridin-5-amine;
N-[7-[4-(dimethylamino)phenyl]-1,6-naphthyridin-5-yl]-1,2-Cyclohexanediamine, (1R,2S)-rel-.
N-[7-[4-(dimethylamino)phenyl]-1,6-naphthyridin-5-yl]-1,2-Benzenedimethanamine;
N-[7-[4-(diethylamino)phenyl]-1,6-naphthyridin-5-yl]-1,4-Butanediamine;
N-[7-[3',5'-bis(trifluoromethyl)[1,1'-biphenyl]-4-yl]1,6-naphthyridin-5-yl]-3-Propanediamine;
N-[7-(3'-methoxy[1,1'-biphenyl]-4-yl)-1,6-naphthyridin-5-yl]-1,3-Propanediamine;
N-[7-(3'-fluoro[1,1'-biphenyl]-4-yl)-1,6-naphthyridin-5-yl]-1,3-Propanediamine;
4-[[7-[4-(dimethylamino)phenyl]-1,6-naphthyridin-5-yl]oxy]-1-Butanol;
N-[7-[4-(dimethylamino)phenyl]-1,6-naphthyridin-5-yl]-1,4-Cyclohexanediamine;
7-[4-(dimethylamino)phenyl]-N-(2,2,6,6-tetramethyl-4-piperidinyl)-1,6-Naphthyridin-5-amine;
N-[7-[3-bromo-4-(dimethylamino)phenyl]-1,6-naphthyridin-5-yl]-1,3-Propanediamine;
N-[7-(1-methyl-1H-indol-5-yl)-1,6-naphthyridin-5-yl)-1,3-Propanediamine;
N-[7-[3-(trifluoromethyl)phenyl]-1,6-naphthyridin-5-yl]-1,3-Propanediamine;
N-[7-[4-(trifluoromethyl)phenyl]-1,6-naphthyridin-5-yl]-1,3-Propanediamine;
N-[7-(3-boromo-4-methoxyphenyl)-1,6-naphthyridin-5-yl]-1,3-Propanediamine;
N-[7-[4-[[3-(dimethylamino)propyl]methylamino]phenyl]-1,6-naphthyridin-5-yl]-1,4-Cyclohexanediamine;
N-[7-[4-[[2-(dimethylamino)ethyl]methylamino]phenyl]-1,6-naphthyridin-5-yl]-1,4-Cyclohexanediamine;
N-[7-[4-(dimethylamino-3-methoxyphenyl]-1,6-naphthyridin-5-yl]-1,4-Cyclohexanediamine;
N-[7-[4-(4-morpholinyl)phenyl]-1,6-naphthyridin-5-yl]-1,4-Cyclohexanediamine;
N-[7-[3-bromo-4-(4-morpholinyl)phenyl]-1,6-naphthyridin-5-yl]-1,4-Cyclohexanediamine;
4-[[7-[4-[[2-(dimethylamino)ethyl]methylamino]phenyl]-1,6-naphthyridin-5-yl]oxy]-Cyclohexanol;
N-[7-[3-bromo-4-(4-morpholinyl)phenyl)-1,6-naphthyridin-5-yl]-1,3-Propanediamine;
N,N-dimethyl-4-[5-(4-methyl-1-piperazinyl)-1,6-naphthyridin-7-yl]-Benzenamine;
4-[[7-[4-[[3-(dimethylamino)propyl]methylamino]phenyl]-1,6-naphthyridin-5-yl]oxy]-Cyclohexanol;
N-[7-[4-[[2-(dimethylamino)ethyl]methylamino]phenyl]-1,6-naphthyridin-5-yl]-1,4-Butanediamine;
[3-[[5-[(3-aminopropyl)amino]-7-(4-methoxyphenyl)-1,6-naphthyridin-2-yl]amino]propyl]-Carbamic acid-1,1-dimethylethyl ester.

### DARREICHUNGSFORMEN

Geeignete Anwendungsformen sind beispielsweise Tabletten, Kapseln, Lösungen, Säfte, Emulsionen oder Inhalationspulver oder -aerosole. Hierbei soll der Anteil der pharmazeutisch wirksamen Verbindung(en) jeweils im Bereich von 0, 1 bis 90 Gew.-%, bevorzugt 0, 5 bis 50 Gew.% der Gesamtzusammensetzung liegen, d.h. in Mengen die ausreichend sind, um den unten angegebenen Dosierungsbereich zu erreichen.

Die orale Gabe kann in Form einer Tablette, als Pulver, als Pulver in einer Kapsel (z.B. Hartgelatinekapsel), als Lösung oder Suspension erfolgen. Im Fall einer inhalativen Gabe kann die Wirkstoffkombination als Pulver, als wässrige oder wässrig-ethanolische Lösung oder mittels einer Treibgasformulierung erfolgen.

Bevorzugt sind deshalb pharmazeutische Formulierungen gekennzeichnet durch den Gehalt an einer oder mehrerer Verbindungen der Formel 1 gemäß der obigen bevorzugten Ausführungsformen.

Besonders bevorzugt ist es, wenn die Verbindungen der Formel 1 oral verabreicht werden, besonders bevorzugt ist es, wenn die Verabreichung ein oder zweimal täglich erfolgt. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein Geschmack verbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Die eine oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.
Geeignete Zäpfchen lassen sich beispielsweise durch Vermischen mit dafür vorgesehenen Trägermitteln, wie Meutralfetten oder Polyäthylenglykol beziehungsweise dessen Derivaten, herstellen.

Als Hilfsstoffe seien beispielsweise Wasser, pharmazeutisch unbedenkliche organische Lösemittel, wie Paraffine (z.B. Erdölfraktionen), Öle pflanzlichen Ursprungs (z.B. Erdnuss- oder Sesamöl), mono- oder polyfunktionelle Alkohole (z.B. Ethanol oder Glycerin), Trägerstoffe wie z.B. natürliche Gesteinsmehle (z.B. Kaoline, Tonerden, Talkum, Kreide) synthetische Gesteinsmehle (z.B. hochdisperse Kieselsäure und Silikate), Zucker (z.B. Rohr-, Milch- und Traubenzucker) Emulgiermittel (z.B. Lignin, Sulfdablaugen, Methylcellulose, Stärke und Polyvinylpyrrolidon) und Gleitmittel (z.B. Magnesiumstearat, Talkum, Stearinsäure und Natriumlaurylsulfat) erwähnt.

Im Falle der oralen Anwendung können die Tabletten selbstverständlich außer den genannten Trägerstoffen auch Zusätze, wie z.B. Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mit verwendet werden. Im Falle wässriger Suspensionen können die Wirkstoffe außer den oben genannten Hilfsstoffen mit verschiedenen Geschmacksaufbesserern oder Farbstoffen versetzt werden.

Ebenfalls bevorzugt ist es, wenn die Verbindungen der Formel 1 inhalativ verabreicht werden, besonders bevorzugt ist es, wenn die Verabreichung ein oder zweimal täglich erfolgt. Hierzu müssen die Verbindungen der Formel 1 in inhalierbaren Darreichungsformen bereitgestellt werden. Als inhalierbare Darreichungsformen kommen Inhalationspulver, treibgashaltige Dosieraerosole oder treibgasfreie Inhalationslösungen in Betracht, die gegebenenfalls im Gemisch mit gebräuchlichen physiologisch verträglichen Hilfsstoffen vorliegen.

Im Rahmen der vorliegenden Erfindung sind von dem Begriff treibgasfreie Inhalationslösungen auch Konzentrate oder sterile, gebrauchsfertige Inhalationslösungen umfasst. Die im Rahmen der vorliegenden Erfindung einsetzbaren Darreichungsformen werden im nachfolgenden Teil der Beschreibung detailliert beschrieben.

### Inhalationspulver

Sind die Verbindungen der Formel **1** im Gemisch mit physiologisch unbedenklichen Hilfsstoffen enthalten, können zur Darstellung der erfindungsgemäßen Inhatationspulver die folgenden physiologisch unbedenklichen Hilfsstoffe zur Anwendung gelangen: Monosaccharide (z.B. Glucose oder Arabinose), Disaccharide (z.B. Lactose, Saccharose, Maltose), Oligo- und Polysaccharide (z.B. Dextrane), Polyalkohole (z.B. Sorbit, Mannit, Xylit), Salze (z.B. Natriumchlorid, Calciumcarbonat) oder Mischungen dieser Hilfsstoffe miteinander. Bevorzugt gelangen Mono- oder Disaccharide zur Anwendung, wobei die Verwendung von Lactose oder Glucose, insbesondere, aber nicht ausschließlich in Form ihrer Hydrate, bevorzugt ist. Als besonders bevorzugt im Sinne der Erfindung gelangt Lactose, höchst bevorzugt Lactosemonohydrat als Hilfsstoff zur Anwendung. Verfahren zur Herstellung der erfindungsgemäßen Inhalationspulver durch Mahlen und Mikronisieren sowie durch abschließendes Mischen der Bestandteile sind aus dem Stand der Technik bekannt

### Treibgashaltige Inhalationsaerosole

Die im Rahmen der erfindungsgemäßen Verwendung einsetzbaren treibgashaltigen Inhalationsaerosole können die Verbindungen nach Formel **1** im Treibgas gelöst oder in dispergierter Form enthalten. Die zur Herstellung der Inhalationsaerosole einsetzbaren Treibgase sind aus dem Stand der Technik bekannt. Geeignete Treibgase sind ausgewählt aus der Gruppe bestehend aus Kohlenwasserstoffen wie n-Propan, n-Butan oder Isobutan und Halogenkohlenwasserstoffen wie bevorzugt fluorierten Derivaten des Methans, Ethans, Propans, Butans, Cyclopropans oder Cyclobutans. Die vorstehend genannten Treibgase können dabei allein oder in Mischungen derselben zur Verwendung kommen. Besonders bevorzugte Treibgase sind fluorierte Alkanderivate ausgewählt aus TG134a (1, 1, 1, 2-Tetrafluorethan), TG227 (1, 1, 1, 2, 3, 3, 3-Heptafluorpropan) und Mischungen derselben. Die im Rahmen der erfindungsgemäßen Verwendung einsetzbaren treibgashaltigen Inhalationsaerosole können ferner weitere Bestandteile wie Co-Solventien, Stabilisatoren, oberflächenaktive Mittel (surfactants), Antioxidantien, Schmiermittel sowie Mittel zur Einstellung des pH-Werts enthalten. All diese Bestandteile sind im Stand der Technik bekannt.

### Treibgasfreie Inhalationslösungen

Die erfindungsgemäße Verwendung von Verbindungen der Formel 1 erfolgt bevorzugt zur Herstellung von treibgasfreien Inhalationslösungen und Inhalationssuspensionen. Als Lösungsmittel kommen hierzu wässrige oder alkoholische, bevorzugt ethanolische Lösungen in Betracht. Das Lösungsmittel kann ausschließlich Wasser sein oder es ist ein Gemisch aus Wasser und Ethanol. Die Lösungen oder Suspensionen werden mit geeigneten Säuren auf einen pH-Wert von 2 bis 7, bevorzugt von 2 bis 5 eingestellt Zur Einstellung dieses pH-Werts können Säuren ausgewählt aus anorganischen oder organischen Säuren Verwendung finden. Beispiele für besonders geeignete anorganische Säuren sind Salzsäure, Bromwasserstoffsäure, Salpetersäure, Schwefelsäure und/oder Phosphorsäure. Beispiele für besonders geeignete organische Säuren sind: Ascorbinsäure, Zitronensäure, Äpfelsäure, Weinsäure, Maleinsäure, Bernsteinsäure, Fumarsäure, Essigsäure, Ameisensäure und/oder Propionsäure und andere. Bevorzugte anorganische Säuren sind Salzsäure, Schwefelsäure. Es können auch die Säuren verwendet werden, die bereits mit einem der Wirkstoffe ein Säureadditionssalz bilden. Unter den organischen Säuren sind Ascorbinsäure, Fumarsäure und Zitronensäure bevorzugt. Gegebenenfalls können auch Gemische der genannten Säuren eingesetzt werden, insbesondere in Fällen von Säuren, die neben ihren Säuerungseigenschaften auch andere Eigenschaften, z.B. als Geschmackstoffe, Antioxidantien oder Komplexbildner besitzen, wie beispielsweise Zitronensäure oder Ascorbinsäure. Erfindungsgemäß besonders bevorzugt wird Salzsäure zur Einstellung des pH-Werts verwendet.

Den im Rahmen der erfindungsgemäßen Verwendung einsetzbaren treibgasfreien Inhalationslösungen können Co-Solventien und/oder weitere Hilfsstoffe zugesetzt werden. Bevorzugte Co-Solventien sind solche, die Hydroxylgruppen oder andere polare Gruppen enthalten, beispielsweise Alkohole - insbesondere Isopropylalkohol, Glykole - insbesondere Propylenglykol, Polyethylenglykol, Polypropylenglykol, Glykolether, Glycerol, Polyoxyethylenalkohole und Polyoxyethylen-Fettsäureester.
Unter Hilfs- und Zusatzstoffen wird in diesem Zusammenhang jeder pharmakologisch verträgliche Stoff verstanden, der kein Wirkstoff ist, aber zusammen mit dem (den) Wirkstoff(en) in dem pharmakologisch geeigneten Lösungsmittel formuliert werden kann, um die qualitativen Eigenschaften der Wirkstoffformulierung zu verbessern. Bevorzugt entfalten diese Stoffe keine oder im Kontext mit der angestrebten Therapie keine nennenswerte oder zumindest keine unerwünschte pharmakologische Wirkung. Zu den Hilfs- und Zusatzstoffen zählen z.B. oberflächenaktive Stoffe, wie z.B. Sojalecithin, Ölsäure, Sorbitanester, wie Polysorbate, Polyvinylpyrrolidon sonstige Stabilisatoren, Komplexbildner, Antioxidantien und/oder Konservierungsstoffe, die die Verwendungsdauer der fertigen Arzneimittelformulierung gewährleisten oder verlängern, Geschmackstoffe, Vitamine und/oder sonstige dem Stand der Technik bekannte Zusatzstoffe. Zu den Zusatzstoffen zählen auch pharmakologisch unbedenkliche Salze wie beispielsweise Natriumchlorid als Isotonantien. Zu den bevorzugten Hilfsstoffen zählen Antioxidantien, wie beispielsweise Ascorbinsäure, sofern nicht bereits für die Einstellung des pH-Werts verwendet, Vitamin A, Vitamin E, Tocopherole und ähnliche im menschlichen Organismus vorkommende Vitamine oder Provitamine. Konservierungsstoffe können eingesetzt werden, um die Formulierung vor Kontamination mit Keimen zu schützen. Als Konservierungsstoffe eignen sich die dem Stand der Technik bekannten, insbesondere Cetylpyridiniumchlorid, Benzalkoniumchlorid oder Benzoesäure bzw. Benzoate wie Natriumbenzoat in der aus dem Stand der Technik bekannten Konzentration.

Für die oben beschriebenen Behandlungsformen werden gebrauchsfertige Packungen eines Medikaments zur Behandlung von Atemwegserkrankungen, beinhaltend eine beigelegte Beschreibung, welche beispielsweise die Worte Atemwegserkrankung, COPD oder Asthma enthalten, Dihydrothienopyrimidin und ein oder mehrere Kombinationspartner ausgewählt aus der oben beschriebenen Gruppe, bereit gestellt.

## Patentansprüche

1. Verbindungen der Formel 1 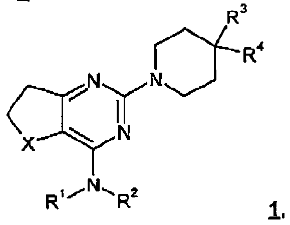 worin
**X** SO oder SO₂,
**R¹** H, C₁₋₆-Alkyl,
**R²** H ist oder ein Rest ausgewählt aus der Gruppe bestehend aus C₁₋₁₀-Alkyl und C₂₋₆-Alkenyl ist, der gegebenenfalls durch einen oder mehrere Reste ausgewählt aus Halogen und C₁₋₃-Fluoroolkyl substituiert sein kann oder der gegebenenfalls durch einen oder mehrere Reste ausgewählt aus der Gruppe bestehend aus OR^{2.1}, COOR^{2.1},CONR^{2.2}R^{2.3}, SR^{2.1},SO-R^{2.1}, SO₂-R²¹, C₆₋₁₀-Aryl, -Het, Hetaryl, einem mono- oder bicyclischem -C₃₋₁₀Cycloalkyl, CH₂-NR^{2.2}R^{2.3} und NR^{2.2}R^{2.3} substituiert sein kann, welcher wiederum gegebenenfalls durch einen oder mehrere Reste ausgewählt aus der Gruppe bestehend aus OH, Halogen, OR^{2.1}, Oxo, CF₃, CHF₂, CH₂F, C₁₋₆-Alkyl, C₁₋₆-Alkanol, C₆₋₁₀-Aryl, COOR^{2.1}, CH₂-NR^{2.2}R^{2.3} und NR^{2.2}R^{2.3} substituiert sein kann,
wobei
**Het** ein drei- bis elfgliedriger, mono- oder bicyclischer, gesättigter oder teilweise gesättigter, gegebenenfalls annellierter oder gegebenenfalls überbrückter Heterocyclus ist, der 1, 2, 3 oder 4 Heteroatome unabhängig voneinander ausgewählt aus der Gruppe bestehend aus N, S oder O enthält, und wobei
**Hetaryl** ein fünf- bis zehngliedriger, mono- oder bicyclisches, gegebenenfalls annelliertes Heteroaryl ist, das 1,2,3 oder 4 Heteroatome unabhängig voneinander ausgewählt aus der Gruppe bestehend aus N, S oder O enthält,
und wobei
**Cycloalkyl** gesättigt oder teilweise gesättigt sein kann,
wobei **R^{2.1}** H ist oder ein Rest ausgewählt ist aus der Gruppe bestehend aus C₁₋₆-Alkyl, C₁₋₆-Alkanol, C₁₋₃-Haloalkyl, mono- oder bicyclisches, -C₃₋₁₀-Cycloalkyl, C₆₋₁₀Aryl-C₁₋₆-alkylen, Hetaryl-C₁₋₆-alkylen-, Het-C₁₋₆-alkylen-, C₃₋₁₀-Cycloalkyl-C₁₋₆-alkylen-, ein mono- oder bicyclisches C₆₋₁₀-Aryl, Heteroaryl und ein -Het, der gegebenenfalls durch einen oder mehrere Reste ausgewählt aus der Gruppe bestehend aus OH, O-(C₁₋₃-Alkyl), Halogen, C₁₋₆-Alkyl und C₆₋₁₀-Aryl substituiert sein kann,
wobei **R^{2.2}** und **R^{2.3}** unabhängig voneinander H sind oder ein Rest ausgewählt sind aus der Gruppe bestehend aus C₁₋₆-Alkyl, mono- oder bicyclisches C₃₋₁₀ Cycloalkyl, C₆₋₁₀-Aryl-C₁₋₆-alkylen, Hetaryl-C₁₋₆-alkylen, mono- oder bicyclisches C₈₋₁₀-Aryl, Het, Hetaryl, CO-NH₂, CO-NHCH₃,-CO-N(CH₃)₂, SO₂-(C₁-C₂-Alkyl), CO-R^{2.1} und COOR^{2.1}, der gegebenenfalls durch einen oder mehrere Reste ausgewählt aus der Gruppe bestehend aus OH, Halogen, C₁₋₆-Alkyl, C₆₋₁₀Aryl und COOR^{2.1} substituiert sein kann,
oder
**R²** ein mono- oder polycyclisches C₃₋₁₀ Cycloalkyl, das gegebenenfalls einfach oder mehrfach über C₁₋₃-Alkylgruppen verbrückt sein kann und das gegebenenfalls mit einem Rest ausgewählt aus der Gruppe bestehend aus verzweigtes oder unverzweigtes C₁₋₆-Alkanol, C₁₋₃-Fluoroalkyl, C₁₋₃-alkylen-OR^{2.1}, OR^{2.1}, COOR^{2.1}, -SO₂-NR^{2.2}R^{2.3}, Het, -NH-CO-O-(C₁₋₆-Alkyl), -NH-CO-(C₁₋₆-Alkyl), -NH-CO-O-(C₆₋₁₀-Aryl), -NH-CO-(C₆₋₁₀-Aryl), -NH-CO-O-Hetaryl, -NH-CO-Hetaryl, -NH-CO-O-(C₁₋₃-Alkylen)-(C₆₋₁₀-Aryl), -NH-CO-(C₁₋₃-Alkylen)-(C₆₋₁₀-Aryl) -N(C₁₋₃-Alkyl)-CO-(C₁₋₆-Alkyl), -N(C₁₋₃-Alkyl)-CO-O-(C₆-₁₀-Aryl), -N(C₁₋₃-Alkyl)-CO-(C₆₋₁₀-Aryl), -N(C₁₋₃-Alkyl)-CO-O-Hetaryl, -N(C₁₋₃-Alkyl)-CO-Hetaryl, -N(C₁₋₃-Alkyl)-CO-O-(C₁₋₃-Alkylen)-(C₆₋₁₀-Aryl),-N(C₁₋₃-Alkyl)-CO-(C₁₋₃-Alkylen)-(C₆₋₁₀-Aryl), C₆₋₁₀-Aryl, C₁₋₆-Alkyl, C₆₋₁₀-Aryl-C₁₋₆-alkylen, Hetaryl-C₁₋₆-alkylen, mono- oder bicyclisches C₃₋₁₀ Cycloalkyl und NR^{2.2}R^{2.3} substituiert sein kann, der gegebenenfalls durch einen oder mehrere Reste ausgewählt aus der Gruppe bestehend aus OH, OR^{2.1}, Oxo, Halogen, CF₃, CHF₂, CH₂F, C₁₋₆-Alkyl, C₆₋₁₀-Aryl und NR^{2.2}R^{2.3} substituiert sein kann,
oder
**R²** ein mono- oder polycyclisches C₆₋₁₀-Aryl, das gegebenenfalls durch OH, SH oder Halogen oder durch einen oder mehrere Reste ausgewählt aus der Gruppe bestehend aus OR^{2.1}, COOR^{2.1}, NR^{2.2}R^{2.3}, CH₂-NR^{2.2}R^{2.3}C₃₋₁₀-Cycloalkyl, Het, C₁₋₆-Alkyl, C₁₋₃-Fluoroalkyl, CF₃, CHF₂, CH₂F, C₆₋₁₀-Aryl-C₁₋₆-alkylen, Het-C₁₋₆-alkylen, Hetaryl-C₁₋₆-alkylen, C₆₋₁₀-Aryl, SO₂-CH₃, SO₂-CH₂CH₃ und SO₂-NR^{2.2}R^{2.3} substituiert sein kann, der wiederum gegebenenfalls durch einen oder mehrere oder mehrere Reste ausgewählt aus der Gruppe bestehend aus OH, OR^{2.1}, CF₃, CHF₂, CH₂F, Oxo, Halogen, CF₃, CHF₂, CH₂F, C₁₋₆-Alkyl, C₆₋₁₀-Aryl und NR^{2.2}R^{2.3} substituiert sein kann,
oder
**R²** ein Rest ausgewählt aus einer Gruppe bestehend aus Het und Hetaryl, welcher gegebenenfalls durch einen oder mehrere Reste ausgewählt aus der Gruppe Halogen, OH, Oxo, CF₃, CHF₂ und CH₂F oder durch einen oder mehrere Reste ausgewählt aus der Gruppe OR^{2.1}, C₁₋₃-alkylen-OR^{2.1}, SR^{2.1},SO-R^{2.1}, SO₂-R^{2.1}, COOR^{2.1}, COR^{2.1}, C₁₋₆-Alkanol, mono- oder bicyclisches C₃₋₁₀-Cycloalkyl, C₆₋₁₀-Aryl, C₁₋₆-Alkyl, C₆₋₁₀-Aryl-C₁₋₆-alkylen, Hetaryl-C₁₋₆-alkylen, Het, Hetaryl, C₁₋₃-alkylen-OR^{2.1} und NR^{2.2}R^{2.3} substituiert sein kann, der gegebenenfalls wiederum durch einen oder mehrere Reste ausgewählt aus der Gruppe bestehend aus OH, OR^{2.1}, Oxo, Halogen, CF₃, CHF₂, CH₂F, C₁₋₆-Alkyl, C₆₋₁₀-Aryl und NR^{2.2}R^{2.3} substituiert sein kann,
oder worin
**NR¹R²** gemeinsam einen heterocyclischen C₄₋₇Ring bedeutet, der gegebenenfalls überbrückt sein kann, der 1, 2 oder 3 Heteroatome ausgewählt aus der Gruppe bestehend aus N, O und S enthält und der gegebenenfalls durch einen oder mehrere Reste ausgewählt aus der Gruppe bestehend aus OH, OR^{2.1}, C₁₋₃-alkylen-O^{R.1}, Oxo, Halogen, C₁₋₆-Alkyl, C₆₋₁₀-Aryl, COOR^{2.1}, CH₂-NR^{2.2}-COO-R^{2.1}, CH₂-NR^{2.2}-CO-R^{2.1}, CH₂-NR^{2.2}-CO-CH₂-NR^{2.2}R^{2.3}, CH₂-NR^{2.2}-SO₂-C₁₋₃-Alkyl, CH₂-NR^{2.2}-SO₂-NR^{2.2}R^{2.3}, CH₂-NR^{2.2}-CO-NR^{2.2}R^{2.3}, CO-NR^{2.2}R^{2.3}, CH₂-NR^{2.2}R^{2.3} und NR^{2.2}R^{2.3} substituiert sein kann,
und worin
**R³** ein C₆₋₁₀-Aryl ist, welches gegebenenfalls in ortho, para oder meta-Stellung mit einem, zwei oder drei Resten unabhängig voneinander ausgewählt aus der Gruppe bestehend aus Fluor, Chlor, Brom, Hydroxy, CN, C₁₋₆-Alkyl, C₁₋₃-Fluoroalkyl, -C₁₋₃-alkylen-OR^{2.1}, -C₁₋₃-alkylen-NR^{2.2}R^{2.3}, -NR^{2.2}R^{2.3}, O-R^{2.1}; SO-R^{2.1}, SO₂-R^{2.1}, COOR^{2.1}, -CO-NH-(C₁₋₆-Alkylen)-Hetaryl, -CO-NH-Hetaryl, -CO-N(CH₃)-Het, -CO-N(CH₃)-(C₁₋₃-Alkylen)-Het,-CO-N(CH₃)-(C₁₋₃-Alkylen)-Hetaryl, -CO-N(C₃₋₇-Cycloalkyl)-Het, -CO-NR^{2.2}R^{2.3}, -CO-NH-(C₁₋₆-Alkylen)-Het, NR^{2.2}-CO-R^{2.1}, C₆₋₁₀-Aryl, C₆₋₁₀-Aryl-C₁₋₂-alkylen, Het-C₁₋₂- alkylen, -Het, -CO-Het, , CO-N(CH₃)-C₃₋₇-Cycloalkyl, C₃₋₇-Cycloalkyl, C₃₋₇-Cycloalkyl-C₁₋₂-alkylen, Hetaryl-C₁₋₂-alkylen und Hetaryl substituiert ist, wobei dieser Rest gegebenenfalls durch einen oder mehrere Reste ausgewählt aus der Gruppe bestehend aus OH, Halogen, -C₁₋₃-Fluoroalkyl, Oxo, Methyl und Phenyl substituiert sein kann,
oder worin
**R³** ein Rest ausgewählt aus der Gruppe bestehend aus Het und Hetaryl ist, der gegebenenfalls durch einen oder mehrere Reste ausgewählt aus der Gruppe bestehend aus Halogen, C₁₋₃-Fluoroalkyl, CN, OH, Oxo, -C₁₋₆-Alkyl, -C₁₋₃-alkylen-NR^{2.2}R^{2.3}, -NR^{2.2}R^{2.3}, SO-R^{2.1}, SO₂-R^{2.1}, -O-R^{2.1}, -COOR^{2.1}, SO₂-(CH₃), SO₂-(CH₂-CH₃), C₆₋₁₀-Aryl, Het, C₃₋₇-Cycloalkyl und Hetaryl substituiert sein kann, der wiederum gegebenenfalls mit einem oder mehreren Resten ausgewählt aus der Gruppe OH, Halogen, -C₁₋₃-Fluoroalkyl, C₁₋₆-Alkyl, C₆₋₁₀-Aryl, -COO(C₁₋₃-Alkyl) und O-(C₁₋₃-Alkyl) substituiert sein kann,
oder worin
**R³** -O-R^{3.1},
wobei R^{3.1} ein Rest ausgewählt aus der Gruppe bestehend aus -C₁₋₆-Alkyl, -C₆₋₁₀-Aryl,-C₁₋₃-Alkylen-C₆₋₁₀-Aryl, Hetaryl und Het ist, welcher gegebenenfalls in ortho, para oder meta-Stellung mit einem, zwei oder drei Resten unabhängig voneinander ausgewählt aus der Gruppe bestehend aus Fluor, Chlor, Brom, Hydroxy, CN, C₁₋₆-Alkyl, C₁₋₃-Fluoroalkyl, CO-(C₁₋₅-Alkyl), -CO-(C₁₋₃-Fluoroalkyl), -CO-NH-(C₁₋₆-Alkylen)-Hetaryl, -CO-N(C₁₋₃-Alkyl)-(C₁₋₆-Alkylen)-Hetaryl, -CO-N(C₁₋₃-Alkyl)-Het, -CO-N(C₃₋₇Cycloalkyl)-Het, -C₁₋₃-alkylen-OR^{2.1}, -C₁₋₃-alkylen-NR^{2.2}R^{2.3}, -NR^{2.2}R^{2.3}, O-R^{2.1}; SO-R^{2.1}, SO₂-R^{2.1}, COOH, COO-(C₁₋₄-Alkyl), -O-C₁₋₃-Alkylen-N(C₁₋₃-Alkyl)₂, CO-NR^{2.2}R^{2.3}, NR^{2.2}-CO-R^{2.1}, C₆₋₁₀-Aryl, C₆₋₁₀-Aryl-C₁₋₂-alkylen, Het-C₁₋₂-alkylen, -CO-Het, Het, -CO-C₃₋₇-Cycloalkyl, -CO-N(C₁₋₃-Alkyl)-C₃₋₇-Cycloalkyl C₃₋₇-Cycloalkyl, C₃₋₇-Cycloalkyl-C₁₋₂-alkylen, Hetaryl-C₁₋₂-alkylen und Hetaryl substituiert ist, welcher wiederum gegebenenfalls mit 1, 2, 3 oder 4 Resten unabhängig voneinander ausgewählt aus der Gruppe bestehend aus F, Cl, Br, Methyl, O-Methyl, Ethyl, O-Ethyl, OH, Oxo und CF₃ substituiert sein kann.
und worin
**R⁴** H, CN, OH, CF₃, CHF₂, CH₂F, F, Methyl, Ethyl, -O-(C₁₋₃-Alkyl), -C₁₋₃-Alkylen-OH, -COO(C₁₋₃-Alkyl), -CO-Het, -(C₁₋₂-Alkylen)-NH-SO₂-(C₁₋₂-Alkyl), -(C₁₋₂Alkylen)-N(C₁₋₃-Alkyl)-SO₂-(C₁₋₂-Alkyl), -(C₁₋₂-Alkylen)-O-(C₁₋₂-Alkylen)-C₆₋₁₀-Aryl, -C₁₋₃-Alkylen-O-C₁₋₃-Alkyl, -(C₁₋₂-Alkylen)-N(C₁₋₃-Alkyl)-CO-(C₁₋₂-Alkyl), -NH-CO-(C₁₋₃-Alkylen)-O-(C₁₋₃-Alkyl), -C₁₋₃-Alkylen-NH-CO-(C₁₋₃-Alkyl), -C₁₋₃-Alkylen-NH-CO-(C₁₋₃-Alkylen)-N(C₁₋₃-Alkyl)₂, -O-(C₁₋₂-Alkylen)-(C₆₋₁₀-Aryl) -C₁₋₃-Alkylen-NH-CO-(C₁₋₃)Alkylen)-O-(C₁₋₃-Alkyl), -CO-(C₆₋₁₀-Aryl), -(C₁₋₂-Alkylen)-N(C₁₋₃-Alkyl)-CO-(C₁₋₂-Alkylen)-O-(C₁₋₃-Alkyl), wobei das Aryl in den obigen Resten gegebenenfalls wiederum mit einem oder mehreren weiteren Resten ausgewählt aus der Gruppe F, Cl, Br, Methyl, Ethyl, Propyl, Isopropyl, Cyclopropyl, -O-Methyl, -O-Ethyl, -O-Propyl, -O-Isopropyl, -O-Cyclopropyl, -OH und CF₃ substituiert sein kann
oder worin
R³ und R⁴ gemeinsam einen mono- oder bicyclischen, ungesättigten, gesättigten oder teilweise gesättigten Heterocyclus bilden, der 1, 2 oder 3 Heteroatome ausgewählt aus der Gruppe bestehend aus N, O und S enthält und der gegebenenfalls mit einem oder mehreren Resten ausgewählt aus der Gruppe bestehend aus Halogen, OH, Oxo, C₁₋₃-Fluoroalkyl, CN, C₁₋₆-Alkyl, -O-R^{2.1},-COOR^{2.1}, SO-R^{2.1}, SO₂-R^{2.1}, -C₁₋₃-alkylen-NR^{2.2}R^{2.3}, -NR^{2.2}R^{2.3}, C₆₋₁₀-Aryl, C₃₋₇-Cycloalkyl, Het und Hetaryl substituiert sein kann,
sowie pharmakologisch verträgliche Salze Diastereomere, Enantiomere, Racemate, Hydrate oder Solvate davon.

2. Verbindungen der Formel **1** nach Anspruch 1, wobei
**X** SO oder SO₂,
**R¹** H
**R²** H ist oder C₁₋₁₀-Alkyl ist, das gegebenenfalls durch einen oder mehrere Reste ausgewählt aus Halogen und C₁₋₃-Fluoroalkyl substituiert sein kann oder das gegebenenfalls durch einen oder mehrere Reste ausgewählt aus der Gruppe bestehend aus OR^{2.1}, COOR^{2.1},CONR^{2.2}R^{2.3}, SR^{2.2},SO-R^{2.1}, SO₂-R^{2.1}, Phenyl, Het, Hetaryl, einem monocyclischem C₃₋₇-Cycloalkyl, CH₂NR^{2.2}R^{2.3} und NR^{2.2}R^{2.3} substituiert sein kann, welcher wiederum gegebenenfalls durch einen oder mehrere Reste ausgewählt aus der Gruppe bestehend aus OH, F, Cl, Br, OR^{2.1}, Oxo, CF₃, CHF₂, CH₂F, C₁₋₆-Alkyl, C₁₋₆-Alkanol, Phenyl, COOR^{2.1}, CH₂-NR^{2.2}R^{2.3} und NR^{2.2}R^{2.3} substituiert sein kann,
wobei
**Het** ein drei- bis siebengliedriger, monocyclischer, gesättigter oder teilweise gesättigter Heterocyclus oder ein sieben- bis elfgliedriger, bicyclischer, gesättigter oder teilweise gesättigter Heterocyclus ist, der 1, 2, 3 oder 4 Heteroatome unabhängig voneinander ausgewählt aus der Gruppe bestehend aus N, S oder O enthält, und wobei
**Hetaryl** ein fünf- bis sechsgliedriges, monocyclisches, aromatisches Heteroaryl oder ein sieben- bis elfgliedriges, bicyclisches, aromatisches Heteroaryl ist, das jeweils 1, 2, 3 oder 4 Heteroatome unabhängig voneinander ausgewählt aus der Gruppe bestehend aus N, S oder O enthält,
und wobei
**Cycloalkyl** gesättigt oder teilweise gesättigt sein kann,
wobei **R^{2.1}** H ist oder ein Rest ausgewählt ist aus der Gruppe bestehend aus C₁₋₆-Alkyl, C₁₋₆-Alkanol, C₁₋₃-Haloalkyl, monocyclisches C₃₋₇ Cycloalkyl, Phenyl-C₁₋₆-alkylen, Hetaryl-C₁₋₆-alkylen, Het-C₁₋₆-alkylen, -C₃₋₇-Cycloalkyl-C₁₋₆-alkylen, Phenyl, Hetaryl und ein Het, der gegebenenfalls durch einen oder mehrere Reste ausgewählt aus der Gruppe bestehend aus OH, F, Cl, C₁₋₆-Alkyl, -O-(C₁₋₃-Alkyl) und Phenyl substituiert sein kann,
wobei **R^{2.2}** und **R^{2.3}** unabhängig voneinander H sind oder ein Rest ausgewählt sind aus der Gruppe bestehend aus C₁₋₆-Alkyl, monocyclisches C₃₋₇ Cycloalkyl, Phenyl-C₁₋₃-alkylen, Hetaryl-C₁₋₃-alkylen, Phenyl, Het, Hetaryl, CO-NH₂, -CO-NHCH₃, -CON(CH₃)₂, SO₂-(C₁₋₂-Alkyl), CO-R^{2.1} und COOR^{2.1}, der gegebenenfalls durch einen oder mehrere Reste ausgewählt aus der Gruppe bestehend aus OH, F, Cl, C₁₋₆-Alkyl, Phenyl und COOR^{2.1} substituiert sein kann,
oder
**R²** ein monocyclisches C₃₋₇ Cycloalkyl, das gegebenenfalls mit einem Rest ausgewählt aus der Gruppe bestehend aus verzweigtes oder unverzweigtes C₁₋₆-Alkanol, C₁₋₃-Fluoroalkyl, OR^{2.1}, C₁₋₃-alkylen-OR^{2.1},OR^{2.1}, COOR^{2.1}, SO₂-NR^{2.2}R^{2.3}, -Het, -NH-CO-O-(Phenyl), Phenyl, C₁₋₆-Alkyl, Phenyl-C₁₋₆-alkylen, -Hetaryl-C₁₋₆-alkylen, monocyclisches C₃₋₇ Cycloalkyl und NR^{2.2}R^{2.3} substituiert sein kann, der gegebenenfalls durch einen oder mehrere Reste ausgewählt aus der Gruppe bestehend aus OH, OR^{2.1}, Oxo, F, Cl, CF₃, CHF₂, CH₂F, C₁₋₆-Alkyl, Phenyl und -NR^{2.2}R^{2.3} substituiert sein kann,
oder
**R²** ein Phenyl, das gegebenenfalls durch OH, SH oder Halogen oder durch einen oder mehrere Reste ausgewählt aus der Gruppe bestehend aus OR^{2.1}, COOR^{2.1}, NR^{2.2}R^{2.3}, CH₂-NR^{2.2}R^{2.9}C₃₋₇Cycloalkyl, C₃₋₇-Heterocyclus, C₁₋₆-Alkyl C₁₋₃-Fluoroalkyl, Phenyl-C₁₋₆-alkylen, -Het-C₁₋₆-alkylen, -Hetaryl-C₁₋₆-alkylen, Phenyl, SO₂-CH₃, SO₂-CH₂CH₃ und SO₂-NR^{2.2}R^{2.3} substituiert sein kann, der wiederum gegebenenfalls durch einen oder mehrere oder mehrere Reste ausgewählt aus der Gruppe bestehend aus OH, OR^{2.1}, Oxo, F, Cl, CF₃, CHF₂, CH₂F, C₁₋₆Alkyl, Phenyl und NR^{2.2}R^{2.3} substituiert sein kann,
oder
**R²** ein Rest ausgewählt aus einer Gruppe bestehend aus Het und Hetaryl, welcher gegebenenfalls durch einen oder mehrere Reste ausgewählt aus der Gruppe F, Cl, OH, Oxo, CF₃, CHF₂ und CH₂F oder durch einen oder mehrere Reste ausgewählt aus der Gruppe OR^{2.1}, -C₁₋₃-alkylen-OR^{2.1}, SR^{2.1},SO-R^{2.1}, SO₂-R^{2.1}, COOR^{2.1}, COR^{2.1}, C₁₋₆-Alkanol, monocyclisches C₃₋₇Cycloalkyl, Phenyl, C₁₋₆-Alkyl, Phenyl-C₁₋₆-alkylen, -Hetaryl-C₁₋₆-alkylen, -Het, -Hetaryl, und NR^{2.2}R^{2.3} substituiert sein kann, der gegebenenfalls wiederum durch einen oder mehrere Reste ausgewählt aus der Gruppe bestehend aus OH, OR^{2.1}, Oxo, F, Cl, CF₃, CHF₂, CH₂F, C₁₋₆-Alkyl, Phenyl und NR^{2.2}R^{2.3} substituiert sein kann,
oder worin
**NR¹R²** gemeinsam einen heterocyclischen C₄₋₇-Ring bedeutet, der gegebenenfalls überbrückt sein kann, der 1, 2 oder 3 Heteroatome ausgewählt aus der Gruppe bestehend aus N, O und S enthält und der gegebenenfalls durch einen oder mehrere Reste ausgewählt aus der Gruppe bestehend aus OH, OR^{2.1}, C₁₋₃-alkylen-O^{R.1}, Oxo, F, Cl, C₁₋₆Alkyl, Phenyl, COOR^{2.1}, CH₂-NR^{2.2}-COO-R^{2.1}, CH₂-NR^{2.2}-CO-R^{2.1}, CH₂-NR^{2.2}-CO-CH₂-NR^{2.2}R^{2.3}, CH₂-NR^{2.2}-SO₂-C₁₋₃-Alkyl, CH₂-NR^{2.2}-SO₂-NR^{2.2}R^{2.3}, CH₂-NR^{2.2}-CO-NR^{2.2}R^{2.3}, CO-NR^{2.2}R^{2.3}, CH₂-NR^{2.2}R^{2.3} und NR^{2.2}R^{2.3} substituiert sein kann,
und worin
**R³** ein Naphthalin oder Phenyl ist, welches gegebenenfalls in ortho, para oder meta-Stellung mit einem oder zwei Resten unabhängig voneinander ausgewählt aus der Gruppe bestehend aus Fluor, Chlor, Brom, Hydroxy, CN, C₁₋₆-Alkyl, C₁₋₃-Fluoroalkyl, -C₁₋₃-alkylen-OR^{2.1}, -C₁₋₃-alkylen-NR^{2.2}R^{2.3}, -NR^{2.2}R^{2.3}, O-R^{2.1}, SO-R^{2.1}, SO₂-R^{2.1}, COOR^{2.1}, -CO-NH-(C₁₋₈-Alkylen)-Hetaryl, -CO-NH-Hetaryl, -CO-N(CH₃)-Het, -CO-N(CH₃)-(C₁₋₃-Alkylen)-Het,-CO-N(CH₃)-(C₁₋₃-Alkylen)-Hetaryl, -CO-N(C₃₋₇-Cycloalkyl)-Het, CO-NR^{2.2}R^{2.3}, -CO-NH-(C₁₋₆-Alkylen)-Het, -NR^{2.2}-CO-R^{2.1}, Phenyl, Phenyl-C₁₋₂-alkylen, -Het-C₁₋₂-alkylen, -Het, -CO-Het, -CO-N(CH₃)-Het, CO-N(CH₃)-C₃₋₇-Cycloalkyl, C₃₋₇Cycloalkyl, C₃₋₇-Cycloalkyl-C₁₋₂-alkylen, -Hetaryl-C₁₋₂-alkylen und -Hetaryl substituiert ist, wobei dieser Rest gegebenenfalls durch einen oder mehrere Reste ausgewählt aus der Gruppe bestehend aus OH, F, Cl, -C₁₋₃-Fluoroalkyl, Oxo, Methyl und Phenyl substituiert sein kann,
oder worin
**R³** ein Rest ausgewählt aus der Gruppe bestehend aus Het und Hetaryl, der gegebenenfalls durch einen oder mehrere Reste ausgewählt aus der Gruppe bestehend aus F, Cl, Br, C₁₋₃-Fluoroalkyl, CN, OH, Oxo, -C₁₋₈-Alkyl, -C₁₋₃-alkylen-NR^{2.2}R^{2.3}, -NR^{2.2}R^{2.3}, SO-R^{2.1}, SO₂-R^{2.1},-O-R^{2.1}, -COOR^{2.1}, SO₂-(CH₃), SO₂-(CH₂-CH₃), Phenyl, Het, C₃₋₇-Cycloalkyl und Hetaryl substituiert sein kann, der wiederum gegebenenfalls mit einem oder mehreren Resten ausgewählt aus der Gruppe OH, F, Cl, Br, -C₁₋₃-Fluoroalkyl, C₁₋₆-Alkyl, Phenyl, -COO(C₁₋₃-Alkyl) und O-(C₁₋₃-Alkyl) substituiert sein kann,
oder worin
**R³** -O-R^{3.1},
wobei **R^{3.1}** ein Rest ausgewählt aus der Gruppe bestehend aus -C₁₋₆-Alkyl, -Phenyl,-C₁₋₃-Alkylen-Phenyl, Hetaryl und Het ist, welcher gegebenenfalls in ortho, para oder meta-Stellung mit einem, zwei oder drei Resten unabhängig voneinander ausgewählt aus der Gruppe bestehend aus Fluor, Chlor, Brom, Hydroxy, CN, C₁₋₆-Alkyl, C₁₋₃-Fluoroalkyl, CO-(C₁₋₅-Alkyl), -CO-(C₁₋₃-Fluoroalkyl), -CO-NH-(C₁₋₆-Alkylen)-Hetaryl, -CO-N(CH₃)-(C₁₋₆-Alkylen)-Hetaryl, -CO-N(CH₃)-Het, -CO-N(C₃₋₇-Cycloalkyl)-Het, -C₁₋₃-alkylen-OR^{2.1}, -C₁₋₃-alkylen-NR^{2.2}R^{2.3}, -NR^{2.2}R^{2.3}, O-R^{2.1}; SO-R^{2.1}, SO₂-R^{2.1}, COOH, COO-(C₁₋₄-Alkyl), -O-C₁₋₃-Alkylen-N(C₁₋₃-Alkyl)₂, CO-NR^{2.2}R^{2.3}, NR^{2.2}-CO-R^{2.1}, Phenyl, Phenyl-C₁₋₂-alkylen, Het-C₁₋₂-alkylen, -CO-Het, Het, -CO-C₃₋₇Cycloalkyl, -CO-N (CH₃)-C₃₋₇-Cycloalkyl C₃₋₇-Cycloalkyl, C₃₋₇-Cycloalkyl-C₁₋₂-alkylen, Hetaryl-C₁₋₂-alkylen und Hetaryl substituiert ist, welcher wiederum gegebenenfalls mit 1, 2, 3 oder 4 Resten unabhängig voneinander ausgewählt aus der Gruppe bestehend aus F, Cl, Br, Methyl, O-Methyl, Ethyl, O-Ethyl, OH, Oxo und CF₃ substituiert sein kann,
und worin
R⁴ H, CN, OH, CF₃, CHF₂, CH₂F, F, Methyl, Ethyl, O-Methyl, O-Ethyl, O-Propyl, O-Isopropyl, -C₁₋₃-Alkylen-OH, -COO(C₁₋₃-Alkyl), -CO-Het, -(C₁₋₂-Alkylen)-NH-SO₂-(C₁₋₂-Alkyl), -(C₁₋₂-Alkylen)-N(CH₃)-SO₂-(C₁₋₂-Alkyl), -(C₁₋₂-Alkylen)-O-(C₁₋₂-Alkylen)-Phenyl, -C₁₋₃-Alkylen-O-C₁₋₃-Alkyl, -(C₁₋₂-Alkylen)-N(CH₃)-CO-(C₁₋₂-Alkyl), -NH-CO-(C₁₋₃-Alkylen)-O-(C₁₋₃-Alkyl), -C₁₋₃-Alkylen-NH-CO-(C₁₋₃-Alkyl), -C₁₋₃-Alkylen-NH-CO-(C₁₋₃-Alkylen)-N(C₁₋₃-Alkyl)₂, -O-(C₁₋₂-Alkylen)-Phenyl -C₁₋₃-Alkylen-NH-CO-(C₁₋₃-Alkylen)-O-(C₁₋₃-Alkyl), -CO-Phenyl, -(C₁₋₂-Alkylen)-N(CH₃)-CO-(C₁₋₂-Alkylen)-O-(C₁₋₃-Alkyl), wobei das Phenyl in den obigen Resten gegebenenfalls mit einem oder mehreren weiteren Resten ausgewählt aus der Gruppe F, Cl, Br, Methyl, Ethyl, Propyl, -O-Methyl, -O-Ethyl, -O-Propyl, -OH und CF₃ substituiert sein kann
oder worin
**R³** und **R⁴** gemeinsam einen mono- oder bicyclischen, ungesättigten, gesättigten oder teilweise gesättigten Heterocyclus bilden, der 1, 2 oder 3 Heteroatome ausgewählt aus der Gruppe bestehend aus N, O und S enthält und der gegebenenfalls mit einem oder mehreren Resten ausgewählt aus der Gruppe bestehend aus F, Cl, Br, OH, Oxo, C₁₋₃-Fluoroalkyl, CN, C₁₋₆-Alkyl, -O-R^{2.1},-COOR^{2.1} SO-R^{2.1}, SO₂-R^{2.1}, -C₁₋₃-alkylen-NR^{2.2}R^{2.3}, -NR^{2.2}R^{2.3}, Phenyl, C₃₋₇-Cycloalkyl, Het und Hetaryl substituiert sein kann,
sowie pharmakologisch verträgliche Salze Diastereomere, Enantiomere, Racemate, Hydrate oder Solvate davon.

3. Verbindungen der Formel **1** nach einem der Ansprüche 1 oder 2, wobei
**X** SO,
**R¹** H
**R²** H ist oder C₁₋₆₋Alkyl ist, das gegebenenfalls durch einen oder mehrere Reste ausgewählt aus F, Cl, CF₃, CHF₂ oder CH₂F substituiert sein kann oder das gegebenenfalls durch einen oder mehrere Reste ausgewählt aus der Gruppe bestehend aus OR^{2.1} COOR^{2.1}, CONR^{2.2}R^{2.3} SR^{2.1}, SO-R^{2.1} SO₂-R^{2.1}, Phenyl, Het, Hetaryl, einem monocyclischem C₃₋₇-Cycloalkyl, CH₂-NR^{2.2}R^{2.1} und NR^{2.2}R^{2.3} substituiert sein kann, welcher wiederum gegebenenfalls durch einen oder mehrere Reste ausgewählt aus der Gruppe bestehend aus OH, F, Cl, Br, CF₃, CHF₂, CH₂F, OR^{2.1} , Oxo, Methyl, Ethyl, Propyl, Isopropyl, Methanol, Ethanol, Phenyl, COOR^{2.1}, CH₂-NR^{2.2}R^{2.3} und NR^{2.2}R^{2.3} substituiert sein kann,
wobei
**Het** ein drei- bis siebengliedriger, monocyclischer, gesättigter oder teilweise gesättigter Heterocyclus ist, der 1, 2 oder 3 Heteroatome unabhängig voneinander ausgewählt aus der Gruppe bestehend aus N, S oder O enthält,
und wobei
**Hetaryl** ein fünf- bis sechsgliedriger, monocyclisches, aromatisches Heteroaryl ist, das 1, 2 oder 3 Heteroatome unabhängig voneinander ausgewählt aus der Gruppe bestehend aus N, S oder O enthält,
und wobei
**Cycloalkyl** gesättigt oder teilweise gesättigt sein kann,
wobei **R^{2.1}** H ist oder ein Rest ausgewählt ist aus der Gruppe bestehend aus Methyl, Ethyl, Propyl, Isopropyl, Methanol, Ethanol, monocyclisches C₃₋₇ Cycloalkyl, Phenyl-C₁₋₂-alkylen, -Hetaryl-C₁₋₂-alkylen, -Het-C₁₋₂-alkylen, C₃₋₇-Cycloalkyl-C₁₋₂-alkylen, Phenyl, Hetaryl und ein Het, der gegebenenfalls durch einen oder mehrere Reste ausgewählt aus der Gruppe bestehend aus OH, F, Cl, Methyl, Ethyl, Propyl, Isopropyl, O-Methyl, O-Ethyl, O-Propyl, O-Isopropyl und Phenyl substituiert sein kann,
wobei **R^{2.2}** und **R^{2.3}** unabhängig voneinander H oder ein Rest ausgewählt sind aus der Gruppe bestehend aus Methyl, Ethyl, Propyl, Isopropyl, monocyclisches C₃₋₇ Cycloalkyl, Phenyl-C₁₋₃-alkylen, Hetaryl-C₁₋₃-alkylen, Phenyl, -Het, -Hetaryl, CO-NH₂, CO-NHCH₃, CON(CH₃)₂, SO₂-(C₁₋₂-Alkyl), CO-R^{2.1} und COOR^{2.1}, der gegebenenfalls durch einen oder mehrere Reste ausgewählt aus der Gruppe bestehend aus OH, F, Cl, Methyl, Ethyl, Propyl, Isopropyl, Phenyl und COOR^{2.1} substituiert sein kann,
oder
**R²** ein monocyclisches C₂₋₇ Cycloalkyl, das gegebenenfalls mit einem Rest ausgewählt aus der Gruppe bestehend aus C₁₋₂-Alkanol, C₁₋₃-Fluoroalkyl C₁₋₃-alkylen-OR^{2.1}, OR^{2.1}, COOR^{2.1}, SO₂-NR^{2.2}R^{2.3}, -Het, -NH-CO-O-(Phenyl), Methyl, Ethyl, Propyl, Isopropyl, Phenyl, Phenyl-C₁₋₂-alkylen, -Hetaryl-C₁₋₂-alkylen, monocyclisches C₃₋₇ Cycloalkyl und NR^{2.2}R^{2.3} substituiert sein kann, der gegebenenfalls durch einen oder mehrere Reste ausgewählt aus der Gruppe bestehend aus OH, OR^{2.1}, Oxo, F, Cl, CF₃, CHF₂, CH₂F, Methyl, Ethyl, Propyl, Isopropyl, Phenyl und NR^{2.2}R^{2.3} substituiert sein kann,
oder
**R²** ein Phenyl, das gegebenenfalls durch OH, SH, F, Cl oder Br oder durch einen oder mehrere Reste ausgewählt aus der Gruppe bestehend aus OR^{2.1}, COOR^{2.1}, NR^{2.2}R^{2.3}, CH₂-NR^{2.2}R^{2.3}, monocyclisches C₃₋₇-Cycloalkyl, -Het, Methyl, Ethyl, Propyl, Isopropyl, CF₃, CHF₂, CH₂F, Phenyl-C₁₋₂-alkylen, Het-C₁₋₂-alkylen, Hetaryl-C₁₋2-alkylen, Phenyl, SO₂-CH₃, SO₂-CH₂CH₃ und SO₂-NR^{2.2}R^{2.3} substituiert sein kann, der wiederum gegebenenfalls durch einen oder mehrere oder mehrere Reste ausgewählt aus der Gruppe bestehend aus OH, OR^{2.1}, Oxo, F, Cl, CF₃, CHF₂, CH₂F, Methyl, Ethyl, Propyl, Isopropyl, Phenyl und NR^{2.2}R^{2.3} substituiert sein kann,
oder
**R²** ein Rest ausgewählt aus einer Gruppe bestehend aus Het und Hetaryl, der gegebenenfalls durch einen oder mehrere Reste ausgewählt aus der Gruppe F, Cl, OH, Oxo, CF₃, CHF₂ und CH₂F oder durch einen oder mehrere Reste ausgewählt aus der Gruppe OR^{2.1}, C₁₋₃-alkylen-OR^{2.1}, SR^{2.1},SO-R^{2.1}, SO₂-R^{2.1}, COOR^{2.1} COR^{2.1}, Methanol, Ethanol, monocyclisches C₃₋₇-Cycloalkyl, Phenyl, Methyl, Ethyl, Propyl, Isopropyl, Phenyl-C₁₋₂-alkylen, Hetaryl-C₁₋₂-alkylen, -Het, -Hetaryl und NR^{2.2}R^{2.3} substituiert sein kann, der gegebenenfalls wiederum durch einen oder mehrere Reste ausgewählt aus der Gruppe bestehend aus OH, OR^{2.1}, Oxo, F, Cl, CF₃, CHF₂, CH₂F, Methyl, Ethyl, Propyl, Isopropyl, Butyl, Isobutyl, Phenyl und NR^{2.2}R^{2.3} substituiert sein kann,
und worin
**R³** ein Naphthalin oder Phenyl ist, welches gegebenenfalls in ortho, para oder meta-Stellung mit einem oder zwei Resten unabhängig voneinander ausgewählt aus der Gruppe bestehend aus Fluor, Chlor, Brom, Hydroxy, CN, Methyl, Ethyl, Propyl, Isopropyl, Cyclopropyl, CF₃, CHF₂, CH₂F, -OCH₃, OCH₂CH₃; SO₂-CH₃, SO-CH₃, COOCH₃, COOCH₂CH₃ , -CO-NH-(Methylen)-Hetaryl, -CO-NH-(Ethylen)-Hetaryl, -CO-NH-Hetaryl, -CO-N(CH₃)-Het,-CO-N(CH₃)-(Methylen)-Het, -CO-N(CH₃)-(Ethylen)-Het, -CO-N(CH₃)-(Methylen)-Hetaryl, -CO-N(CH₃)-(Ethylen)-Hetaryl, -CO-N(Cyclopropyl)-Het, CO-NH₂, CONH(CH₃), CON(CH₃)₂, -CO-NH-(Methylen)-Het, -CO-NH-(Ethylen)-Het, -NH-CO-Methyl, NCH₃-CO-Methyl,-NH-CO-Ethyl, NCH₃-CO-Ethyl, -NH-CO-Propyl, NCH₃-CO-Propyl, -NH-CO-Isopropyl , NCH₃-CO-Isopropyl, Phenyl, Phenyl-Methylen, Phenyl-Ethylen, Het-Methylen, Het-Ethylen, -Het, -CO-Het, -CO-N(CH₃)-Het, CO- N(CH₃)-Cyclopropyl, C₃₋₇-Cycloalkyl, C₃₋₇-Cycloalkyl-Methylen, C₃₋₇-Cycloalkyl-Ethylen, Hetaryl-methylen, Hetaryl-Ethylen, -Hetaryl, CH₂-NH₂, CH₂-NH(CH₃), CH₂-N(CH₃)₂, -NH₂, -NH(CH₃) und -N(CH₃)₂ substituiert sein kann, wobei dieser Rest gegebenenfalls durch einen oder mehrere Reste ausgewählt aus der Gruppe bestehend aus OH, F, Cl, -CF₃, CHF₇, CH₂F, Oxo, Methyl und Phenyl substituiert sein kann
oder worin
**R³** ein Rest ausgewählt aus der Gruppe bestehend aus einem Het und Hetaryl, der gegebenenfalls durch einen oder mehrere Reste ausgewählt aus der Gruppe bestehend aus F, Cl, Br, CF₃, CHF₂, CH₂F, CN, OH, Oxo, Methyl, Ethyl, Propyl, Isopropyl, Butyl, Isobutyl, Cyclopropyl, -O-Methyl, -O-Ethyl, -O-Propyl, -O-Isopropyl,-COO-Methyl, -COO-Ethyl, -COO-Propyl, -COO-Isopropyl, SO-(CH₃), SO-(CH₂-CH₃), SO₂-(CH₃), SO₂-(CH₂-CH₃), Phenyl, CH₂-NH₂, CH₂-NH(CH₃), CH₂-N(CH₃)₂, -NH₂,-NH(CH₃), -N(CH₃)₂, Het und Hetaryl substituiert sein kann, der wiederum gegebenenfalls mit einem oder mehreren Resten ausgewählt aus der Gruppe OH, F, Cl, CF₃, CHF₂, CH₂F, Methyl, Ethyl, Propyl, Isopropyl, Phenol, -COO-Methyl, -COO-Ethyl und O-Methyl, O-Ethyl substituiert sein kann,
oder worin
**R³** -O-R^{3.1},
wobei **R^{3.1}** ein Rest ausgewählt aus der Gruppe bestehend aus -C₁₋₃-Alkyl, -Phenyl,-C₁₋₃-Alkylen-Phenyl, Hetaryl und Het ist, welcher gegebenenfalls in ortho, para oder meta-Stellung mit einem, zwei oder drei Resten unabhängig voneinander ausgewählt aus der Gruppe bestehend aus Fluor, Chlor, Brom, Hydroxy, CN, Methyl, Ethyl, Propyl, Isopropyl, Butyl, Isobutyl, CF₃, CHF₂, CH₂F, CO-(Methyl), CO-(Ethyl), CO-(Propyl), CO-(Isopropyl), -CO-(CF₃), -CO-NH-(Methylen)-Hetaryl, -CO-NH-(Ethylen)-Hetaryl, -CO-N(CH₃)-(Methylen)-Hetaryl, -CO-N(CH₃)-(Ethylen)-Hetaryl, -CO-N(CH₃)-(Propylen)-Hetaryl, -CO-N(CH₃)-(Isopropylen)-Hetaryl -CO-N(CH₃)-Het, -CO-N(Cyclopropyl)-Het, -CO-N(C₅₋₇-Cycloalkyl)-Het, -Methylen-O-Methyl, -Ethylen-O-Methyl, -Propylen-O-Methyl, -Methylen-O-Ethyl, -Ethylen-O-Ethyl, -Propylen-O-Ethyl, -Methylen-NH₂, -Methylen-NHCH₃, -Methylen-N(CH₃)₂, -Ethylen-NH₂, -Ethylen-NHCH₃, -Ethylen-N(CH₃)₂, NH₂, N(CH₃)₂, NHCH₃, -O-Methyl, O-Ethyl, O-Propyl, O-Isopropyl, O-Butyl, O-Isobutyl, -SO-CH₃, SO-Ethyl, -SO-Propyl, -SO-Isopropyl, , SO₂-Methyl, -SO₂-Ethyl, SO₂-Propyl, SO₂-Isopropyl, COOH, COO-(Methyl), COO-(Ethyl), COO-(Propyl), COO-(Isopropyl), -O-Methylen-N(Methyl)₂, -O-Ethylen-N(Methylh, -O-Methylen-N(Ethyl)₂, -O-Ethylen-N(Ethyl)₂, CO-NH₂, CO-NH(CH₃), CO-N(CH₃)₂, -NH-CO-Methyl, -NCH₃-CO-Methyl, -NH-CO-Ethyl, NCH₃-CO-Ethyl, Phenyl, Phenyl-Methylen-, Phenyl-Ethylen-, Het-Methylen-, Het-Ethylen-, -CO-Het, Het, -CO-C₅₋₇-Cycloalkyl, -CO-Cyclopropyl, -CO-N(CH₃)-C₅₋₇-Cycloalkyl, -CO-N(CH₃)-Cyclopropyl, C₅₋₇-Cycloalkyl, Cyclopropyl, C₅₋₇-Cycloalkyl-Methylen, C₅₋₇-Cycloalkyl-Ethylen, Cyclopropyl-Methylen, Cyclopropyl-Ethylen, Hetaryl-Methylen, Hetaryl-Ethylen und Hetaryl substituiert ist, welcher wiederum gegebenenfalls mit 1, 2, 3 oder 4 Resten unabhängig voneinander ausgewählt aus der Gruppe bestehend aus F, Cl, Br, Methyl, O-Methyl, Ethyl, O-Ethyl, OH, Oxo und CF₃ substituiert sein kann,
und worin
**R⁴** H, CN, OH, CF₃, CHF₂, CH₂F, F, Methyl, Ethyl, O-Methyl oder O-Ethyl, -Methylen-OH, -Ethylen-OH, -Propylen-OH, Isopropylen-OH, -COO(Methyl), -COO(Ethyl), -COO(Propyl), -COO(Isopropyl), -CO-Het, -(Methylen)-NH-SO₂-(Methyl), -(Methylen)-NH-SO₂-(Ethyl), -(Ethylen)-NH-SO₂-(Methyl), -(Ethylen)-NH-SO₂-(Ethyl), -(Methylen)-N(CH₃)-SO₂-(Methyl), -(Methylen)-N(CH₃)-SO₂-(Ethyl), -(Ethylen)-N(CH₃)-SO₂-(Methyl), -(Ethylen)-N(CH₃)-SO₂-(Ethyl), -(Methylen)-O-(Methylen)-Phenyl, -(Methylen)-O-(Ethylen)-Phenyl, -(Ethylen)-O-(Methylen)-Phenyl, -(Ethylen)-O-(Ethylen)-Phenyl, -Methylen-O-Methyl, -Methylen-O-Ethyl, -Ethylen-O-Methyl -Ethylen-O-Ethyl, -(Methylen)-N(CH₃)-CO-(Methyl), -(Methylen)-N(CH₃)-CO-(Ethyl) -(Ethylen)-N(CH₃)-CO-(Methyl), -(Ethylen)-N(CH₃)-CO-(Ethyl), -NH-CO-(Methylen)-O-(Methyl), -NH-CO-(Methylen)-O-(Ethyl), -NH-CO-(Ethylen)-O-(Methyl), -NH-CO-(Ethylen)-O-(Ethyl), -Methylen-NH-CO-(Methyl), -Methylen-NH-CO-(Ethyl), -Ethylen-NH-CO-(Methyl), -Ethylen-NH-CO-(Ethyl), -Methylen-NH-CO-(Methylen)-N(Methyl)₂, -Methylen-NH-CO-(Ethylen)-N(Methyl)₂, -Ethylen-NH-CO-(Methylen)-N(Methyl)₂, -Ethylen-NH-CO-(Ethylen)-N(Methyl)₂, -Methylen-NH-CO-(Methylen)-O-(Methyl), -Methylen-NH-CO-(Ethylen)-O-(Methyl), -Ethylen-NH-CO-(Methylen)-O-(Methyl), -Methylen-NH-CO-(Methylen)-O-(Ethyl), -Methylen-NH-CO-(Ethylen)-O-(Ethyl), -Ethylen-NH-CO-(Methylen)-O-(Ethyl), -(Methylen)-N(CH₃)-CO-(Methylen)-O-(Methyl), -(Methylen)-N(CH₃)-CO-(Ethylen)-O-(Methyl), -(Ethylen)-N(CH₃)-CO-(Methylen)-O-(Methyl), -(Methylen)-N(CH₃)-CO-(Methylen)-O-(Ethyl), -(Methylen)-N(CH₃)-CO-(Ethylen)-O-(Ethyl), -(Ethylen)-N(CH₃)-CO-(Methylen)-O-(Ethyl), -O-(Methylen)-Phenyl, -O-(Ethylen)-Phenyl, -CO-Phenyl, wobei das Phenyl in den obigen Resten gegebenenfalls mit einem oder mehreren weiteren Resten ausgewählt aus der Gruppe F, Cl, Br, Methyl, Ethyl, Propyl, -O-Methyl, -O-Ethyl, -O-Propyl, -OH und CF₃ substituiert sein kann
oder worin
**R³** und **R⁴** gemeinsam einen mono- oder bicyclischen, ungesättigten, gesättigten oder teilweise gesättigten Heterocyclus bilden, der 1, 2 oder 3 Heteroatome ausgewählt aus der Gruppe bestehend aus N, O und S enthält und der gegebenenfalls mit einem oder mehreren Resten ausgewählt aus der Gruppe bestehend aus F, Cl, Br, OH, Oxo, CF₃, CHF₂, CH₂F, CN, Methyl, Ethyl, Propyl, Isopropyl, Cyclopropyl, COO-Methyl, -COO-Ethyl, O-Methyl, O-Ethyl, SO₂-(CH₃), SO₂-(CH₂CH₃), SO-(CH₃), SO-(CH₂CH₃), CH₂-NH₂, CH₂-NH(CH₃), CH₂-N(CH₃)₂, -NH₂, -NH(CH₃), -N(CH₃)₂, Phenyl, C₅₋₇-Cycloalkyl, Het und Hetaryl substituiert sein kann,
sowie pharmakologisch verträgliche Salze, Diastereomere, Enantiomere, Racemate, Hydrate oder Solvate davon.

4. Verbindungen der Formel **1** nach einem der Ansprüche 1 bis 3, wobei
**R²** ein Rest nach Formel **2** 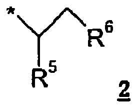 ist,
worin **R⁶** OH oder NH₂ ist und
worin **R⁵** ein Rest ausgewählt aus der Gruppe bestehend aus C₁₋₄-Alkyl, einem fünf- bis sechsgliedrigen Heteroaryl mit 1, 2 oder 3 Heteroatomen aus der Gruppe S, O und N und Phenyl, welcher gegebenenfalls mit einem oder mehreren Resten ausgewählt aus der Gruppe bestehend aus OH, F, Br, OR^{2.1}, Oxo, Methyl, Ethyl, Methanol, Ethanol, Phenyl, COOR^{2.1}, CH₂-NR^{2.2}R^{2.3} und NR^{2.2}R^{2.3} substituiert sein kann,
sowie pharmakologisch verträgliche Salze, Diastereomere, Enantiomere, Racemate, Hydrate oder Solvate davon.

5. Verbindungen der Formel **1** nach einem der Ansprüche 1 bis 3, wobei
**R²** ein monocyclischer drei-, vier-, fünf-, sechs oder siebengliedriger Cycloalkyl-Ring ist, der gegebenenfalls in spiro-Stellung mit einem Rest ausgewählt aus der Gruppe bestehend aus -CH₂-OR^{2.1}, verzweigtes oder unverzweigtes C₂₋₆-Alkylen-OR^{2.1}, Methyl, Ethyl, Propyl, Isopropyl, Butyl, Isobutyl, Cyclopropyl, -CF₃, CHF₂, CH₂F und C₂₋₄-Fluoroalkyl substituiert sein kann, wobei
R^{2.1} ausgewählt ist aus der Gruppe bestehend aus Methyl, Ethyl, Propyl, Isopropyl, Butyl, Isobutyl,
sowie pharmakologisch verträgliche Salze, Diastereomere, Enantiomere, Racemate, Hydrate oder Solvate davon.

6. Verbindungen der Formel **1** nach einem der Ansprüche 1, 2 oder 3, worin
R² ein Cyclopropyl ist, das gegebenenfalls mit einem weiteren Rest ausgewählt aus der Gruppe bestehend aus -NH₂, CH₂- NH₂, -NH(CH₃), -N(CH₃)₂, Methyl, Ethyl, Propyl, Isopropyl, -NH-CO-(tert-Butyl), -NH-CO-O-(tert-Butyl), -N(CH₃)-CO-(tert-Butyl), -N(CH₃)-CO-O-(tert-Butyl), -CF₃, -CHF₂, CH₂F, F, Cl und Br substituiert sein, sowie pharmakologisch verträgliche Salze, Diastereomere, Enantiomere, Racemate, Hydrate oder Solvate davon.

7. Verbindungen der Formel **1** nach einem der Ansprüche 1 bis 3, wobei
**R²** ein Phenyl ist, das gegebenenfalls in einer oder in beiden meta-Stellungen durch einen oder mehrere Reste ausgewählt aus der Gruppe bestehend aus Methyl, Ethyl, Propyl, Isopropyl, Cyclopropyl, F, Cl, Br, OH, OR^{2.1}, COOR^{2.1}, CF₃, CHF₂, CH₂F, NH₂, NH(CH₃) und N(CH₃)₂ substituiert sein kann, wobei R^{2.1} H, Methyl oder Ethyl, sein kann,
sowie pharmakologisch verträgliche Salze, Diastereomere, Enantiomere, Racemate, Hydrate oder Solvate davon.

8. Verbindungen der Formel **1** nach einem der Ansprüche 1 bis 3, wobei
**R²** ein Rest ausgewählt aus einer Gruppe bestehend aus monocyclischen, gesättigten drei-, vier-, fünf-, sechs- oder siebengliedrigem Heterocyclus mit 1, 2 oder 3 Heteroatomen jeweils ausgewählt aus der Gruppe bestehend aus N, O und S, der gegebenenfalls durch einen oder mehrere Reste ausgewählt aus der Gruppe Fluor, Chlor, Brom, CF₃, CHF₂, CH₂F, OH und Oxo oder durch einen oder mehrere Reste ausgewählt aus der Gruppe OR^{2.1}, C₁₋₃-alkylen-OR^{2.1} SR^{2.1},SO-R^{2.1}, SO₂-R^{2.1}, COOR^{2.1}, COR^{2.1}, C₁₋₆-Alkanol, C₃₋₁₀-Cycloalkyl, Phenyl, C₁₋₆-Alkyl, Phenyl-C₁₋₆-alkylen, Hetaryl-C₁₋₆-alkylen, Het, Hetaryl und NR^{2.2}R^{2.3} substituiert sein kann, der gegebenenfalls wiederum durch einen oder mehrere Reste ausgewählt aus der Gruppe bestehend aus OH, OR^{2.1}, Oxo, F, Cl, CF₃, CHF, CH₂F, C₁₋₆-Alkyl, Phenyl und NR^{2.2}R^{2.3} substituiert sein kann,
wobei **R^{2.1}**, **R^{2.2}** und **R^{2.3}** wie in Anspruch 1 definiert sind,
sowie pharmakologisch verträgliche Salze, Diastereomere, Enantiomere, Racemate, Hydrate oder Solvate davon.

9. Verbindungen der Formel **1** nach Anspruch 8, wobei
**R²** ein Rest ausgewählt aus einer Gruppe bestehend aus einem monocyclischen, gesättigten sechsgliedrigem Heterocyclus mit einem Heteroatom ausgewählt aus der Gruppe bestehend aus N, O und S ist, der gegebenenfalls durch einen oder mehrere Reste ausgewählt aus der Gruppe F, Cl, Br, CF₃, CHF₂, CH₂F, OH, Oxo, NH₂, NHCH₃ und N(CH₃)₂, Methyl, Ethyl, Propyl, Isopropyl, Cyclopropyl, Methoxy und Ethoxy substituiert sein kann,
sowie pharmakologisch verträgliche Salze, Diastereomere, Enantiomere, Racemate, Hydrate oder Solvate davon.

10. Verbindungen der Formel **1** nach Anspruch 8 oder 9, wobei
**R**² ein Rest ausgewählt aus einer Gruppe bestehend aus Piperidin oder Tetrahydropyran, der gegebenenfalls durch einen oder mehrere Reste ausgewählt aus der Gruppe F, Cl, Br, OH, CF₃, CHF₂, CH₂F, NH₂, NHCH₃, N(CH₃)₂, Oxo, Methyl und Methoxy substituiert sein kann,
sowie pharmakologisch verträgliche Salze, Diastereomere, Enantiomere, Racemate, Hydrate oder Solvate davon.

11. Verbindungen der Formel **1** nach einem der Ansprüche 1 bis 3, wobei
**R³** ein Naphthalin oder Phenyl ist, welches gegebenenfalls in beliebiger Stellung mit einem, zwei oder drei Resten unabhängig voneinander ausgewählt aus der Gruppe bestehend aus Fluor, Chlor, Brom, Hydroxy, CN, Methyl, Ethyl, Propyl, Isopropyl, Cyclopropyl, CF₃, CHF₂, CH₂F,-OCH₃, OCH₂CH₃; SO₂-CH₃, SO₂-CH₂CH₃, COOCH₃ und CO-O-CH₂CH₃ substituiert sein kann,
sowie pharmakologisch verträgliche Salze, Diastereomere, Enantiomere, Racemate, Hydrate oder Solvate davon.

12. Verbindungen der Formel **1** nach einem der Ansprüche 1 oder 2, wobei
**R³** ein Rest ausgewählt aus der Gruppe bestehend aus Het und Hetaryl, der gegebenenfalls durch einen oder mehrere Reste ausgewählt aus der Gruppe bestehend aus F, Cl, Br, CF₃, CHF₂, CH₂F, CN, OH, Oxo, Methyl, Ethyl, Propyl, Isopropyl, Butyl, Isobutyl, Cyclopropyl, C₆₋₇-Cycloalkyl, -O-Methyl, -O-Ethyl, -O-Propyl, -O-Isopropyl, -COO-Methyl, -COO-Ethyl, -COO-Propyl, -COO-Isopropyl, SO₂-(CH₃), SO₂-(CH₂-CH₃), SO-(CH₃). SO-(CH₂-CH₃), Phenyl, -CH₂-NH₂, -CH₂-NHCH₃, -CH₂-N(CH₃)₂, NH₂, NHCH₃, N(CH₃)₂, Het und Hetaryl substituiert sein kann, der wiederum mit einem oder mehreren Resten ausgewählt aus der Gruppe OH, F, Cl, Br, CF₃, CHF₂, CH₂F, Methyl, Ethyl, Propyl, isopropyl, Phenyl, -COO-Methyl, -COO-Ethyl, -COO-Propyl, -COO-Isopropyl und O-Methyl, O-Ethyl, O-Propyl, und O-Isopropyl substituiert sein kann,
und worin
**R⁴** H, CN, OH, CF₃, CHF₂, CH₂F, F, Methyl, Ethyl, O-Methyl oder O-Ethyl,
wobei
**Het** ein drei- bis siebengliedriger, monocyclischer, gesättigter oder teilweise gesättigter Heterocyclus oder ein sieben- bis elfgliedriger, bicyclischer, annellierter, gesättigter oder teilweise gesättigter Heterocyclus ist, der 1, 2 oder 3 Heteroatome unabhängig voneinander ausgewählt aus der Gruppe bestehend aus N, S oder O enthält,
und wobei
**Hetaryl** ein fünf- bis sechsgliedriges, monocyclisches, aromatisches Heteroaryl oder ein sieben- bis elfgliedriges, bicyclisches, annelliertes, aromatisches Heteroaryl ist, das jeweils 1, 2 oder 3 Heteroatome unabhängig voneinander ausgewählt aus der Gruppe bestehend aus N, S oder O enthält,
und wobei
Cycloalkyl gesättigt oder teilweise gesättigt sein kann,
sowie pharmakologisch verträgliche Salze, Diastereomere, Enantiomere Racemate, Hydrate oder Solvate davon.

13. Verbindungen der Formel 1 nach Anspruch 12, wobei
R³ ein Rest ausgewählt aus einem bicyclischen, sieben- bis elfgliedrigem, gesättigten oder teilweise gesättigten Heterocyclus oder einem bicyclischen, sieben- bis elfgliedrigem Heteroaryl ist, der ausgewählt ist aus der Gruppe bestehend aus Indol, Dihydroindol, Chinazolin, Dihydrochinazolin, Tetrahydrochinazolin, Benzoisoxazol, Dihydrobenzoisoxazol, Benzooxazin, Dihydrobenzooxazin, Benzothiazol, Dihydrobenzothiazol, Triazolopyridin, Dihydrotriazolopyridin, Benzofuran, Dihydrobenzofuran, Isobenzofuran und Dihydroisobenzofuran, welcher gegebenenfalls mit einem oder mehreren Resten ausgewählt aus der Gruppe bestehend aus aus F, Cl, Br, CF₃, CHF₂, CH₂F, CN, OH, Oxo, Methyl, Ethyl, Propyl, Isopropyl, Butyl, Isobutyl, Cyclopropyl, -O-Methyl, -O-Ethyl, -O-Propyl, -O-Isopropyl,-COO-Methyl, -COO-Ethyl, -COO-Propyl, -COO-Isopropyl, SO₂-(CH₃), SO₂-(CH₂-CH₃), SO-(CH₃), SO-(CH₂-CH₃), Phenyl, -CH₂-NH₂, -CH₂-NHCH₃, -CH₂-N(CH₃)₂, NH₂, NHCH₃, N(CH₃)₂, Furanyl und Pyridinyl substituiert sein kann, der wiederum mit einem oder mehreren Resten ausgewählt aus der Gruppe OH, F, Cl, Br, CF₃, CHF₂, CH₂F, Methyl, Ethyl, Propyl, Isopropyl, Phenyl, -COO-Methyl,-COO-Ethyl und O-Methyl, O-Ethyl substituiert sein kann,
sowie pharmakologisch verträgliche Salze, Diastereomere, Enantiomere, Racemate, Hydrate oder Solvate davon.

14. Verbindungen der Formel **1** nach Anspruch 12, wobei
R³ ein Rest ausgewählt aus einem monocyclischen, gesättigten oder teilweise gesättigten, drei- bis siebengliedrigem Heterocyclus oder einem monocyclischen fünf-bis sechsgliedrigem Heteroaryl ist, der ausgewählt ist aus der Gruppe bestehend aus Imidazol, Dihydroimidazol, Oxadiazol, Oxadiazolidin, Pyrazol, Pyridin und Dihydropyrazol, welcher gegebenenfalls mit einem oder mehreren Resten ausgewählt aus der Gruppe bestehend aus aus F, Cl, Br, CF₃, CHF₂, CH₂F, CN, OH, Oxo, Methyl, Ethyl, Propyl, Isopropyl, Butyl, Isobutyl, Cyclopropyl, -O-methyl, -O-Ethyl, -O-Propyl, -O-Isopropyl,-COO-Methyl, -COO-Ethyl, -COO-Propyl, -COO-Isopropyl, SO₂-(CH₃), SO₂-(CH₂-CH₃), SO-(CH₃), SO-(CH₂-CH₃), Phenyl, -CH₂-NH₂, -CH₂-NHCH₃, -CH₂-N(CH₃)₂, NH₂, NHCH₃, N(CH₃)₂, Furanyl und Pyridinyl substituiert sein kann, der wiederum mit einem oder mehreren Resten ausgewählt aus der Gruppe OH, F, Cl, Br, CF₃, CHF₂, CH₂F, Methyl, Ethyl, Propyl, Isopropyl, Phenyl, -COO-Methyl, -COO-Ethyl und O-Methyl, O-Ethyl substituiert sein kann,
sowie pharmakologisch verträgliche Salze, Diastereomere, Enantiomere, Racemate, Hydrate oder Solvate davon.

15. Verbindungen der Formel 1 nach einem der Ansprüche 1 bis 3, wobei
**R³** und **R⁴** gemeinsam einen mono- oder bicyclischen, ungesättigten oder teilweise gesättigten, drei- bis elfgliedrigen Heterocyclus bilden, der 1, 2 oder 3 Heteroatome ausgewählt aus der Gruppe bestehend aus N, O und S enthält und der gegebenenfalls mit einem oder mehreren Resten ausgewählt aus der Gruppe bestehend aus F, Cl, Br, OH, Oxo, CF₃, CHF₂, CH₂F, CN, Methyl, Ethyl, Propyl, Isopropyl, Cyclopropyl, COO-Methyl, -COO-Ethyl, O-Methyl, O-Ethyl, SO₂-(CH₃), SO₂-(CH₂-CH₃), SO-(CH₃), SO-(CH₂-CH₃), Phenyl, -CH₂-NH₂, -CH₂NHCH₃, -CH₂-N(CH₃)₂, NH₂, NHCH₃, N(CH₃)₂, einem gesättigtem oder teilweise gesättigtem, fünf- bis sechsgliedrigem Heterocyclus und einem fünf- bis sechsgliedrigem Heteroaryl substituiert sein kann,
sowie pharmakologisch verträgliche Salze, Diastereomere, Enantiomere, Racemate, Hydrate oder Solvate davon.

16. Verbindungen der Formel 1 nach Anspruch 15, wobei
R³ und R⁴ gemeinsam einen bicyclischen Heterocyclus ausgewählt aus der Gruppe bestehend aus Tetrahydrochinazolin, Tetrahydrobenzoxazin und Dihydroindol, Dihydroisobenzofuran bilden, der gegebenenfalls mit einem oder mehreren Resten ausgewählt aus der Gruppe bestehend aus F, Cl, Br, OH, Oxo, CF₃, CHF₂, CH₂F, CN, Methyl, Ethyl, Propyl, Isopropyl, Cyclopropyl, COO-Methyl, -COO-Ethyl, O-Methyl, O-Ethyl, SO₂-(CH₃), SO₂-(CH₂-CH₃), Phenyl, -CH₂-NH₂, -CH₂NHCH₃, -CH₂-N(CH₃)₂, NH₂, NHCH₃, N(CH₃)₂, einem gesättigtem oder teilweise gesättigen, fünf- oder sechsgliedrigem Heterocyclus und einem fünf- oder sechsgliedrigem Heteroaryl substituiert sein kann,
sowie pharmakologisch verträgliche Salze, Diastereomere, Enantiomere, Racemate, Hydrate oder Solvate davon.

17. Verbindungen der Formel 1 nach einem der Ansprüche 1 oder 2,
wobei
R³ -O-R^{3.1} ist,
wobei R^{3.1} ein Rest ausgewählt aus der Gruppe bestehend aus Methyl, Ethyl, Propyl, Isopropyl, Butyl, Isobutyl, Pentyl, Isopentyl, -Phenyl, -Methylen-Phenyl, -Ethylen-Phenyl, -Propylen-Phenyl, -Isopropylen-Phenyl, Hetaryl und Het ist, welcher gegebenenfalls in ortho, para oder meta-Stellung mit einem, zwei oder drei Resten unabhängig voneinander ausgewählt aus der Gruppe bestehend aus Fluor, Chlor, Brom, Hydroxy, CN, Methyl, Ethyl, Propyl, Isopropyl, Butyl, Isobutyl, -CF₃, CHF₂, CH₂F, CO-(Methyl), CO-(Ethyl), CO-(Propyl), CO-(Isopropyl), CO-(Butyl), CO-(Isobutyl), -CO-(CF₃), -CO-(CH₂F), -CO-(CHF₂), -CO-NH-(Methylen)-Hetaryl, -CO-NH-(Ethylen)-Hetaryl, -CO-NH-(Propylen)-Hetaryl, -CO-NH-(Isopropylen)-Hetaryl, -CO-N(CH₃)-(Methylen)-Hetaryl, -CO-N(CH₃)-(Ethylen)-Hetaryl, -CO-N(CH₃)-(Propylen)-Hetaryl, -CO-N(CH₃)-(Isopropylen)-Hetaryl, -CO-N(CH₃)-Het, -CO-N(C₃₋₇-Cycloalkyl)-Het, -Methylen-O-Methyl, -Ethylen-O-Methyl, -Methylen-O-Ethyl, -Ethylen-O-Ethyl, -Methylen-NH₂, -Ethylen-NH₂, -Methylen-NHCH₃, -Ethylen-NHCH₃, -Methylen-N(CH₃)₂, -Ethylen-N(CH₃)₂, -NH₂, -NHCH₃, -N(CH₃)₂, -O-Methyl, -O-Ethyl, -O-Propyl, -O-Isopropyl, -SO-CH₃, -SO-(CH₂CH₃), -SO₂-CH₃, -SO₂-(CH₂CH₃), COOH, COO-(Methyl), COO-(Ethyl), COO-(Propyl), COO-(Isopropyl), -O-Methylen-N(Methyl)₂, -O-Ethylen-N(Methyl)₂,-O-Methylen-N(Ethyl)₂, -O-Ethylen-N(Ethyl)₂, CO-NH₂, CO-NHCH₂, CO-N(CH₃)₂, NH-CO-Methyl, NCH₃-CO-Methyl, NH-CO-Ethyl, N(CH₃)-CO-Ethyl, Phenyl, Phenyl-Methylen-, Phenyl-Ethylen-, Het-Methylen-, Het-Ethylen-, -CO-Het, Het, -CO-C₄₋₇-Cycloalkyl, -CO-Cyclopropyl, -CO-N(CH₃)-Cyclopropyl, -CO-N(CH₃)-C₄₋₇-Cycloalkyl, C₄₋₇-Cycloalkyl, Cyclopropyl, C₄₋₇-Cycloalkyl-Methylen-, Cyclopropyl-Methylen-, C₄₋₇-Cycloalkyl-Ethylen-, Cyclopropyl-Ethylen-, Hetaryl-Methylen-, Hetaryl-Ethylen- und Hetaryl substituiert ist, welcher wiederum gegebenenfalls mit 1, 2, 3 oder 4 Resten unabhängig voneinander ausgewählt aus der Gruppe bestehend aus F, Cl, Br, Methyl, O-Methyl, Ethyl, O-Ethyl, OH, Oxo und CF₃ substituiert sein kann,
sowie pharmakologisch verträgliche Salze, Diastereomere, Enantiomere, Racemate, Hydrate oder Solvate davon.

18. Verbindungen der Formel 1 nach einem der Ansprüche 1, 2 oder 3,
worin
R⁴ H, CN, OH, CF₃, CHF₂, CH₂F, F, Methyl, Ethyl, O-Methyl oder O-Ethyl, -Methylen-OH, -Ethylen-OH, -Propylen-OH, Isopropylen-OH, -COO(Methyl), -COO(Ethyl), -COO(Propyl), -COO(Isopropyl), -CO-Het, -(Methylen)-NH-SO₂-(Methyl), -(Methylen)-NH-SO₂-(Ethyl), -(Ethylen)-NH-SO₂-(Methyl), -(Ethylen)-NH-SO₂-(Ethyl), -(Methylen)-N(CH₃)-SO₂-(Methyl), -(Methylen)-N(CH₃)-SO₂-(Ethyl), -(Ethylen)-N(CH₃)-SO₂-(Methyl), -(Ethylen)-N(CH₃)-SO₂-(Ethyl), -(Methylen)-O-(Methylen)-Phenyl, -(Methylen)-O-(Ethylen)-Phenyl, -(Ethylen)-O-(Methylen)-Phenyl, -(Ethylen)-O-(Ethylen)-Phenyl, -Methylen-O-Methyl, -Methylen-O-Ethyl, -Ethylen-O-Methyl -Ethylen-O-Ethyl, -(Methylen)-N(CH₃)-CO-(Methyl), -(Methylen)-N(CH₃)-CO-(Ethyl) -(Ethylen)-N(CH₃)-CO-(Methyl), -(Ethylen)-N(CH₃)-CO-(Ethyl), -NH-CO-(Methylen)-O-(Methyl), -NH-CO-(Methylen)-O-(Ethyl), -NH-CO-(Ethylen)-O-(Methyl), -NH-CO-(Ethylen)-O-(Ethyl), -Methylen-NH-CO-(Methyl), -Methylen-NH-CO-(Ethyl), -Ethylen-NH-CO-(Methyl), -Ethylen-NH-CO-(Ethyl), -Methylen-NH-CO-(Methylen)-N(Methyl)₂, -Methylen-NH-CO-(Ethylen)-N(Methyl)₂, -Ethylen-NH-CO-(Methylen)-N(Methyl)₂, -Ethylen-NH-CO-(Ethylen)-N(Methyl)₂, -Methylen-NH-CO-(Methylen)-O-(Methyl), -Methylen-NH-CO-(Ethylen)-O-(Methyl), -Ethylen-NH-CO-(Methylen)-O-(Methyl), -Methylen-NH-CO-(Methylen)-O-(Ethyl), -Methylen-NH-CO-(Ethylen)-O-(Ethyl), -Ethylen-NH-CO-(Methylen)-O-(Ethyl), -(Methylen)-N(CH₃)-CO-(Methylen)-O-(Methyl), -(Methylen)-N(CH₂)-CO-(Ethylen)-O-(Methyl), -(Ethylen)-N(CH₃)-CO-(Methylen)-O-(Methyl), -(Methylen)-N(CH₃)-CO-(Methylen)-O-(Ethyl), -(Methylen)-N(CH₃)-CO-(Ethylen)-O-(Ethyl), -(Ethylen)-N(CH₃)-CO-(Methylen)-O-(Ethyl), -O-(Methylen)-Phenyl, -O-(Ethylen)-Phenyl, -CO-Phenyl,
wobei das Phenyl in den obigen Resten gegebenenfalls mit einem oder mehreren weiteren Resten ausgewählt aus der Gruppe F, Cl, Br, Methyl, Ethyl, Propyl, -O-Methyl, -O-Ethyl, -O-Propyl, -OH und CF₃ substituiert sein kann
sowie pharmakologisch verträgliche Salze, Diastereomere, Enantiomere, Racemate, Hydrate oder Solvate davon.

19. Verbindungen der Formel **1** nach einem der Ansprüche 1, 2 oder 3,
worin
R³ ein Rest ausgewählt ist aus der Gruppe bestehend aus Oxazol, Imidazol und Thiazol, wobei dieser Rest gegebenenfalls durch einen, zwei oder drei weitere Reste unabhängig voneinander ausgewählt aus der Gruppe bestehend aus Methyl, Ethyl, Propyl, Isopropyl, O-Methyl, O-Ethyl, O-Propyl, O-Isopropyl, OH, F, Cl, Br, CF₃, Phenyl, Hetaryl und C₃₋₆-Cycloalkyl substituiert sein kann,
sowie pharmakologisch verträgliche Salze davon.

20. Verbindungen der Formel **1** nach einem der Ansprüche 1 bis 3, wobei die Verbindungen ausgewählt sind aus der Gruppe bestehend aus 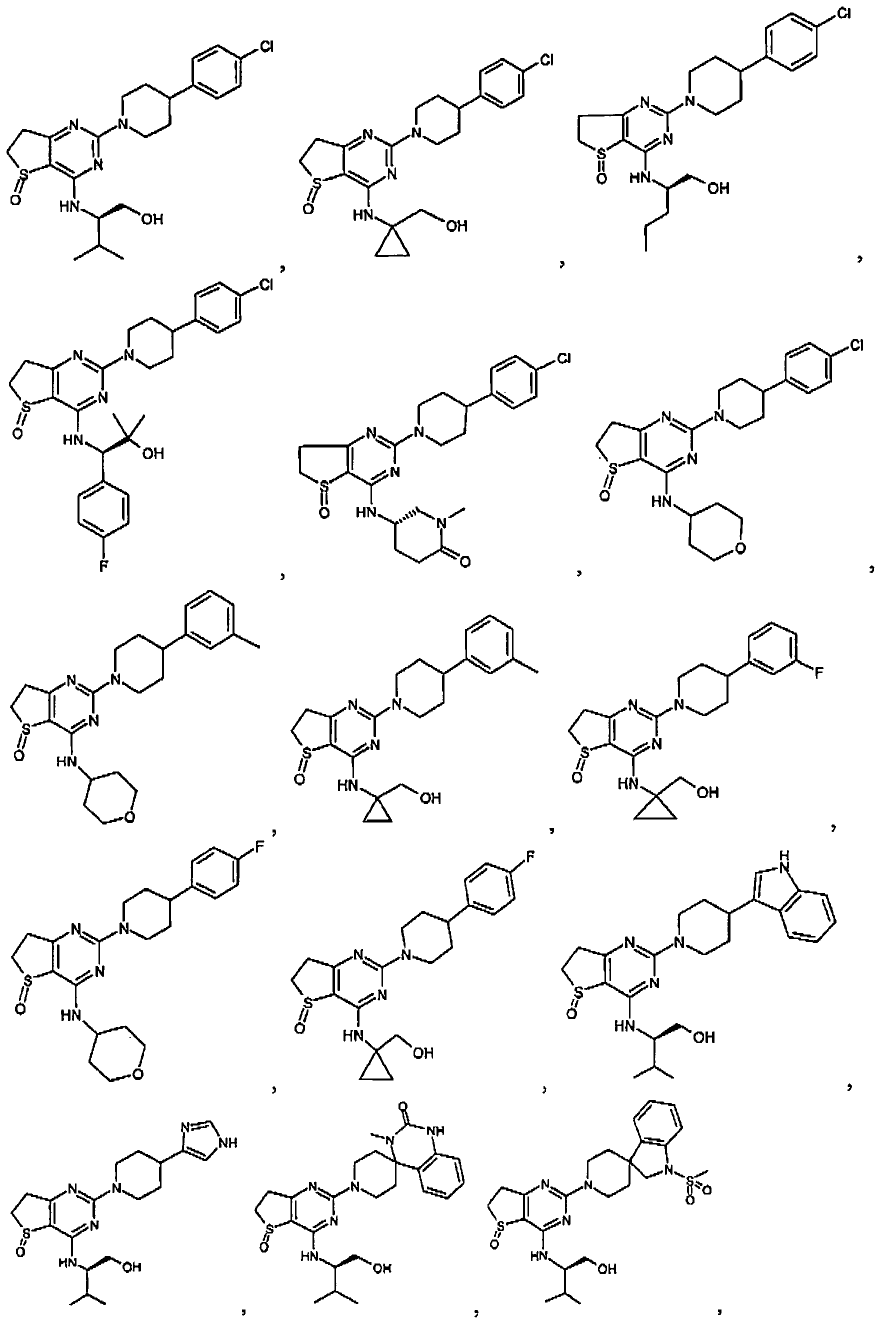 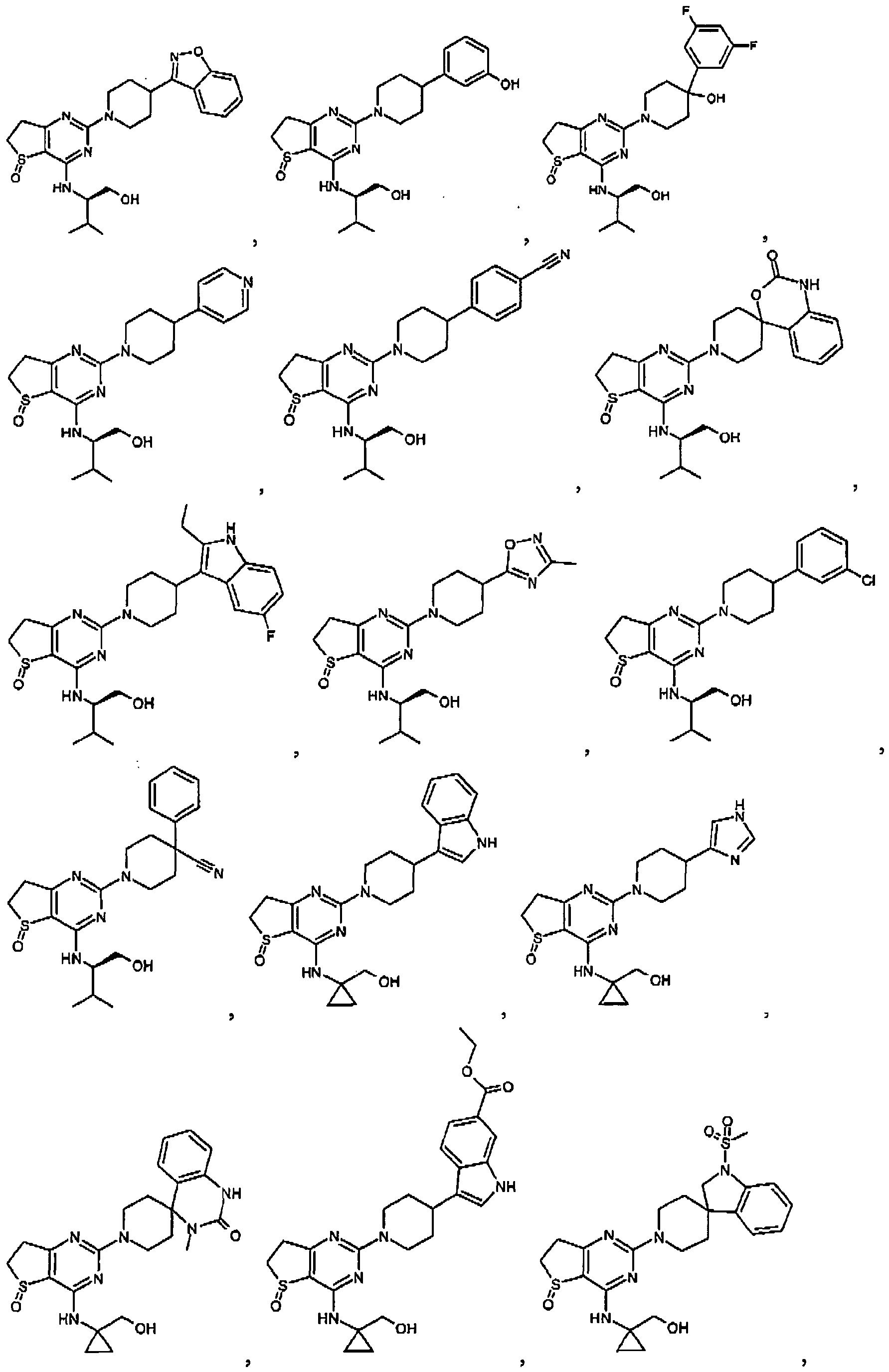 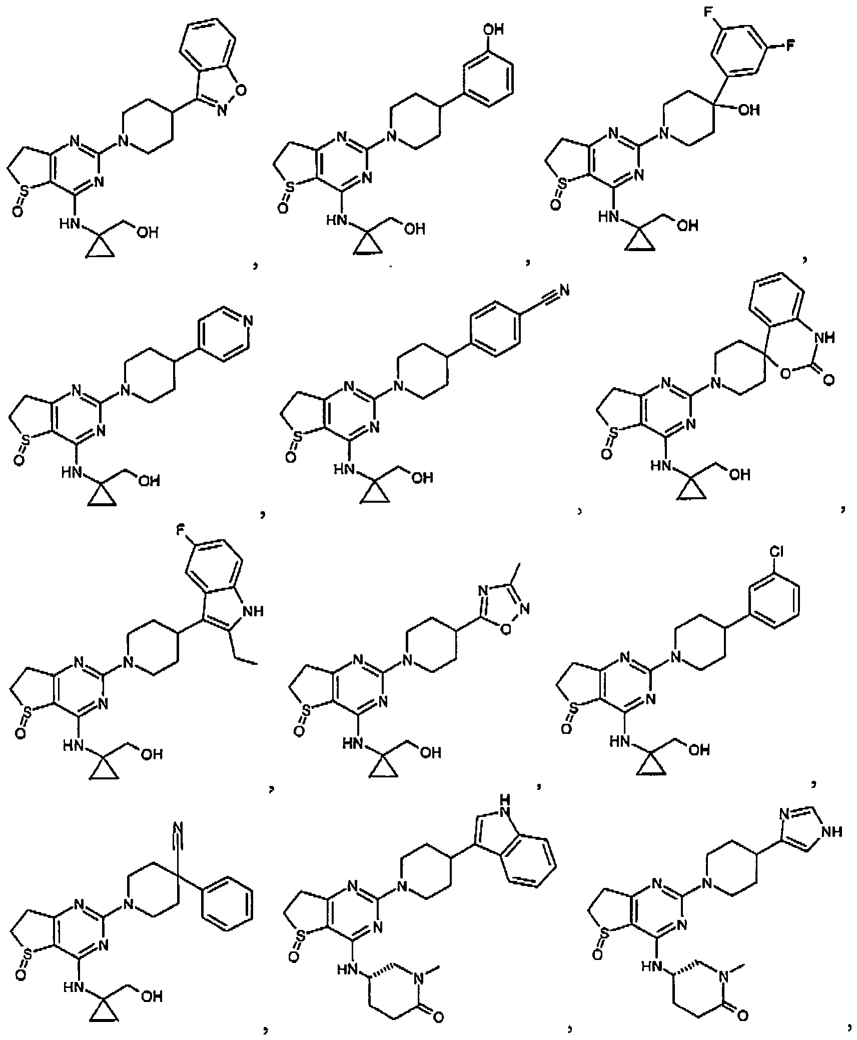 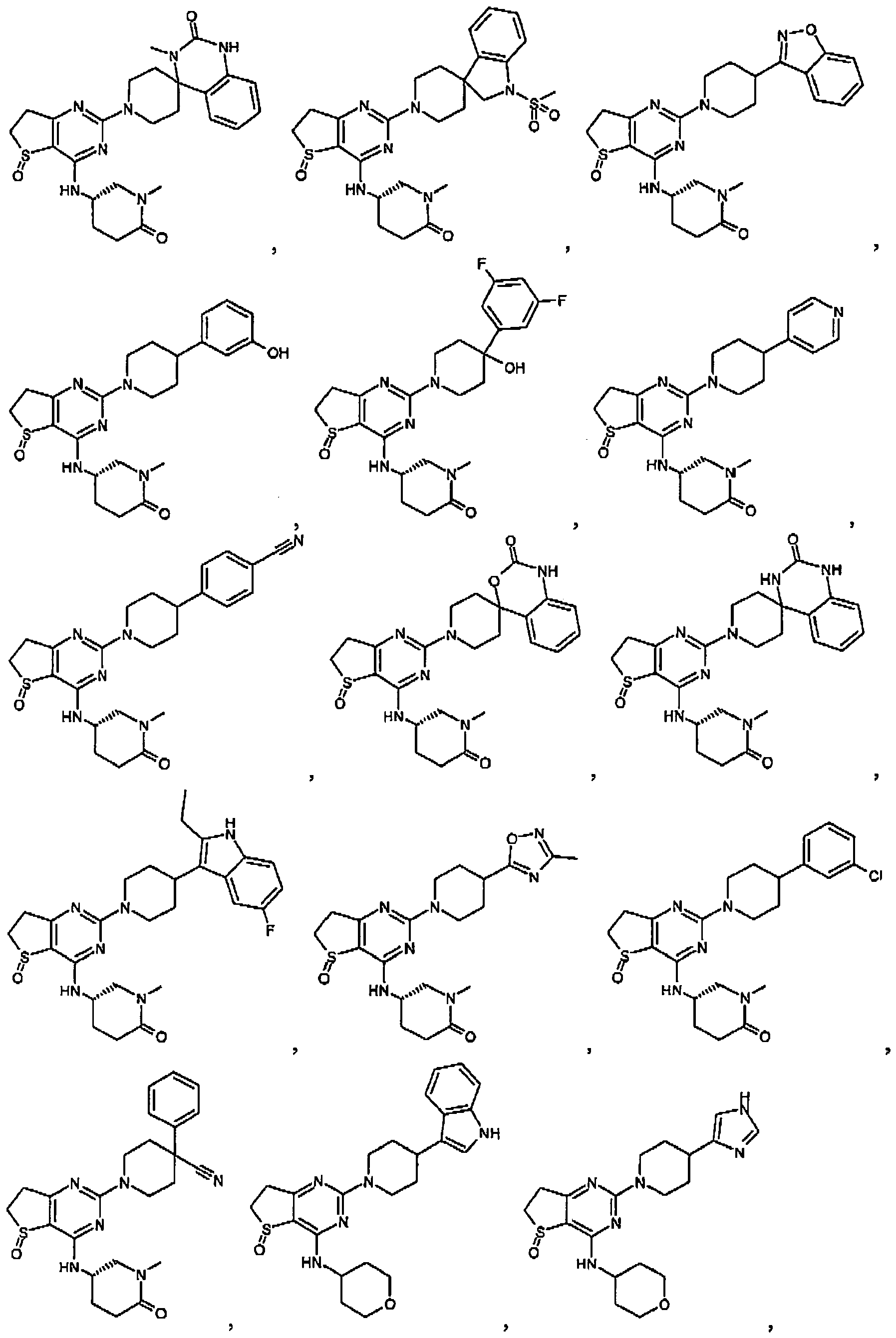 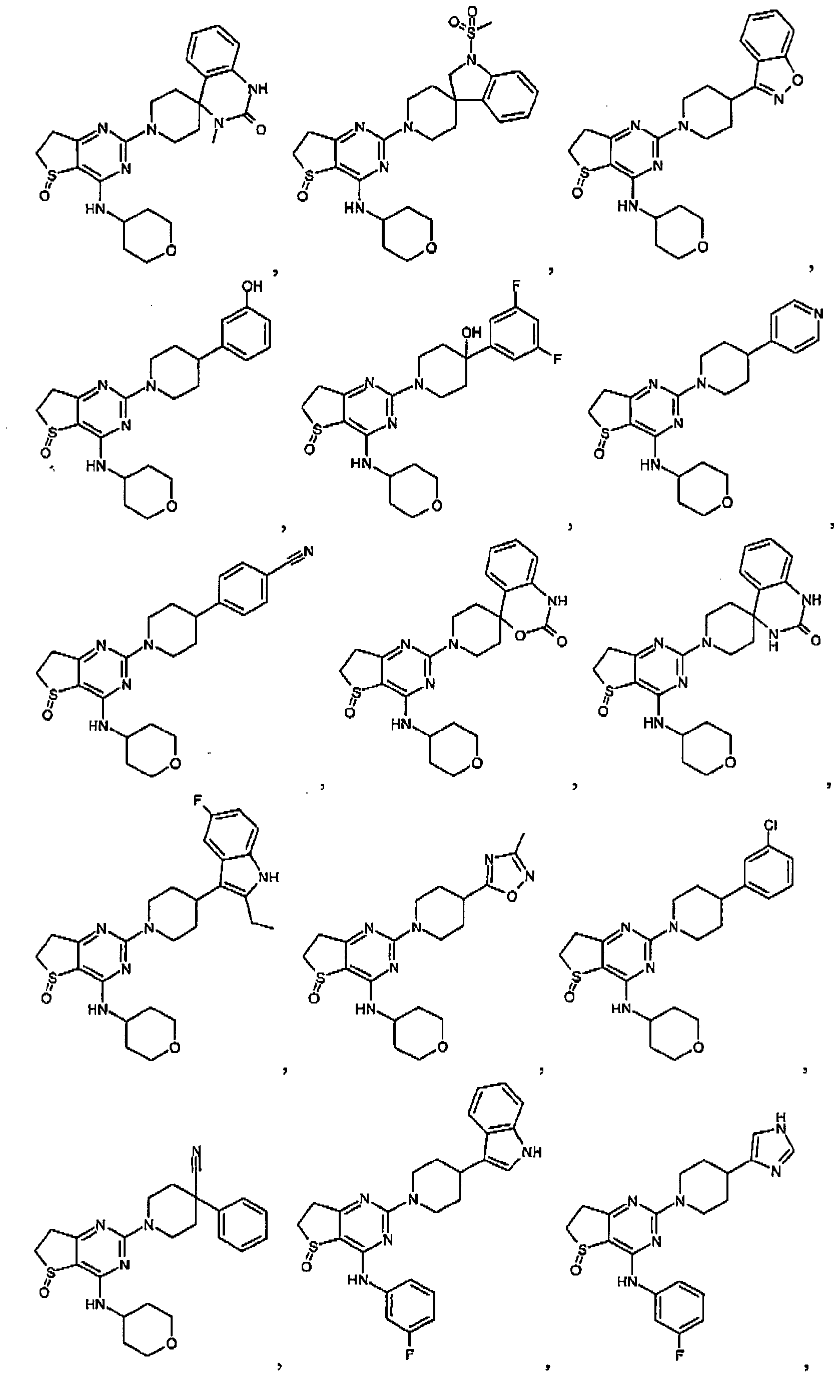 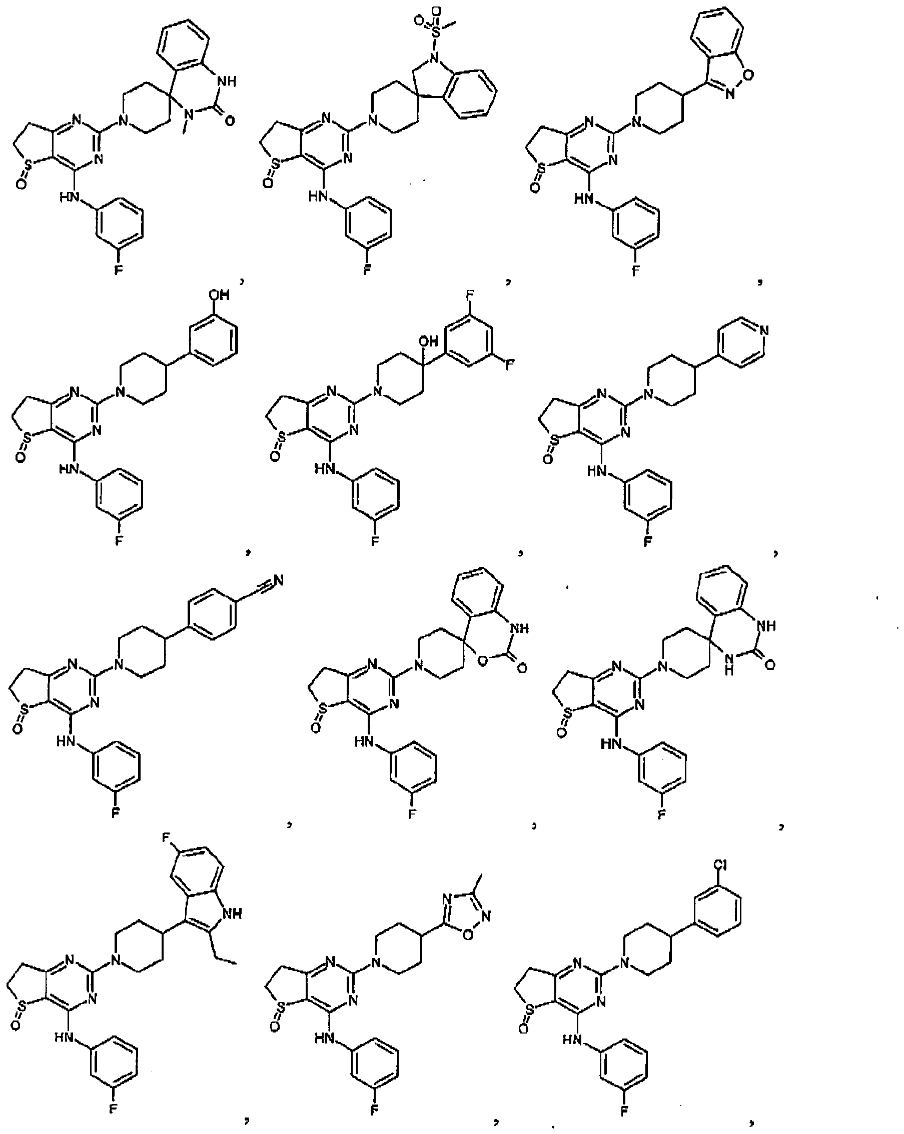 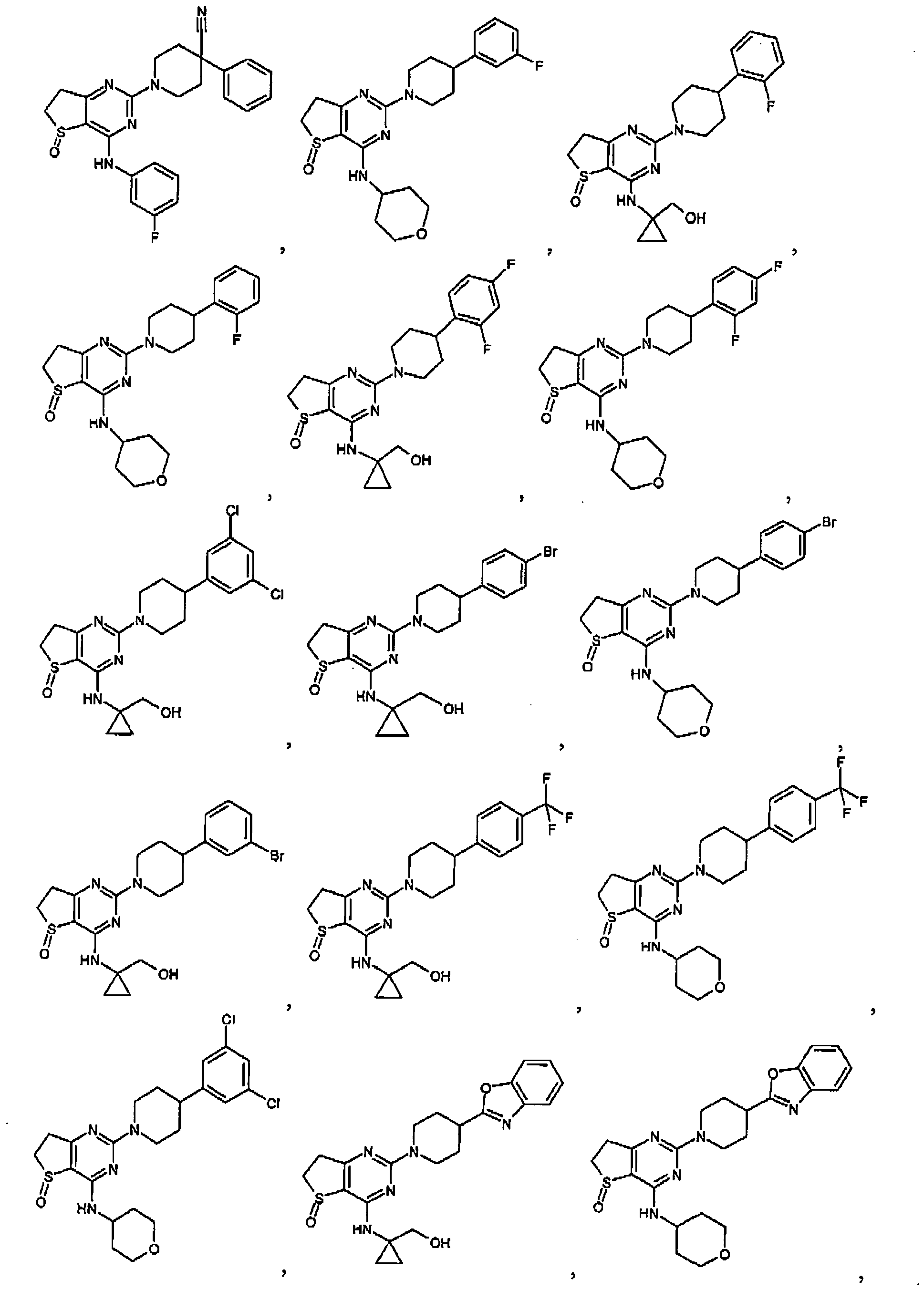 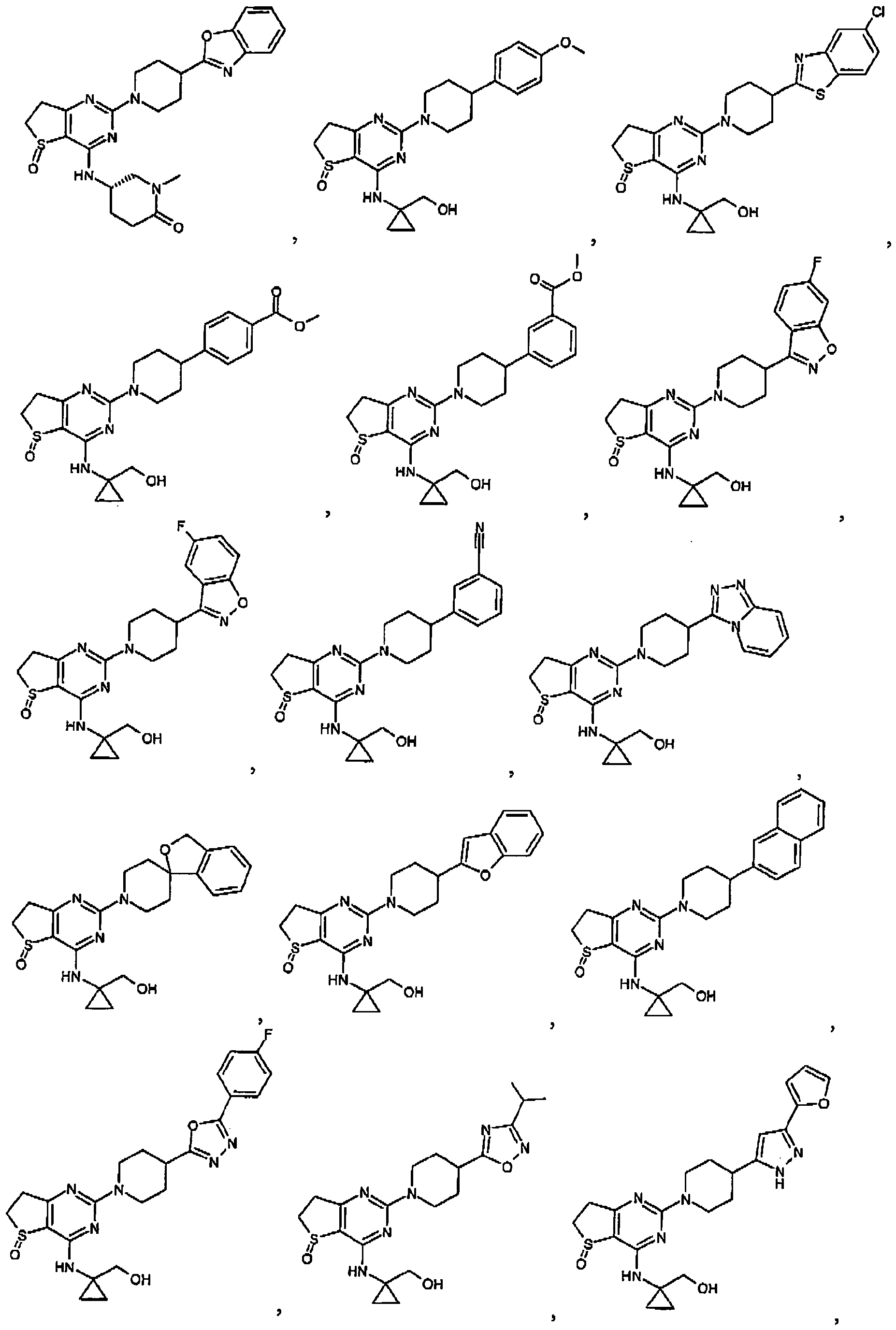 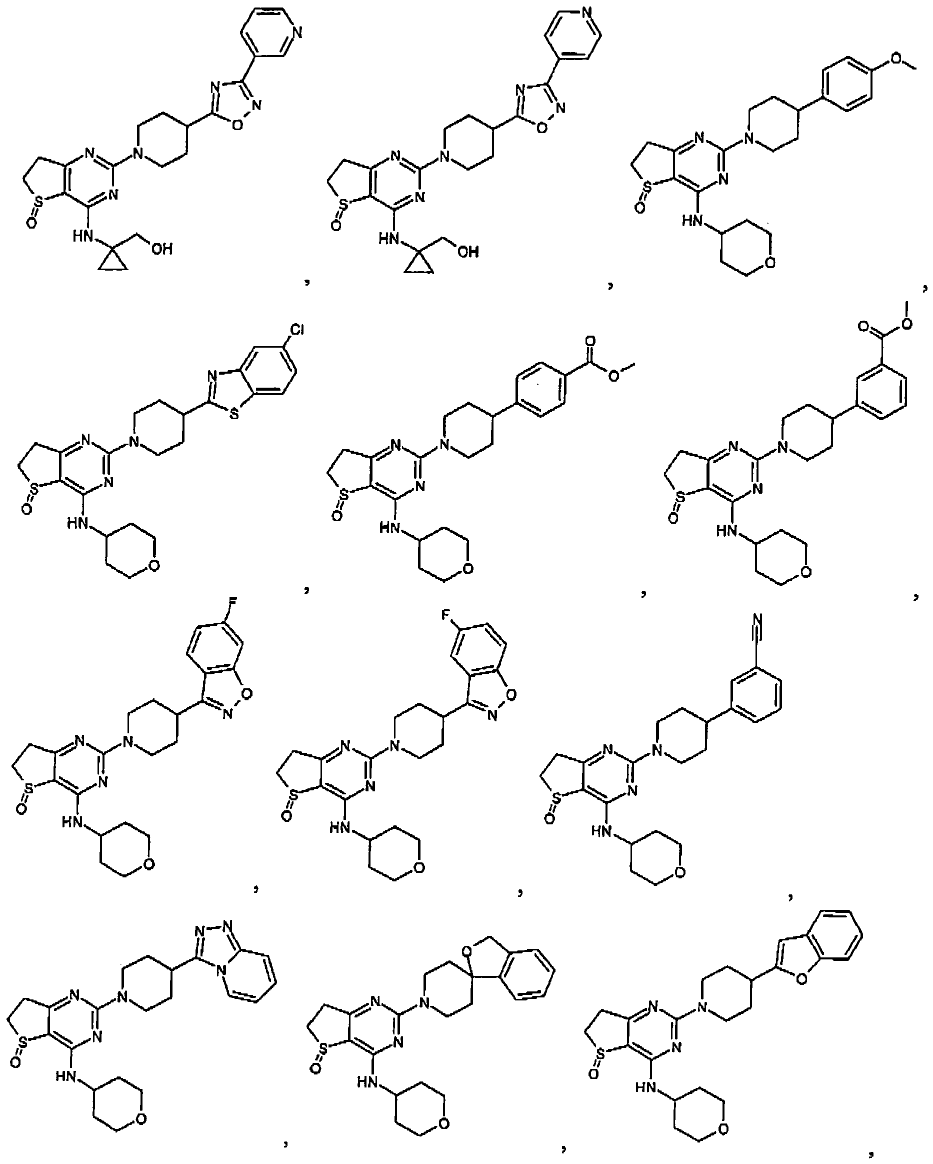 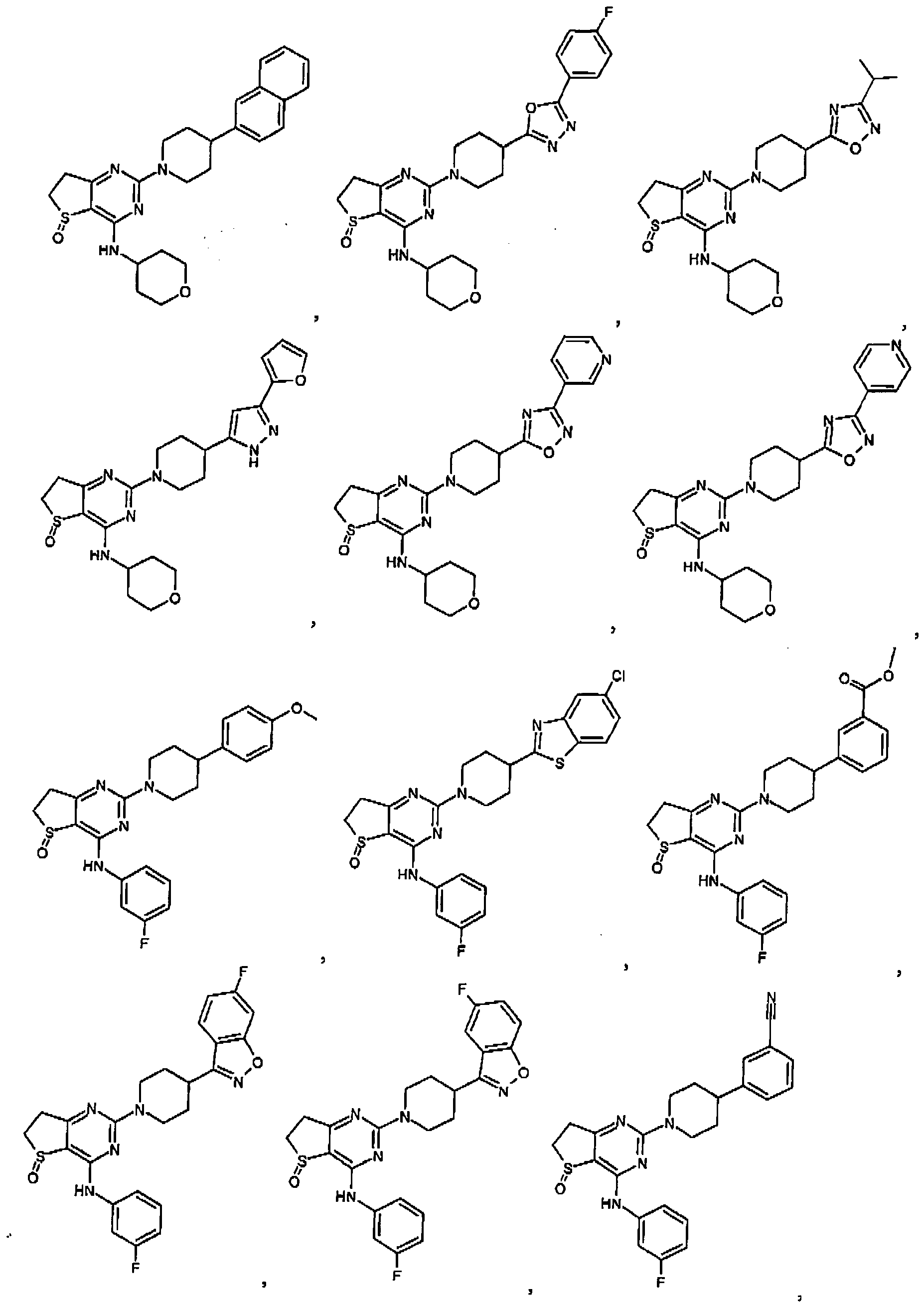 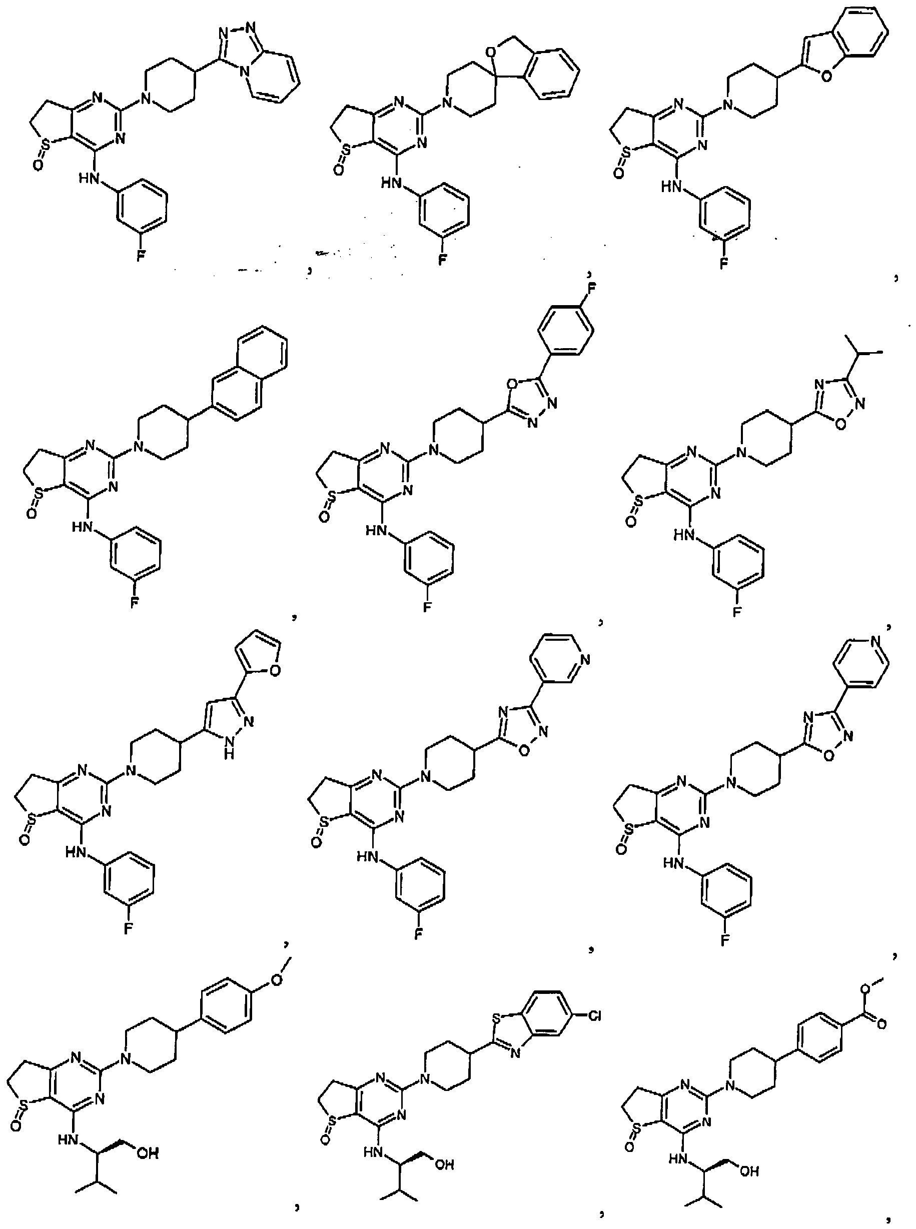 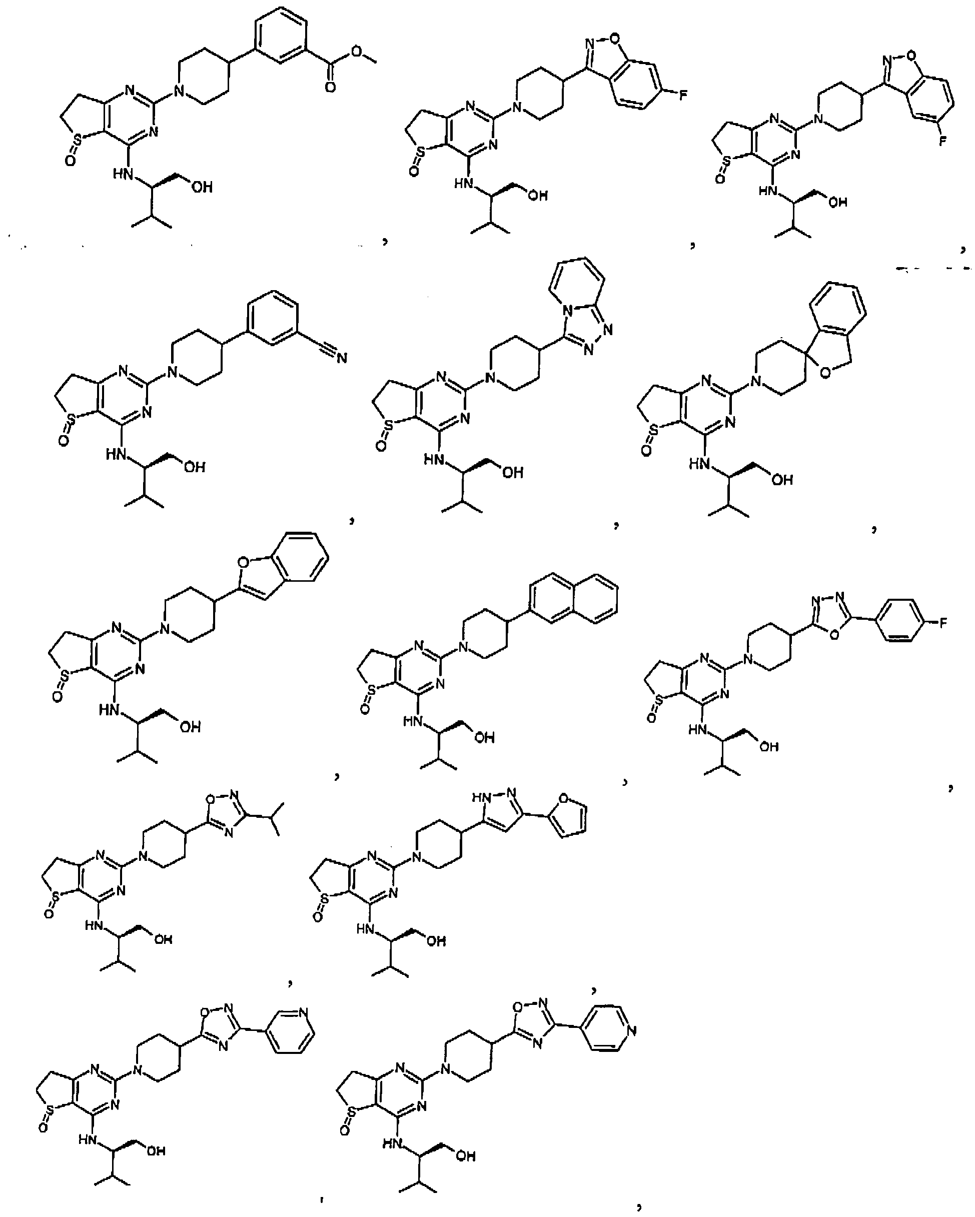 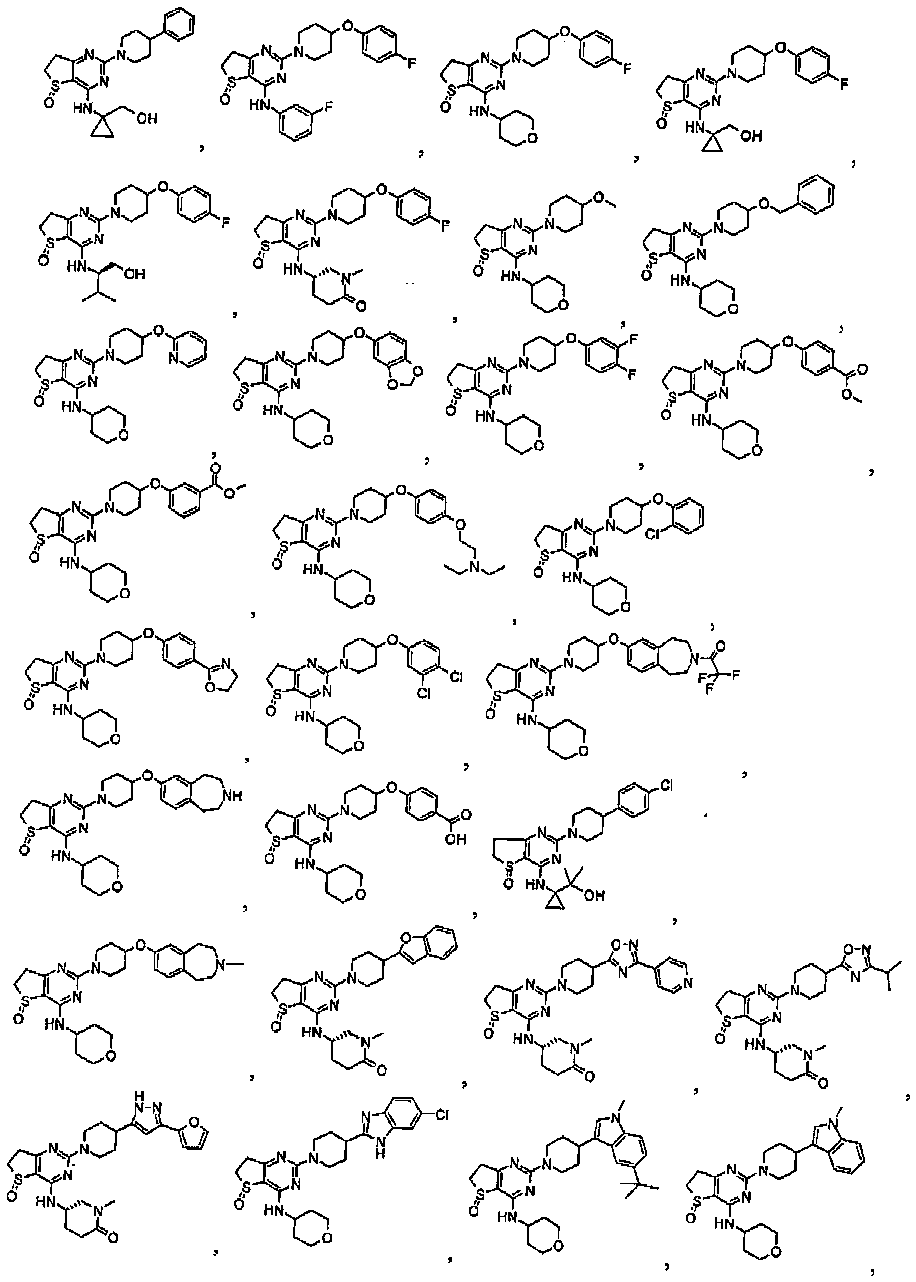 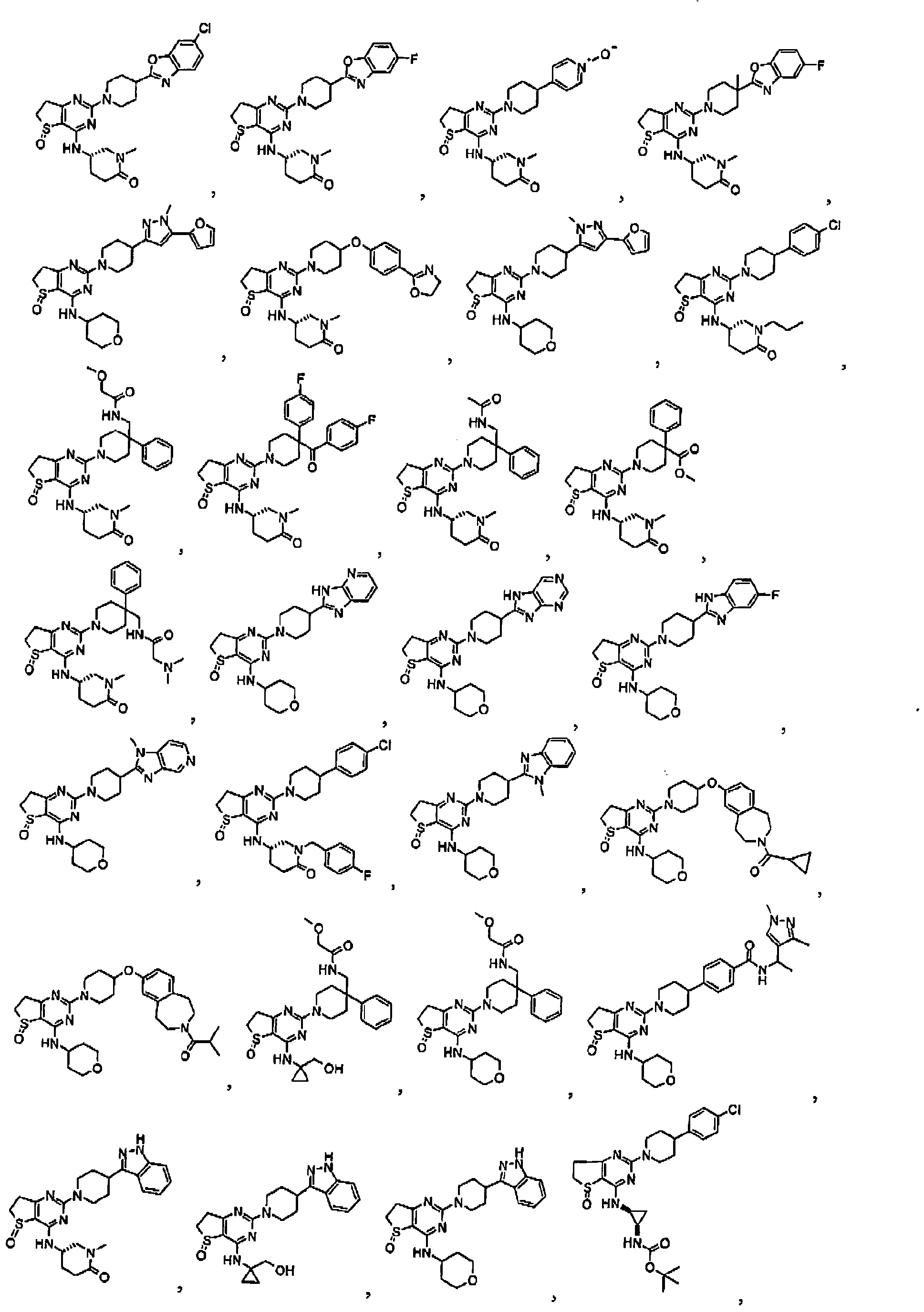 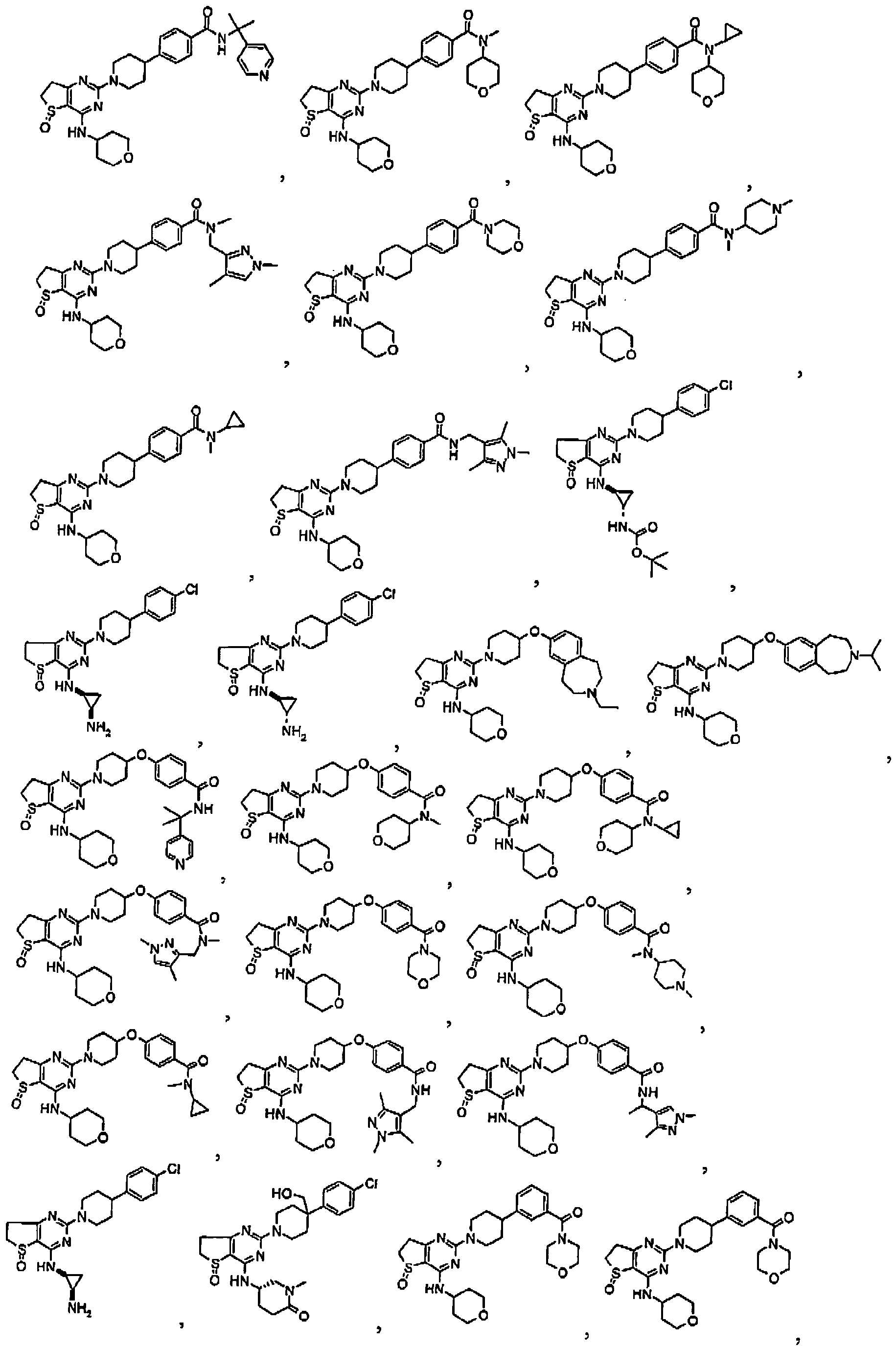 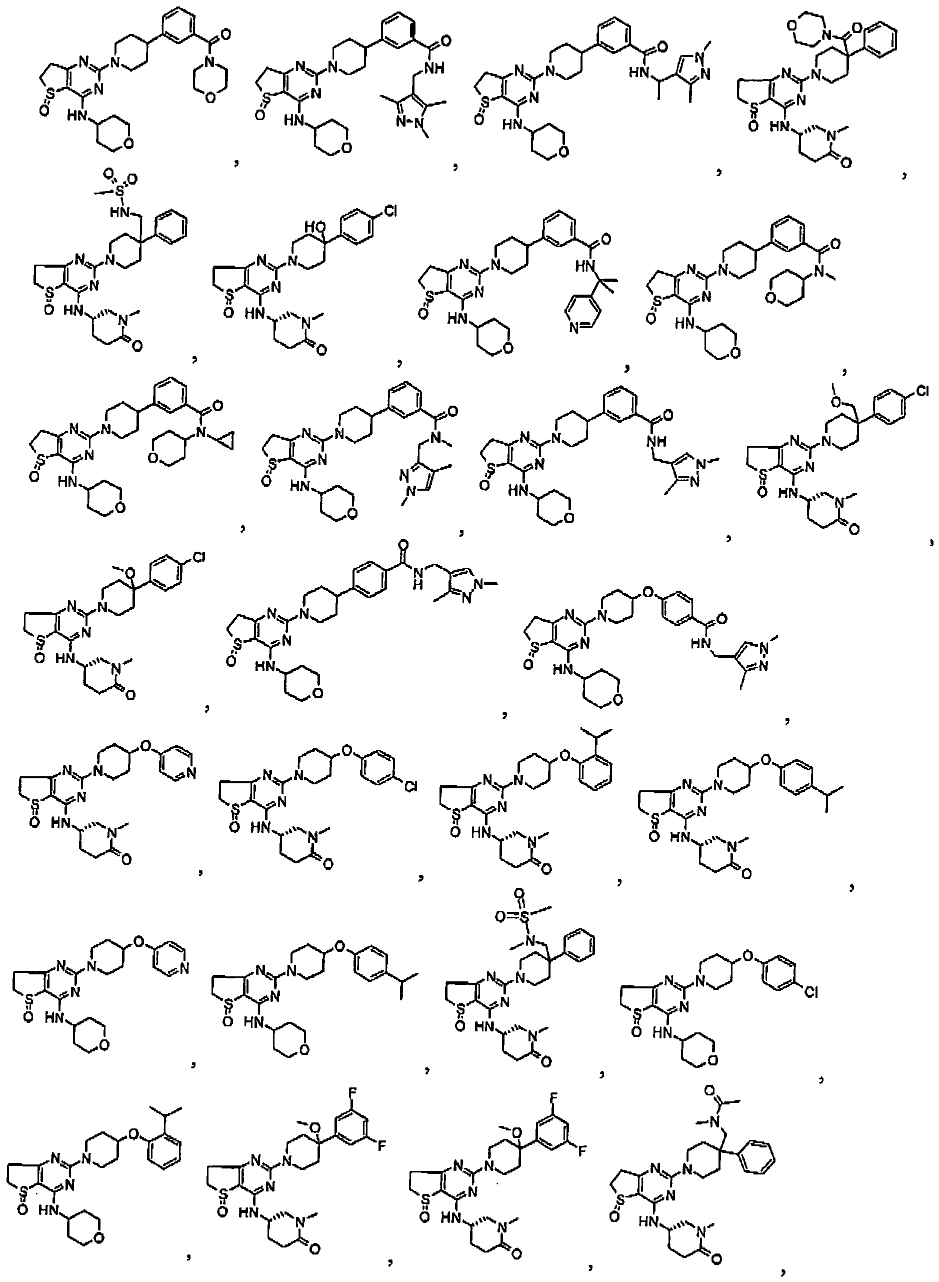 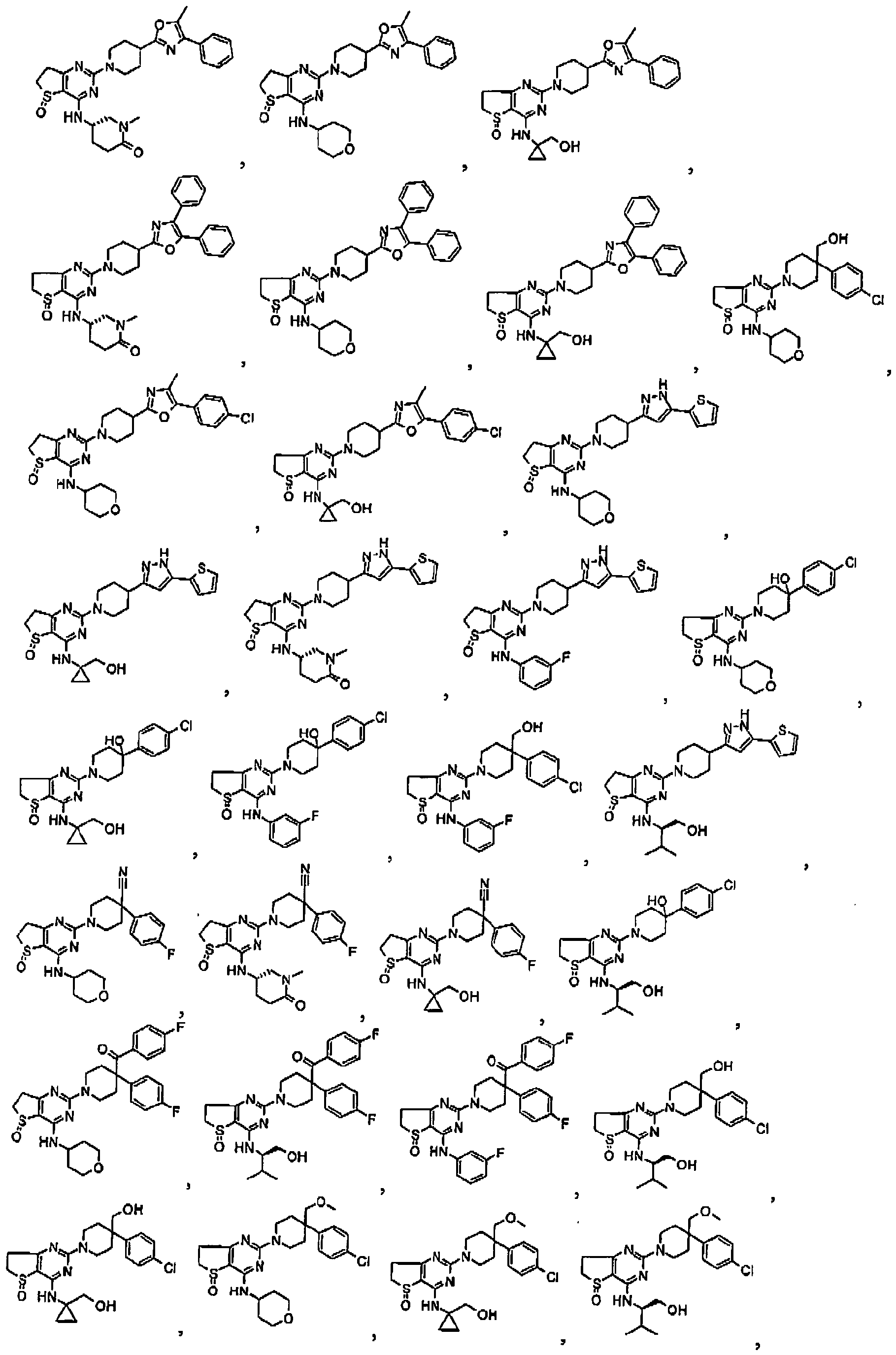 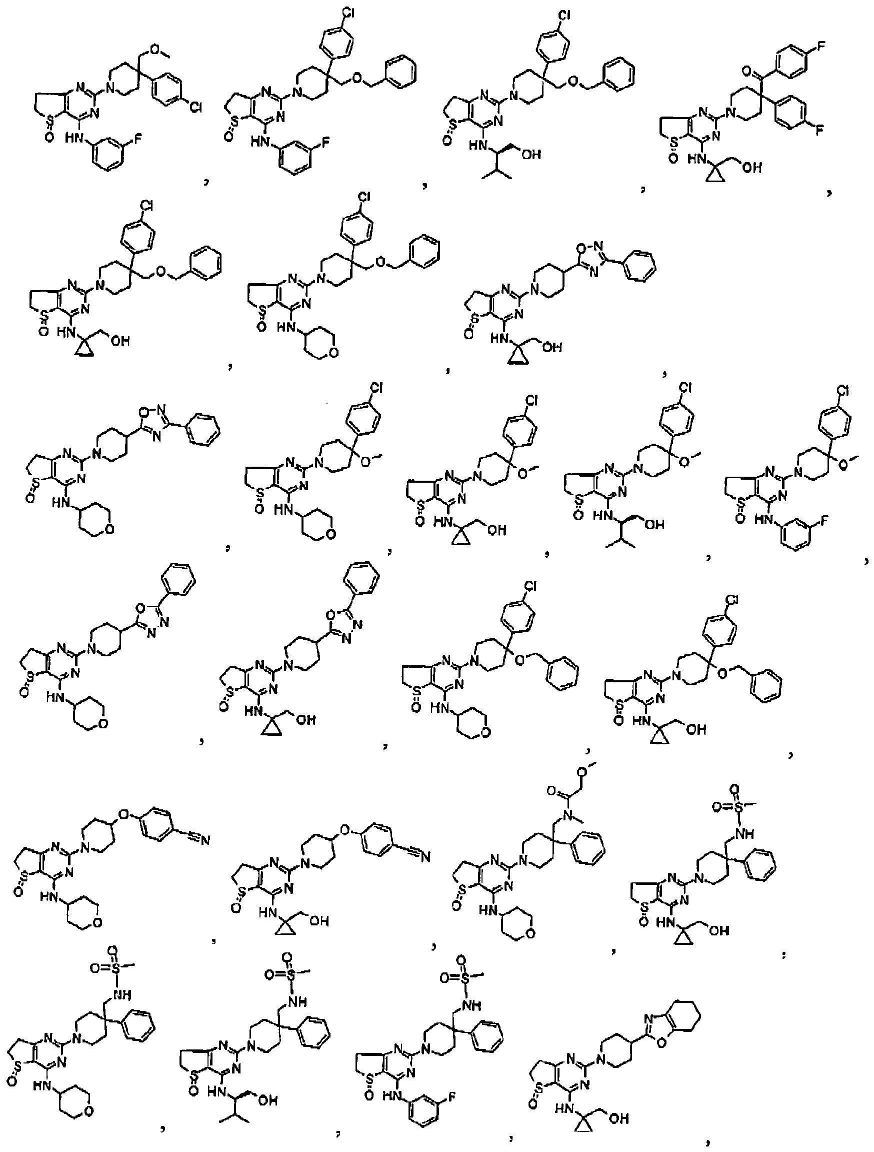 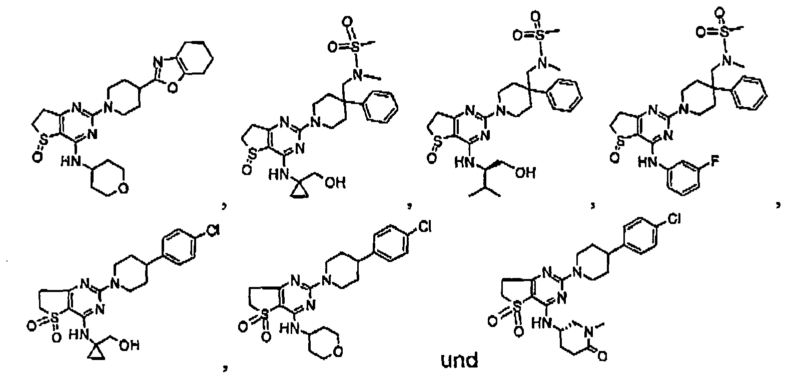 sowie pharmakologisch verträgliche Salze Diastereomere, Enantiomere, Racemate, Hydrate oder Solvate davon.

21. Verbindung der Formel 5 nach einem der Ansprüche 1 bis 3 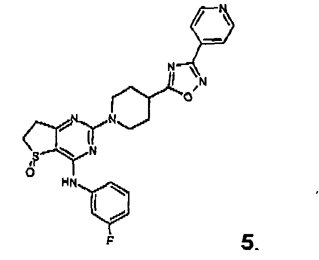 sowie pharmakologisch verträgliche Salze, Diastereomere, Enantiomere, Racemate, Hydrate oder Solvate davon.

22. Verbindung der Formel 6 nach einem der Ansprüche 1 bis 3 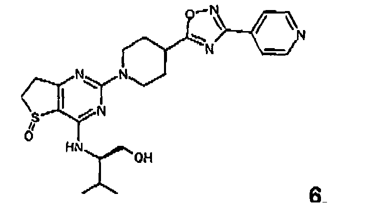 sowie pharmakologisch verträgliche Salze, Diastereomere. Enantiomere, Racemate Hydrate oder Solvate davon.

23. Verbindung der Formel 7 nach einem der Ansprüche 1 bis 3 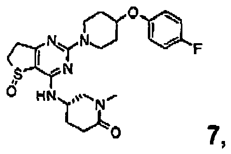 sowie pharmakologisch verträgliche Salze, Diastereomere, Enantiomere, Racemate, Hydrate oder Solvate davon.

24. Verbindung der Formel 8 nach einem der Ansprüche 1 bis 3 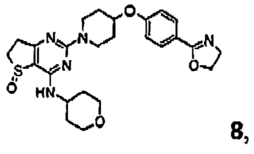 sowie pharmakologisch verträgliche Salze, Diastereomere, Enantiomere, Racemate, Hydrate oder Solvate davon.

25. Verbindung der Formel 9 nach einem der Ansprüche 1 bis 3 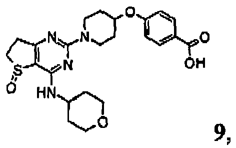 sowie pharmakologisch verträgliche Salze, Diastereomere, Enantiomere, Racemate, Hydrate oder Solvate davon.

26. Verbindung der Formel 10 nach einem der Ansprüche 1 bis 3 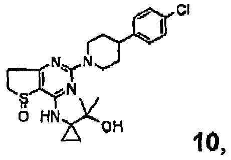 sowie pharmakologisch verträgliche Salze, Diastereomere, Enantiomere, Racemate, Hydrate oder Solvate davon.

27. Verbindung der Formel **11** nach einem der Ansprüche 1 bis 3 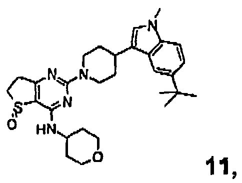 sowie pharmakologisch verträgliche Salze, Diastereomere, Enantiomere, Racemate, Hydrate oder Solvate davon.

28. Verbindung der Formel 12 nach einem der Ansprüche 1 bis 3 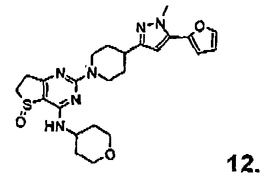 sowie pharmakologisch verträgliche Salze, Diastereomere, Enantiomere, Racemate, Hydrate oder Solvate davon.

29. Verbindung der Formel 13 nach einem der Ansprüche 1 bis 3 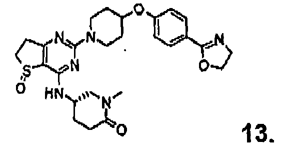 sowie pharmakologisch verträgliche Salze, Diastereomere_Enantiomere, Racemate, Hydrate oder Solvate davon.

30. Verbindung der Formel 14 nach einem der Ansprüche 1 bis 3 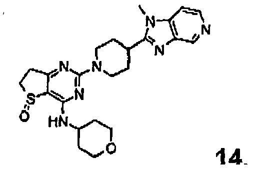 sowie pharmakologisch verträgliche Salze, Diastereomere, Enantiomere_Racemate, Hydrate oder Solvate davon.

31. Verbindung der Formel 15 nach einem der Ansprüche 1 bis 3 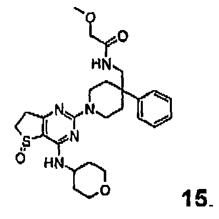 sowie pharmakologisch verträgliche Salze, Diastereomere, Enantiomere, Racemate, Hydrate oder Solvate davon.

32. Verbindung der Formel **16** nach einem der Ansprüche 1 bis 3 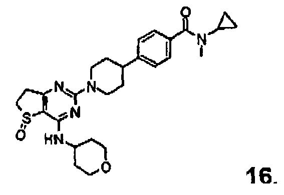 sowie pharmakologisch verträgliche Salze, Diastereomere, Enantiomere, Racemate, Hydrate oder Solvate davon.

33. Verbindung der Formel **17** nach einem der Ansprüche 1 bis 3 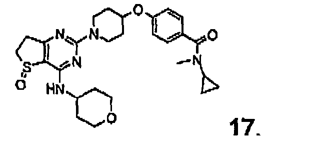 sowie pharmakologisch verträgliche Salze, Diastereomere, Enantiomere, Racemate, Hydrate oder Solvate davon.

34. Verbindung der Formel **18** nach einem der Ansprüche 1 bis 3 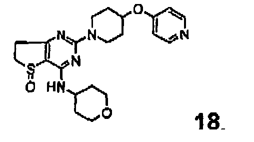 sowie pharmakologisch verträgliche Salze, Diastereomere, Enantiomere, Racemate, Hydrate oder Solvate davon.

35. Verbindung der Formel **19** nach einem der Ansprüche 1 bis 3 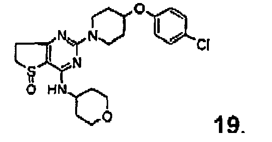 sowie pharmakologisch verträgliche Salze, Diastereomere, Enantiomere, Racemate, Hydrate oder Solvate davon.

36. Verbindung der Formel **20** nach einem der Ansprüche 1 bis 3 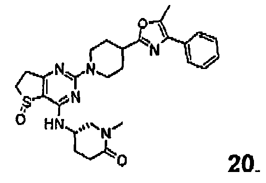 sowie pharmakologisch verträgliche Salze, Diastereomere, Enantiomere, Racemate, Hydrate oder Solvate davon.

37. Verbindung der Formel 21 nach einem der Ansprüche 1 bis 3 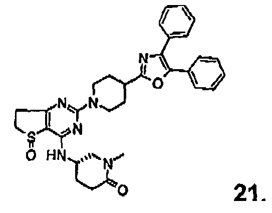 sowie pharmakologisch verträgliche Salze, Diastereomere, Enantiomere, Racemate, Hydrate oder Solvate davon.

38. Verbindung der Formel 22 nach einem der Ansprüche 1 bis 3 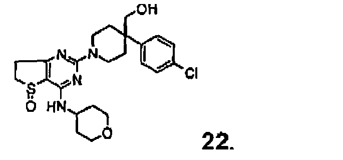 sowie pharmakologisch verträgliche Salze, Diastereomere, Enantiomere, Racemate, Hydrate oder Solvate davon.

39. Verbindung der Formel 23 nach einem der Ansprüche 1 bis 3 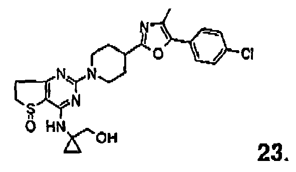 sowie pharmakologisch verträgliche Salze, Diastereomere, Enantiomere, Racemate, Hydrate oder Solvate davon.

40. Verbindung der Formel 24 nach einem der Ansprüche 1 bis 3 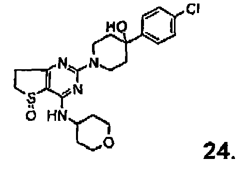 sowie pharmakologisch verträgliche Salze, Diastereomere, Enantiomere, Racemate, Hydrate oder Solvate davon.

41. Verbindung der Formel 25 nach einem der Ansprüche 1 bis 3 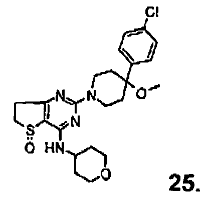 sowie pharmakologisch verträgliche Salze, Diastereomere, Enantiomere, Racemate, Hydrate oder Solvate davon.

42. Verbindung der Formel 26 nach einem der Ansprüche 1 bis 3 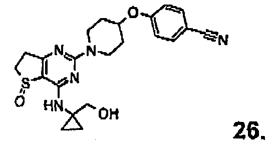 sowie pharmakologisch verträgliche Salze, Diastereomere, Enantiomere, Racemate, Hydrate oder Solvate davon.

43. Verbindung der Formel 27 nach einem der Ansprüche 1 bis 3 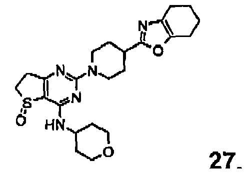 sowie pharmakologisch verträgliche Salze, Diastereomere, Enantiomere, Racemate, Hydrate oder Solvate davon.

44. Verbindung der Formel 28 nach einem der Ansprüche 1 bis 3 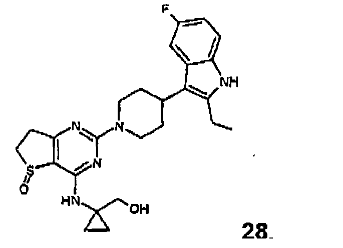 sowie pharmakologisch verträgliche Salze, Diastereomere, Enantiomere, Racemate, Hydrate oder Solvate davon.

45. Verbindungen nach einem der Ansprüche 1 bis 44 als Arzneimittel.

46. Verwendung von Verbindungen nach einem der Ansprüche 1 bis 44 zur Herstellung eines Medikaments zur Behandlung von Atemwegs- oder gastrointestinalen Beschwerden oder Erkrankungen, wie auch entzündliche Erkrankungen der Gelenke, der Haut oder der Augen, Krebserkrankungen, sowie von Erkrankungen des periphären oder zentralen Nervensystems.

47. Verwendung von Verbindungen nach einem der Ansprüche 1 bis 44 zur Herstellung eines Medikaments zur Vorbeugung und Behandlung von Atemwegs- oder Lungenerkrankungen, die mit einer erhöhten Schleimproduktion, Entzündungen und / oder obstruktiven Erkrankungen der Atemwege einhergehen.

48. Verwendung von Verbindungen nach einem der Ansprüche 1 bis 44 zur Herstellung eines Medikament zur Behandlung von entzündlichen und obstruktiven Erkrankungen wie COPD, chronischer Sinusitis, Asthma, Morbus Crohn, Colitis ulcerosa.

49. Verwendung von Verbindungen nach einem der Ansprüche 1 bis 44 zur Herstellung eines Medikaments zur Vorbeugung und Behandlung von Erkrankungen des periphären oder zentralen Nervensystems wie Depression, bipolare oder manische Depression, akute und chronische Angstzustände, Schizophrenie, Alzheimer'sche Erkrankung, Parkinson'sche Erkrankung, akute und chronische Multiple Sklerose oder akute und chronische Schmerzzustände sowie Verletzungen des Gehirns hervorgerufen durch Schlaganfall, Hypoxie oder Schädel-Him-Trauma.

50. Pharmazeutische Formulierungen **gekennzeichnet durch** den Gehalt an einer oder mehreren Verbindungen der Formel 1 nach einem der Ansprüche 1 bis 44 in Kombination mit einem oder mehreren Wirkstoffen ausgewählt aus der Gruppe bestehend aus Anticholinergika. Betamimetika, Corticosteroiden, weiteren PDE4-Inhibitoren, EGFR-Hemmem und LTD4-Antagonisten, CCR3-Inhibitoren, iNOS-Inhibitoren und SYK-Inhibitoren.

## Claims

1. Compounds of formula 1 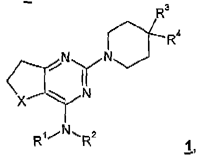 wherein
X denotes SO or SO₂,
R¹ denotes H, C₁₋₆-alkyl,
R² is H or a group selected from among C₁₋₁₀-alkyl and C₂₋₆-alkenyl, which may optionally be substituted by one or more groups selected from halogen and C₁₋₃-fluoroalkyl or which may optionally be substituted by one or more groups selected from among OR^{2.1}, COOR^{2.1}, CONR^{2.2}R^{2.3}, SR^{2.1}, SO-R^{2.1}, SO₂-R^{2.1}, C₆₋₁₀-aryl, -het, hetaryl, a mono- or bicyclic -C₃₋₁₀-cycloalkyl, CH₂-NR^{2.2}R^{2.3} and NR^{2.2}R^{2.3}, which in turn may optionally be substituted by one or more groups selected from among OH, halogen, OR^{2.1}, oxo, CF₃, CHF₂, CH₂F, C₁₋₆-alkyl, C₁₋₆-alkanol, C₆₋₁₀-aryl, COOR^{2.1}, CH₂-NR^{2.2}R^{2.3} and NR^{2.2}R^{2.3},
wherein
**het** denotes a three- to eleven-membered, mono- or bicyclic, saturated or partially saturated, optionally anellated or optionally bridged heterocycle, which contains 1, 2, 3 or 4 heteroatoms selected independently of one another from among N, S or O, and wherein
**hetaryl** is a five- to ten-membered, mono- or bicyclic, optionally anellated heteroaryl, which contains 1, 2, 3 or 4 heteroatoms selected independently of one another from among N, S or O,
and wherein
**cycloalkyl** may be saturated or partially saturated,
wherein R^{2.1} is H or a group selected from among C₁₋₆-alkyl, C₁₋₆-alkanol, C₁₋₃-haloalkyl; mono- or bicyclic -C₃₋₁₀-cycloalkyl, C₆₋₁₀-aryl-C₁₋₆-alkylene, hetaryl-C₁₋₆-alkylene, hot-C₁₋₆-alkylene, C₃₋₁₀-cycloalkyl-C₁₋₆-alkylene, a mono- or bicyclic C₆₋₁₀-aryl, heteroaryl and a -het, which may optionally be substituted by one or more groups selected from among OH, O-(C₁₋₃-alkyl), halogen, C₁₋₆-alkyl and C₆₋₁₀-aryl,
wherein **R**^{2.2} and **R**^{2.3} independently of one another denote H or a group selected from among C₁₋₆-alkyl, mono- or bicyclic C₃₋₁₀ cycloalkyl, C₆₋₁₀aryl-C₁₋₆-alkylene, hetaryl-C₁₋₆-alkylene, mono- or bicyclic C₆₋₁₀-aryl, het, hetaryl, CO-NH₂, CO-NHCH₃, -CO-N(CH₃)₂, SO₂-(C₁-C₂-alkyl), CO-R^{2.1} and COOR^{2.1}, which may optionally be substituted by one or more groups selected from among OH, halogen, C₁₋₆-alkyl, C₆₋₁₀-aryl and COOR^{2.1},
or
**R**² denotes a mono- or polycyclic C₃₋₁₀ cycloalkyl, which may optionally be bridged one or more times via C₁₋₃-alkyl groups and which may optionally be substituted by a group selected from among branched or unbranched C₁₋₆-alkanol, C₁₋₃-fluoroalkyl, C₁₋₃-alkylene-OR^{2.1}, OR^{2.1}, COOR^{2.1}, -SO₂-NR^{2.2}R^{2.3}, het, -NH-CO-O-(C₁₋₆-alkyl), -NH-CO-(C₁₋₆-alkyl), -NH-CO-O-(C₆₋₁₀-aryl), -NH-CO-(C₆₋₁₀-aryl), -NH-CO-O-hetaryl, -NH-CO-hetaryl, -NH-CO-O-(C₁₋₃-alkylene)-(C₆₋₁₀-aryl), -NH-CO-(C₁₋₃-alkylene)-(C₈₋₁₀-aryl), -N(C₁₋₃-alkyl)-CO-(C₁₋₆-alkyl), -N(C₁₋₃-alkyl)-CO-O-(C₆₋₁₀-aryl), -N(C₁₋₃-alkyl)-CO-(C₈₋₁₀-aryl), -N(C₁₋₃-alkyl)-CO-O-hetaryl, -N(C₁₋₃-alkyl)-CO-hetaryl, -N(C₁₋₃-alkyl)-CO-O-(C₁₋₃-alkylene)-(C₆₋₁₀-aryl), -N(C₁₋₃-alkyl)-CO-(C₁₋₃-alkylene)-(C₆₋₁₀-aryl), C₆₋₁₀-aryl, C₁₋₆-alkyl, C₆₋₁₀-aryl-C₁₋₅-alkylene, hetaryl-C₁₋₆-alkylene, mono- or bicyclic C₃₋₁₀ cycloalkyl and NR^{2.2}R^{2.3}, which may optionally be substituted by one or more groups selected from among OH, OR^{2.1}, oxo, halogen, CF₃, CHF₂, CH₂F, C₁₋₆-alkyl, C₆₋₁₀-aryl and NR^{2.2}R^{2.3},
or
R² denotes a mono- or polycyclic C₆₋₁₀-aryl, which may optionally be substituted by OH, SH or halogen or by one or more groups selected from among OR^{2.1}, COOR^{2.1}, NR^{2.2}R^{2.3}, CH₂-NR^{2.2}R^{2.3},C₃₋₁₀-cycloalkyl, het, C₁₋₆-alkyl, C₁₋₃-fluoroalkyl, CF₃, CHF₂, CH₂F, C₆₋₁₀-aryl-C₁₋₆-alkylene, het-C₁₋₆-alkylene, hetaryl-C₁₋₆-alkylene, C₆₋₁₀-aryl, SO₂-CH₃, SO₂-CH₂CH₃ and SO₂-NR^{2.2}R^{2.3}, which may in turn optionally be substituted by one or more groups selected from among OH, OR^{2.1}, OF₃, CHF₂, CH₂F, oxo, halogen, CF₃, CHF₂, CH₂F, C₁₋₆-alkyl, C₆₋₁₀-aryl and NR^{2.2}R^{2.3},
or
**R²** denotes a group selected from among het and hetaryl, which may optionally be substituted by one or more groups selected from among halogen, OH, oxo, CF₃, CHF₂ and CH₂F or by one or more groups selected from among OR^{2.1}, C₁₋₃-alkylene-OR^{2.1}, SR^{2.1}, SO-R^{2.1}, SO₂-R^{2.1}, COOR^{2.1}, COR^{2.1}, C₁₋₆-alkanol, mono- or bicyclic C₃₋₁₀-cycloalkyl, C₆₋₁₀-aryl, C₁₋₆-alkyl, C₆₋₁₀-aryl-C₁₋₆-alkylene, hetaryl-C₁₋₆-alkylene, het, hetaryl, C₁₋₃-alkylene-OR^{2.1} and NR^{2.2}R^{2.3}, which may in turn optionally be substituted by one or more groups selected from among OH, OR^{2.1}, oxo, halogen, CF₃, CHF₂, CH₂F, C₁₋₆-alkyl, C₆₋₁₀-aryl and NR^{2.2}R^{2.3},
or wherein
**NR¹R²** together denotes a heterocyclic C₄₋₇ ring, which may optionally be bridged, which contains 1, 2 or 3 heteroatoms selected from among N, 0 and S and which may optionally be substituted by one or more groups selected from among OH, OR^{2.1}, C₁₋₃-alkylene-O^{R.1}, oxo, halogen, C₁₋₆-alkyl, C₆₋₁₀-aryl, COOR^{2.1}, CH₂-NR^{2.2}-COO-R^{2.1}, CH₂-NR^{2.2}-CO-R^{2.1}, CH₂-NR^{2.2}-CO-CH₂-NR^{2.2}R^{2.3}, CH₂-NR^{2.2}-SO₂-C₁₋₃-alkyl, CH₂-NR^{2.2}-SO₂-NR^{2.2}R^{2.3}, CH₂-NR^{2.2}-CO-NR^{2.2}R^{2.3}, CO-NR^{2.2}R^{2.3}, CH₂-NR^{2.2}R^{2.3} and NR^{2.2}R^{2.3},
and wherein
**R²** is a C₆₋₁₀-aryl, which may optionally be substituted in the ortho, para or meta position by one, two or three groups selected independently of one another from among fluorine, chlorine, bromine, hydroxy, CN, C₁₋₆-alkyl, C₁₋₃-fluoroalkyl, -C₁₋₃-alkylene-OR^{2.1}, -C₁₋₃-alkylene-NR^{2.2}R^{2.3}, -NR^{2.2}R^{2.3}, O-R^{2.1}; SO-R^{2.1}, SO₂-R^{2.1}, COOR^{2.1}, -CO-NH-(C₁₋₆-alkylene)-hetaryl, -CO-NH-hetaryl, -CO-N(CH₃)-het, -CO-N(CH₃)-(C₁₋₃-alkylene)-het, -CO-N(CH₃)-(C₁₋₃-alkylene)-hetaryl, -CO-N(C₃₋₇-cycloalkyl)-het, -CO-NR^{2.2}R^{2.3}, -CO-NH-(C₁₋₆-alkylene)-het, NR^{2.2}-CO-R²¹, C₆₋₁₀-aryk, C₆₋₁₀-aryl-C₁₋₂-alkylene, het-C₁₋₂-alkylene, -het, -CO-het, CO-N(CH₃)-C₃₋₇-cycloalkyl, C₃₋₇-cycloalkyl, C₃₋₇-cycloalkyl-C₁₋₂-alkylene, hetaryl-C₁₋₂-alkylene and hetaryl, while this group may optionally be substituted by one or more groups selected from among OH, halogen, -C₁₋₃-fluoroalkyl, oxo, methyl and phenyl,
or wherein
**R³** is a group selected from among het and hetaryl, which may optionally be substituted by one or more groups selected from among halogen, C₁₋₃-fluoroalkyl, CN, OH, oxo, -C₁₋₆-alkyl, -C₁₋₃--alkylene-NR^{2.2}R^{2.3}, -NR^{2.2}R^{2.3}, SO-R^{2.1}, SO₂-R^{2.1}, -O-R^{2.1}, -COOR^{2.1}, SO₂-(CH₃), SO₂-(CH₂-CH₃), C₆₋₁₀-aryl, het, C₃₋₇-cycloalkyl and hetaryl, which may in turn optionally be substituted by one or more groups selected from among OH, halogen, -C₁₋₃-fluoroalkyl, C₁₋₆-alkyl, C₆₋₁₀-aryl, -COO(C₁₋₃-alkyl) and O-(C₁₋₃-alkyl),
or wherein
**R³** denotes -O-R^{3.1},
wherein **R^{3.1}** is a group selected from among -C₁₋₆-alkyl, -C₆₋₁₀-aryl, -C₁₋₃-alkylene-C₆₋₁₀-aryl, hetaryl and het, which may optionally be substituted in the ortho, para or meta position by one, two or three groups selected independently of one another from among fluorine, chlorine, bromine, hydroxy, CN, C₁₋₆-alkyl, C₁₋₃-fluoroalkyl, CO-(C₁₋₅-alkyl), -CO-(C₁₋₃-fluoroalkyl), -CO-NH-(C₁₋₆-alkylene)-hetaryl, -CO-N(C₁₋₃-alkyl)-(C₁₋₆-alkylene)-hetaryl, -CO-N(C₁₋₃-alkyl)-het, -CO-N(C₃₋₇-cycloalkyl)-het, -C₁₋₃-alkylene-OR^{2.1}, -C₁₋₃-alkylene-NR^{2.2}R^{2.3}, -NR^{2.2}R^{2.3}, O-R^{2.1}; SO-R^{2.1}, SO₂-R^{2,1}, COOH, COO-(C₁₋₄-alkyl), -O-C₁₋₃-alkylene-N(C₁₋₃-alkyl)₂, CO-NR^{2.2}R^{2.3}, NR^{2.2}CO-R^{2.1}, C₆₋₁₀-aryl, C₆₋₁₀-aryl-C₁₋₂-alkylene, het-C₁₋₂-alkylene, -CO-het, het, -CO-C₃₋₇-cycloalkyl, -CO-N(C₁₋₃-alkyl)-C₃₋₇-cycloalkyl, C₃₋₇-cycloalkyl, C₃₋₇-cycloalkyl-C₁₋₂-alkylene, hetaryl-C₁₋₂-alkylene and hetaryl, which may in turn optionally be substituted by 1, 2, 3 or 4 groups selected independently of one another from among F, Cl, Br, methyl, O-methyl, ethyl, O-ethyl, OH, oxo and CF₃. and wherein
**R⁴** denotes H, CN, OH, CF₃, CHF₂, CH₂F, F, methyl, ethyl, -O-(C₁₋₃-alkyl), -C₁₋₃-alkylene-OH, -COO(C₁₋₃-alkyl), -CO-het, -(C₁₋₂-alkylene)-NH-SO₂-(C₁₋₂-alkyl), -(C₁₋₂-alkylene)-N(C₁₋₃-alkyl)-SO₂-(C₁₋₂-alkyl), -(C₁₋₂-alkylene)-O-(C₁₋₂-alkylene)-C₆₋₁₀-aryl, -C₁₋₃-alkylene-O-C₁₋₃-alkyl, -(C₁₋₂-alkylene)-N(C₁₋₃-alkyl)-CO-(C₁₋₂-alkyl), -NH-CO-(C₁₋₃-alkylene)-O-(C₁₋₃-alkyl), -C₁₋₃-alkylene-NH-CO-(C₁₋₃-alkyl), -C₁₋₃-alkylene-NH-CO-(C₁₋₃-alkylene)-N(C₁₋₃-alkyl)₂, -O-(C₁₋₂-alkylene)-(C₆₋₁₀-aryl), -C₁₋₃-alkylene-NH-CO-(C₁₋₃-alkylene)-O-(C₁₋₃-alkyl), -CO-(C₆₋₁₀-aryl), -(C₁₋₂-alkylene)-N(C₁₋₃-alkyl)-CO-(C₁₋₂-alkylene)-O-(C₁₋₃-alkyl), wherein the aryl in the above groups may in turn optionally be substituted by one or more other groups selected from among F, Cl, Br, methyl, ethyl, propyl, isopropyl, cyclopropyl, -O-methyl, -O-ethyl, -O-propyl, -O-isopropyl, -O-cyclopropyl, -OH and CF₃
or wherein
**R³** and **R⁴** together form a mono- or bicyclic, unsaturated, saturated or partially saturated heterocycle, which contains 1, 2 or 3 heteroatoms selected from among N, O and S and which may optionally be substituted by one or more groups selected from among halogen, OH, oxo, C₁₋₃-fluoroalkyl, CN, C₁₋₆ alkyl, -O-R^{2.1}, -COOR^{2.1}, SO-R^{2.1}, SO₂-R^{2.1}, -C₁₋₃-alkylene-NR^{2.2}R^{2.3}, -NR^{2.2}R^{2.3}, C₆₋₁₀-aryl, C₃₋₇-cycloalkyl, het and hetaryl,
as well as pharmacologically acceptable salts, diastereomers, enantiomers, racemates, hydrates or solvates thereof.

2. Compounds of formula **1** according to claim 1, wherein
**X** denotes SO or SO₂,
**R¹** denotes H
**R²** denotes H or C₁₋₁₀-alkyl, which may optionally be substituted by one or more groups selected from halogen and C₁₋₃-fluoroalkyl or which may optionally be substituted by one or more groups selected from among OR^{2.1}, COOR^{2.1}, CONR^{2.2}R^{2.3}, SR^{2.1}, SO-R^{2.1}, SO₂-R^{2.1}, phenyl, het, hetaryl, a monocyclic C₃₋₇-cycloalkyl, CH₂-NR^{2.2}R^{2.3} and NR^{2.2}R^{2.3}, which in turn may optionally be substituted by one or more groups selected from among OH, F, Cl, Br, OR^{2.1}, oxo, CF₃, CHF₂, CH₂F, C₁₋₆-alkyl, C₁₋₆-alkanol, phenyl, COOR^{2.1}, CH₂-NR^{2.2}R^{2.3} and NR^{2.2}R^{2.3},
wherein
**het** is a three- to seven-membered, monocyclic, saturated or partially saturated heterocycle or a seven- to eleven-membered, bicyclic, saturated or partially saturated heterocycle, which contains 1, 2, 3 or 4 heteroatoms selected independently of one another from among N, S or O, and wherein
**hetaryl** is a five- to six-membered, monocyclic, aromatic heteroaryl or a seven- to eleven-membered, bicyclic, aromatic heteroaryl, which contains in each case 1, 2, 3 or 4 heteroatoms selected independently of one another from among N, S or O,
and wherein
**cycloalkyl** may be saturated or partially saturated,
wherein **R^{2.1}** is H or a group selected from among C₁₋₈-alkyl, C₁₋₆-alkanol, C₁₋₃-haloalkyl, monocyclic C₃₋₇ cycloalkyl, phenyl-C₁₋₆-alkylene, hetaryl-C₁₋₆-alkylene, het-C₁₋₆-alkylene, -C₃₋₇-cycloalkyl-C₁₋₆-alkylene, phenyl, hetaryl and a het,
which may optionally be substituted by one or more groups selected from among OH, F, Cl, C₁₋₆-alkyl, -O-(C₁₋₃-alkyl) and phenyl,
wherein **R^{2.2}** and **R^{2.3}** independently of one another denote H or a group selected from among C₁₋₆-alkyl, monocyclic C₃₋₇ cycloalkyl, phenyl-C₁₋₃-alkylene, hetaryl-C₁₋₃-alkylene, phenyl, het, hetaryl, CO-NH₂, -CO-NHCH₃, -CON(CH₃)₂, SO₂-(C₁₋₂-alkyl),
CO-R^{2.1} and COOR^{2.1},
which may optionally be substituted by one or more groups selected from among OH, F, Cl, C₁₋₆-alkyl, phenyl and COOR^{2.1},
or
**R²** denotes a monocyclic C₃₋₇ cycloalkyl, which may optionally be substituted by a group selected from among branched or unbranched C₁₋₆-alkanol, C₁₋₃-fluoroalkyl, OR^{2.1}, C₁₋₃-alkylene-OR^{2.1}, OR^{2.1}, COOR^{2.1}, SO₂-NR^{2.2}R^{2.3}, -het, -NH-CO-O-(phenyl), phenyl, C₁₋₆-alkyl, phenyl-C₁₋₆-alkylene, -hetaryi-C₁₋₆-alkylene, monocyclic C₃₋₇ cycloalkyl and NR^{2.2}R^{2.3}, which may optionally be substituted by one or more groups selected from among OH, OR^{2.1}, oxo, F, Cl, CF₃, CHF₂, CH₂F, C₁₋₆-alkyl, phenyl and -NR^{2.2}R^{2.3},
or
**R²** denotes a phenyl, which may optionally be substituted by OH, SH or halogen or by one or more groups selected from among OR^{2.1}, COOR^{2.1}, NR^{2.2}R^{2.3}, CH₂-NR^{2.2}R^{2.3}, C₃₋₇-cycloalkyl, C₃₋₇ heterocycle, C₁₋₆-alkyl, C₁₋₃-fluoroalkyl, phenyl-C₁₋₆-alkylene, -het-C₁₋₆-alkylene, -hetaryl-C₁₋₆-alkylene, phenyl, SO₂-CH₃, SO₂-CH₂CH₃ and SO₂-NR^{2.2}R^{2.3}, which may in turn optionally be substituted by one or more groups selected from among OH, OR^{2.1}, oxo, F, Cl, CF₃, CHF₂, CH₂F, C₁₋₆-alkyl, phenyl and NR^{2.2}R^{2.3},
or
**R²** denotes a group selected from among het and hetaryl, which may optionally be substituted by one or more groups selected from among F, Cl, OH, oxo, CF₃, CHF₂ and CH₂F or by one or more groups selected from among OR^{2.1}, -C₁₋₃-alkylene-OR^{2.1}, SR^{2.1}, SO-R^{2.1}, SO₂-R^{2.1}, COOR^{2.1}, COR^{2.1}, C₁₋₆-alkanol, monocyclic C₈₋₇-cycloalkyl, phenyl, C₁₋₆-alkyl, phenyl-C₁₋₆-alkylene, -hetaryl-C₁₋₆-alkylene, -het, -hetaryl, and NR^{2.2}R^{2.3}, which may in turn optionally be substituted by one or more groups selected from among OH, OR^{2.1}, oxo, F, Cl, CF₃, CHF₂, CH₂F, C₁₋₆-alkyl, phenyl and NR^{2.2}R^{2.3},
or wherein
**NR¹R²** together denotes a heterocyclic C₄₋₇ ring which may optionally be bridged, which contains 1, 2 or 3 heteroatoms selected from among N, O and S and which may optionally be substituted by one or more groups selected from among OH, OR^{2.1}, C₁₋₃-alkylene-O^{R.1}, oxo, F, Cl, C₁₋₆-alkyl, phenyl, COOR^{2.1}, CH₂-NR^{2.2}-COO-R^{2.1}, CH₂-NR^{2.2}-CO-R^{2.1}, CH₂-NR^{2.2}-CO-CH₂-NR^{2.2}R^{2.3}, CH₂-NR^{2.2}-SO₂-C₁₋₃-alkyl, CH₂-NR^{2.2}-SO₂-NR^{2.2}R^{2.3}_{,} CH₂-NR^{2.2}-CO-NR^{2.2}R^{2.3}, CO-NR^{2.2}R^{2.3}, CH₂-NR^{2.2}R^{2.3} and NR^{2.2}R^{2.3},
and wherein
**R³** is a naphthalene or phenyl, which may optionally be substituted in the ortho, para or meta position by one or two groups selected independently of one another from among fluorine, chlorine, bromine, hydroxy, CN, C₁₋₆-alkyl, C₁₋₃-fluoroalkyl, -C₁₋₃-alkylene-OR^{2.1}, -C₁₋₃-alkylene-NR^{2.2}R^{2.3}, -NR^{2.2}R^{2.3}, O-R^{2.1}; SO-R^{2.1}, SO₂-R^{2.1}, COOR^{2.1}, -CO-NH-(C₁₋₆-alkylene)-hetaryl, -CO-NH-hetaryl, -CO-N(CH₃)-het, -CO-N(CH₃)-(C₁₋₃-alkylene)-het, -CO-N(CH₃)-(C₁₋₃-alkylene)-hetaryl, -CO-N(C₃₋₇-cycloalkyl)-het, CO-NR^{2.2}R^{2.3}, -CO-NH-(C₁₋₆-alkylene)-het, -NR^{2.2}-CO-R^{2.1}, phenyl, phenyl-C₁₋₂-alkylene, -het-C₁₋₂-alkylene, -het, -CO-het, -CO-N(CH₃)-het, CO-N(CH₃)-C₃₋₇₋cycloalkyl, C₃₋₇-cycloalkyl, C₃₋₇-cycloalkyl-C₁₋₂-alkylene, -hataryl-C₁₋₂-alkylene and -hetaryl, while this group may optionally be substituted by one or more groups selected from among OH, F, Cl, -C₁₋₃-fluoroalkyl, oxo, methyl and phenyl,
or wherein
**R³** denotes a group selected from among het and hetaryl, which may optionally be substituted by one or more groups selected from among F, Cl, Br, C₁₋₃-fluoroalkyl, CN, OH, oxo, -C₁₋₆-alkyl, -C₁₋₃-alkylene-NR^{2.2}R^{2.3}, -NR^{2.2}R^{2.3}, SO-R^{2.1}, SO₂-R^{2.1}, -O-R^{2.1}, -COOR^{2.1}, SO₂-(CH₃), SO₂-(CH₂-CH₃), phenyl, het, C₃₋₇-cycloalkyl and hetaryl, which may in turn optionally be substituted by one or more groups selected from among OH, F, Cl, Br, -C₁₋₃-fluoroalkyl, C₁₋₆-alkyl, phenyl, -COO(C₁₋₃-alkyl) and O-(C₁₋₃-alkyl),
or wherein
**R³** denotes -O-R^{3.1},
wherein **R^{3.1}** is a group selected from among -C₁₋₆-alkyl, -phenyl, -C₁₋₃-alkylene-phenyl, hetaryl and het, which is optionally substituted in the ortho, para or meta position by one, two or three groups selected independently of one another from among fluorine, chlorine, bromine, hydroxy, CN, C₁₋₆-alkyl, C₁₋₃-fluoroalkyl, CO-(C₁₋₆-alkyl), -CO-(C₁₋₃-fluoroalkyl), -CO-NH-(C₁₋₆-alkylene)-hetaryl, -CO-N(CH₃)-(C₁₋₆-alkylene)-hetaryl, -CO-N(CH₃)-het, -CO-N(C₃₋₇-cycloalkyl)-het, -C₁₋₃-alkylene-OR^{2.1}, -C₁₋₃-alkylene-NR^{2.2}R^{2.3}, -NR^{2.2}R^{2.3}, O-R^{2.1}; SO-R^{2.1}, SO₂-R^{2.1}, COOH, COO-(C₁₋₄-alkyl), -O-C₁₋₃-alkylene-N(C₁₋₃-alkyl)₂, CO-NR^{2.2}R^{2.3}, NR^{2.2}-CO-R^{2.1}, phenyl, phenyl-C₁₋₂-alkylene, het-C₁₋₂-alkylene, -CO-het, het, -CO-C₃₋₇-cycloalkyl, -CO-N(CH₃)-C₃₋₇-cycloalkyl, C₃₋₇cycloalkyl, C₃₋₇-cycloalkyl-C₁₋₂-alkylene, hetaryl-C₁₋₂-alkylene and hetaryl, which may in turn optionally be substituted by 1, 2, 3 or 4 groups selected independently of one another from among F, Cl, Br, methyl, O-methyl, ethyl, O-ethyl, OH, oxo and CF₃,
and wherein
**R⁴** denotes H, CN, OH, CF₃, CHF₂, CH₂F, F, methyl, ethyl, O-methyl, O-ethyl, O-propyl, O-isopropyl, -C₁₋₃-alkylene-OH, -COO(C₁₋₃-alkyl), -CO-het, -(C₁₋₂-alkylene)-NH-SO₂-C₁₋₂-alkyl), -(C₁₋₂-alkylene)-N(CH₃)-SO₂-(C₁₋₂-alkyl), -(C₁₋₂-alkylene)-O-(C₁₋₂-alkylene)-phenyl, -C₁₋₃-alkylene-O-C₁₋₃-alkyl, -(C₁₋₂-alkylene)-N(CH₃)-CO-(C₁₋₂-alkyl), -NH-CO-(C₁₋₃-alkylene)-O-(C₁₋₃-alkyl), -C₁₋₃-alkylene-NH-CO-(C₁₋₃-alkyl), -C₁₋₃-alkylene-NH-CO-(C₁₋₃-alkylene)-N(C₁₋₃-alkyl)_{z}, -O-(C₁₋₂-alkylene)-phenyl -C₁₋₃-alkylene-NH-CO-(C₁₋₃-alkylene)-O-(C₁₋₃-alkyl), -CO-phenyl, -(C₁₋₂-alkylene)-N(CH₃)-CO-(C₁₋₂-alkylene)-O-(C₁₋₃-alkyl), wherein the phenyl in the above groups may optionally be substituted by one or more other groups selected from among F, Cl, Br, methyl, ethyl, propyl, -O-methyl, -O-ethyl, -O-propyl, -OH and CF₃
or wherein
**R³** and **R⁴** together form a mono- or bicyclic, unsaturated, saturated or partially saturated heterocycle, which contains 1, 2 or 3 heteroatoms selected from among N, O and S and which may optionally be substituted by one or more groups selected from among F, Cl, Br, OH, oxo, C₁₋₃-fluoroalkyl, CN, C₁₋₆-alkyl, -O-R^{2.1}, -COOR^{2.1}, SO-R^{2.1}, SO₂-R^{2.1}, -C₁₋₃-alkylene-NR^{2.2}R^{2.3}, -NR^{2.2}R^{2.3}, phenyl, C₃₋₇-cycloalkyl, het and hetaryl,
as well as pharmacologically acceptable salts, diastereomers, enantiomers, racemates, hydrates or solvates thereof.

3. Compounds of formula **1** according to one of Claims 1 or 2, wherein
**X** denotes SO,
**R¹** denotes H
**R²** denotes H or C₁₋₆-alkyl, which may optionally be substituted by one or more groups selected from F, Cl, CF₃, CHF₂ or CH₂F or which may optionally be substituted by one or more groups selected from among OR^{2.1}, COOR^{2.1}, CONR^{2.2}R^{2.3}, SR^{2.1}, SO-R^{2.1}, SO₂-R^{2.1}, phenyl, het, hetaryl, a monocyclic C₃₋₇-cycloalkyl, CH₂-NR^{2.2}R^{2.3} and NR^{2.2}R^{2.3}, which in turn may optionally be substituted by one or more groups selected from among OH, F, Cl, Br, CF₃. CHF₂, CH₂F, OR^{2.1}, oxo, methyl, ethyl, propyl, isopropyl, methanol, ethanol, phenyl, COOR^{2.1}, CH₂-NR^{2.2}R^{2.3}, and NR^{2.2}R^{2.3},
wherein
**het** is a three- to seven-membered, monocyclic, saturated or partially saturated heterocycle, which contains 1, 2 or 3 heteroatoms selected independently of one another from among N, S or O,
and wherein
**hetaryl** is a five-to six-membered, monocyclic, aromatic heteroaryl which contains 1, 2 or 3 heteroatoms selected independently of one another from among N, S or O,
and wherein
**cycloalkyl** may be saturated or partially saturated, wherein **R^{2.1}** is H or a group selected from among methyl, ethyl, propyl, isopropyl, methanol, ethanol, monocyclic C₃₋₇ cycloalkyl, phenyl-C₁₋₂-alkylene, -hetaryl-C₁₋₂-alkylene, -het-C₁₋₂-alkylene, C₃₋₇-cycloalkyl-C₁₋₂-alkylene, phenyl, hetaryl and a het, which may optionally be substituted by one or more groups selected from among OH, F, Cl, methyl, ethyl, propyl, isopropyl, O-methyl, O-ethyl, O-propyl, O-isopropyl and phenyl, wherein **R^{2.2}** and **R^{2.3}** independently of one another denote H or a group selected from among methyl, ethyl, propyl, isopropyl, monocyclic C^{3.7} cycloalkyl, phenyl-C₁₋₃-alkylene, hetaryl-C₁₋₃-alkylene, phenyl, -het, -hetaryl, CO-NH₂, CO-NHCH₃, CON(CH₃)₂, SO₂-(C₁₋₂-alkyl), CO-R^{2.1} and COOR^{2.1}, which may optionally be substituted by one or more groups selected from among OH, F, Cl, methyl, ethyl, propyl, isopropyl, phenyl and COOR^{2.1},
or
**R²** denotes a monocyclic C₃₋₇ cycloalkyl, which may optionally be substituted by a group selected from among C₁₋₂-alkanol, C₁₋₃-fluoroalkyl, C₁₋₃-alkylene-OR^{2.1}, OR^{2.1}, COOR^{2.1}, SO₂-NR^{2.2}R^{2.3}, -het, -NH-CO-O-(phenyl), methyl, ethyl, propyl, isopropyl, phenyl, phenyl-C₁₋₂-alkylene, -hetaryl-C₁₋₂-alkylene, monocyclic C₃₋₇ cycloalkyl and NR^{2.2}R^{2.3}, which may optionally be substituted by one or more groups selected from among OH, OR^{2.1}, oxo, F, Cl, CF₃, CHF₂, CH₂F, methyl, ethyl, propyl, isopropyl, phenyl and NR^{2.2}R^{2.3},
or
**R²** denotes a phenyl, which may optionally be substituted by OH, SH, F, Cl or Br or by one or more groups selected from among OR^{2.1}, COOR^{2.1}, NR^{2.2}R^{2.3,} CH₂-NR^{2.2}R^{2.3}, monocyclic C₃₋₇-cycloalkyl, -het, methyl, ethyl, propyl, isopropyl, CF₃, CHF₂, CH₂F, phenyl-C₁₋₂-alkylene, het-C₁₋₂-alkylene, hetaryl-C₁₋₂-alkylene, phenyl, SO₂-CH₃, SO₂-CH₂CH₃ and SO₂-NR^{2.2}R^{2.3}, which may in turn optionally be substituted by one or more groups selected from among OH, OR^{2.1}, oxo, F, Cl, CF₃, CHF₂. CH₂F, methyl, ethyl, propyl, isopropyl, phenyl and NR^{2.2}R^{2.3},
or
**R²** denotes a group selected from among het and hetaryl, which may optionally be substituted by one or more groups selected from among F, Cl, OH, oxo, CF₃, CHF₂ and CH₂F or by one or more groups selected from among OR^{2.1}, C₁₋₃-alkylene-OR^{2.1}, SR^{2.1}, SO-R^{2.1}, SO₂-R^{2.1}, COOR^{2.1}, COR^{2.1}, methanol, ethanol, monocyclic C₃₋₇-cycloalkyl, phenyl, methyl, ethyl, propyl, isopropyl, phenyl-C₁₋₂-alkylene, hetaryl-C₁₋₂-alkylene, -het, -hetaryl and NR^{2.2}R^{2.3}, which may in turn optionally be substituted by one or more groups selected from among OH, OR^{2.1}, oxo, F, Cl, CF₃, CHF₂, CH₂F, methyl, ethyl, propyl, isopropyl, butyl, isobutyl, phenyl and NR^{2.2}R^{2.3},
and wherein
**R³** is a naphthalene or phenyl, which may optionally be substituted in the ortho, para or meta position by one or two groups selected independently of one another from among fluorine, chlorine, bromine, hydroxy, CN, methyl, ethyl, propyl, isopropyl, cyclopropyl, CF₃, CHF₂, CH₂F, -OCH₃, OCH₂CH₃; SO₂-CH₃, SO-CH₃, COOCH₃, COOCH₂CH₃, -CO-NH-(methylene)-hetaryl, -CO-NH-(ethylene)-hetaryl, -CO-NH-hetaryl, -CO-N(CH₃)-het, -CO-N(CH₃)-(methylene)-het, -CO-N(CH₃)-(ethylene)-het, -CO-N(CH₃)-(methylene)-hetaryl, -CO-N(CH₃)-(ethylene)-hetaryl, -CO-N(cyclopropyl)-het, CO-NH₂, CONH(CH₃), CON(CH₃)₂, -CO-NH-(methylene)-het, -CO-NH-(ethylene)-het. -NH-CO-methyl, NCH₃-CO-methyl, -NH-CO-ethyl, NCH₃-CO-ethyl, -NH-CO-propyl , NCH₃-CO-propyl, -NH-CO-isopropyl, NCH₃-CO-isopropyl, phenyl, phenyl-methylene, phenyl-ethylene, het-methylene, het-ethylene, -het, -CO-het, -CO-N(CH₃)-het, CO-N(CH₃)-cyclopropyl, C₃₋₇-cycloalkyl, C₃₋₇-cycloalkyl-methylene, C₃₋₇-cycloalkyl-ethylene, hetaryl-methylene, hetaryl-ethylene, -hetaryl, CH₂-NH2., CH₂-NH(CH₃), CH₂-N(CH₃)₂, -NH₂, -NH(CH₃) and -N(CH₃)₂, while this group may optionally be substituted by one or more groups selected from among OH, F, Cl, -CF₃, CHF₂, CH₂F, oxo, methyl and phenyl
or wherein
**R³** denotes a group selected from among a het and hetaryl, which may optionally be substituted by one or more groups selected from among F, Cl, Br, CF₃, CHF₂, CH₂F, CN, OH, oxo, methyl, ethyl, propyl, isopropyl, butyl, isobutyl, cyclopropyl, -O-methyl, -O-ethyl, -O-propyl, -O-isopropyl, -COO-methyl, -COO-ethyl, -COO-propyl, -COO-isopropyl, SO-(CH₃), SO-(CH₂-CH₃), SO₂-(CH₃), SO₂-(CH₂-CH₃), phenyl, CH₂-NH₂, CH₂-NH(CH₃), CH₂-N(CH₃)₂, -NH₂, -NH(CH₃), -N(CH₃)₂, het and hetaryl, which may in turn optionally be substituted by one or more groups selected from among OH, F, Cl, CF₃, CHF₂, CH₂F, methyl, ethyl, propyl, isopropyl, phenyl, -COO-methyl, -COO-ethyl and O-methyl, O-ethyl,
or wherein
**R³** denotes -O-R^{3.1},
wherein **R^{3.1}** denotes a group selected from among -C₁₋₃-alkyl, -phenyl, -C₁₋₃-alkylene-phenyl, hetaryl and het, which may optionally be substituted in the ortho, para or meta position by one, two or three groups selected independently of one another from among fluorine, chlorine, bromine, hydroxy, CN, methyl, ethyl, propyl, isopropyl, butyl, isobutyl, CF₃, CHF₂, CH₂F, CO-(methyl), CO-(ethyl), CO-(propyl), CO-(isopropyl), -CO-(CF₃), -CO-NH-(methylene)-hetaryl, -CO-NH-(ethylene)-hetaryl, -CO-N(CH₃)-(methylene)-hetaryl, -CO-N(CH₃)-(ethylene)-hetaryl, -CO-N(CH₃)-(propylene)-hetaryl, -CO-N(CH₃)-(isopropylene)-hetaryl -CO-N(CH₃)-het, -CO-N(cyclopropyl)-het, -CO-N(C₅₋₇-cycloalkyl)-het, -methylene-O-methyl, -ethylene-O-methyl, -propylene-O-methyl, -methylene-O-ethyl, -ethylene-O-ethyl, -propylene-O-ethyl,-methylene-NH₂, -methylene-NHCH₃, -methylene-N(CH₃)₂, -ethylene-NH₂, -ethylene-NHCH₃, -ethylene-N(CH₃)₂, NH₂, N(CH₃)₂, NHCH₃, -O-methyl, O-ethyl, O-propyl, O-isopropyl, O-butyl, O-isobutyl, -SO-CH₃, SO-ethyl, -SO-propyl, -SO-isopropyl, , SO₂-methyl, -SO₂-ethyl, SO₂-propyl, SO₂-isopropyl, COOH, COO-(methyl), COO-(ethyl), COO-(propyl), COO-(isopropyl), -O-methylene-N(methyl)₂, -O-ethylene-N(methyl)₂, -O-methylene-N(ethyl)₂, -O-ethylene-N(ethyl)₂, CO-NH₂, CO-NH(CH₃), CO-N(CH₃)₂, -NH-CO-methyl, -NCH₃-CO-methyl, -NH-CO-ethyl, NCH₃-CO-ethyl, phenyl, phenyl-methylene, phenyl-ethylene, het-methylene, het-ethylene, -CO-het, het, -CO-C₅₋₇-cycloalkyl, -CO-cyclopropyl, -CO-N(CH₃)-C₅₋₇-cycloalkyl, -CO-N(CH₃)-cyclopropyl, C₅₋₇-cycloalkyl, cyclopropyl, C₅₋₇-cycloalkyl-methylene, C₅₋₇-cycloalkyl-ethylene, cyclopropyl-methylene, cyclopropyl-ethylene, hetaryl-methylene, hetaryl-ethylene and hetaryl, which may in turn optionally be substituted by 1, 2, 3 or 4 groups selected independently of one another from among F, Cl, Br, methyl, O-methyl, ethyl, O-ethyl, OH, oxo and CF₃,
and wherein
**R⁴** denotes H, CN, OH, CF₃, CHF₂, CH₂F, F, methyl, ethyl, O-methyl or O-ethyl, -methylene-OH, -ethylene-OH, -propylene-OH, isopropylene-OH, -COO(methyl), -COO(ethyl), -COO(propyl), -COO(isopropyl), -CO-het, -(methylene)-NH-SO₂-(methyl), -(methylene)-NH-SO₂-(ethyl), -(ethylene)-NH-SO₂-(methyl), -(ethylene)-NH-SO₂-(ethyl), -(methylene)-N(CH₃)-SO₂-(methyl), -(methylene)-N(CH₃)-SO₂-(ethyl), -(ethylene)-N(CH₃)-SO₂-(methyl), -(ethylene)-N(CH₃)-SO₂-(ethyl), -(methylene)-O-(methylene)-phenyl, -(methylene)-O-(ethylene)-phenyl, -(ethylene)-O-(methylene)-phenyl, -(ethylene)-O-(ethylene)-phenyl, -methylene-O-methyl, -methylene-O-ethyl, -ethylene-O-methyl -ethylene-O-ethyl, -(methylene)-N(CH₃)-CO-(methyl), -(methylene)-N(CH₃)-CO-(ethyl) -(ethylene)-N(CH₃)-CO-(methyl), -(ethylene)-N(CH₃)-CO-(ethyl), -NH-CO-(methylene)-O-(methyl), -NH-CO-(methylene)-O-(ethyl), -NH-CO-(ethylene)-O-(methyl), -NH-CO-(ethylene)-O-(ethyl), -methylene-NH-CO-(methyl), -methylene-NH-CO-(ethyl), -ethylene-NH-CO-(methyl), -ethylene-NH-CO-(ethyl), -methylene-NH-CO-(methylene)-N(methyl)₂, -methylene-NH-CO-(ethylene)-N(methyl)₂, -ethylene-NH-CO-(methylene)-N(methyl)₂, -ethylene-NH-GO-(ethylene)-N(methyl)₂, -methylene-NH-CO-(methylene)-O-(methyl), -methylene-NH-CO-(ethylene)-O-(methyl), -ethylene-NH-CO-(methylene)-O-(methyl), -methylene-NH-CO-(methylene)-O-(ethyl), -methylene-NH-CO-(ethylene)-O-(ethyl), -ethylene-NH-CO-(methylene)-O-(ethyl), -(methylene)-N(CH₃)-CO-(methylene)-O-(methyl), -(methylene)-N(CH₃)-CO-(ethylene)-O-(Methyl), -(ethylene )-N(CH₃)-CO-(methylene)-O-(methyl), -(methylene)-N(CH₃)-CO-(methylene)-O-(ethyl), -(methylene)-N(CH₃)-CO-(ethylene)-O-(ethyl), -(ethylene)-N(CH₃)-CO-(Methylene)-O-(ethyl), -O-(methylene)-phenyl, -O-(ethylene)-phenyl, -CO-phenyl,
wherein the phenyl in the above groups may optionally be substituted by one or more other groups selected from among F, Cl, Br, methyl, ethyl, propyl, -O-methyl, -O-ethyl, -O-propyl, -OH and CF₃
or wherein
R³ and R⁴ together form a mono- or bicyclic, unsaturated, saturated or partially saturated heterocycle, which contains 1, 2 or 3 heteroatoms selected from among N, O and S and which may optionally be substituted by one or more groups selected from among F, Cl, Br, OH, oxo, CF₃, CHF₂, CH₂F, CN, methyl, ethyl, propyl, isopropyl, cyclopropyl, COO-methyl, -COO-ethyl, O-methyl, O-ethyl, SO₂-(CH₃), SO₂-(CH₂CH₃), SO-(CH₃), SO-(CH₂CH₃), CH₂-NH₂, CH₂-NH(CH₃), CH₂-N(CH₃)₂, -NH₂, -NH(CH₃), -N(CH₃)₂, phenyl, C₅₋₇-cycloalkyl, het and hetaryl,
as well as pharmacologically acceptable salts, diastereomers, enantiomers, racemates, hydrates or solvates thereof.

4. Compounds of formula **1** according to one of Claims 1 to 3, wherein
R² denotes a group according to formula **2** 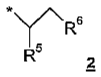
wherein **R⁶** is OH or NH₂ and
wherein **R⁵** is a group selected from among C₁₋₄-alkyl, a five- to six-membered heteroaryl with 1, 2 or 3 heteroatoms selected from among S, O and N and phenyl, which may optionally be substituted by one or more groups selected from among OH, F, Br, OR²_{.\} oxo, methyl, ethyl, methanol, ethanol, phenyl, COOR^{2.1}. CH₂-NR^{2.2}R^{2.3} and NR^{2.2}R^{2.3},
as well as pharmacologically acceptable salts, diastereomers, enantiomers, racemates, hydrates or solvates thereof.

5. Compounds of formula **1** according to one of claims 1 to 3, wherein
**R²** is a monocyclic three-, four-, five-, six- or seven-membered cycloalkyl ring which may optionally be substituted in the spiro position by a group selected from among -CH₂-OR^{2.1}, branched or unbranched C₂₋₆-alkylene-OR^{2.1}, methyl, ethyl, propyl, isopropyl, butyl, isobutyl, cyclopropyl, -CF₃, CHF₂, CH₂F and C₂₋₄-fluoroalkyl, wherein
R^{2.1} is selected from among methyl, ethyl, propyl, isopropyl, butyl, isobutyl,
as well as pharmacologically acceptable salts, diastereomers, enantiomers, racemates, hydrates or solvates thereof.

6. Compounds of formula **1** according to one of Claims 1, 2 or 3, wherein
**R²** is a cyclopropyl which may optionally be substituted by another group selected from among -NH₂, CH₂-NH₂, -NH(CH₃), -N(CH₃)₂, methyl, ethyl, propyl, isopropyl, -NH-CO-(tert-butyl), -NH-CO-O-(tert-butyl), -N(CH₃)-CO-(tert-butyl), -N(CH₃)-CO-O-(tert-butyl), -CF₃, -CHF₂, CH₂F, F, Cl and Br,
as well as pharmacologically acceptable salts, diastereomers, enantiomers, racemates, hydrates or solvates thereof.

7. Compounds of formula **1** according to one of Claims 1 to 3, wherein
**R²** denotes a phenyl which may optionally be substituted in one or both meta positions by one or more groups selected from among methyl, ethyl, propyl, isopropyl, cyclopropyl, F, Cl, Br, OH, OR^{2.1}, COOR^{2.1}, CF₃, CHF₂, CH₂F, NH₂, NH(CH₃) and N(CH₃)₂, wherein R^{2.1} may be H, methyl or ethyl,
as well as pharmacologically acceptable salts, diastereomers, enantiomers, racemates, hydrates or solvates thereof.

8. Compounds of formula **1** according to one of Claims 1 to 3, wherein
**R²** is a group selected from among monocyclic, saturated three-, four-, five-, six- or seven-membered heterocycles with 1, 2 or 3 heteroatoms selected in each case from among N, O and S, which may optionally be substituted by one or more groups selected from among fluorine, chlorine, bromine, CF₃, CHF₂, CH₂F, OH and oxo or by one or more groups selected from among OR^{2.1}, C₁₋₃-alkylene-OR^{2.1}, SR^{2.1}, SO-R^{2.1}, SO₂-R^{2.1}, COOR^{2.1}, COR^{2.1}, C₁₋₆-alkanol, C₃₋₁₀-cycloalkyl, phenyl, C₁₋₆-alkyl, phenyl-C₁₋₆-alkylene, hetaryl-C₁₋₆-alkylene, het, hetaryl and NR^{2.2}R^{2.3}, which may in turn optionally be substituted by one or more groups selected from among OH, OR^{2.1}, oxo, F, Cl. CF₃, CHF₂, CH₂F, C₁₋₆-alkyl, phenyl and NR^{2.2}R^{2.3},
wherein **R^{2.1}, R^{2.2}** and **R^{2.3}** are defined as in claim 1,
as well as pharmacologically acceptable salts, diastereomers, enantiomers, racemates, hydrates or solvates thereof.

9. Compounds of formula **1** according to claim 8, wherein
**R²** denotes a group selected from among a monocyclic, saturated six-membered heterocycle with a heteroatom selected from among N, O and S, which may optionally be substituted by one or more groups selected from among F, Cl, Br, CF₃, CHF₂, CH₂F, OH, oxo, NH₂, NHCH₃ and N(CH₃)₂, methyl, ethyl, propyl, isopropyl, cyclopropyl, methoxy and ethoxy,
as well as pharmacologically acceptable salts, diastereomers, enantiomers, racemates, hydrates or solvates thereof.

10. Compounds of formula **1** according to claim 8 or 9, wherein
**R²** denotes a group selected from among piperidine or tetrahydropyran, which may optionally be substituted by one or more groups selected from among F, Cl, Br, OH, CF₃, CHF₂, CH₂F, NH₂, NHCH₃, N(CH₃)₂, oxo, methyl and methoxy,
as well as pharmacologically acceptable salts, diastereomers, enantiomers, racemates, hydrates or solvates thereof.

11. Compounds of formula **1** according to one of Claims 1 to 3, wherein
**R³** is a naphthalene or phenyl, which may optionally be substituted in any position by one, two or three groups selected independently of one another from among fluorine, chlorine, bromine, hydroxy, CN, methyl, ethyl, propyl, isopropyl, cyclopropyl, CF₃, CHF₂, CH₂F, -OCH₃, OCH₂CH₃; SO₂-CH₃, SO₂-CH₂CH₃ COOCH₃ and CO-O-CH₂CH₃,
as well as pharmacologically acceptable salts, diastereomers, enantiomers, racemates, hydrates or solvates thereof.

12. Compounds of formula **1** according to one of Claims 1 or 2, wherein
**R³** denotes a group selected from among het and hetaryl, which may optionally be substituted by one or more groups selected from among F, Cl, Br, CF₃, CHF₂, CH₂F, CN, OH, oxo, methyl, ethyl, propyl, isopropyl, butyl, isobutyl, cyclopropyl, C₅₋₇-cycloalkyl, -O-methyl, -O-ethyl, -O-propyl, -O-isopropyl, -COO-methyl, -COO-ethyl, -COO-propyl, -COO-isopropyl, SO₂-(CH₃), SO₂-(CH₂-CH₃), SO-(CH₃), SO-(CH₂-CH₃), phenyl, -CH₂-NH₂, -CH₂-NHCH₃, -CH₂-N(CH₃)₂, NH₂, NHCH₃, N(CH₃)₂, het and hetaryl, which may in turn optionally be substituted by one or more groups selected from among OH, F, Cl, Br, CF₃, CHF₂, CH₂F, methyl, ethyl, propyl, isopropyl, phenyl, -COO-methyl, -COO-ethyl, -COO-propyl, -COO-isopropyl and O-methyl, O-ethyl, O-propyl and O-isopropyl,
and wherein
**R⁴** denotes H, CN, OH, CF₃. CHF₂, CH₂F, F, methyl, ethyl, O-methyl or O-ethyl,
wherein
**het** is a three- to seven-membered, monocyclic, saturated or partially saturated heterocycle or a seven- to eleven-membered, bicyclic, anellated, saturated or partially saturated heterocycle which contains 1, 2 or 3 heteroatoms selected independently of one another from among N, S or O,
and wherein
**hetaryl** is a five- to six-membered, monocyclic, aromatic heteroaryl or a seven- to eleven-membered, bicyclic, anellated, aromatic heteroaryl, which contains in each case 1, 2 or 3 heteroatoms selected independently of one another from among N, S or O,
and wherein
**cycloalkyl** may be saturated or partially saturated,
as well as pharmacologically acceptable salts, diastereomers, enantiomers, racemates, hydrates or solvates thereof.

13. Compounds of formula **1** according to claim 12, wherein
**R³** denotes a group selected from a bicyclic, seven-to eleven-membered, saturated or partially saturated heterocycle or a bicyclic, seven- to eleven-membered heteroaryl, which is selected from among indole, dihydroindole, quinazoline, dihydroquinazoline, tetrahydroquinazoline, benzisoxazole, dihydrobenzisoxazole, benzoxazine, dihydrobenzoxazine, benzothiazole, dihydrobenzothiazole, triazolopyridine, dihydrotriazolopyridine, benzofuran, dihydrobenzofuran, isobenzofuran and dihydroisobenzofuran, which may optionally be substituted by one or more groups selected from among F, Cl, Br, CF₃, CHF₂, CH₂F, CN, OH, oxo, methyl, ethyl, propyl, isopropyl, butyl, isobutyl, cyclopropyl, -O-methyl, -O-ethyl, -O-propyl, -O-isopropyl, -COO-methyl, -COO-ethyl, -COO-propyl, -COO-isopropyl, SO₂-(CHa), SO₂-(CH₂-CH₃), SO-(CH₃), SO-(CH₂-CH₃), phenyl, -CH₂-NH₂, -CH₂-NHCH₃, -CH₂-N(CH₃)₂, NH₂, NHCH₃, N(CH₃)₂, furanyl and pyridinyl, which in turn may be substituted by one or more groups selected from among OH, F, Cl, Br, CF₃, CHF₂, CH₂F, methyl, ethyl, propyl, isopropyl, phenyl, -COO-methyl, -COO-ethyl and O-methyl, O-ethyl,
as well as pharmacologically acceptable salts, diastereomers, enantiomers, racemates, hydrates or solvates thereof.

14. Compounds of formula **1** according to claim 12, wherein
**R³** denotes a group selected from a monocyclic, saturated or partially saturated, three- to seven-membered heterocycle or a monocyclic five- to six-membered heteroaryl, which is selected from among imidazole, dihydroimidazole, oxadiazole, oxadiazolidine, pyrazole, pyridine and dihydropyrazole, which may optionally be substituted by one or more groups selected from among F, Cl, Br, CF₃, CHF₂, CH₂F, CN, OH, oxo, methyl, ethyl, propyl, isopropyl, butyl, isobutyl, cyclopropyl, -O-methyl, -O-ethyl, -O-propyl, -O-isopropyl, -COO-methyl, -COO-ethyl, -COO-propyl, -COO-isopropyl, SO₂- (CH₃), SO₂-(CH₂-CH₃), SO-(CH₃), SO-(CH₂-CH₃), phenyl, -CH₂-NH₂, -CH₂-NHCH₃, -CH₂-N(CH₃)₂, NH₂, NHCH₃, N(CH₃)₂, furanyl and pyridinyl, which may in turn optionally be substituted by one or more groups selected from among OH, F, Cl, Br, CF₃, CHF₂, CH₂F, methyl, ethyl, propyl, isopropyl, phenyl, -COO-methyl, -COO-ethyl and O-methyl, O-ethyl,
as well as pharmacologically acceptable salts, diastereomers, enantiomers, racemates, hydrates or solvates thereof.

15. Compounds of formula **1** according to one of Claims 1 to 3, wherein
**R³** and **R⁴** together form a mono- or bicyclic, unsaturated or partially saturated, three-to eleven-membered heterocycle which contains 1, 2 or 3 heteroatoms selected from among N, O and S and which may optionally be substituted by one or more groups selected from among F, Cl, Br, OH, oxo, CF₃, CHF₂, CH₂F, CN, methyl, ethyl, propyl, isopropyl, cyclopropyl, COO-methyl, -COO-ethyl, O-methyl, O-ethyl, SO₂-(CH₃), SO₂-(CH₂-CH₃), SO-(CH₃), SO-(CH₂-CH₃), phenyl, -CH₂-NH₂, -CH₂NHCH₃, -CH₂-N(CH₃)₂, NH₂, NHCH₃, N(CH₃)₂, a saturated or partially saturated, five- to six-membered heterocycle and a five- to six-membered heteroaryl,
as well as pharmacologically acceptable salts, diastereomers, enantiomers, racemates, hydrates or solvates thereof.

16. Compounds of formula **1** according to claim 15, wherein
**R³** and **R⁴** together form a bicyclic heterocycle selected from among tetrahydroquinazoline, tetrahydrobenzoxazine and dihydroindole, dihydroisobenzofuran, which may optionally be substituted by one or more groups selected from among F, Cl, Br, OH, oxo, CF₃, CHF₂, CH₂F, CN, methyl, ethyl, propyl, isopropyl, cyclopropyl, COO-methyl, -COO-ethyl, O-methyl, O-ethyl, SO₂-(CH₃), SO₂-(CH₂-CH₃), phenyl, -CH₂-NH₂, -CH₂NHCH₃, -CH₂-N(CH₃)₂, NH₂, NHCH₃, N(CH₃)₂, a saturated or partially saturated, five-to six-membered heterocycle and a five- to six-membered heteroaryl,
as well as pharmacologically acceptable salts, diastereomers, enantiomers, racemates, hydrates or solvates thereof.

17. Compounds of formula **1** according to one of Claims 1 or 2, wherein
**R³** is -O-R^{3.1},
wherein **R^{3.1}** is a group selected from among methyl, ethyl, propyl, isopropyl, butyl, isobutyl, pentyl, isopentyl, -phenyl, -methylene-phenyl, -ethylene-phenyl, -propylene-phenyl, -isopropylene-phenyl, hetaryl and het, which may optionally be substituted in the ortho, para or meta position by one, two or three groups selected independently of one another from among fluorine, chlorine, bromine, hydroxy, CN, methyl, ethyl, propyl, isopropyl, butyl, isobutyl, -CF₃, CHF₂, CH₂F, CO-(methyl), CO-(ethyl), CO-(propyl), CO-(isopropyl), CO-(butyl), CO-(isobutyl), -CO-(CF₃), -CO-(CH₂F), -CO-(CHF₂), -CO-NH-(methylene)-hetaryl, -CO-NH-(ethylene)-hetaryl, -CO-NH-(propylene)-hetaryl, -CO-NH-(isopropylene)-hetaryl, -CO-N(CH₃)-(methylene)-hetaryl, -CO-N(CH₃)-(ethylene)-hetaryl, -CO-N(CH₃)-(propylene)-hetaryl, -CO-N(CH₃)-(isopropylene)-hetaryl, CO-N(CH₃)-het, -CO-N(C₃₋₇-cycloalkyl)-het, -methylene-O-methyl, -ethylene-O-methyl, -methylene-O-ethyl, -ethylene-O-ethyl, -methylene-NH₂, -ethylene-NH₂, -methylene-NHCH₃, -ethylene-NHCH₃, -methylene-N(CH₃)₂,-ethylene-N(CH₃)₂, -NH₂, -NHCH₃, -N(CH₃)₂, -O-methyl, -O-ethyl, -O-propyl, -O-isopropyl, -SO-CH₃, -SO-(CH₂CH₃), -SO₂-CH₃, -SO₂-(CH₂CH₃), COOH, COO-(methyl), COO-(ethyl), COO-(propyl), COO-(isopropyl), -O-methylene-N(methyl)₂, -O-ethylene-N(methyl)₂,-O-methylene-N(ethyl)₂, -O-ethylene-N(ethyl)₂, CO-NH₂, CO-NHCH₃, CO-N(CH₃)₂, NH-CO-methyl, NCH₃-CO-methyl, NH-CO-ethyl, N(CH₃)-CO-ethyl, phenyl, phenyl-methylene, phenyl-ethylene, het-methylene, het-ethylene, -CO-het, het, -CO-C₄₋₇-cycloalkyl, -CO-cyclopropyl, -CO-N(CH₃)-cyclopropyl, -CO-N(CH₃)-C₄₋₇-cycloalkyl, C₄₋₇-cycloalkyl, cyclopropyl, C₄₋₇-cycloalkyl-methylene, cyclopropyl-methylene, C₄₋₇-cycloalkyl-ethylene, cyclopropyl-ethylene, hetaryl-methylene, hetaryl-ethylene and hetaryl, which may in turn optionally be substituted by 1, 2, 3 or 4 groups selected independently of one another from among F, Cl, Br, methyl, O-methyl, ethyl, O-ethyl, OH, oxo and CF₃,
as well as pharmacologically acceptable salts, diastereomers, enantiomers, racemates, hydrates or solvates thereof.

18. Compounds of formula **1** according to one of Claims 1, 2 or 3, wherein
**R⁴** denotes H, CN, OH, CF₃, CHF₂, CH₂F, F, methyl, ethyl, O-methyl or O-ethyl, -methylene-OH, -ethylene-OH, -propylene-OH, isopropylene-OH, -COO(methyl), -COO(ethyl), -COO(propyl), -COO(isopropyl), -CO-het, -(methylene)-NH-SO₂-(methyl), -(methylene)-NH-SO₂-(ethyl),-(ethylene)-NH-SO₂-(methyl), -(ethylene)-NH-SO₂-(ethyl), -(methylene)-N(CH₃)-SO₂-(methyl), -(methylene)-N(CH₃)-SO₂-(ethyl), -(ethylene)-N(CH₃)-SO₂-(methyl), -(ethylene)-N(CH₃)-SO₂-(ethyl), -(methylene)-O-(methylene)-phenyl, -(methylene)-O-(ethylene)-phenyl, -(ethylene)-O-(methylene)-phenyl, -(ethylene)-O-(ethylene)-phenyl, -methylene-O-methyl, -methylene-O-ethyl, -ethylene-O-methyl -ethylene-O-ethyl, -(methylene)-N(CH₃)-CO-(methyl), -(methylene)-N(CH₃)-CO-(ethyl) -(ethylene)-N(CH₃)-CO-(methyl), -(ethylene)-N(CH₃)-CO-(ethyl), -NH-CO-(methylene)-O-(methyl), -NH-CO-(methylene)-O-(ethyl), -NH-CO-(ethylene)-O-(methyl), -NH-CO-(ethylene)-O-(ethyl), -methylene-NH-CO-(methyl), -methylene-NH-CO-(ethyl), -ethylene-NH-CO-(methyl), -ethylene-NH-CO-(ethyl), -methylene-NH-CO-(methylene)-N(methyl)₂, -methylene-NH-CO-(ethylene)-N(methyl)₂, -ethylene-NH-CO-(methylene)-N(methyl)₂, -ethylene-NH-CO-(ethylene)-N(methyl)₂, -methylene-NH-CO-(methylene)-O-(methyl), -methylene-NH-CO-(ethylene)-O-(methyl), -ethylene-NH-CO-(methylene)-O-(methyl), -methylene-NH-CO-(methylene)-O-(ethyl), -methylene-NH-CO-(ethylene)-O-(ethyl), -ethylene-NH-CO-(methylene)-O-(ethyl), -(methylene)-N(CH₃)-CO-(methylene)-O-(methyl), -(methylene)-N(CH₃)-CO-(ethylene)-O-(methyl), -(ethylene)-N(CH₃)-CO-(methylene)-O-(methyl), -(methylene)-N(CH₃)-CO-(methylene)-O-(ethyl), -(methylene)-N(CH₃)-CO-(ethylene)-O-(ethyl), -(ethylene)-N(CH₃)-CO-(methylene)-O-(ethyl), -O-(methylene)-phenyl, -O-(ethylene)-phenyl, -CO-phenyl, while the phenyl in the above groups may optionally be substituted by one or more other groups selected from among F, Cl, Br, methyl, ethyl, propyl, -O-methyl, -O-ethyl, -O-propyl, -OH and CF₃,
as well as pharmacologically acceptable salts, diastereomers, enantiomers, racemates, hydrates or solvates thereof.

19. Compounds of formula **1** according to one of Claims 1, 2 or 3,
wherein
**R³** is a group selected from among oxazole, imidazole and thiazole, while these groups may optionally be substituted by one, two or three further groups selected independently of one another from among methyl, ethyl, propyl, isopropyl, O-methyl, O-ethyl, O-propyl, O-isopropyl, OH, F, Cl, Br, CF₃, phenyl, hetaryl and C₃₋₆-cycloalkyl,
as well as pharmacologically acceptable salts thereof.

20. Compounds of formula **1** according to one of Claims 1 to 3,
wherein the compounds are selected from among 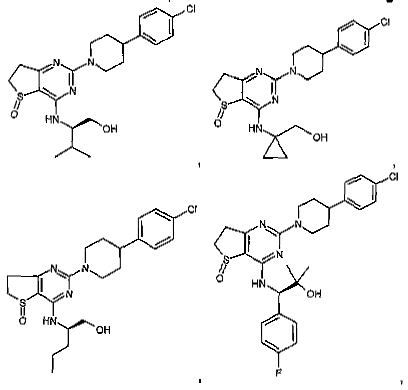 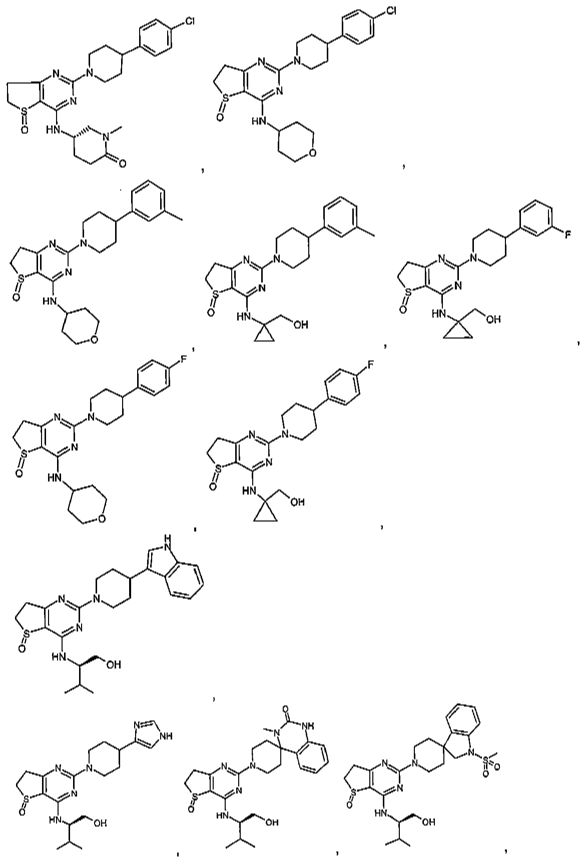 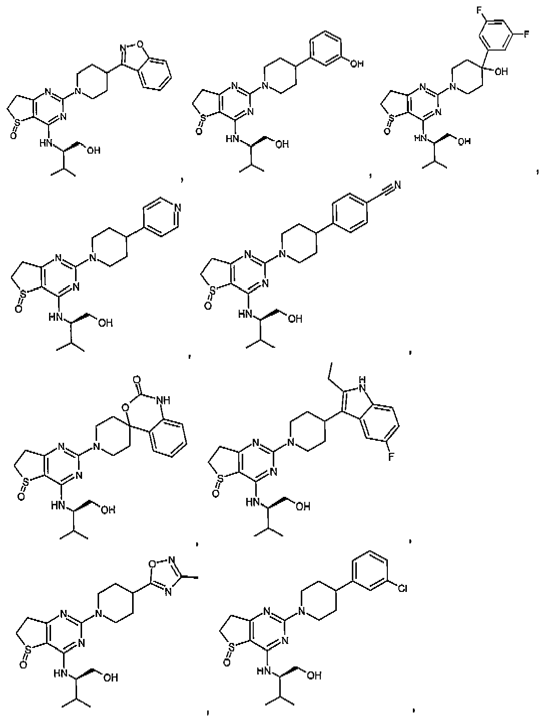 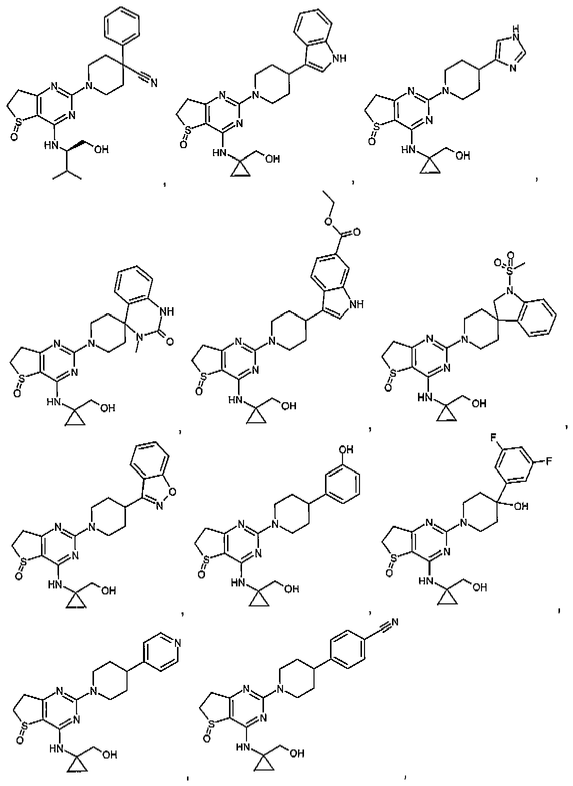 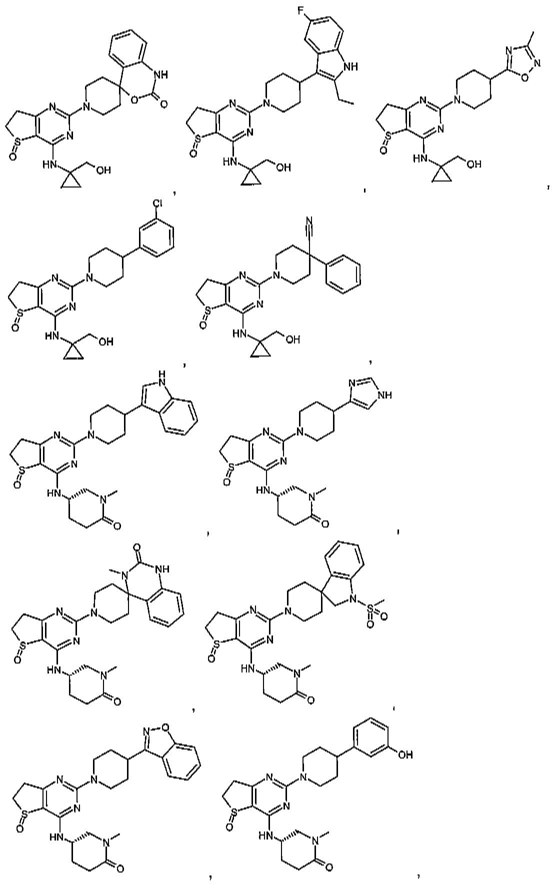 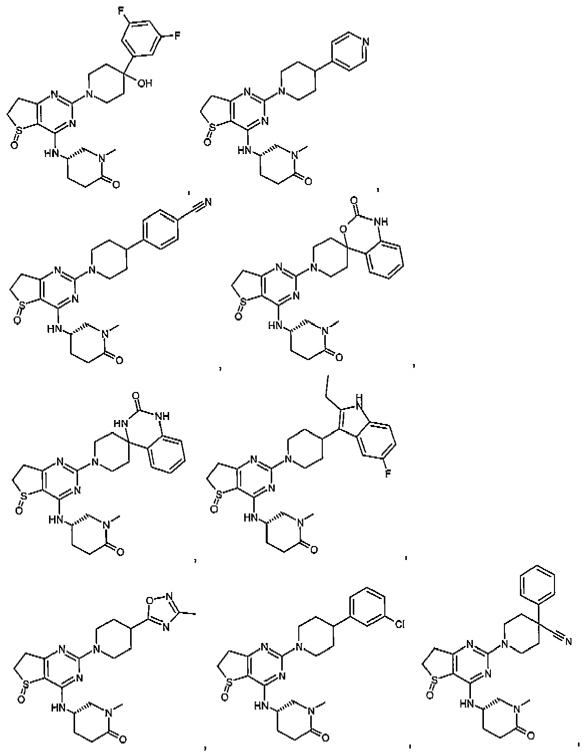 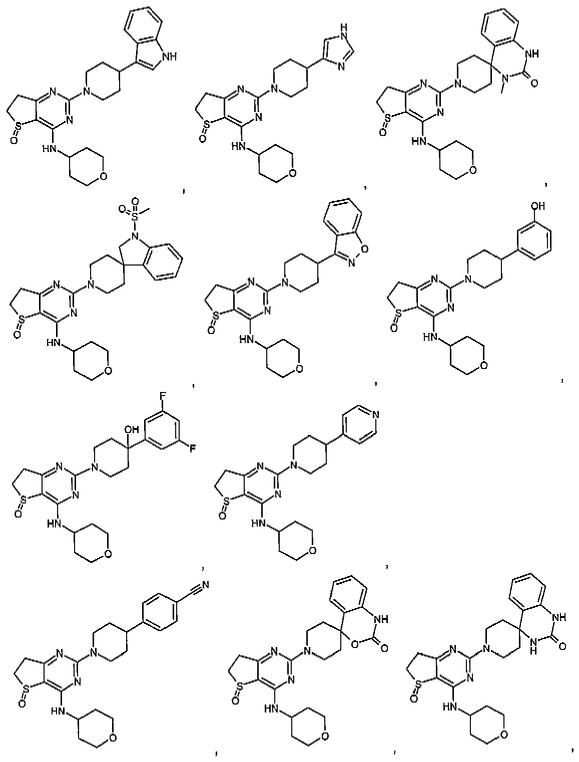 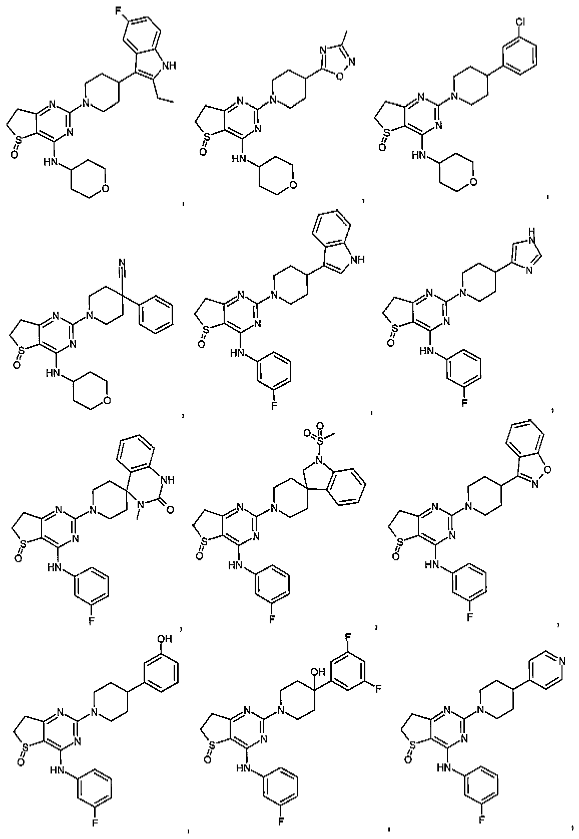 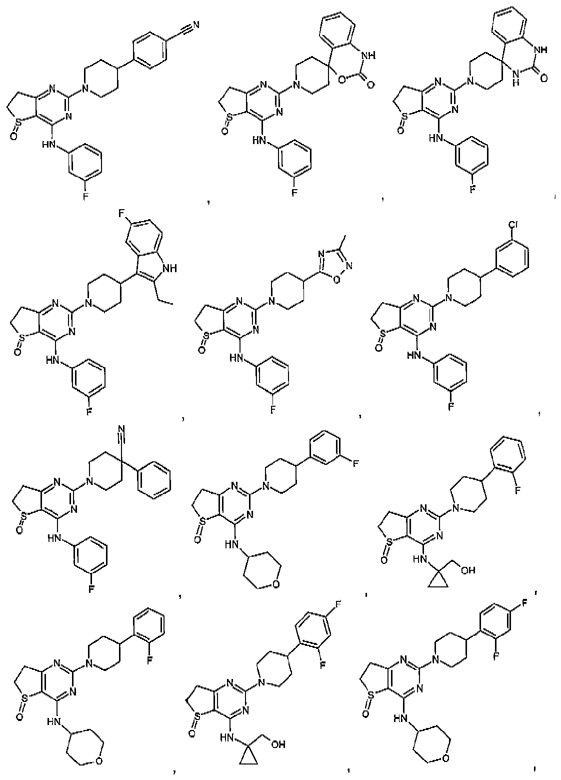 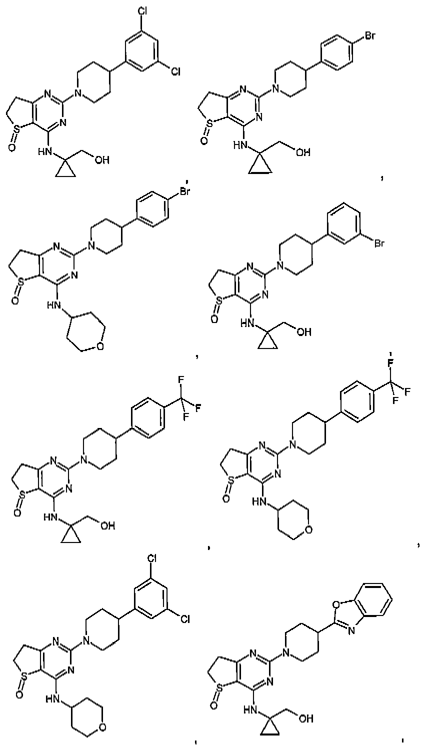 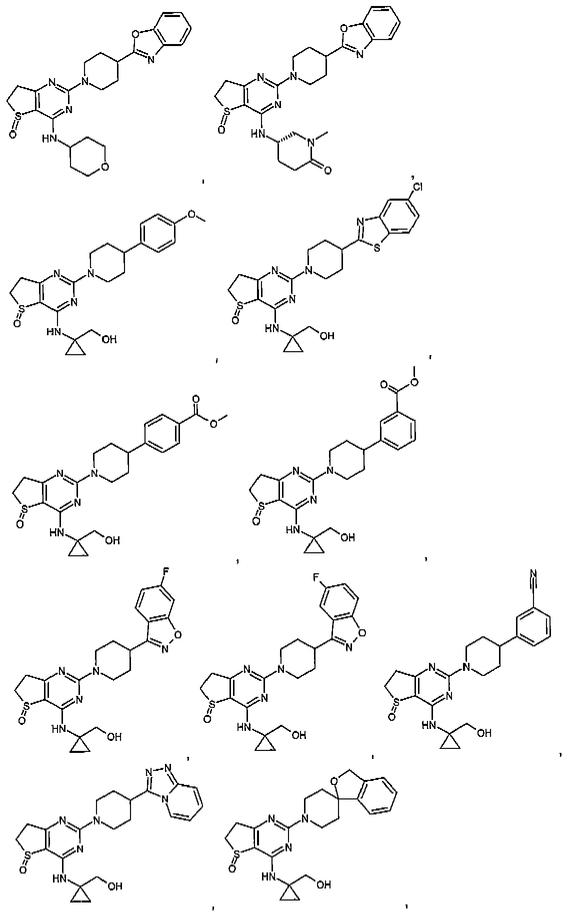 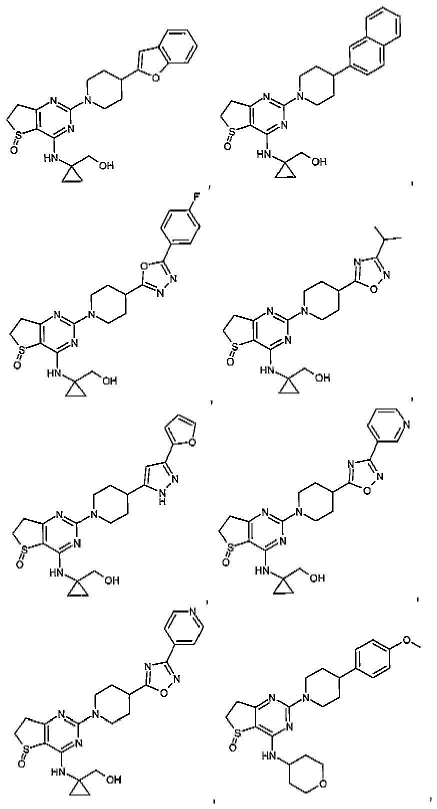 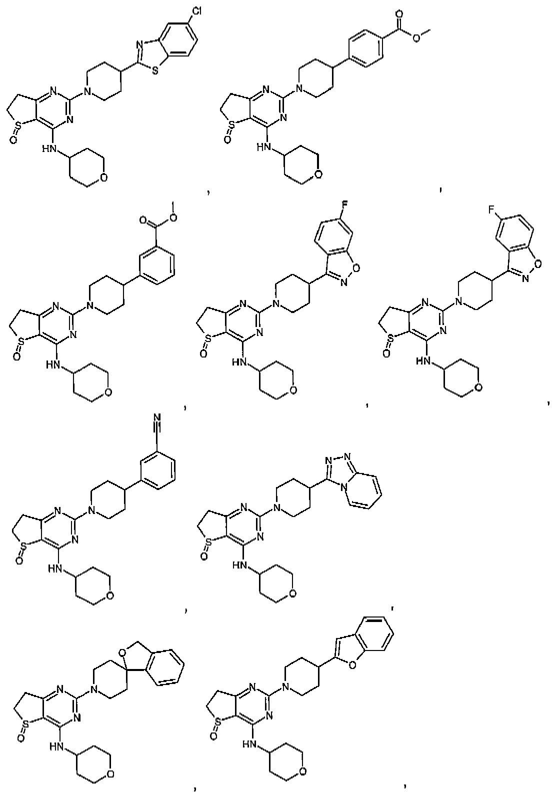 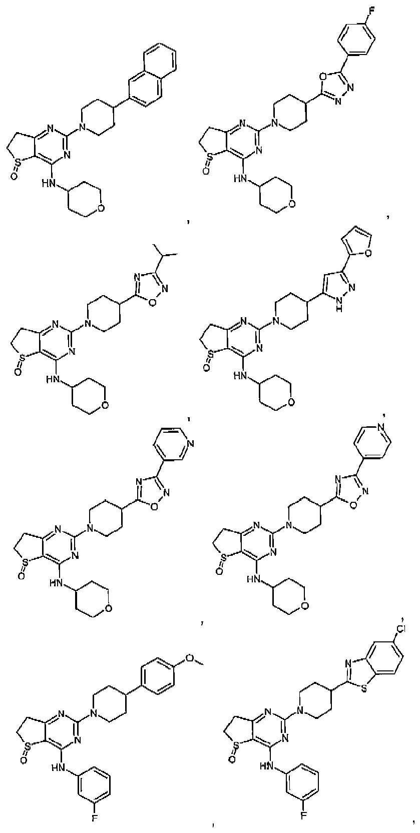 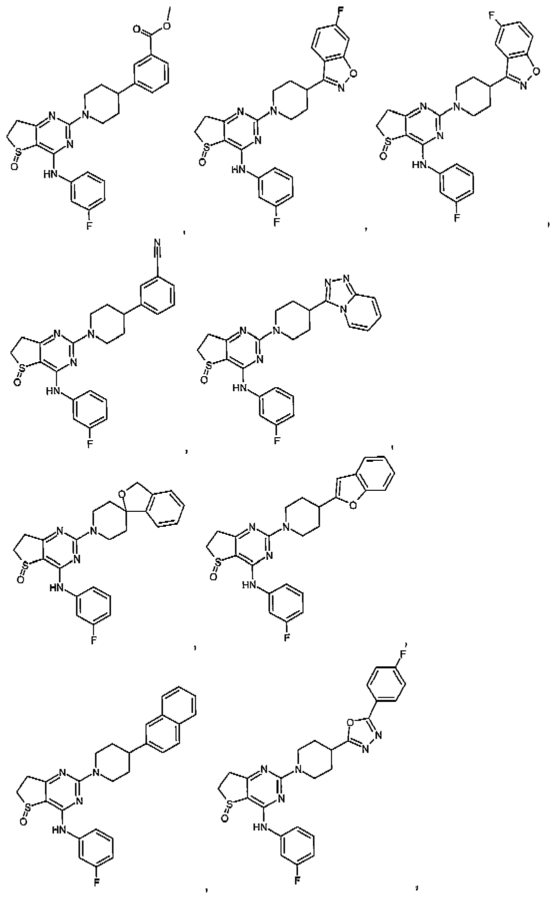 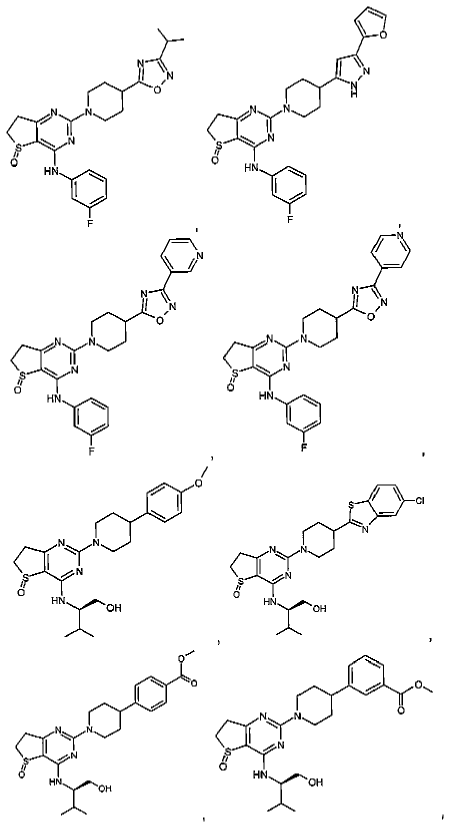 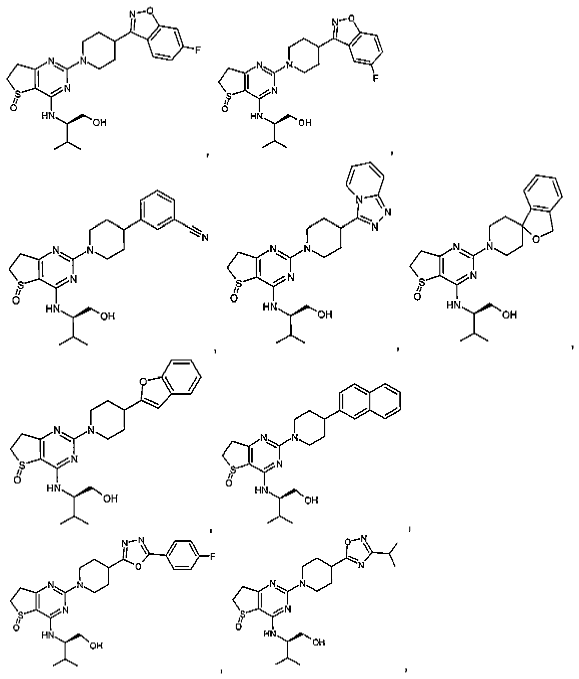 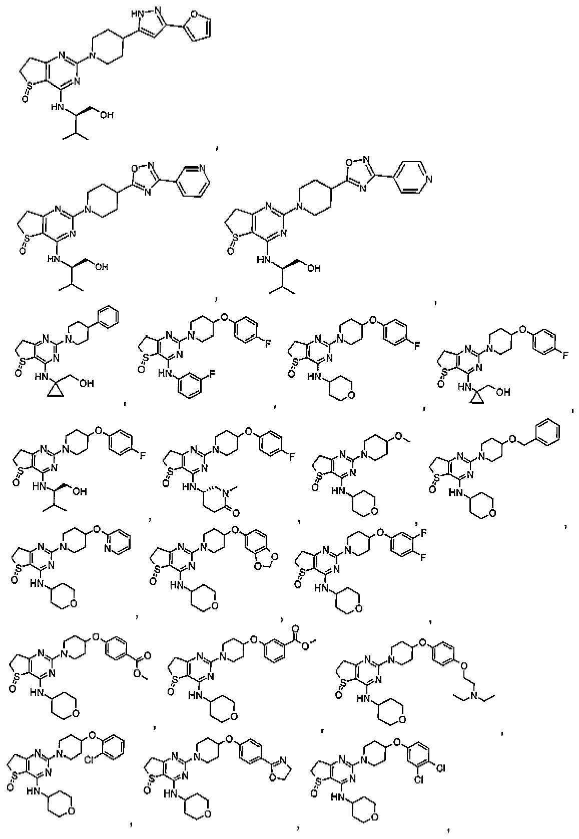 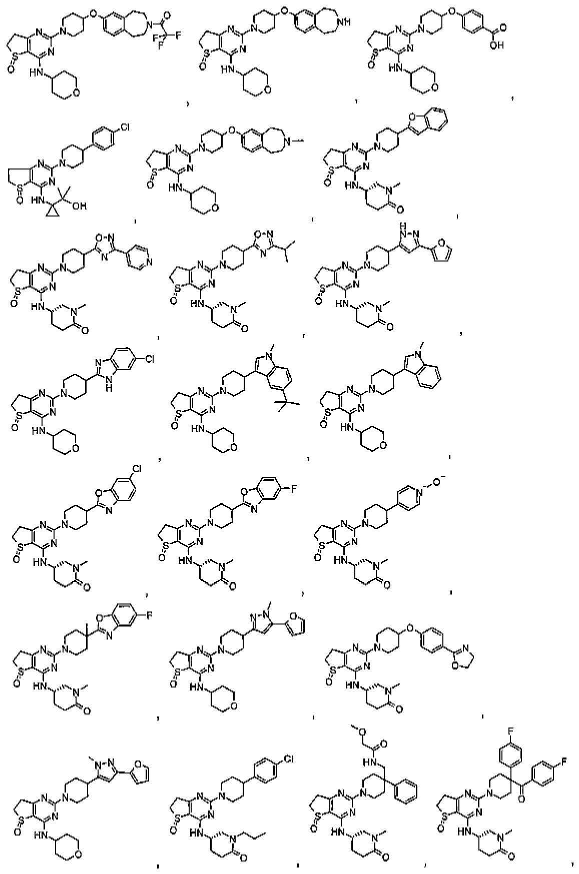 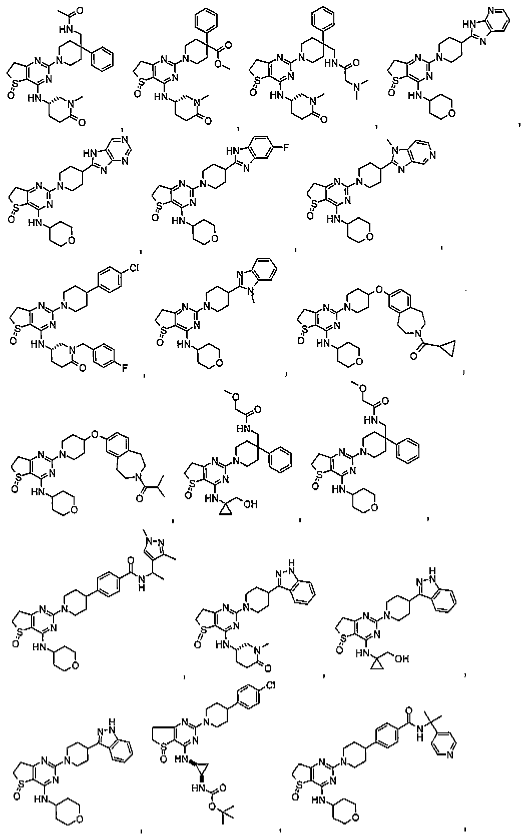 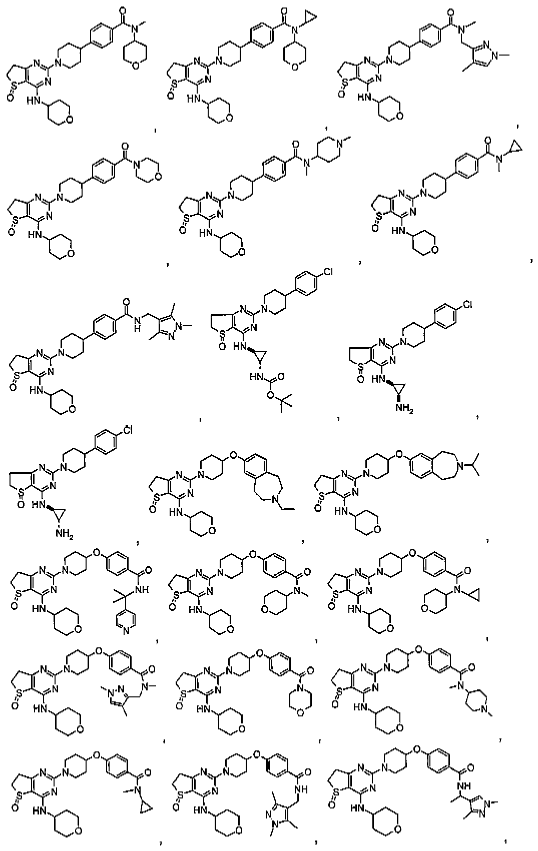 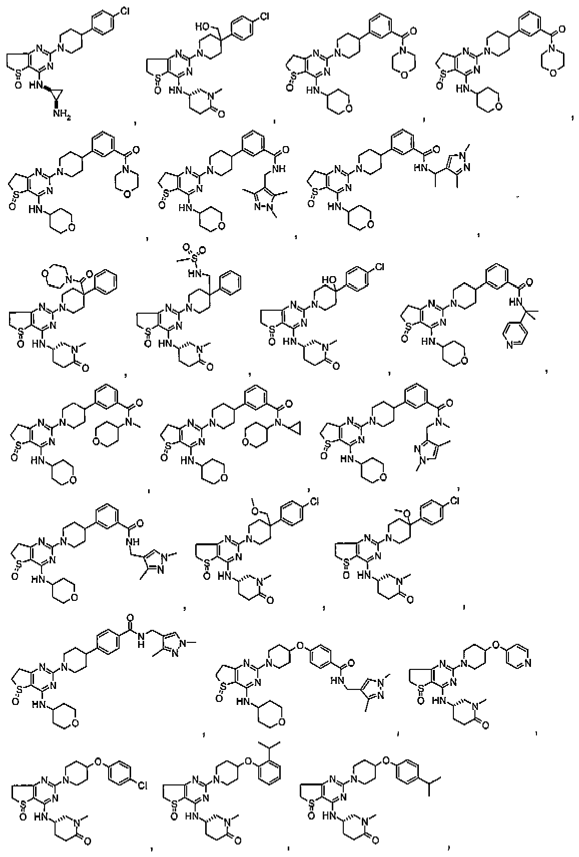 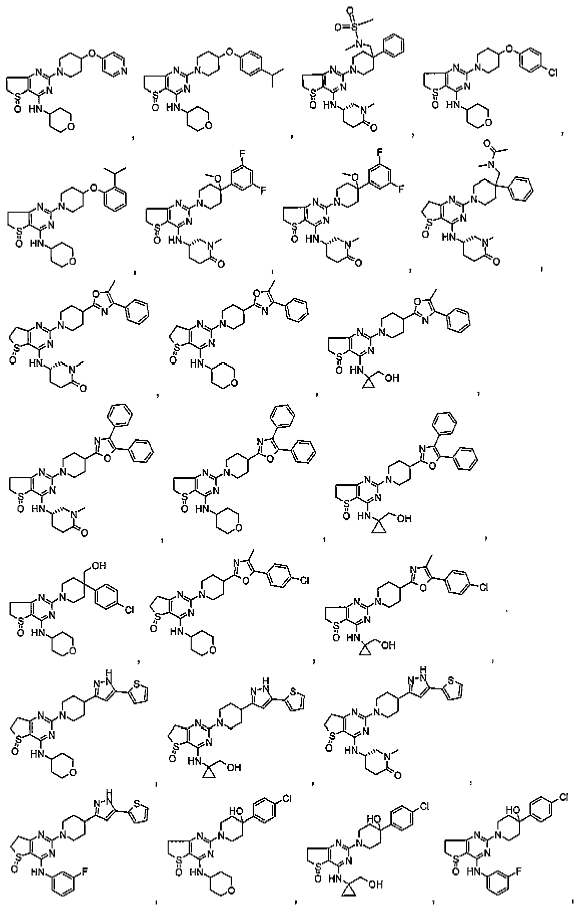 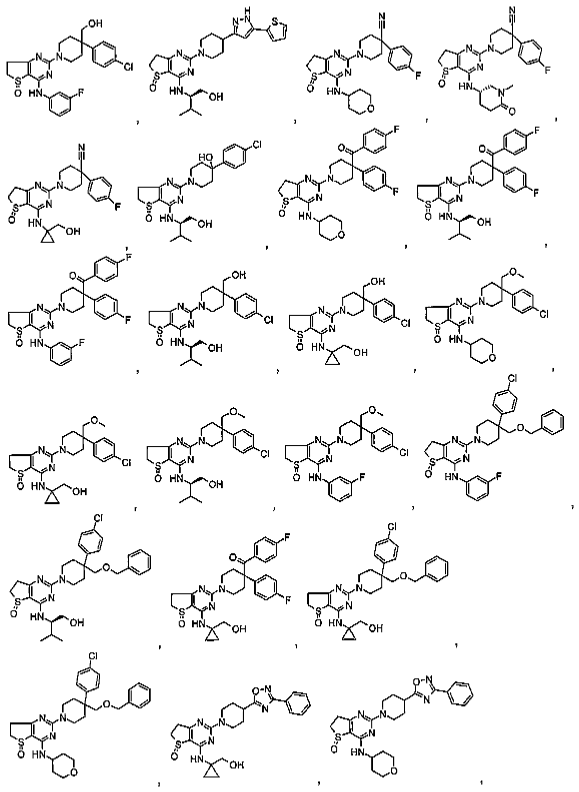 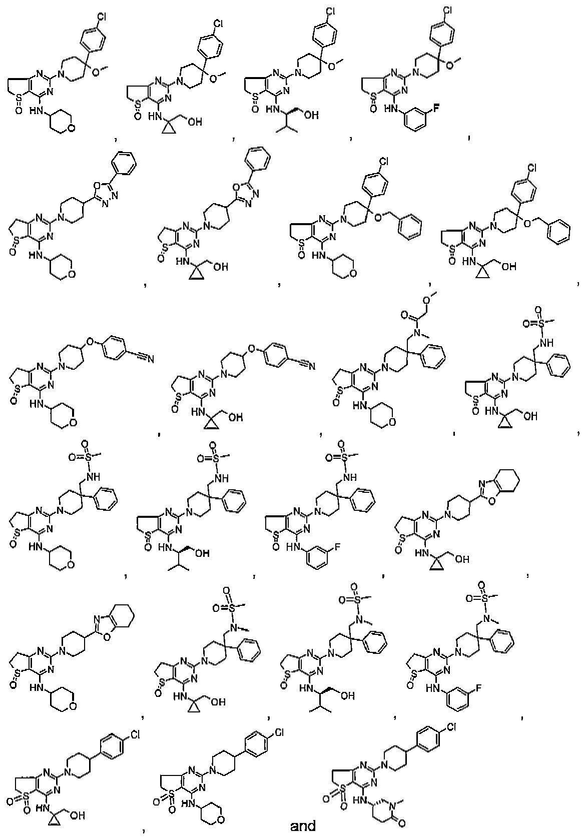 as well as pharmacologically acceptable salts, diastereomers, enantiomers, racemates, hydrates or solvates thereof.

21. Compound of formula **5** according to one of claims 1 to 3 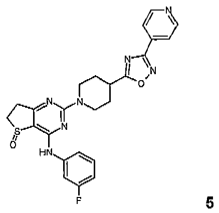 as well as pharmacologically acceptable salts, diastereomers, enantiomers, racemates, hydrates or solvates thereof.

22. Compound of formula **6** according to one of claims 1 to 3 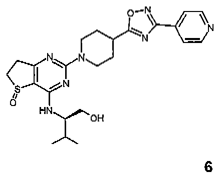 as well as pharmacologically acceptable salts, diastereomers, enantiomers, racemates, hydrates or solvates thereof.

23. Compound of formula **7** according to one of claims 1 to 3 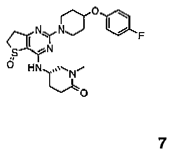 as well as pharmacologically acceptable salts, diastereomers, enantiomers, racemates, hydrates or solvates thereof.

24. Compound of formula **8** according to one of claims 1 to 3 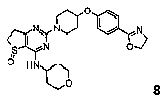 as well as pharmacologically acceptable salts, diastereomers, enantiomers, racemates, hydrates or solvates thereof.

25. Compound of formula **9** according to one of claims 1 to 3 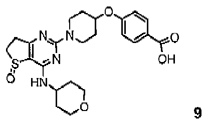 as well as pharmacologically acceptable salts, diastereomers, enantiomers, racemates, hydrates or solvates thereof.

26. Compound of formula **10** according to one of claims 1 to 3 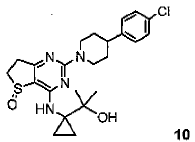 as well as pharmacologically acceptable salts, diastereomers, enantiomers, racemates, hydrates or solvates thereof.

27. Compound of formula **11** according to one of claims 1 to 3 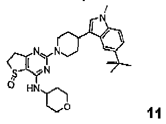 as well as pharmacologically acceptable salts, diastereomers, enantiomers, racemates, hydrates or solvates thereof.

28. Compound of formula **12** according to one of claims 1 to 3 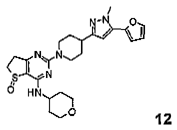 as well as pharmacologically acceptable salts, diastereomers, enantiomers, racemates, hydrates or solvates thereof.

29. Compound of formula **13** according to one of claims 1 to 3 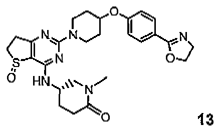 as well as pharmacologically acceptable salts, diastereomers, enantiomers, racemates, hydrates or solvates thereof.

30. Compound of formula **14** according to one of claims 1 to 3 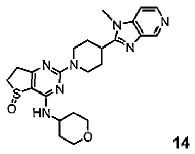 as well as pharmacologically acceptable salts, diastereomers, enantiomers, racemates, hydrates or solvates thereof.

31. Compound of formula **15** according to one of claims 1 to 3 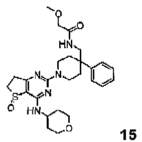 as well as pharmacologically acceptable salts, diastereomers, enantiomers, racemates, hydrates or solvates thereof.

32. Compound of formula **16** according to one of claims 1 to 3 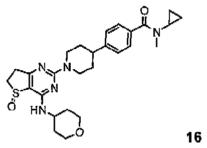 as well as pharmacologically acceptable salts, diastereomers, enantiomers, racemates, hydrates or solvates thereof.

33. Compound of formula **17** according to one of claims 1 to 3 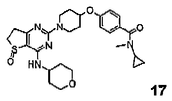 as well as pharmacologically acceptable salts, diastereomers, enantiomers, racemates, hydrates or solvates thereof.

34. Compound of formula **18** according to one of claims 1 to 3 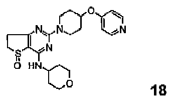 as well as pharmacologically acceptable salts, diastereomers, enantiomers, racemates, hydrates or solvates thereof.

35. Compound of formula **19** according to one of claims 1 to 3 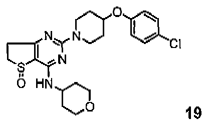 as well as pharmacologically acceptable salts, diastereomers, enantiomers, racemates, hydrates or solvates thereof.

36. Compound of formula **20** according to one of claims 1 to 3 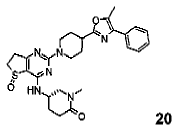 as well as pharmacologically acceptable salts, diastereomers, enantiomers, racemates, hydrates or solvates thereof.

37. Compound of formula **21** according to one of claims 1 to 3 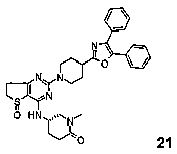 as well as pharmacologically acceptable salts, diastereomers, enantiomers, racemates, hydrates or solvates thereof.

38. Compound of formula **22** according to one of claims 1 to 3 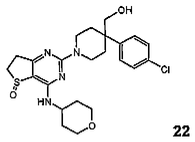 as well as pharmacologically acceptable salts, diastereomers, enantiomers, racemates, hydrates or solvates thereof.

39. Compound of formula **23** according to one of claims 1 to 3 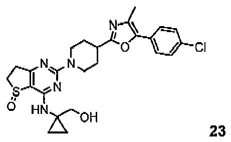 as well as pharmacologically acceptable salts, diastereomers, enantiomers, racemates, hydrates or solvates thereof.

40. Compound of formula **24** according to one of claims 1 to 3 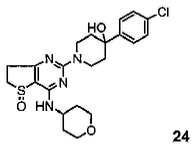 as well as pharmacologically acceptable salts, diastereomers, enantiomers, racemates, hydrates or solvates thereof.

41. Compound of formula **25** according to one of claims 1 to 3 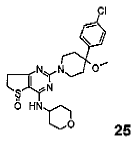 as well as pharmacologically acceptable salts, diastereomers, enantiomers, racemates, hydrates or solvates thereof.

42. Compound of formula **26** according to one of claims 1 to 3 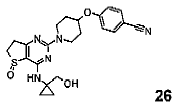 as well as pharmacologically acceptable salts, diastereomers, enantiomers, racemates, hydrates or solvates thereof.

43. Compound of formula **27** according to one of claims 1 to 3 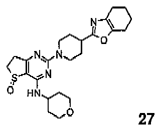 as well as pharmacologically acceptable salts, diastereomers, enantiomers, racemates, hydrates or solvates thereof.

44. Compound of formula **28** according to one of claims 1 to 3 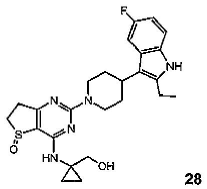 as well as pharmacologically acceptable salts, diastereomers, enantiomers, racemates, hydrates or solvates thereof.

45. Compounds according to one of Claims 1 to 44 as medicaments.

46. Use of compounds according to one of Claims 1 to 44, for preparing a medicament for the treatment of respiratory or gastrointestinal diseases or complaints, as well as inflammatory diseases of the joints, skin or eyes, cancers, and diseases of the peripheral or central nervous system.

47. Use of compounds according to one of Claims 1 to 44 for preparing a medicament for the prevention and treatment of respiratory or pulmonary diseases which are accompanied by increased mucus production, inflammations and/or obstructive diseases of the respiratory tract.

48. Use of compounds according to one of Claims 1 to 44 for preparing a medicament for the treatment of inflammatory and obstructive diseases such as COPD, chronic sinusitis, asthma, Crohn's disease and ulcerative colitis.

49. Use of compounds according to one of Claims 1 to 44 for preparing a medicament for the prevention and treatment of diseases of the peripheral or central nervous system such as depression, bipolar or manic depression, acute and chronic anxiety states, schizophrenia, Alzheimer's disease, Parkinson's disease, acute and chronic multiple sclerosis or acute and chronic pain and brain damage caused by stroke, hypoxia or cranio-cerebral trauma.

50. Pharmaceutical formulations, **characterised in that** they contain one or more compounds of formula 1 according to one of claims 1 to 44 in combination with one or more active substances selected from among anticholinergics, betamimetics, corticosteroids, other PDE4-inhibitors, EGFR-inhibitors and LTD4-antagonists, CCR3-inhibitors, iNOS-inhibitors and SYK-inhibitors.

## Revendications

1. Composés de formule 1 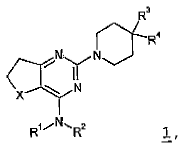 dans laquelle
X est SO ou SO₂,
R¹ est H, alkyle en C₁₋₆,
R² est H ou un reste choisi dans le groupe constitué par alkyle en C₁₋₁₀ et alcényle en C₂₋₆, qui peut être facultativement substitué par un ou plusieurs restes choisis parmi un halogène et un fluoroalkyle en C₁₋₃ ou qui peut être facultativement substitué par un ou plusieurs restes choisis dans le groupe constitué par OR^{2.1}, COOR^{2.1}, CONR^{2.2}R^{2.3}, SR^{2.1}, SO-R^{2.1}, SO₂-R^{2.1}, aryle en C₆₋₁₀, -hét, hétaryle, -cycloalkyle en C₃₋₁₀ mono- ou bicyclique, CH₂-NR^{2.2}R^{2.3} et NR^{2.2}R^{2.3},
lequel peut lui-même être facultativement substitué par un ou plusieurs restes choisis dans le groupe constitué par OH, halogène, OR^{2.1}, oxo, CF₃, CHF₂, CH₂F, alkyle en C₁₋₆, alcanol en C₁₋₆, aryle en C₆₋₁₀, COOR^{2.1}, CH₂-NR^{2.2}R^{2.3} et NR^{2.2}R^{2.3},
où
hét désigne un hétérocycle de trois à onze termes mono- ou bicyclique, saturé ou partiellement saturé, facultativement condensé ou facultativement ponté, qui contient 1, 2, 3 ou 4 hétéroatomes choisis indépendamment les uns des autres dans le groupe constitué par N, S ou O,
et où
hétaryle désigne un hétéroaryle de cinq à dix termes mono- ou bicyclique, facultativement condensé, qui contient 1, 2, 3 ou 4 hétéroatomes choisis indépendamment les uns des autres dans le groupe constitué par N, S ou 0,
et où
le cycloalkyle peut être saturé ou partiellement saturé,
où R^{2.1} est H ou un reste choisi dans le groupe constitué par alkyle en C₁₋₆, alcanol en C₁₋₆, haloalkyle en C₁₋₃, -cycloalkyle en C₃₋₁₀ mono- ou bicyclique, aryle en C₆₋₁₀-alkylène en C₁₋₆, hétarylalkylène en C₁₋₆-, hét-alkylène en C₁₋₆-, cycloalkyle en C₃₋₁₀-alkylène en C₁₋₆-, aryle en C₆₋₁₀ mono- ou bicyclique, hétéroaryle et -hét,
qui peut facultativement être substitué par un ou plusieurs restes choisis dans le groupe constitué par OH, O-(alkyle en C₁₋₃), halogène, alkyle en C₁₋₆ et aryle en C₆₋₁₀,
où R^{2.2} et R^{2.3} sont indépendamment l'un de l'autre H ou un reste choisi dans le groupe constitué par alkyle en C₁₋₆, cycloalkyle en C₃₋₁₀ mono- ou bicyclique, aryle en C₆₋₁₀-alkylène en C₁₋₆, hétarylalkylène en C₁₋₆, aryle en C₆₋₁₀ mono- ou bicyclique, hét, hétaryle, CO-NH₂, CO-NHCH₃, -CO-N(CH₃)₂, SO₂- (alkyle en C₁-C₂), CO-R^{2.1} et COOR^{2.1},
qui peut facultativement être substitué par un ou plusieurs restes choisis dans le groupe constitué par OH, halogène, alkyle en C₁₋₆, aryle en C₆₋₁₀ et COOR^{2.1},
ou
R² est un cycloalkyle en C₃₋₁₀ mono- ou polycyclique qui peut facultativement être ponté par un ou plusieurs groupes alkyle en C₁₋₃ et qui peut être facultativement substitué par un reste choisi dans le groupe constitué par alcanol en C₁₋₆ ramifié ou non ramifié, fluoroalkyle en C₁₋₃, alkylène en C₁₋₃-OR^{2.1}, OR^{2.1}, COOR^{2.1}, SO₂-NR^{2.2}-**R^{2.3}**, hét, -NH-CO-O-(alkyle en C₁₋₆), -NH-CO-(alkyle en C₁₋₆) , -NH-CO-O-(aryle en C₆₋₁₀), -NH-CO-(aryle en C₆₋₁₀), -NH-CO-O-hétaryle, -NH-CO-hétaryle, -NH-CO-O-(alkylène en C₁₋₃)-(aryle en C₆₋₁₀), -NH-CO-(alkylène en C₂₋₃)-(aryle en C₆₋₁₀) , -N(alkyle en C₁₋₃)-CO-(alkyle en C₁₋₆), -N-(alkyle en C₁₋₃)-CO-O-(aryle en C₆₋₁₀), -N(alkyle en C₁₋₃)-CO-(aryle en C₆₋₁₀), -N (alkyle en C₁₋₃)-CO-O-hétaryle, -N(alkyle en C₁₋₃)-CO-hétaryle, -N(alkyle en C₁₋₃)-CO-O-(alkylène en C₁₋₃)-(aryle en C₆₋₁₀), -N(alkyle en C₁₋₃)-CO-(alkylène en C₁₋₃)-(aryle en C₆₋₁₀), aryle en C₆₋₁₀, alkyle en C₁₋₆, aryle en C₆₋₁₀-alkylène en C₁₋₆, hétarylalkylène en C₁₋₆, cycloalkyle en C₃₋₁₀ mono- ou bicyclique et NR^{2.2}R^{2.3},
qui peut facultativement être substitué par un ou plusieurs restes choisis dans le groupe constitué par OH, OR^{2.1}, oxo, halogène, CF₃, CHF₂, CH₂F, alkyle en C₁₋₆, aryle en C₆₋₁₀ et NR^{2.2}R^{2.3},
ou
R² est un aryle en C₆₋₁₀ mono- ou polycyclique, qui peut facultativement être substitué par OH, SH ou un halogène ou par un ou plusieurs restes choisis dans le groupe constitué par OR^{2.1}, COOR2.1, NR^{2.2}R^{2.3}, CH₂-NR^{2.2}R^{2.3}, cycloalkyle en C₃₋₁₀, hét, alkyle en C₁₋₆, fluoroalkyle en C₁₋₃, CF₃, CHF₂, CH₂F, aryle en C₆₋₁₀-alkylène en C₁₋₆, hét-alkylène en C₁₋₆, hétarylalkylène en C₁₋₆, aryle en C₆₋₁₀, SO₂-CH₃, SO₂-CH₂CH₃ et SO₂-NR^{2.2}R^{2.3},
qui peut être lui-même facultativement substitué par un ou plusieurs restes choisis dans le groupe constitué par OH, OR^{2.1}, CF₃, CHF₂, CH₂F, oxo, halogène, CF₃, CHF₂, CH₂F, alkyle en C₁₋₆, aryle en C₆₋₁₀ et NR^{2.2}R^{2.3},
ou
R² est un reste choisi dans un groupe constitué par hét et hétaryle, lequel peut être facultativement substitué par un ou plusieurs restes choisis dans le groupe halogène, OH, oxo, CF₃, CHF₂ et CH₂F ou par un ou plusieurs restes choisis dans le groupe OR^{2.1}, alkylène en C₁₋₃-OR^{2.1}, SR^{2.1}, SO-R^{2.1}, SO₂-R^{2.1}, COOR^{2.1}, COR^{2.1}, alcanol en C₁₋₆, cycloalkyle en C₃₋₁₀ mono- ou bicyclique, aryle en C₆₋₁₀, alkyle en C₁₋₆, aryle en C₆₋₁₀-alkylène en C₁₋₆, hétarylalkylène en C₁₋₆, hét, hétaryle, alkylène en C₁₋₃-OR^{2.1} et NR^{2.2}R^{2.3},
qui peut être lui-même facultativement substitué par un ou plusieurs restes choisis dans le groupe constitué par OH, OR^{2.1}, oxo, halogène, CF₃, CHF₂, CH₂F, alkyle en C₁₋₆, aryle en C₆₋₁₀ et NR^{2.2}R^{2.3},
ou dans laquelle
NR¹R² représente dans son ensemble un noyau hétérocyclique en C₄₋₇, qui peut facultativement être ponté, qui contient 1, 2 ou 3 hétéroatomes choisis dans le groupe constitué par N, O et S et qui peut être facultativement substitué par un ou plusieurs restes choisis dans le groupe constitué par OH, OR^{2.1}, alkylène en C₁₋₃-O^{R.1}, oxo, halogène, alkyle en C₁₋₆, aryle en C₆₋₁₀, COOR^{2.1}, CH₂-NR^{2.2}-COO-R^{2.1}, CH₂-NR^{2.2}-CO-R^{2.1}, CH₂-NR^{2.2}-COCH₂-NR^{2.2}R^{2.3}, CH₂-NR^{2.2}-SO₂-alkyle en C₁₋₃, CH₂-NR^{2.2}-SO₂-NR^{2.2}R^{2.3}, CH₂NR^{2.2}- CO-NR^{2.2}R^{2.3}, CO-NR^{2.2}R^{2.3}, CH₂-NR^{2.2}R^{2.3} et NR^{2.2}R^{2.3},
et dans laquelle
R³ est un aryle en C₆₋₁₀
qui est facultativement substitué en position ortho, para ou méta par un, deux ou trois restes choisis indépendamment les uns des autres dans le groupe constitué par fluoro, chloro, bromo, hydroxy, CN, alkyle en C₁₋₆, fluoroalkyle en C₁₋₃, -alkylène en C₁₋₃-OR^{2.1}, -alkylène en C₁₋₃-NR^{2.2}R^{2.3}, -NR^{2.2}R^{2.3}, O-R^{2.1}, SO-R^{2.1}, SO₂-R^{2.1}, COOR^{2.1}, -CO-NH-(alkylène en C₁₋₆)-hétaryle, -CO-NH-hétaryle, -CO-N(CH₃)-hét, -CO-N(CH₃)-(alkylène en C₁₋₃)-hét, -CO-N(CH₃)-(alkylène en C₁₋₃)-hétaryle, -CO-N(cycloalkyle en C₃₋₇)-hét, -CO-NR^{2.2}R^{2.3}, -CO-NH-(alkylène en C₁₋₆)-hét, NR^{2.2}CO-R^{2.1}, aryle en C₆₋₁₀, aryle en C₆₋₁₀-alkylène en C₁₋₂, hét-alkylène en C₁₋₂, -hét, -CO-hét, CO-N(CH₃)-cycloalkyle en C₃₋₇, cycloalkyle en C₃₋₇, cycloalkyle en C₃₋₇-alkylène en C₁₋₂, hétarylalkylène en C₁₋₂ et hétaryle,
ce reste pouvant être facultativement substitué par un ou plusieurs restes choisis dans le groupe constitué par OH, halogène, -fluoroalkyle en C₁₋₃, oxo, méthyle et phényle,
ou dans laquelle
R³ est un reste choisi dans le groupe constitué par hét et hétaryle, qui peut être facultativement substitué par un ou plusieurs restes choisis dans le groupe constitué par halogène, fluoroalkyle en C₁₋₃, CN, OH, oxo, -alkyle en C₁₋₆, -alkylène en C₁₋₃NR^{2.2}R^{2.3}, -NR^{2.2}R^{2.3}, SO- R^{2.1}, SO₂-R^{2.1}, -O-R^{2.1}, -COOR^{2.1}, SO₂-(CH₃), SO₂-(CH₂-CH₃), aryle en C₆₋₁₀, hét, cycloalkyle en C₃₋₇ et hétaryle,
qui peut lui-même être facultativement substitué par un ou plusieurs restes choisis dans le groupe OH, halogène, -fluoroalkyle en C₁₋₃, alkyle en C₁₋₆, aryle en C₆₋₁₀, -COO(alkyle en C₁₋₃) et O-(alkyle en C₁₋₃),
ou dans laquelle
R³ est -O-R^{3.1},
où R^{3.1} est un reste choisi dans le groupe constitué par -alkyle en C₁₋₆, -aryle en C₆₋₁₀, -alkylène en C₁₋₃-aryle en C₆₋₁₀, hétaryle et hét,
lequel est facultativement substitué en position ortho, para ou méta par un, deux ou trois restes choisis indépendamment les uns des autres dans le groupe constitué par fluoro, chloro, bromo, hydroxy, CN, alkyle en C₁₋₆, fluoroalkyle en C₁₋₃, CO-(alkyle en C₁₋₅), -CO-(fluoroalkyle en C₁₋₃), -CO-NH-(alkylène en C₁₋₆)-hétaryle, -CO-N(alkyle en C₁₋₃)-(alkylène en C₁₋₆)-hétaryle, -CO-N(alkyle en C₁₋₃)-hét, -CO-N(cycloalkyle en C₃₋₇)-hét, -alkylène en C₁₋₃-OR^{2.1}, -alkylène en C₁₋₃-NR^{2.2}R^{2.3}, -NR^{2.2}R^{2.3}, O-R^{2.1}, SO-R^{2.1}, SO₂-R^{2.1}, COOH, COO-(alkyle en C₁₋₄), -O-alkylène en C₁₋₃- N(alkyle en C₁₋₃)₂, CO-NR^{2.2}R^{2.3}, NR^{2.2}-CO-R^{2.1}, aryle en C₆₋₁₀, aryle en C₆₋₁₀-alkylène en C₁₋₂, hét-alkylène en C₁₋₂, -CO-hét, hét, -CO-cycloalkyle en C₃₋₇, -CO-N(alkyle en C₁₋₃)-cycloalkyle en C₃₋₇, cycloalkyle en C₃₋₇, cycloalkyle en C₃₋₇-alkylène en C₁₋₂, hétarylalkylène en C₁₋₂ et hétaryle, lequel peut lui-même être facultativement substitué par 1, 2, 3 ou 4 restes choisis indépendamment les uns des autres dans le groupe constitué par F, Cl, Br, méthyle, O-méthyle, éthyle, O-éthyle, OH, oxo et CF₃ ,
et dans laquelle
R⁴ est H, CN, OH, CF₃, CHF₂, CH₂F, F, méthyle, éthyle, -O-(alkyle en C₁₋₃), -alkylène en C₁₋₃-OH, -COO-(alkyle en C₁₋₃), -CO-hét, -(alkylène en C₁₋₂)-NH-SO₂-(alkyle en C₁₋₂), -(alkylène en C₁₋₂)-N(alkyle en C₁₋₃)-SO₂-(alkyle en C₁₋₂), -(alkylène en C₁₋₂)-O-(alkylène en C₁₋₂)-aryle en C₆₋₁₀, -alkylène en C₁₋₃-O-alkyle en C₁₋₃, -(alkylène en C₁₋₂)-N(alkyle en C₁₋₃)-CO-(alkyle en C₁₋₂), -NH-CO-(alkylène en C₁₋₃)-O-(alkyle en C₁₋₃), -alkylène en C₁₋₃-NH-CO-(alkyle en C₁₋₃), -alkylène en C₁₋₃-NH-CO-(alkylène en C₁₋₃)-N(alkyle en C₁₋₃)₂, -O-(alkylène en C₁₋₂)-(aryle en C₆₋₁₀), -alkylène en C₁₋₃-NH-CO-(alkylène en C₁₋₃)-O-(alkyle en C₁₋₃), -CO-(aryle en C₆₋₁₀), -(alkylène en C₁₋₂)-N(alkyle en C₁₋₃)-Co-(alkylène en C₁₋₂)-O-(alkyle en C₁₋₃),
où l'aryle dans les restes ci-dessus peut être lui-même facultativement substitué par un ou plusieurs autres restes choisis dans le groupe F, Cl, Br, méthyle, éthyle, propyle, isopropyle, cyclopropyle, -O-éthyle, -O-éthyle, -O-propyle, -O-isopropyle, -O-cyclopropyle, -OH et CF₃,
ou dans laquelle
R³ et R⁴ forment ensemble un hétérocycle mono- ou bicyclique insaturé, saturé ou partiellement saturé, qui contient 1, 2 ou 3 hétéroatomes choisis dans le groupe constitué par N, 0 et S et qui peut être facultativement substitué par un ou plusieurs restes choisis dans le groupe constitué par halogène, OH, oxo, fluoroalkyle en C₁₋₃, CN, alkyle en C₁₋₆, -O-R^{2.1}, -COOR^{2.1}, SO-R^{2.1}, SO₂-R^{2.1} -alkylène en C₁₋₃-NR^{2.2}R^{2.3}, -NR^{2.2}R^{2.3}, aryle en C₆₋₁₀, cycloalkyle en C₃₋₇, hét et hétaryle,
ainsi que des sels, diastéréoisomères, énantiomères, racémates, hydrates ou solvates pharmacologiquement compatibles de ces composés.

2. Composés de formule 1 selon la revendication 1, dans laquelle
X est SO ou SO₂,
R¹ est H
R² est H ou alkyle en C₁₋₁₀, qui peut être facultativement substitué par un ou plusieurs restes choisis parmi halogène et fluoroalkyle en C₁₋₃ ou qui peut être facultativement substitué par un ou plusieurs restes choisis dans le groupe constitué par OR^{2.1}, COOR^{2.1}, CONR^{2.2}R^{2.3}, SR^{2.1}, SO-R^{2.1}, SO₂-R^{2.1}, phényle, hét, hétaryle, cycloalkyle en C₃₋₇ monocyclique, CH₂-NR^{2.2}R^{2.3} et NR^{2.2}R^{2.3}, lequel peut lui-même être facultativement substitué par un ou plusieurs restes choisis dans ·le groupe constitué par OH, F, Cl, Br, OR^{2.1}, oxo, CF₃, CHF₂, CH₂F, alkyle en C₁₋₆, alcanol en C₁₋₆, phényle, COOR^{2.1}, CH₂NR^{2.2}R^{2.3} et NR^{2.2}R^{2.3},
où
hét désigne un hétérocycle monocyclique de trois à sept termes, saturé ou partiellement saturé, ou un hétérocycle bicyclique de sept à onze termes, saturé ou partiellement saturé, qui contient 1, 2, 3 ou 4 hétéroatomes choisis indépendamment les uns des autres dans le groupe constitué par N, S ou 0,
et où
hétaryle désigne un hétéroaryle aromatique monocyclique de cinq à six termes ou un hétéroaryle aromatique bicyclique de sept à onze termes, qui contient respectivement 1, 2, 3 ou 4 hétéroatomes choisis indépendamment les uns des autres dans le groupe constitué par N, S ou 0,
et où
le cycloalkyle peut être saturé ou partiellement saturé,
où R^{2.1} est H ou un reste choisi dans le groupe constitué par alkyle en C₁₋₆, alcanol en C₁₋₆, haloalkyle en C₁₋₃, cycloalkyle en C₃₋₇ monocyclique, phénylalkylène en C₁₋₆, hétarylalkylène en C₁₋₆, hét-alkylène en C₁₋₆, -cycloalkyle en C₃₋₇-alkylène en C₁₋₆, phényle, hétaryle et hét,
qui peut facultativement être substitué par un ou plusieurs restes choisis dans le groupe constitué par OH, F, Cl, alkyle en C₁₋₆, -O-(alkyle en C₁₋₃) et phényle,
où R^{2.2} et R^{2.3} sont indépendamment l'un de l'autre H ou un reste choisi dans le groupe constitué par alkyle en C₁₋₆, cycloalkyle en C₃₋₇ monocyclique, phénylalkylène en C₁₋₃, hétarylalkylène en C₁₋₃, phényle, hét, hétaryle, CO-NH₂, -CO-NHCH₃, -CON(CH₃)₂, SO₂-(alkyle en C₁₋₂) , CO-R^{2.1} et COOR^{2.1}, qui peut être facultativement substitué par un ou plusieurs restes choisis dans le groupe constitué par OH, F, Cl, alkyle en C₁₋₆, phényle et COOR^{2.1},
ou
R² est un cycloalkyle en C₃₋₇ monocyclique qui peut être facultativement substitué par un reste choisi dans le groupe constitué par alcanol en C₁₋₆ ramifié ou non ramifié, fluoroalkyle en C₁₋₃, OR^{2.1}, alkylène en C₁₋₃-OR^{2.1}, OR^{2.1}, COOR^{2.1}, SO₂-NR^{2.2}R^{2.3}, -hét, -NH-CO-O-(phényle), phényle, alkyle en C₁₋₆, phénylalkylène en C₁₋₃, -hétarylalkylène en C₁₋₆, cycloalkyle en C₃₋₇ monocyclique et NR^{2.2}R^{2.3},
qui peut être facultativement substitué par un ou plusieurs restes choisis dans le groupe constitué par OH, OR^{2.1} oxo, F, Cl, CF₃, CHF₂, CH₂F, alkyle en C₁₋₆, phényle et -NR^{2.2}R^{2.3},
ou
R² est un phényle, qui peut être facultativement substitué par OH, SH ou halogène ou par un ou plusieurs restes choisis dans le groupe constitué par OR^{2.1}, COOR^{2.1}, NR^{2.2}R^{2.3}, CH₂-NR^{2.2}R^{2.3}, cycloalkyle en C₃₋₇, hétérocycle en C₃₋₇, alkyle en C₁₋₆, fluoroalkyle en C₁₋₃, phénylalkylène en C₁₋₆, -hét-alkylène en C₁₋₆,-hétarylalkylène en C₁₋₆, phényle, SO₂-CH₃, SO₂-CH₂CH₃ et SO₂-NR^{2.2}R^{2.3},
qui peut lui-même être facultativement substitué par un ou plusieurs restes choisis dans le groupe constitué par OH, OR^{2.1}, oxo, F, Cl, CF₃, CHF₂, CH₂F, alkyle en C₁₋₆, phényle et NR^{2.2}R^{2.3},
ou
R² est un reste choisi dans un groupe constitué par hét et hétaryle, lequel peut être facultativement substitué par un ou plusieurs restes choisis dans le groupe F, Cl, OH, oxo, CF₃, CHF₂ et CH₂F ou par un ou plusieurs restes choisis dans le groupe OR^{2.1}, -alkylène en C₁₋₃-OR^{2.1}, SR^{2.1}, SO-R^{2.1}, SO₂-R^{2.1}, COOR^{2.1}, COR^{2.1}, alcanol en C₁₋₆, cycloalkyle en C₃₋₇ monocyclique, phényle, allyle en C₁₋₆, phénylalkylène en C₁₋₆, -hétarylalkylène en C₁₋₆, -hét, -hétaryle et NR^{2.2}R^{2.3},
qui peut lui-même être facultativement substitué par un ou plusieurs restes choisis dans le groupe constitué par OH, OR^{2.1}, oxo, F, Cl, CF₃, CHF₂, CH₂F, alkyle en C₁₋₆, phényle et NR^{2.2}R^{2.3},
ou dans laquelle
NR¹R² représente dans son ensemble un noyau hétéro- cyclique en C₄₋₇ qui peut facultativement être ponté, qui contient 1, 2 ou 3 hétéroatomes choisis dans le groupe constitué par N, 0 et S et qui peut être facultativement substitué par un ou plusieurs restes choisis dans le groupe constitué par OH, OR^{2.1}, alkylène en C₁₋₃-O^{R.1}, oxo, F, Cl, alkyle en C_{1-6,} phényle, COOR^{2.1}, CH₂-NR^{2.2}-COO-R^{2.1}, CH₂-NR^{2.2}-CO-R^{2.1}, CH₂-NR^{2.2}-CO-CH₂-NR^{2.2}R^{2.3}, CH₂-NR^{2.2}-SO₂-alkyle en C₁₋₃, CH₂-NR^{2.2}-SO₂-NR^{2.2}R^{2.3}, CH₂-NR^{2.2}-CO-NR^{2.2}R^{2.3}, CO-NR^{2.2}R^{2.3}, CH₂-NR^{2.2}R^{2.3} et NR^{2.2}R^{2.3},
et dans laquelle
R³ est un naphtalène ou un phényle qui est facultativement substitué en position ortho, para ou méta par un ou deux restes choisis indépendamment l'un de l'autre dans le groupe constitué par fluoro, chloro, bromo, hydroxy, CN, alkyle en C₁₋₆, fluoroalkyle en C₁₋₃, -alkylène en C₁₋₃-OR^{2.1}, -alkylène en C₁₋₃-NR^{2.2}R^{2.3}, -NR^{2.2}-R^{2.3}, O-R^{2.1}, SO- R^{2.1}, SO₂-R^{2.1}, COOR^{2.1}, -CO-NH-(alkylène en C₁₋₆)-hétaryle, -CO-NH-hétaryle, -CO-N(CH₃)-hét, -CON(CH₃)-(alkylène en C₁₋₃)-hét, -CO-N(CH₃)-(alkylène en C₁₋₃)-hétaryle, -CO-N(cycloalkyle en C₃₋₇)-hét, CO-NR^{2.2}R^{2.3}, -CO-NH-(alkylène en C₁₋₆)-hét, -NR^{2.2}-CO-R^{2.1}, phényle, phénylalkylène en C₁₋₂, -hét-alkylène en C₁₋₂, -hét, -CO-hét, -CO-N(CH₃)-hét, CO-N(CH₃)-cycloalkyle en C₃₋₇, cycloalkyle en C₃₋₇, cycloalkyle en C₃₋₇-alkylène en C₁₋₂, -hétarylalkylène en C₁₋₂ et -hétaryle,
ce reste pouvant facultativement être substitué par un ou plusieurs restes choisis dans le groupe constitué par OH, F, Cl, -fluoroalkyle en C₁₋₃, oxo, méthyle et phényle,
ou dans laquelle
R³ est un reste choisi dans le groupe constitué par hét et hétaryle, qui peut être facultativement substitué par un ou plusieurs restes choisis dans le groupe constitué par F, Cl, Br, fluoroalkyle en C₁₋₃, CN, OH, oxo, -alkyle en C₁₋₆, -alkylène en C₁₋₃-NR^{2.2}R^{2.3}, -NR^{2.2}R^{2.3}, SO- R^{2.1}, SO₂-R^{2.1}, -O-R^{2.1}, -COOR^{2.1}, SO₂-(CH₃), SO₂-(CH₂-CH₃), phényle, hét, cycloalkyle en C₃₋₇ et hétaryle,
qui peut lui-même être facultativement substitué par un ou plusieurs restes choisis dans le groupe OH, F, Cl, Br, -fluoroalkyle en C₁₋₃, alkyle en C₁₋₆, phényle, -COO-(alkyle en C₁₋₃) et O-(alkyle en C₁₋₃),
ou dans laquelle
R³ est -O-R^{3.1},
où R^{3.1} est un reste choisi dans le groupe constitué par -alkyle en C₁₋₆, -phényle, -alkylène en C₁₋₃-phényle, hétaryle et hét,
lequel est facultativement substitué en position ortho, para ou méta par un, deux ou trois restes choisis indépendamment les uns des autres dans le groupe constitué par fluoro, chloro, bromo, hydroxy, CN, alkyle en C₁₋₆, fluoroalkyle en C₁₋₃, CO- (alkyle en C₁₋₆), -CO-(fluoroalkyle en C₁₋₃), -CO-NH-(alkylène en C₁₋₆)-hétaryle, -CO-N(CH₃)-(alkylène en C₁₋₆) -hétaryle, -CO-N(CH₃)-hét, -CO-N(cycloalkyle en C₃₋₇)-hét, -alkylène en C₁₋₃-OR^{2.1}, -alkylène en C₁₋₃-NR^{2.2}R^{2.3}, -NR^{2.2}R^{2.3}, O-R^{2.1}, SO-R^{2.1}, SO₂-R^{2.1}, COOH, COO-(alkyle en C₁₋₄), -O-alkylène en C₁₋₃-N(alkyle en C₁₋₃)₂, CO-NR^{2.2}R^{2.3}, NR^{2.2}-CO-R^{2.1}, phényle, phénylalkylène en C₁₋₂, hét-alkylène en C₁₋₂, -CO-hét, hét, -CO-cycloalkyle en C₃₋₇, -CO-N(CH₃)-cycloalkyle en C₃₋₇, cycloalkyle en C₃₋₇, cycloalkyle en C₃₋₇-alkylène en C₁₋₂, hétarylalkylène en C₁₋₂ et hétaryle,
lequel peut lui-même être facultativement substitué par 1, 2, 3 ou 4 restes choisis indépendamment les uns des autres dans le groupe constitué par F, Cl, Br, méthyle, O-méthyle, éthyle, O-éthyle, OH, oxo et CF₃,
et dans laquelle
R⁴ est H, CN, OH, CF₃, CHF₂, CH₂F, F, méthyle, éthyle, O-méthyle, O-éthyle, O-propyle, O-isopropyle, -alkylène en C₁₋₃-OH, -COO(alkyle en C₁₋₃), -CO-hét, -(alkylène en C₁₋₂)-NH-SO₂-(alkyle en C₁₋₂), -(alkylène en C₁₋₂)-N(CH₃)-SO₂-(alkyle en C₁₋₂), -(alkylène en C₁₋₂)-O-(alkylène en C₁₋₂)-phényle, -alkylène en C₁₋₃-O-alkyle en C₁₋₃, -(alkylène en C₁₋₂)-N(CH₃)-CO-(alkyle en C₁₋₂), -NH-CO-(alkylène en C₁₋₃)-O-(alkyle en C₁₋₃), -alkylène en C₁₋₃-NH-CO-(alkyle en C₁₋₃), -alkylène en C₁₋₃-NH-CO-(alkylène en C₁₋₃)-N(alkyle en C₁₋₃)₂, -O-(alkylène en C₁₋₂)-phényle, -alkylène en C₁₋₃-NH-CO-(alkylène en C₁₋₃)-O-(alkyle en C₂₋₃), -CO-phényle, -(alkylène en C₁₋₂)-N(CH₃)-CO-(alkylène en C₁₋₂)-O-(alkyle en C₁₋₃),
où le phényle dans les restes ci-dessus peut être facultativement substitué par un ou plusieurs autres restes choisis dans le groupe F, Cl, Br, méthyle, éthyle, propyle, -O-méthyle, -O-éthyle, -O-propyle, -OH et CF₃,
ou dans laquelle
R³ et R⁴ forment ensemble un hétérocycle mono- ou bicyclique insaturé, saturé ou partiellement saturé, qui contient 1, 2 ou 3 hétéroatomes choisis dans le groupe constitué par N, O et S et qui peut être facultativement substitué par un ou plusieurs restes choisis dans le groupe constitué par F, Cl, Br, OH, oxo, fluoroalkyle en C₁₋₃, CN, alkyle en C₁₋₆, -O-R^{2.1}, -COOR^{2.1} SO-R^{2.1}, SO₂-R^{2.1}, -alkylène en C₁₋₃-NR^{2.2}R^{2.3}, -NR^{2.2}R^{2.3}, phényle, cycloalkyle en C₃₋₇, hét et hétaryle,
ainsi que des sels, diastéréoisomères, énantiomères, racémates, hydrates ou solvates pharmacalogiquement compatibles de ces composés.

3. Composés de formule 1 selon la revendication 1 ou la revendication 2, dans laquelle
X est SO,
R¹ est H
R² est H ou alkyle en C₁₋₆, qui peut être facultativement substitué par un ou plusieurs restes choisis parmi F, Cl, CF₃, CHF₂ ou CH₂F ou qui peut être facultativement substitué par un ou plusieurs restes choisis dans le groupe constitué par OR^{2.1}, COOR^{2.1}, CONR^{2.2}R^{2.3}, SR^{2.1}, SO-R^{2.1}, SO2-R^{2.1}, phényle, hét, hétaryle, cycloalkyle en C₃₋₇ monocyclique, CH₂-NR^{2.2}R^{2.3} et NR^{2.2}R^{2.3},
lequel peut lui-même être facultativement substitué par un ou plusieurs restes choisis dans le groupe constitué par OH, F, Cl, Br, CF₃, CHF₂, CH₂F, OR^{2.1}, oxo, méthyle, éthyle, propyle, isopropyle, méthanol, éthanol, phényle, COOR^{2.1}, CH₂-NR^{2.2}R^{2.3} et NR^{2.2}R^{2.3},
où
hét désigne un hétérocycle monocyclique de trois à sept termes, saturé ou partiellement saturé, qui contient 1, 2 ou 3 hétéroatomes choisis indépendamment les uns des autres dans le groupe constitué par N, S ou O,
et où
hétaryle désigne un hétéroaryle aromatique monocyclique de cinq à six termes qui contient 1, 2 ou 3 hétéroatomes choisis indépendamment les uns des autres dans le groupe constitué par N, S ou O,
et où
le cycloalkyle peut être saturé ou partiellement saturé,
où R^{2.1} est H ou un reste choisi dans le groupe constitué par méthyle, éthyle, propyle, isopropyle, méthanol, éthanol, cycloalkyle en C₃₋₇ monocyclique, phénylalkylène en C₁₋₂, -hétarylalkylène en C₁₋₂, -hét-alkylène en C₂₋₂, cycloalkyle en C₃₋₇-alkylène en C₁₋₂, phényle, hétaryle et un hét,
qui peut être facultativement substitué par un ou plusieurs restes choisis dans le groupe constitué par OH, F, Cl, méthyle, éthyle, propyle, isopropyle, O-méthyle, O-éthyle, O-propyle, O-isopropyle et phényle,
où R^{2.2} et R^{2.3} sont indépendamment l'un de l'autre H ou un reste choisi dans le groupe constitué par méthyle, éthyle, propyle, isopropyle, cycloalkyle en C₃₋₇ monocyclique, phénylalkylène en C₁₋₃, hétarylalkylène en C₁₋₃, phényle, -hét, -hétaryle, CO-NH₂, CO-NHCH₃, CON(CH₃)₂, SO₂-(alkyle en C₁₋₂), CO-R^{2.1} et COOR^{2.1},
qui peut être facultativement substitué par un ou plusieurs restes choisis dans le groupe constitué par OH, F, Cl, méthyle, éthyle, propyle, isopropyle, phényle et COOR^{2.1},
ou
R² est un cycloalkyle en C₃₋₇ monocyclique qui peut être facultativement substitué par un reste choisi dans le groupe constitué par alcanol en C₁₋₂, fluoroalkyle en C₁₋₃, alkylène en C₁₋₃-OR^{2.1}, OR^{2.1}, COOR^{2.1} SO₂-NR^{2.2}R^{2.3}, -hét, -NH-CO-O-(phényle), méthyle, éthyle, propyle, isopropyle, phényle, phénylalkylène en C₁₋₂, -hétarylalkylène en C₁₋₂, cycloalkyle en C₃₋₇ monocyclique et NR^{2.2}R^{2.3},
qui peut être facultativement substitué par un ou plusieurs restes choisis dans le groupe constitué par OH, OR^{2.1}, oxo, F, Cl, CF₃, CHF₂, CH₂F, méthyle, éthyle, propyle, isopropyle, phényle et NR^{2.2}R^{2.3},
ou
R² est un phényle, qui peut être facultativement substitué par OH, SH, F, Cl ou Br ou par un ou plusieurs restes choisis dans le groupe constitué par OR^{2.1}, COOR^{2.1}, NR^{2.2}R^{2.3}, CH₂-NR^{2.2}R^{2.3}, cycloalkyle en C₃₋₇ monocyclique, -hét, méthyle, éthyle, propyle, isopropyle, CF₃, CHF₂, CH₂F, phénylalkylène en C₁₋₂, hét-alkylène en C₁₋₂, hétarylalkylène en C₁₋₂, phényle, SO₂-CH₃, SO₂-CH₂CH₃ et SO₂-NR^{2.2}R^{2.3},
qui peut lui-même être facultativement substitué par un ou plusieurs restes choisis dans le groupe constitué par OH, OR^{2.1}, oxo, F, Cl, CF₃, CHF₂, CH₂F, méthyle, éthyle, propyle, isopropyle, phényle et NR^{2.2}R^{2.3},
ou
R² est un reste choisi dans un groupe constitué par hét et hétaryle, qui peut être facultativement substitué par un ou plusieurs restes choisis dans le groupe F, Cl, OH, OxO, CF₃, CHF₂ et CH₂F ou par un ou plusieurs restes choisis dans le groupe OR^{2.1}, alkylène en C₁₋₃-OR^{2.1}, SR^{2.1} SO-R^{2.1}, SO₂-R^{2.1}, COOR^{2.1}, COR^{2.1}, méthanol, éthanol, cycloalkyle en C₃₋₇ monocyclique, phényle, méthyle, éthyle, propyle, isopropyle, phénylalkylène en C₁₋₂, hétarylalkylène en C₁₋₂, -hét, -hétaryle et NR^{2.2}R^{2.3},
qui peut lui-même être facultativement substitué par un ou plusieurs restes choisis dans le groupe constitué par OH, OR^{2.1}, oxo, F, Cl, CF₃, CHF₂, CH₂F, méthyle, éthyle, propyle, isopropyle, butyle, isobutyle, phényle et NR^{2.2}R^{2.3},
et dans laquelle
R³ est un naphtalène ou un phényle
qui peut être facultativement substitué en position ortho, para ou méta par un ou deux restes choisis indépendamment l'un de l'autre dans le groupe constitué par fluoro, chloro, bromo, hydroxy, CN, méthyle, éthyle, propyle, isopropyle, cyclopropyle, CF₃, CHF₂, CH₂F, -OCH₃, OCH₂CH₃, SO₂-CH₃, SO-CH₃, COOCH₃, COOCH₂CH₃, -CO-NH-(méthylène)-hétaryle, -CO-NH-(éthylène)-hétaryle, -CO-NH-hétaryle, -CO-N(CH₃)-hét, -CO-N(CH₃)-(méthylène)-hét, -CO-N(CH₃)-(éthylène)-hét, -CO-N(CH₃)-(méthylène)-hétaryle, -CO-N(CH₃)-(éthylène)-hétaryle, -CO-N(cyclopropyle)-hét, CO-NH₂, CONH(CH₃), CON(CH₃)₂, CO-NH-(méthylène)-hét, -CO-NH-(éthylène)-hét, -NH-CO-méthyle, NCH₃-CO-méthyle, -NH-CO-éthyle, NCH₃-CO-éthyle, -NH-CO-propyle, NCH₃-CO-propyle, -NH-CO-isopropyle, NCH₃-CO-isopropyle, phényle, phénylméthylène, phényléthylène, hét-méthylène, hét-éthylène, -hét, -CO-hét, -CO-N(CH₃)- hét, CO-N(CH₃)-cyclopropyle, cycloalkyle en C₃₋₇, cycloalkyle en C₃₋₇-méthylène, cycloalkyle en C₃₋₇-éthylène, hétarylméthylène, hétaryléthylène, -hétaryle, CH₂-NH₂, CH₂-NH(CH₃), CH₂-N(CH₃)₂, -NH₂, -NH(CH₃) et -N(CH₃)₂,
ce reste pouvant être facultativement substitué par un ou plusieurs restes choisis dans le groupe constitué par OH, F, Cl, CF₃, CHF₂, CH₂F, oxo, méthyle et phényle,
ou dans laquelle
R³ est un reste choisi dans le groupe constitué par hét et hétaryle, qui peut être facultativement substitué par un ou plusieurs restes choisis dans le groupe constitué par F, Cl, Br, CF₃, CHF₂, CH₂F, CN, OH, oxo, méthyle, éthyle, propyle, isopropyle, butyle, isobutyle, cyclopropyle, -O-méthyle, -O-éthyle, -O-propyle, -O-isopropyle, -COO-méthyle, -COO-éthyle, -COO-propyle, -COO- isopropyle, SO-(CH₃), SO-(CH₂-CH₃), SO₂-(CH₃), SO₂-(CH₂- CH₃), phényle, CH₂-NH₂, CH₂-NH(CH₃), CH₂-N(CH₃)₂, -NH₂, -NH(CH₃), -N(CH₃)₂, hét et hétaryle,
qui peut lui-même être facultativement substitué par un ou plusieurs restes choisis dans lie groupe OH, F, Cl, CF₃, CHF₂, CH₂F, méthyle, éthyle, propyle, isopropyle, phényle, -COO-méthyle, -COO-éthyle, O-méthyle et O-éthyle,
ou dans laquelle
R³ est -O-R^{3.1},
où R^{3.1} est un reste choisi dans le groupe constitué par -alkyle en C₁₋₃, -phényle, - alkylène en C₁₋₃-phényle, hétaryle et hét,
lequel est facultativement substitué en position ortho, para ou méta par un, deux ou trois restes choisis indépendamment les uns des autres dans le groupe constitué par fluoro, chloro, bromo, hydroxy, CN, méthyle, éthyle, propyle, isopropyle, butyle, isobutyle, CF₃, CHF₂, CH₂F, CO-(méthyle), CO-(éthyle), CO-(propyle), CO-(isopropyle), -CO-(CF₃), -CO-NH-(méthylène)-hétaryle, -CO-NH-(éthylène)-hétaryle, -CO-N(CH₃)-(méthylène)-hétaryle, -CO-N(CH₃)-(éthylène)-hétaryle, -CO-N(CH₃)-(propylène)- hétaryle, -CO-N(CH₃)-(isopropylène)-hétaryle, -CO-N(CH₃)-hét, -CO-N(cyclopropyle)-hét, -CO-N(cycloalkyle en C₅₋₇)-hét, -méthylène-O-méthyle, -éthylène-O-méthyle, -propylène-O-méthyle, -méthylène-O-éthyle, -éthylène-O-éthyle, -propylène-O-éthyle, -méthylène-NH₂, -méthylène-NHCH₃, -méthylène-N-(CH₃)₂, -éthylène-NH₂, -éthylène-NHCH₃, -éthylène-N(CH₃)₂, NH₂, N(CH₃)₂, NHCH₃, -O-méthyle, O-éthyle, O-propyle, O-isopropyle, O-butyle, O-isobutyle, -SO-CH₃, SO-éthyle, -SO-propyle, -SO-isopropyle, SO₂-méthyle, -SO₂-éthyle, SO₂-propyle, SO₂-isopropyle, COOH, COO-(méthyle), COO-(éthyle), COO-(propyle), COO-(isopropyle), -O-méthylène-N(méthyle)₂, -O-éthylène-N-(méthyle)₂, -O-méthylène-N-(éthyle)₂, -O-éthylène-N-(éthyle)₂, CO-NH₂, CO-NH(CH₃), CO-N(CH₃)₂, -NH-CO-méthyle, -NCH₃-CO-méthyle, -NH-CO-éthyle, NCH₃-CO-éthyle, phényle, phénylméthylène-, phényléthylène-, hét-méthylène-, hét-éthylène-, -CO-hét, hét, -CO-cycloalkyle en C₅₋₇, -CO-cyclopropyle, -CO-N(CH₃)-cycloalkyle en C₅₋₇, -CO-N(CH₃)-cyclopropyle, cycloalkyle en C₅₋₇, cyclopropyle, cycloalkyle en C₅₋₇-méthylène, cycloalkyle en C₅₋₇-éthylène, cyclopropylméthylène, cyclopropyléthylène, hétarylméthylène, hétaryléthylène et hétaryle,
lequel peut lui-même être facultativement substitué par 1, 2, 3 ou 4 restes choisis indépendamment les uns des autres dans le groupe constitué par F, Cl, Br, méthyle, O-méthyle, éthyle, O-éthyle, OH, oxo et CF₃,
et dans laquelle
R⁴ est H, CN, OH, CF₃, CHF₂, CH₂F, F, méthyle, éthyle, O-méthyle ou O-éthyle, -méthylène-OH, -éthylène-OH, -propylène-OH, isopropylène-OH, -COO-(méthyle), -COO(éthyle), -COO(propyle), -COO(isopropyle), -CO-hét, -(méthylène)-NH-SO₂-(méthyle), -(méthylène)-NH-SO₂-(éthyle), - (éthylène)-NH-SO₂-(méthyle), -(éthylène)-NH-SO₂-(éthyle), -(méthylène)-N(CH₃)-SO₂-(méthyle), -(méthylène)-N(CH₃)-SO₂-(éthyle), -(éthylène)-N(CH₃)-SO₂-(méthyle), - (éthylène)-N(CH₃)-SO₂-(éthyle), -(méthylène)-O-(méthylène)-phényle, -(méthylène)-O-(éthylène)-phényle, -(éthylène)-O-(méthylène)-phényle, -(éthylène)-O-(éthylène)-phényle, -méthylène-O-méthyle, -méthylène-O-éthyle, -éthylène-O-méthyle, -éthylène-O-éthyle, -(méthylène)-N(CH₃)-CO-(méthyle), -(méthylène)-N(CH₃)-CO-(éthyle), -(éthylène)-N(CH₃)-CO-(méthyle), -(éthylène)-N(CH₃)-CO-(éthyle), -NH-CO-(méthylène)-O-(méthyle), -NH-CO-(méthylène)-O-(éthyle), -NH-CO-(éthylène)-O-(méthyle), -NH-CO-(éthylène)-O-(éthyle), -méthylène-NH-CO-(méthyle), -méthylène-NH-CO-(éthyle), -éthylène-NH-CO-(méthyle), -éthylène-NH-CO-(éthyle), -méthylène-NH-CO-(méthylène)-N(méthyle)₂, -méthylène-NH-CO-(éthylène)-N(méthyle)₂, -éthylène-NH-CO- (méthylène) -N (méthyle) ₂, -éthylène-NH-CO-(éthylène)-N(méthyle)₂, -méthylène-NH-CO-(méthylène)-O-(méthyle), -méthylène-NH-CO-(éthylène)-O-(méthyle), -éthylène-NH-CO-(méthylène)-O-(méthyle), -méthylène-NH-CO-(méthylène)-O-(éthyle), -méthylène-NH-CO-(éthylène)-O-(éthyle), -éthylène-NH-CO-(méthylène)-O-(éthyle), -(méthylène)-N(CH₃)-CO-(méthylène)-O-(méthyle), -(méthylène)-N(CH₃)-CO-(éthylène)-O-(méthyle), -(éthylène) -N(CH₃)-CO-(méthylène)-O-(méthyle), -(méthylène)-N(CH₃)-CO-(méthyléne)-O-(éthyle), (méthylène)-N(CH₃)-CO-(éthylène)-O-(éthyle), -(éthylène)-N(CH₃)-CO-(méthylène)-O-(éthyle), -O-(méthylène)-phényle, -O-(éthylène)-phényle, -CO-phényle,
le phényle dans les restes ci-dessus pouvant être facultativement substitué par un ou plusieurs autres restes choisis dans le groupe F, Cl, Br, méthyle, éthyle, propyle, -O-méthyle, -O-éthyle, -O-propyle, -OH et CF₃
ou dans laquelle
R³ et R⁴ forment ensemble un hétérocycle mono- ou bicyclique, insaturé, saturé ou partiellement saturé, qui contient 1, 2 ou 3 hétéroatomes choisis dans le groupe constitué par N, O et S et qui peut être facultativement substitué par un ou plusieurs restes choisis dans le groupe constitué par F, Cl, Br, OH, oxo, CF₃, CHF₂, CH₂F, CN, méthyle, éthyle, propyle, isopropyle, cyclopropyle, COO-méthyle, -COO-éthyle, O-méthyle, O-éthyle, SO₂-(CH₃), SO₂-(CH₂CH₃), SO-(CH₃), SO-(CH₂CH₃), CH₂-NH₂, CH₂-NH (CH₃), CH₂-N(CH₃)₂, -NH₂, -NH(CH₃), -N(CH₃)₂, phényle, cycloalkyle en C₅₋₇, hét et hétaryle,
ainsi que des sels, diastéréoisomères, énantiomères, racémates, hydrates ou solvates pharmacologiquement compatibles de ces composés.

4. Composés de formule 1 selon l'une des revendications 1 à 3, dans laquelle
R² est un reste de formule 2 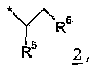
dans laquelle R⁶ est OH ou NH₂ et
dans laquelle R⁵ est un reste choisi dans le groupe constitué par alkyle en C₁₋₄, un hétéroaryle de cinq à six termes contenant 1, 2 ou 3 hétéroatomes du groupe S, O et N, et phényle, lequel peut être facultativement substitué par un ou plusieurs restes choisis dans le groupe constitué par OH, F, Br, OR^{2.1}, oxo, méthyle, éthyle, méthanol, éthanol, phényle, COOR^{2.1}, CH₂-NR^{2.2}R^{2.3} et NR^{2.2}R^{2.3},
ainsi que des sels, diastéréoisomères, énantiomères, racémates, hydrates ou solvates pharmacologiquement compatibles de ces composés.

5. Composés de formule 1 selon l'une des revendications 1 à 3, dans laquelle
R² est un noyau cycloalkyle monocyclique de trois, quatre, cinq, six ou sept termes qui peut être facultativement substitué en position spiro par un reste choisi dans le groupe constitué par -CH₂-OR^{2.1}, alkylène en C₂₋₆-OR^{2.1} ramifié ou non ramifié, méthyle, éthyle, propyle, isopropyle, butyle, isobutyle, cyclopropyle, -CF₃, CHF₂, CH₂F et fluoroalkyle en C₂₋₄, où
R^{2.1} est choisi dans le groupe constitué par méthyle, éthyle, propyle, isopropyle, butyle, isobutyle,
ainsi que des sels, diastéréoisomères, énantiomères, racémates, hydrates ou solvates pharmacologiguement compatibles de ces composés.

6. Composés de formule 1 selon l'une des revendications 1, 2 ou 3, dans laquelle
R² est un cyclopropyle, qui peut être facultativement substitué par un autre reste choisi dans le groupe constitué par -NH₂, CH₂-, NH₂, -NH(CH₃), -N(CH₃)₂, méthyle, éthyle, propyle, isopropyle, -NH-CO-(tert-butyle), -NH-CO-O-(tert-butyle), -N(CH₃)-CO-(tert-butyle), -N(CH₃)-CO-O-(tert-butyle), -CF₃, -CHF₂, CH₂F, F, Cl et Br,
ainsi que des sels, diastéréoisomères, énantiomères, racémates, hydrates ou solvates pharmacologiquement compatibles de ces composés.

7. Composés de formule 1 selon l'une des revendications 1 à 3, où
R² est un phényle qui peut être facultativement substitué dans une des positions méta ou dans les deux par un ou plusieurs restes choisis dans le groupe constitué par méthyle, éthyle, propyle, isopropyle, cyclopropyle, F, Cl, Br, OH, OR^{2.1}, COOR^{2.1}, CF₃, CHF₂, CH₂F, NH₂, NH(CH) et N(CH₃)₂, R^{2.1} pouvant être H, méthyle ou éthyle,
ainsi que des sels, diastéréoisomères, énantiomères, racémates, hydrates ou solvates pharmacologiquement compatibles de ces composés.

8. Composés de formule 1 selon l'une des revendications 1 à 3, dans laquelle
R² est un reste choisi dans un groupe constitué par un hétérocycle monocyclique saturé de trois, quatre, cinq, six ou sept termes contenant 1, 2 ou 3 hétéroaromes respectivement choisis dans le groupe constitué par N, O et S, qui peut être facultativement substitué par un ou plusieurs restes choisis dans le groupe fluoro, chloro, bromo, CF₃, CHF₂, CH₂F, OH et oxo ou par un ou plusieurs restes choisis dans le groupe OR^{2.1}, alkylène en C₁₋₃-OR^{2.1}, SR^{2.1}, SO-R^{2.1}, SO₂-R^{2.1}, COOR^{2.1}, COR^{2.1}, alcanol en C₁₋₆, cycloalkyle en C₃₋₁₀, phényle, alkyle en C₁₋₆, phénylalkylène en C₁₋₆, hétarylalkylène en C₁₋₆, hét, hétaryle et NR^{2.2}R^{2.3}, qui peut lui-même être facultativement substitué par un ou plusieurs restes choisis dans le groupe constitué par OH, OR^{2.1}, oxo, F, Cl, CF₃, CHF₂, CH₂F, alkyle en C₁₋₆, phényle et NR^{2.2}R^{2.3},
où R^{2.1}, R^{2.2} et R^{2.3} sont tels que définis dans la revendication 1,
ainsi que des sels, diastéréoisomères, énantiomères, racémates, hydrates ou solvates pharmacologiquement compatibles de ces composés.

9. Composés de formule 1 selon la revendication 8, dans laquelle
R² est un reste choisi dans un groupe constitué par un hétérocycle monocyclique saturé de six termes contenant un hétéroatome choisi dans le groupe constitué par N, 0 et s, qui peut être facultativement substitué par un ou plusieurs restes choisis dans le groupe F, Cl, Br, CF₃, CHF₂, CH₂F, OH, oxo, NH₂, NHCH₃ et N(CH₃)₂, méthyle, éthyle, propyle, isopropyle, cyclopropyle, méthoxy et éthoxy,
ainsi que des sels, diastéréoisomères, énantiomères, racémates, hydrates ou solvates pharmacologiquement compatibles de ces composés.

10. Composés de formule 1 selon la revendication 8 ou la revendication 9, dans laquelle
R² est un reste choisi dans un groupe constitué par la pipéridine ou le tétrahydropyrane, qui peut être facultativement substitué par un ou plusieurs restes choisis dans le groupe F, Cl, Br, OH, CF₃, CHF₂, CH₂F, NH₂, NHCH₃, N(CH₃)₂, oxo, méthyle et méthoxy,
ainsi que des sels, diastéréoisomères, énantiomères, racémates, hydrates ou solvates pharmacologiquement compatibles de ces composés.

11. Composés de formule 1 selon l'une des revendications 1 à 3, dans laquelle
R³ est un naphtalène ou un phényle,
qui peut être facultativement substitué dans une position quelconque par un, deux ou trois restes choisis indépendamment les uns des autres dans le groupe constitué par fluoro, chloro, bromo, hydroxy, CN, méthyle, éthyle, propyle, isopropyle, cyclopropyle, CF₃, CHF₂, CH₂F, -OCH₃, OCH₂CH₃, SO₂-CH₃, SO₂-CH₂CH₃, COOCH₃ et CO-O-CH₂CH₃,
ainsi que des sels, diastéréoisomères, énantiomères, racémates, hydrates ou solvates pharmacologiquement compatibles de ces composés.

12. Composés de formule 1 selon la revendication 1 ou la revendication 2, où
R³ est un reste choisi dans le groupe constitué par hét et hétaryle, qui peut être facultativement substitué par un ou plusieurs restes choisis dans le groupe constitué par F, Cl, Br, CF₃, CHF₂, CH₂F, CN, OH, oxo, méthyle, éthyle, propyle, isopropyle, butyle, isobutyle, cyclopropyle, cycloalkyle en C₅₋₇, -O-méthyle, -O-éthyle, O-propyle, -O-isopropyle, -COO-méthyle, -COO-éthyle, -COO-propyle, -COO-isopropyle, SO₂-(CH₃), SO₂-(CH₂-CH₃), SO-(CH₃) , SO-(CH₂-CH₃), phényle, -CH₂-NH₂, -CH₂-NHCH₃ , -CH₂-N(CH₃)₂, NH₂, NHCH₃ , N(CH₃)₂, hét et hétaryle, qui peut lui-même être substitué par un ou plusieurs restes choisis dans le groupe OH, F, Cl, Br, CF₃, CHF₂, CH₂F, méthyle, éthyle, propyle, isopropyle, phényle, -COO-méthyle, -COO-éthyle, -COO-propyle, -COO-isopropyle, 0-méthyle, O-éthyle, O-propyle et 0-isopropyle,
et dans laquelle
R⁴ est H, CN, OH, CF₃, CHF₂, CH₂F, F, méthyle, éthyle, O-méthyle ou O-éthyle,
où
hét désigne un hétérocycle monocyclique de trois à sept termes, saturé ou partiellement saturé, ou un hétérocycle bicyclique condensé de sept à onze termes, saturé ou partiellement saturé, qui contient 1, 2 ou 3 hétéroatomes choisis indépendamment les uns des autres dans le groupe constitué par N, S ou 0,
et où
hétaryle désigne un hétéroaryle aromatique monocyclique de cinq à six termes, ou un hétéroaryle aromatique bicyclique condensé de sept à onze termes, qui contient respectivement 1, 2 ou 3 hétéroatomes choisis indépendamment les uns des autres dans le groupe constitué par N, S ou 0,
et où
le cycloalkyle peut être saturé ou partiellement saturé,
ainsi que des sels, diastéréoisomères, énantiomères, racémates, hydrates ou solvates pharmacologiquement compatibles de ces composés.

13. Composés de formule 1 selon la revendication 12, dans laquelle
R³ est un reste choisi parmi un hétérocycle bicyclique de sept à onze termes, saturé ou partiellement saturé, ou un hétéroaryle bicyclique de sept à onze termes qui est choisi dans le groupe constitué par les indole, dihydroindole, quinazoline, dihydroquinazoline, tétrahydroquinazoline, benzoisoxazole, dihydrobenzoisoxazole, benzoxazine, dihydrobenzoxazine, benzothiazole, dihydrobenzothiazole, triazolopyridine, dihydrotriazolopyridine, benzofurane, dihydrobenzofurane, isobenzofurane et dihydroisobenzofurane,
lequel peut être facultativement substitué par un ou plusieurs restes choisis dans le groupe constitué par F, Cl, Br, CF₃, CHF₂, CH₂F, CN, OH, oxo, méthyle, éthyle, propyle, isopropyle, butyle, isobutyle, cyclopropyle, -O-méthyle, -O-éthyle, -O-propyle, -O-isopropyle, -COO-méthyle, -COO-éthyle, -COO-propyle, -COO-isopropyle, SO₂-(CH₃), SO₂-(CH₂-CH₃), SO-(CH₃), SO-(CH₂-CH₃) , phényle, -CH₂-NH₂, -CH₂-NHCH₃, -CH₂-N(CH₃)₂, NH₂, NHCH₃, N(CH₃)₂, furanyle et pyridinyle,
qui peut lui-même être substitué par un ou plusieurs restes choisis dans le groupe OH, F, Cl, Br, CF₃, CHF₂, CH₂F, méthyle, éthyle, propyle, isopropyle, phényle, -COO-méthyle, -COO-éthyle, O-méthyle et O-éthyle,
ainsi que des sels, diastéréoisomères, énantiomères, racémates, hydrates ou solvates pharmacologiquement compatibles de ces composés.

14. Composés de formule 1 selon la revendication 12, dans laquelle
R³ est un reste choisi parmi un hétérocycle monocyclique de trois à sept termes, saturé ou partiellement saturé, ou un hétéroaryle monocyclique de cinq à six termes qui est choisi dans le groupe constitué par les imidazole, dihydroimidazole, oxadiazole, oxadiazolidine, pyrazole, pyridine et dihydropyrazole,
lequel peut être facultativement substitué par un ou plusieurs restes choisis dans le groupe constitué par F, Cl, Br, CF₃, CHF₂, CH₂F, CN, OH, oxo, méthyle, éthyle, propyle, isopropyle, butyle, isobutyle, cyclopropyle, -O-méthyle, -O-éthyle, -O-propyle, -0-isopropyle, -COO- méthyle, -COO-éthyle, -COO-propyle, -COO-isopropyle, SO₂-(CH₃), SO₂-(CH₂-CH₃), SO-(CH₃), SO-(CH₂-CH₃), phényle, -CH₂-NH₂, -CH₂-NHCH₃, -CH₂-N(CH₃)₂, NH₂, NHCH₃, N(CH₃)₂, furanyle et pyridinyle, qui peut lui-même être substitué par un ou plusieurs restes choisis dans le groupe OH, F, Cl, Br, CF₃, CHF₂, CH₂F, méthyle, éthyle, propyle, isopropyle, phényle, -COO-méthyle, -COO-éthyle, O-méthyle et 0-éthyle,
ainsi que des sels, diastéréoisomères, énantiomères, racémates, hydrates ou solvates pharmacologiquement compatibles de ces composés.

15. Composés de formule 1 selon l'une des revendications 1 à 3, dans laquelle
A³ et R⁴ forment ensemble un hétérocycle de trois à onze termes mono- ou bicyclique, insaturé ou partiellement saturé, qui contient 1, 2 ou 3 hétéroatomes choisis dans le groupe constitué par N, O et S et qui peut être facultativement substitué par un ou plusieurs restes choisis dans le groupe constitué par F, Cl, Br, OH, oxo, CF₃, CHF₂, CH₂F, CN, méthyle, éthyle, propyle, isopropyle, cyclopropyle, COO-méthyle, -COO-éthyle, O-méthyle, O-éthyle, SO₂-(CH₃), SO₂-(CH₂-CH₃), SO-(CH₃), SO-(CH₂-CH₃), phényle, -CH₂-NH₂, -CH₂-NHCH₃, -CH₂-N(CH₃)₂, NH₂, NHCH₃, N(CH₃)₂, un hétérocycle de cinq à six termes saturé ou partiellement saturé et un hétéroaryle de cinq à six termes,
ainsi que des sels, diastéréoisomères, énantiomères, racémates, hydrates ou solvates pharmacologiquement compatibles de ces composés.

16. composés de formule 1 selon la revendication 15, dans laquelle
R³ et R⁴ forment ensemble un hétérocycle bicyclique choisi dans le groupe constitué par les tétrahydroquinazoline, tétrahydrobenzoxazine, dihydroindole et dihydroisobenzofurane, qui peut être facultativement substitué par un ou plusieurs restes choisis dans le groupe constitué par F, Cl, Br, OH, oxo, CF₃, CHF₂, CH₂F, CN, méthyle, éthyle, propyle, isopropyle, cyclopropyle, COO-méthyle, -COO-éthyle, O-méthyle, O-éthyle, SO₂-(CH₃), SO₂-(CH₂-CH₃), phényle, -CH₂-NH₂, -CH₂-NHCH₃, -CH₂-N(CH₃)₂, NH₂, NHCH₃, N(CH₃)₂, un hétérocycle de cinq ou six termes saturé ou partiellement saturé, et un hétéroaryle de cinq ou six termes,
ainsi que des sels, diastéréoisomères, énantiomères, racémates, hydrates ou solvates pharmacologiquement compatibles de ces composés.

17. Composés de formule 1 selon la revendication 1 ou la revendication 2, dans laquelle
R³ est -O-R^{3.1},
où R^{3.1} est un reste choisi dans le groupe constitué par les méthyle, éthyle, propyle, isopropyle, butyle, isobutyle, pentyle, isopentyle, -phényle,-méthylène- phényle, -éthylène-phényle, -propylène-phényle, -isopropylène-phényle, hétaryle et hét,
lequel est facultativement substitué en position ortho, para ou méta par un, deux ou trois restes choisis indépendamment les uns des autres dans le groupe constitué par fluoro, chloro, bromo, hydroxy, CN, méthyle, éthyle, propyle, isopropyle, butyle, isobutyle, -CF₃, CHF₂, CH₂F, CO-(méthyle), CO-(éthyle), CO-(propyle), CO-(isopropyle), CO-(butyle), CO-(isobutyle), -CO-(CF₃), -CO-(CH₂F), -CO-(CHF₂), -CO-NH-(méthylène)-hétaryle, -CO-NH-(éthylène)-hétaryle, -CO-NH-(propylène)-hétaryle, -CO-NH-(isopropylène)-hétaryle, -CO-N(CH₃)-(méthylène)-hétaryle, -CO-N(CH₃)-(éthylène)-hétaryle, -CO-N(CH₃)-(propylène)-héraryle, -CO-N(CH₃)-(isopropylène)-hétaryle, -CO-N(CH₃)-hét, -CO-N(cycloalkyle en C₃₋₇)-hét, -méthylène-O-méthyle, -éthylène-O-méthyle, -méthylène-O-éthyle, -éthylène-O-éthyle, -méthylène-NH₂, -éthylène-NH₂, -méthylène-NHCH₃, -éthylène-NHCH₃, -méthylène-N(CH₃)₂, -éthylène-N(CH₃)₂, -NH₂, -NHCH₃, -N(CH₃)₂, -O-méthyle, -O-éthyle, -O-propyle, -O-isopropyle, -SO-CH₃, -SO-(CH₂CH₃), -SO₂-CH₃, -SO₂-(CH₂CH₃), COOH, COO-(méthyle), COO-(éthyle), COO-(propyle), COO-(isopropyle), -O-méthylène-N(méthyle)₂, -O-éthylène-N(méthyle)₂, -O-méthylène-N(éthyle)₂, -O-éthylène-N(éthyle)₂, CO-NH₂, CO-NHCH₃, CO-N(CH₃)₂, NH-CO-méthyle, NCH₃-CO-méthyle, NH-CO-éthyle, N(CH₃)-CO-éthyle, phényle, phénylméthylène-, phényléthylène-, hét-méthylène-, hét-éthylène-, -CO-hét, hét, -Co-cycloalkyle an C₄₋₇, -Co-cyclopropyle, -CO-N(CH₃)-cyclopropyle, -CO-N(CH₃)-cycloalkyle en C₄₋₇, cycloalkyle en C₄₋₇, cyclopropyle, cycloalkyle en C₄₋₇-méthylène, cyclopropylméthylène-,cycloalkyle en C₄₋₇-éthylène-, cyclopropyléthylène-, hétarylméthylène-, hétaryléthylène- et hétaryle,
lequel peut lui-même être facultativement substitué par 1, 2, 3 ou 4 restes choisis indépendamment les uns des autres dans le groupe constitué par F, Cl, Br, méthyle, O-méthyle, éthyle, O-éthyle, OH, oxo et CF₃,
ainsi que des sels, diastéréoisomères, énantiomères, racémates, hydrates ou solvates pharmacologiquement compatibles de ces composés.

18. Composés de formule 1 selon l'une des revendications 1, 2 ou 3, dans laquelle
R⁴ est H, CN, OH, CF₃, CHF₂, CH₂F, F, méthyle, éthyle, 0-méthyle ou O-éthyle, -méthylène-OH, -éthylène-OH, -propylène-OH, isopropylène-OH, -COO-(méthyle), -COO- (éthyle), -COO(propyle), -COO-(isopropyle), -CO-hét, -(méthylène)-NH-SO₂-(méthyle), -(méthylène)-NH-SO₂-(éthyle), -(éthylène)-NH-SO₂-(méthyle), -(éthylène)-NH-SO₂-(éthyle), -(méthylène)-N(CH₃)-SO₂-(méthyle), -(méthylène)-N(CH₃)-SO₂-(éthyle), -(éthylène)-N(CH₃)-SO₂-(méthyle), -(éthylène)-N(CH₃)-SO₂-(éthyle), -(méthylène)-O-(méthylène)-phényle, -(méthylène)-O-(éthylène)-phényle, -(éthylène)-O-(méthylène)-phényle, -(éthylène)-O-(éthylène)-phényle, -méthylène-O-méthyle, -méthylène-O-éthyle, -éthylène-O-méthyle, -éthylène-O-éthyle, -(méthylène)-N(CH₃)-CO-(méthyle), - (méthylène)-N(CH₃)-CO-(éthyle), -(éthylène)-N(CH₃)-CO-(méthyle), -(éthylène)-N(CH₃)-CO-(éthyle), -NH-CO-(méthylène)-O-(méthyle), -NH-CO-(méthylène)-O-(éthyle), -NH-CO-(éthylène)-O-(méthyle), -NH-CO-(éthylène)-O-(éthyle), -méthylène-NH-CO-(méthyle), -méthylène-NH-CO-(éthyle), -éthylène-NH-CO-(méthyle), -éthylène-NH-CO-(éthyle), -méthylène-NH-CO-(méthylène)-N(méthyle)₂, -méthylène-NH-CO-(éthylène)-N(méthyle)2, -éthylène-NH-CO-(méthylène)-N(méthyle)₂, -éthylène-NH-CO-(éthylène)-N(méthyle)₂, -méthylène-NH-CO-(méthylène)-O-(méthyle), -méthylène-NH-CO-(éthylène)-O-(méthyle), -éthylène-NH-CO-(méthylène)-O-(méthyle), -méthylène-NH-CO-(méthylène)-O-(éthyle), -méthylène-NH-CO-(éthylène)-O-(éthyle), -éthylène-NH-CO-(méthylène)-O-(éthyle), -(méthylène)-N(CH₃)-CO-(méthylène)-O-(méthyle), -(méthylène)-N(CH₃)-CO-(éthylène)-O-(méthyle), -(éthylène)-N(CH₃)-CO-(méthylène)-O-(méthyle), -(méthylène)-N(CH₃)-CO-(méthylène)-O-(éthyle), -(méthylène)-N(CH₃)-CO-(éthylène)-O-(éthyle), -(éthylène)-N(CH₃)-CO-(méthylène)-O-(éthyle), -O-(méthylène)-phényle, -O-(éthylène)-phényle, -CO-phényle,
le phényle dans les restes ci-dessus pouvant être facultativement substitué par un ou plusieurs autres restes choisis dans le groupe F, Cl, Br, méthyle, éthyle, propyle, -O-méthyle, -O-éthyle, -O-propyle, -OH et CF₃
ainsi que des sels, diastéréoisomères, énantiomères, racémates, hydrates ou solvates pharmacologiquement compatibles de ces composés.

19. Composés de formule 1 selon l'une des revendications 1, 2 ou 3, dans laquelle
R³ est un reste choisi dans le groupe constitué par les oxazole, imidazole et thiazole, ce reste pouvant être facultativement substitué par un, deux ou trois autres restes choisis indépendamment les uns des autres dans le groupe constitué par méthyle, éthyle, propyle, isopropyle, O-méthyle, O-éthyle, O-propyle, O-isopropyle, OH, F, Cl, Br, CF₃, phényle, hétaryle et cycloalkyle en C₃₋₆,
ainsi que des sels, diastéréoisomères, énantiomères, racémates, hydrates ou solvates pharmacologiquement compatibles de ces composés.

20. Composés de formule 1 selon l'une des revendications 1 à 3, lesdits composés étant choisis dans le groupe constitué par 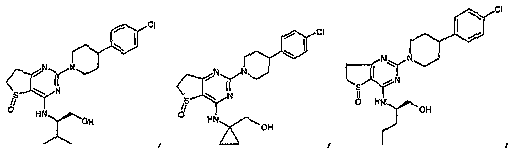 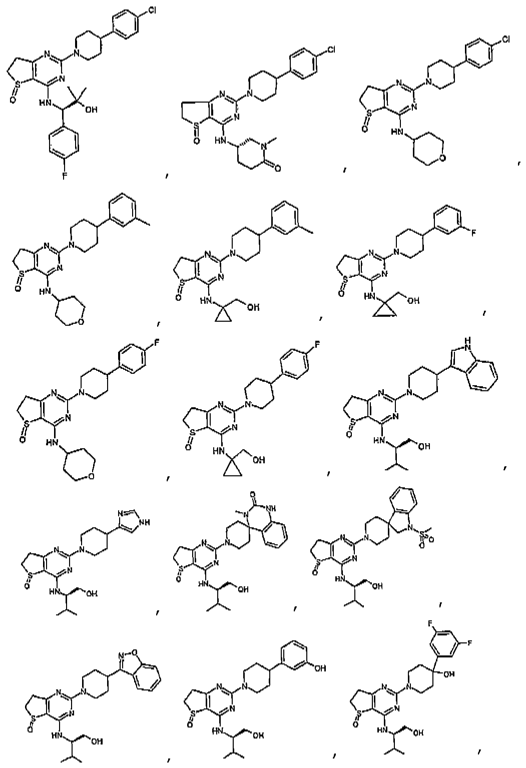 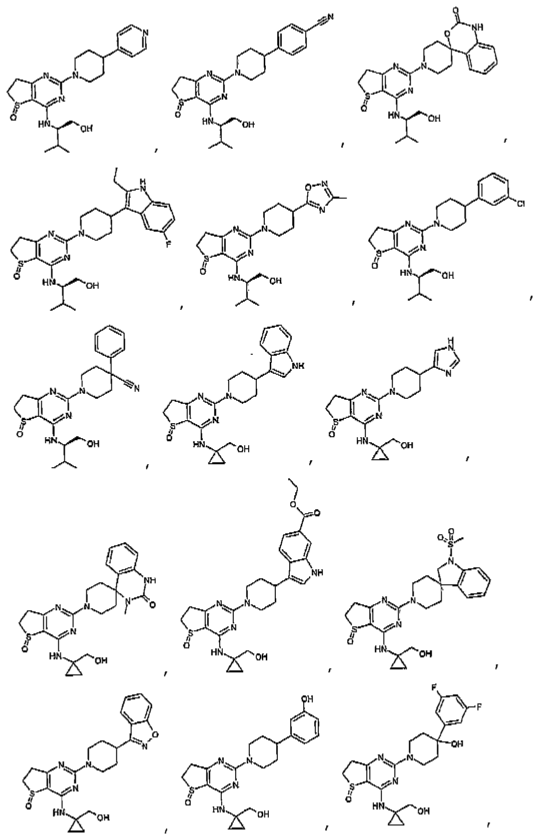 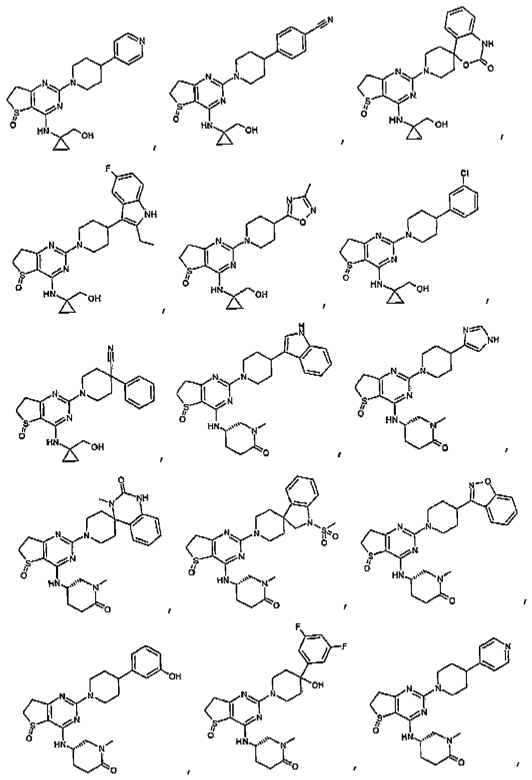 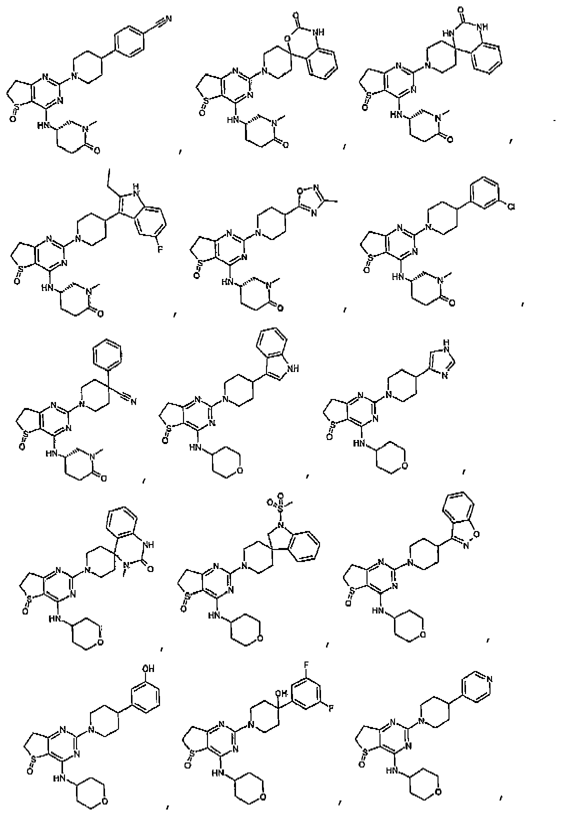 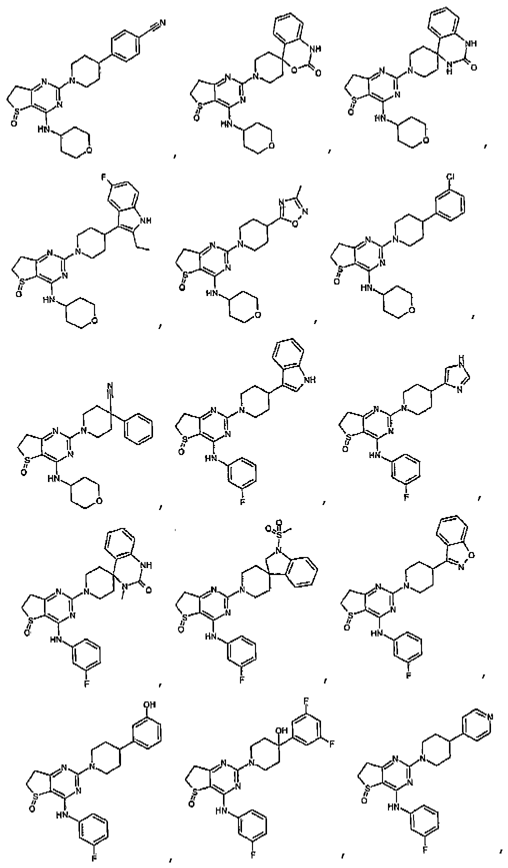 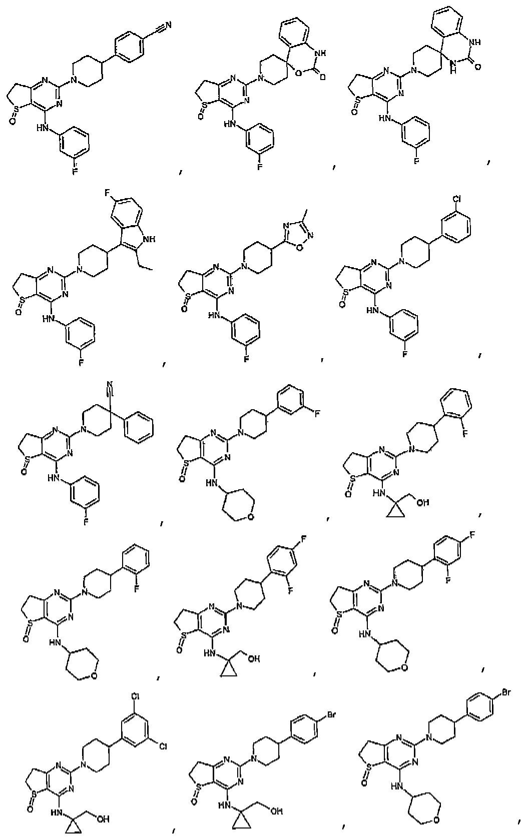 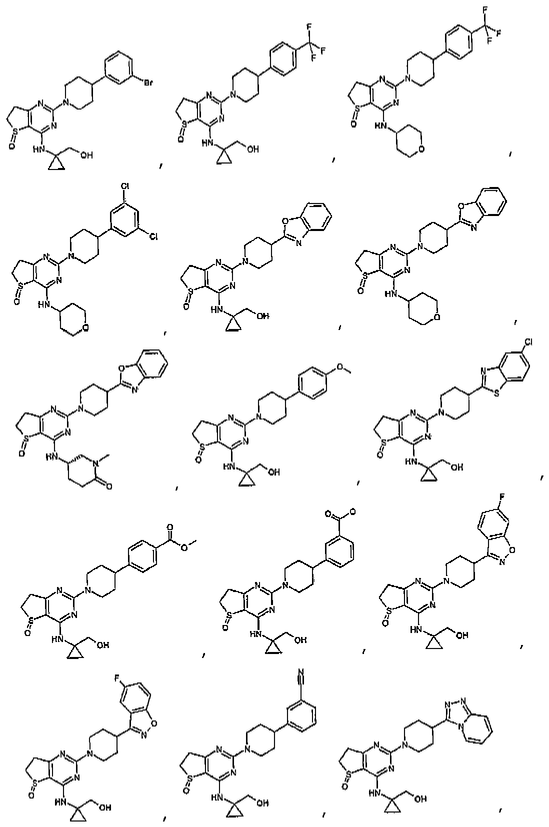 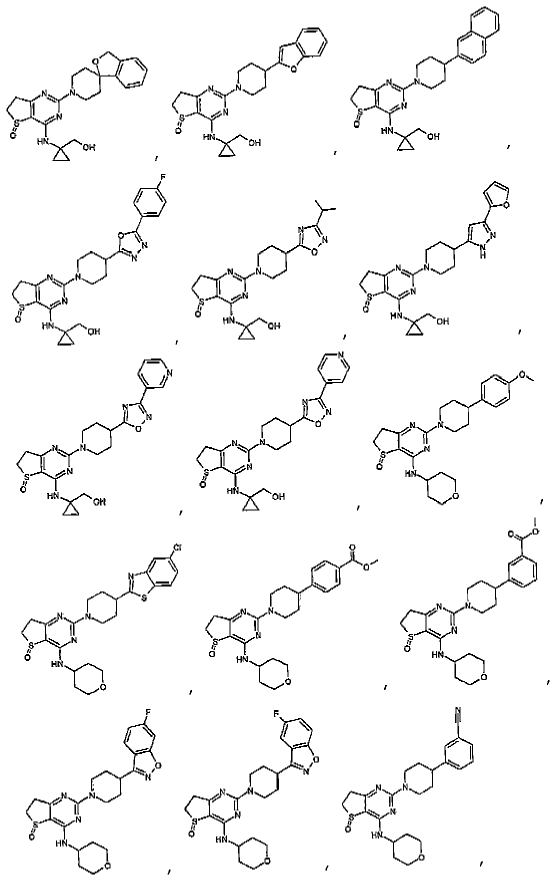 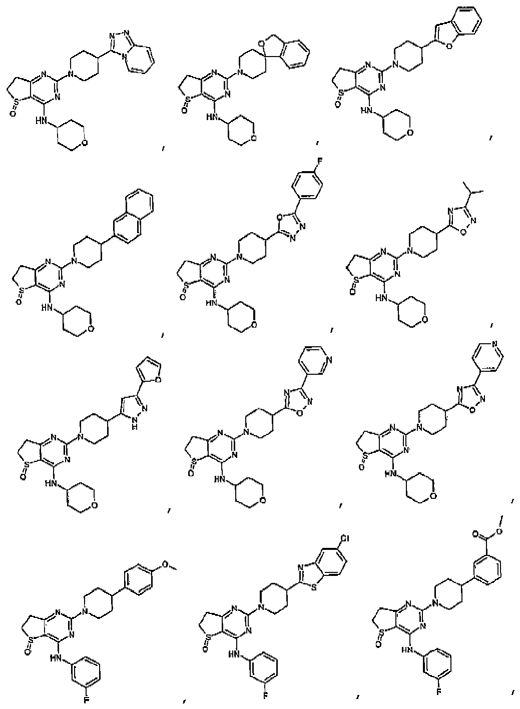 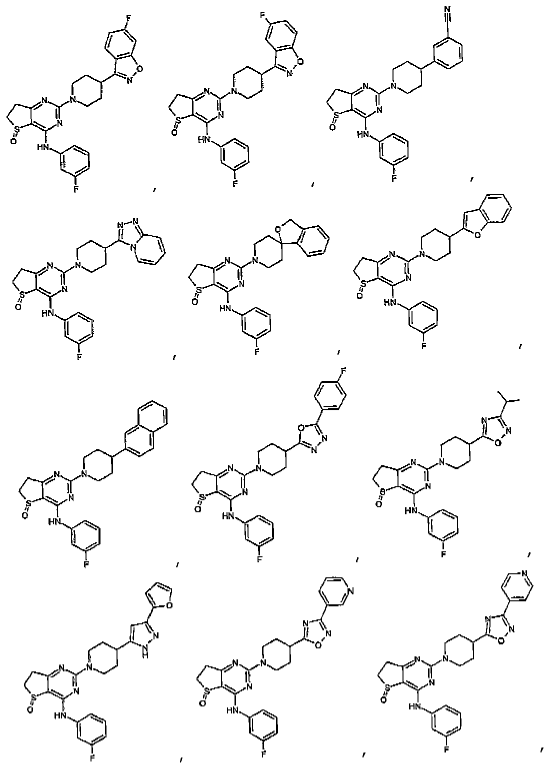 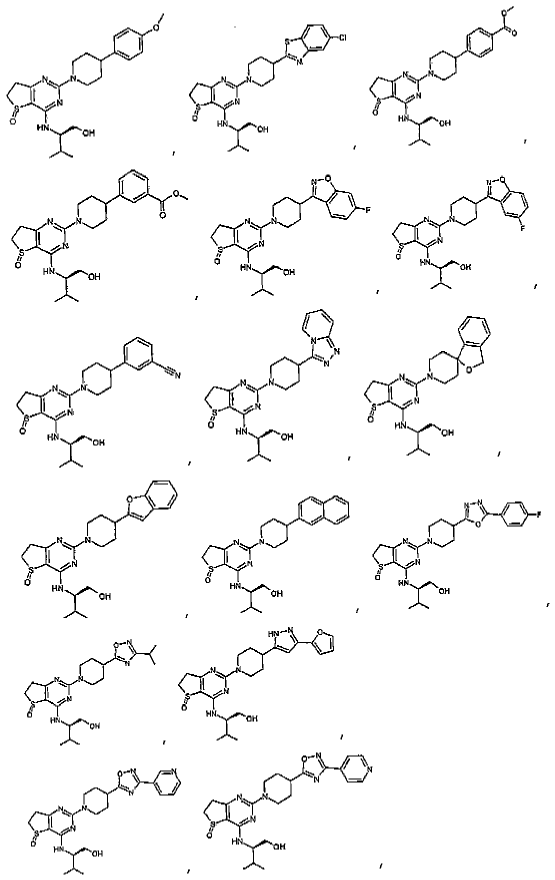 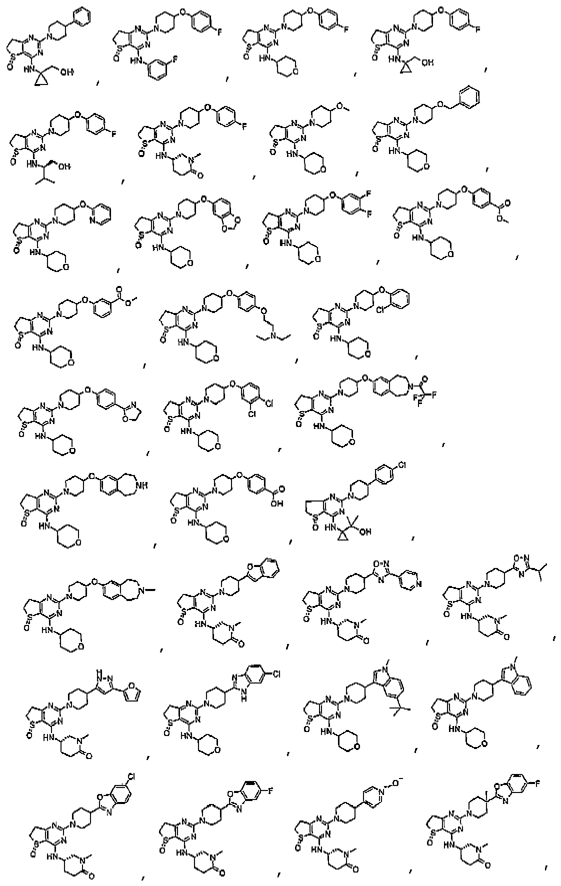 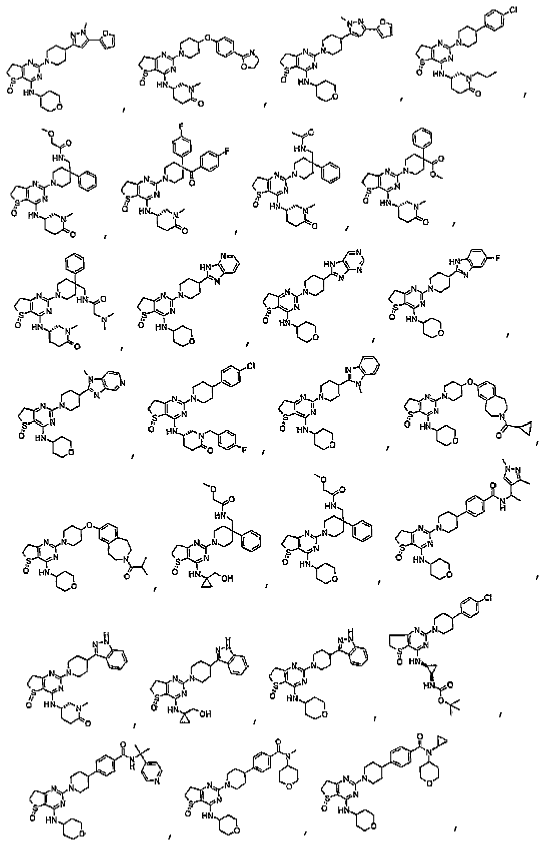 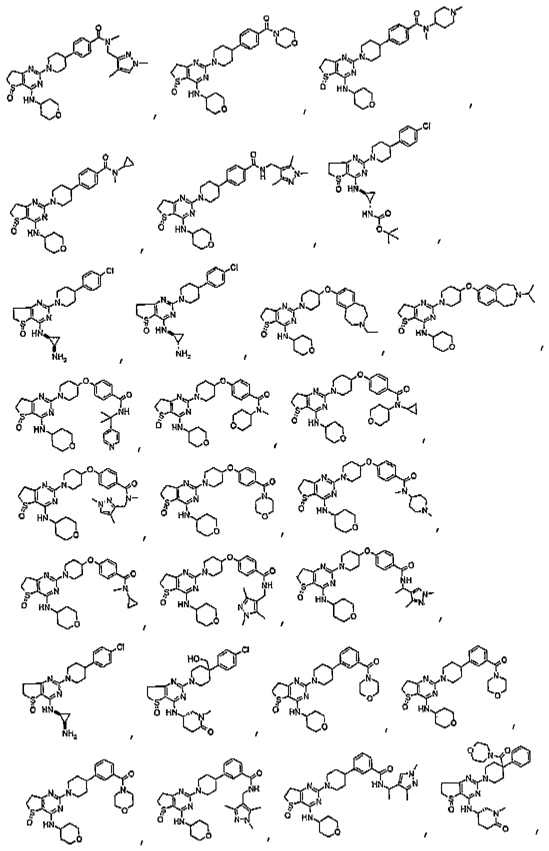 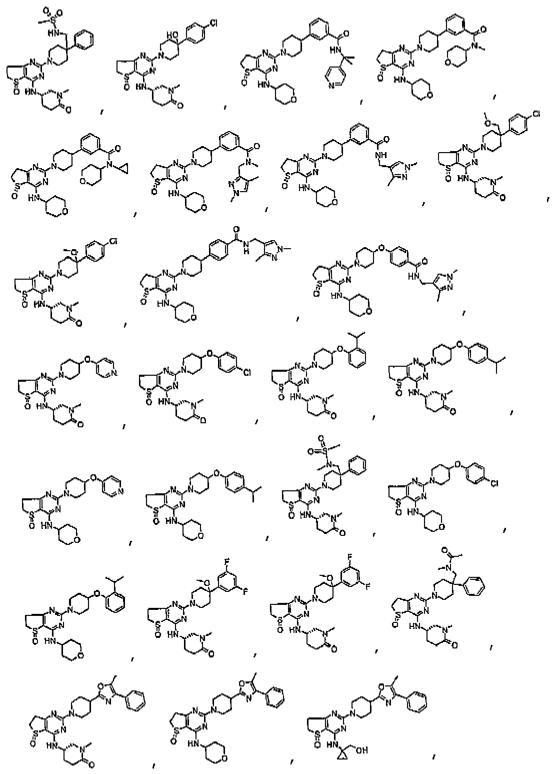 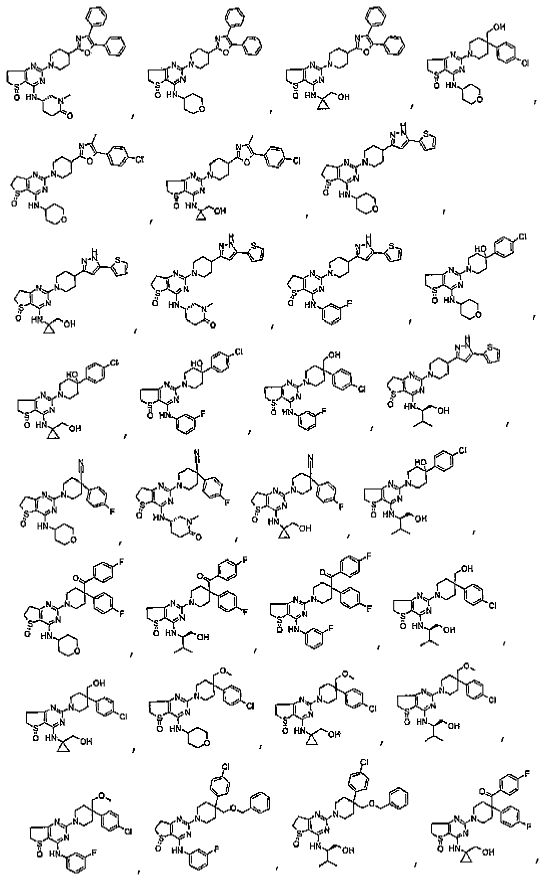 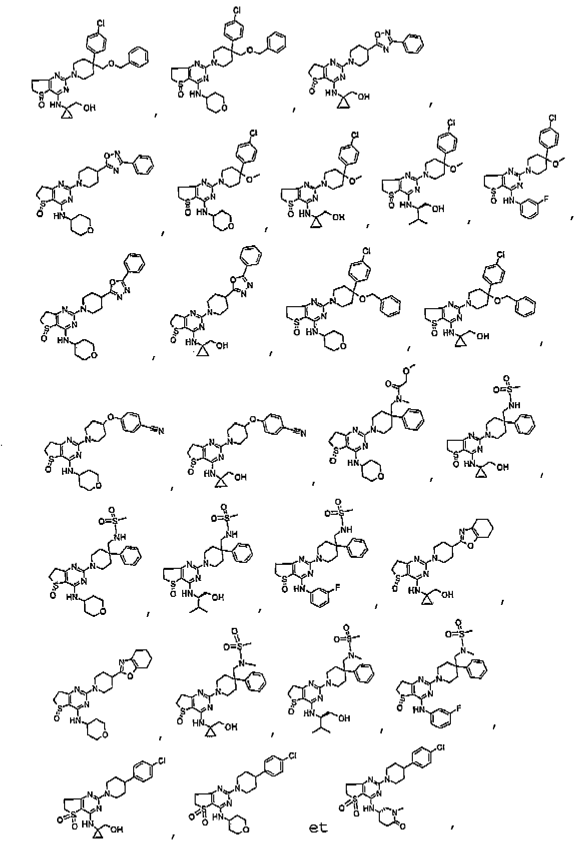 ainsi que des sels, diastéréoisomères, énantiomères, racémates, hydrates ou solvates pharmacologiquement compatibles de ces composés.

21. Composé de formule 5 selon l'une des revendications 1 à 3 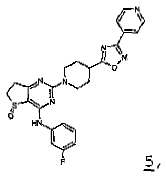 ainsi que des sels, diastéréoisomères, énantiomères, racémates, hydrates ou solvates pharmacologiquement compatibles de ce composé.

22. Composé de formule 6 selon l'une des revendications 1 à 3 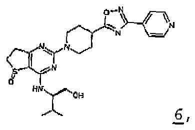 ainsi que des sels, diastéréoisomères, énantiomères, racémates, hydrates ou solvates pharmacologiquement compatibles de ce composé.

23. Composé de formule 7 selon l'une des revendications 1 à 3 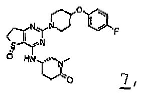 ainsi que des sels, diastéréoisomères, énantiomères, racémates, hydrates ou solvates pharmacologiquement compatibles de ce composé.

24. Composé de formule 8 selon l'une des revendications 1 à 3 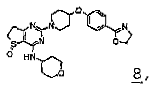 ainsi que des sels, diastéréoisomères, énantiomères, racémates, hydrates ou solvates pharmacologiquement compatibles de ce composé.

25. Composé de formule 9 selon l'une des revendications 1 à 3 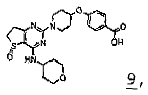 ainsi que des sels, diastéréoisomères, énantiomères, racémates, hydrates ou solvates pharmacologiquement compatibles de ce composé.

26. Composé de formule 10 selon l'une des revendications 1 à 3 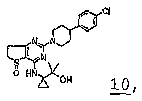 ainsi que des sels, diastéréoisomères, enantiomères, racémates, hydrates ou solvates pharmacologiquement compatibles de ce composé.

27. composé de formule 11 selon l'une des revendications 1 à 3 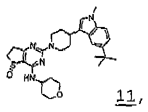 ainsi que des sels, diastéréoisomères, énantiomères, racémates, hydrates ou solvates pharmacologiquement compatibles de ce composé.

28. Composé de formule 12 selon l'une des revendications 1 à 3 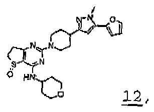 ainsi que des sels, diastéréoisomères, énantiomères, racémates, hydrates ou solvates pharmacologiquement compatibles de ce composé.

29. Composé de formule 13 selon l'une des revendications 1 à 3 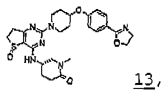 ainsi que des sels, diastéréoisomères, énantiomères, racémates, hydrates ou solvates pharmacologiquement compatibles de ce composé.

30. Composé de formule 14 selon l'une des revendications 1 à 3 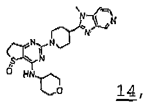 ainsi que des sels, diastéréoisomères, énantiomères, racémates, hydrates ou solvates pharmacologiquement compatibles de ce composé.

31. Composé de formule 15 selon l'une des revendications 1 à 3 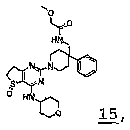 ainsi que des sels, diastéréoisomères, énantiomères, racémates, hydrates ou solvates pharmacologiquement compatibles de ce composé.

32. Composé de formule 16 selon l'une des revendications 1 à 3 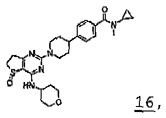 ainsi que des sels, diastéréoisomères, énantiomères, racémates, hydrates ou solvates pharmacologiquement compatibles de ce composé.

33. composé de formule 17 selon l'une des revendications 1 à 3 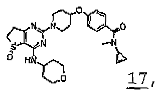 ainsi que des sels, diastéréoisomères, énantiomères, racémates, hydrates ou solvates pharmacologiquement compatibles de ce composé.

34. Composé de formule 18 selon l'une des revendications 1 à 3 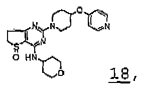 ainsi que des sels, diastéréoisomères, énantiomères, racémates, hydrates ou solvates pharmacologiquement compatibles de ce composé.

35. Composé de formule 19 selon l'une des revendications 1 à 3 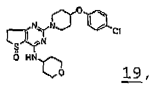 ainsi que des sels, diastéréoisomères, énantioméres, racémates, hydrates ou solvates pharmacologiquement compatibles de ce composé.

36. Composé de formule 20 selon l'une des revendications 1 à 3 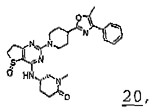 ainsi que des sels, diastéréoisomères, énantiomères, racémates, hydrates ou solvates pharmacologiquement compatibles de ce composé.

37. Composé de formule 21 selon l'une des revendications 1 à 3 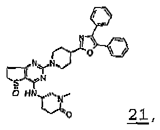 ainsi que des sels, diastéréoisomères, énantiomères, racémates, hydrates ou solvates pharmacologiquement compatibles de ce composé.

38. Composé de formule 22 selon l'une des revendications 1 à 3 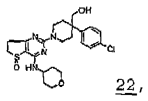 ainsi que des sels, diastéréoisomères, énantiomères, racémates, hydrates ou solvates pharmacalogiquement compatibles de ce composé.

39. Composé de formule 23 selon l'une des revendications 1 à 3 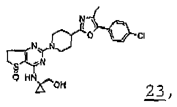 ainsi que des sels, diastéréoisomères, énantiomères, racémates, hydrates ou solvates pharmacologiquement compatibles de ce composé.

40. Composé de formule 24 selon l'une des revendications 1 à 3 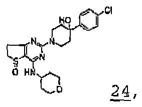 ainsi que des sels, diastéréoisomères, énantiomères, racémates, hydrates ou solvates pharmacologiquement compatibles de ce composé.

41. Composé de formule 25 selon l'une des revendication 1 à 3 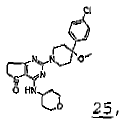 ainsi que des sels, diastéréoisomères, énantiomères, racémates, hydrates ou solvates pharmacologiquement compatibles de ce composé.

42. Composé de formule 26 selon l'une des revendications 1 à 3 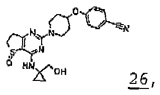 ainsi que des sels, diastéréoisomères, énantiomères, racémates, hydrates ou solvates pharmacologiquement compatibles de ce composé.

43. Composé de formule 27 selon l'une des revendications 1 à 3 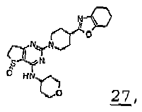 ainsi que des sels, diastéréoisomères, énantiomères, racémates, hydrates ou solvates pharmacologiquement compatibles de ce composé.

44. Composé de formule 28 selon l'une des revendications 1 à 3 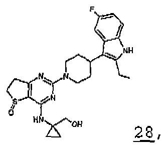 ainsi que des sels, diastéréoisomères, énantiomères, racémates, hydrates ou solvates pharmacologiquement compatibles de ce composé.

45. Composés selon l'une des revendications 1 à 44 en tant que médicaments.

46. Utilisation de composés selon l'une des revendications 1 à 44 pour préparer un médicament destiné au traitement de troubles ou de maladies des voies respiratoires ou du système gastro-intestinal, ainsi que de maladies inflammatoires des articulations, de la peau ou des yeux, de maladies cancéreuses ainsi que de maladies du système nerveux périphérique ou central.

47. Utilisation de composés selon l'une des revendications 1 à 44 pour préparer un médicament destiné à la prévention et au traitement de maladies des voies respiratoires ou des pourrons qui s'accompagnent d'une production accrue de mucosités, d'inflammations et/ou de maladies obstructives des voies respiratoires.

48. Utilisation de composés selon l'une des revendications 1 à 44 pour préparer un médicament destiné au traitement de maladies inflammatoires et obstructives telles que la BPCO, la sinusite chronique, l'asthme, l'iléite régionale, la rectocolite hémorragique.

49. Utilisation de composés selon l'une des revendications 1 à 44 pour préparer un médicament destiné à la prévention et au traitement de maladies du système nerveux périphérique ou central telles que la dépression, la dépression bipolaire ou maniaque, les états d'angoisse aiguë et chronique, la schizophrénie, la maladie d'Alzheimer, la maladie de Parkinson, la sclérose en plaques aiguë et chronique ou les états de douleur aiguë et chronique ainsi que les lésions cérébrales provoquées par un accident vasculaire, une hypoxie ou un traumatisme crânio-cérébral.

50. Formulations pharmaceutiques **caractérisées par** une teneur en un ou plusieurs composés de formule 1 selon l'une des revendications 1 à 44 en combinaison avec une ou plusieurs substances actives choisies dans le groupe constitué par les anticholinergiques, les bétamimétiques, les corticostéroïdes, d'autres inhibiteurs de la PDE4, des inhibiteurs de l'EGFR et des antagonistes du LTD4, des inhibiteurs du CCR3, des inhibiteurs de l'iNOS et des inhibiteurs de la SYK.
